(19) Europäisches Patentamt
European Patent Office
Office européen des brevets

(11) EP 4 238 953 A1

(12) **EUROPEAN PATENT APPLICATION**
published in accordance with Art. 153(4) EPC

(43) Date of publication:
**06.09.2023 Bulletin 2023/36**

(21) Application number: **21885810.8**

(22) Date of filing: **01.10.2021**

(51) International Patent Classification (IPC):
***C07C 13/66*** (2006.01) ***C07D 335/04*** (2006.01)
***C09K 11/06*** (2006.01) ***C07D 311/78*** (2006.01)
***H05B 33/12*** (2006.01) ***H01L 51/50*** (2006.01)

(52) Cooperative Patent Classification (CPC):
**C07C 13/66; C07D 311/78; C07D 335/04; C09K 11/06; H05B 33/12; H10K 50/00;**
Y02E 10/549

(86) International application number:
**PCT/JP2021/036397**

(87) International publication number:
**WO 2022/091691 (05.05.2022 Gazette 2022/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.10.2020 JP 2020179704**
**19.02.2021 JP 2021025304**
**31.03.2021 JP 2021062404**
**07.09.2021 JP 2021145819**

(71) Applicant: **Idemitsu Kosan Co., Ltd**
**Tokyo 100-8321 (JP)**

(72) Inventors:
- **ITOI Hiroaki**
  **Tokyo 100-8321 (JP)**
- **NAKANO Yuki**
  **Tokyo 100-8321 (JP)**
- **YAMAKI Taro**
  **Tokyo 100-8321 (JP)**
- **IIDA Maiko**
  **Tokyo 100-8321 (JP)**
- **MORITA Takamoto**
  **Tokyo 100-8321 (JP)**
- **BAN Shintaro**
  **Tokyo 100-8321 (JP)**
- **TAKAHASHI Ryota**
  **Tokyo 100-8321 (JP)**
- **KUDO Yu**
  **Tokyo 100-8321 (JP)**
- **SHIRASAKI Yoshinao**
  **Tokyo 100-8321 (JP)**

(74) Representative: **Gille Hrabal Partnerschaftsgesellschaft mbB**
**Patentanwälte**
**Brucknerstraße 20**
**40593 Düsseldorf (DE)**

(54) **COMPOUND, ORGANIC ELECTROLUMINESCENT ELEMENT AND ELECTRONIC DEVICE**

(57) A compound includes: at least one group represented by a formula (11) below; and a single benz[de]anthracene derivative skeleton represented by a formula (1000) below in a molecule, in which $Ar_1$ is a substituted or unsubstituted aryl group including at least four rings, at least one of $R_{10}$ to $R_{19}$ is a group represented by the formula (11), $L_1$ is a substituted or unsubstituted arylene group having 6 to 15 ring carbon atoms or a substituted or unsubstituted divalent heterocyclic group having 5 to 15 ring atoms, and mx is 1, 2, or 3.

EP 4 238 953 A1

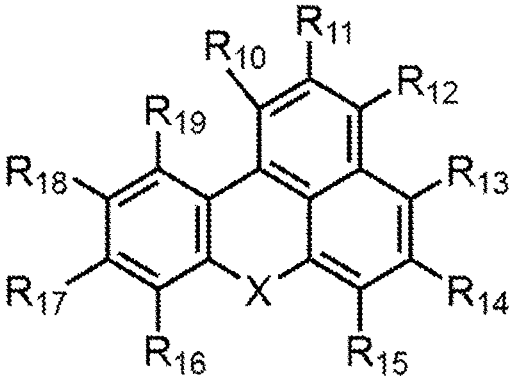

(1000)

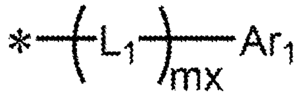

(11)

## Description

TECHNICAL FIELD

The present invention relates to a compound, an organic electroluminescence device, and an electronic device.

BACKGROUND ART

An organic electroluminescence device (hereinafter, occasionally referred to as "organic EL device") has found its application in a full-color display for mobile phones, televisions and the like. When a voltage is applied to an organic EL device, holes are injected from an anode and electrons are injected from a cathode into an emitting layer. The injected electrons and holes are recombined in the emitting layer to form excitons. Specifically, according to the electron spin statistics theory, singlet excitons and triplet excitons are generated at a ratio of 25%:75%.

Various studies have been made on a compound to be used for an organic EL device in order to enhance the performance of the organic EL device (see, for instance, Patent Literatures 1 to 4). The performance of the organic EL device is evaluable in terms of, for instance, luminance, emission wavelength, chromaticity, luminous efficiency, drive voltage, and lifetime.

CITATION LIST

PATENT LITERATURE(S)

Patent Literature 1 US Patent Application Publication No. 2015/270498
Patent Literature 2 US Patent Application Publication No. 2015/001479
Patent Literature 3 US Patent Application Publication No. 2015/069344
Patent Literature 4 US Patent Application Publication No. 2017/331051

SUMMARY OF THE INVENTION

PROBLEM(S) TO BE SOLVED BY THE INVENTION

An object of the invention is to provide a compound capable of improving performance of an organic EL device, an organic electroluminescence device containing the compound, and an electronic device including the organic electroluminescence device.

Another object of the invention is to provide a compound capable of providing an organic electroluminescence device, in which a plurality of emitting layers are layered, in a favorable balance between a luminous efficiency and a lifetime when the compound is used in an emitting layer close to an anode of the organic electroluminescence device, and to provide an electronic device including the organic electroluminescence device.

MEANS FOR SOLVING THE PROBLEM(S)

According to an aspect of the invention, there is provided a compound having at least one group represented by a formula (11) below and a single benz[de]anthracene derivative skeleton represented by a formula (1000) below in a molecule.

[Formula 1]

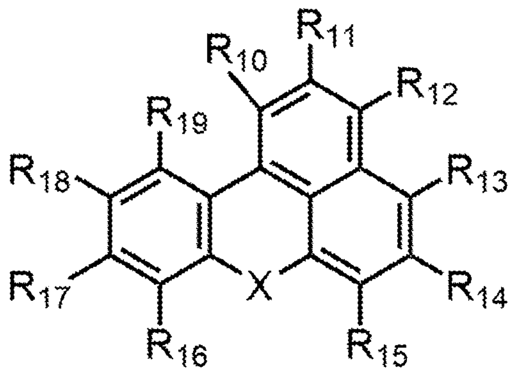

(1000)

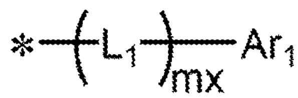

(11)

In the formula (1000):

X is an oxygen atom, a sulfur atom, $C(R_{2001})(R_{2002})$, or $Si(R_{2003})(R_{2004})$;

$R_{2001}$ to $R_{2004}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms;

at least one combination of adjacent two or more of $R_{10}$ to $R_{19}$ are mutually bonded to form a substituted or unsubstituted monocyclic ring, mutually bonded to form a substituted or unsubstituted fused ring, or not mutually bonded;

$R_{10}$ to $R_{19}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by $-C(=O)R_{801}$, a group represented by $-COOR_{802}$, a halogen atom, a nitro group, a substituted or unsubstituted aryl group having 6 to 50 ring carbon atoms, a substituted or unsubstituted heterocyclic group having 5 to 50 ring atoms, or a group represented by the formula (11), and

at least one of $R_{10}$ to $R_{19}$ is a group represented by the formula (11);

when a plurality of groups represented by the formula (11) are present, the plurality of groups represented by the formula (11) are mutually the same or different;

$L_1$ is a substituted or unsubstituted arylene group having 6 to 15 ring carbon atoms, or a substituted or unsubstituted divalent heterocyclic group having 5 to 15 ring atoms;

mx is 1, 2, or 3;

when two or more $L_1$ are present, the two or more $L_1$ are mutually the same or different;

$Ar_1$ is a substituted or unsubstituted aryl group including four or more rings;

when two or more $Ar_1$ are present, the two or more $Ar_1$ are mutually the same or different;

* in the formula (11) represents a bonding position;

in a compound represented by the formula (1000), $R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{901}$ are present, the plurality of $R_{901}$ are mutually the same or different;

when a plurality of $R_{902}$ are present, the plurality of $R_{902}$ are mutually the same or different;

when a plurality of $R_{903}$ are present, the plurality of $R_{903}$ are mutually the same or different;

when a plurality of $R_{904}$ are present, the plurality of $R_{904}$ are mutually the same or different;

when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{801}$ are present, the plurality of $R_{801}$ are mutually the same or different; and
when a plurality of $R_{802}$ are present, the plurality of $R_{802}$ are mutually the same or different.

According to another aspect of the invention, there is provided a compound having at least one group represented by a formula (110A) below and a single benz[de]anthracene derivative skeleton represented by a formula (1000A) below in a molecule.

[Formula 2]

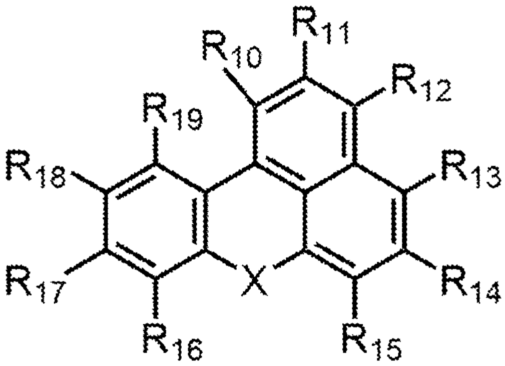

(1000A)

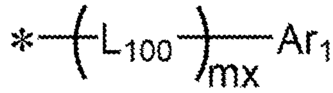

(110A)

In the formula (1000A):

X is an oxygen atom, a sulfur atom, $C(R_{2001})(R_{2002})$, or $Si(R_{2003})(R_{2004})$;
$R_{2001}$ to $R_{2004}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms;
at least one combination of adjacent two or more of $R_{10}$ to $R_{19}$ are mutually bonded to form a substituted or unsubstituted monocyclic ring, mutually bonded to form a substituted or unsubstituted fused ring, or not mutually bonded;
$R_{10}$ to $R_{19}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $- S-(R_{905})$, a group represented by $-N(R_{906})(R_{907})$, a substituted or unsubstituted aralkyl group having 7 to 50 carbon atoms, a group represented by $-C(=O)R_{801}$, a group represented by $-COOR_{802}$, a halogen atom, a nitro group, a substituted or unsubstituted aryl group having 6 to 50 ring carbon atoms, a substituted or unsubstituted heterocyclic group having 5 to 50 ring atoms, or a group represented by the formula (110A);
at least one of $R_{13}$ or $R_{18}$ is a group represented by the formula (110A);
when a plurality of groups represented by the formula (110A) are present, the plurality of groups represented by the formula (110A) are mutually the same or different;
$L_{100}$ is a single bond, a substituted or unsubstituted arylene group having 6 to 50 ring carbon atoms, or a substituted or unsubstituted divalent heterocyclic group having 5 to 50 ring atoms;
mx is 1, 2, or 3;
when two or more $L_{100}$ are present, the two or more $L_{100}$ are mutually the same or different;
$Ar_1$ is a substituted or unsubstituted aryl group including four or more rings;

when two or more $Ar_1$ are present, the two or more $Ar_1$ are mutually the same or different;
* in the formula (110A) represents a bonding position;
in a compound represented by the formula (1000A), $R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;
when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{801}$ are present, the plurality of $R_{801}$ are mutually the same or different; and
when a plurality of $R_{802}$ are present, the plurality of $R_{802}$ are mutually the same or different.

According to still another aspect of the invention, there is provided an organic electroluminescence device including: a cathode, an anode, and at least one organic layer provided between the cathode and the anode, in which the at least one organic layer includes an emitting layer and at least one of the at least one organic layer contains the compound according to the above aspect of the invention.

According to a further aspect of the invention, there is provided an organic electroluminescence device including: a cathode, an anode, and at least one emitting layer provided between the cathode and the anode, in which the at least one emitting layer includes a first emitting layer and a second emitting layer, the first emitting layer contains a first compound, and the first compound is a compound represented by a formula (1000B) below and having at least one group represented by a formula (110) below.

[Formula 3]

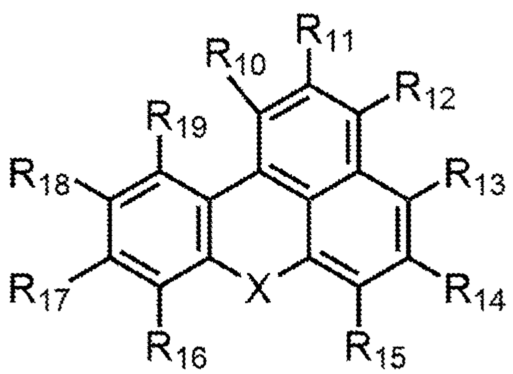

(1000B)

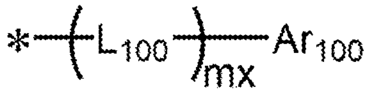

(110)

In the formula (1000B):

X is an oxygen atom, a sulfur atom, $C(R_{2001})(R_{2002})$, or $Si(R_{2003})(R_{2004})$;
$R_{2001}$ to $R_{2004}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms;
at least one combination of adjacent two or more of $R_{10}$ to $R_{19}$ are mutually bonded to form a substituted or unsubstituted monocyclic ring, mutually bonded to form a substituted or unsubstituted fused ring, or not mutually bonded;
$R_{10}$ to $R_{19}$ forming neither the substituted or unsubstituted monocyclic ring nor the substituted or unsubstituted fused ring are each independently a hydrogen atom, a substituted or unsubstituted alkyl group having 1 to 50 carbon

atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by $-C(=O)R_{801}$, a group represented by $-COOR_{802}$, a halogen atom, a nitro group, a substituted or unsubstituted aryl group having 6 to 50 ring carbon atoms, a substituted or unsubstituted heterocyclic group having 5 to 50 ring atoms, or a group represented by the formula (110);
at least one of $R_{10}$ to $R_{19}$ is a group represented by the formula (110);
when a plurality of groups represented by the formula (110) are present, the plurality of groups represented by the formula (110) are mutually the same or different;
$L_{100}$ is a single bond, a substituted or unsubstituted arylene group having 6 to 50 ring carbon atoms, or a substituted or unsubstituted divalent heterocyclic group having 5 to 50 ring atoms;
mx is 1, 2, or 3;
when two or more $L_{100}$ are present, the two or more $L_{100}$ are mutually the same or different;
$Ar_{100}$ is a substituted or unsubstituted aryl group including three or more rings or a substituted or unsubstituted heterocyclic group including two or more aromatic rings and one or more heterocyclic rings;
$Ar_{100}$ does not include an anthracene ring;
when two or more $Ar_{100}$ are present, the two or more $Ar_{100}$ are mutually the same or different; and
* in the formula (110) represents a bonding position;
in the first compound represented by the formula (1000B), $R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;
when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{801}$ are present, the plurality of $R_{801}$ are mutually the same or different; and
when a plurality of $R_{802}$ are present, the plurality of $R_{802}$ are mutually the same or different.

According to a still further aspect of the invention, there is provided an electronic device including the organic electroluminescence device according to the above aspect of the invention.

According to the above aspects of the invention, there can be provided a compound capable of improving performance of an organic EL device, an organic electroluminescence device containing the compound, and an electronic device including the organic electroluminescence device.

Moreover, according to the above aspects of the invention, there can be provided a compound capable of providing an organic electroluminescence device, in which a plurality of emitting layers are layered, in a favorable balance between a luminous efficiency and a lifetime when the compound is used in an emitting layer close to an anode of the organic electroluminescence device, and to provide an electronic device including the organic electroluminescence device.

## BRIEF DESCRIPTION OF DRAWING(S)

Fig. 1 schematically shows an exemplary arrangement of an organic electroluminescence device according to an exemplary embodiment of the invention.

## DESCRIPTION OF EMBODIMENT(S)

### Definitions

Herein, a hydrogen atom includes isotopes having different numbers of neutrons, specifically, protium, deuterium and tritium.

In chemical formulae herein, it is assumed that a hydrogen atom (i.e. protium, deuterium and tritium) is bonded to each of bondable positions that are not annexed with signs "R" or the like or "D" representing a deuterium.

Herein, the ring carbon atoms refer to the number of carbon atoms among atoms forming a ring of a compound (e.g., a monocyclic compound, fused-ring compound, crosslinking compound, carbon ring compound, and heterocyclic compound) in which the atoms are bonded with each other to form the ring. When the ring is substituted by a substituent(s), carbon atom(s) contained in the substituent(s) is not counted in the ring carbon atoms. Unless otherwise specified, the same applies to the "ring carbon atoms" described later. For instance, a benzene ring has 6 ring carbon atoms, a naphthalene ring has 10 ring carbon atoms, a pyridine ring has 5 ring carbon atoms, and a furan ring has 4 ring carbon atoms. Further, for instance, 9,9-diphenylfluorenyl group has 13 ring carbon atoms and 9,9'-spirobifluorenyl group has 25 ring carbon atoms.

When a benzene ring is substituted by a substituent in a form of, for instance, an alkyl group, the number of carbon atoms of the alkyl group is not counted in the number of the ring carbon atoms of the benzene ring. Accordingly, the benzene ring substituted by an alkyl group has 6 ring carbon atoms. When a naphthalene ring is substituted by a substituent in a form of, for instance, an alkyl group, the number of carbon atoms of the alkyl group is not counted in the number of the ring carbon atoms of the naphthalene ring. Accordingly, the naphthalene ring substituted by an alkyl group has 10 ring carbon atoms.

Herein, the ring atoms refer to the number of atoms forming a ring of a compound (e.g., a monocyclic compound, fused-ring compound, crosslinking compound, carbon ring compound, and heterocyclic compound) in which the atoms are bonded to each other to form the ring (e.g., monocyclic ring, fused ring, and ring assembly). Atom(s) not forming the ring (e.g., hydrogen atom(s) for saturating the valence of the atom which forms the ring) and atom(s) in a substituent by which the ring is substituted are not counted as the ring atoms. Unless otherwise specified, the same applies to the "ring atoms" described later. For instance, a pyridine ring has 6 ring atoms, a quinazoline ring has 10 ring atoms, and a furan ring has 5 ring atoms. For instance, the number of hydrogen atom(s) bonded to a pyridine ring or the number of atoms forming a substituent are not counted as the pyridine ring atoms. Accordingly, a pyridine ring bonded with a hydrogen atom(s) or a substituent(s) has 6 ring atoms. For instance, the hydrogen atom(s) bonded to carbon atom(s) of a quinazoline ring or the atoms forming a substituent are not counted as the quinazoline ring atoms. Accordingly, a quinazoline ring bonded with hydrogen atom(s) or a substituent(s) has 10 ring atoms.

Herein, "XX to YY carbon atoms" in the description of "substituted or unsubstituted ZZ group having XX to YY carbon atoms" represent carbon atoms of an unsubstituted ZZ group and do not include carbon atoms of a substituent(s) of the substituted ZZ group. Herein, "YY" is larger than "XX," "XX" representing an integer of 1 or more and "YY" representing an integer of 2 or more.

Herein, "XX to YY atoms" in the description of "substituted or unsubstituted ZZ group having XX to YY atoms" represent atoms of an unsubstituted ZZ group and do not include atoms of a substituent(s) of the substituted ZZ group. Herein, "YY" is larger than "XX," "XX" representing an integer of 1 or more and "YY" representing an integer of 2 or more.

Herein, an unsubstituted ZZ group refers to an "unsubstituted ZZ group" in a "substituted or unsubstituted ZZ group," and a substituted ZZ group refers to a "substituted ZZ group" in a "substituted or unsubstituted ZZ group."

Herein, the term "unsubstituted" used in a "substituted or unsubstituted ZZ group" means that a hydrogen atom(s) in the ZZ group is not substituted with a substituent(s). The hydrogen atom(s) in the "unsubstituted ZZ group" is protium, deuterium, or tritium.

Herein, the term "substituted" used in a "substituted or unsubstituted ZZ group" means that at least one hydrogen atom in the ZZ group is substituted with a substituent. Similarly, the term "substituted" used in a "BB group substituted by AA group" means that at least one hydrogen atom in the BB group is substituted with the AA group.

## Substituents Mentioned Herein

Substituents mentioned herein will be described below.

An "unsubstituted aryl group" mentioned herein has, unless otherwise specified herein, 6 to 50, preferably 6 to 30, more preferably 6 to 18 ring carbon atoms.

An "unsubstituted heterocyclic group" mentioned herein has, unless otherwise specified herein, 5 to 50 ring atoms, preferably 5 to 30 ring atoms, more preferably 5 to 18 ring atoms.

An "unsubstituted alkyl group" mentioned herein has, unless otherwise specified herein, 1 to 50 carbon atoms, preferably 1 to 20 carbon atoms, more preferably 1 to 6 carbon atoms.

An "unsubstituted alkenyl group" mentioned herein has, unless otherwise specified herein, 2 to 50, preferably 2 to 20, more preferably 2 to 6 carbon atoms.

An "unsubstituted alkynyl group" mentioned herein has, unless otherwise specified herein, 2 to 50 carbon atoms, preferably 2 to 20 carbon atoms, more preferably 2 to 6 carbon atoms.

An "unsubstituted cycloalkyl group" mentioned herein has, unless otherwise specified herein, 3 to 50, preferably 3 to 20, more preferably 3 to 6 ring carbon atoms.

An "unsubstituted arylene group" mentioned herein has, unless otherwise specified herein, 6 to 50 ring carbon atoms, preferably 6 to 30 ring carbon atoms, more preferably 6 to 18 ring carbon atoms.

An "unsubstituted divalent heterocyclic group" mentioned herein has, unless otherwise specified herein, 5 to 50 ring atoms, preferably 5 to 30 ring atoms, more preferably 5 to 18 ring atoms.

An "unsubstituted alkylene group" mentioned herein has, unless otherwise specified herein, 1 to 50 carbon atoms, preferably 1 to 20 carbon atoms, more preferably 1 to 6 carbon atoms.

Substituted or Unsubstituted Aryl Group

Specific examples (specific example group G1) of the "substituted or unsubstituted aryl group" mentioned herein include unsubstituted aryl groups (specific example group G1A) below and substituted aryl groups (specific example group G1B). Herein, an unsubstituted aryl group refers to an "unsubstituted aryl group" in a "substituted or unsubstituted aryl group," and a substituted aryl group refers to a "substituted aryl group" in a "substituted or unsubstituted aryl group." A simply termed "aryl group" herein includes both of an "unsubstituted aryl group" and a "substituted aryl group."

The "substituted aryl group" refers to a group derived by substituting at least one hydrogen atom in an "unsubstituted aryl group" with a substituent. Examples of the "substituted aryl group" include a group derived by substituting at least one hydrogen atom in the "unsubstituted aryl group" in the specific example group G1A below with a substituent, and examples of the substituted aryl group in the specific example group G1B below. It should be noted that the examples of the "unsubstituted aryl group" and the "substituted aryl group" mentioned herein are merely exemplary, and the "substituted aryl group" mentioned herein includes a group derived by further substituting a hydrogen atom bonded to a carbon atom of a skeleton of a "substituted aryl group" in the specific example group G1B below, and a group derived by further substituting a hydrogen atom of a substituent of the "substituted aryl group" in the specific example group G1B below.

Unsubstituted Aryl Group (Specific Example Group G1A):

phenyl group, p-biphenyl group, m-biphenyl group, o-biphenyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-terphenyl-4-yl group, o-terphenyl-3-yl group, o-terphenyl-2-yl group, 1-naphthyl group, 2-naphthyl group, anthryl group, benzanthryl group, phenanthryl group, benzophenanthryl group, phenalenyl group, pyrenyl group, chrysenyl group, benzochrysenyl group, triphenylenyl group, benzotriphenylenyl group, tetracenyl group, pentacenyl group, fluorenyl group, 9,9'-spirobifluorenyl group, benzofluorenyl group, dibenzofluorenyl group, fluoranthenyl group, benzofluoranthenyl group, perylenyl group, and a monovalent aryl group derived by removing one hydrogen atom from cyclic structures represented by formulae (TEMP-1) to (TEMP-15) below.

[Formula 4]

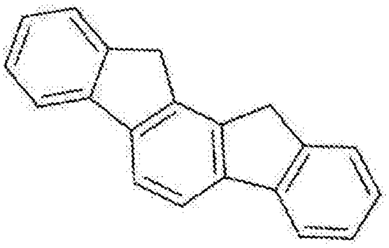

(TEMP-1)

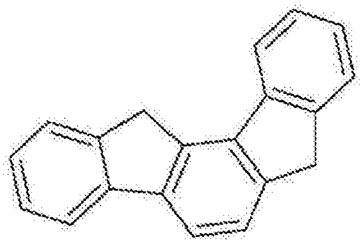

(TEMP-2)

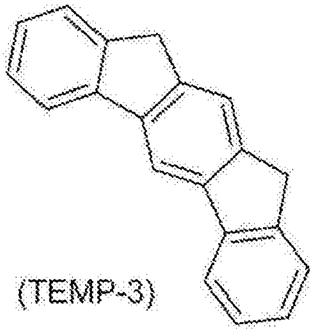

(TEMP-3)

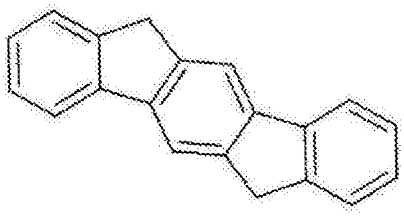

(TEMP-4)

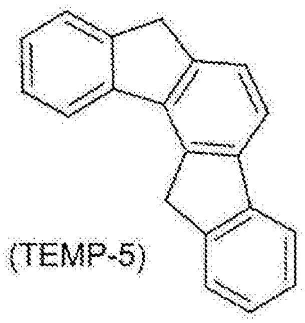

(TEMP-5)

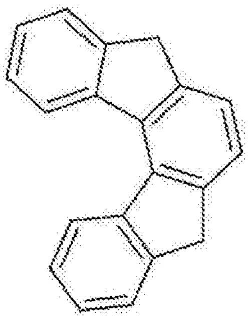

(TEMP-6)

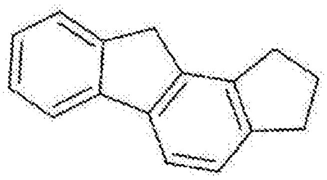

(TEMP-7)

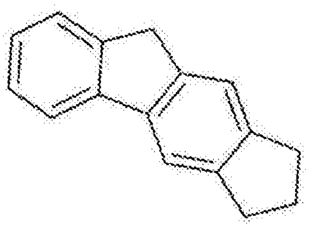

(TEMP-8)

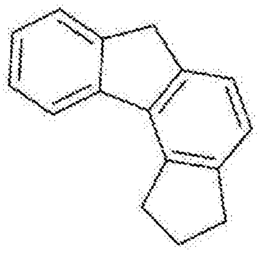

(TEMP-9)

[Formula 5]

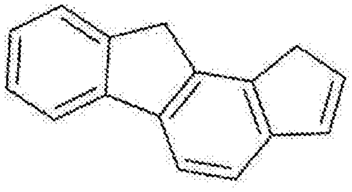

(TEMP-10)

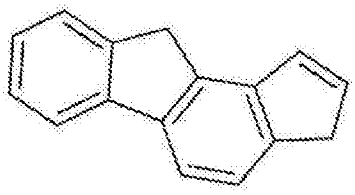

(TEMP-11)

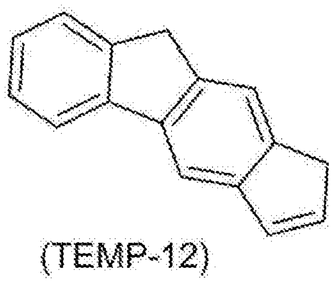

(TEMP-12)

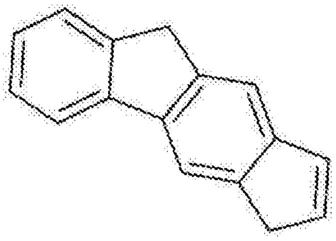

(TEMP-13)

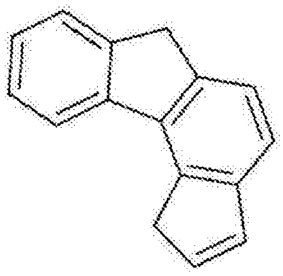

(TEMP-14)

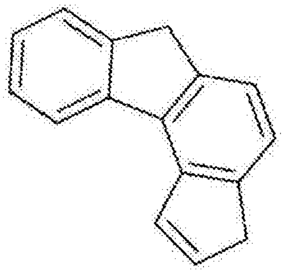

(TEMP-15)

Substituted Aryl Group (Specific Example Group G1B):

o-tolyl group, m-tolyl group, p-tolyl group, para-xylyl group, meta-xylyl group, ortho-xylyl group, para-isopropylphenyl group, meta-isopropylphenyl group, ortho-isopropylphenyl group, para-t-butylphenyl group, meta-t-butylphenyl group, ortho-t-butylphenyl group, 3,4,5-trimethylphenyl group, 9,9-dimethylfluorenyl group, 9,9-diphenylfluorenyl group, 9,9-bis(4-methylphenyl)fluorenyl group, 9,9-bis(4-isopropylphenyl)fluorenyl group, 9,9-bis(4-t-butylphenyl)fluorenyl group, cyanophenyl group, triphenylsilylphenyl group, trimethylsilylphenyl group, phenylnaphthyl group, naphthylphenyl group, and a group derived by substituting at least one hydrogen atom of a monovalent group derived from the cyclic structures represented by the formulae (TEMP-1) to (TEMP-15) with a substituent.

Substituted or Unsubstituted Heterocyclic Group

The "heterocyclic group" mentioned herein refers to a cyclic group having at least one hetero atom in the ring atoms. Specific examples of the hetero atom include a nitrogen atom, oxygen atom, sulfur atom, silicon atom, phosphorus atom, and boron atom.
The "heterocyclic group" mentioned herein is a monocyclic group or a fused-ring group.
The "heterocyclic group" mentioned herein is an aromatic heterocyclic group or a non-aromatic heterocyclic group.
Specific examples (specific example group G2) of the "substituted or unsubstituted heterocyclic group" mentioned herein include unsubstituted heterocyclic groups (specific example group G2A) and substituted heterocyclic groups (specific example group G2B). Herein, an unsubstituted heterocyclic group refers to an "unsubstituted heterocyclic group" in a "substituted or unsubstituted heterocyclic group," and a substituted heterocyclic group refers to a "substituted heterocyclic group" in a "substituted or unsubstituted heterocyclic group." A simply termed "heterocyclic group" herein includes both of an "unsubstituted heterocyclic group" and a "substituted heterocyclic group."
The "substituted heterocyclic group" refers to a group derived by substituting at least one hydrogen atom in an "unsubstituted heterocyclic group" with a substituent. Specific examples of the "substituted heterocyclic group" include a group derived by substituting at least one hydrogen atom in the "unsubstituted heterocyclic group" in the specific example group G2A below with a substituent, and examples of the substituted heterocyclic group in the specific example group G2B below. It should be noted that the examples of the "unsubstituted heterocyclic group" and the "substituted heterocyclic group" mentioned herein are merely exemplary, and the "substituted heterocyclic group" mentioned herein includes a group derived by further substituting a hydrogen atom bonded to a ring atom of a skeleton of a "substituted heterocyclic group" in the specific example group G2B below, and a group derived by further substituting a hydrogen atom of a substituent of the "substituted heterocyclic group" in the specific example group G2B below.
The specific example group G2A includes, for instance, unsubstituted heterocyclic groups including a nitrogen

atom (specific example group G2A1) below, unsubstituted heterocyclic groups including an oxygen atom (specific example group G2A2) below, unsubstituted heterocyclic groups including a sulfur atom (specific example group G2A3) below, and monovalent heterocyclic groups (specific example group G2A4) derived by removing a hydrogen atom from cyclic structures represented by formulae (TEMP-16) to (TEMP-33) below.

The specific example group G2B includes, for instance, substituted heterocyclic groups including a nitrogen atom (specific example group G2B1) below, substituted heterocyclic groups including an oxygen atom (specific example group G2B2) below, substituted heterocyclic groups including a sulfur atom (specific example group G2B3) below, and groups derived by substituting at least one hydrogen atom of the monovalent heterocyclic groups (specific example group G2B4) derived from the cyclic structures represented by formulae (TEMP-16) to (TEMP-33) below.

Unsubstituted Heterocyclic Groups Including Nitrogen Atom (Specific Example Group G2A1):

pyrrolyl group, imidazolyl group, pyrazolyl group, triazolyl group, tetrazolyl group, oxazolyl group, isoxazolyl group, oxadiazolyl group, thiazolyl group, isothiazolyl group, thiadiazolyl group, pyridyl group, pyridazynyl group, pyrimidinyl group, pyrazinyl group, triazinyl group, indolyl group, isoindolyl group, indolizinyl group, quinolizinyl group, quinolyl group, isoquinolyl group, cinnolyl group, phthalazinyl group, quinazolinyl group, quinoxalinyl group, benzimidazolyl group, indazolyl group, phenanthrolinyl group, phenanthridinyl group, acridinyl group, phenazinyl group, carbazolyl group, benzocarbazolyl group, morpholino group, phenoxazinyl group, phenothiazinyl group, azacarbazolyl group, and diazacarbazolyl group.

Unsubstituted Heterocyclic Groups Including Oxygen Atom (Specific Example Group G2A2):

furyl group, oxazolyl group, isoxazolyl group, oxadiazolyl group, xanthenyl group, benzofuranyl group, isobenzofuranyl group, dibenzofuranyl group, naphthobenzofuranyl group, benzoxazolyl group, benzisoxazolyl group, phenoxazinyl group, morpholino group, dinaphthofuranyl group, azadibenzofuranyl group, diazadibenzofuranyl group, azanaphthobenzofuranyl group, and diazanaphthobenzofuranyl group.

Unsubstituted Heterocyclic Groups Including Sulfur Atom (Specific Example Group G2A3):

thienyl group, thiazolyl group, isothiazolyl group, thiadiazolyl group, benzothiophenyl group (benzothienyl group), isobenzothiophenyl group (isobenzothienyl group), dibenzothiophenyl group (dibenzothienyl group), naphthobenzothiophenyl group (nahthobenzothienyl group), benzothiazolyl group, benzisothiazolyl group, phenothiazinyl group, dinaphthothiophenyl group (dinaphthothienyl group), azadibenzothiophenyl group (azadibenzothienyl group), diazadibenzothiophenyl group (diazadibenzothienyl group), azanaphthobenzothiophenyl group (azanaphthobenzothienyl group), and diazanaphthobenzothiophenyl group (diazanaphthobenzothienyl group).

Monovalent Heterocyclic Groups Derived by Removing One Hydrogen Atom from Cyclic Structures Represented by Formulae (TEMP-16) to (TEMP-33) (Specific Example Group G2A4):

[Formula 6]

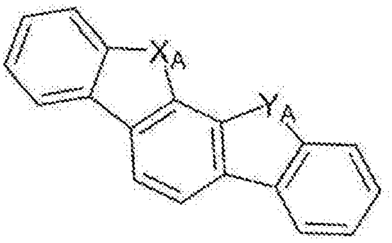

(TEMP-16)

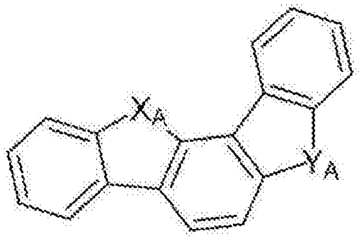

(TEMP-17)

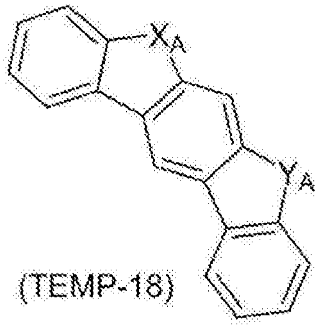

(TEMP-18)

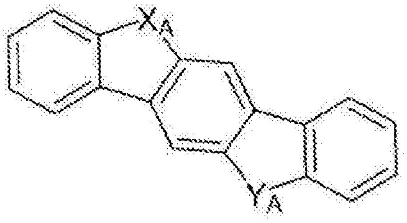

(TEMP-19)

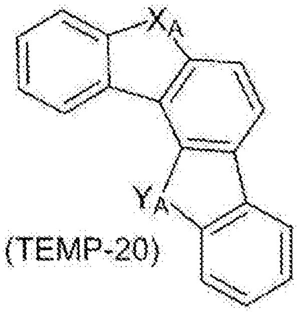

(TEMP-20)

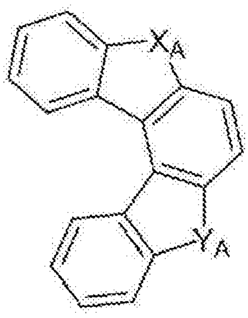

(TEMP-21)

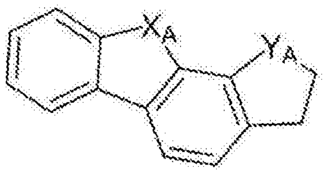

(TEMP-22)

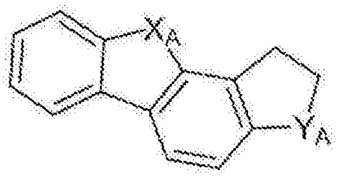

(TEMP-23)

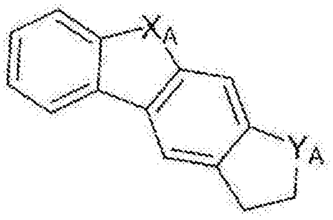

(TEMP-24)

[Formula 7]

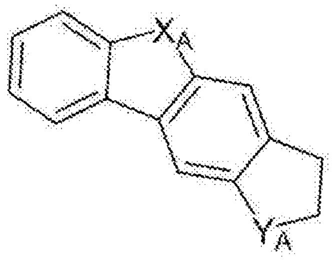
(TEMP-25)

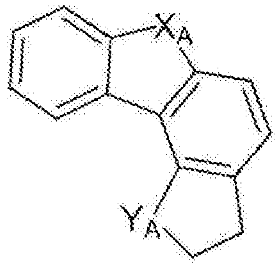
(TEMP-26)

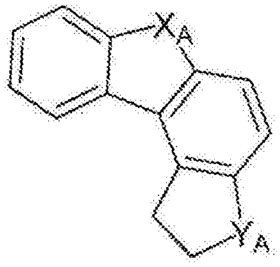
(TEMP-27)

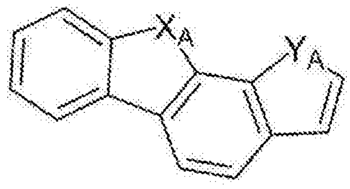
(TEMP-28)

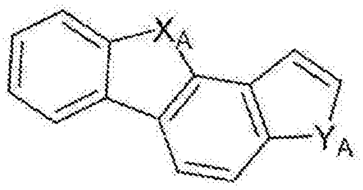
(TEMP-29)

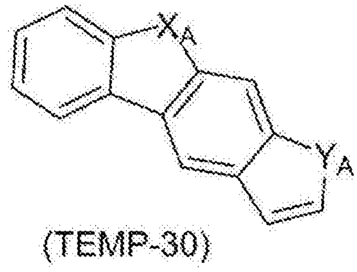
(TEMP-30)

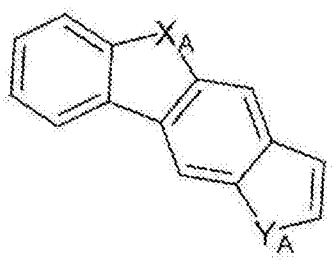
(TEMP-31)

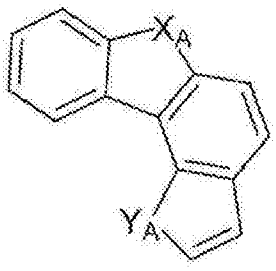
(TEMP-32)

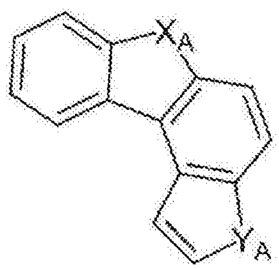
(TEMP-33)

In the formulae (TEMP-16) to (TEMP-33), $X_A$ and $Y_A$ are each independently an oxygen atom, a sulfur atom, NH, or $CH_2$. However, at least one of $X_A$ or $Y_A$ is an oxygen atom, a sulfur atom, or NH.
When at least one of $X_A$ or $Y_A$ in the formulae (TEMP-16) to (TEMP-33) is NH or $CH_2$, the monovalent heterocyclic groups derived from the cyclic structures represented by the formulae (TEMP-16) to (TEMP-33) include a monovalent group derived by removing one hydrogen atom from NH, or $CH_2$.

Substituted Heterocyclic Groups Including Nitrogen Atom (Specific Example Group G2B1):

(9-phenyl)carbazolyl group, (9-biphenylyl)carbazolyl group, (9-phenyl)phenylcarbazolyl group, (9-naphthyl)carbazolyl group, diphenylcarbazole-9-yl group, phenylcarbazole-9-yl group, methylbenzimidazolyl group, ethylbenzimidazolyl group, phenyltriazinyl group, biphenylyltriazinyl group, diphenyltriazinyl group, phenylquinazolinyl group, and biphenylquinazolinyl group.

Substituted Heterocyclic Groups Including Oxygen Atom (Specific Example Group G2B2):

phenyldibenzofuranyl group, methyldibenzofuranyl group, t-butyldibenzofuranyl group, and monovalent residue of spiro[9H-xanthene-9,9'-[9H]fluorene].

Substituted Heterocyclic Groups Including Sulfur Atom (Specific Example Group G2B3):

phenyldibenzothiophenyl group, methyldibenzothiophenyl group, t-butyldibenzothiophenyl group, and monovalent residue of spiro[9H-thioxanthene-9,9'-[9H]fluorene].

Groups Obtained by Substituting at Least One Hydrogen Atom of Monovalent Heterocyclic Group Derived from Cyclic Structures Represented by Formulae (TEMP-16) to (TEMP-33) with Substituent (Specific Example Group G2B4):

The "at least one hydrogen atom of a monovalent heterocyclic group" means at least one hydrogen atom selected from a hydrogen atom bonded to a ring carbon atom of the monovalent heterocyclic group, a hydrogen atom bonded to a nitrogen atom of at least one of $X_A$ or $Y_A$ in a form of NH, and a hydrogen atom of one of $X_A$ and $Y_A$ in a form of a methylene group ($CH_2$).

Substituted or Unsubstituted Alkyl Group

Specific examples (specific example group G3) of the "substituted or unsubstituted alkyl group" mentioned herein include unsubstituted alkyl groups (specific example group G3A) and substituted alkyl groups (specific example group G3B below). Herein, an unsubstituted alkyl group refers to an "unsubstituted alkyl group" in a "substituted or unsubstituted alkyl group," and a substituted alkyl group refers to a "substituted alkyl group" in a "substituted or unsubstituted alkyl group." A simply termed "alkyl group" herein includes both of an "unsubstituted alkyl group" and a "substituted alkyl group."

The "substituted alkyl group" refers to a group derived by substituting at least one hydrogen atom in an "unsubstituted alkyl group" with a substituent. Specific examples of the "substituted alkyl group" include a group derived by substituting at least one hydrogen atom of an "unsubstituted alkyl group" (specific example group G3A) below with a substituent, and examples of the substituted alkyl group (specific example group G3B) below. Herein, the alkyl group for the "unsubstituted alkyl group" refers to a chain alkyl group. Accordingly, the "unsubstituted alkyl group" include linear "unsubstituted alkyl group" and branched "unsubstituted alkyl group." It should be noted that the examples of the "unsubstituted alkyl group" and the "substituted alkyl group" mentioned herein are merely exemplary, and the "substituted alkyl group" mentioned herein includes a group derived by further substituting a hydrogen atom of a skeleton of the "substituted alkyl group" in the specific example group G3B, and a group derived by further substituting a hydrogen atom of a substituent of the "substituted alkyl group" in the specific example group G3B.

Unsubstituted Alkyl Group (Specific Example Group G3A):

methyl group, ethyl group, n-propyl group, isopropyl group, n-butyl group, isobutyl group, s-butyl group, and t-butyl group.

Substituted Alkyl Group (Specific Example Group G3B):

heptafluoropropyl group (including isomer thereof), pentafluoroethyl group, 2,2,2-trifluoroethyl group, and trifluoromethyl group.

Substituted or Unsubstituted Alkenyl Group

Specific examples (specific example group G4) of the "substituted or unsubstituted alkenyl group" mentioned herein include unsubstituted alkenyl groups (specific example group G4A) and substituted alkenyl groups (specific example group G4B). Herein, an unsubstituted alkenyl group refers to an "unsubstituted alkenyl group" in a "substituted or unsubstituted alkenyl group," and a substituted alkenyl group refers to a "substituted alkenyl group" in a "substituted or unsubstituted alkenyl group." A simply termed "alkenyl group" herein includes both of an "unsubstituted alkenyl group" and a "substituted alkenyl group."

The "substituted alkenyl group" refers to a group derived by substituting at least one hydrogen atom in an "unsubstituted alkenyl group" with a substituent. Specific examples of the "substituted alkenyl group" include an "unsubstituted alkenyl group" (specific example group G4A) substituted by a substituent, and examples of the substituted alkenyl group (specific example group G4B) below. It should be noted that the examples of the "unsubstituted alkenyl group" and the "substituted alkenyl group" mentioned herein are merely exemplary, and the "substituted alkenyl group" mentioned herein includes a group derived by further substituting a hydrogen atom of a skeleton of the "substituted alkenyl group" in the specific example group G4B with a substituent, and a group derived by further substituting a hydrogen atom of a substituent of the "substituted alkenyl group" in the specific example group G4B with a substituent.

Unsubstituted Alkenyl Group (Specific Example Group G4A):

vinyl group, allyl group, 1-butenyl group, 2-butenyl group, and 3-butenyl group.

Substituted Alkenyl Group (Specific Example Group G4B):

1,3-butanedienyl group, 1-methylvinyl group, 1-methylallyl group, 1,1-dimethylallyl group, 2-methylallyl group, and 1,2-dimethylallyl group.

Substituted or Unsubstituted Alkynyl Group

Specific examples (specific example group G5) of the "substituted or unsubstituted alkynyl group" mentioned herein include unsubstituted alkynyl groups (specific example group G5A) below. Herein, an unsubstituted alkynyl group refers to an "unsubstituted alkynyl group" in a "substituted or unsubstituted alkynyl group." A simply termed "alkynyl group" herein includes both of an "unsubstituted alkynyl group" and a "substituted alkynyl group."

The "substituted alkynyl group" refers to a group derived by substituting at least one hydrogen atom in an "unsubstituted alkynyl group" with a substituent. Specific examples of the "substituted alkynyl group" include a group derived by substituting at least one hydrogen atom of the "unsubstituted alkynyl group" (specific example group G5A) below with a substituent.

Unsubstituted Alkynyl Group (Specific Example Group G5A):

ethynyl group.

Substituted or Unsubstituted Cycloalkyl Group

Specific examples (specific example group G6) of the "substituted or unsubstituted cycloalkyl group" mentioned herein include unsubstituted cycloalkyl groups (specific example group G6A) and substituted cycloalkyl groups (specific example group G6B). Herein, an unsubstituted cycloalkyl group refers to an "unsubstituted cycloalkyl group" in a "substituted or unsubstituted cycloalkyl group," and a substituted cycloalkyl group refers to a "substituted cycloalkyl group" in a "substituted or unsubstituted cycloalkyl group." A simply termed "cycloalkyl group" herein includes both of an "unsubstituted cycloalkyl group" and a "substituted cycloalkyl group."

The "substituted cycloalkyl group" refers to a group derived by substituting at least one hydrogen atom of an "unsubstituted cycloalkyl group" with a substituent. Specific examples of the "substituted cycloalkyl group" include a group derived by substituting at least one hydrogen atom of the "unsubstituted cycloalkyl group" (specific example group G6A) below with a substituent, and examples of the substituted cycloalkyl group (specific example group G6B) below. It should be noted that the examples of the "unsubstituted cycloalkyl group" and the "substituted cycloalkyl group" mentioned herein are merely exemplary, and the "substituted cycloalkyl group" mentioned herein includes a group derived by substituting at least one hydrogen atom bonded to a carbon atom of a skeleton of the "substituted cycloalkyl group" in the specific example group G6B with a substituent, and a group derived by further substituting a hydrogen atom of a substituent of the "substituted cycloalkyl group" in the specific example group G6B with a substituent.

Unsubstituted Cycloalkyl Group (Specific Example Group G6A):

cyclopropyl group, cyclobutyl group, cyclopentyl group, cyclohexyl group, 1-adamantyl group, 2-adamantyl group, 1-norbornyl group, and 2-norbornyl group.

Substituted Cycloalkyl Group (Specific Example Group G6B):

4-methylcyclohexyl group

Group represented by $-Si(R_{901})(R_{902})(R_{903})$

Specific examples (specific example group G7) of the group represented herein by $-Si(R_{901})(R_{902})(R_{903})$ include: -Si(G1)(G1)(G1); -Si(G1)(G2)(G2); - Si(G1)(G1)(G2); -Si(G2)(G2)(G2); -Si(G3)(G3)(G3); and -Si(G6)(G6)(G6).

Herein: G1 represents a "substituted or unsubstituted aryl group" in the specific example group G1;
G2 represents a "substituted or unsubstituted heterocyclic group" in the specific example group G2;
G3 represents a "substituted or unsubstituted alkyl group" in the specific example group G3; and
G6 represents a "substituted or unsubstituted cycloalkyl group" in the specific example group G6.

A plurality of G1 in -Si(G1)(G1)(G1) are mutually the same or different.

A plurality of G2 in -Si(G1)(G2)(G2) are mutually the same or different.
A plurality of G1 in -Si(G1)(G1)(G2) are mutually the same or different.
A plurality of G2 in -Si(G2)(G2)(G2) are mutually the same or different.
The plurality of G3 in -Si(G3)(G3)(G3) are mutually the same or different.
A plurality of G6 in -Si(G6)(G6)(G6) are mutually the same or different.

Group Represented by -O-($R_{904}$)

Specific examples (specific example group G8) of a group represented by - O-($R_{904}$) herein include: -O(G1); -O(G2); -O(G3); and -O(G6).

Herein: G1 represents a "substituted or unsubstituted aryl group" in the specific example group G1;
G2 represents a "substituted or unsubstituted heterocyclic group" in the specific example group G2;
G3 represents a "substituted or unsubstituted alkyl group" in the specific example group G3; and
G6 represents a "substituted or unsubstituted cycloalkyl group" in the specific example group G6.

Group Represented by -S-($R_{905}$)

Specific examples (specific example group G9) of a group represented herein by -S-($R_{905}$) include: -S(G1); -S(G2); -S(G3); and -S(G6).

Herein: G1 represents a "substituted or unsubstituted aryl group" in the specific example group G1;
G2 represents a "substituted or unsubstituted heterocyclic group" in the specific example group G2;
G3 represents a "substituted or unsubstituted alkyl group" in the specific example group G3; and
G6 represents a "substituted or unsubstituted cycloalkyl group" in the specific example group G6.

Group Represented by -N($R_{906}$)($R_{907}$)

Specific examples (specific example group G10) of a group represented herein by -N($R_{906}$)($R_{907}$) include: -N(G1)(G1); -N(G2)(G2); -N(G1)(G2); -N(G3)(G3); and -N(G6)(G6).

Herein: G1 represents a "substituted or unsubstituted aryl group" in the specific example group G1;
G2 represents a "substituted or unsubstituted heterocyclic group" in the specific example group G2;
G3 represents a "substituted or unsubstituted alkyl group" in the specific example group G3; and
G6 represents a "substituted or unsubstituted cycloalkyl group" in the specific example group G6.

A plurality of G1 in -N(G1)(G1) are mutually the same or different.
A plurality of G2 in -N(G2)(G2) are mutually the same or different.
A plurality of G3 in -N(G3)(G3) are mutually the same or different.
A plurality of G6 in -N(G6)(G6)) are mutually the same or different.

Halogen Atom

Specific examples (specific example group G11) of "halogen atom" mentioned herein include a fluorine atom, chlorine atom, bromine atom, and iodine atom.

Substituted or Unsubstituted Fluoroalkyl Group

The "substituted or unsubstituted fluoroalkyl group" mentioned herein refers to a group derived by substituting at least one hydrogen atom bonded to at least one of carbon atoms forming an alkyl group in the "substituted or unsubstituted alkyl group" with a fluorine atom, and also includes a group (perfluoro group) derived by substituting all of hydrogen atoms bonded to carbon atoms forming the alkyl group in the "substituted or unsubstituted alkyl group" with fluorine atoms. An "unsubstituted fluoroalkyl group" has, unless otherwise specified herein, 1 to 50 carbon atoms, preferably 1 to 30 carbon atoms, more preferably 1 to 18 carbon atoms. The "substituted fluoroalkyl group" refers to a group derived by substituting at least one hydrogen atom in a "fluoroalkyl group" with a substituent. It should be noted that the examples of the "substituted fluoroalkyl group" mentioned herein include a group derived by further substituting at least one hydrogen atom bonded to a carbon atom of an alkyl chain of a "substituted fluoroalkyl group" with a substituent, and a group derived by further substituting at least one hydrogen atom of a substituent of the "substituted fluoroalkyl

group" with a substituent. Specific examples of the "substituted fluoroalkyl group" include a group derived by substituting at least one hydrogen atom of the "alkyl group" (specific example group G3) with a fluorine atom.

Substituted or Unsubstituted Haloalkyl Group

The "substituted or unsubstituted haloalkyl group" mentioned herein refers to a group derived by substituting at least one hydrogen atom bonded to carbon atoms forming the alkyl group in the "substituted or unsubstituted alkyl group" with a halogen atom, and also includes a group derived by substituting all hydrogen atoms bonded to carbon atoms forming the alkyl group in the "substituted or unsubstituted alkyl group" with halogen atoms. An "unsubstituted haloalkyl group" has, unless otherwise specified herein, 1 to 50 carbon atoms, preferably 1 to 30 carbon atoms, more preferably 1 to 18 carbon atoms. The "substituted haloalkyl group" refers to a group derived by substituting at least one hydrogen atom in a "haloalkyl group" with a substituent. It should be noted that the examples of the "substituted haloalkyl group" mentioned herein include a group derived by further substituting at least one hydrogen atom bonded to a carbon atom of an alkyl chain of a "substituted haloalkyl group" with a substituent, and a group derived by further substituting at least one hydrogen atom of a substituent of the "substituted haloalkyl group" with a substituent. Specific examples of the "substituted haloalkyl group" include a group derived by substituting at least one hydrogen atom of the "alkyl group" (specific example group G3) with a halogen atom. The haloalkyl group is sometimes referred to as a halogenated alkyl group.

Substituted or Unsubstituted Alkoxy Group

Specific examples of a "substituted or unsubstituted alkoxy group" mentioned herein include a group represented by -O(G3), G3 being the "substituted or unsubstituted alkyl group" in the specific example group G3. An "unsubstituted alkoxy group" has, unless otherwise specified herein, 1 to 50 carbon atoms, preferably 1 to 30 carbon atoms, more preferably 1 to 18 carbon atoms.

Substituted or Unsubstituted Alkylthio Group

Specific examples of a "substituted or unsubstituted alkylthio group" mentioned herein include a group represented by -S(G3), G3 being the "substituted or unsubstituted alkyl group" in the specific example group G3. An "unsubstituted alkylthio group" has, unless otherwise specified herein, 1 to 50 carbon atoms, preferably 1 to 30 carbon atoms, more preferably 1 to 18 carbon atoms.

Substituted or Unsubstituted Aryloxy Group

Specific examples of a "substituted or unsubstituted aryloxy group" mentioned herein include a group represented by -O(G1), G1 being the "substituted or unsubstituted aryl group" in the specific example group G1. An "unsubstituted aryloxy group" has, unless otherwise specified herein, 6 to 50 ring carbon atoms, preferably 6 to 30 ring carbon atoms, more preferably 6 to 18 ring carbon atoms.

Substituted or Unsubstituted Arylthio Group

Specific examples of a "substituted or unsubstituted arylthio group" mentioned herein include a group represented by -S(G1), G1 being the "substituted or unsubstituted aryl group" in the specific example group G1. An "unsubstituted arylthio group" has, unless otherwise specified herein, 6 to 50 ring carbon atoms, preferably 6 to 30 ring carbon atoms, more preferably 6 to 18 ring carbon atoms.

Substituted or Unsubstituted Trialkylsilyl Group

Specific examples of a "trialkylsilyl group" mentioned herein include a group represented by -Si(G3)(G3)(G3), G3 being the "substituted or unsubstituted alkyl group" in the specific example group G3. The plurality of G3 in -Si(G3)(G3)(G3) are mutually the same or different. Each of the alkyl groups in the "trialkylsilyl group" has, unless otherwise specified herein, 1 to 50 carbon atoms, preferably 1 to 20 carbon atoms, more preferably 1 to 6 carbon atoms.

Substituted or Unsubstituted Aralkyl Group

Specific examples of a "substituted or unsubstituted aralkyl group" mentioned herein include a group represented by (G3)-(G1), G3 being the "substituted or unsubstituted alkyl group" in the specific example group G3, G1 being the

"substituted or unsubstituted aryl group" in the specific example group G1. Accordingly, the "aralkyl group" is a group derived by substituting a hydrogen atom of the "alkyl group" with a substituent in a form of the "aryl group," which is an example of the "substituted alkyl group." An "unsubstituted aralkyl group," which is an "unsubstituted alkyl group" substituted by an "unsubstituted aryl group," has, unless otherwise specified herein, 7 to 50 carbon atoms, preferably 7 to 30 carbon atoms, more preferably 7 to 18 carbon atoms.

Specific examples of the "substituted or unsubstituted aralkyl group" include a benzyl group, 1-phenylethyl group, 2-phenylethyl group, 1-phenylisopropyl group, 2-phenylisopropyl group, phenyl-t-butyl group, α-naphthylmethyl group, 1-α-naphthylethyl group, 2-α-naphthylethyl group, 1-α-naphthylisopropyl group, 2-α-naphthylisopropyl group, β-naphthylmethyl group, 1-β-naphthylethyl group, 2-β-naphthylethyl group, 1-β-naphthylisopropyl group, and 2-β-naphthylisopropyl group.

Preferable examples of the substituted or unsubstituted aryl group mentioned herein include, unless otherwise specified herein, a phenyl group, p-biphenyl group, m-biphenyl group, o-biphenyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-terphenyl-4-yl group, o-terphenyl-3-yl group, o-terphenyl-2-yl group, 1-naphthyl group, 2-naphthyl group, anthryl group, phenanthryl group, pyrenyl group, chrysenyl group, triphenylenyl group, fluorenyl group, 9,9'-spirobifluorenyl group, 9,9-dimethylfluorenyl group, and 9,9-diphenylfluorenyl group.

Preferable examples of the substituted or unsubstituted heterocyclic group mentioned herein include, unless otherwise specified herein, a pyridyl group, pyrimidinyl group, triazinyl group, quinolyl group, isoquinolyl group, quinazolinyl group, benzimidazolyl group, phenanthrolinyl group, carbazolyl group (1-carbazolyl group, 2-carbazolyl group, 3-carbazolyl group, 4-carbazolyl group, or 9-carbazolyl group), benzocarbazolyl group, azacarbazolyl group, diazacarbazolyl group, dibenzofuranyl group, naphthobenzofuranyl group, azadibenzofuranyl group, diazadibenzofuranyl group, dibenzothiophenyl group, naphthobenzothiophenyl group, azadibenzothiophenyl group, diazadibenzothiophenyl group, (9-phenyl)carbazolyl group ((9-phenyl)carbazole-1-yl group, (9-phenyl)carbazole-2-yl group, (9-phenyl)carbazole-3-yl group, or (9-phenyl)carbazole-4-yl group), (9-biphenylyl)carbazolyl group, (9-phenyl)phenylcarbazolyl group, diphenylcarbazole-9-yl group, phenylcarbazole-9-yl group, phenyltriazinyl group, biphenylyltriazinyl group, diphenyltriazinyl group, phenyldibenzofuranyl group, and phenyldibenzothiophenyl group.

The carbazolyl group mentioned herein is, unless otherwise specified herein, specifically a group represented by one of formulae below.

[Formula 8]

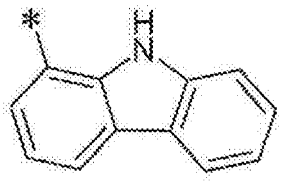

(TEMP-Cz1)

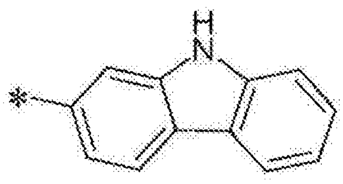

(TEMP-Cz2)

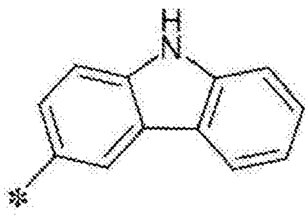

(TEMP-Cz3)

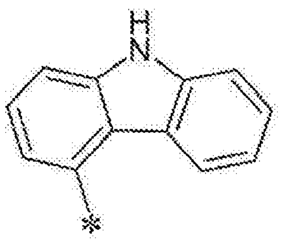

(TEMP-Cz4)

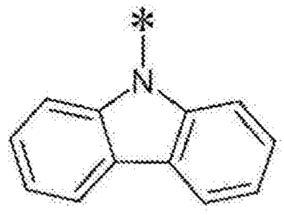

(TEMP-Cz5)

The (9-phenyl)carbazolyl group mentioned herein is, unless otherwise specified herein, specifically a group represented by one of formulae below.

[Formula 9]

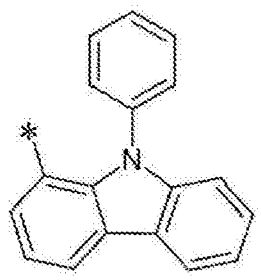

(TEMP-Cz6)

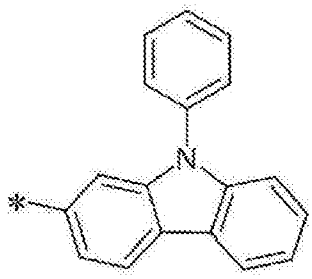

(TEMP-Cz7)

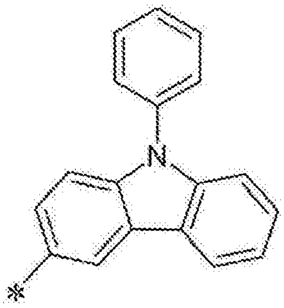

(TEMP-Cz8)

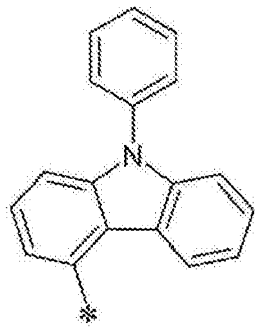

(TEMP-Cz9)

In the formulae (TEMP-Cz1) to (TEMP-Cz9), * represents a bonding position.

The dibenzofuranyl group and dibenzothiophenyl group mentioned herein are, unless otherwise specified herein, each specifically represented by one of formulae below.

[Formula 10]

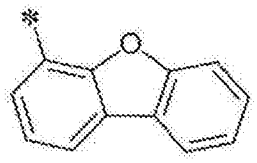

(TEMP-34)

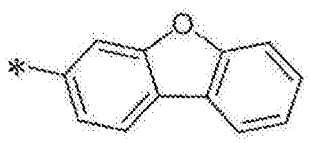

(TEMP-35)

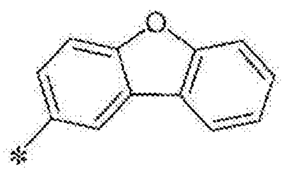

(TEMP-36)

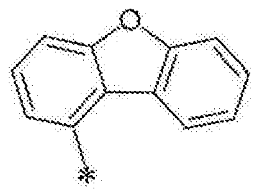

(TEMP-37)

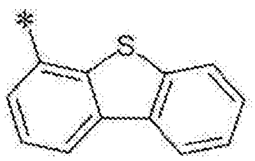

(TEMP-38)

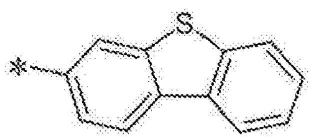

(TEMP-39)

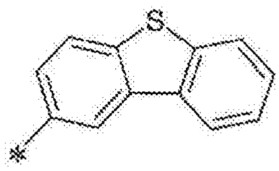

(TEMP-40)

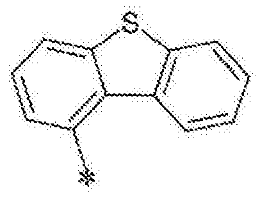

(TEMP-41)

In the formulae (TEMP-34) to (TEMP-41), * represents a bonding position.

Preferable examples of the substituted or unsubstituted alkyl group mentioned herein include, unless otherwise specified herein, a methyl group, ethyl group, propyl group, isopropyl group, n-butyl group, isobutyl group, and t-butyl group.

Substituted or Unsubstituted Arylene Group

The "substituted or unsubstituted arylene group" mentioned herein is, unless otherwise specified herein, a divalent group derived by removing one hydrogen atom on an aryl ring of the "substituted or unsubstituted aryl group." Specific examples of the "substituted or unsubstituted arylene group" (specific example group G12) include a divalent group derived by removing one hydrogen atom on an aryl ring of the "substituted or unsubstituted aryl group" in the specific example group G1.

Substituted or Unsubstituted Divalent Heterocyclic Group

The "substituted or unsubstituted divalent heterocyclic group" mentioned herein is, unless otherwise specified herein, a divalent group derived by removing one hydrogen atom on a heterocyclic ring of the "substituted or unsubstituted heterocyclic group." Specific examples of the "substituted or unsubstituted heterocyclic group" (specific example group

G13) include a divalent group derived by removing one hydrogen atom on a heterocyclic ring of the "substituted or unsubstituted heterocyclic group" in the specific example group G2.

Substituted or Unsubstituted Alkylene Group

The "substituted or unsubstituted alkylene group" mentioned herein is, unless otherwise specified herein, a divalent group derived by removing one hydrogen atom on an alkyl ring of the "substituted or unsubstituted alkyl group." Specific examples of the "substituted or unsubstituted alkylene group" (specific example group G14) include a divalent group derived by removing one hydrogen atom on an alkyl ring of the "substituted or unsubstituted alkyl group" in the specific example group G3.

The substituted or unsubstituted arylene group mentioned herein is, unless otherwise specified herein, preferably any one of groups represented by formulae (TEMP-42) to (TEMP-68) below.

[Formula 11]

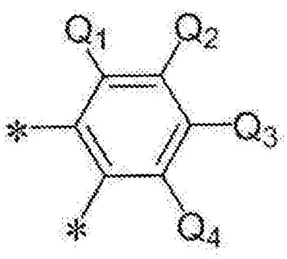

(TEMP-42)

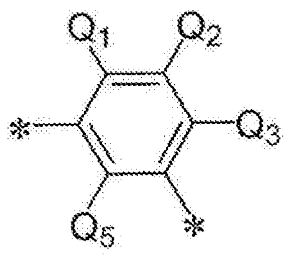

(TEMP-43)

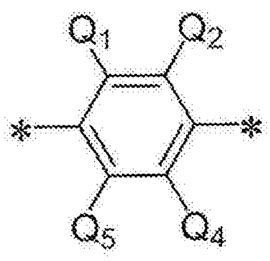

(TEMP-44)

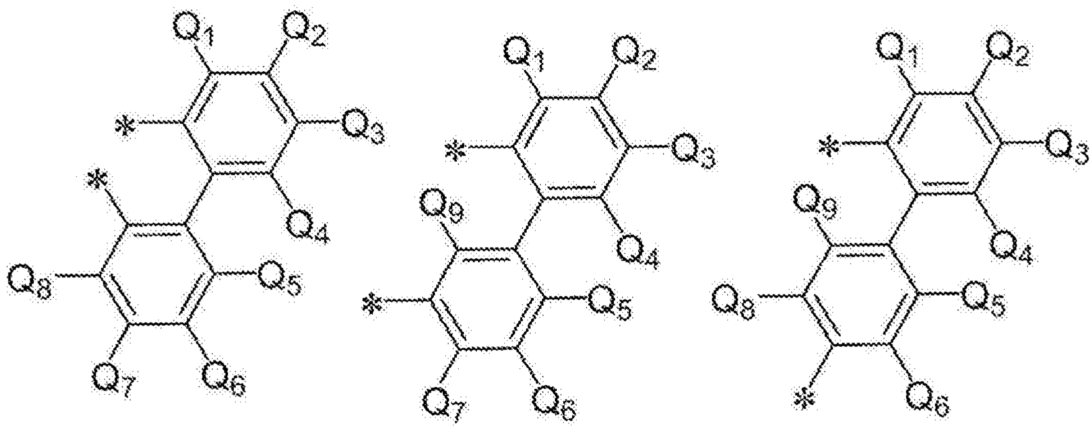

(TEMP-45) (TEMP-46) (TEMP-47)

[Formula 12]

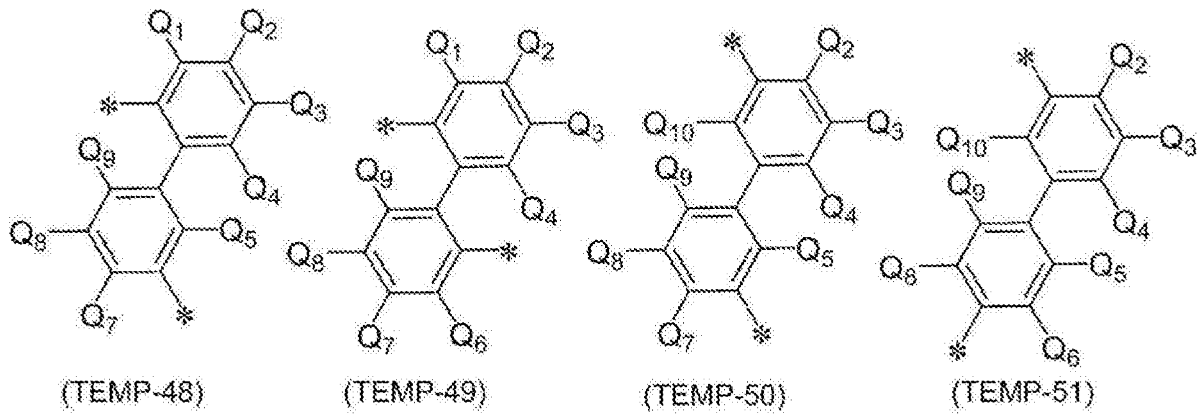

(TEMP-48) (TEMP-49) (TEMP-50) (TEMP-51)

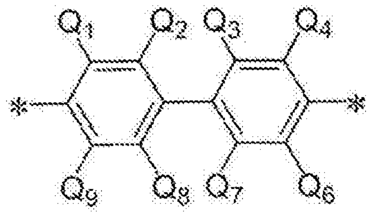

(TEMP-52)

In the formulae (TEMP-42) to (TEMP-52), $Q_1$ to $Q_{10}$ each independently are a hydrogen atom or a substituent.

In the formulae (TEMP-42) to (TEMP-52), * represents a bonding position.

[Formula 13]

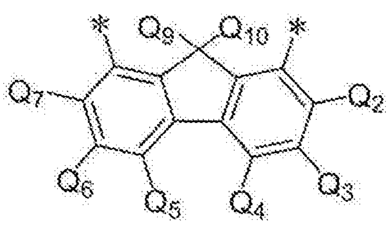

(TEMP-53)

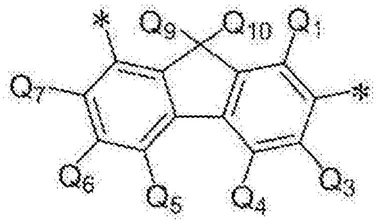

(TEMP-54)

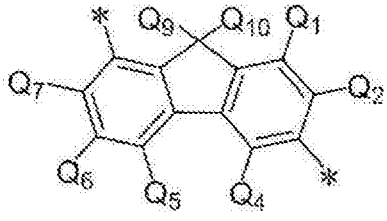

(TEMP-55)

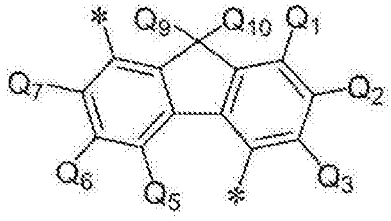

(TEMP-56)

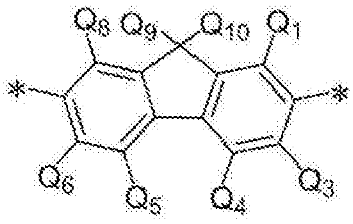

(TEMP-57)

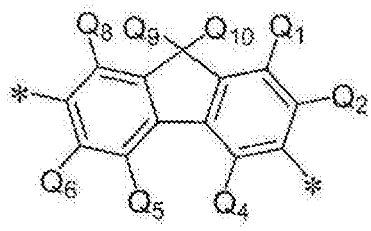

(TEMP-58)

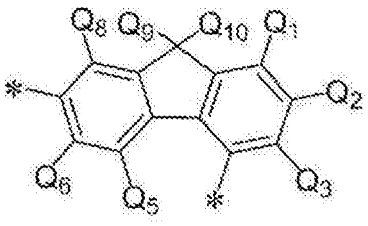

(TEMP-59)

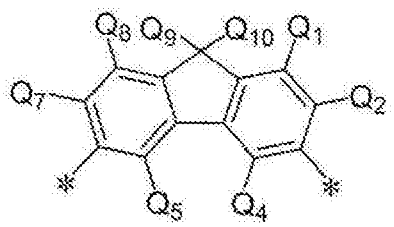

(TEMP-60)

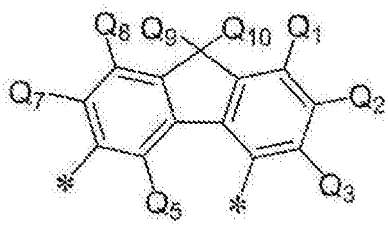

(TEMP-61)

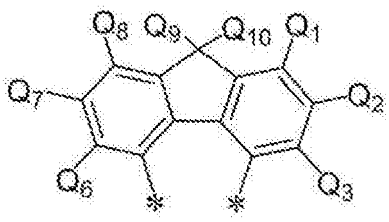

(TEMP-62)

In the formulae (TEMP-53) to (TEMP-62), $Q_1$ to $Q_{10}$ each independently are a hydrogen atom or a substituent.

In the formulae, $Q_9$ and $Q_{10}$ may be mutually bonded through a single bond to form a ring.

In the formulae (TEMP-53) to (TEMP-62), * represents a bonding position.

[Formula 14]

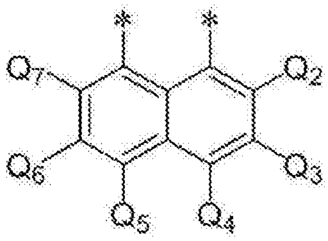
(TEMP-63)

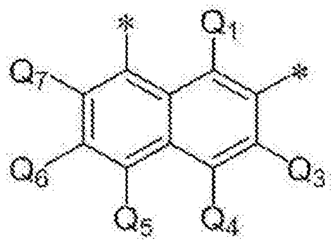
(TEMP-64)

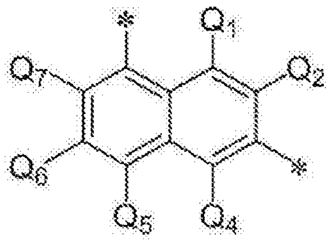
(TEMP-65)

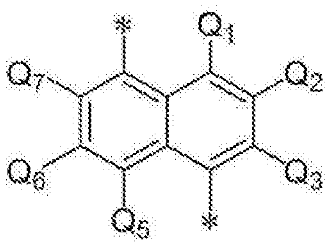
(TEMP-66)

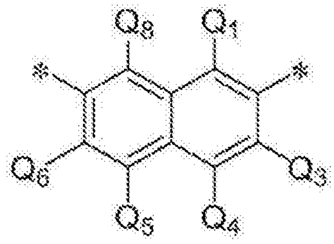
(TEMP-67)

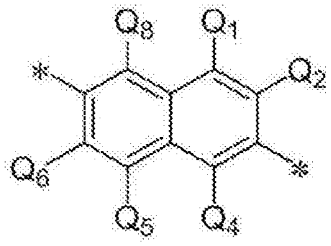
(TEMP-68)

In the formulae (TEMP-63) to (TEMP-68), $Q_1$ to $Q_8$ each independently are a hydrogen atom or a substituent.

In the formulae (TEMP-63) to (TEMP-68), * represents a bonding position.

The substituted or unsubstituted divalent heterocyclic group mentioned herein is, unless otherwise specified herein, preferably a group represented by any one of formulae (TEMP-69) to (TEMP-102) below.

[Formula 15]

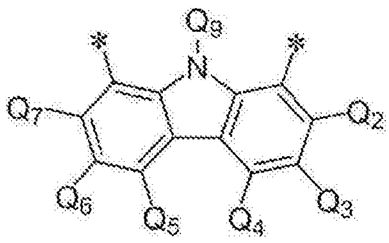
(TEMP-69)

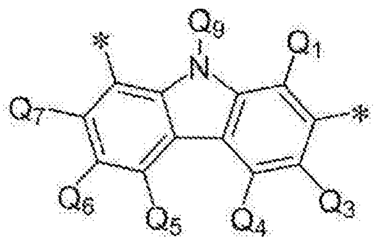
(TEMP-70)

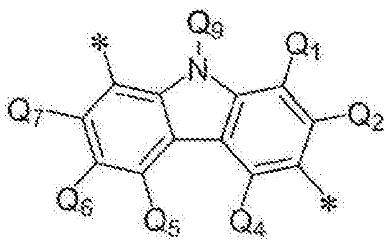
(TEMP-71)

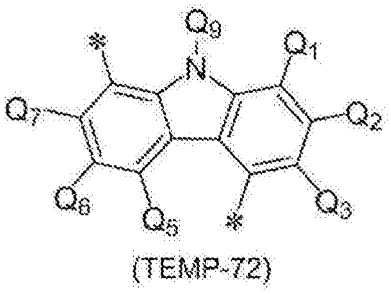
(TEMP-72)

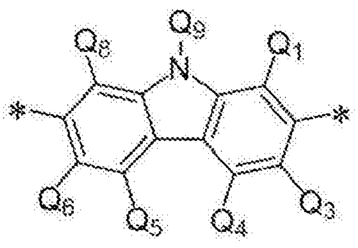
(TEMP-73)

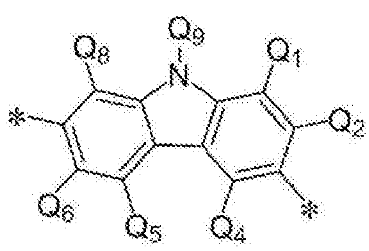
(TEMP-74)

[Formula 16]

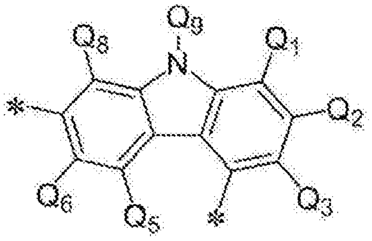

(TEMP-75)

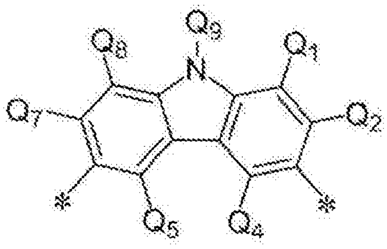

(TEMP-76)

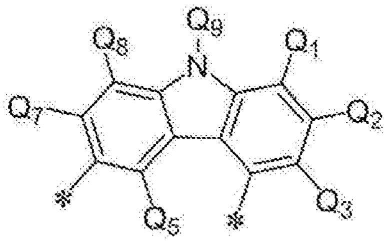

(TEMP-77)

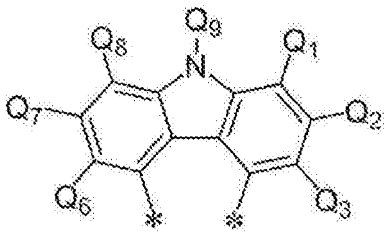

(TEMP-78)

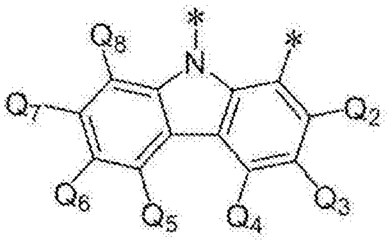

(TEMP-79)

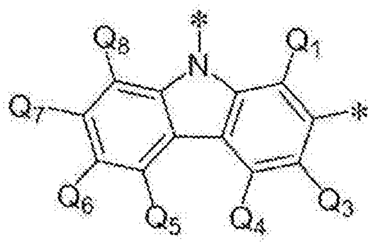

(TEMP-80)

[Formula 17]

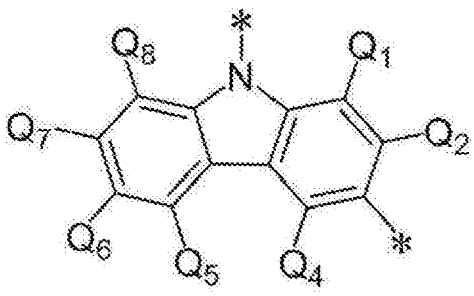

(TEMP-81)

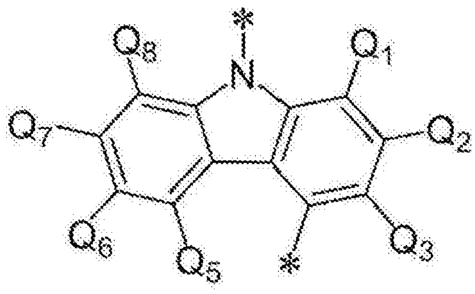

(TEMP-82)

In the formulae (TEMP-69) to (TEMP-82), $Q_1$ to $Q_9$ each independently are a hydrogen atom or a substituent.

[Formula 18]

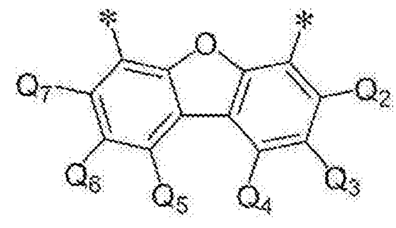

(TEMP-83)

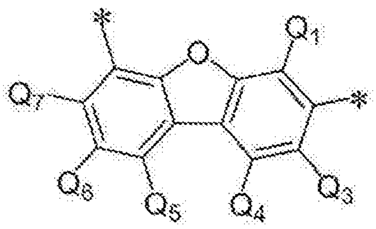

(TEMP-84)

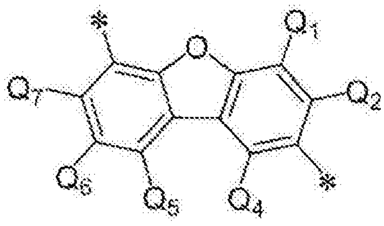

(TEMP-85)

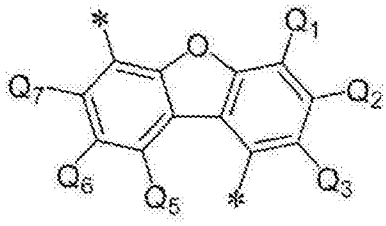

(TEMP-86)

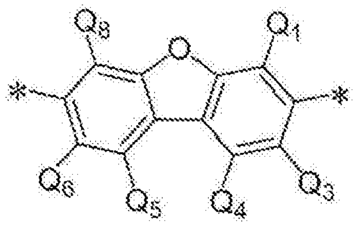

(TEMP-87)

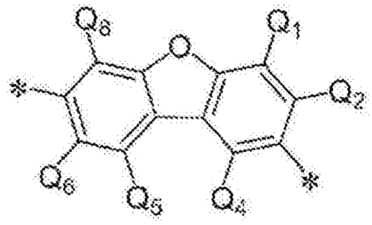

(TEMP-88)

[Formula 19]

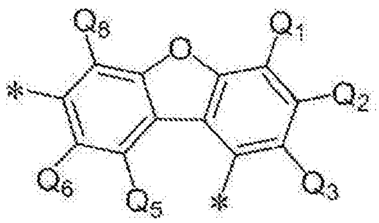

(TEMP-89)

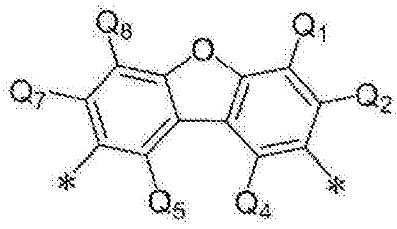

(TEMP-90)

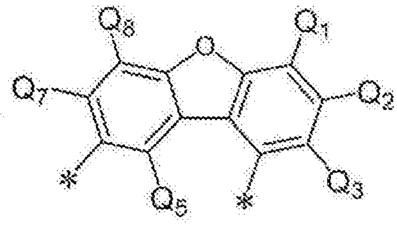

(TEMP-91)

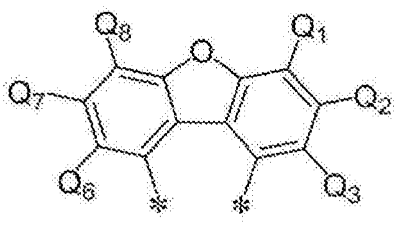

(TEMP-92)

[Formula 20]

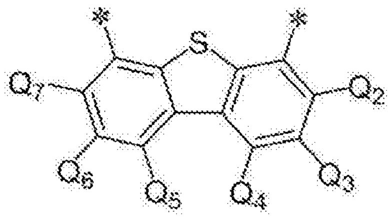
(TEMP-93)

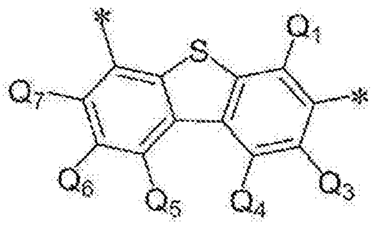
(TEMP-94)

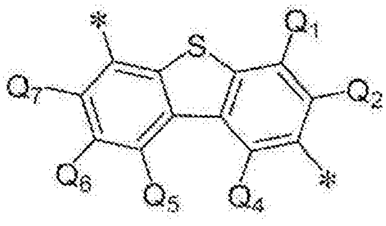
(TEMP-95)

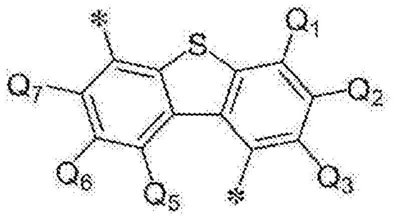
(TEMP-96)

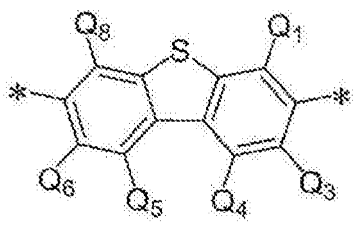
(TEMP-97)

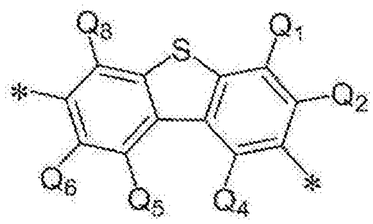
(TEMP-98)

[Formula 21]

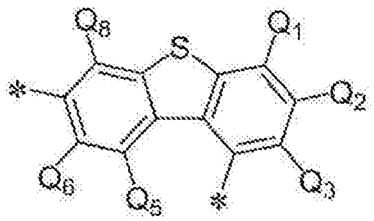
(TEMP-99)

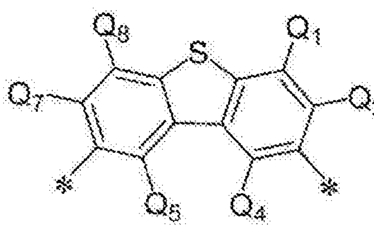
(TEMP-100)

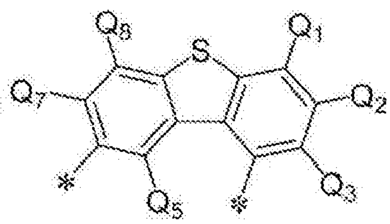
(TEMP-101)

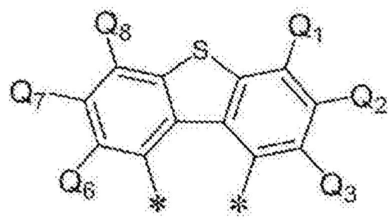
(TEMP-102)

In the formulae (TEMP-83) to (TEMP-102), $Q_1$ to $Q_8$ each independently are a hydrogen atom or a substituent.
The substituent mentioned herein has been described above.

Instance of "Bonded to Form Ring"

Instances where "at least one combination of adjacent two or more (of... ) are mutually bonded to form a substituted or unsubstituted monocyclic ring, mutually bonded to form a substituted or unsubstituted fused ring, or not mutually bonded" mentioned herein refer to instances where "at least one combination of adjacent two or more (of...) are mutually bonded to form a substituted or unsubstituted monocyclic ring," at least one combination of adjacent two or more (of... ) are mutually bonded to form a substituted or unsubstituted fused ring," and "at least one combination of adjacent two or more (of...) are not mutually bonded."
Instances where "at least one combination of adjacent two or more (of... ) are mutually bonded to form a substituted or unsubstituted monocyclic ring" and "at least one combination of adjacent two or more (of...) are mutually bonded to form a substituted or unsubstituted fused ring" mentioned herein (these instances will be sometimes collectively referred to as an instance of "bonded to form a ring" hereinafter) will be described below. An anthracene compound having a basic skeleton in a form of an anthracene ring and represented by a formula (TEMP-103) below will be used as an example for the description.

[Formula 22]

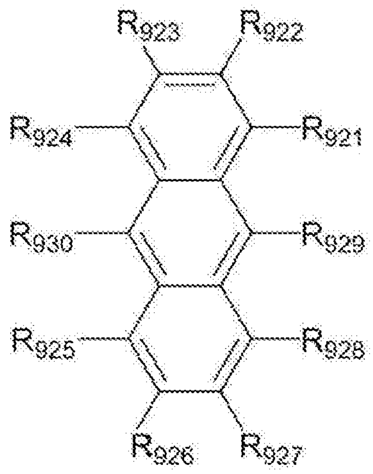

(TEMP-103)

For instance, when "at least one combination of adjacent two or more of" $R_{921}$ to $R_{930}$ "are mutually bonded to form a ring," the combination of adjacent ones of $R_{921}$ to $R_{930}$ (i.e. the combination at issue) is a combination of $R_{921}$ and a combination of $R_{922}$, $R_{922}$ and $R_{923}$, a combination of $R_{923}$ and $R_{924}$, a combination of $R_{924}$ and $R_{930}$, a combination of $R_{930}$ and $R_{925}$, a combination of $R_{925}$ and $R_{926}$, a combination of $R_{926}$ and $R_{927}$, a combination of $R_{927}$ and $R_{928}$, a combination of $R_{928}$ and $R_{929}$, or a combination of $R_{929}$ and $R_{921}$.

The term "at least one combination" means that two or more of the above combinations of adjacent two or more of $R_{921}$ to $R_{930}$ may simultaneously form rings. For instance, when $R_{921}$ and $R_{922}$ are mutually bonded to form a ring $Q_A$ and $R_{925}$ and $R_{926}$ are simultaneously mutually bonded to form a ring $Q_B$, the anthracene compound represented by the formula (TEMP-103) is represented by a formula (TEMP-104) below.

[Formula 23]

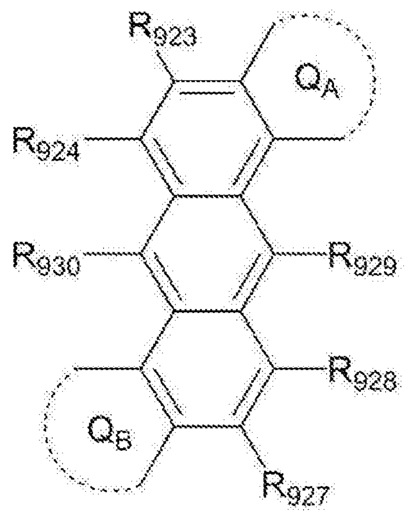

(TEMP-104)

The instance where the "combination of adjacent two or more" form a ring means not only an instance where the "two" adjacent components are bonded but also an instance where adjacent "three or more" are bonded. For instance, $R_{921}$ and $R_{922}$ are mutually bonded to form a ring $Q_A$ and $R_{922}$, $R_{923}$ are mutually bonded to form a ring Qc, and mutually adjacent three components ($R_{921}$, $R_{922}$ and $R_{923}$) are mutually bonded to form a ring fused to the anthracene basic skeleton. In this case, the anthracene compound represented by the formula (TEMP-103) is represented by a formula (TEMP-105) below. In the formula (TEMP-105) below, the ring $Q_A$ and the ring Qc share $R_{922}$.

[Formula 24]

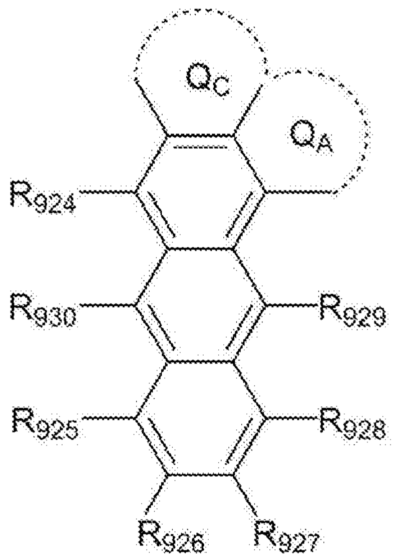

(TEMP-105)

The formed "monocyclic ring" or "fused ring" may be, in terms of the formed ring in itself, a saturated ring or an unsaturated ring. When the "combination of adjacent two" form a "monocyclic ring" or a "fused ring," the "monocyclic ring" or "fused ring" may be a saturated ring or an unsaturated ring. For instance, the ring $Q_A$ and the ring $Q_B$ formed in the formula (TEMP-104) are each independently a "monocyclic ring" or a "fused ring." Further, the ring $Q_A$ and the ring Qc formed in the formula (TEMP-105) are each a "fused ring." The ring $Q_A$ and the ring Qc in the formula (TEMP-105) are fused to form a fused ring. When the ring $Q_A$ in the formula (TMEP-104) is a benzene ring, the ring $Q_A$ is a monocyclic ring. When the ring $Q_A$ in the formula (TMEP-104) is a naphthalene ring, the ring $Q_A$ is a fused ring.

The "unsaturated ring" represents an aromatic hydrocarbon ring or an aromatic heterocycle. The "saturated ring" represents an aliphatic hydrocarbon ring or a non-aromatic heterocycle.

Specific examples of the aromatic hydrocarbon ring include a ring formed by terminating a bond of a group in the specific example of the specific example group G1 with a hydrogen atom.

Specific examples of the aromatic heterocyclic ring include a ring formed by terminating a bond of an aromatic heterocyclic group in the specific example of the specific example group G2 with a hydrogen atom.

Specific examples of the aliphatic hydrocarbon ring include a ring formed by terminating a bond of a group in the specific example of the specific example group G6 with a hydrogen atom.

The phrase "to form a ring" herein means that a ring is formed only by a plurality of atoms of a basic skeleton, or by a combination of a plurality of atoms of the basic skeleton and one or more optional atoms. For instance, the ring $Q_A$ formed by mutually bonding $R_{921}$ and $R_{922}$ shown in the formula (TEMP-104) is a ring formed by a carbon atom of the anthracene skeleton bonded with $R_{921}$, a carbon atom of the anthracene skeleton bonded with $R_{922}$, and one or more optional atoms. Specifically, when the ring $Q_A$ is a monocyclic unsaturated ring formed by $R_{921}$ and $R_{922}$, the ring formed by a carbon atom of the anthracene skeleton bonded with $R_{921}$, a carbon atom of the anthracene skeleton bonded with $R_{922}$, and four carbon atoms is a benzene ring.

The "optional atom" is, unless otherwise specified herein, preferably at least one atom selected from the group consisting of a carbon atom, nitrogen atom, oxygen atom, and sulfur atom. A bond of the optional atom (e.g. a carbon atom and a nitrogen atom) not forming a ring may be terminated by a hydrogen atom or the like or may be substituted by an "optional substituent" described later. When the ring includes an optional element other than carbon atom, the resultant ring is a heterocycle.

The number of "one or more optional atoms" forming the monocyclic ring or fused ring is, unless otherwise specified herein, preferably in a range from 2 to 15, more preferably in a range from 3 to 12, further preferably in a range from 3 to 5.

Unless otherwise specified herein, the ring, which may be a "monocyclic ring" or "fused ring," is preferably a "monocyclic ring."

Unless otherwise specified herein, the ring, which may be a "saturated ring" or "unsaturated ring," is preferably an "unsaturated ring."

Unless otherwise specified herein, the "monocyclic ring" is preferably a benzene ring.

Unless otherwise specified herein, the "unsaturated ring" is preferably a benzene ring.

When "at least one combination of adjacent two or more" (of...) are "mutually bonded to form a substituted or unsubstituted monocyclic ring" or "mutually bonded to form a substituted or unsubstituted fused ring," unless otherwise specified herein, at least one combination of adjacent two or more of components are preferably mutually bonded to

form a substituted or unsubstituted "unsaturated ring" formed of a plurality of atoms of the basic skeleton, and 1 to 15 atoms of at least one element selected from the group consisting of carbon, nitrogen, oxygen and sulfur.

When the "monocyclic ring" or the "fused ring" has a substituent, the substituent is the substituent described in later-described "optional substituent." When the "monocyclic ring" or the "fused ring" has a substituent, specific examples of the substituent are the substituents described in the above under the subtitle "Substituents Mentioned Herein."

When the "saturated ring" or the "unsaturated ring" has a substituent, the substituent is, for instance, the substituent described in later-described "optional substituent." When the "monocyclic ring" or the "fused ring" has a substituent, specific examples of the substituent are the substituents described in the above under the subtitle "Substituents Mentioned Herein."

The above is the description for the instances where "at least one combination of adjacent two or more (of...) are mutually bonded to form a substituted or unsubstituted monocyclic ring" and "at least one combination of adjacent two or more (of...) are mutually bonded to form a substituted or unsubstituted fused ring" mentioned herein (sometimes referred to as an instance "bonded to form a ring".

Substituent for Substituted or Unsubstituted Group

In an exemplary embodiment herein, the substituent for the substituted or unsubstituted group (sometimes referred to as an "optional substituent") is selected from the group consisting of, for instance, an unsubstituted alkyl group having 1 to 50 carbon atoms, an unsubstituted alkenyl group having 2 to 50 carbon atoms, an unsubstituted alkynyl group having 2 to 50 carbon atoms, an unsubstituted cycloalkyl group having 3 to 50 ring carbon atoms, $-Si(R_{901})(R_{902})(R_{903})$, $-O-(R_{904})$, $-S-(R_{905})$, $-N(R_{906})(R_{907})$, a halogen atom, a cyano group, a nitro group, an unsubstituted aryl group having 6 to 50 ring carbon atoms, and an unsubstituted heterocyclic group having 5 to 50 ring atoms.

Herein, $R_{901}$ to $R_{907}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms.

When two or more $R_{901}$ are present, the two or more $R_{901}$ are mutually the same or different.

When two or more $R_{902}$ are present, the two or more $R_{902}$ are mutually the same or different.

When two or more $R_{903}$ are present, the two or more $R_{903}$ are mutually the same or different.

When two or more $R_{904}$ are present, the two or more $R_{904}$ are mutually the same or different.

When two or more $R_{905}$ are present, the two or more $R_{905}$ are mutually the same or different.

When two or more $R_{906}$ are present, the two or more $R_{906}$ are mutually the same or different.

When two or more $R_{907}$ are present, the two or more $R_{907}$ are mutually the same or different.

In an exemplary embodiment, the substituent for "substituted or unsubstituted" group is selected from the group consisting of an alkyl group having 1 to 50 carbon atoms, an aryl group having 6 to 50 ring carbon atoms, and a heterocyclic group having 5 to 50 ring atoms.

In an exemplary embodiment, the substituent for "substituted or unsubstituted" group is selected from the group consisting of an alkyl group having 1 to 18 carbon atoms, an aryl group having 6 to 18 ring carbon atoms, and a heterocyclic group having 5 to 18 ring atoms.

Specific examples of the above optional substituent are the same as the specific examples of the substituent described in the above under the subtitle "Substituent Mentioned Herein."

Unless otherwise specified herein, adjacent ones of the optional substituents may form a "saturated ring" or an "unsaturated ring," preferably a substituted or unsubstituted saturated five-membered ring, a substituted or unsubstituted saturated six-membered ring, a substituted or unsubstituted saturated five-membered ring, or a substituted or unsubstituted unsaturated six-membered ring, more preferably a benzene ring.

Unless otherwise specified herein, the optional substituent may further include a substituent. Examples of the substituent for the optional substituent are the same as the examples of the optional substituent.

Herein, numerical ranges represented by "AA to BB" represents a range whose lower limit is the value (AA) recited before "to" and whose upper limit is the value (BB) recited after "to."

First Exemplary Embodiment

Compound

A compound according to a first exemplary embodiment is a compound represented by a formula (1000B) below and having at least one group represented by a formula (110) below.

[Formula 25]

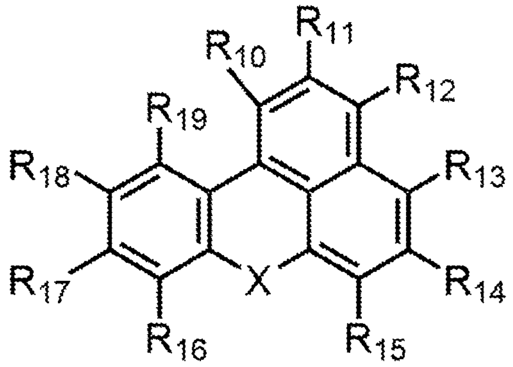

(1000B)

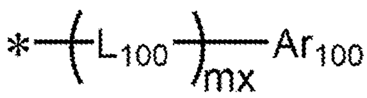

(110)

In the formula (1000B):

X is an oxygen atom, a sulfur atom, $C(R_{2001})(R_{2002})$, or $Si(R_{2003})(R_{2004})$;
at least one combination of adjacent two or more of $R_{10}$ to $R_{19}$ are mutually bonded to form a substituted or unsubstituted monocyclic ring, mutually bonded to form a substituted or unsubstituted fused ring, or not mutually bonded;
$R_{10}$ to $R_{19}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by - $S-(R_{905})$, a group represented by $-N(R_{906})(R_{907})$, a substituted or unsubstituted aralkyl group having 7 to 50 carbon atoms, a group represented by $-C(=O)R_{801}$, a group represented by $-COOR_{802}$, a halogen atom, a nitro group, a substituted or unsubstituted aryl group having 6 to 50 ring carbon atoms, a substituted or unsubstituted heterocyclic group having 5 to 50 ring atoms, or a group represented by the formula (110);
at least one of $R_{10}$ to $R_{19}$ is a group represented by the formula (110);
when a plurality of groups represented by the formula (110) are present, the plurality of groups represented by the formula (110) are mutually the same or different;
$L_{100}$ is a single bond, a substituted or unsubstituted arylene group having 6 to 50 ring carbon atoms, or a substituted or unsubstituted divalent heterocyclic group having 5 to 50 ring atoms;
mx is 1, 2, or 3;
when two or more $L_{100}$ are present, the two or more $L_{100}$ are mutually the same or different;
$Ar_{100}$ is a substituted or unsubstituted aryl group including three or more rings or a substituted or unsubstituted heterocyclic group including two or more aromatic rings and one or more heterocyclic rings;
$Ar_{100}$ does not include an anthracene ring;
when two or more $Ar_{100}$ are present, the two or more $Ar_{100}$ are mutually the same or different; and
* in the formula (110) represents a bonding position.

A compound represented by the formula (1000B) is preferably a compound represented by a formula (100) below and having at least one group represented by the formula (110).

[Formula 26]

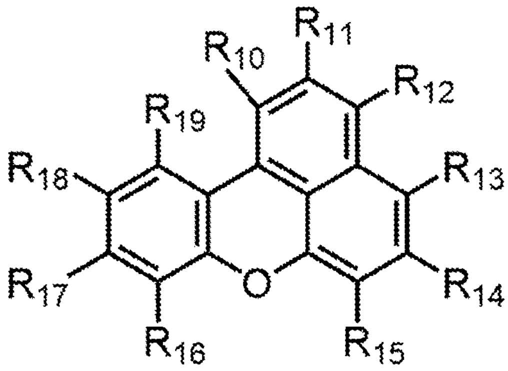

(100)

In the formula (100): $R_{10}$ to $R_{19}$ each independently represent the same as $R_{10}$ to $R_{19}$ in the formula (1000B); none of combinations of adjacent two or more of $R_{10}$ to $R_{19}$ are mutually bonded; and $Ar_{100}$, $L_{100}$, and mx respectively represent the same as $Ar_{100}$, $L_{100}$, and mx in the formula (110).

LUMO (Lowest Unoccupied Molecular Orbital) of a compound in which a cyano group is directly bonded to a benzoxanthene ring (hereinafter, referred to as a cyano-substituted benzoxanthene compound) is lower than LUMO of a compound in which a cyano group is not bonded to a benzoxanthene ring. In a case where a cyano-substituted benzoxanthene compound is used as a host material in the emitting layer, the host material may suffer a large damage at an interface of the emitting layer close to the hole transporting layer to lower a lifetime of an organic EL device. It is inappropriate to use a cyano-substituted benzoxanthene compound particularly in the emitting layer close to the hole transporting layer in an organic EL device in which a plurality of emitting layers are layered.

The compound according to the exemplary embodiment is also preferably represented by a formula (101) or (102) below.

[Formula 27]

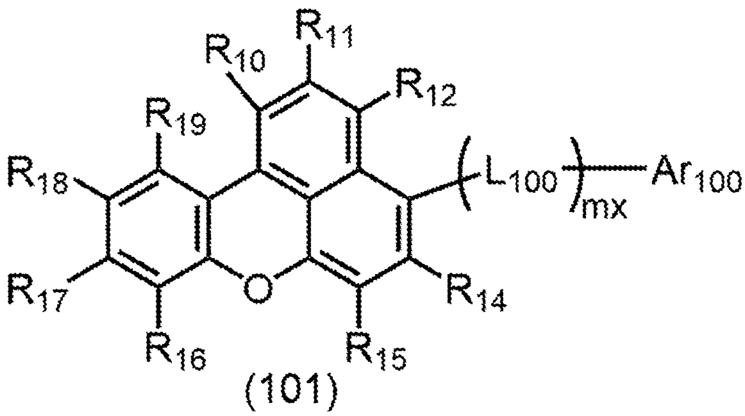

(101)

[Formula 28]

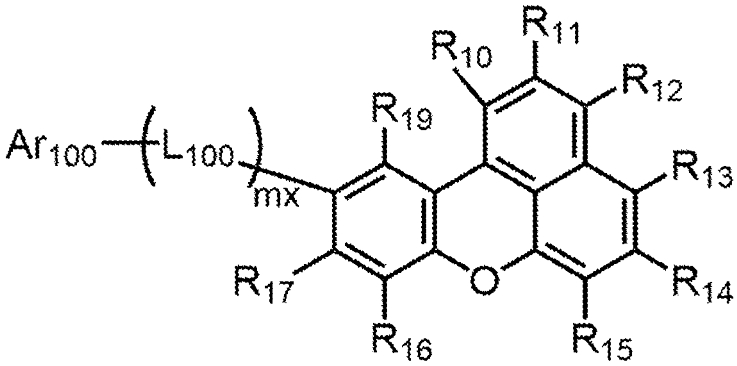

(102)

In the formulae (101) and (102): $R_{10}$ to $R_{19}$ each independently represent the same as $R_{10}$ to $R_{19}$ in the formula (1000B); none of combinations of adjacent two or more of $R_{10}$ to $R_{19}$ are mutually bonded; and $Ar_{100}$, $L_{100}$, and mx respectively represent the same as $Ar_{100}$, $L_{100}$, and mx in the formula (110).

In the compound according to the exemplary embodiment, it is preferable that $R_{10}$ to $R_{19}$ not being a group represented by the formula (110) are each independently a hydrogen atom, a substituted or unsubstituted alkyl group having 1 to 50 carbon atoms, a substituted or unsubstituted cycloalkyl group having 3 to 50 ring carbon atoms, or a substituted or unsubstituted aryl group having 6 to 50 ring carbon atoms.

In the compound according to the exemplary embodiment, $R_{10}$ to $R_{19}$ not being a group represented by the formula (110) are each preferably a hydrogen atom.

In the compound according to the exemplary embodiment, $L_{100}$ is preferably a single bond or an arylene group including at most three substituted or unsubstituted benzene rings.

In the compound according to the exemplary embodiment, $L_{100}$ is preferably not a substituted or unsubstituted anthrylene group.

In the compound according to the exemplary embodiment, $L_{100}$ is also preferably not a single bond.

In the compound according to the exemplary embodiment, a group represented by $-(L_{100})_{mx}-$ in the formula (110) is also preferably a group represented by one of formulae (111) to (120) below.

[Formula 29]

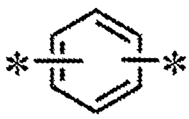

(111)

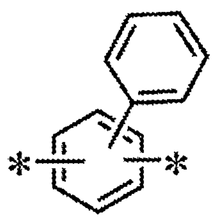

(112)

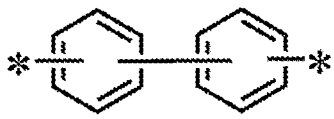

(113)

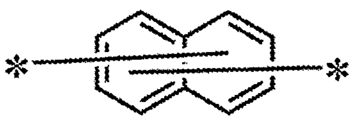

(114)

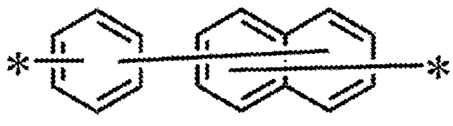

(115)

[Formula 30]

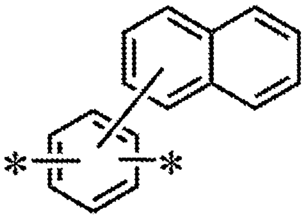

(116)

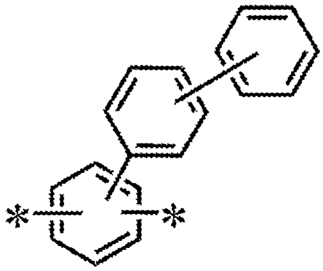

(117)

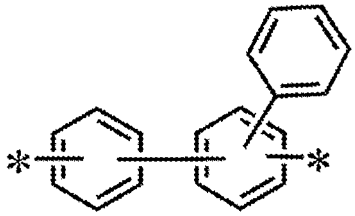

(118)

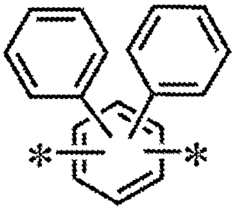

(119)

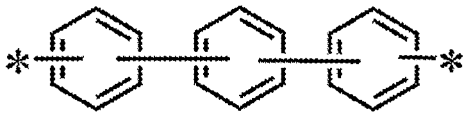

(120)

**[0171]** * in the formulae (111) to (120) represents a bonding position.

A group represented by $-(L_{100})_{mx}-$ in the formula (110) is preferably a group represented by the formula (111) or (112).

In the compound according to the exemplary embodiment, $Ar_{100}$ is preferably an aryl group in which at least four substituted or unsubstituted benzene rings are fused.

In the compound according to the exemplary embodiment, $Ar_{100}$ is preferably an aryl group in which four substituted or unsubstituted benzene rings are fused or an aryl group in which five substituted or unsubstituted benzene rings are fused.

In the compound according to the exemplary embodiment, $Ar_{100}$ is preferably a group represented by a formula (1100), (1200), (1300), (1400), (1500), (1600), (1700), or (1800).

[Formula 31]

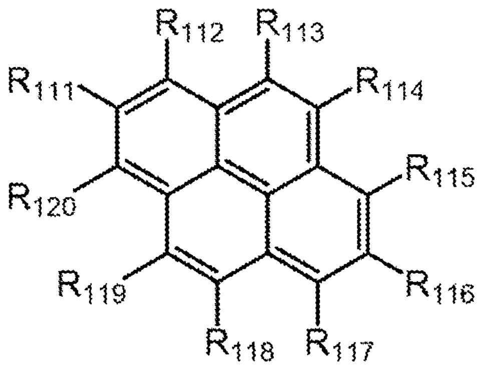

(1100)

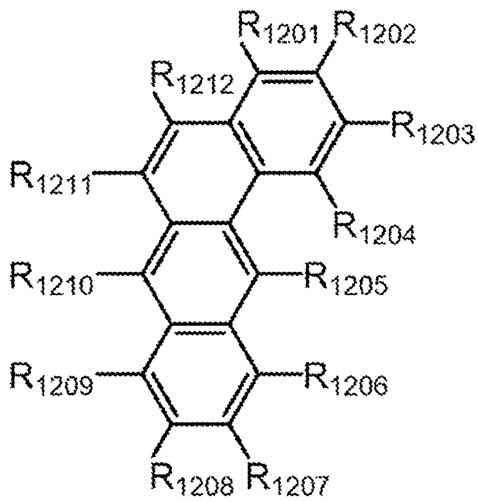

(1200)

[Formula 32]

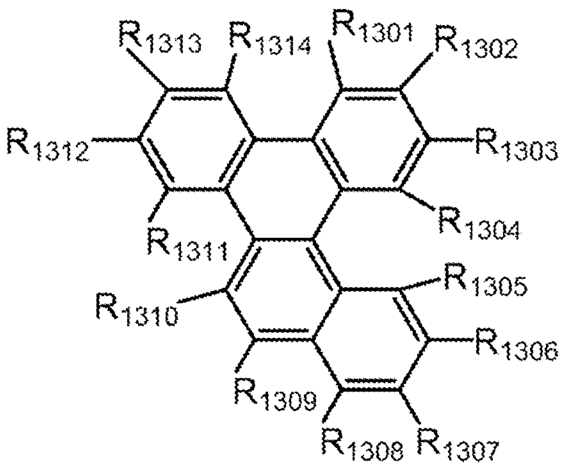

(1300)

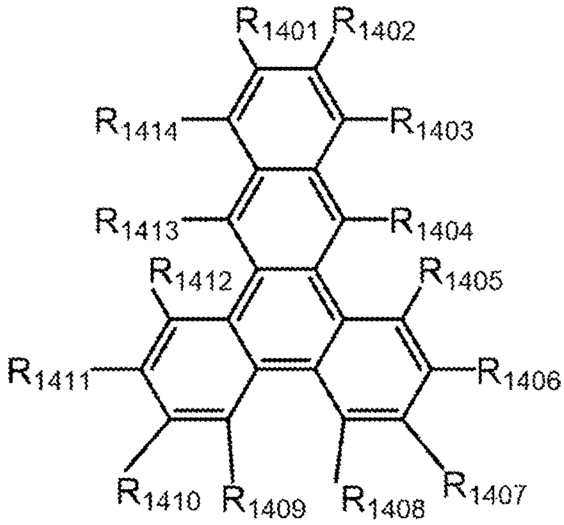

(1400)

[Formula 33]

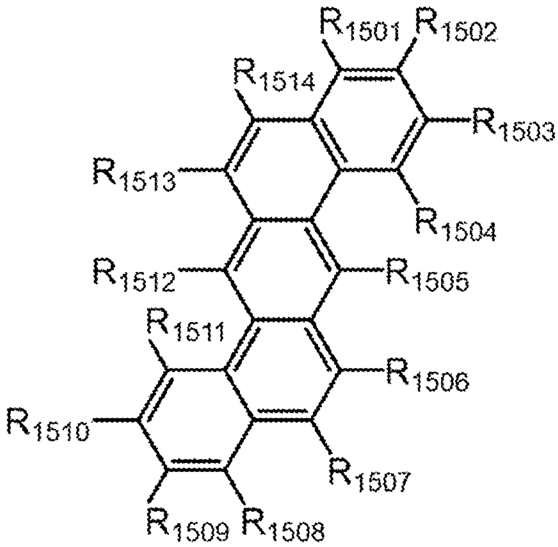

(1500)

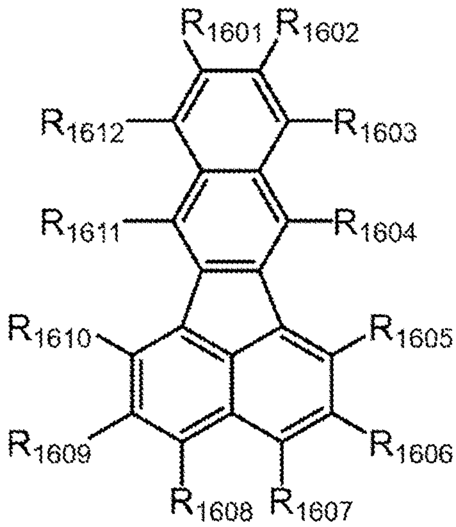

(1600)

[Formula 34]

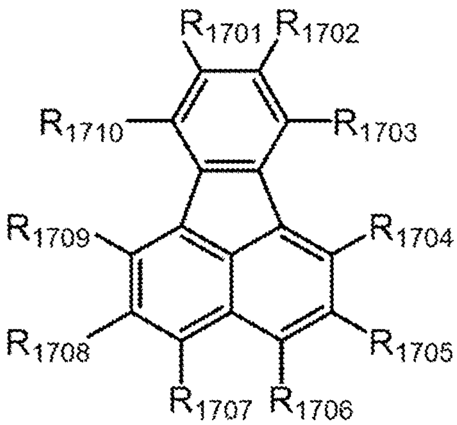

(1700)

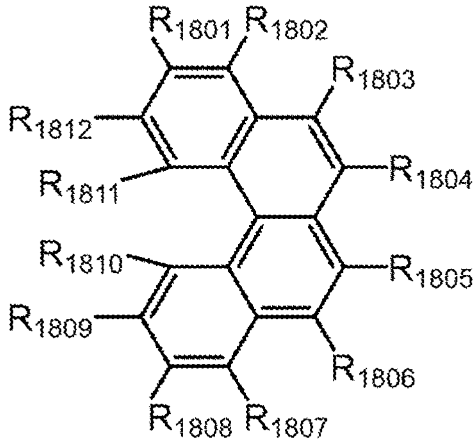

(1800)

In the formula (1100), one of $R_{111}$ to $R_{120}$ is a bond.
In the formula (1200), one of $R_{1201}$ to $R_{1212}$ is a bond.
In the formula (1300), one of $R_{1301}$ to $R_{1314}$ is a bond.
In the formula (1400), one of $R_{1401}$ to $R_{1414}$ is a bond.
In the formula (1500), one of $R_{1501}$ to $R_{1514}$ is a bond.
In the formula (1600), one of $R_{1601}$ to $R_{1612}$ is a bond.
In the formula (1700), one of $R_{1701}$ to $R_{1710}$ is a bond.
In the formula (1800), one of $R_{1801}$ to $R_{1812}$ is a bond.
$R_{111}$ to $R_{120}$ not being a bond, $R_{1201}$ to $R_{1212}$ not being a bond, $R_{1301}$ to $R_{1314}$ not being a bond, $R_{1401}$ to $R_{1414}$ not being a bond, $R_{1501}$ to $R_{1514}$ not being a bond, $R_{1601}$ to $R_{1612}$ not being a bond, $R_{1701}$ to $R_{1710}$ not being a bond, and $R_{1801}$ to $R_{1812}$ not being a bond are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by $-C(=O)R_{801}$, a group represented by $-COOR_{802}$, a halogen atom, a nitro group, a substituted or unsubstituted aryl group having 6 to 50 ring carbon atoms, or a substituted or unsubstituted heterocyclic group having 5 to 50 ring atoms.
A group represented by the formula (1100) in which $R_{111}$ is a bond is represented by a formula (1112) below. A group represented by the formula (1100) in which $R_{120}$ is a bond is represented by a formula (1113) below. A group represented by the formula (1100) in which $R_{119}$ is a bond is represented by a formula (1114) below.

[Formula 35]

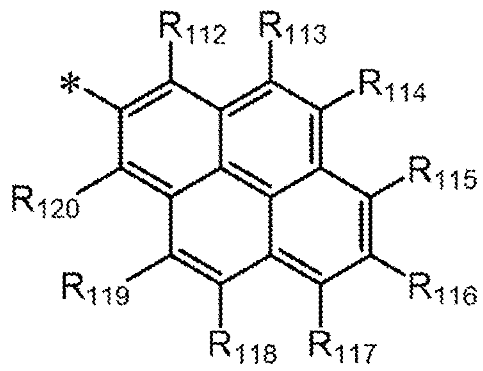

(1112)

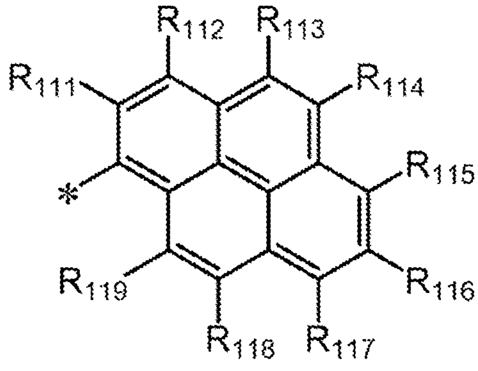

(1113)

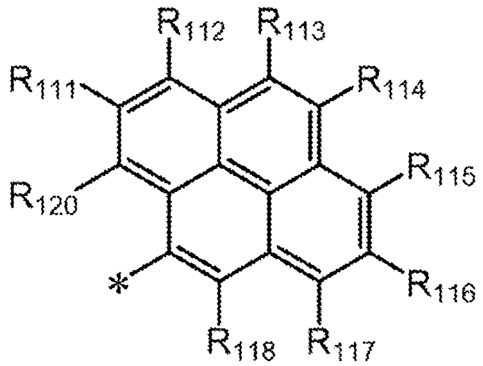

(1114)

In the formulae (1112), (1113), and (1114):

$R_{111}$ to $R_{120}$ are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by $-C(=O)R_{801}$, a group represented by $-COOR_{802}$, a halogen atom, a nitro group, a substituted or unsubstituted aryl group having 6 to 50 ring carbon atoms, or a substituted or unsubstituted heterocyclic group having 5 to 50 ring atoms; and

* in the formulae (1112) to (1114) represents a bonding position.

In the compound according to the exemplary embodiment, $R_{111}$ to $R_{120}$ not being a bond, $R_{1201}$ to $R_{1212}$ not being a bond, $R_{1301}$ to $R_{1314}$ not being a bond, $R_{1401}$ to $R_{1414}$ not being a bond, $R_{1501}$ to $R_{1514}$ not being a bond, $R_{1601}$ to $R_{1612}$ not being a bond, $R_{1701}$ to $R_{1710}$ not being a bond, and $R_{1801}$ to $R_{1812}$ not being a bond are preferably each independently a hydrogen atom, a substituted or unsubstituted alkyl group having 1 to 50 carbon atoms, a substituted or unsubstituted cycloalkyl group having 3 to 50 ring carbon atoms, or a substituted or unsubstituted aryl group having 6 to 50 ring carbon atoms.

In the compound according to the exemplary embodiment, $R_{111}$ to $R_{120}$ not being a bond, $R_{1201}$ to $R_{1212}$ not being a bond, $R_{1301}$ to $R_{1314}$ not being a bond, $R_{1401}$ to $R_{1414}$ not being a bond, $R_{1501}$ to $R_{1514}$ not being a bond, $R_{1601}$ to $R_{1612}$ not being a bond, $R_{1701}$ to $R_{1710}$ not being a bond, and $R_{1801}$ to $R_{1812}$ not being a bond are preferably each a hydrogen atom.

In the compound according to the exemplary embodiment, $Ar_{100}$ as a substituted or unsubstituted heterocyclic group including at least two aromatic rings and at least one heterocyclic group is also preferably, for instance, a substituted or unsubstituted benzoxanthenyl group, a substituted or unsubstituted dibenzofuranyl group, a substituted or unsubstituted naphthobenzofuranyl group, or a substituted or unsubstituted dibenzothienyl group.

The compound according to the exemplary embodiment preferably does not include a cyano group in a molecule.

The compound according to the exemplary embodiment also preferably includes one or two benzoxanthene rings in a molecule.

The compound according to the exemplary embodiment also preferably includes only a single benzoxanthene ring in a molecule.

The compound according to the exemplary embodiment is also preferably a compound having at least one group represented by a formula (11) below and a single benz[de]anthracene derivative skeleton represented by a formula (1000) below in a molecule.

[Formula 36]

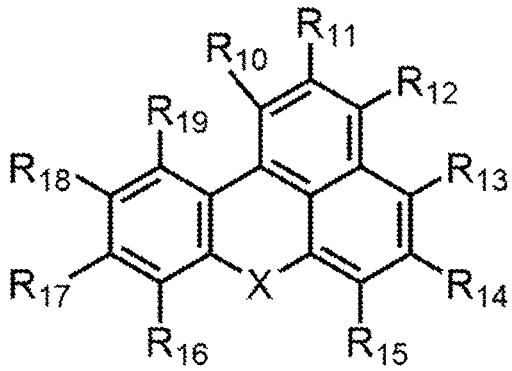

(1000)

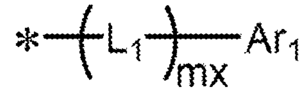

(11)

In the formula (1000):

X is an oxygen atom, a sulfur atom, $C(R_{2001})(R_{2002})$, or $Si(R_{2003})(R_{2004})$;
at least one combination of adjacent two or more of $R_{10}$ to $R_{19}$ are mutually bonded to form a substituted or unsubstituted monocyclic ring, mutually bonded to form a substituted or unsubstituted fused ring, or not mutually bonded;
$R_{10}$ to $R_{19}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by $-C(=O)R_{801}$, a group represented by $-COOR_{802}$, a halogen atom, a nitro group, a substituted or unsubstituted aryl group having 6 to 50 ring carbon atoms, a substituted or unsubstituted heterocyclic group having 5 to 50 ring atoms, or a group represented by the formula (11),
at least one of $R_{10}$ to $R_{19}$ is a group represented by the formula (11);
when a plurality of groups represented by the formula (11) are present, the plurality of groups represented by the formula (11) are mutually the same or different;
$L_1$ is a substituted or unsubstituted arylene group having 6 to 15 ring carbon atoms, or a substituted or unsubstituted divalent heterocyclic group having 5 to 15 ring atoms;
mx is 1, 2, or 3;
when two or more $L_1$ are present, the two or more $L_1$ are mutually the same or different;
$Ar_1$ is a substituted or unsubstituted aryl group including four or more rings;
when two or more $Ar_1$ are present, the two or more $Ar_1$ are mutually the same or different; and
* in the formula (11) represents a bonding position.

A compound represented by the formula (1000) preferably includes at least one group represented by the formula (11) and a single benzoxanthene ring represented by a formula (1) below in a molecule.

[Formula 37]

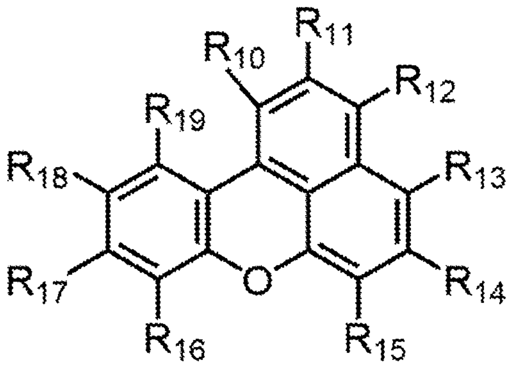

(1)

In the formula (1): $R_{10}$ to $R_{19}$ each independently represent the same as $R_{10}$ to $R_{19}$ in the formula (1000); none of combinations of adjacent two or more of $R_{10}$ to $R_{19}$ are mutually bonded; and $Ar_1$, $L_1$, and mx respectively represent the same as Ar1, $L_1$, and mx in the formula (11).

In a compound represented by the formula (1), $L_1$ that is a substituted or unsubstituted arylene group having 6 to 15 ring carbon atoms or a substituted or unsubstituted divalent heterocyclic group having 5 to 15 ring atoms is provided between a benzoxanthene ring and $Ar_1$ that is a substituted or unsubstituted aryl group having four or more rings, whereby a decrease in a hole mobility can be inhibited and a decrease in a luminous efficiency of an organic EL device can be also inhibited.

The compound including at least one group represented by the formula (11) and a single benzoxanthene ring represented by the formula (1) in a molecule is preferably represented by a formula (121) or (122).

[Formula 38]

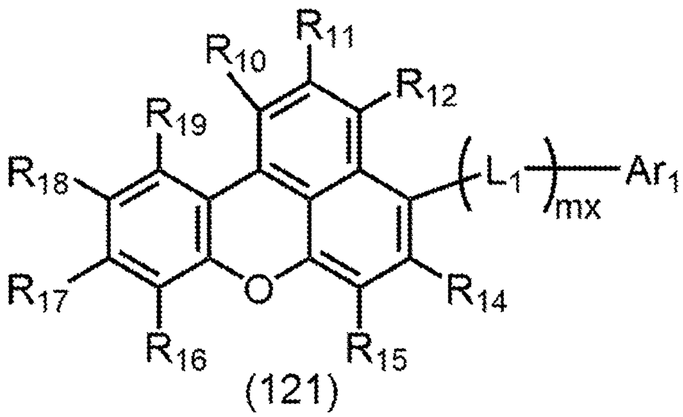

(121)

[Formula 39]

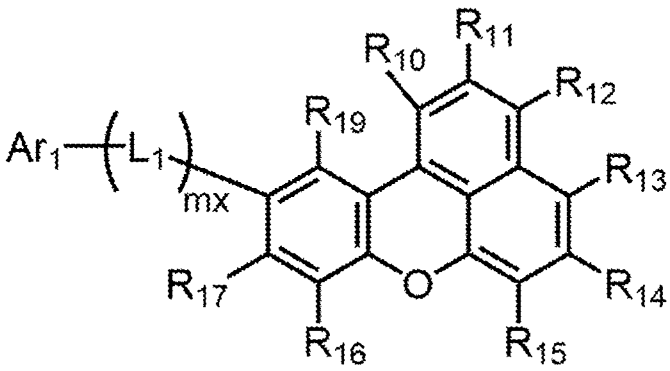

(122)

In the formulae (121) and (122): $R_{10}$ to $R_{19}$ each independently represent the same as $R_{10}$ to $R_{19}$ in the formula (1); and Ar1, $L_1$, and mx respectively represent the same as $Ar_1$, $L_1$, and mx in the formula (11).

In the formula (121), $R_{10}$ to $R_{12}$ and $R_{14}$ to $R_{19}$ are each preferably not a group represented by the formula (11).

In the formula (122), $R_{10}$ to $R_{17}$ and $R_{19}$ are each preferably not a group represented by the formula (11).

In a compound represented by the formula (1), $R_{10}$ to $R_{19}$ not being a group represented by the formula (11) are preferably each independently a hydrogen atom, a substituted or unsubstituted alkyl group having 1 to 50 carbon atoms, a substituted or unsubstituted cycloalkyl group having 3 to 50 ring carbon atoms, or a substituted or unsubstituted aryl group having 6 to 50 ring carbon atoms.

In a compound represented by the formula (1), $R_{10}$ to $R_{19}$ not being a group represented by the formula (11) are preferably each a hydrogen atom.

In a compound represented by the formula (1), $L_1$ is preferably an arylene group including at most three substituted or unsubstituted benzene rings.

In a compound represented by the formula (1), $L_1$ is preferably not a substituted or unsubstituted anthrylene group.

In a compound represented by the formula (1), a group represented by - $(L_1)_{mx}$- in the formula (11) is also preferably a group represented by one of the formulae (111) to (120).

In a compound represented by the formula (1), a group represented by - $(L_1)m_x$- in the formula (11) is preferably a group represented by one of the formulae (111) and (112).

The compound including at least one group represented by the formula (11) and a single benz[de]anthracene derivative skeleton represented by the formula (1000) in a molecule is also preferably represented by a formula (123), (124), (125), or (126).

[Formula 40]

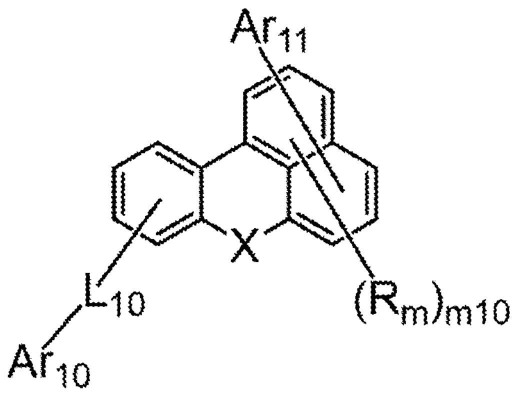

(123)

[Formula 41]

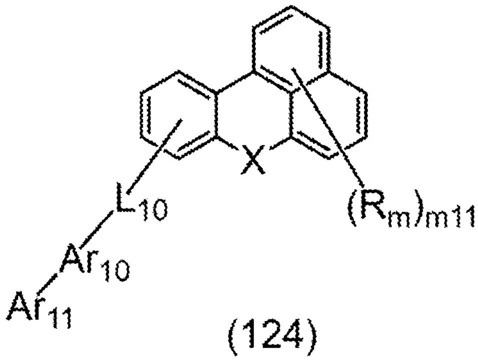

(124)

[Formula 42]

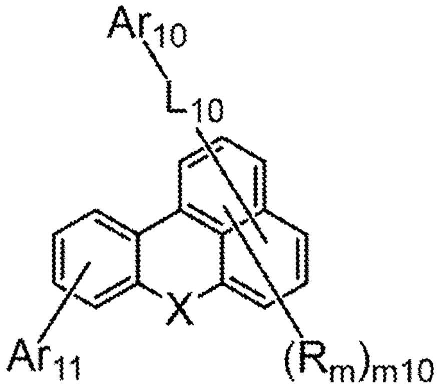

(125)

[Formula 43]

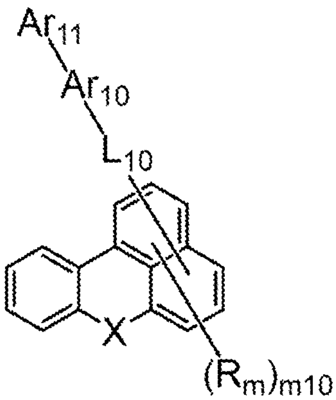

(126)

In the formulae (123), (124), (125), and (126):

X is an oxygen atom, a sulfur atom, $C(R_{2001})(R_{2002})$, or $Si(R_{2003})(R_{2004})$;

$R_{2001}$ to $R_{2004}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms;

$L_{10}$ is a substituted or unsubstituted arylene group having 6 to 15 ring carbon atoms, or a substituted or unsubstituted divalent heterocyclic group having 5 to 15 ring atoms;

$Ar_{10}$ is an aryl group in which at least four substituted or unsubstituted benzene rings are fused;

$Ar_{11}$ is a substituted or unsubstituted aryl group having 6 to 14 ring carbon atoms;

$Ar_{11}$ is not a substituted or unsubstituted anthryl group;

m10 is 5;

m11 is 6;

Rm is each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by $-C(=O)R_{801}$, a group represented by $-COOR_{802}$, a halogen atom, a nitro group, a substituted or unsubstituted aryl group having 6 to 50 ring carbon atoms, or a substituted or unsubstituted heterocyclic group having 5 to 50 ring atoms;

a plurality of Rm are not mutually bonded;

$R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{901}$ are present, the plurality of $R_{901}$ are mutually the same or different;

when a plurality of $R_{902}$ are present, the plurality of $R_{902}$ are mutually the same or different;

when a plurality of $R_{903}$ are present, the plurality of $R_{903}$ are mutually the same or different;

when a plurality of $R_{904}$ are present, the plurality of $R_{904}$ are mutually the same or different;

when a plurality of $R_{905}$ are present, the plurality of $R_{905}$ are mutually the same or different;

when a plurality of $R_{906}$ are present, the plurality of $R_{906}$ are mutually the same or different;

when a plurality of $R_{907}$ are present, the plurality of $R_{907}$ are mutually the same or different;

when a plurality of $R_{801}$ are present, the plurality of $R_{801}$ are mutually the same or different; and

when a plurality of $R_{802}$ are present, the plurality of $R_{802}$ are mutually the same or different.

As in a compound represented by the formula (123) and a compound represented by the formula (125), a benz[de]anthracene derivative skeleton is substituted by $Ar_{11}$ (a substituted or unsubstituted aryl group having 6 to 14 ring carbon atoms except for a substituted or unsubstituted anthryl group), whereby a singlet energy $S_1$ can be decreased and an excited state can be expected to be stabilized, so that a lifetime of an organic EL device can be prolonged.

As in a compound represented by the formula (124) and a compound represented by the formula (126), $Ar_{10}$ (an aryl group in which at least four substituted or unsubstituted benzene rings are fused) is substituted by $Ar_{11}$ (a substituted or unsubstituted aryl group having 6 to 14 ring carbon atoms except for a substituted or unsubstituted anthryl group), whereby electron transportability can be improved and a carrier balance of an organic EL device using the compound can be expected to be improved.

A compound represented by the formula (123) is more preferably represented by a formula (1230) below. A compound represented by the formula (124) is more preferably represented by a formula (1240) below. A compound represented by the formula (125) is more preferably represented by a formula (1250) below. A compound represented by the formula (126) is more preferably represented by a formula (1260) below.

[Formula 44]

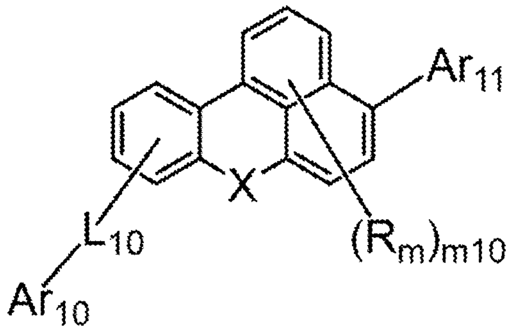

(1230)

[Formula 45]

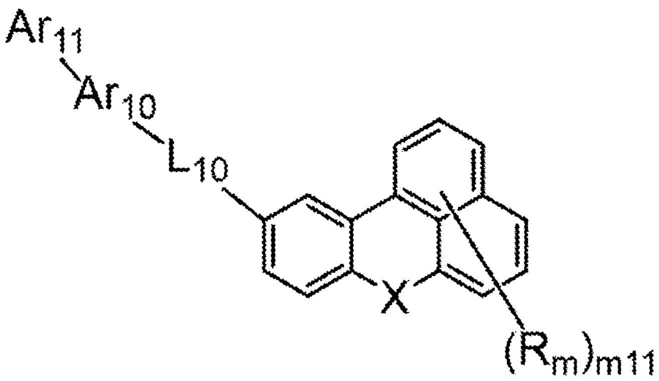

(1240)

[Formula 46]

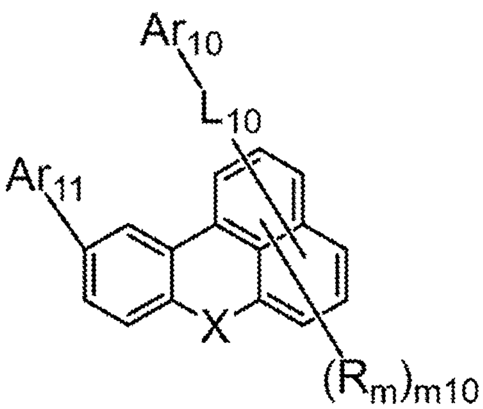

(1250)

[Formula 47]

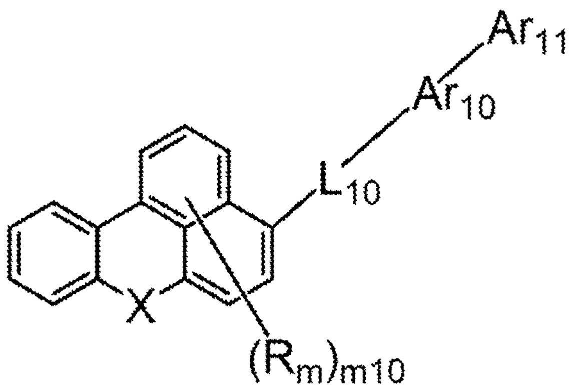

(1260)

In the formulae (1230), (1240), (1250), and (1260), X, $L_{10}$, $Ar_{10}$, $Ar_{11}$, m10, m11, and Rm each independently represent the same as $L_{10}$, $Ar_{10}$, $Ar_{11}$, m10, m11, and Rm in the formulae (123), (124), (125), and (126).

In a compound represented by the formula (123), (124), (125), (126), (1230), (1240), (1250), or (1260), X is preferably an oxygen atom.

In a compound represented by the formula (123), (124), (125), (126), (1230), (1240), (1250), or (1260), $L_{10}$ is preferably an arylene group including at most three substituted or unsubstituted benzene rings.

In a compound represented by the formula (123), (124), (125), (126), (1230), (1240), (1250), or (1260), a group represented by $-L_{10}-$ is preferably a group represented by one of the formulae (111) to (120).

In a compound represented by the formula (123), (124), (125), (126), (1230), (1240), (1250), or (1260), a group represented by $-L_{10}-$ is preferably a group represented by the formula (111) or (112).

The compound according to the exemplary embodiment is also preferably a compound having at least one group represented by a formula (110A) below and a single benz[de]anthracene derivative skeleton represented by a formula (1000A) below in a molecule.

[Formula 48]

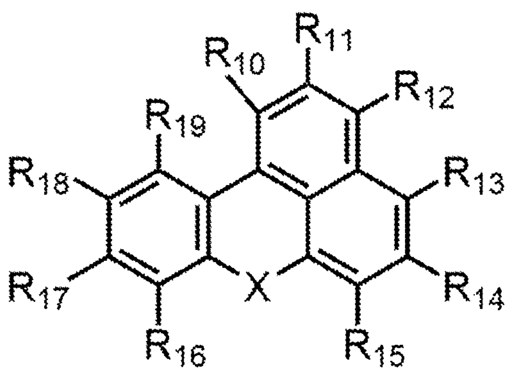

(1000A)

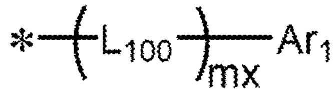

(110A)

In the formula (1000A):

X is an oxygen atom, a sulfur atom, $C(R_{2001})(R_{2002})$, or $Si(R_{2003})(R_{2004})$;
at least one combination of adjacent two or more of $R_{10}$ to $R_{19}$ are mutually bonded to form a substituted or unsubstituted monocyclic ring, mutually bonded to form a substituted or unsubstituted fused ring, or not mutually bonded;

$R_{10}$ to $R_{19}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by - $S-(R_{905})$, a group represented by $-N(R_{906})(R_{907})$, a substituted or unsubstituted aralkyl group having 7 to 50 carbon atoms, a group represented by $-C(=O)R_{801}$, a group represented by $-COOR_{802}$, a halogen atom, a nitro group, a substituted or unsubstituted aryl group having 6 to 50 ring carbon atoms, a substituted or unsubstituted heterocyclic group having 5 to 50 ring atoms, or a group represented by the formula (110A);

at least one of $R_{13}$ or $R_{18}$ is a group represented by the formula (110A);

when a plurality of groups represented by the formula (110A) are present, the plurality of groups represented by the formula (110A) are mutually the same or different;

$L_{100}$ is a single bond, a substituted or unsubstituted arylene group having 6 to 50 ring carbon atoms, or a substituted or unsubstituted divalent heterocyclic group having 5 to 50 ring atoms;

mx is 1, 2, or 3;

when two or more $L_{100}$ are present, the two or more $L_{100}$ are mutually the same or different;

$Ar_1$ is a substituted or unsubstituted aryl group including four or more rings;

when two or more $Ar_1$ are present, the two or more $Ar_1$ are mutually the same or different; and

* in the formula (110A) represents a bonding position.

A compound represented by the formula (1000A) preferably includes at least one group represented by the formula (110A) and a single benzoxanthene ring represented by a formula (100A) below in a molecule.

[Formula 49]

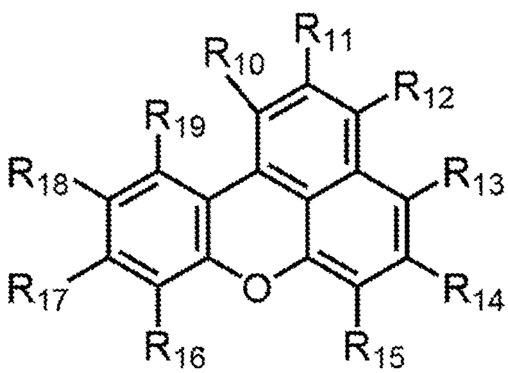

(100A)

In the formula (100A): $R_{10}$ to $R_{19}$ each independently represent the same as $R_{10}$ to $R_{19}$ in the formula (1000A); none of combinations of adjacent two or more of $R_{10}$ to $R_{19}$ are mutually bonded; and $Ar_1$, $L_1$, and mx respectively represent the same as Ar1, $L_1$, and mx in the formula (110A).

In a compound represented by the formula (100A), at least one of $R_{13}$ or $R_{18}$ is a group having $Ar_1$ that is a substituted or unsubstituted aryl group including four or more rings. Hole injectability of the compound according to the exemplary embodiment is improved by $Ar_1$ being thus bonded to a benzoxanthene ring at a para position with respect to an oxygen atom (O) through a single bond, a substituted or unsubstituted arylene group having 6 to 50 ring carbon atoms, or a substituted or unsubstituted divalent heterocyclic group having 5 to 50 ring atoms. Therefore, the luminous efficiency of the organic EL device containing the compound according to the exemplary embodiment can be expected to be improved.

A compound represented by the formula (100A) is also preferably represented by a formula (121A) or (122A) below.

[Formula 50]

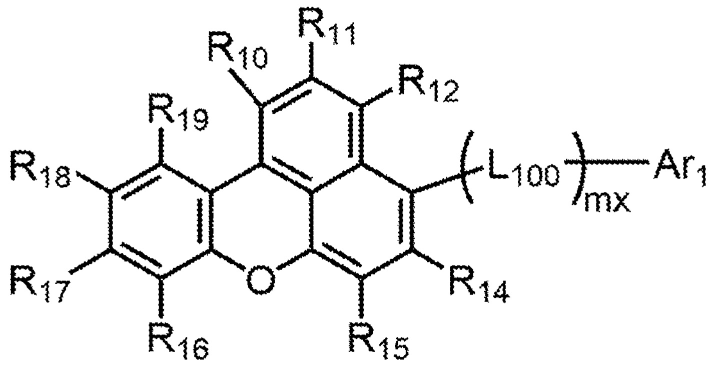

(121A)

[Formula 51]

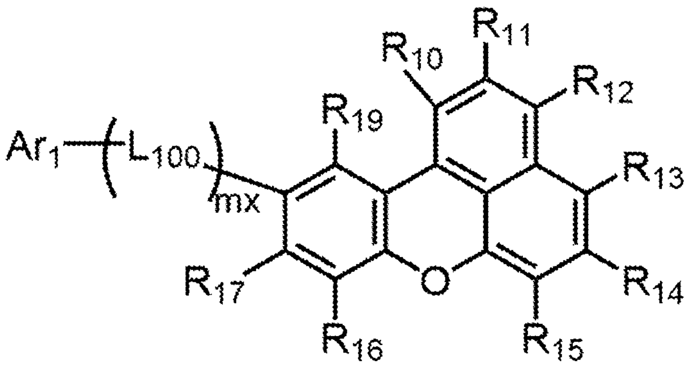

(122A)

In the formulae (121A) and (122A): $R_{10}$ to $R_{19}$ each independently represent the same as $R_{10}$ to $R_{19}$ in the formula (100A); and Ar1, $L_{100}$, and mx respectively represent the same as Ar1, $L_{100}$, and mx in the formula (110A).

In the formula (121A), $R_{10}$ to $R_{12}$ and $R_{14}$ to $R_{19}$ are each preferably not a group represented by the formula (110A).

In the formula (122A), $R_{10}$ to $R_{17}$ and $R_{19}$ are each preferably not a group represented by the formula (110A).

In the compound represented by the formula (100A), $R_{10}$ to $R_{19}$ not being a group represented by the formula (110A) are preferably each independently a hydrogen atom, a substituted or unsubstituted alkyl group having 1 to 50 carbon atoms, a substituted or unsubstituted cycloalkyl group having 3 to 50 ring carbon atoms, or a substituted or unsubstituted aryl group having 6 to 50 ring carbon atoms.

In a compound represented by the formula (100A), $R_{10}$ to $R_{19}$ not being a group represented by the formula (110A) are preferably each a hydrogen atom.

In a compound represented by the formula (100A), $L_{100}$ is preferably a single bond or an arylene group including at most three substituted or unsubstituted benzene rings.

In a compound represented by the formula (100A), a group represented by - $(L_{100})_{mx}$- is preferably a group represented by one of the formulae (111) to (120).

In a compound represented by the formula (100A), a group represented by - $(L_{100})_{mx}$- is preferably a group represented by the formula (111) or (112).

In the compound according to the exemplary embodiment, $Ar_1$ is preferably an aryl group in which at least four substituted or unsubstituted benzene rings are fused.

In the compound according to the exemplary embodiment, $Ar_1$ or $Ar_{10}$ is preferably a substituted or unsubstituted aryl group in which four benzene rings are fused or a substituted or unsubstituted aryl group in which five benzene rings are fused.

In the compound according to the exemplary embodiment, $Ar_1$ or $Ar_{10}$ is preferably a group represented by a formula (1100), (1200), (1300), (1400), (1500), (1600), (1700), or (1800).

In the compound according to the exemplary embodiment, $Ar_1$ or $Ar_{10}$ is preferably a group represented by the formula (1100) or (1200).

In the compound according to the exemplary embodiment, it is preferable that all of the "substituted or unsubstituted" groups are "unsubstituted" groups.

In the compound according to the exemplary embodiment (e.g., a compound represented by the formula (1000B), a compound represented by the formula (1000), a compound represented by the formula (1000A)), in a case of "at least one of combinations of adjacent two or more of $R_{10}$ to $R_{19}$ are mutally bonded to form a ring (substituted or unsubstituted monocyclic ring or substituted or unsubstituted fused ring), examples of the combinations of adjacent two or more include a combination of $R_{10}$ and $R_{11}$, a combination of $R_{11}$ and $R_{12}$, a combination of $R_{12}$ and $R_{13}$, a combination of $R_{13}$ and $R_{14}$, a combination of $R_{14}$ and $R_{15}$, a combination of $R_{16}$ and $R_{17}$, a combination of $R_{17}$ and $R_{18}$, a combination of $R_{18}$ and $R_{19}$, and a combination of $R_{19}$ and $R_{10}$.

In the compound according to the exemplary embodiment (e.g., a compound represented by the formula (1000B), a compound represented by the formula (1000), a compound represented by the formula (1000A)), it is preferable that a combination of $R_{13}$ and $R_{14}$, a combination of $R_{16}$ and $R_{17}$, or a combination of $R_{19}$ and $R_{10}$ are mutually bonded to form a substituted or unsubstituted benzene ring.

In the compound according to the exemplary embodiment (e.g., a compound represented by the formula (1000B), a compound represented by the formula (1000), a compound represented by the formula (1000A)), $R_{2001}$ to $R_{2004}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

In the compound according to the exemplary embodiment (e.g., a compound represented by the formula (1000B), a compound represented by the formula (1000), a compound represented by the formula (1000A)), $R_{2001}$ to $R_{2004}$ are preferably each independently a hydrogen atom, a substituted or unsubstituted alkyl group having 1 to 50 carbon atoms, a halogen atom, a cyano group, a nitro group, a substituted or unsubstituted aryl group having 6 to 50 ring carbon atoms, or a substituted or unsubstituted heterocyclic group having 5 to 50 ring atoms.

In the compound according to the exemplary embodiment (e.g., a compound represented by the formula (1000B), a compound represented by the formula (1000), a compound represented by the formula (1000A)):

$R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;
when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{801}$ are present, the plurality of $R_{801}$ are mutually the same or different; and
when a plurality of $R_{802}$ are present, the plurality of $R_{802}$ are mutually the same or different.

A hydrogen atom in the compound according to the exemplary embodiment is a protium atom, a deuterium atom, or a tritium atom. In an exemplary embodiment, a hydrogen atom in the compound according to the exemplary embodiment is a protium atom. In an exemplary embodiment, a hydrogen atom in the compound according to the exemplary embodiment is a deuterium atom.

Manufacturing Method of Compound According to First Exemplary Embodiment

The compound according to the exemplary embodiment can be manufactured by application of known substitution reactions and materials depending on a target compound, in accordance with or based on synthesis methods described later in Examples.

Specific Examples of Compound According to First Exemplary Embodiment

Examples of the compound according to the exemplary embodiment include the following compounds. However, the invention is not limited to these specific examples. In the chemical formulae herein, a deuterium atom is denoted by D and a protium atom is denoted by H or a description for a protium is omitted.

[Formula 52]

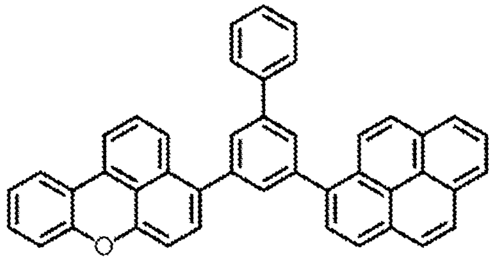

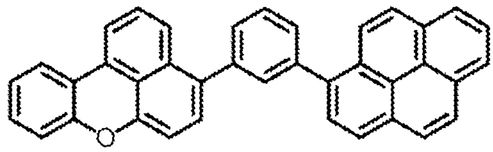

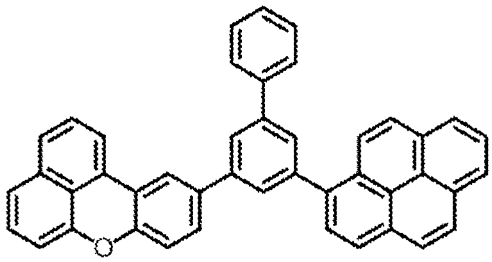

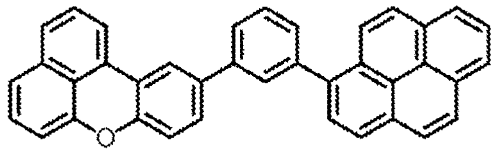

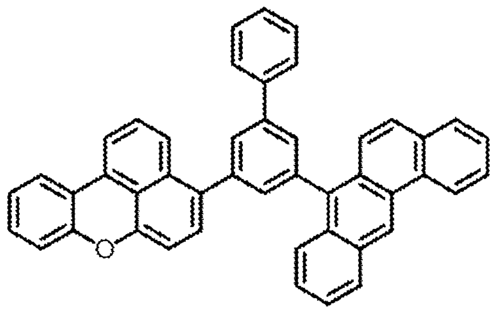

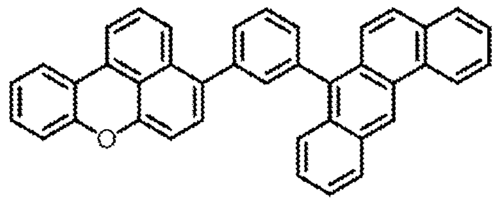

[Formula 53]

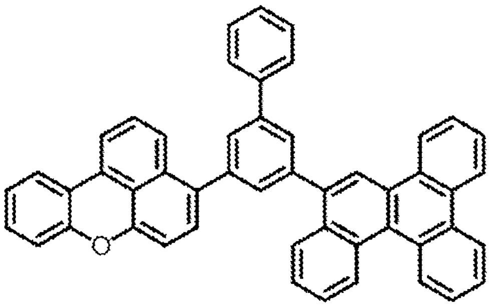

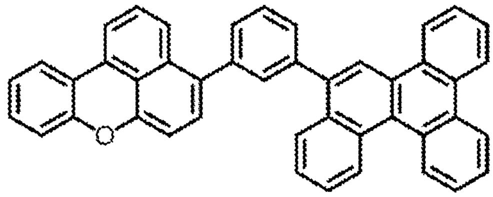

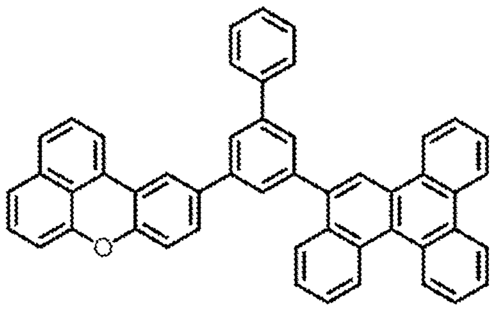

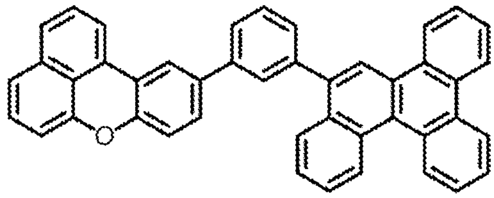

[Formula 54]

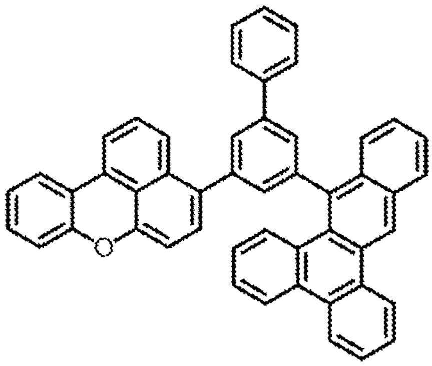

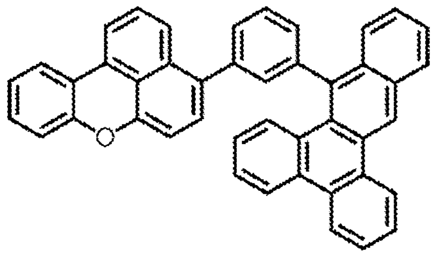

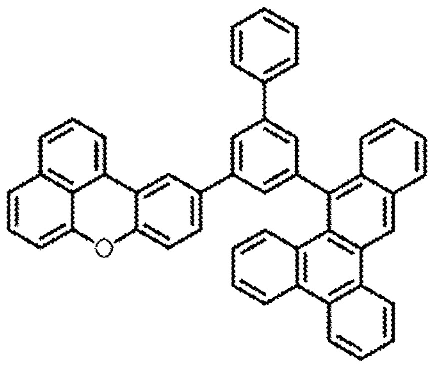

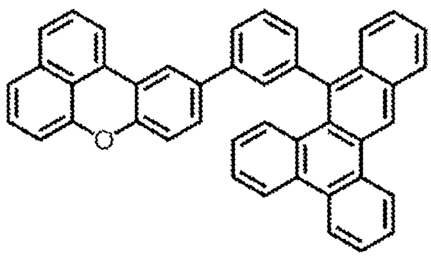

[Formula 55]

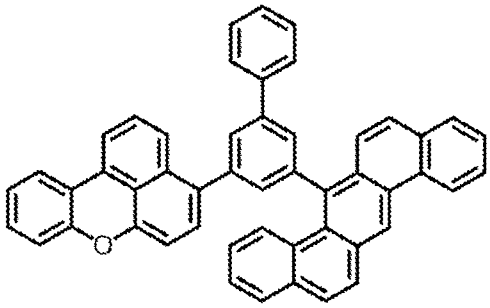

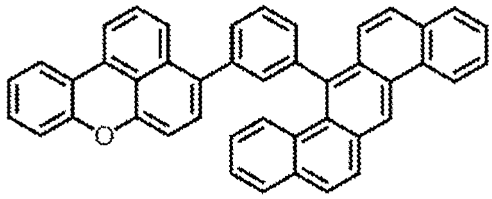

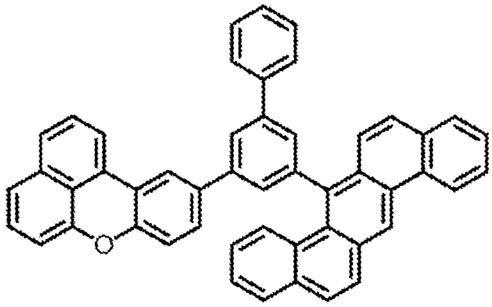

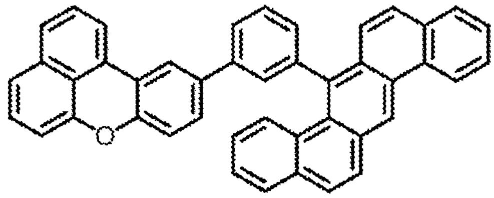

[Formula 56]

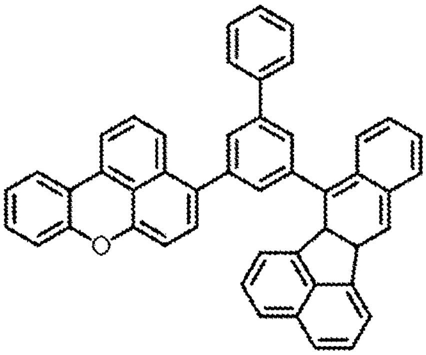

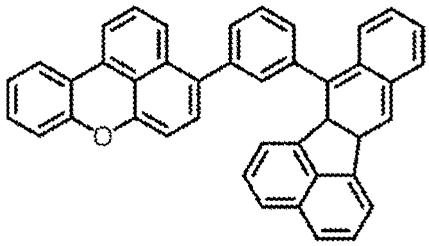

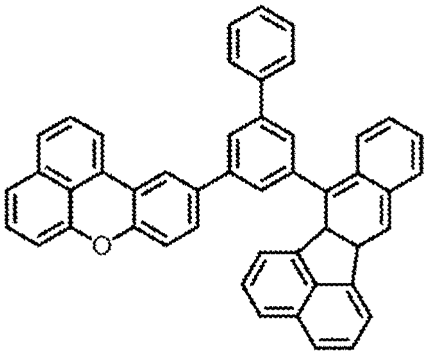

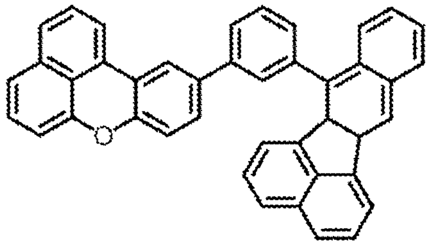

[Formula 57]

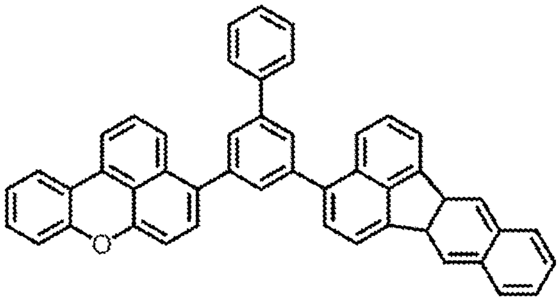

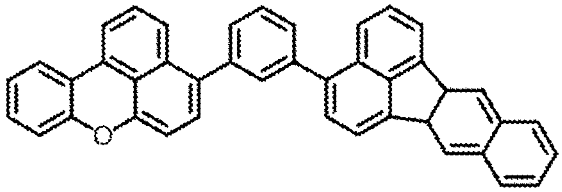

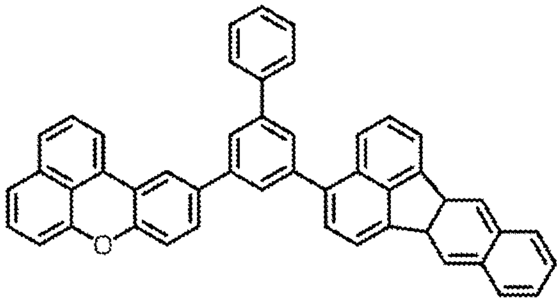

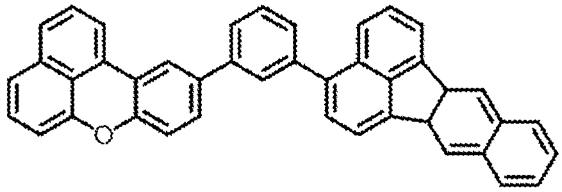

[Formula 58]

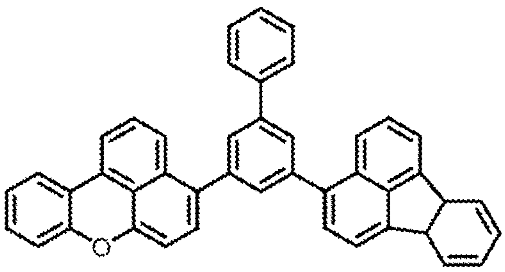

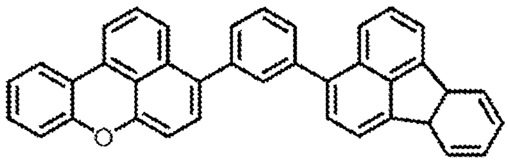

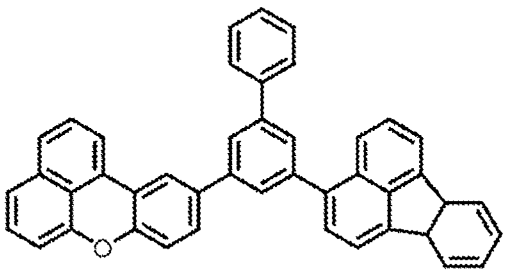

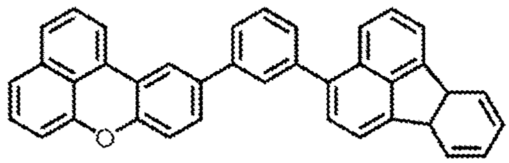

[Formula 59]

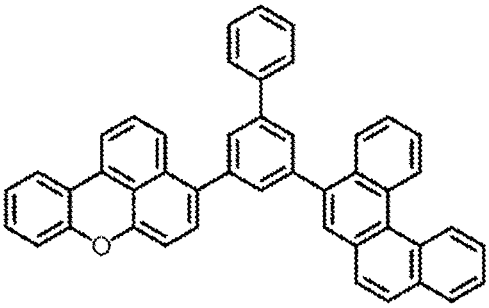
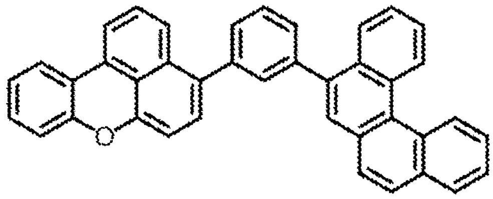
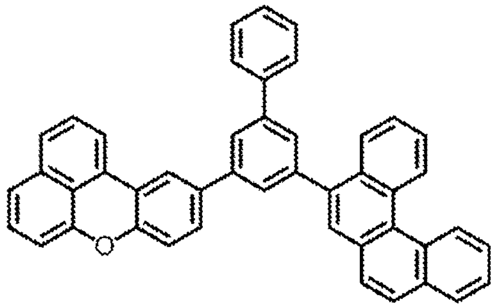
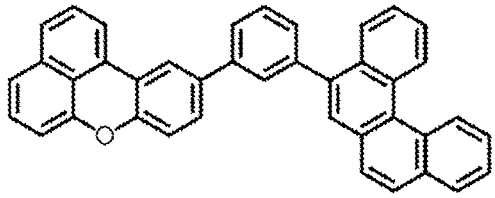

[Formula 60]

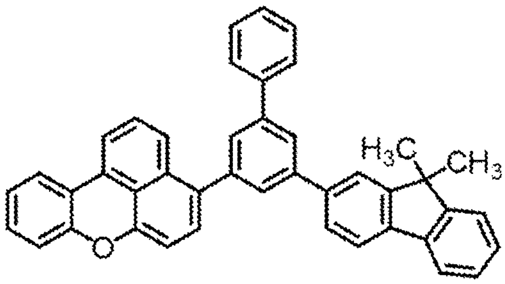
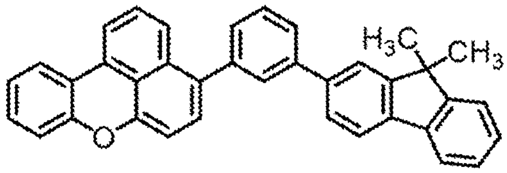
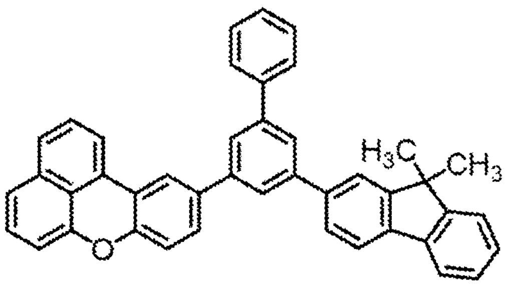
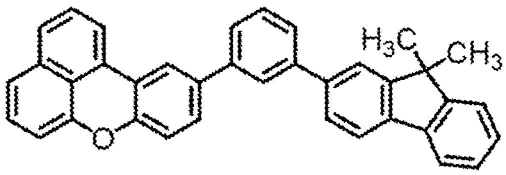

[Formula 61]

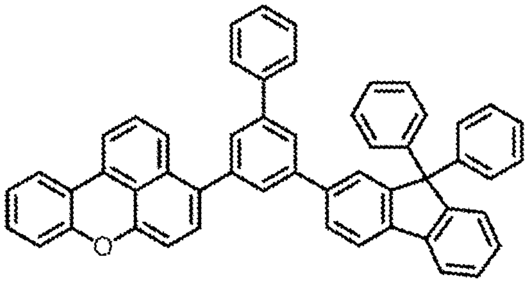

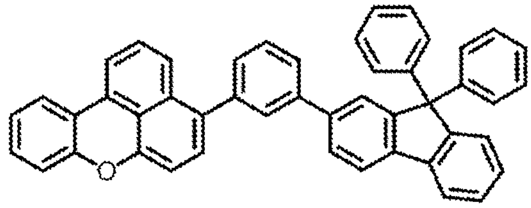

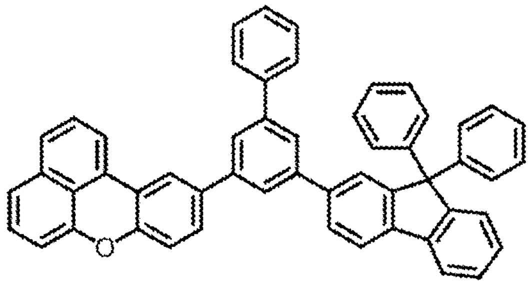

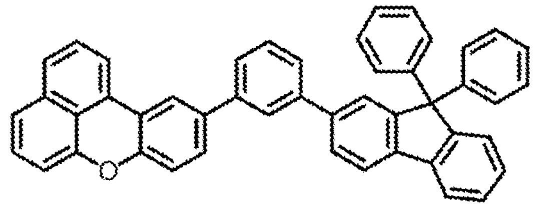

[Formula 62]

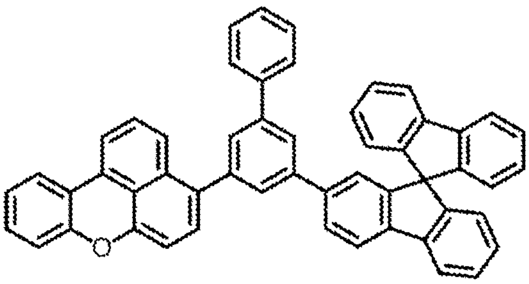

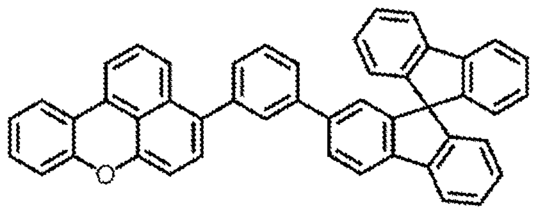

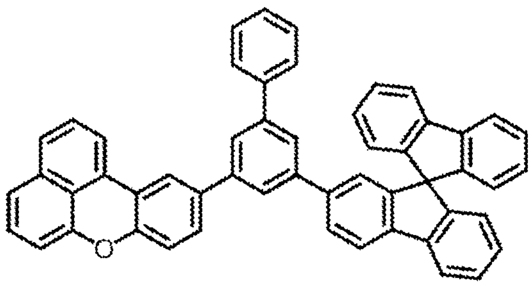

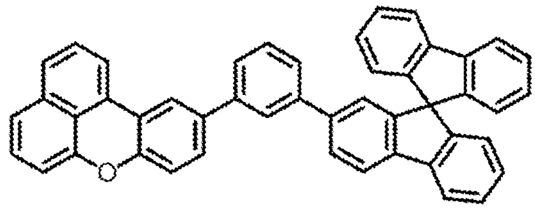

[Formula 63]

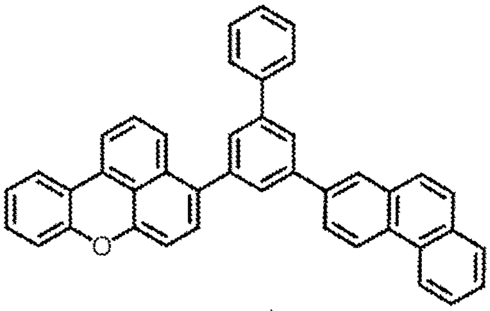

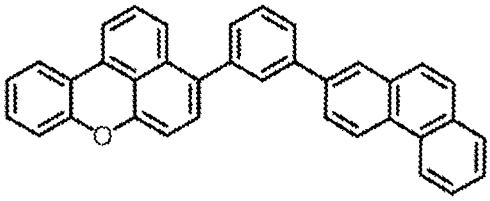

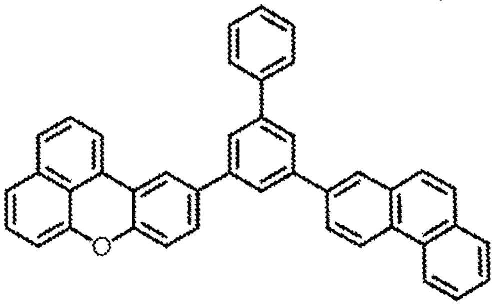

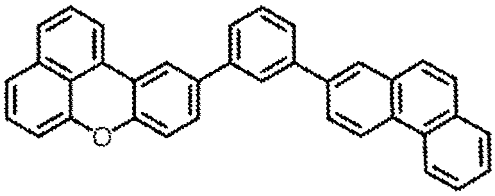

[Formula 64]

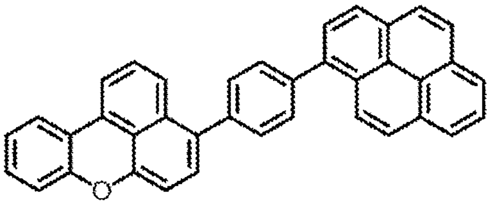

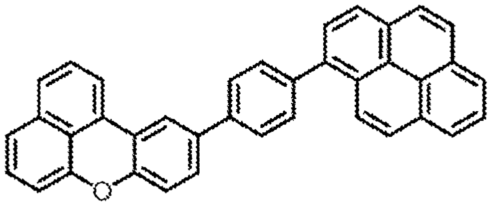

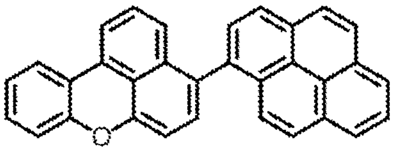

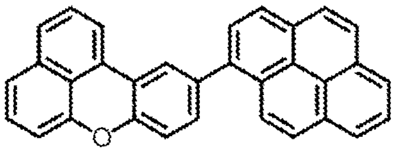

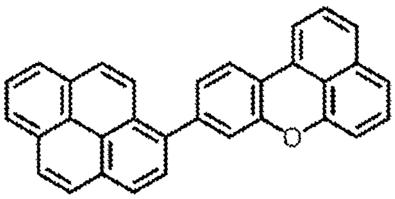

[Formula 65]

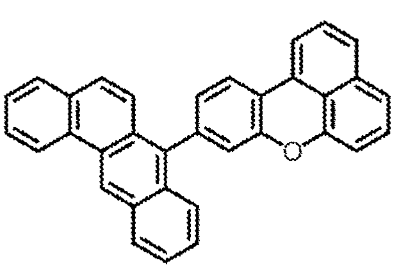
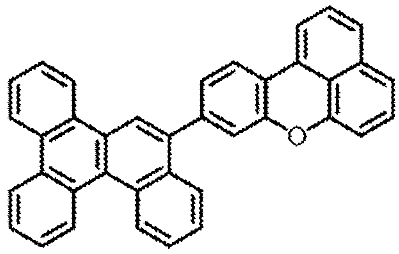
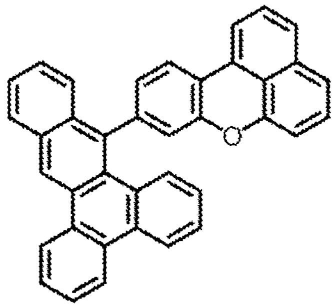

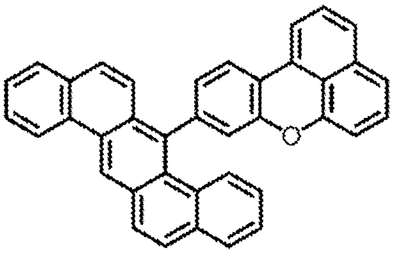
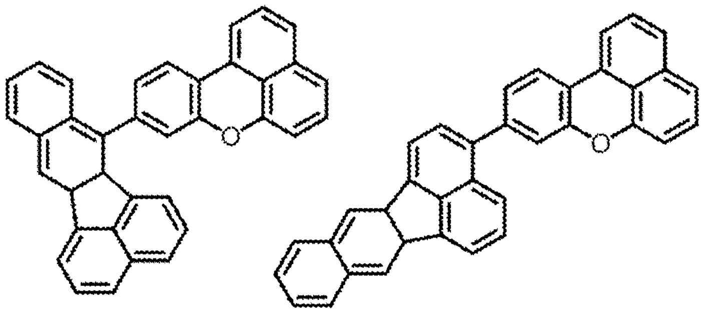

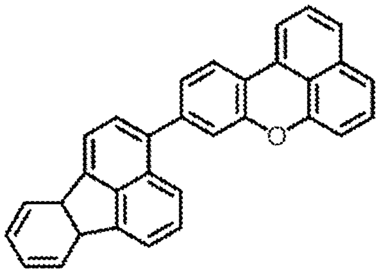
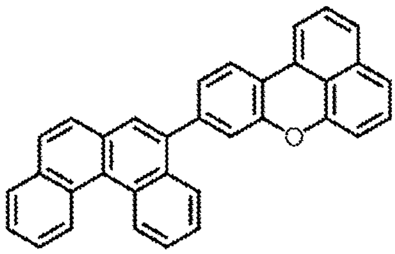

[Formula 66]

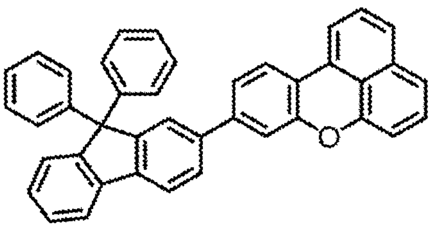
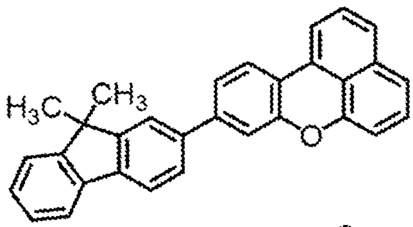

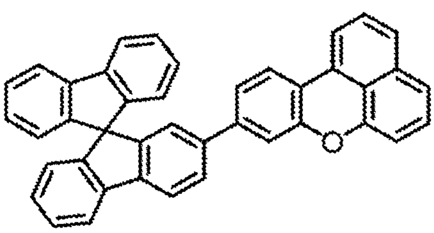
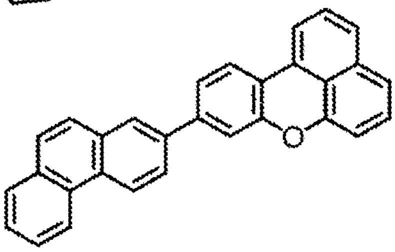

[Formula 67]

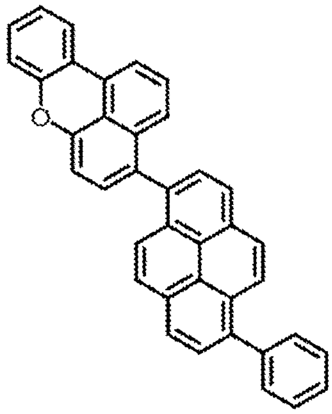

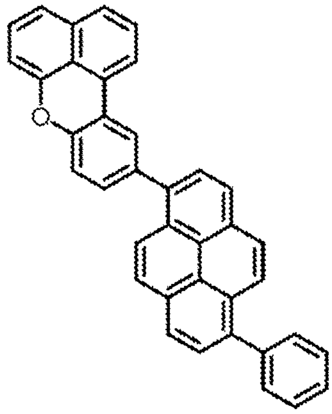

[Formula 68]

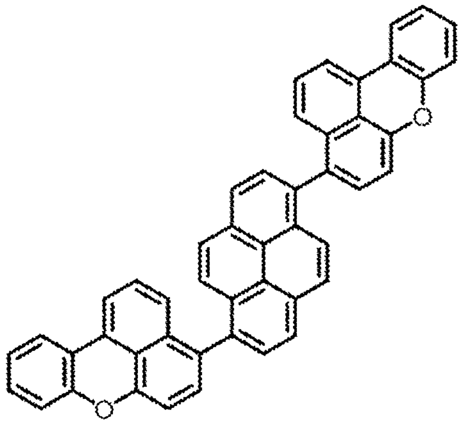

[Formula 69]

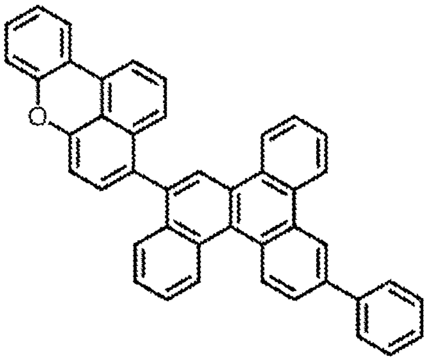

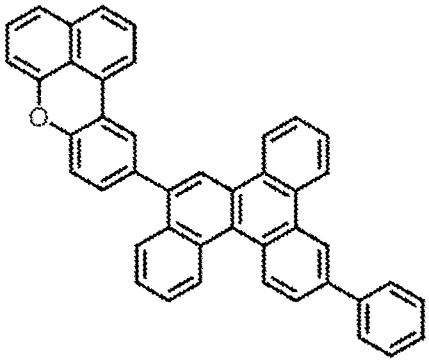

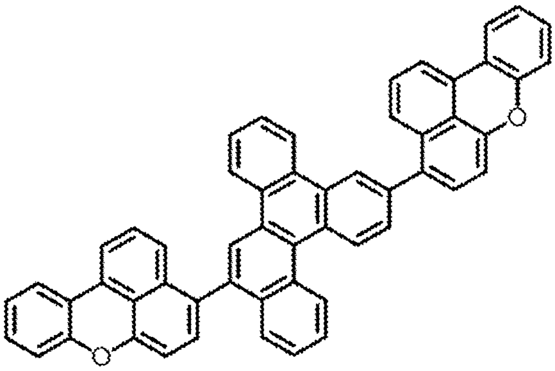

[Formula 70]

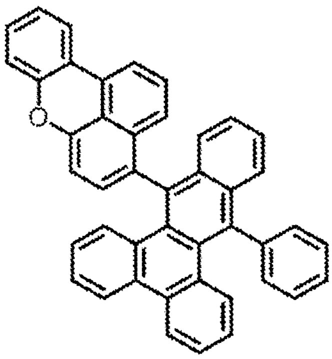

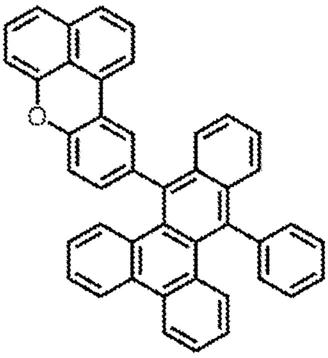

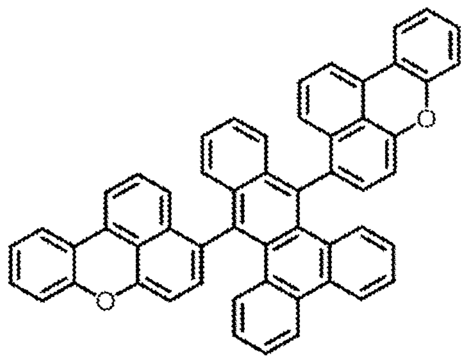

[Formula 71]

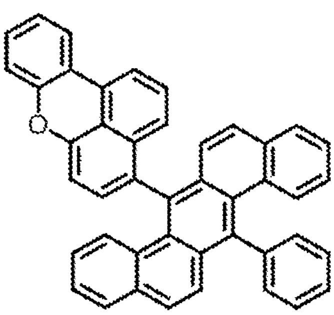

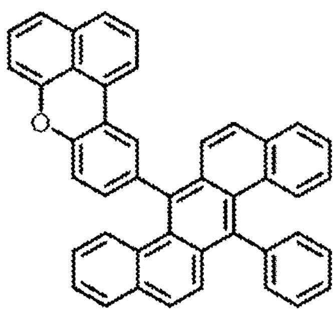

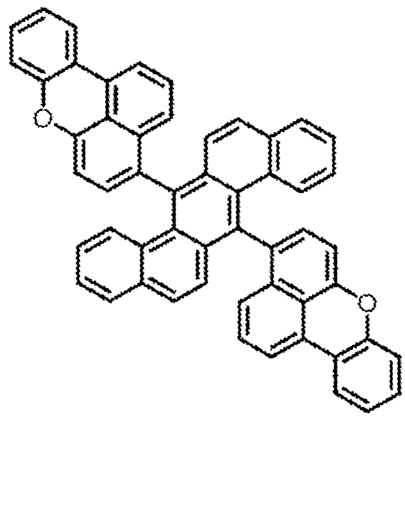

[Formula 72]

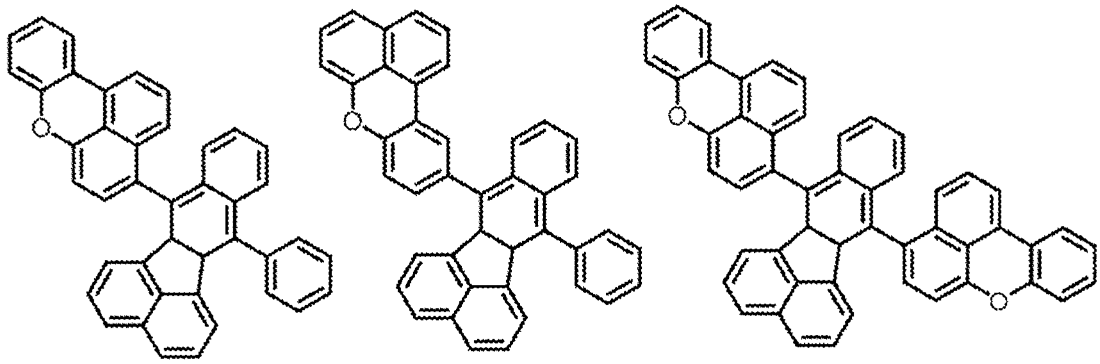

[Formula 73]

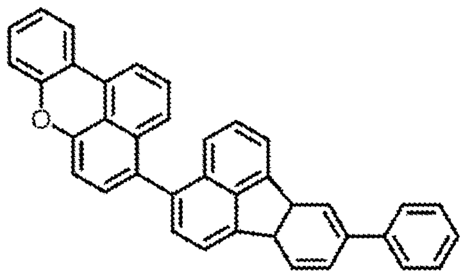

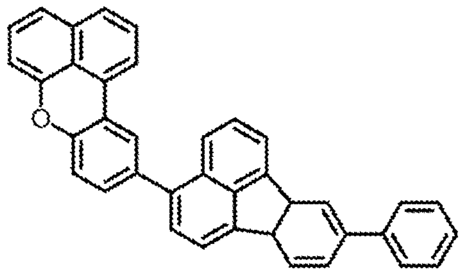

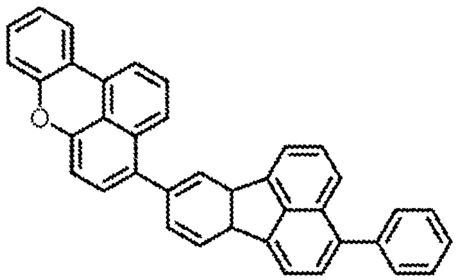

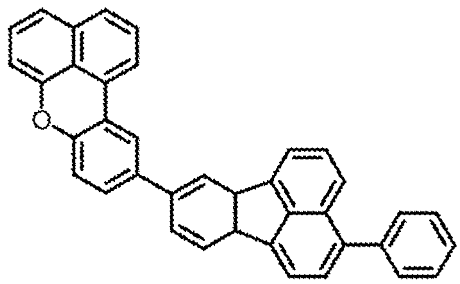

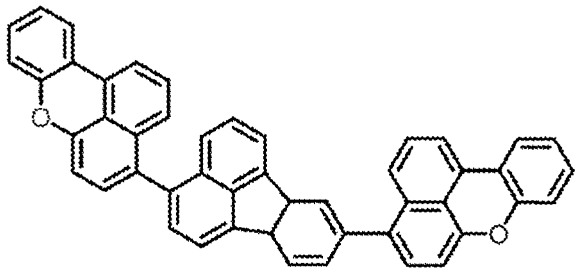

[Formula 74]

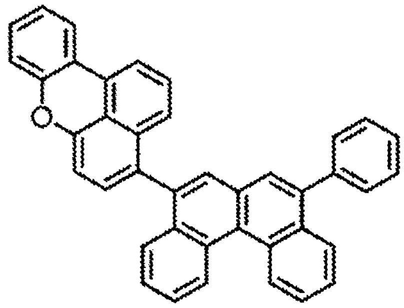

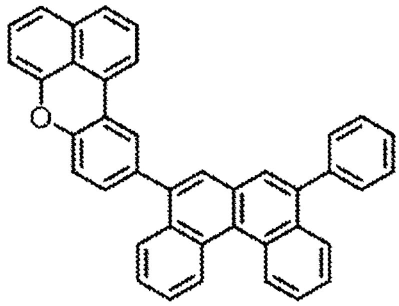

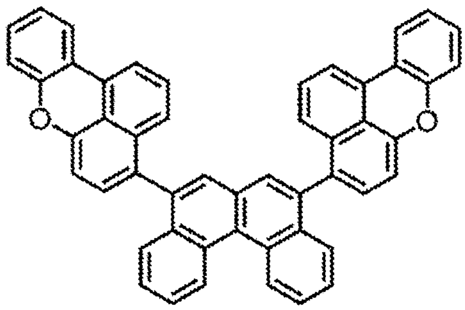

[Formula 75]

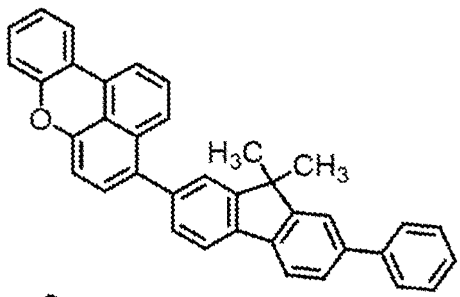

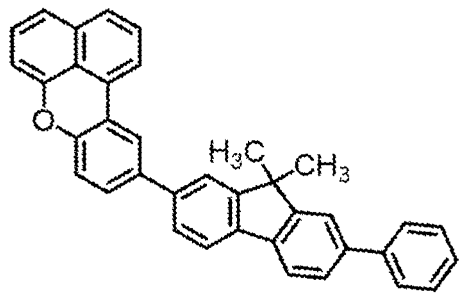

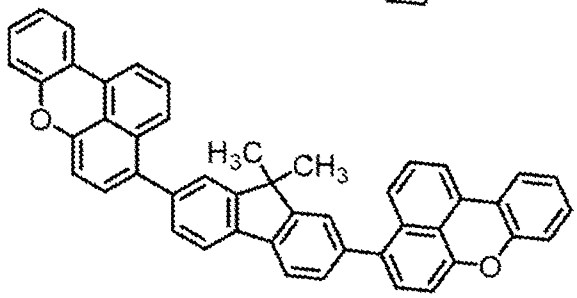

[Formula 76]

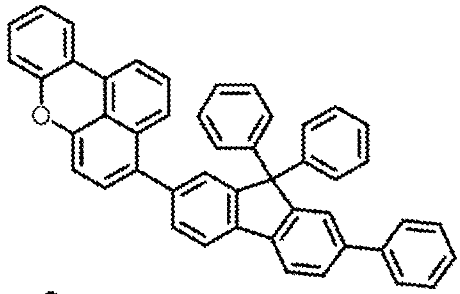

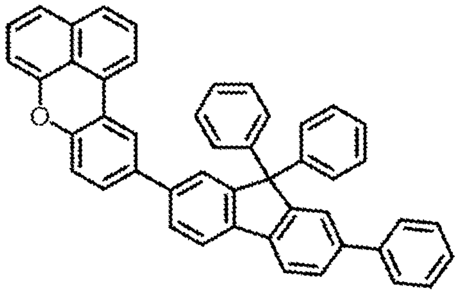

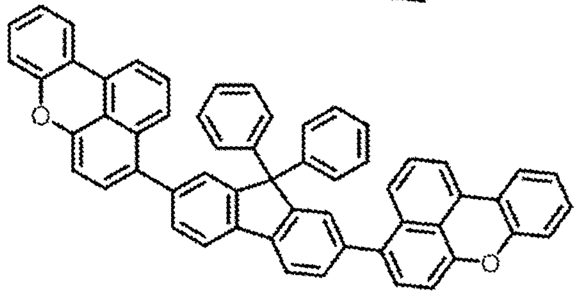

[Formula 77]

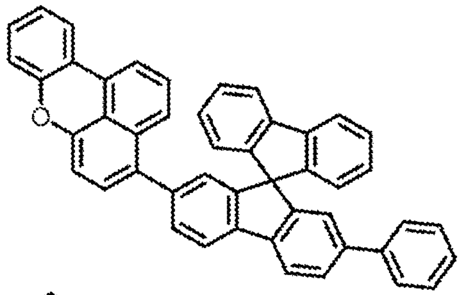

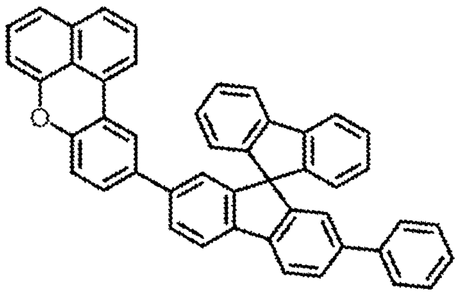

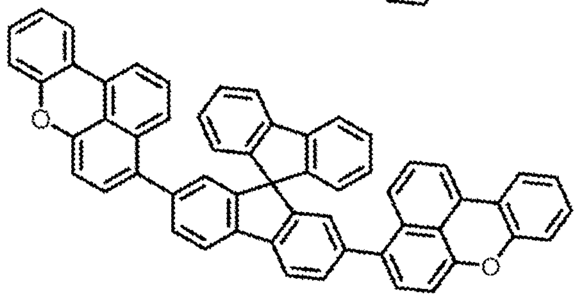

[Formula 78]

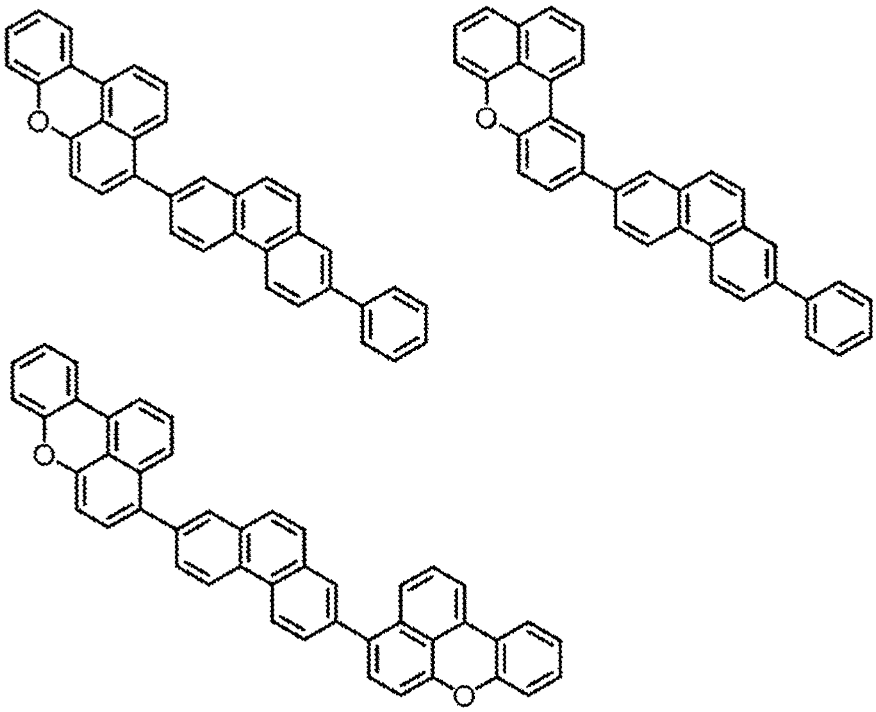

[Formula 79]

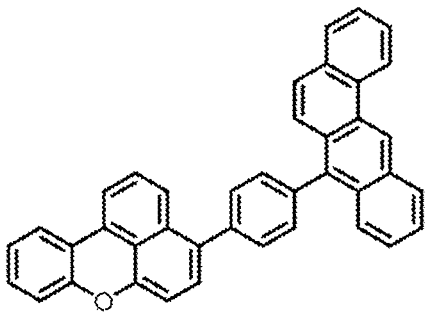

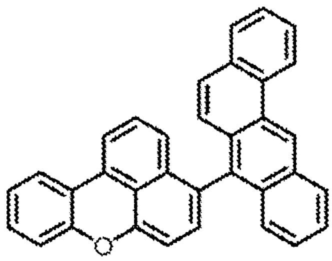

[Formula 80]

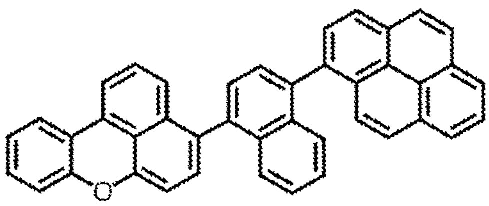

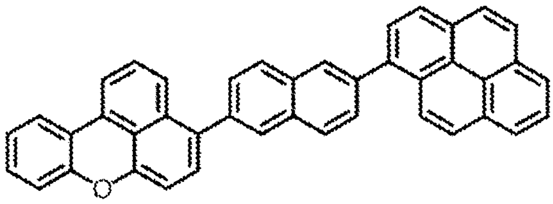

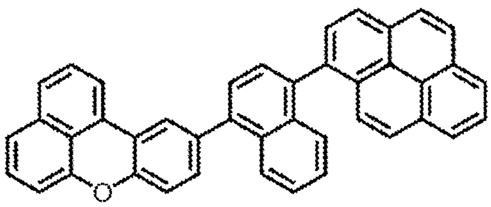

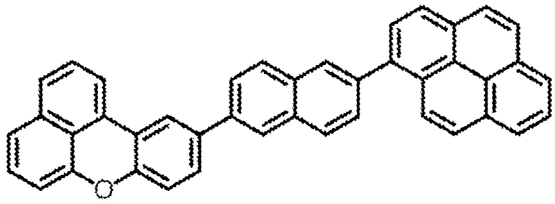

[Formula 81]

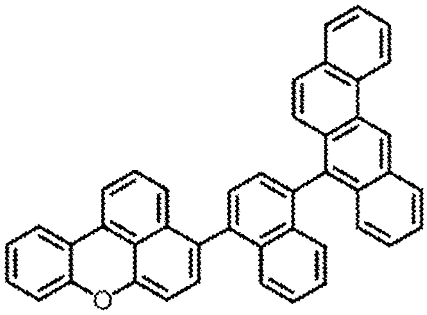
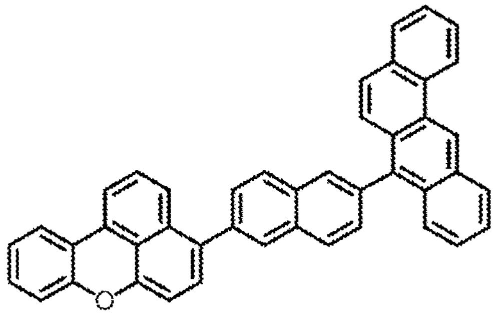
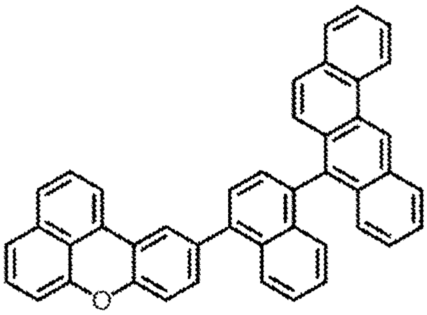
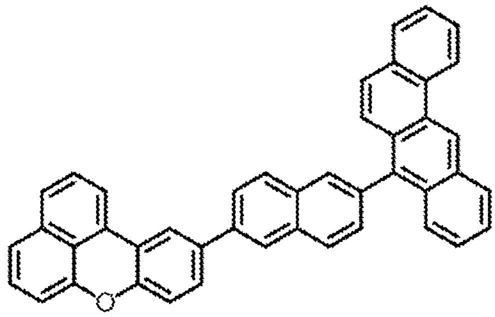

[Formula 82]

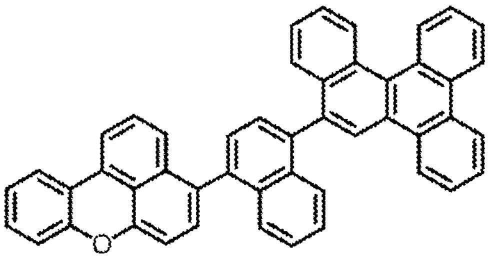
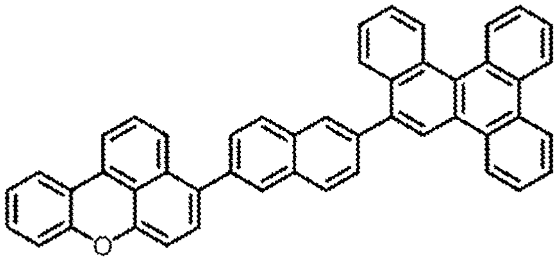
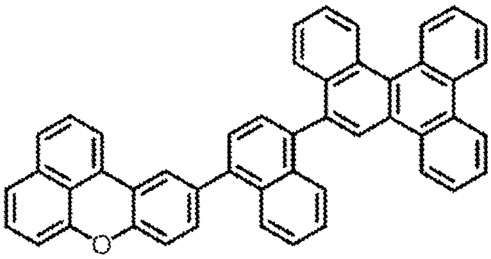
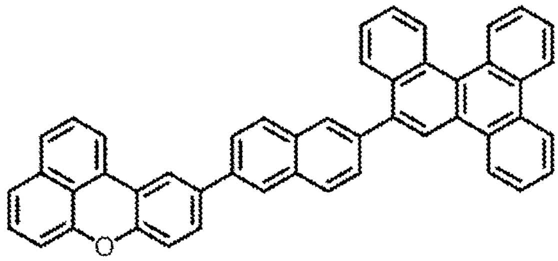

[Formula 83]

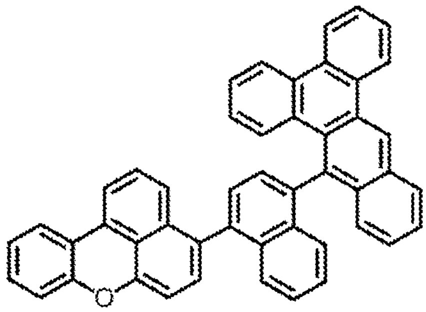

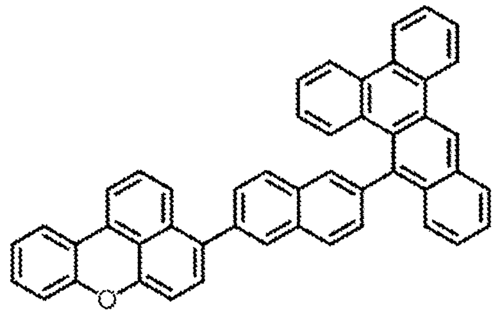

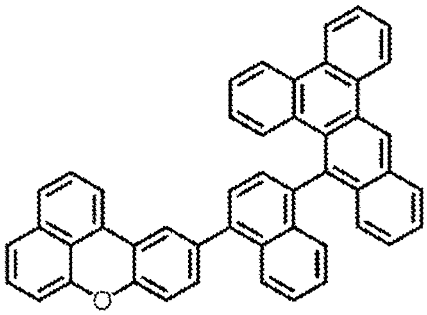

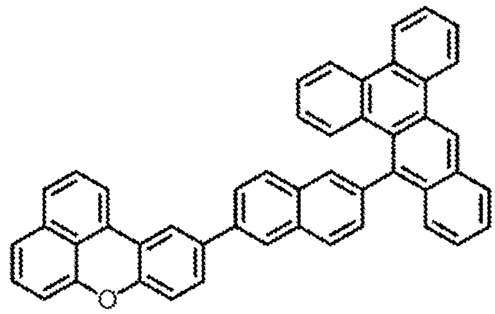

[Formula 84]

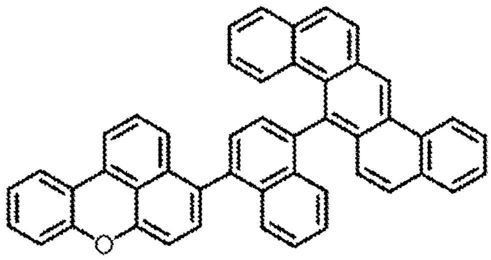

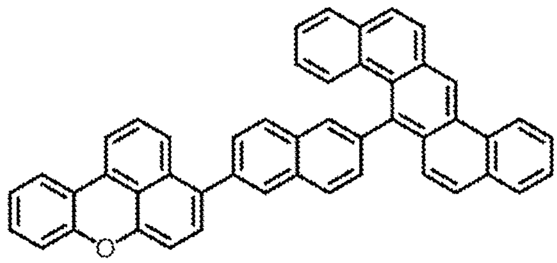

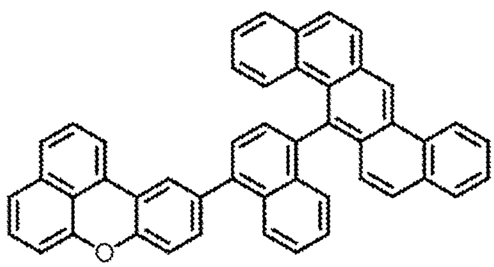

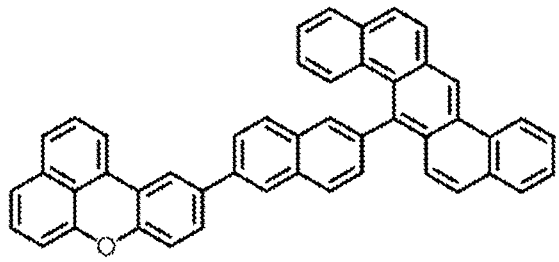

[Formula 85]

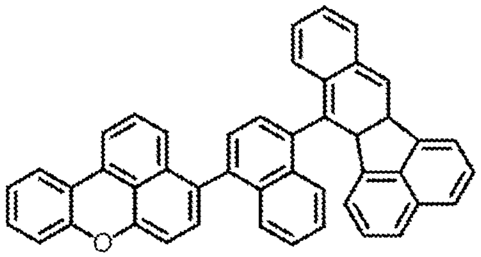

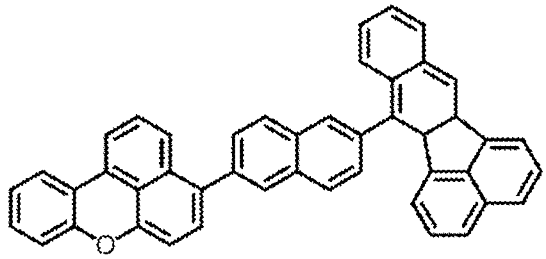

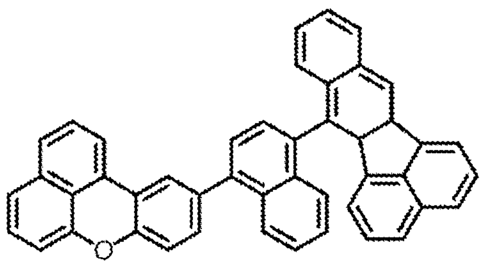

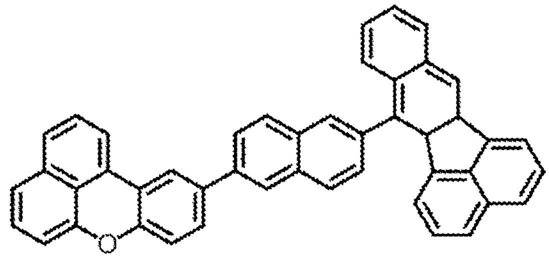

[Formula 86]

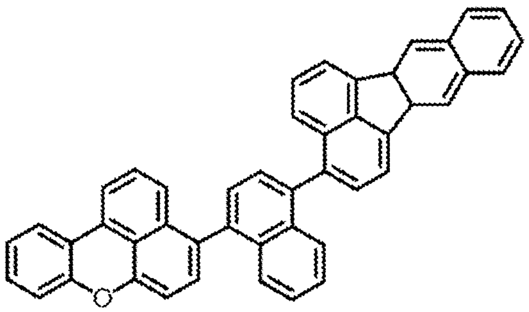

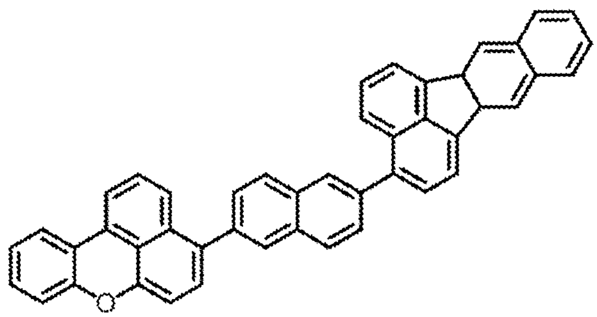

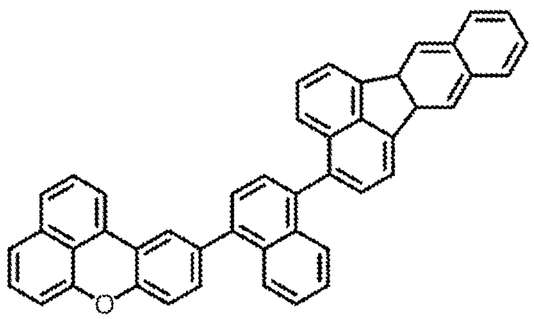

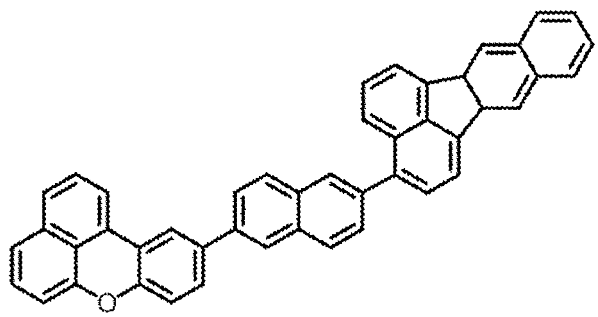

[Formula 87]

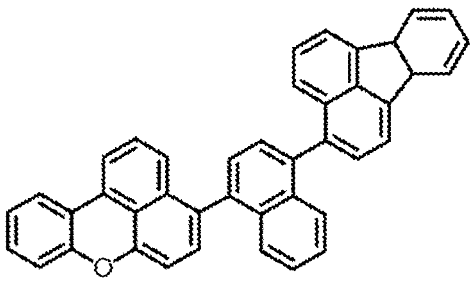

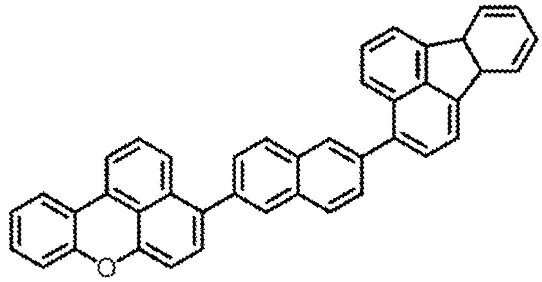

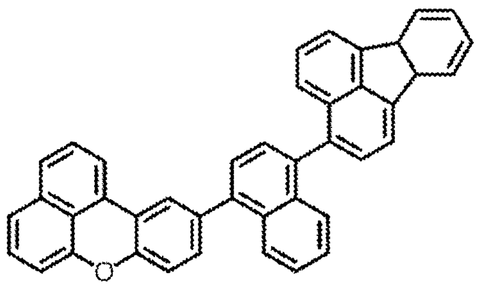

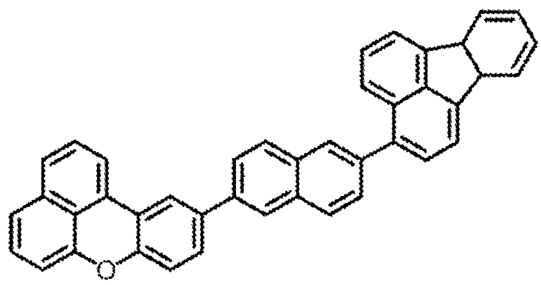

[Formula 88]

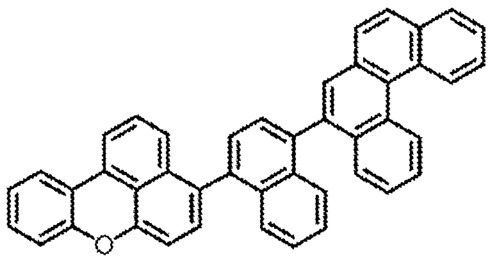

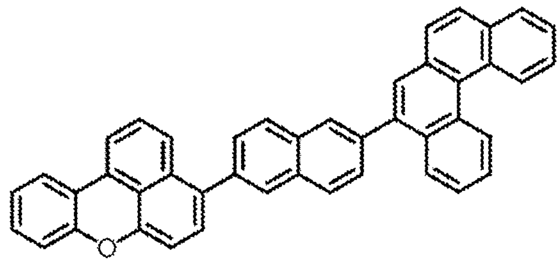

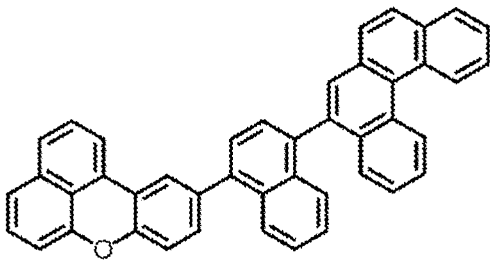

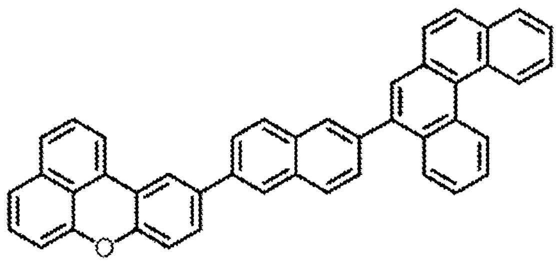

[Formula 89]

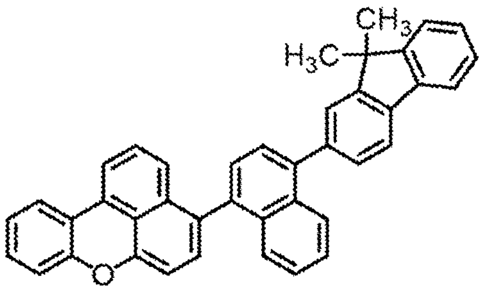

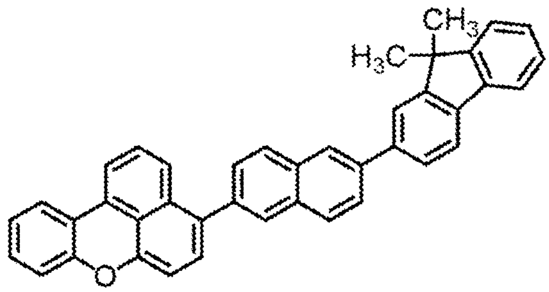

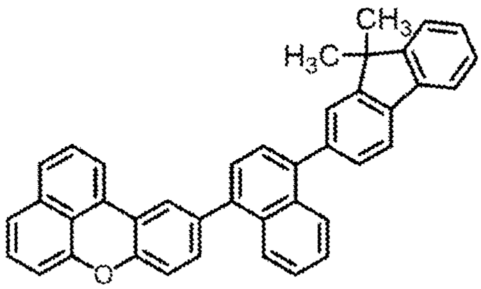

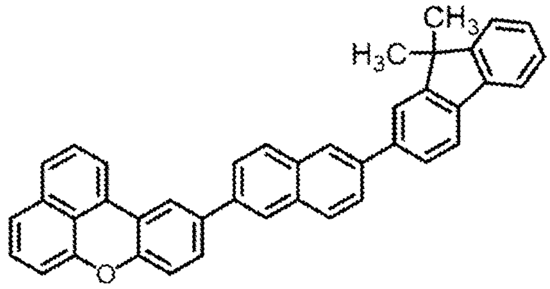

[Formula 90]

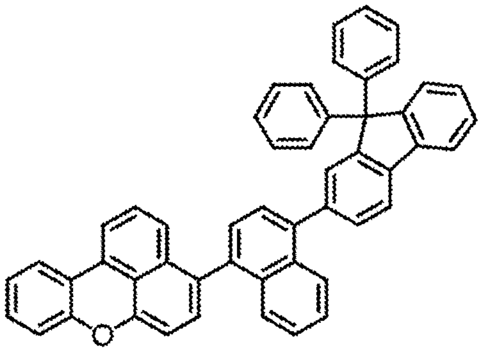

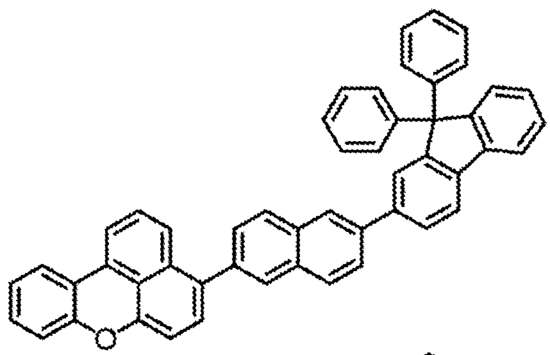

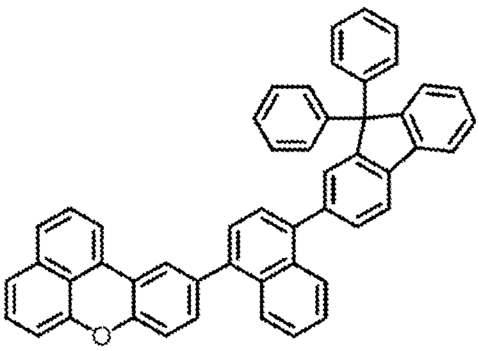

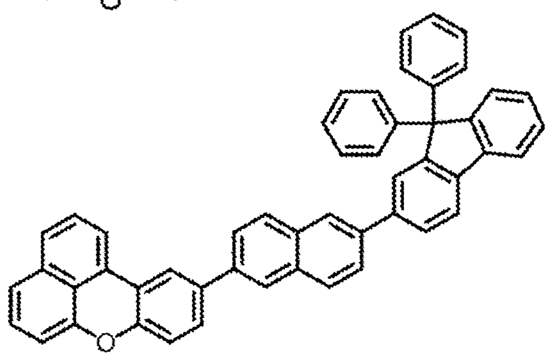

[Formula 91]

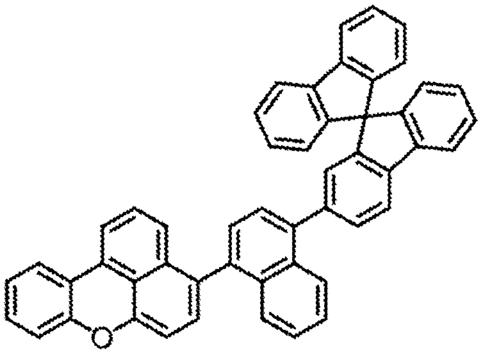

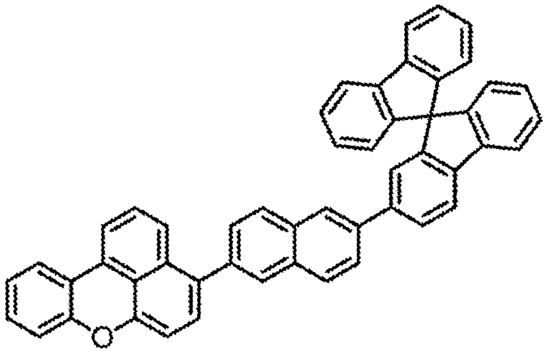

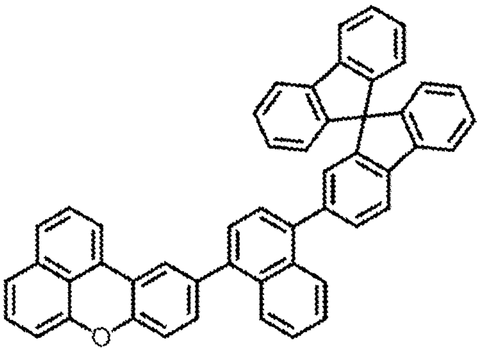

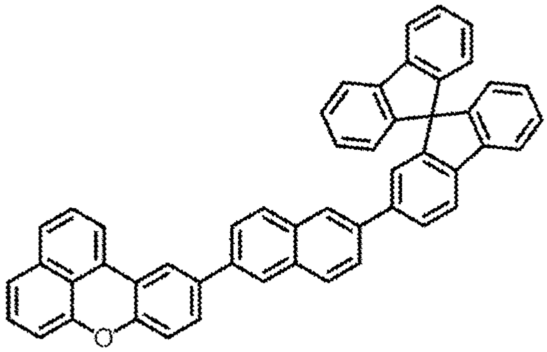

[Formula 92]

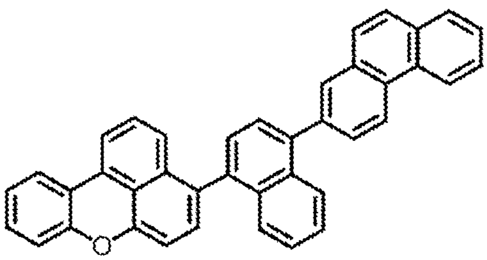

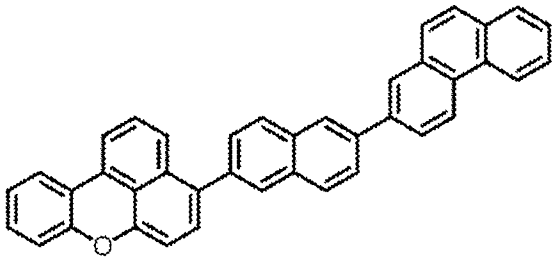

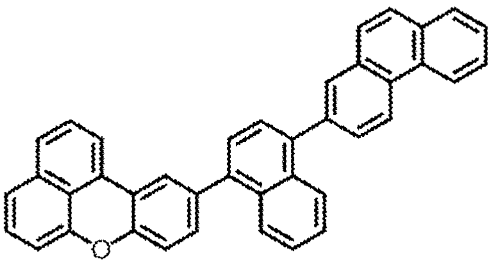

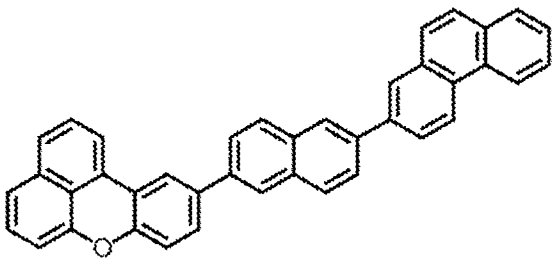

[Formula 93]

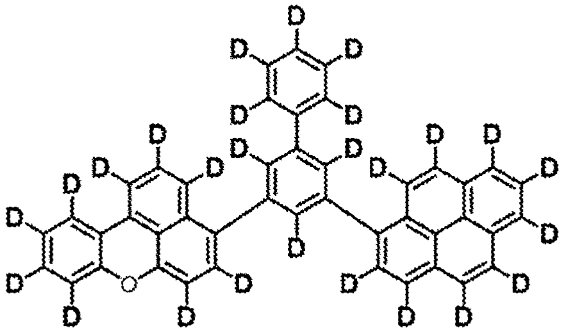

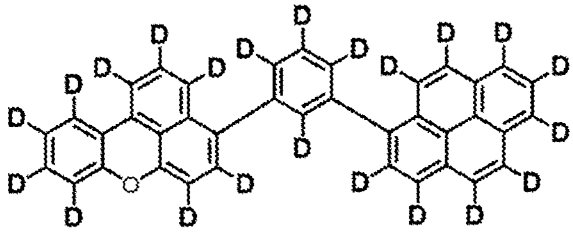

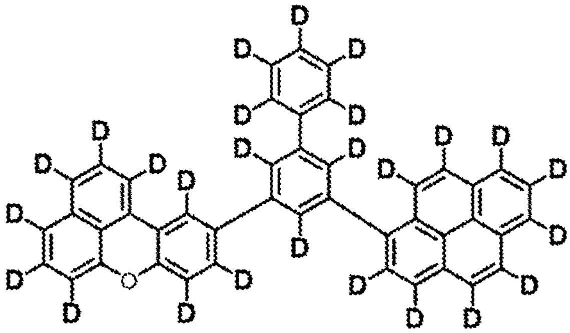

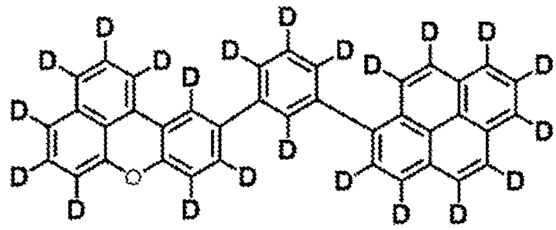

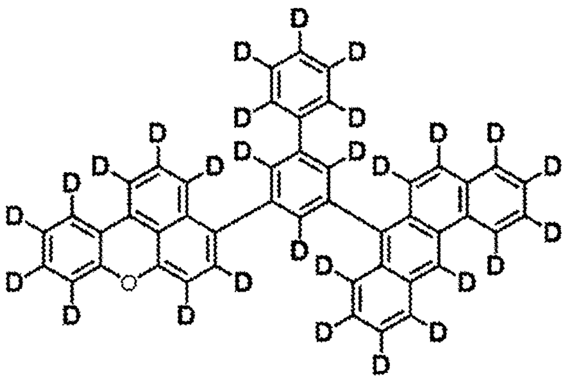

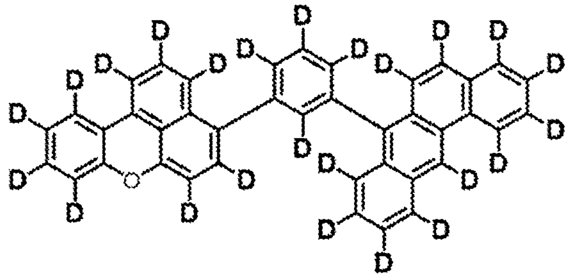

[Formula 94]

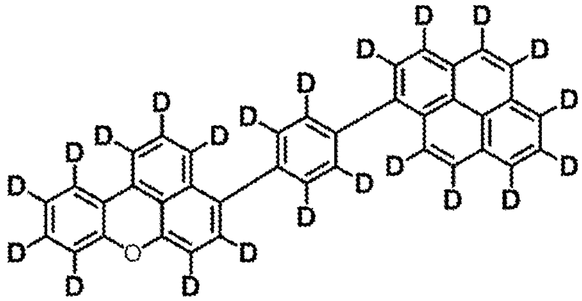

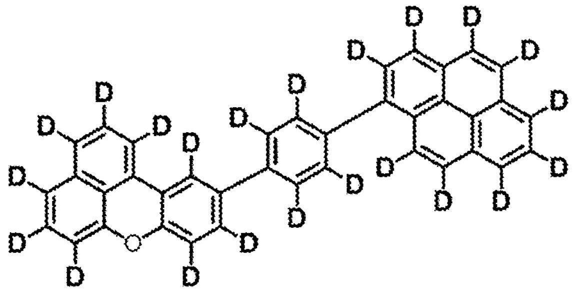

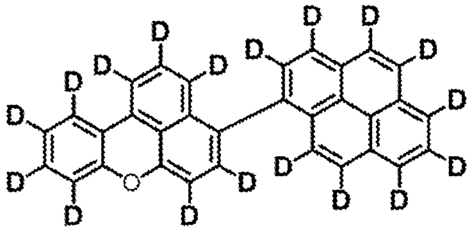

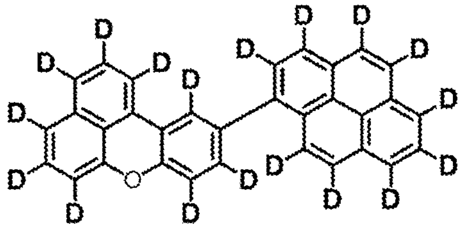

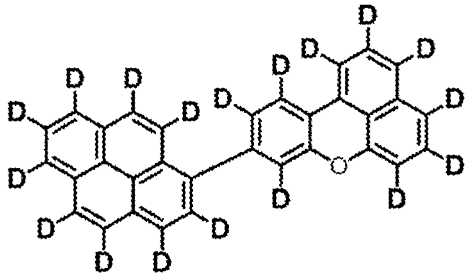

[Formula 95]

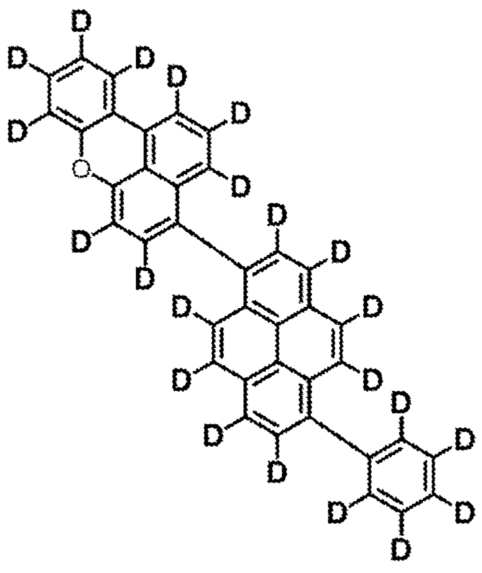

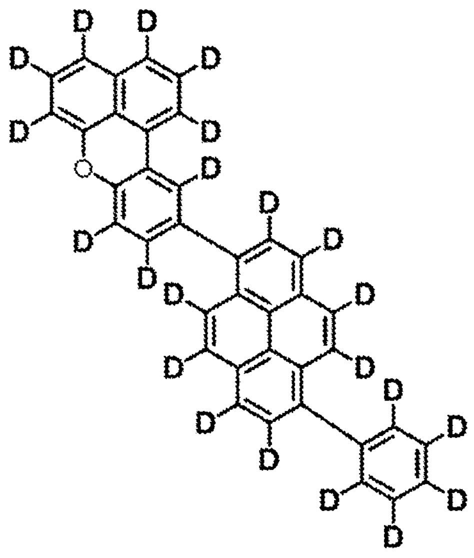

[Formula 96]

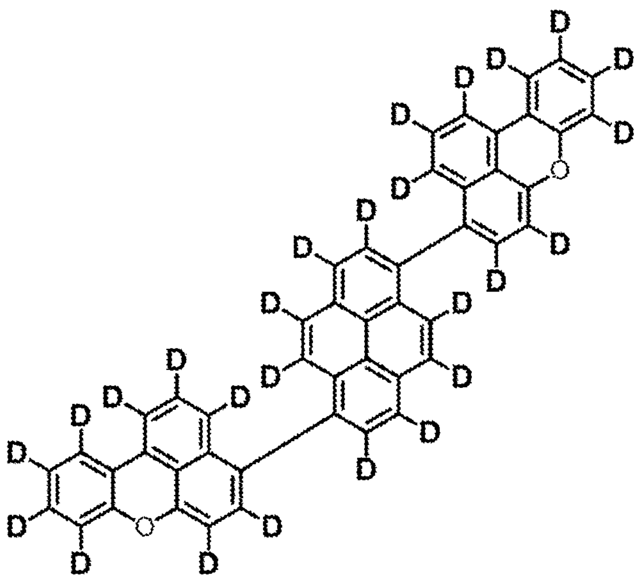

[Formula 97]

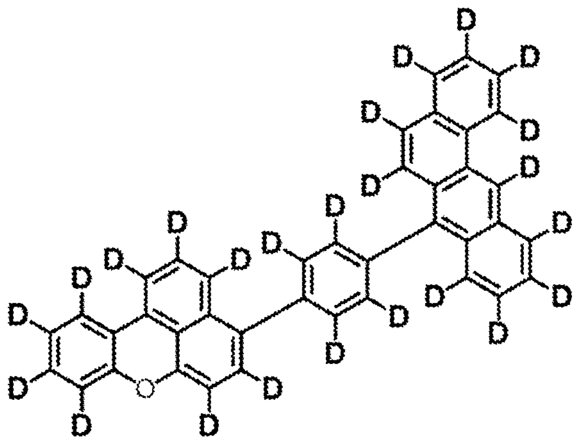

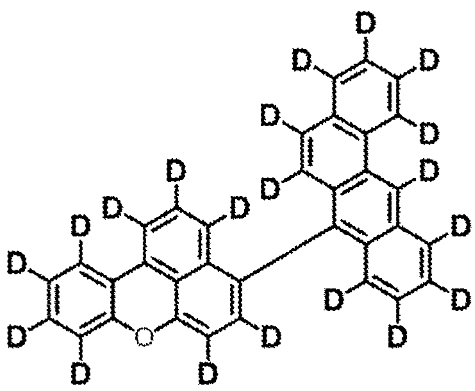

[Formula 98]

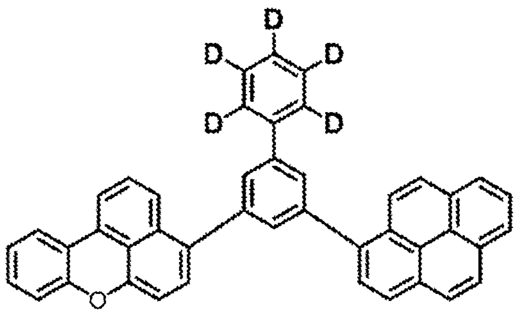

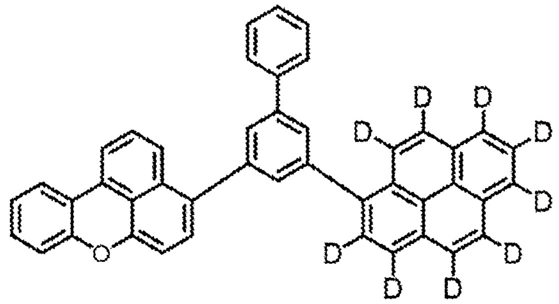

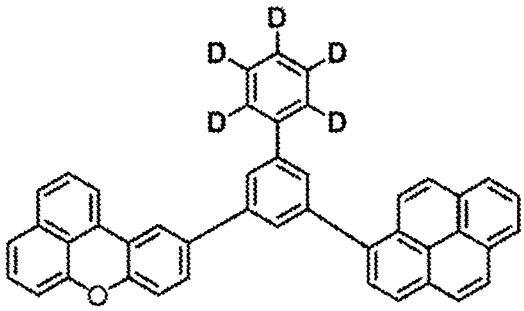

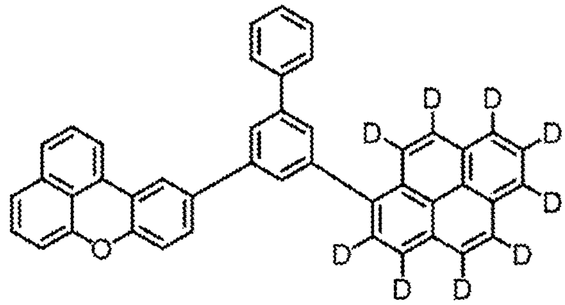

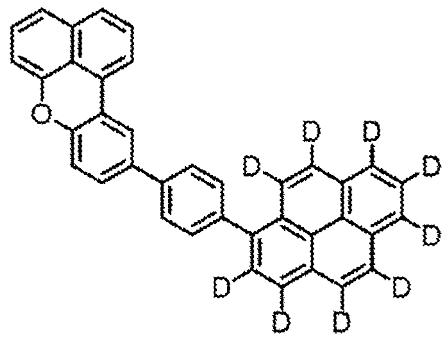

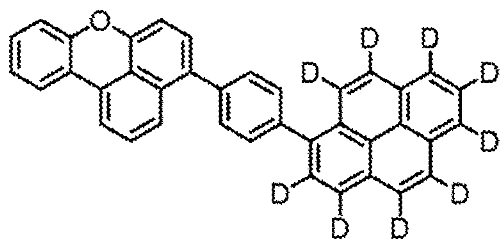

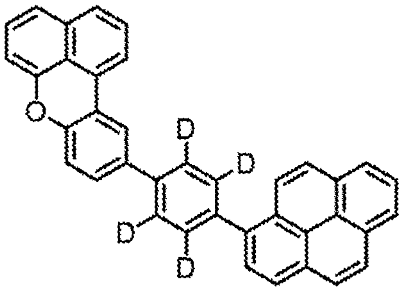

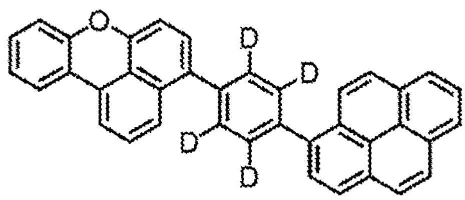

[Formula 99]

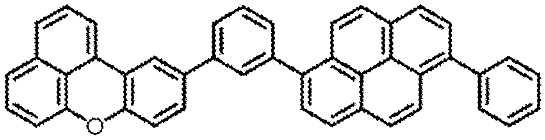
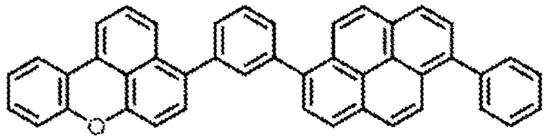

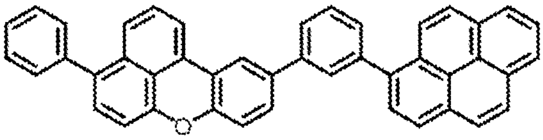
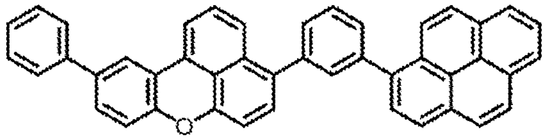

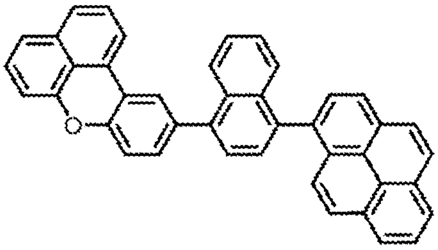
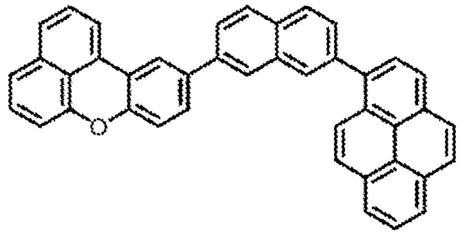

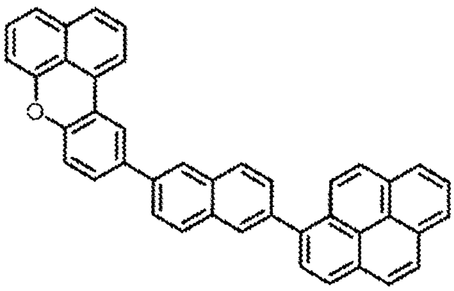
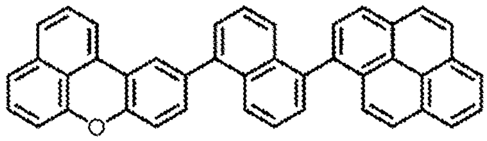

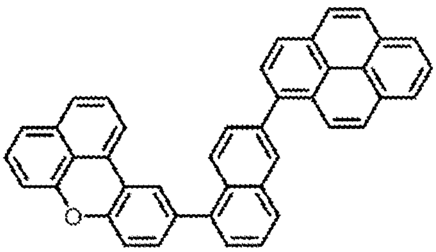

[Formula 100]

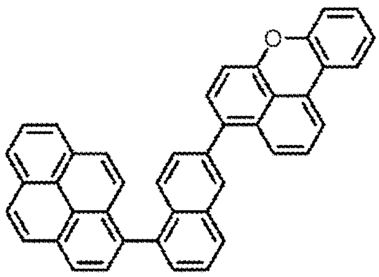

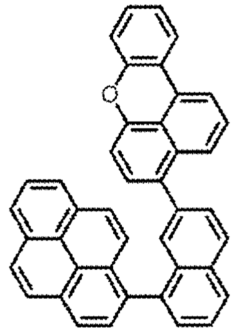

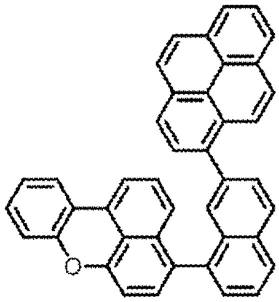

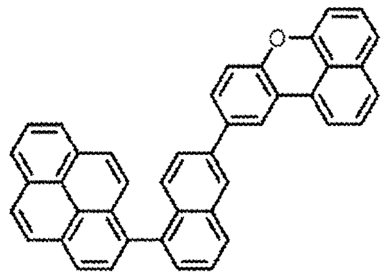

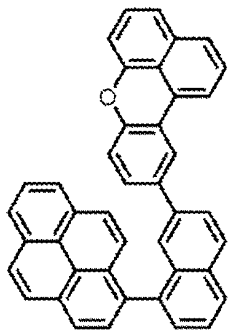

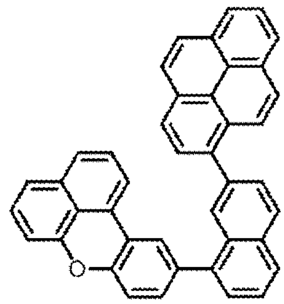

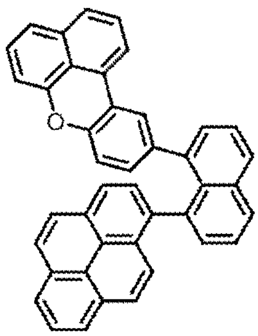

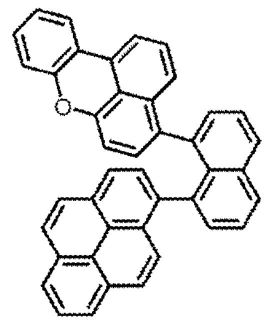

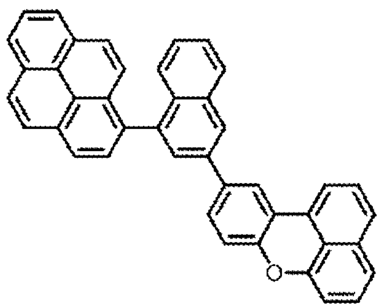

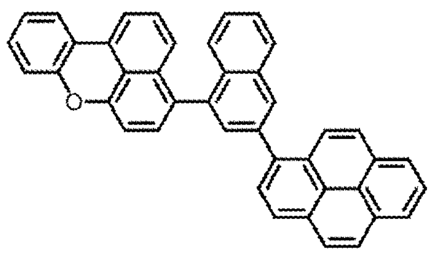

[Formula 101]

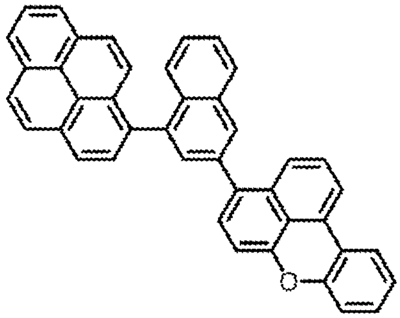

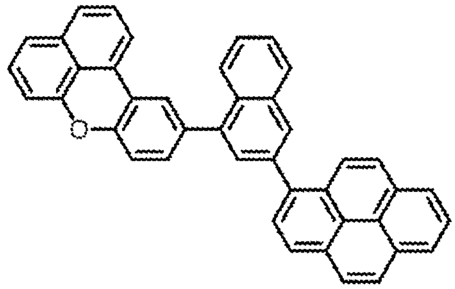

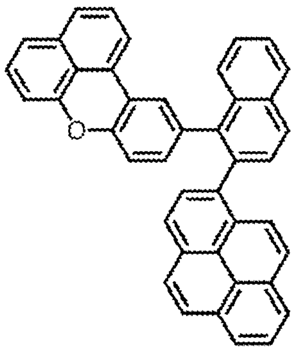

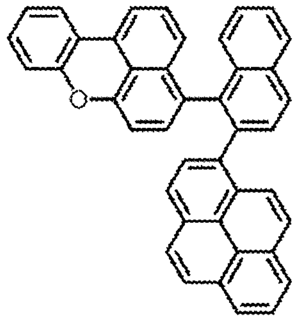

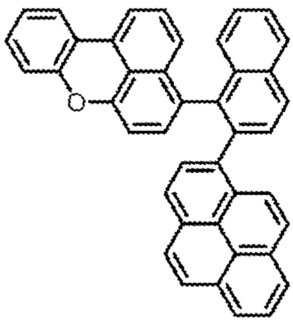

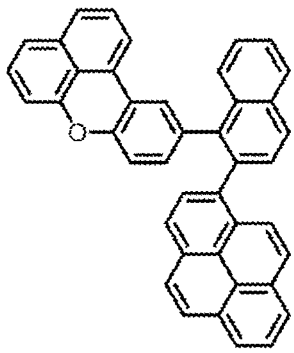

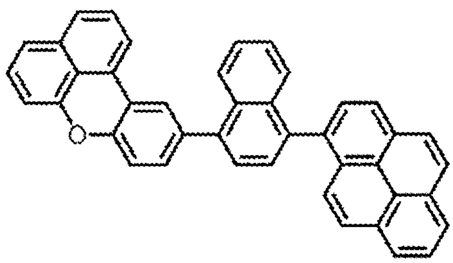

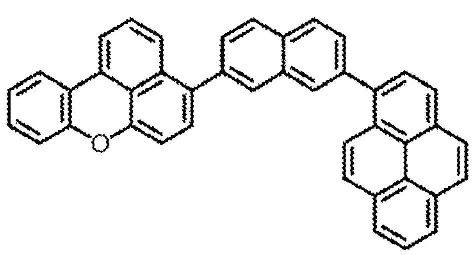

[Formula 102]

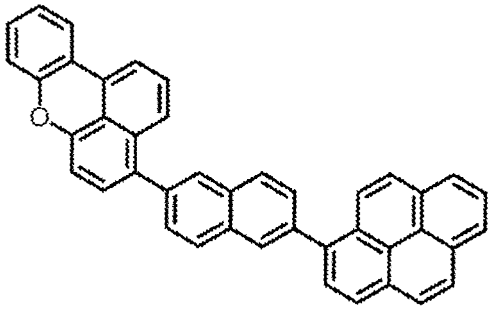

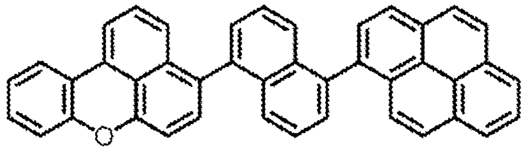

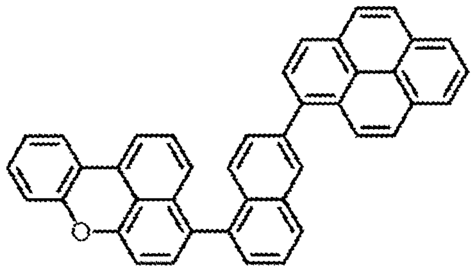

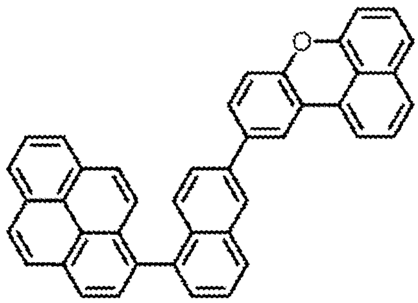

[Formula 103]

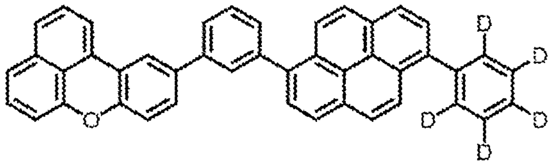

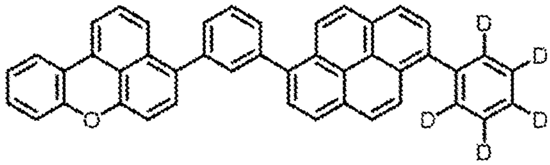

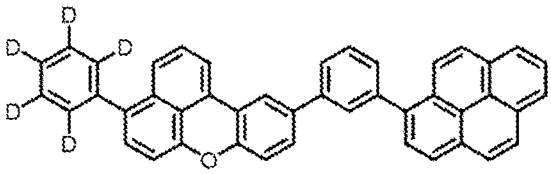

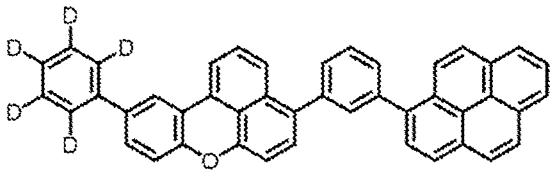

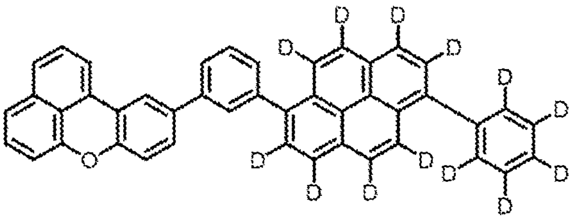

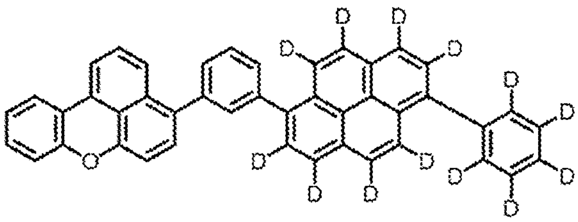

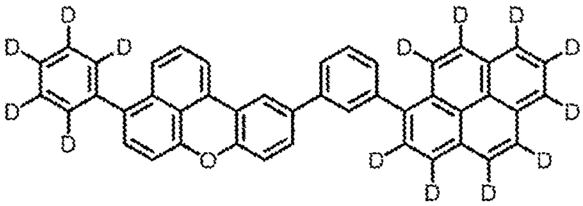

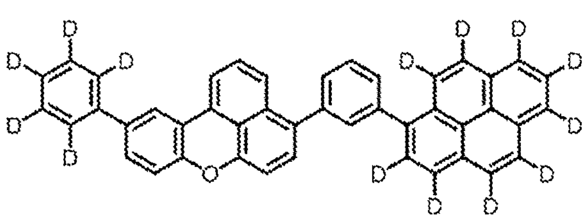

[Formula 104]

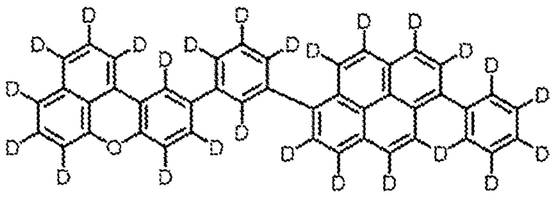
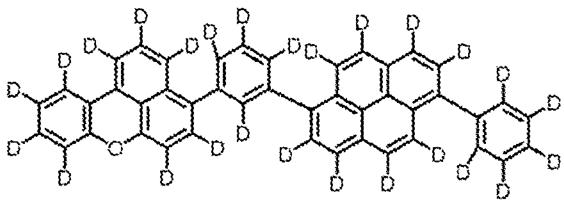
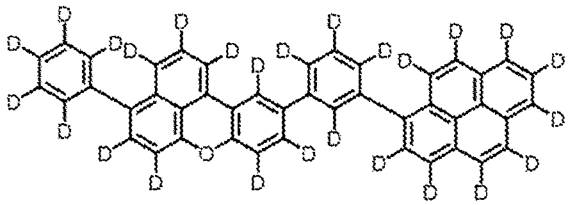
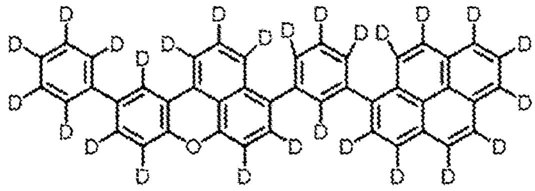
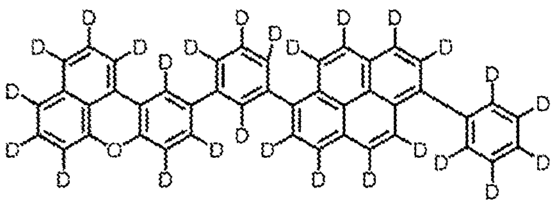
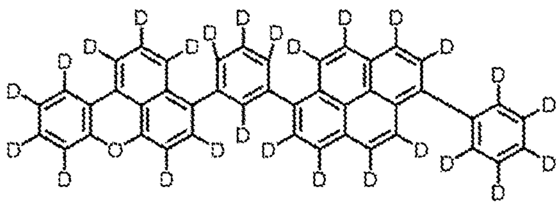
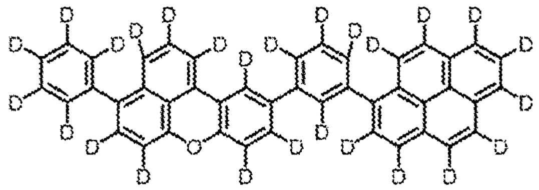
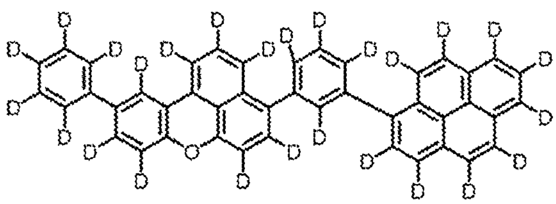

[Formula 105]

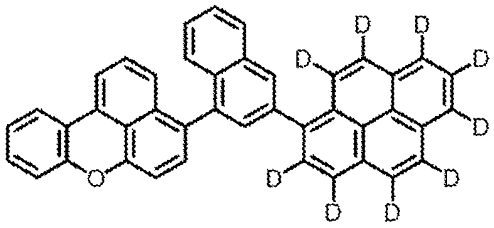

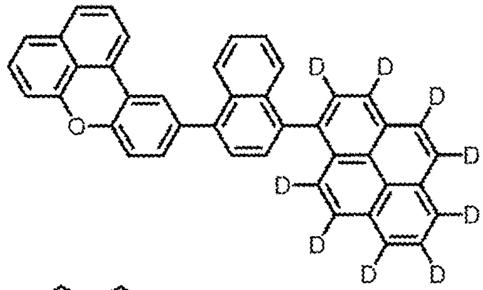

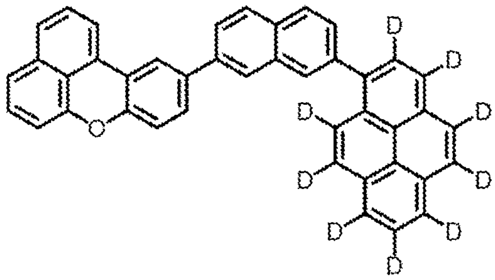

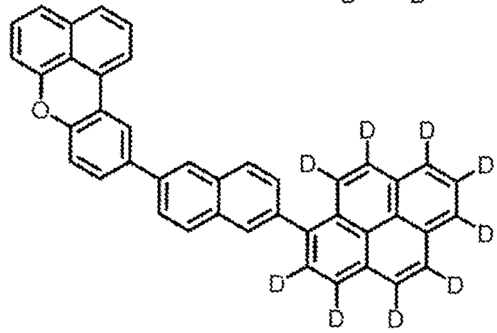

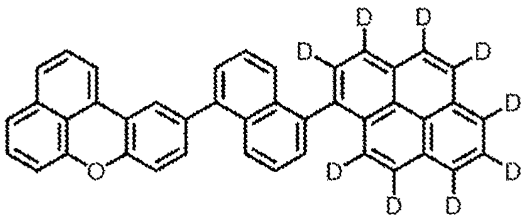

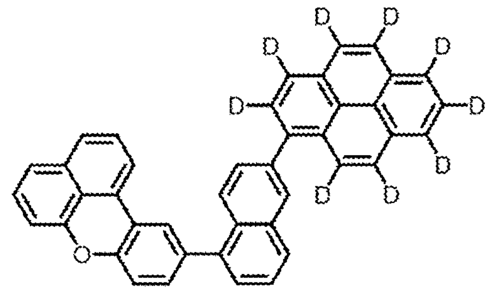

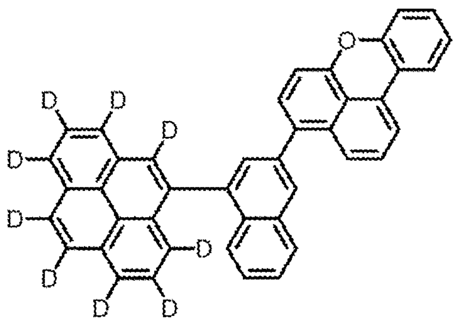

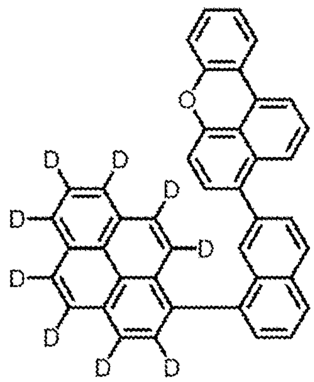

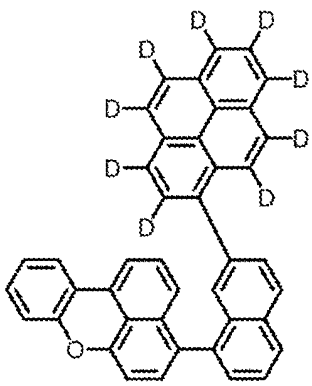

[Formula 106]

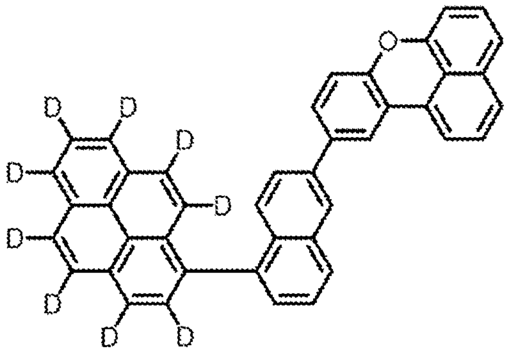

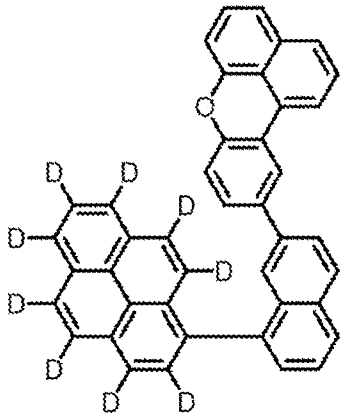

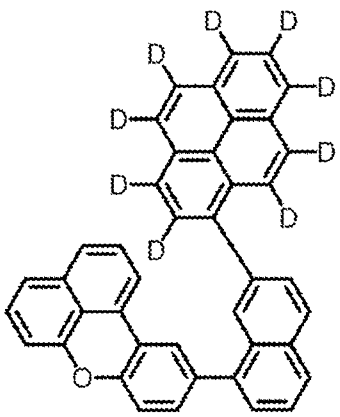

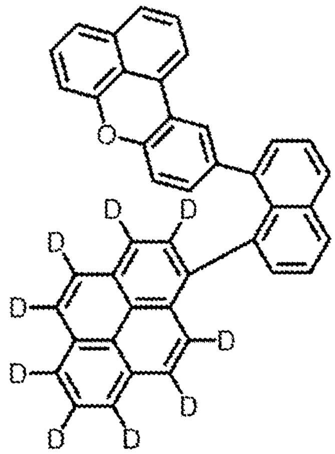

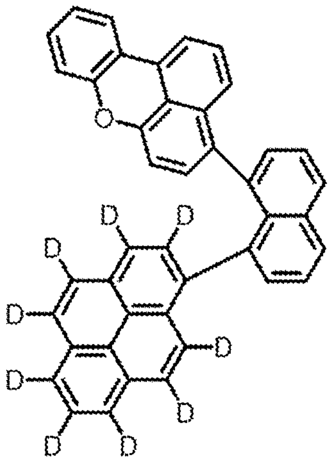

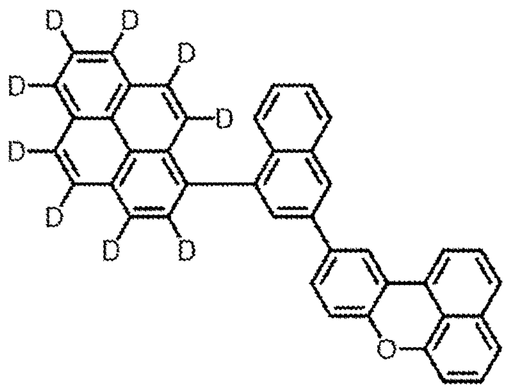

[Formula 107]

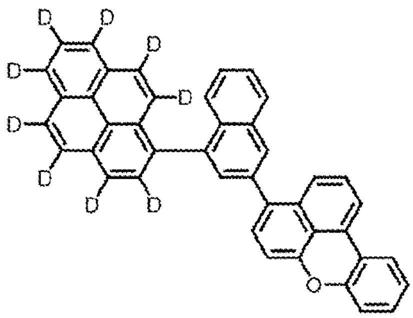

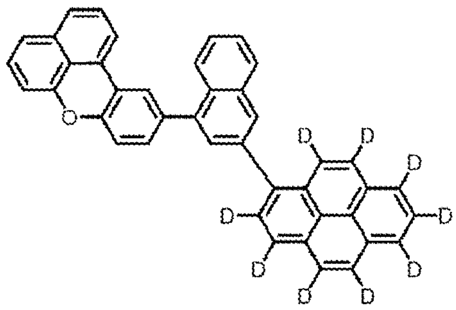

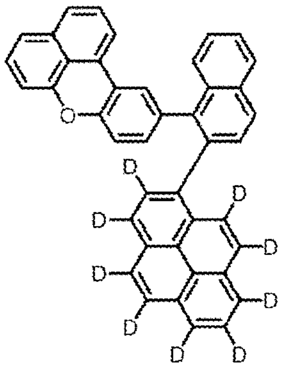

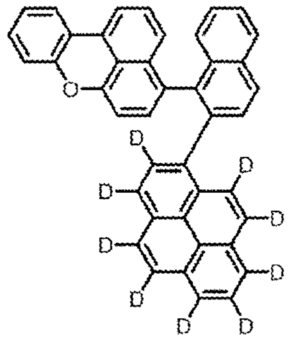

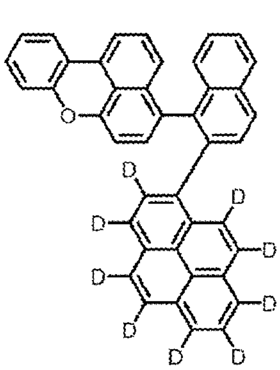

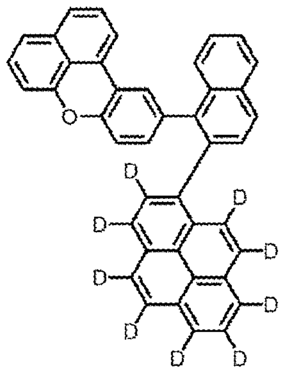

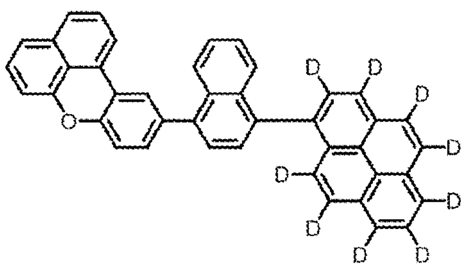

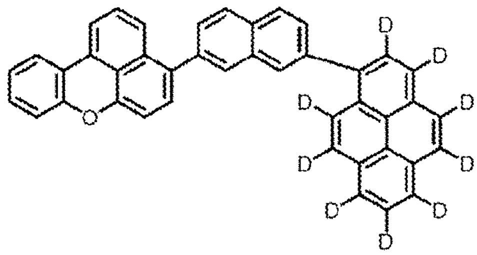

[Formula 108]

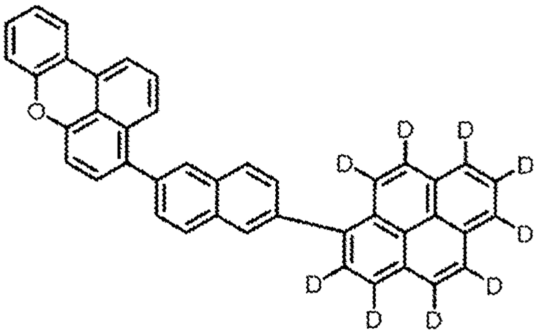

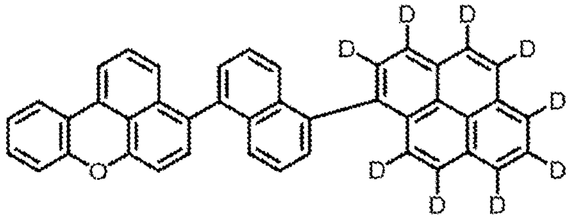

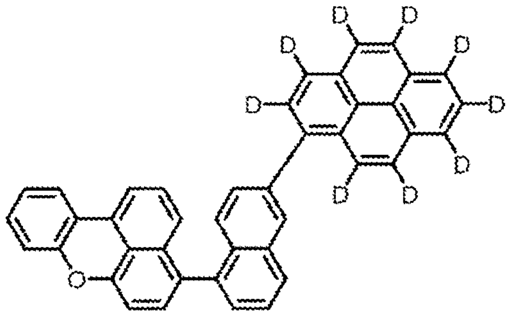

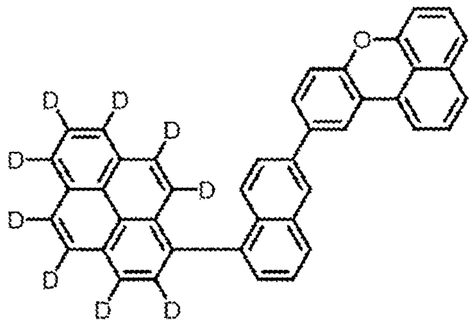

[Formula 109]

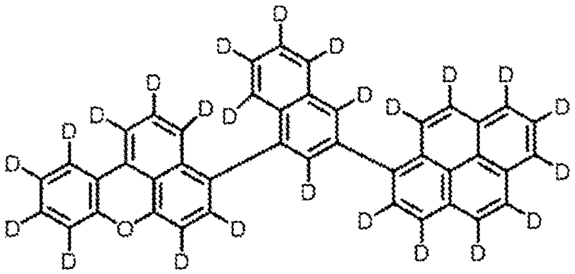

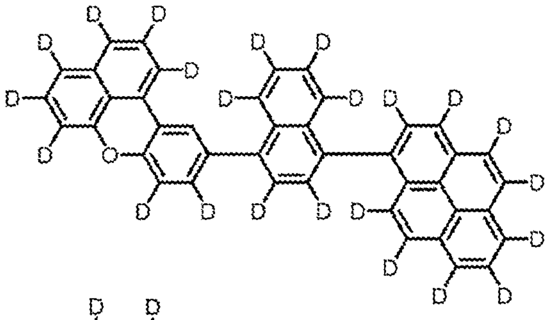

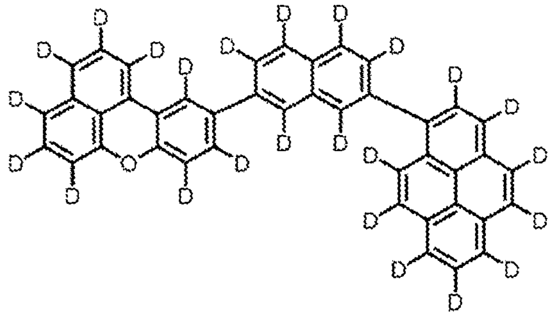

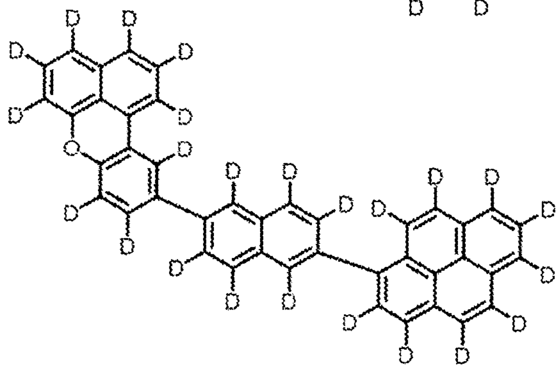

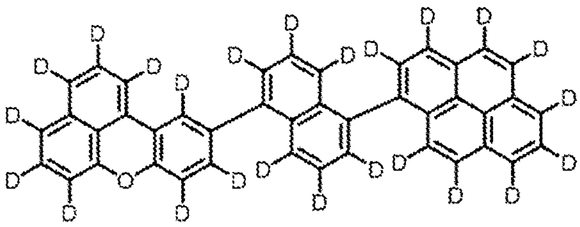

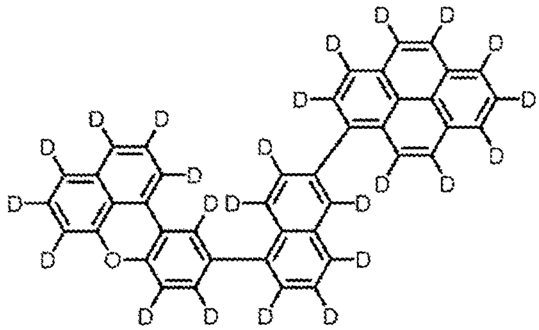

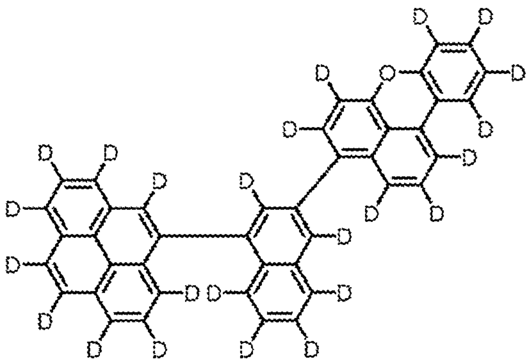

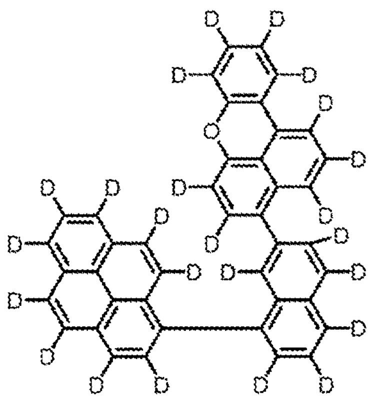

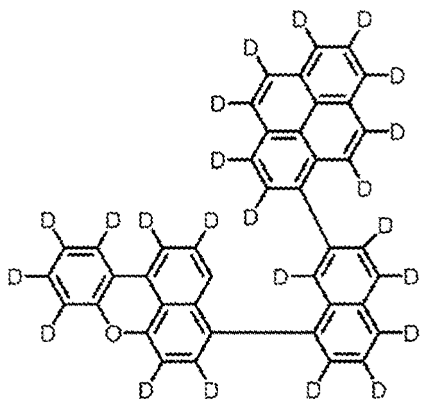

[Formula 110]

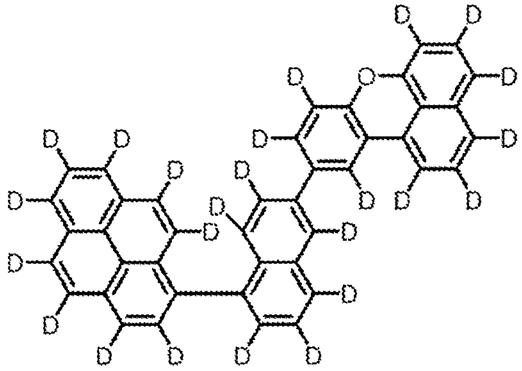

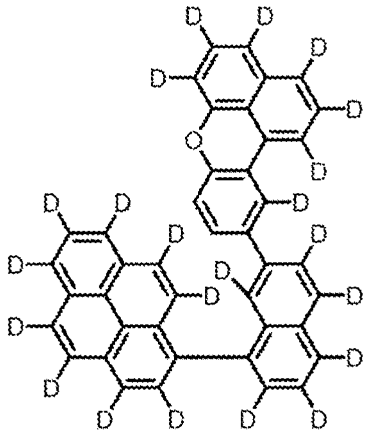

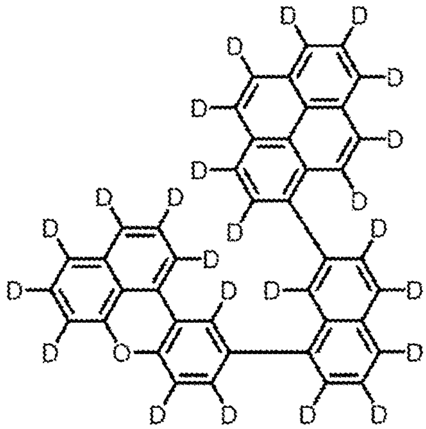

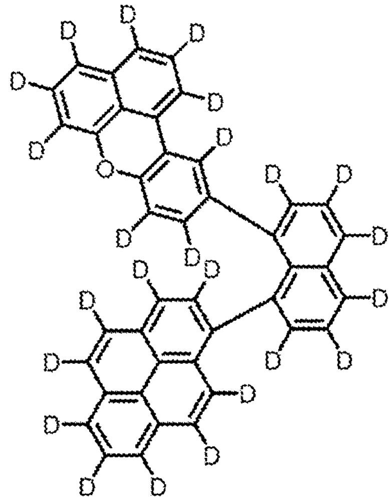

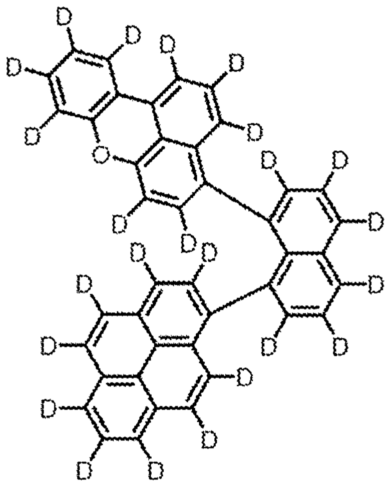

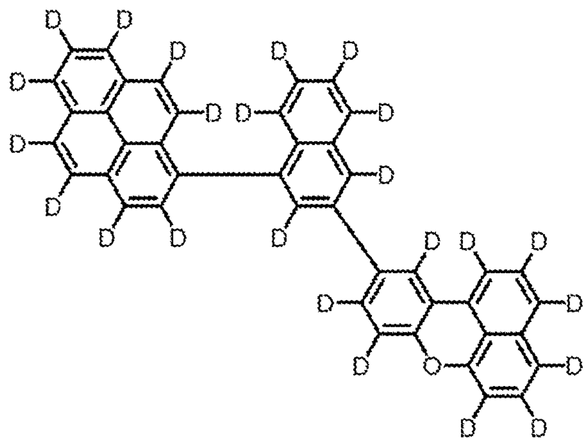

[Formula 111]

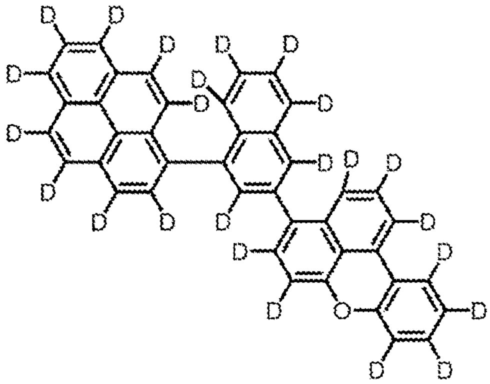

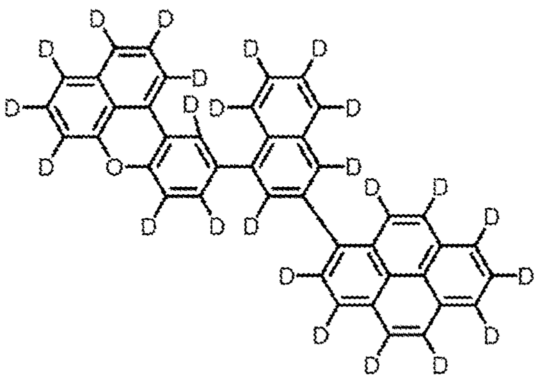

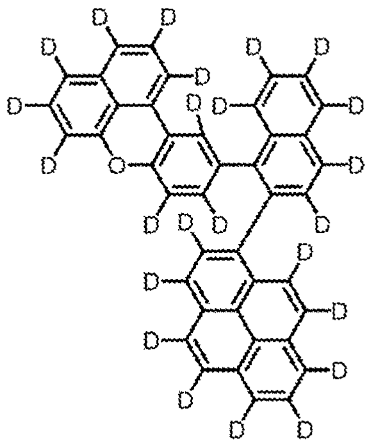

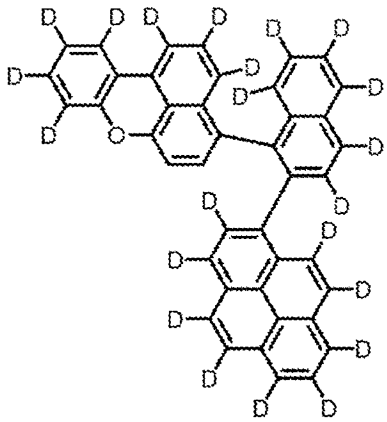

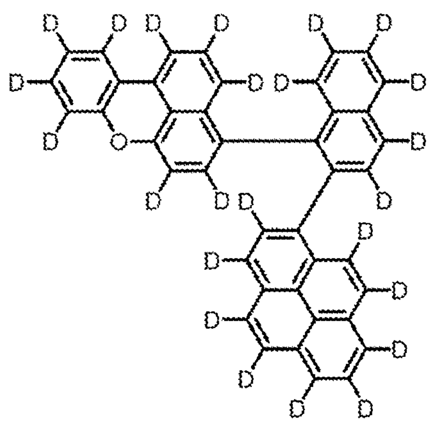

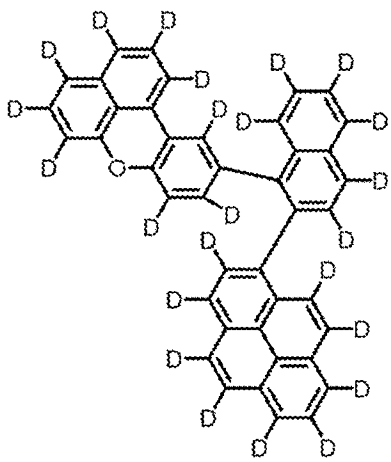

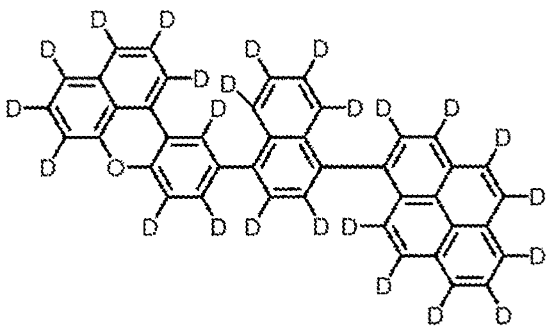

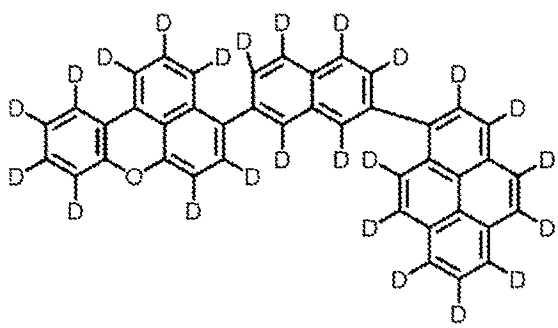

[Formula 112]

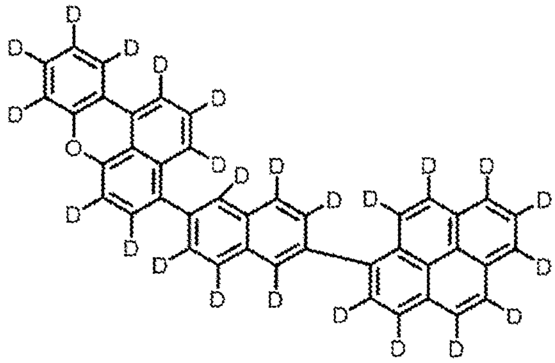

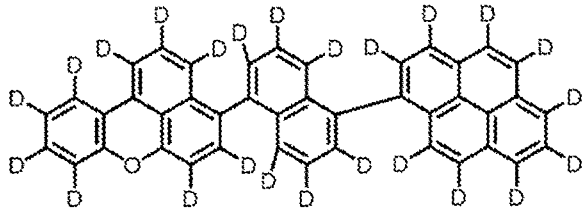

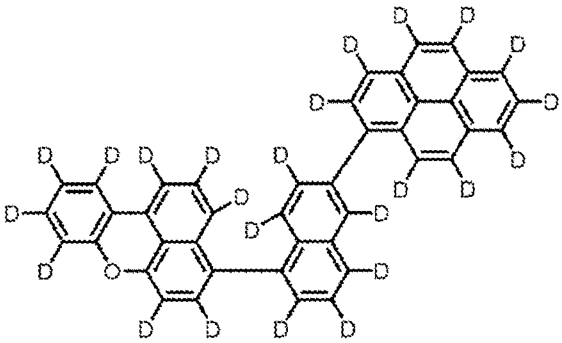

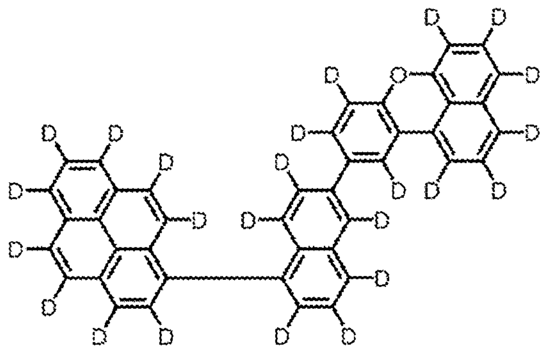

[Formula 113]

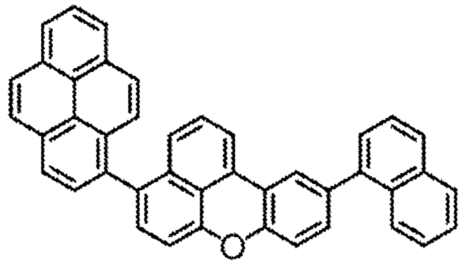

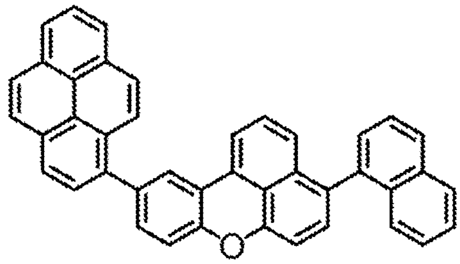

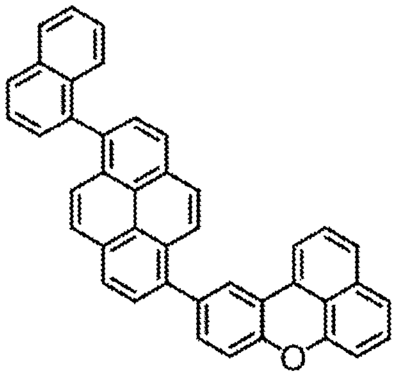

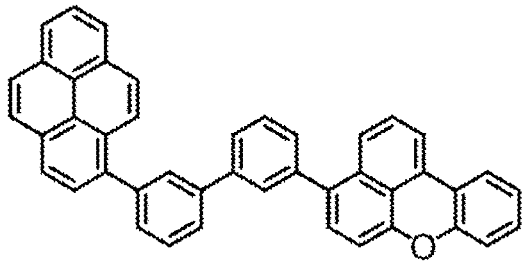

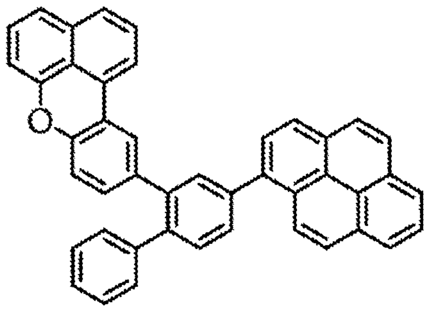

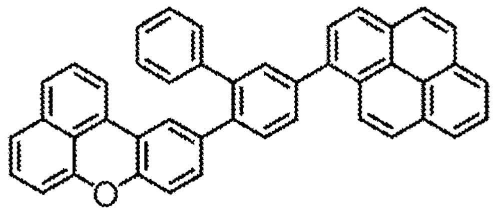

[Formula 114]

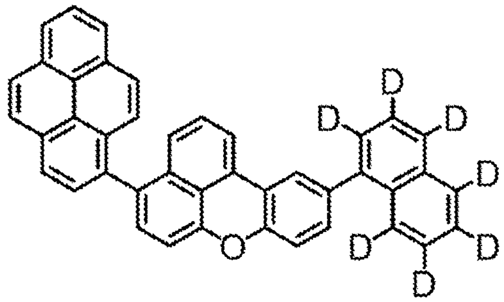

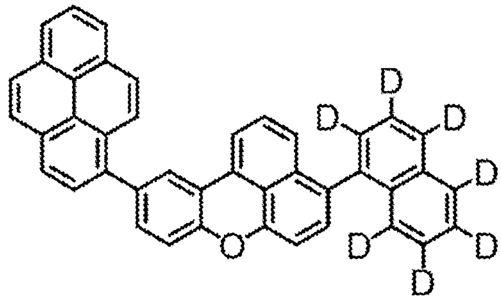

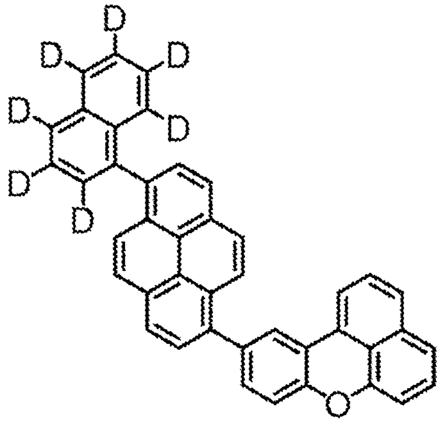

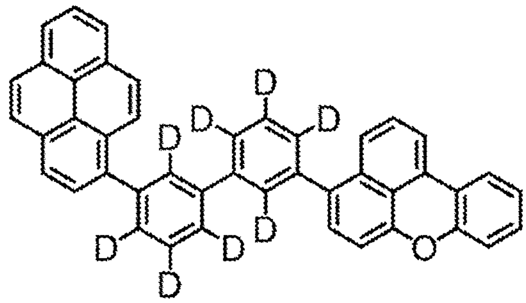

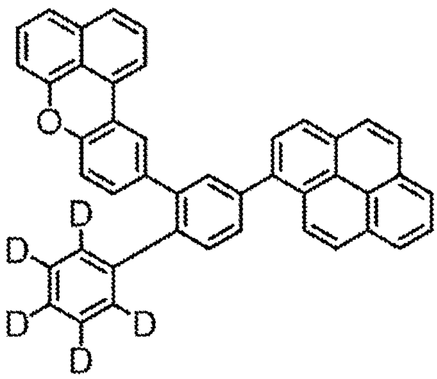

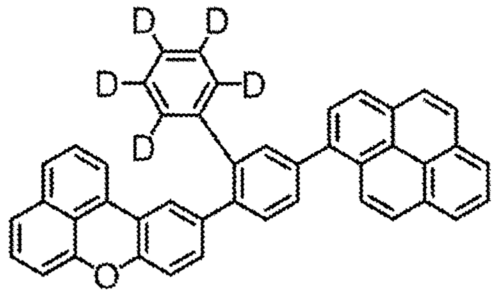

[Formula 115]

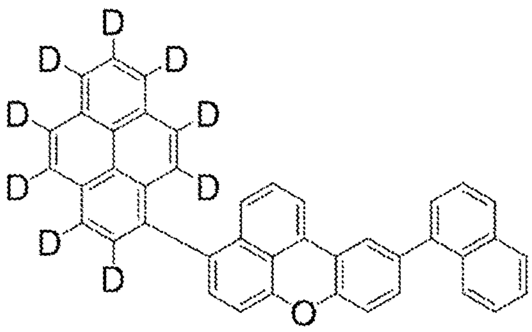

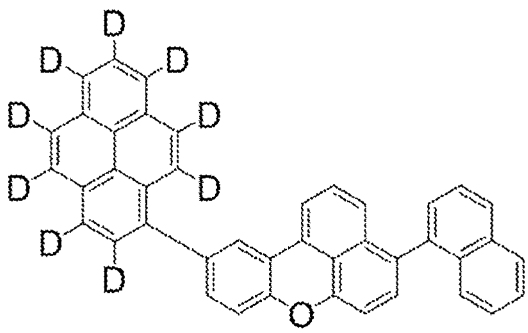

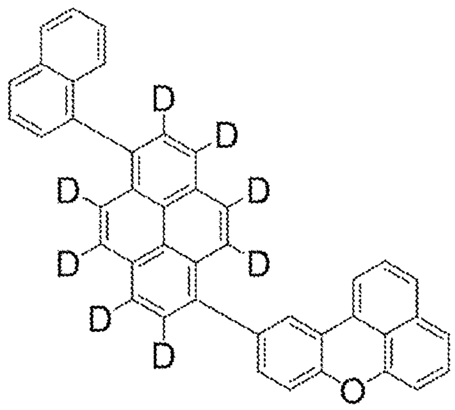

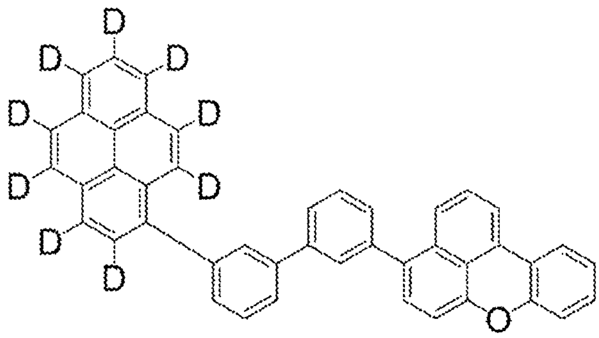

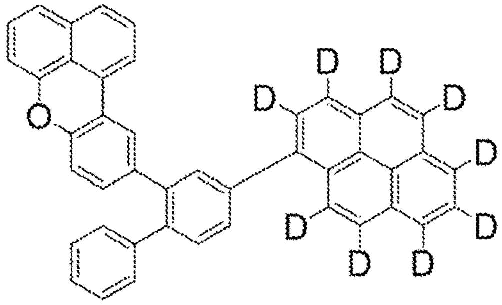

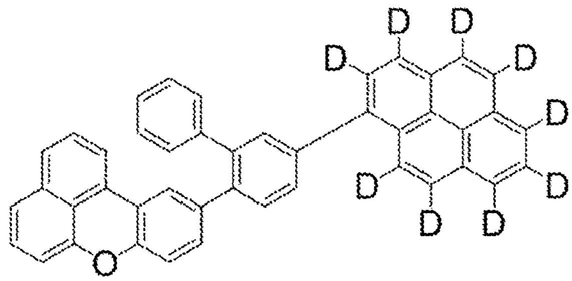

[Formula 116]

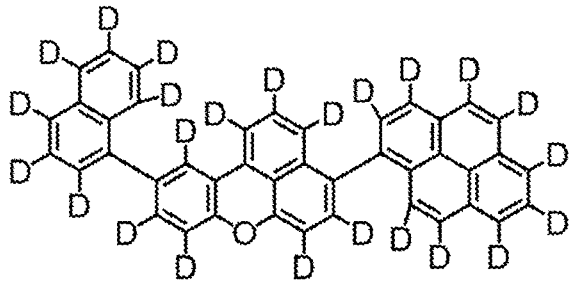

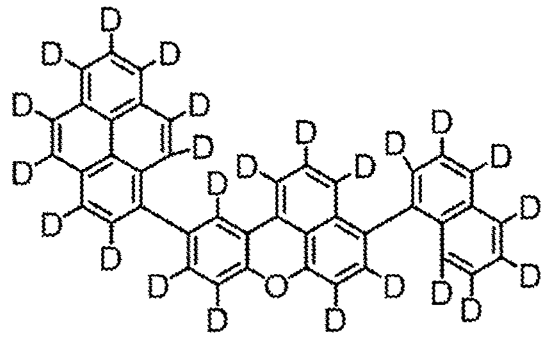

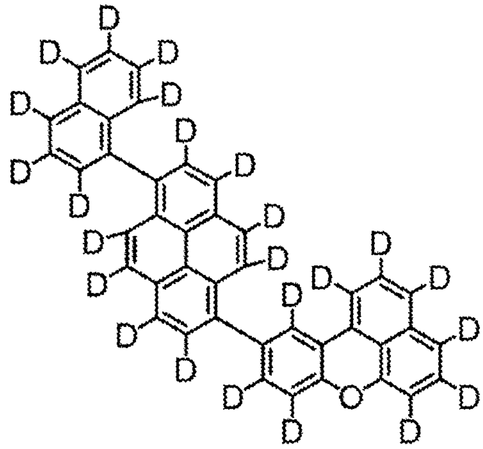

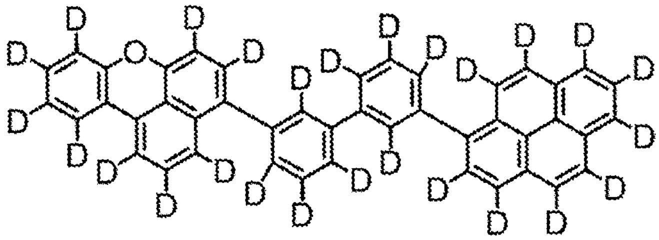

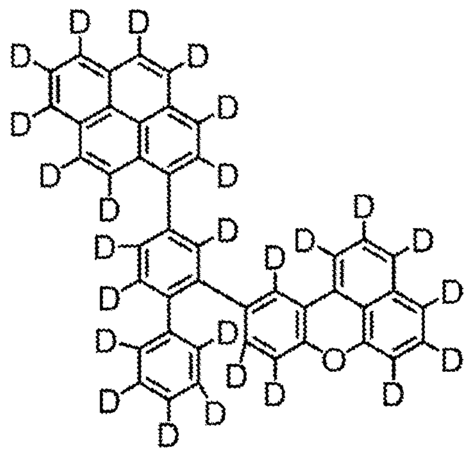

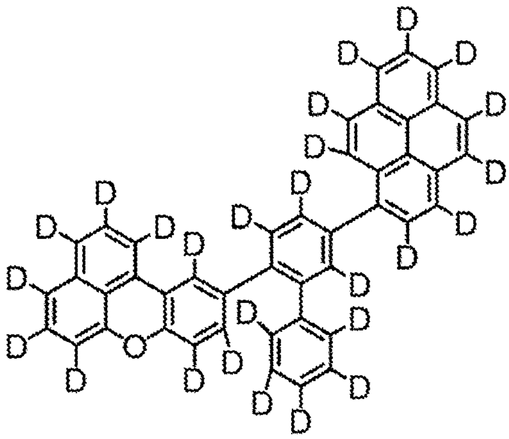

[Formula 117]

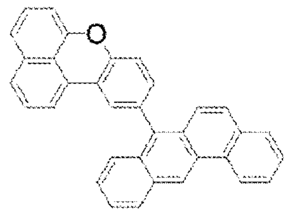
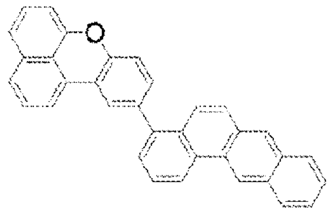
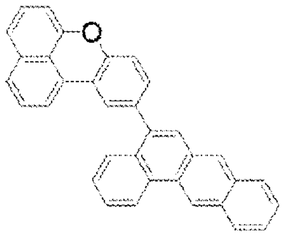
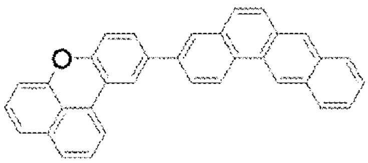
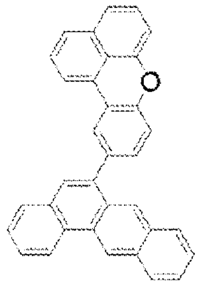
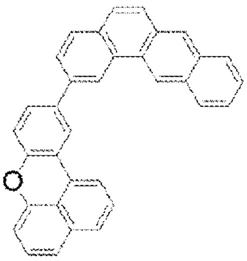
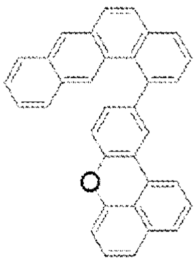
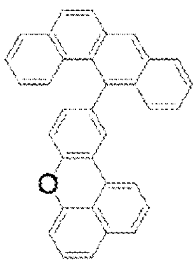
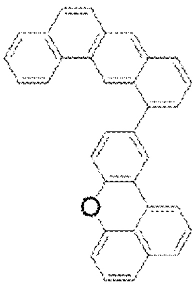
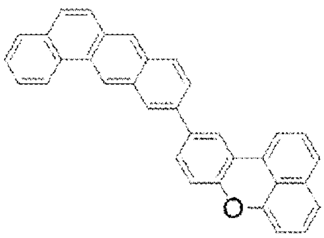
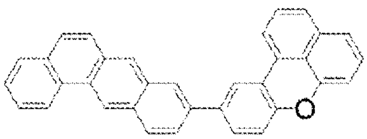
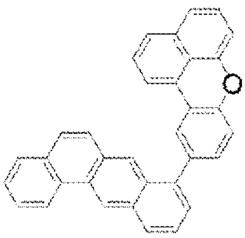
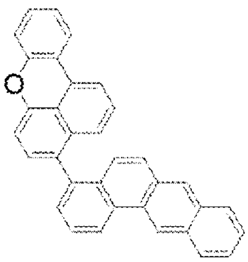
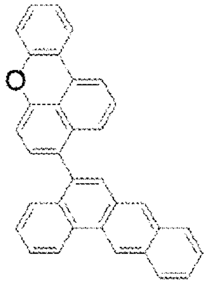
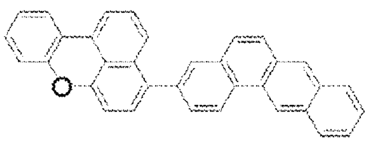
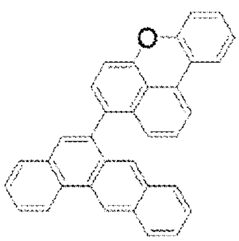

[Formula 118]

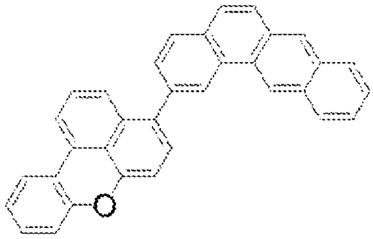
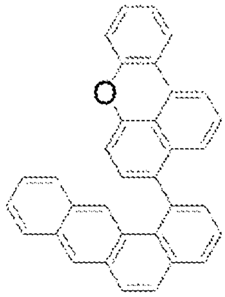
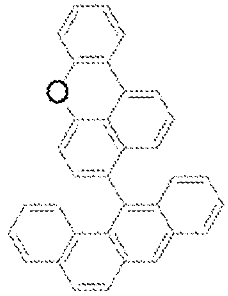
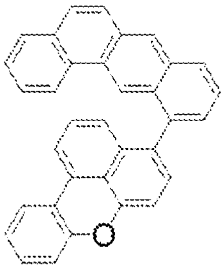
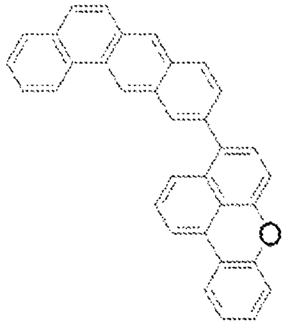
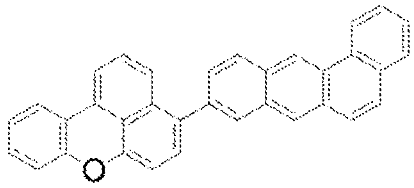
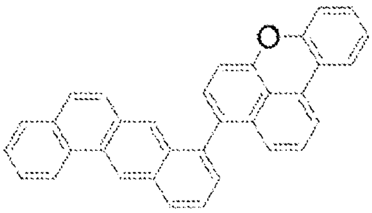
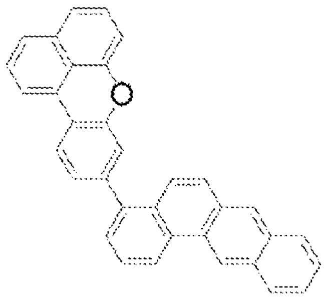
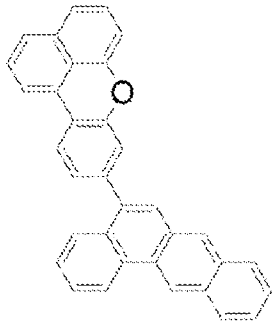
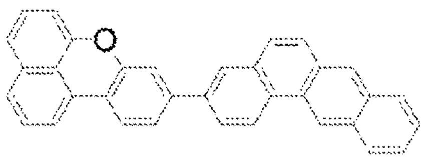
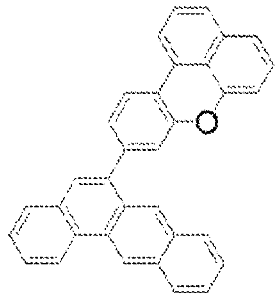

[Formula 119]

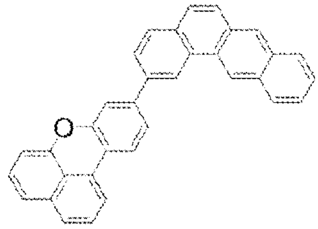
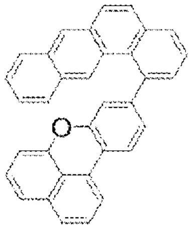
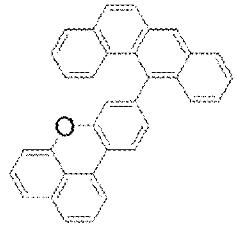

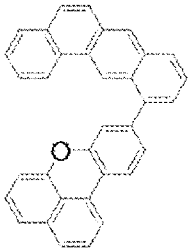
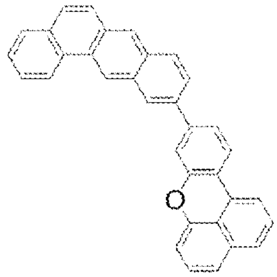
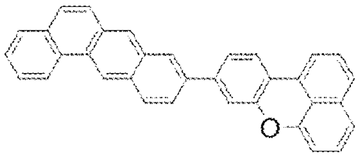

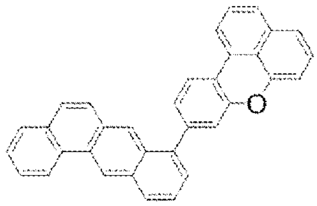
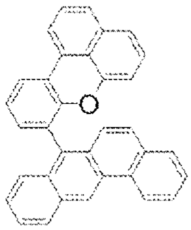
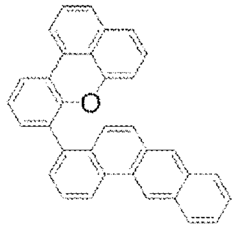

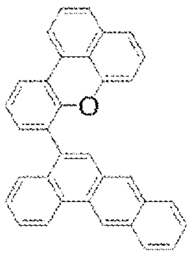
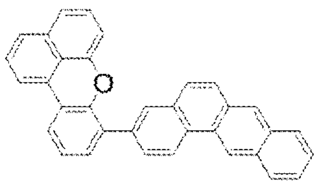
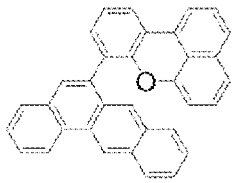

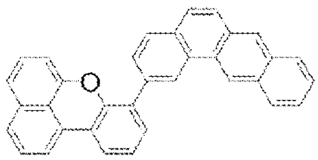
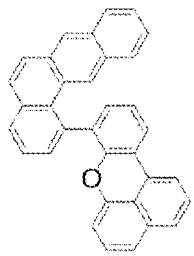
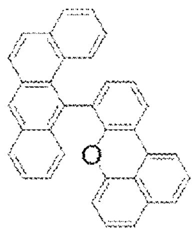

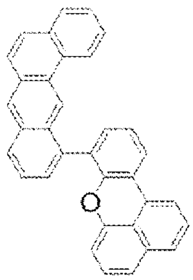
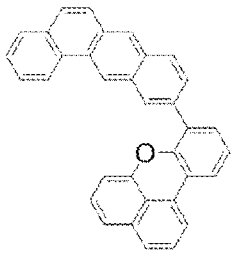

[Formula 120]

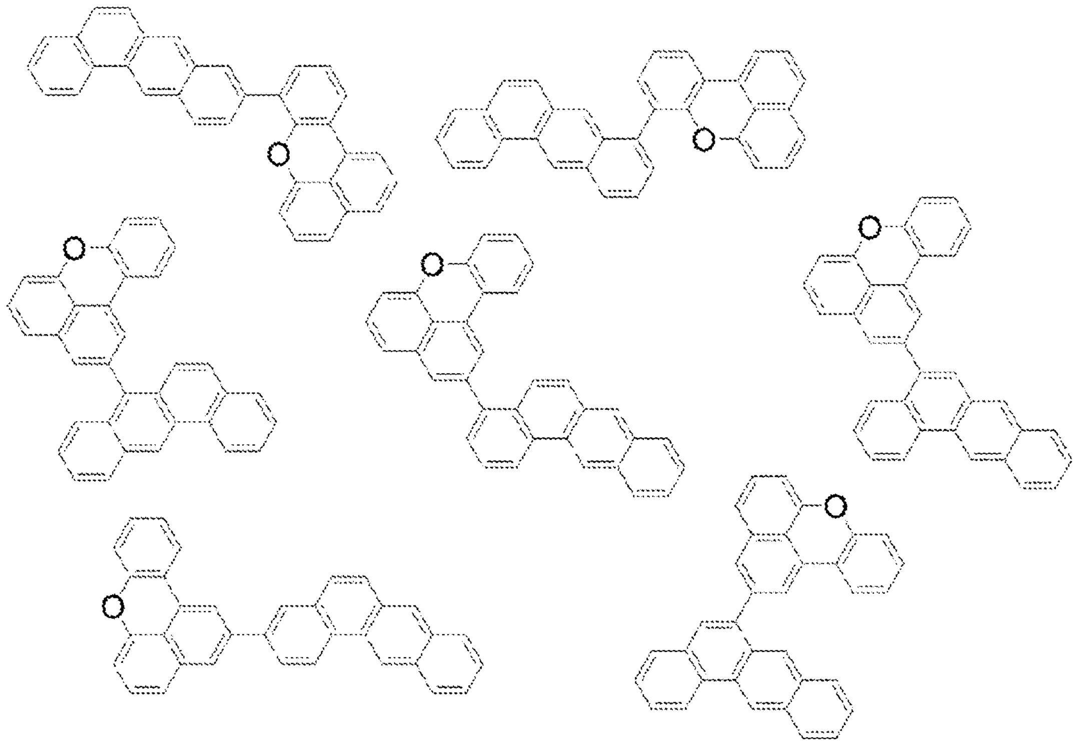

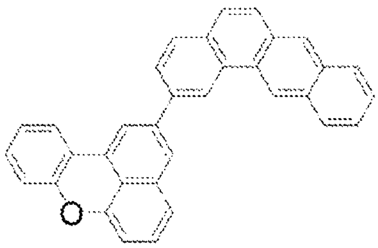

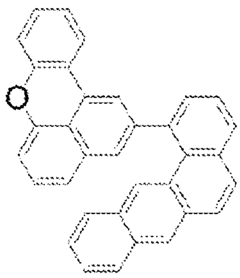

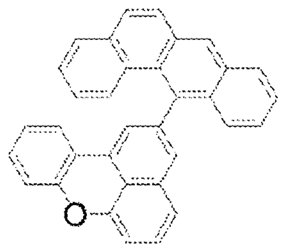

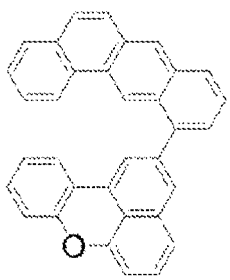

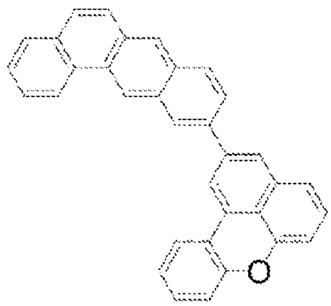

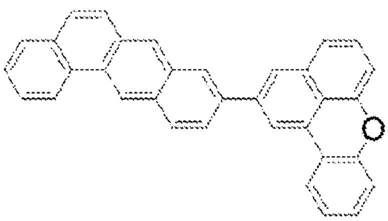

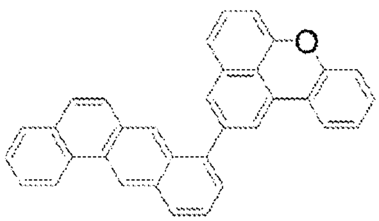

[Formula 121]

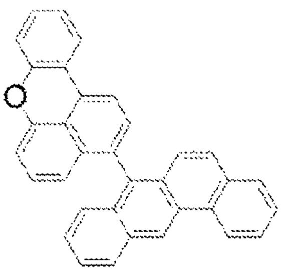
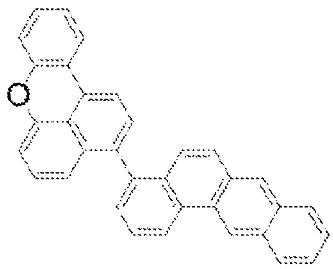
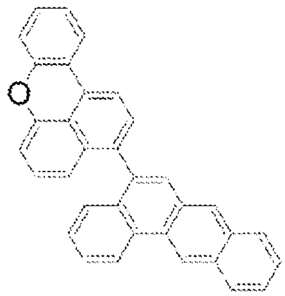
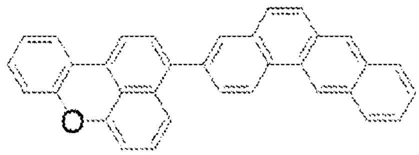
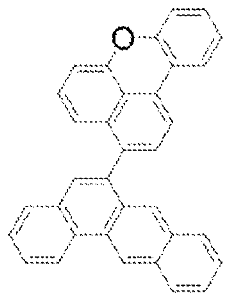
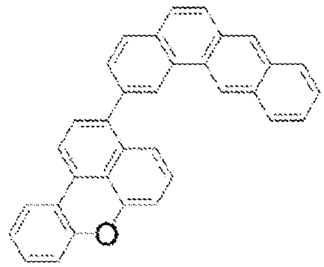
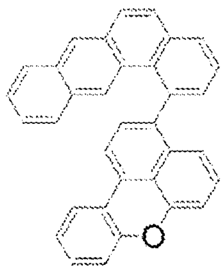
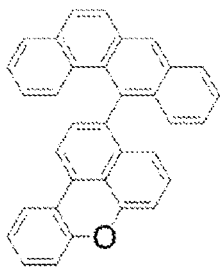
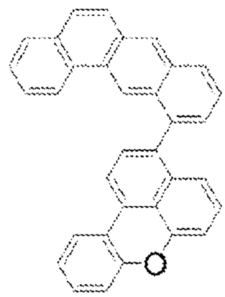
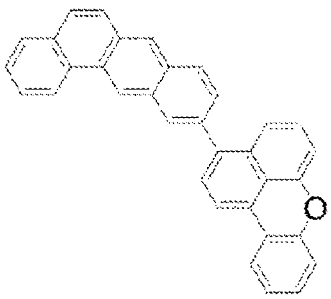
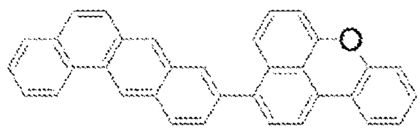
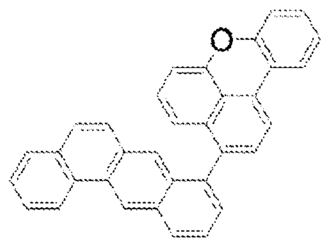
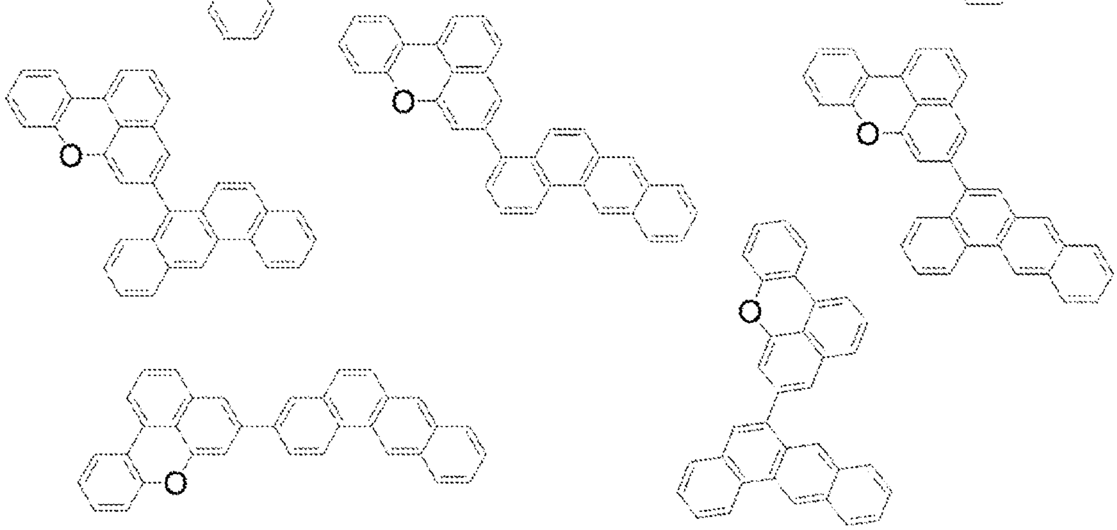

[Formula 122]

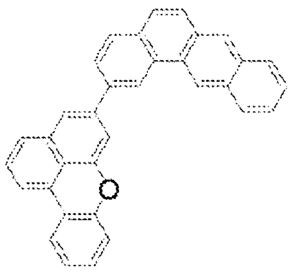
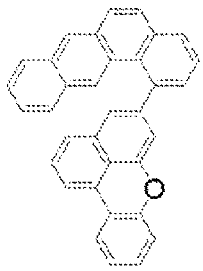
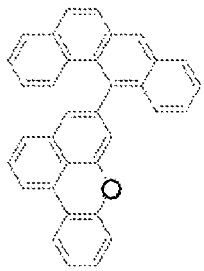
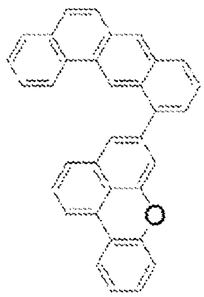
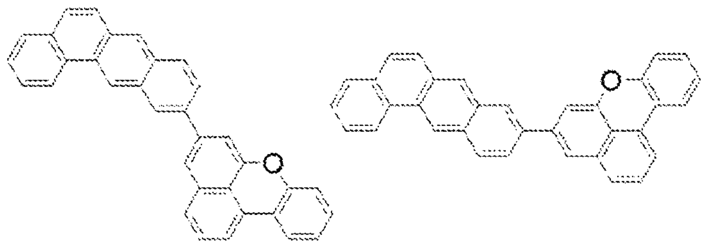
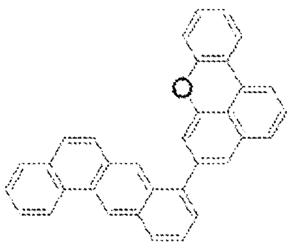
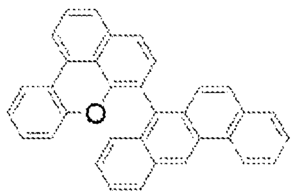
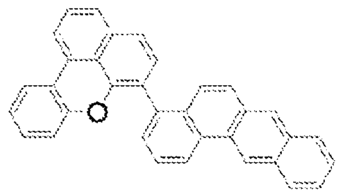
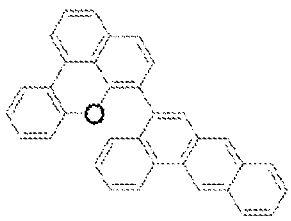
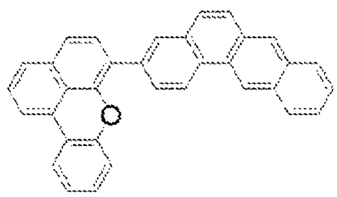
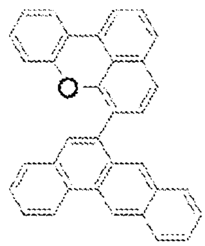
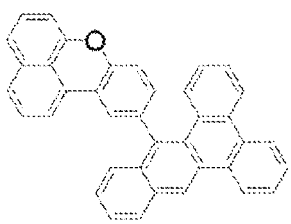
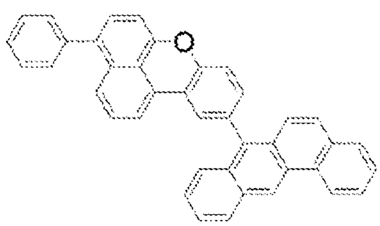
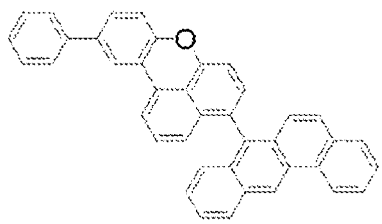
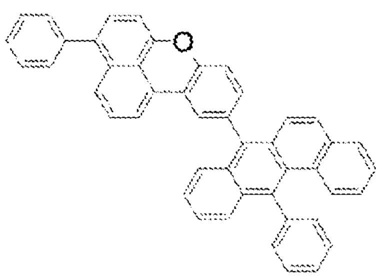
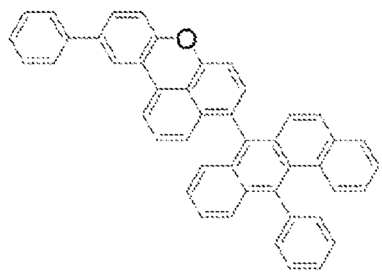

[Formula 123]

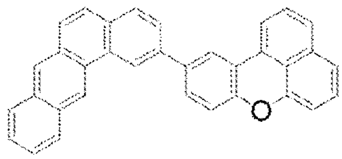
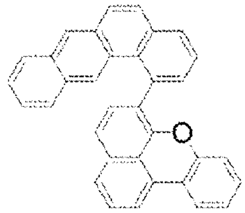
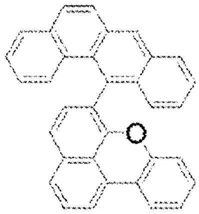
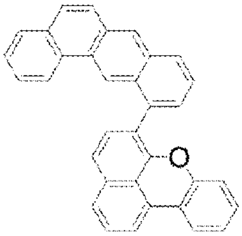
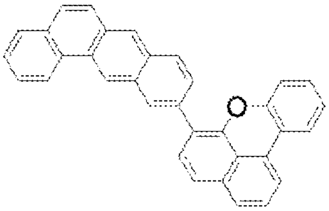
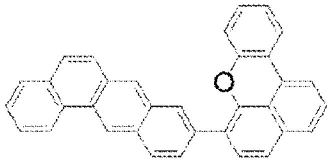
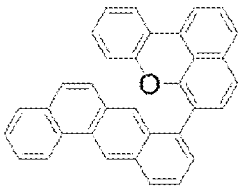
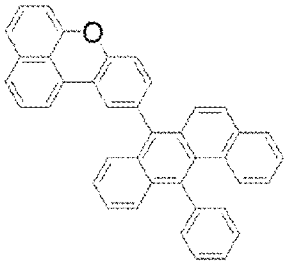
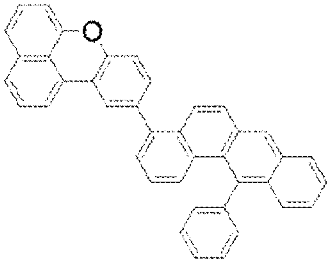
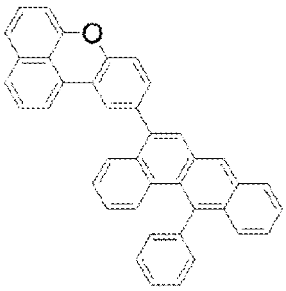
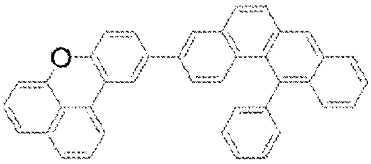
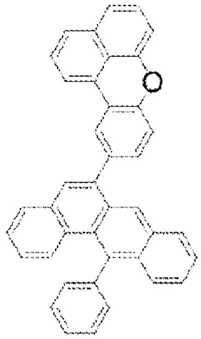
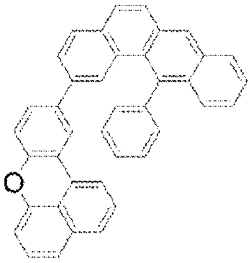
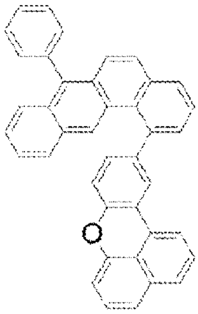
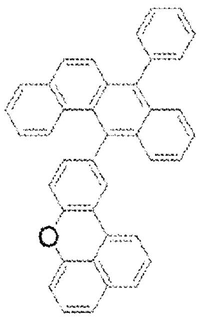
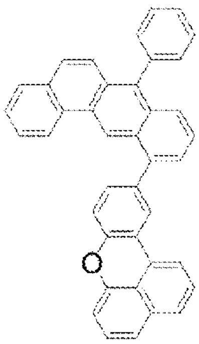
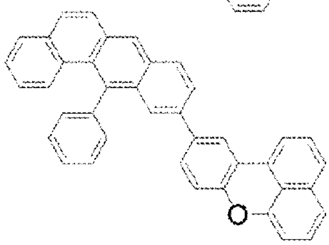

[Formula 124]

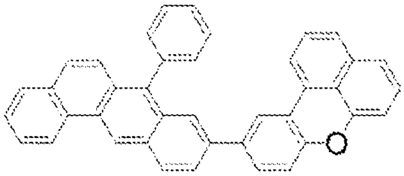

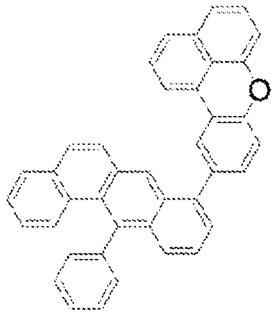

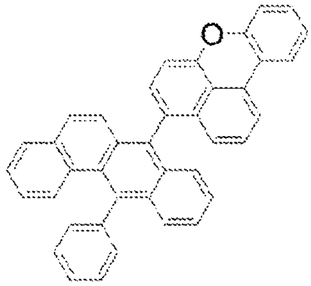

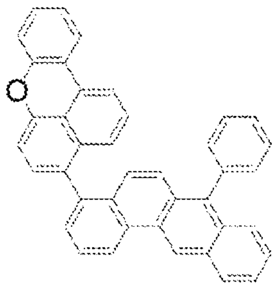

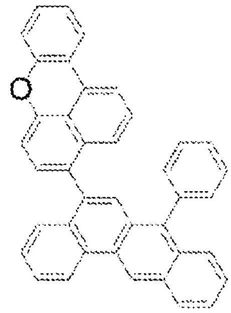

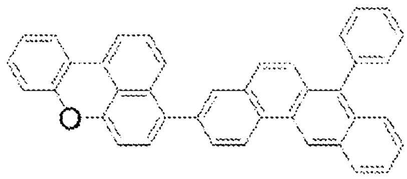

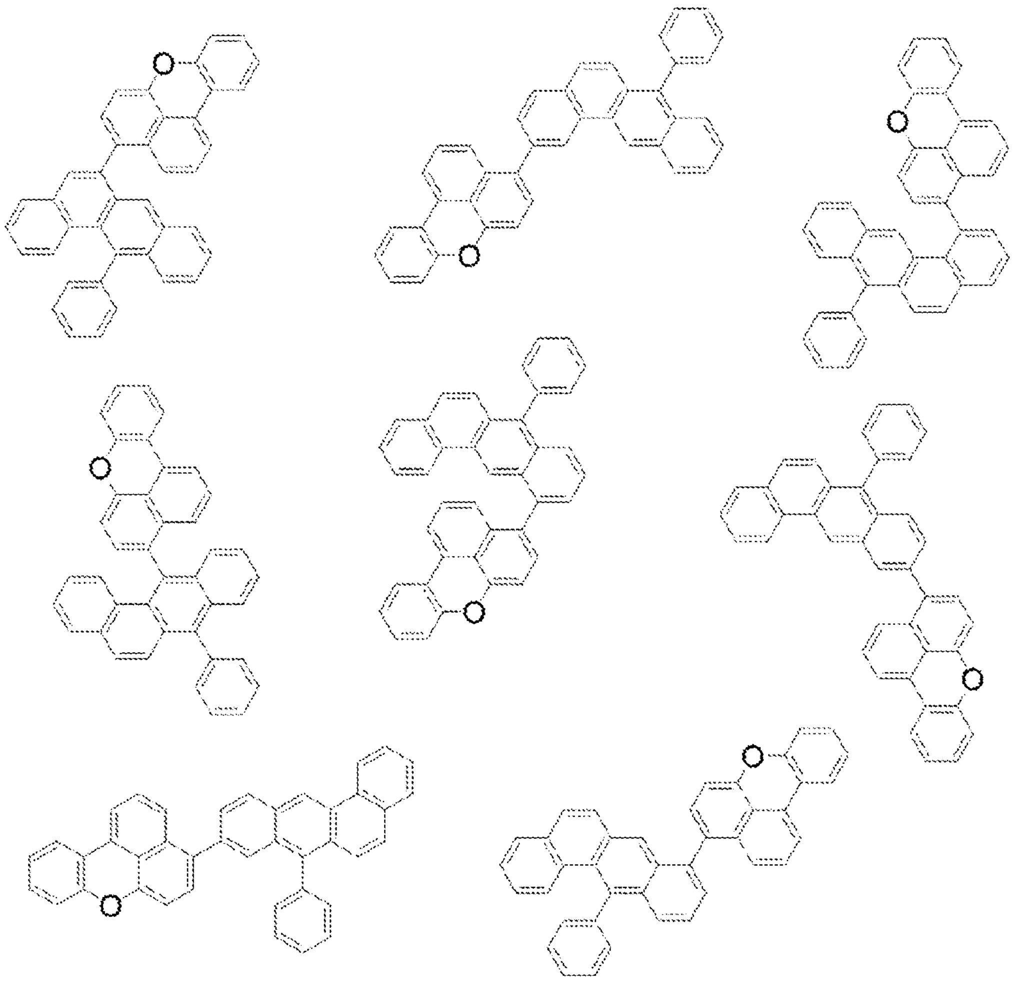

[Formula 125]

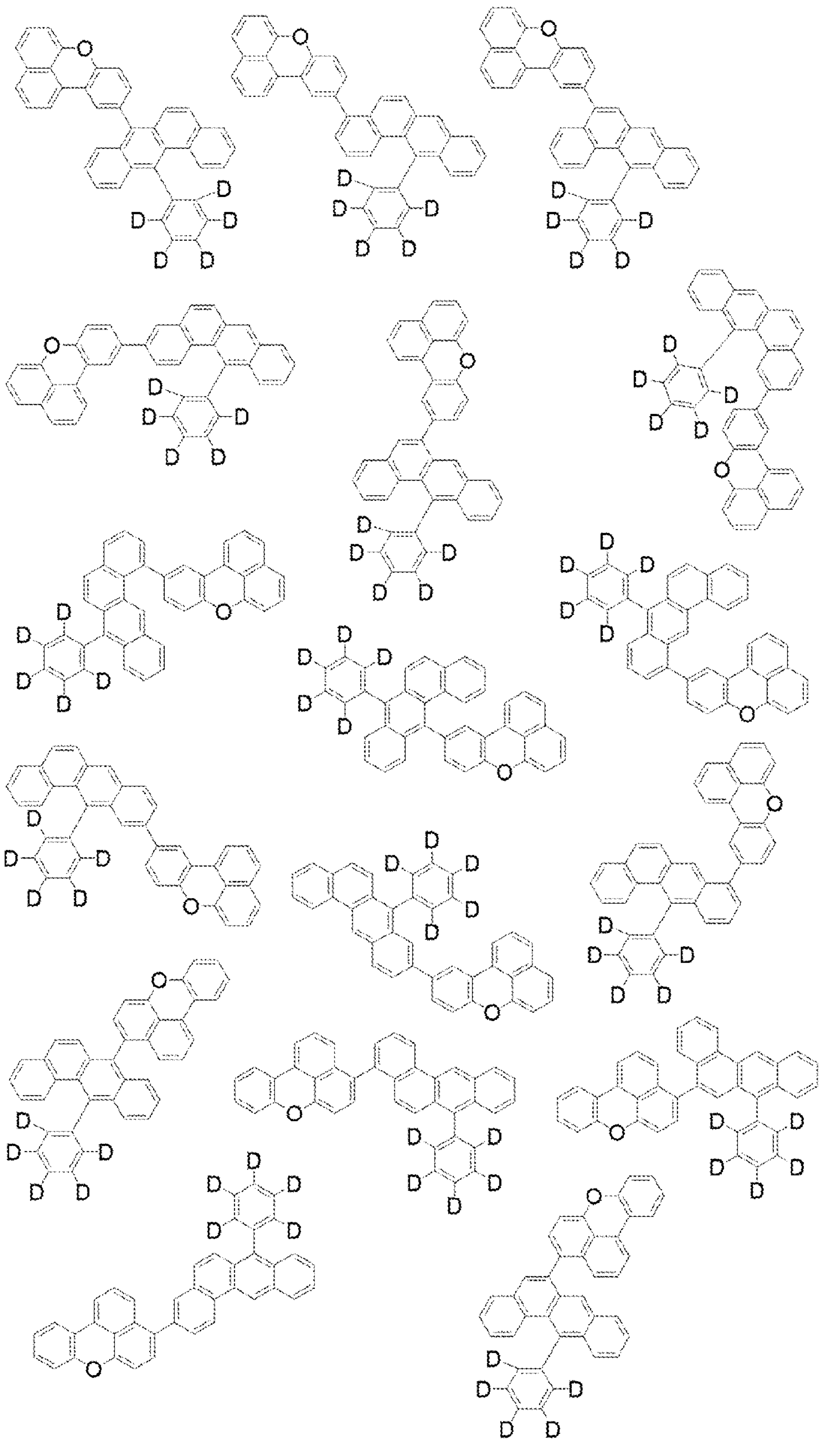

[Formula 126]

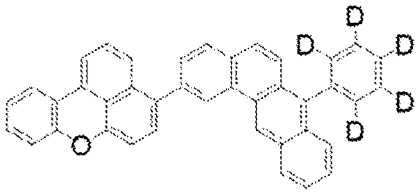
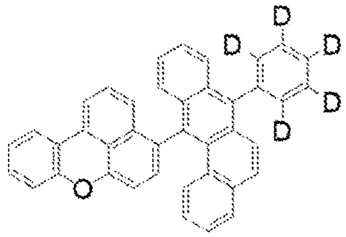
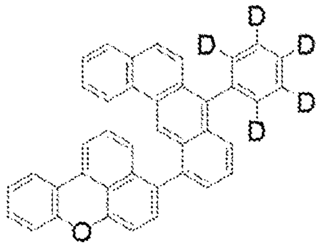
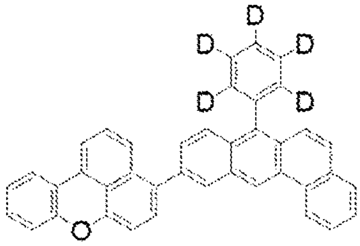
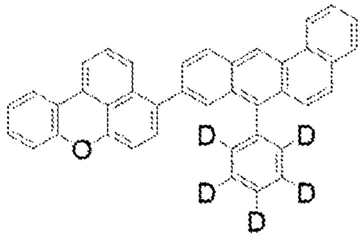
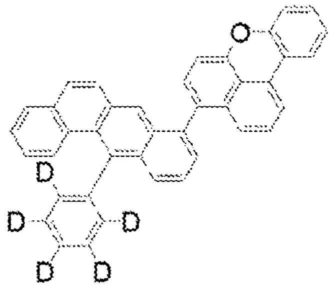
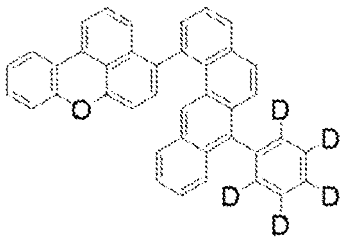
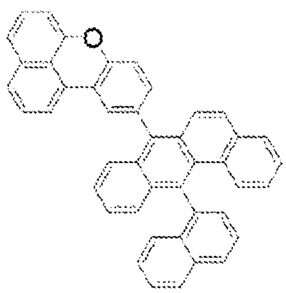
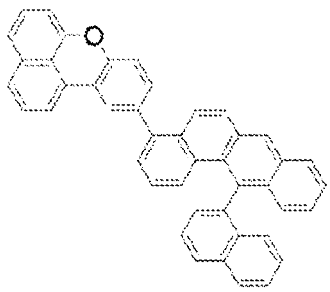
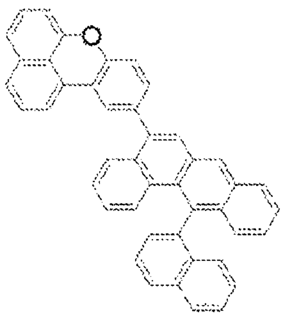
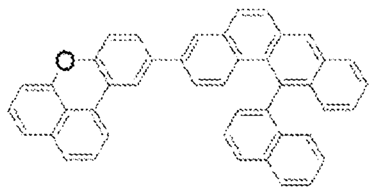
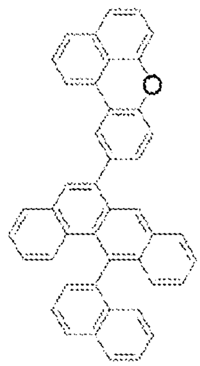

[Formula 127]

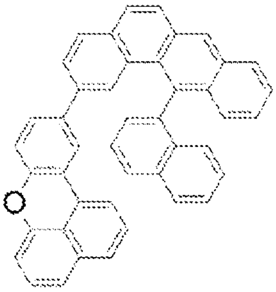

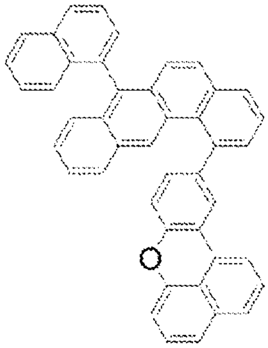

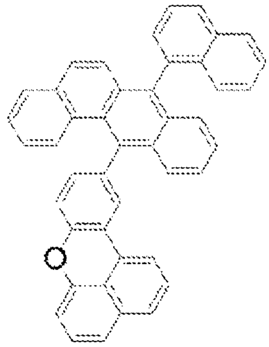

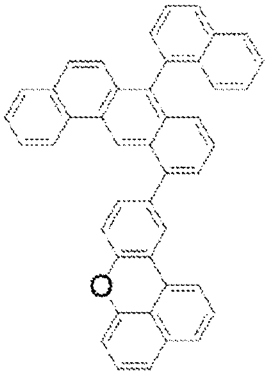

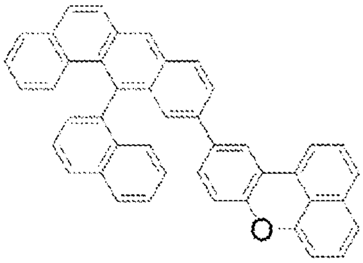

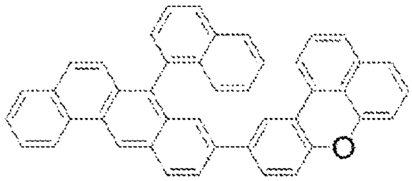

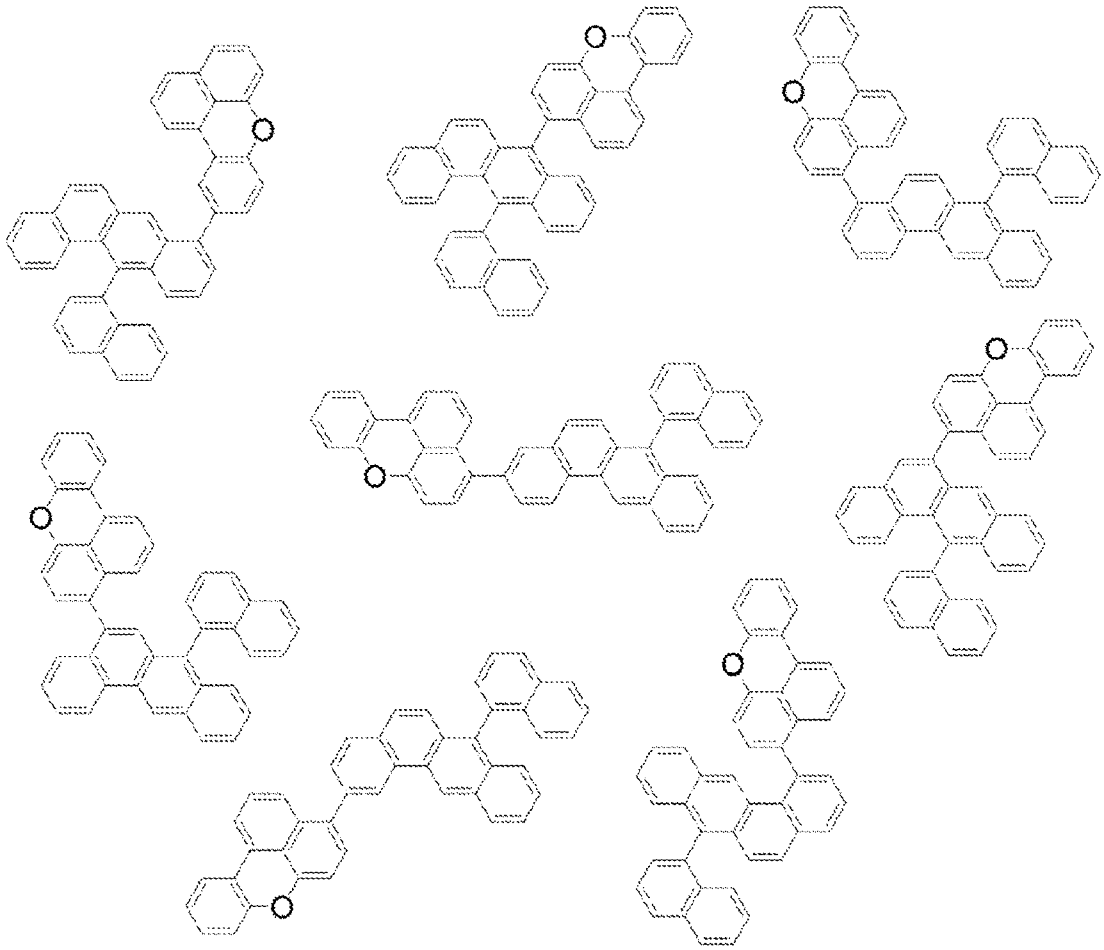

[Formula 128]

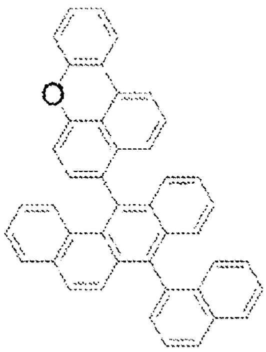
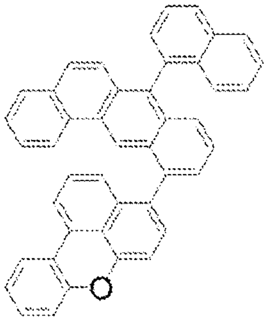
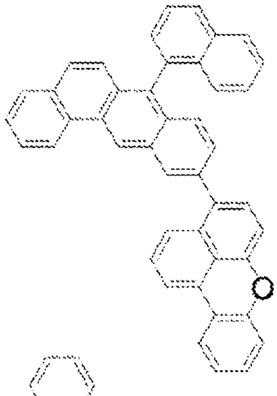
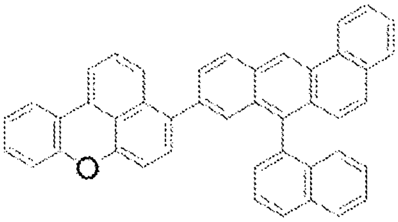
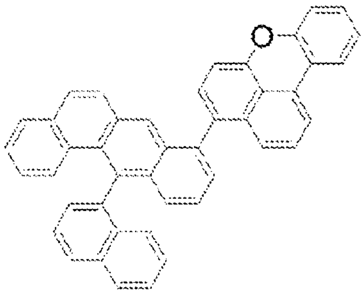
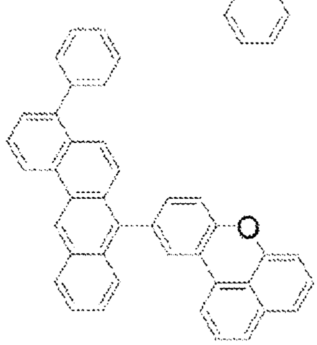
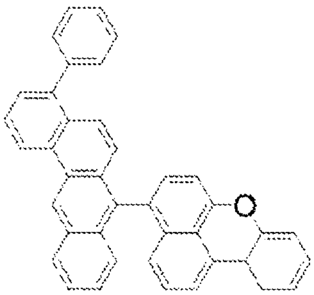
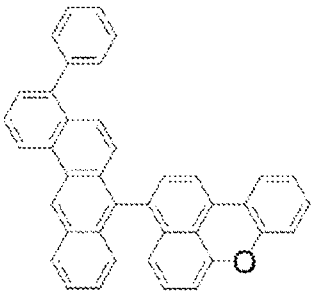
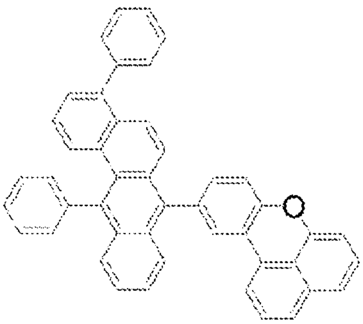
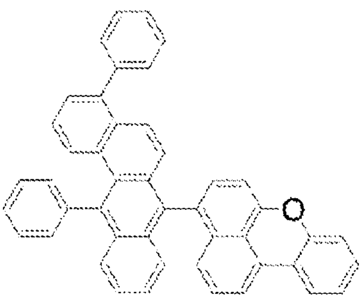
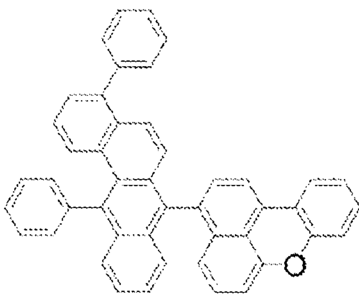
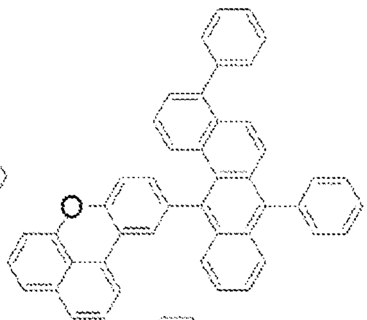
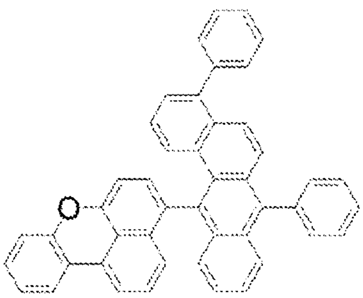
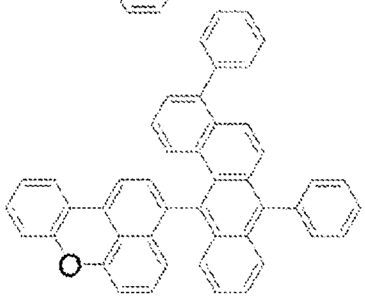

[Formula 129]

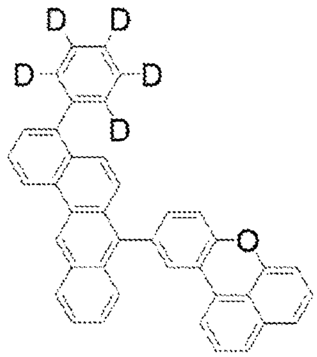

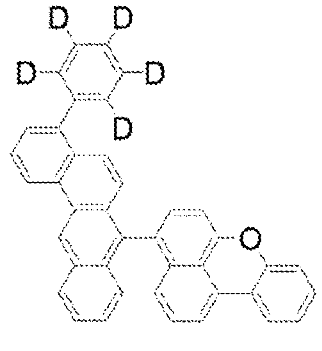

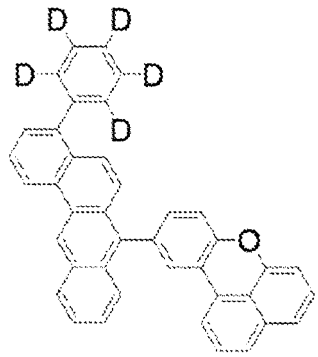

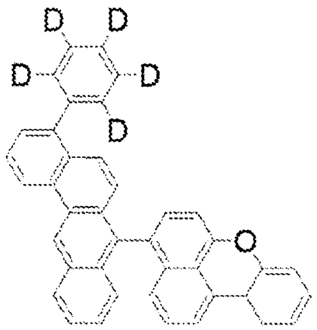

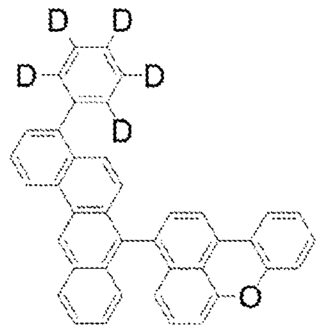

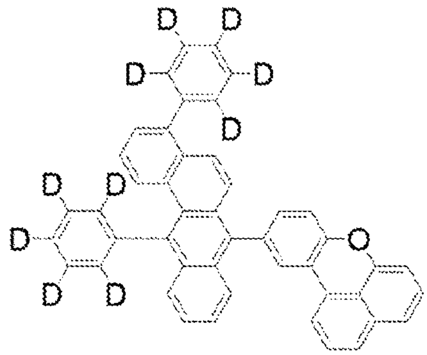

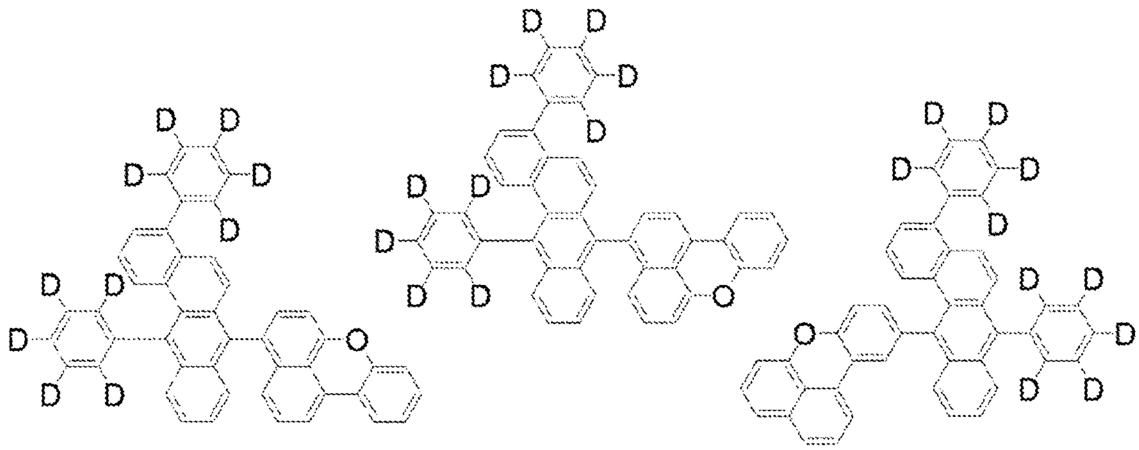

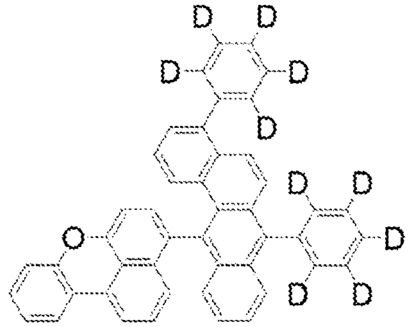

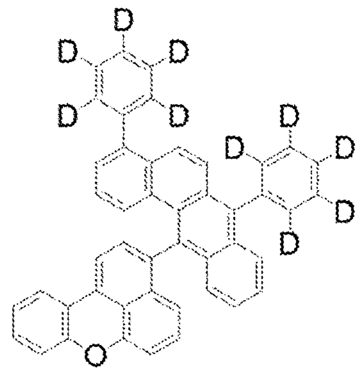

[Formula 130]

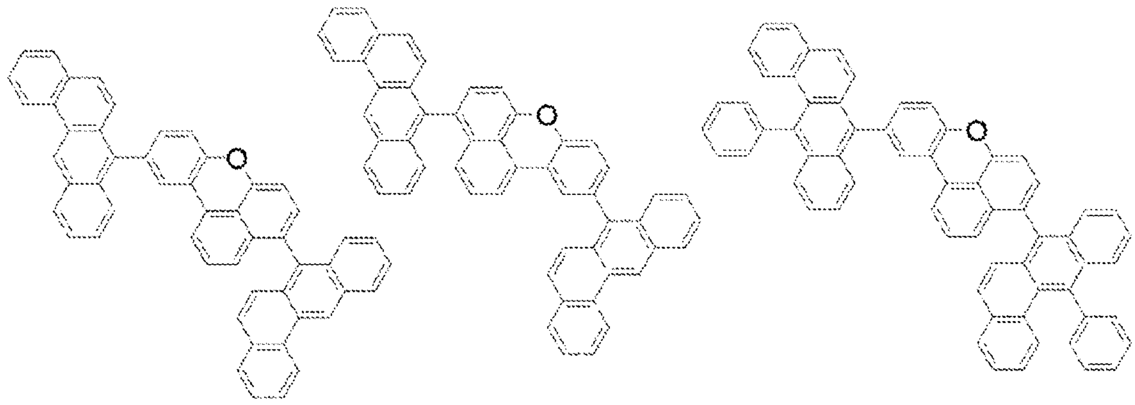

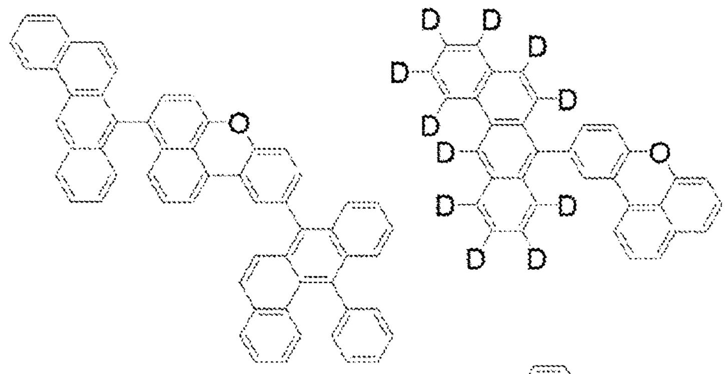

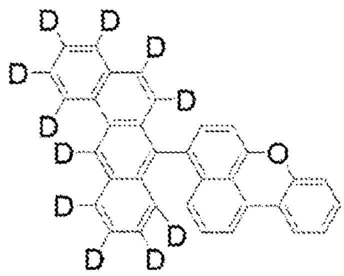

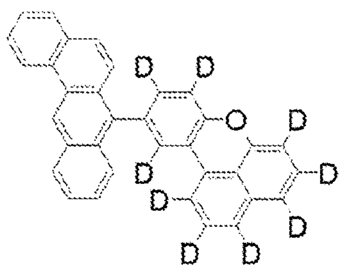

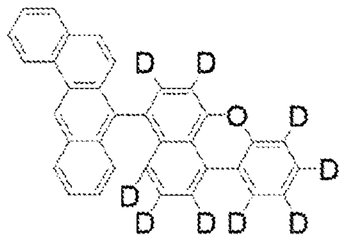

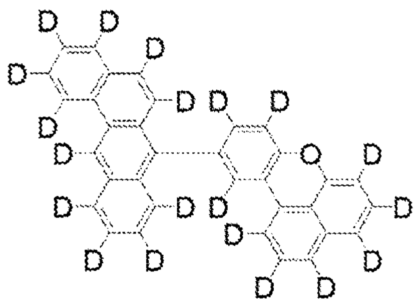

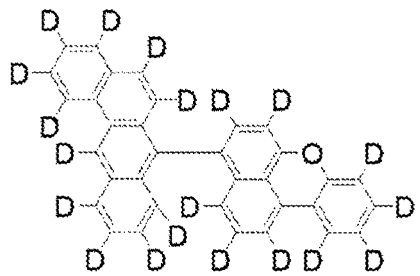

[Formula 131]

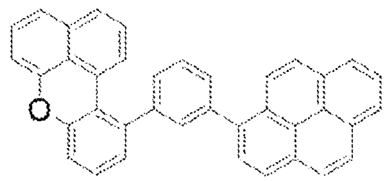

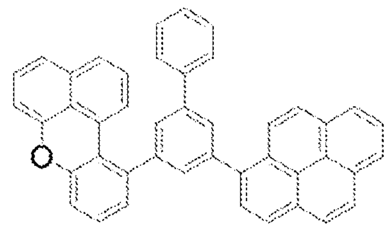

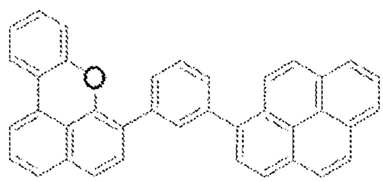

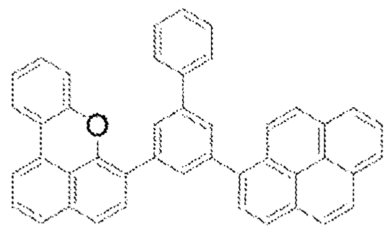

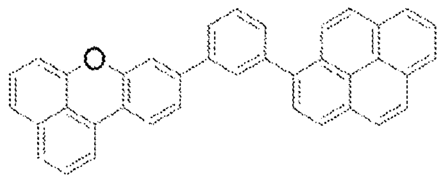

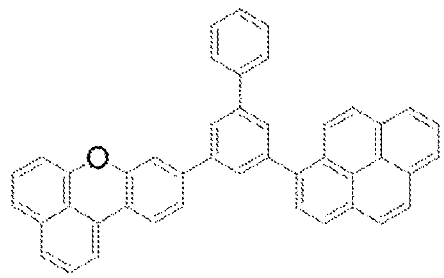

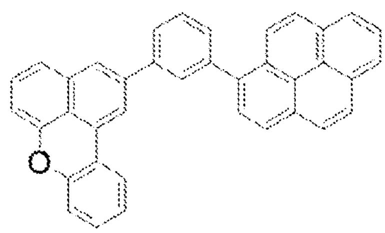

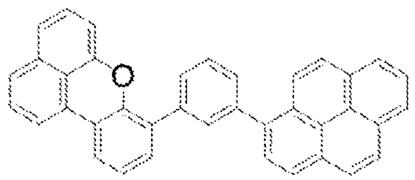

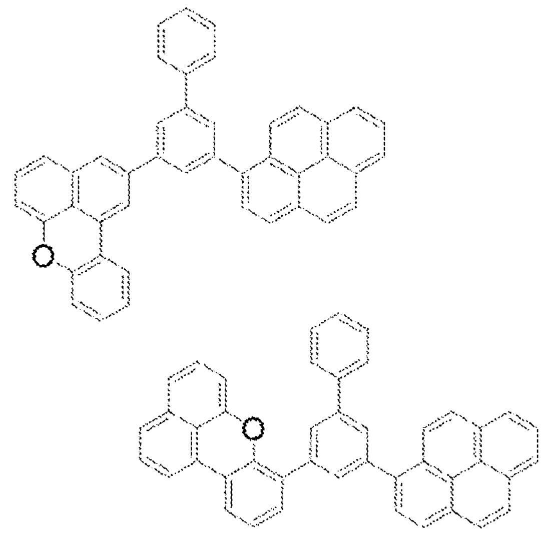

[Formula 132]

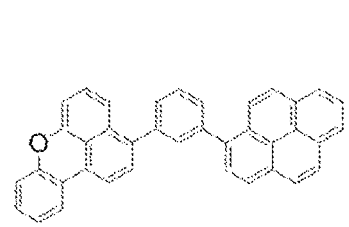

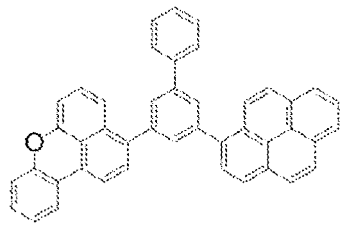

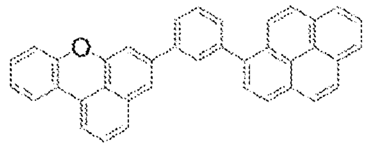

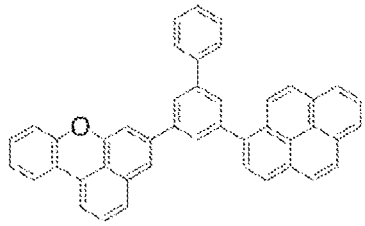

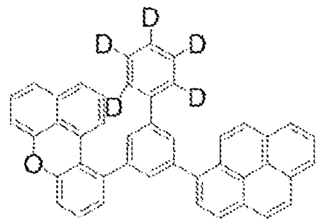

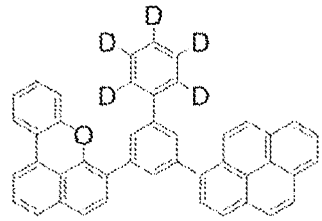

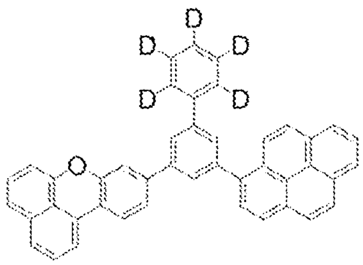

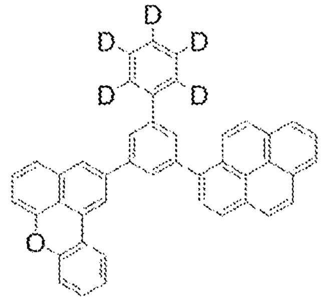

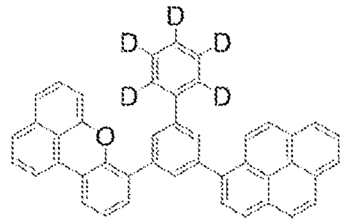

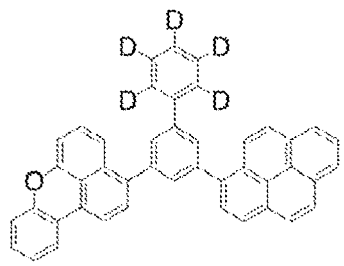

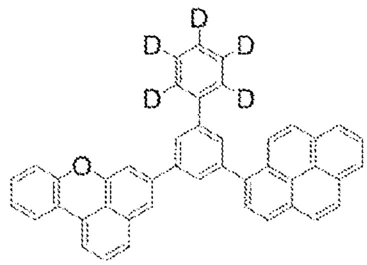

[Formula 133]

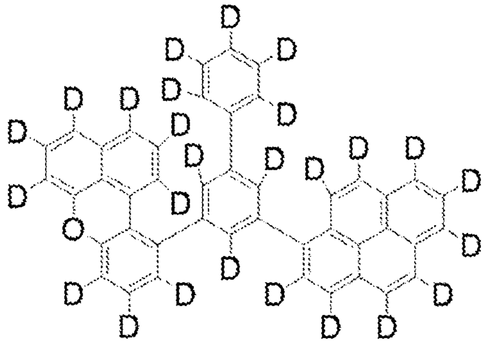

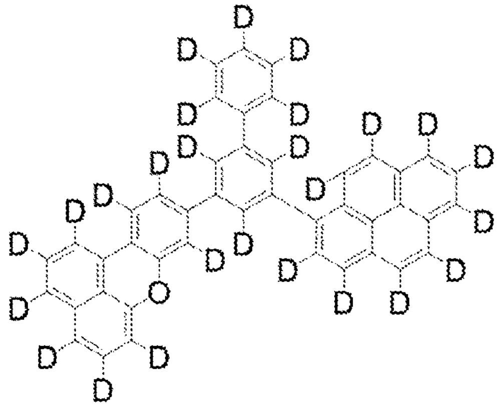

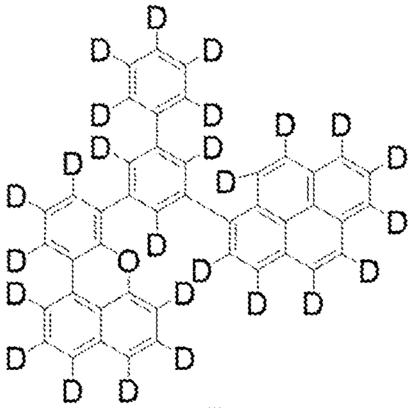

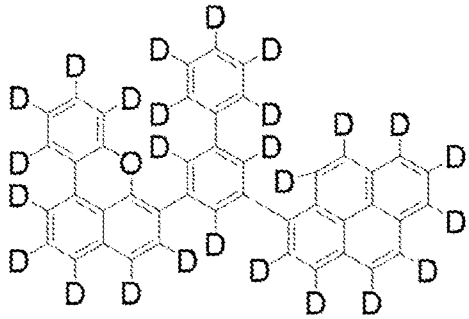

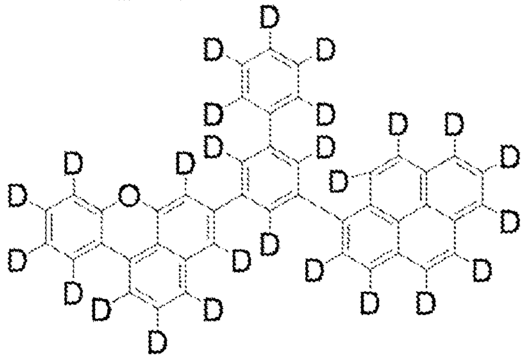

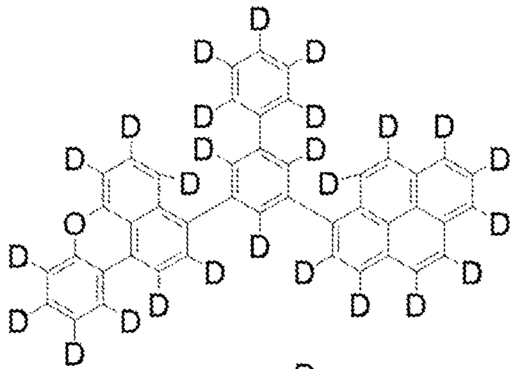

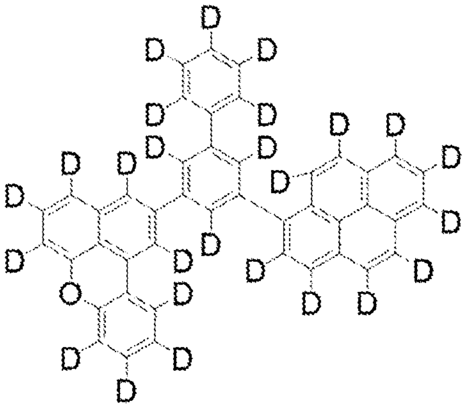

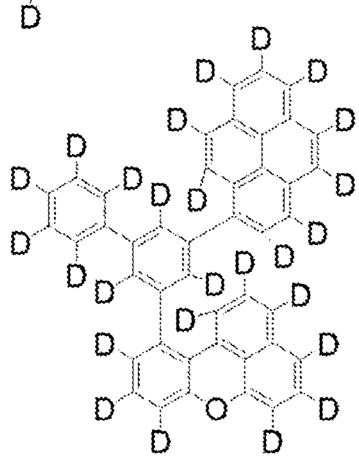

[Formula 134]

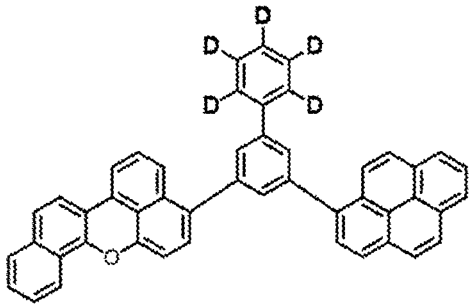

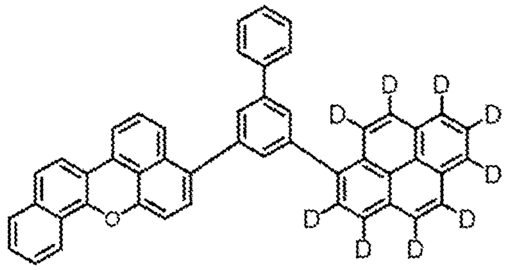

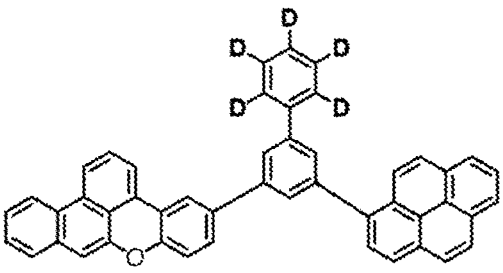

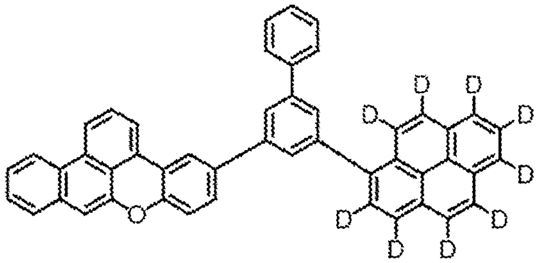

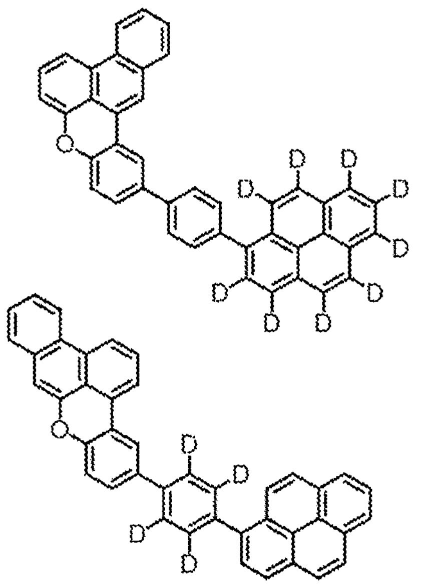

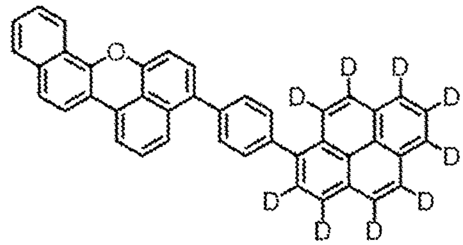

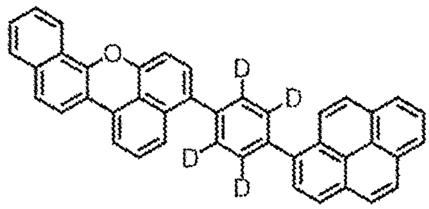

[Formula 135]

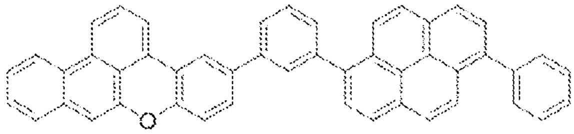

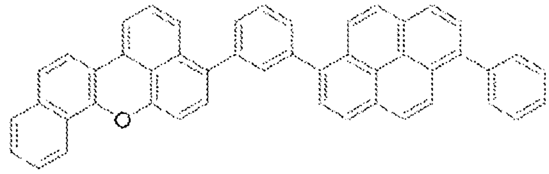

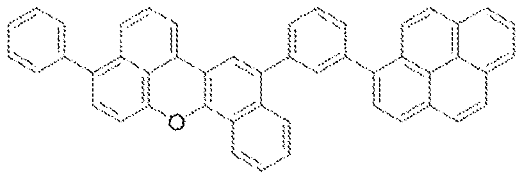

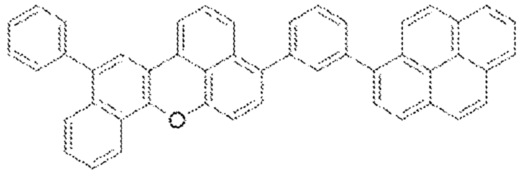

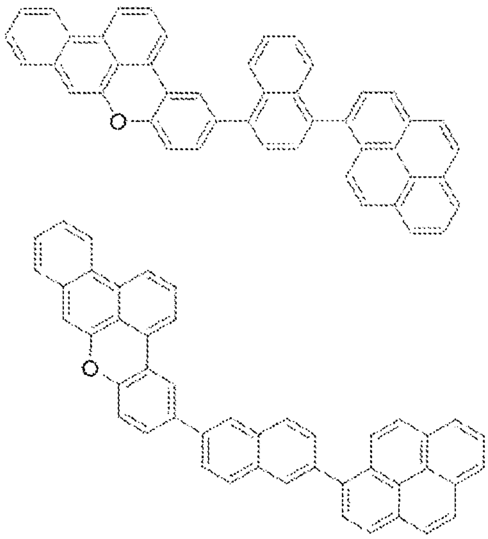

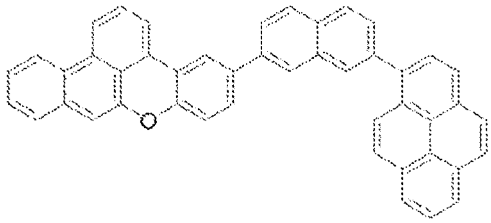

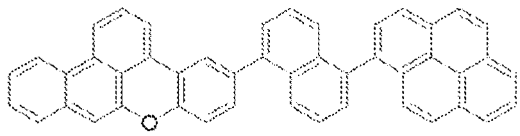

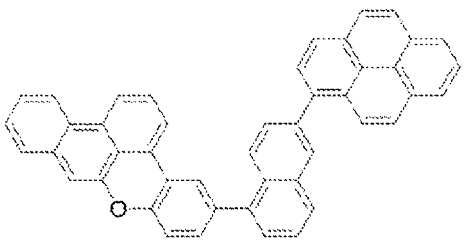

[Formula 136]

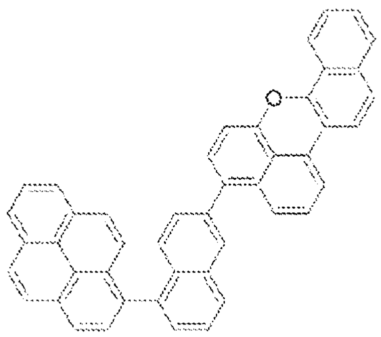

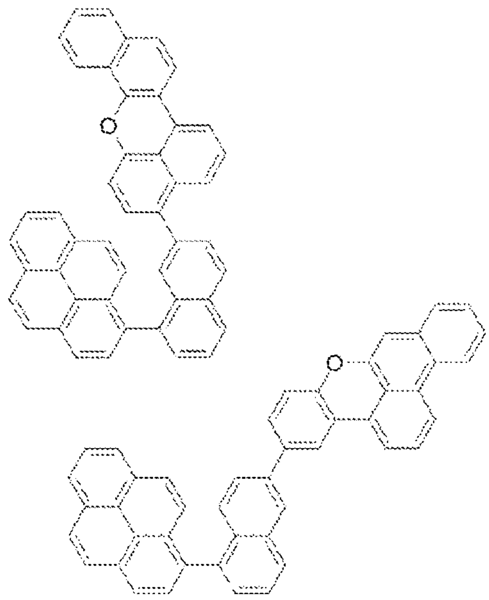

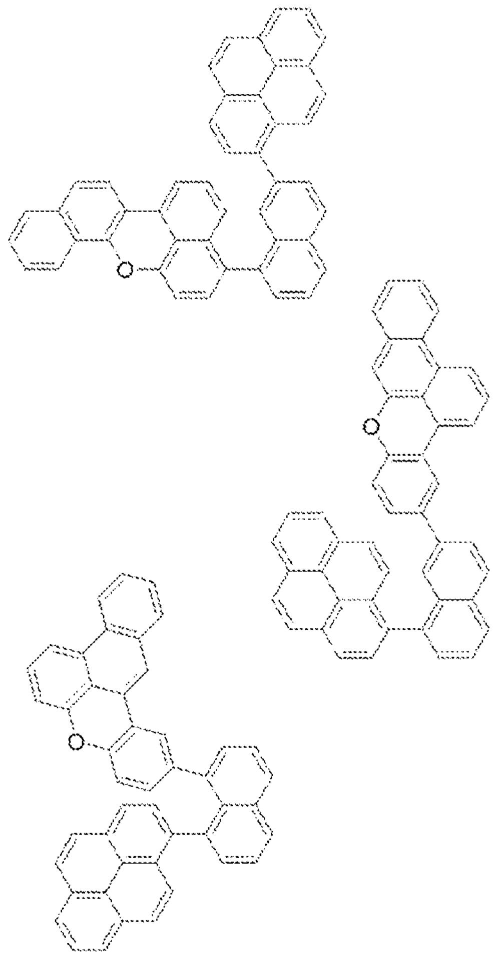

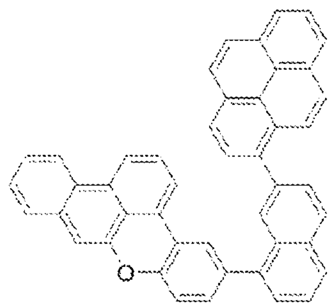

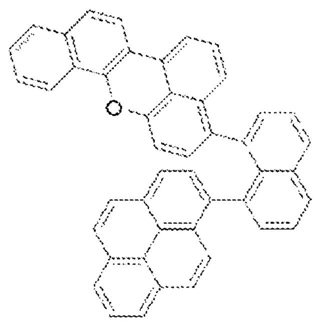

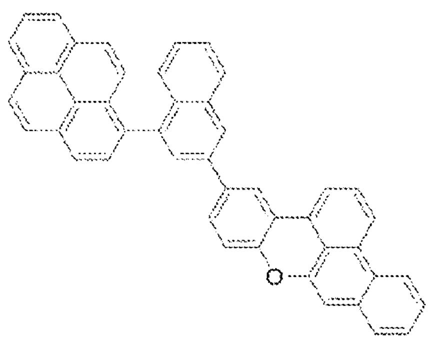

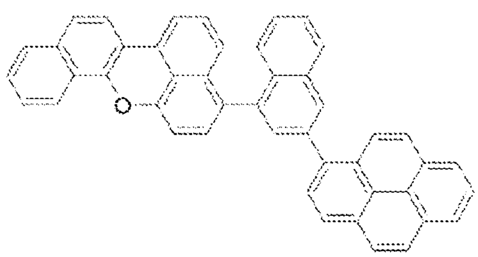

[Formula 137]

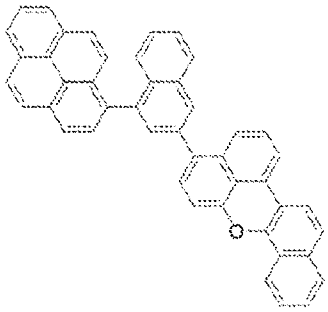

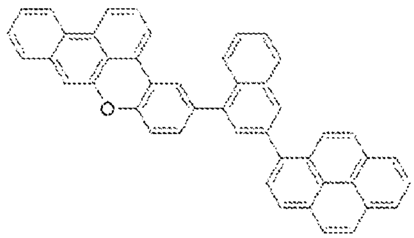

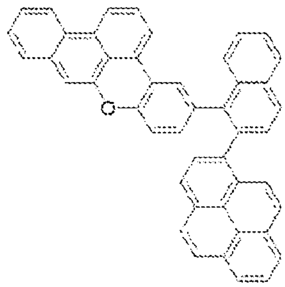

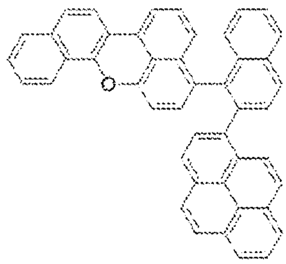

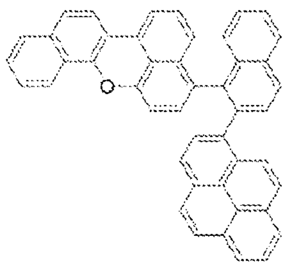

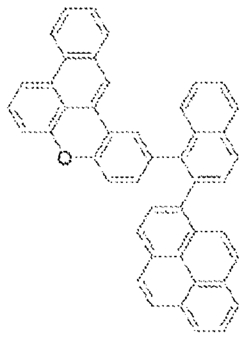

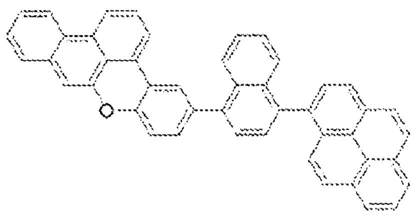

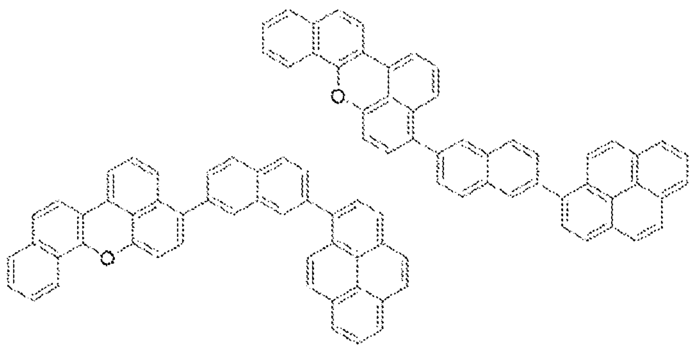

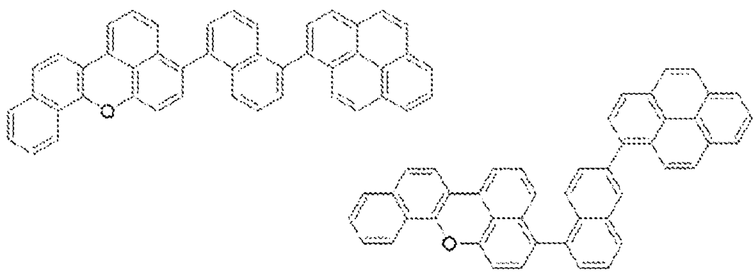

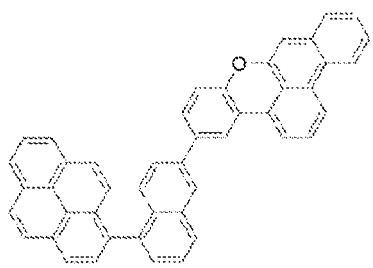

[Formula 138]

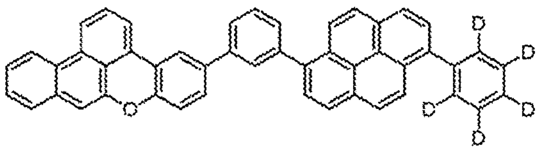

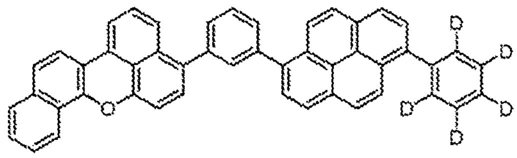

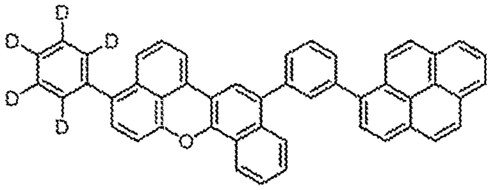

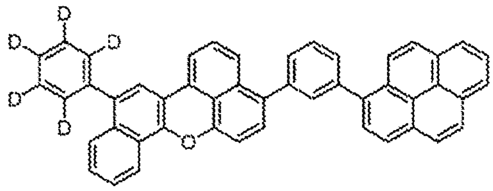

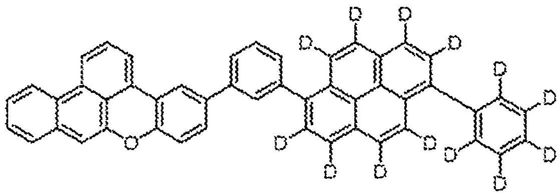

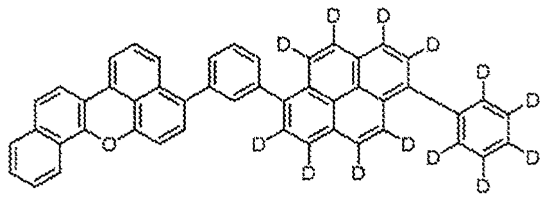

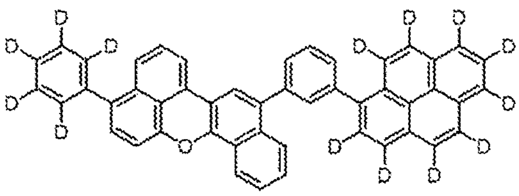

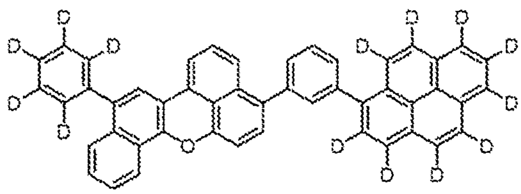

[Formula 139]

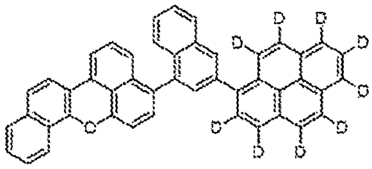
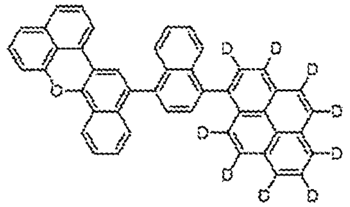
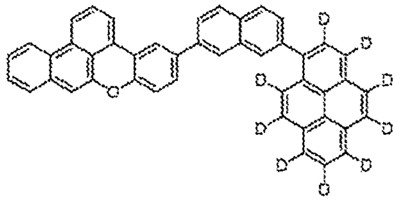
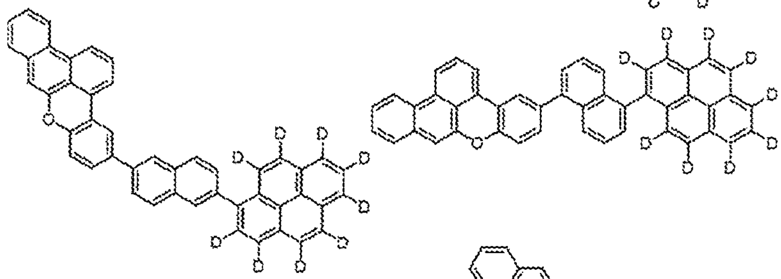
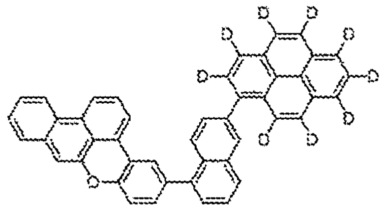
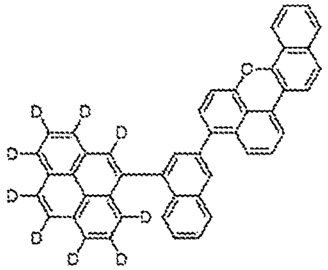
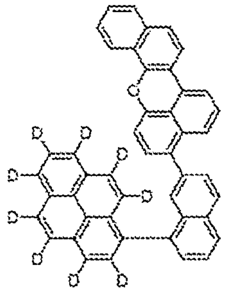
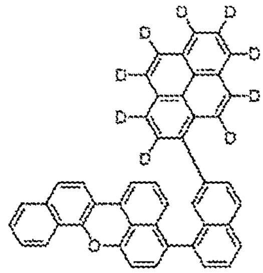
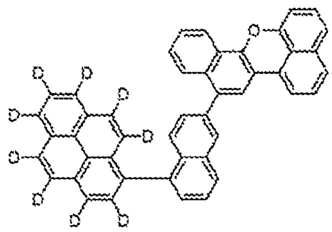
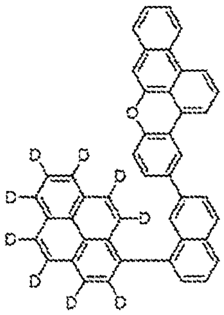
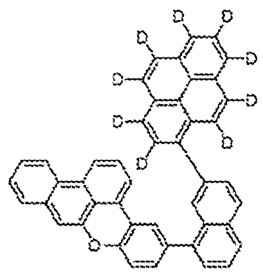
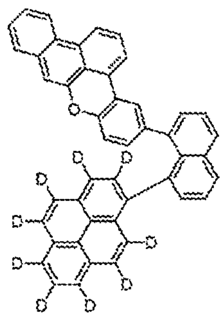
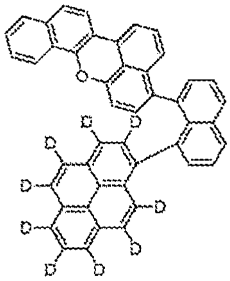
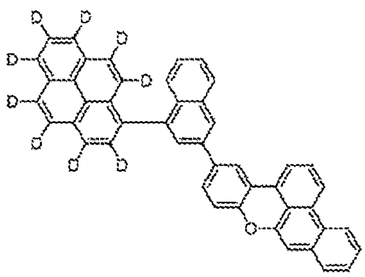

[Formula 140]

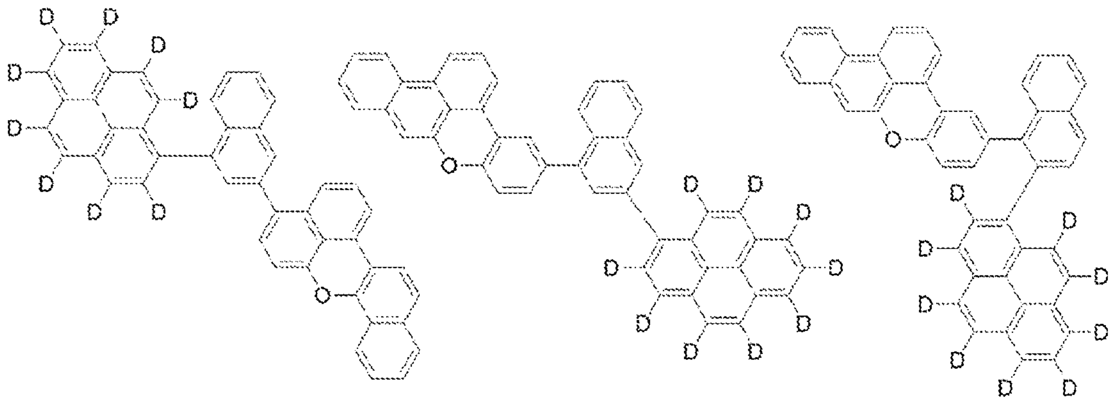

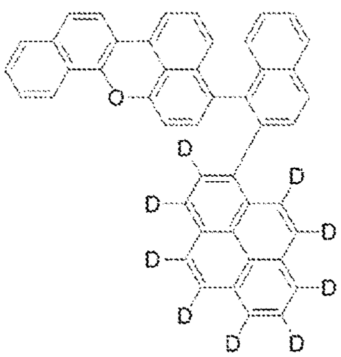

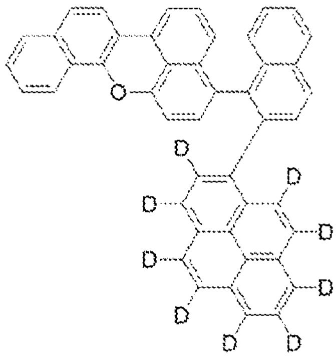

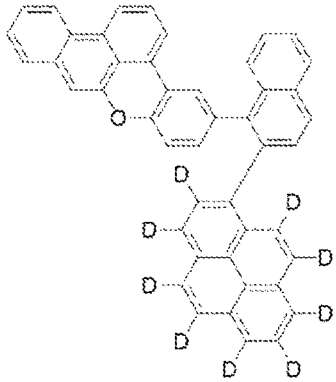

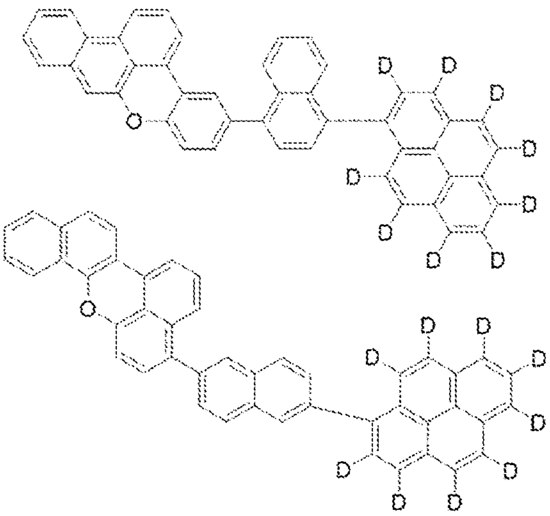

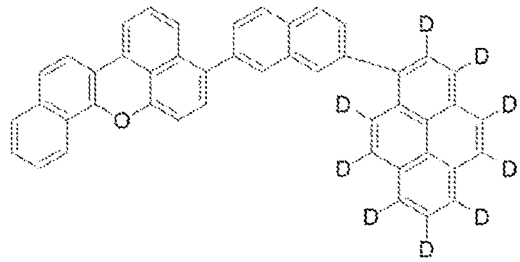

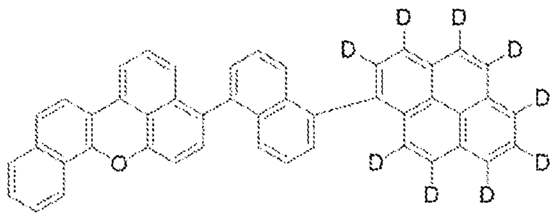

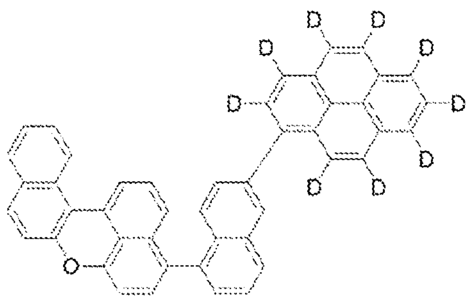

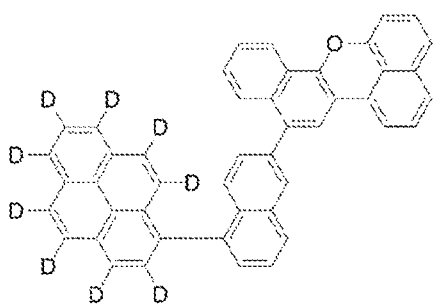

[Formula 141]

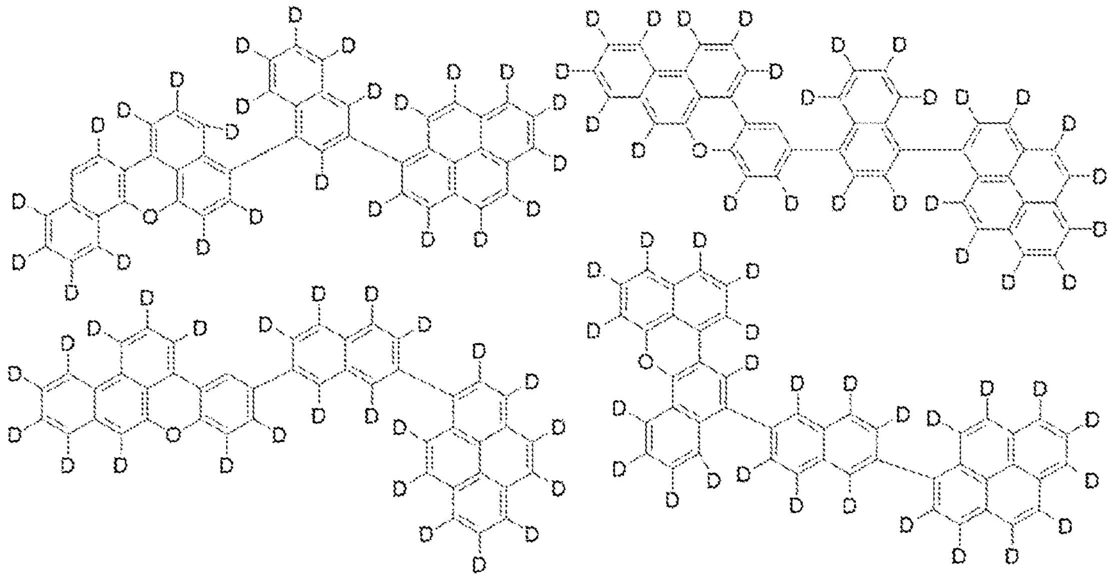

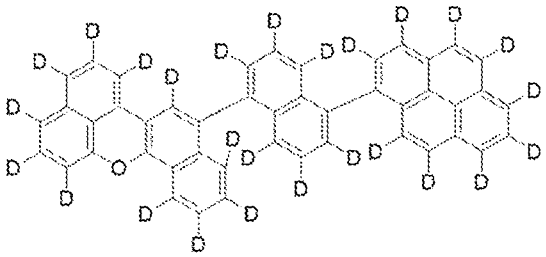

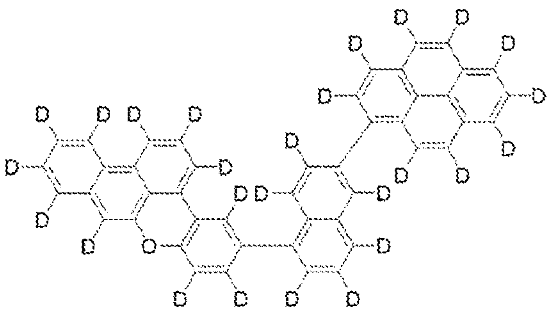

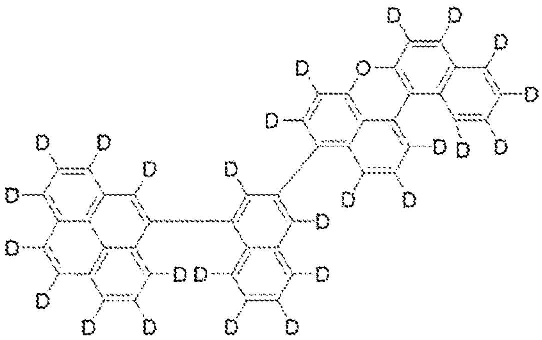

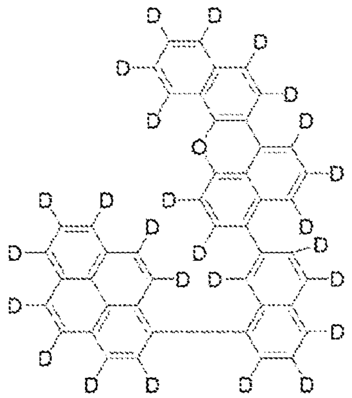

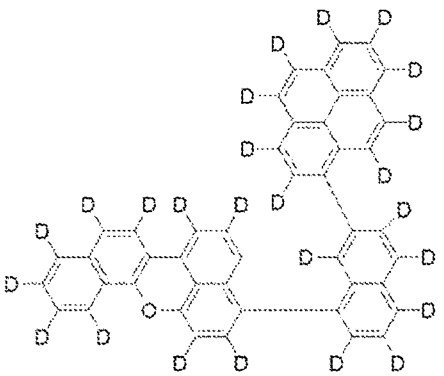

[Formula 142]

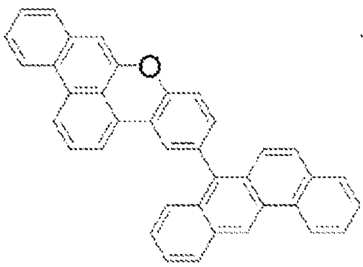
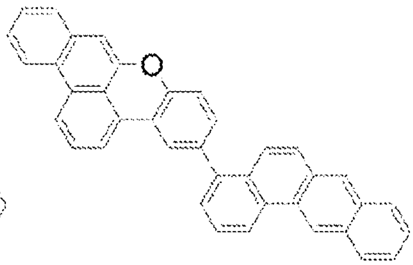
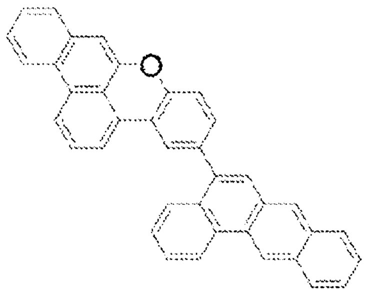

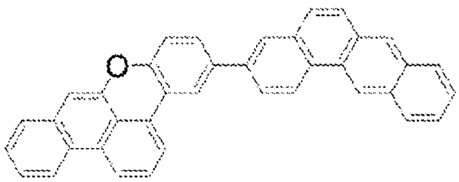
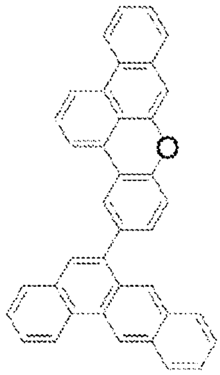
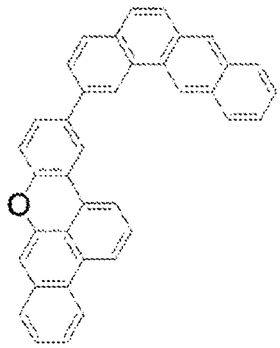

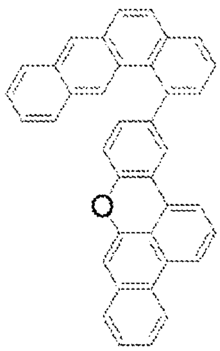
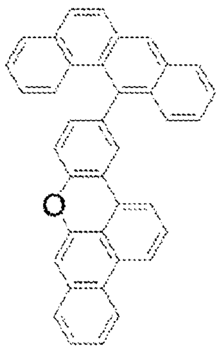
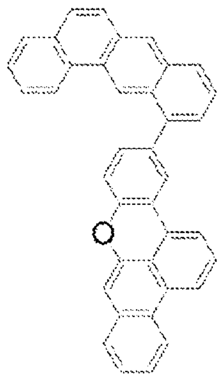

[Formula 143]

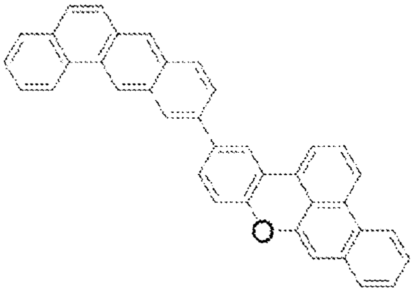

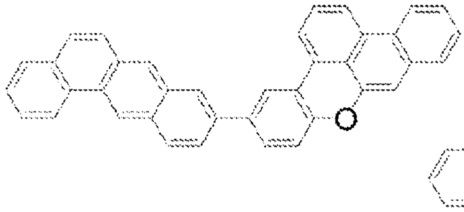

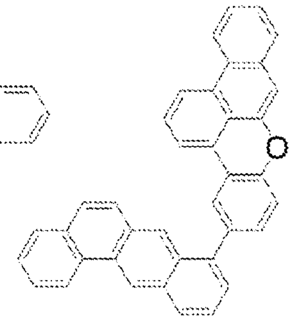

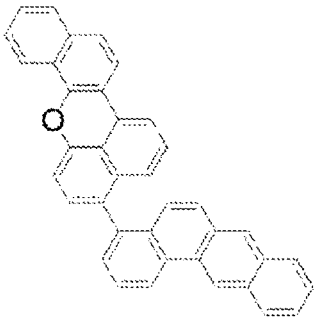

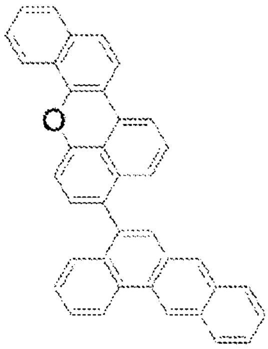

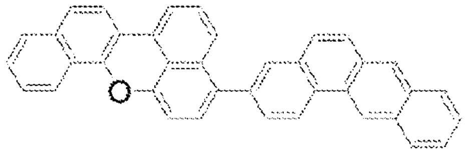

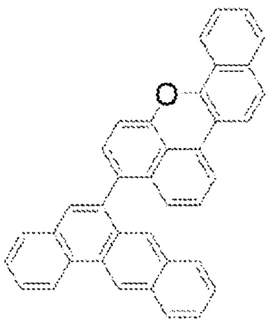

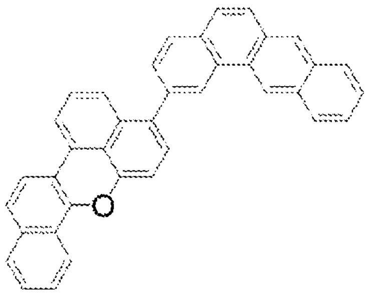

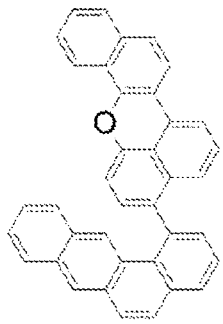

[Formula 144]

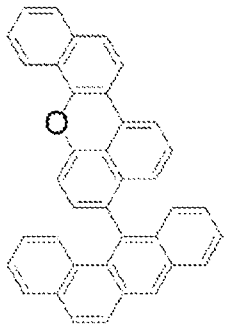
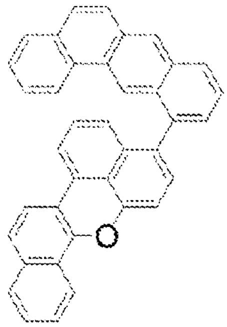
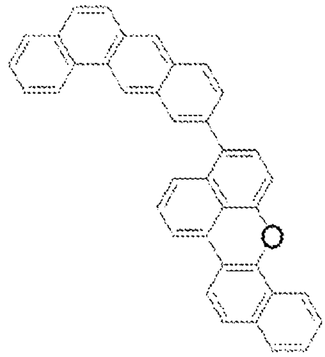
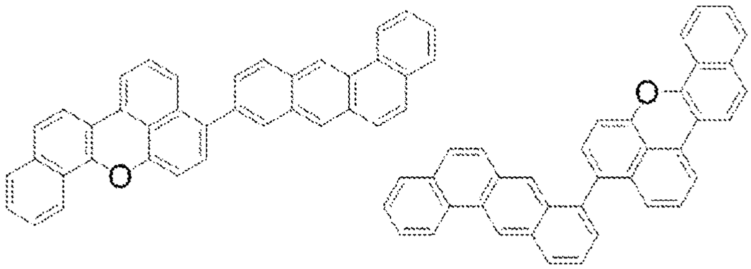
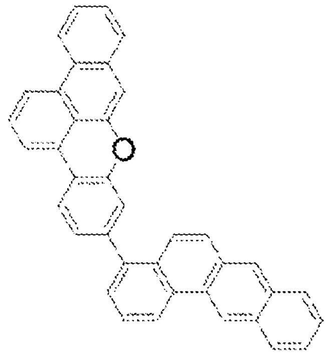
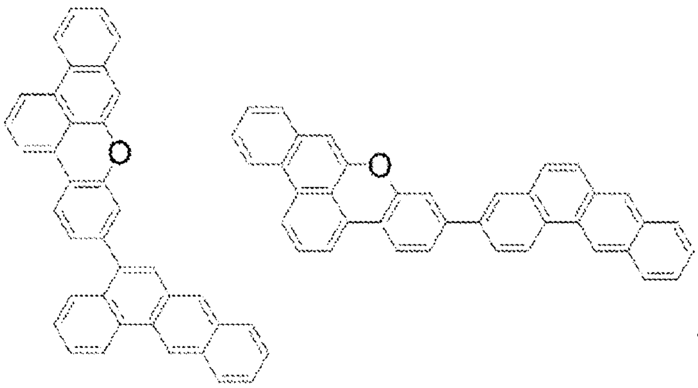
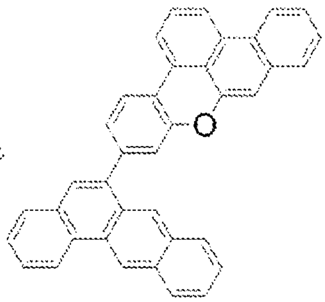

[Formula 145]

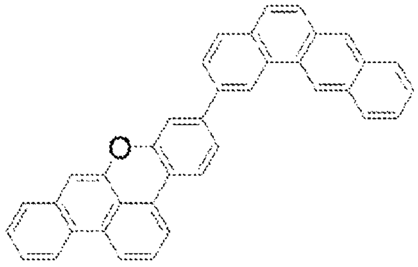

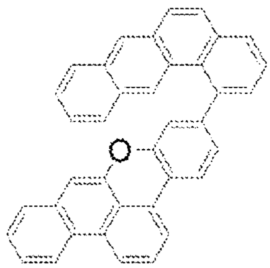

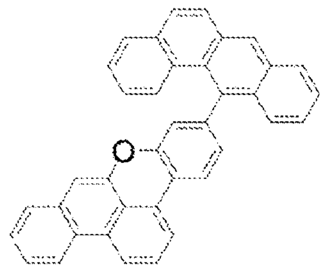

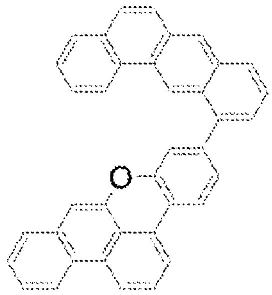

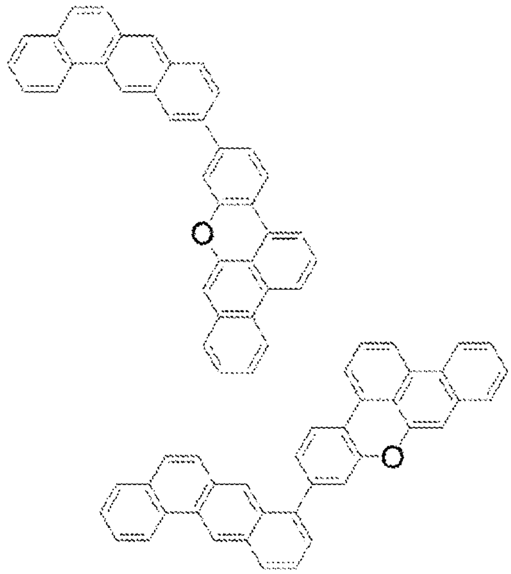

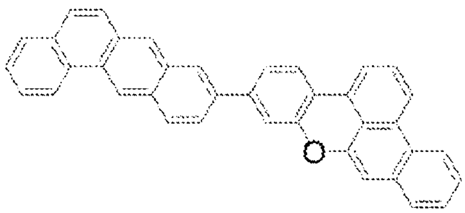

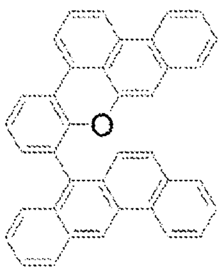

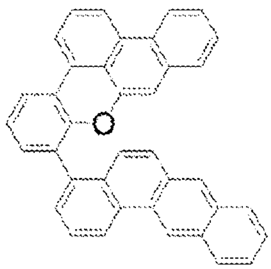

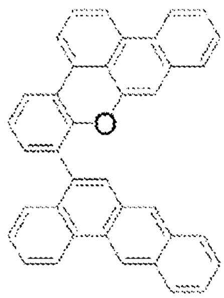

[Formula 146]

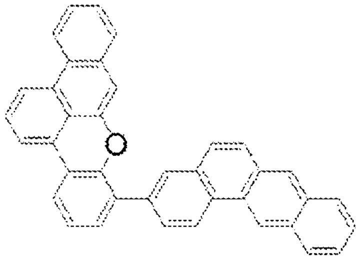
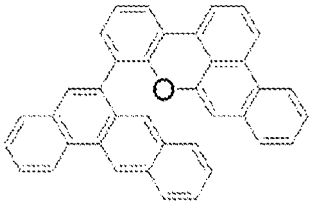
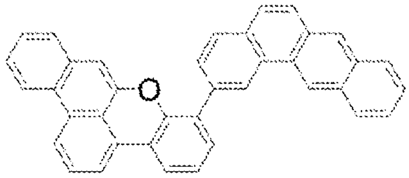
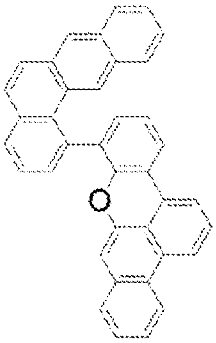
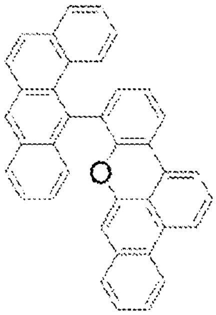
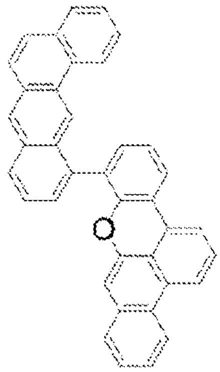
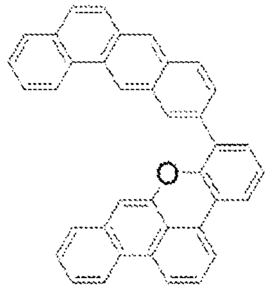
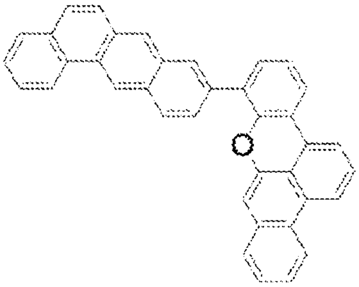
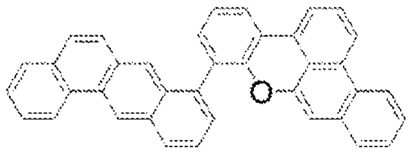
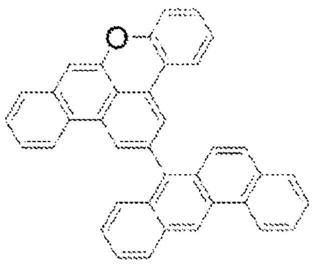
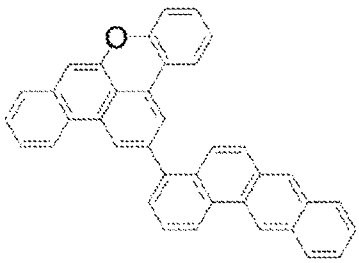
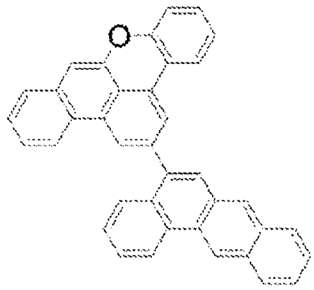

[Formula 147]

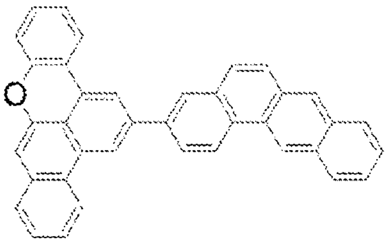
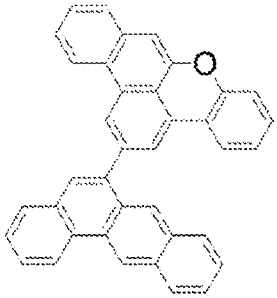
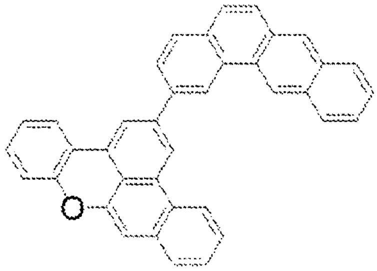
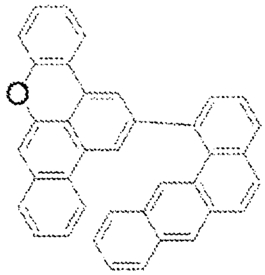
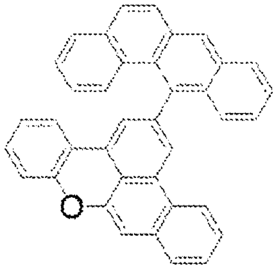
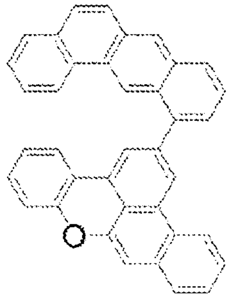
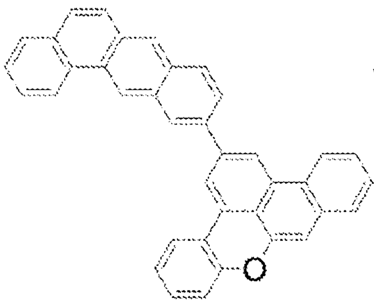
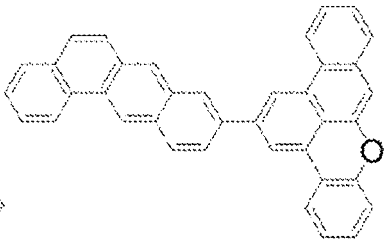
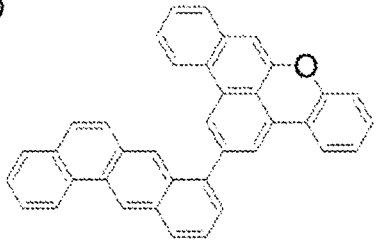

[Formula 148]

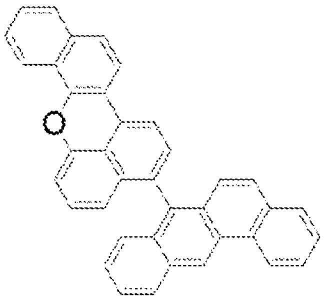
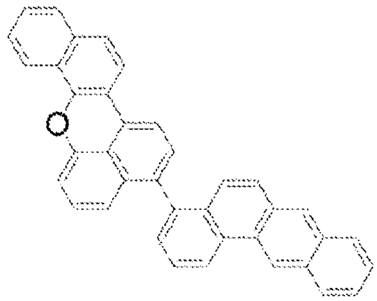
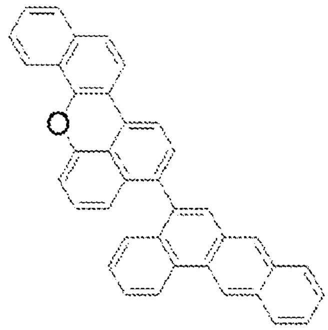

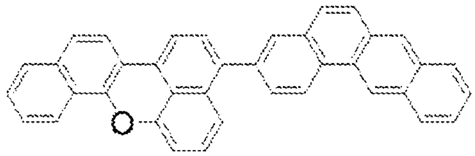
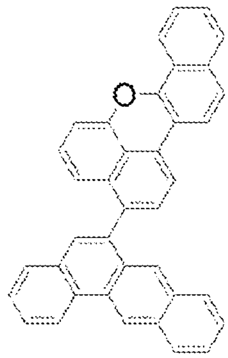
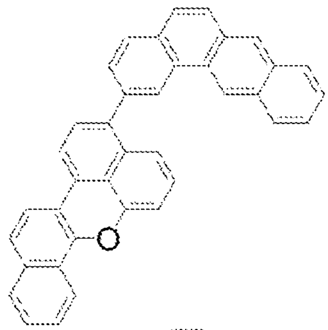

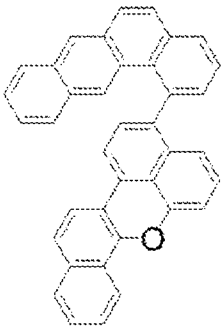
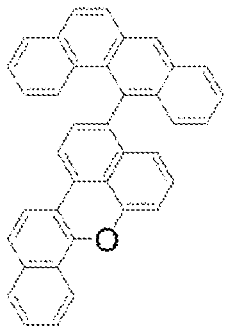
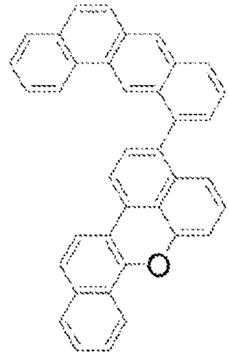

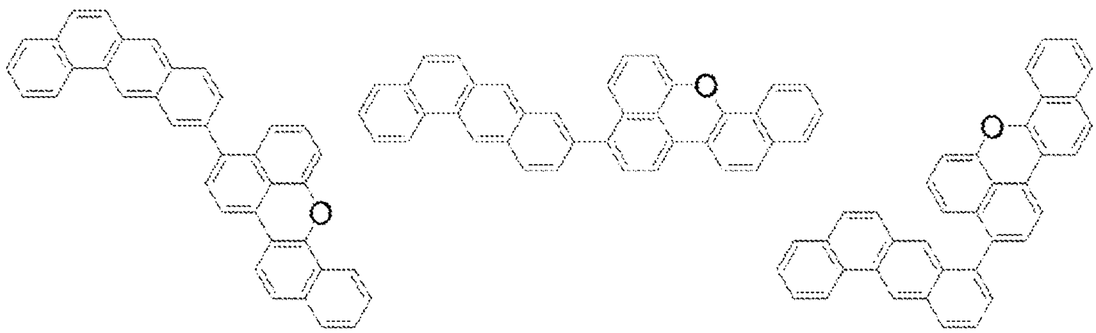

[Formula 149]

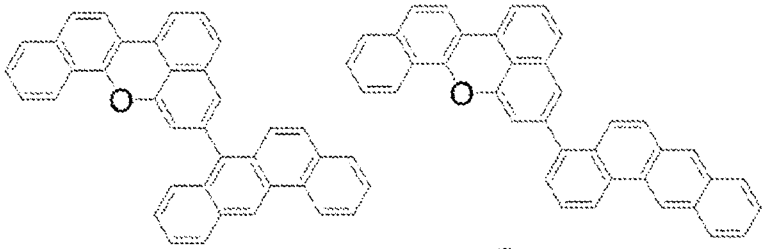

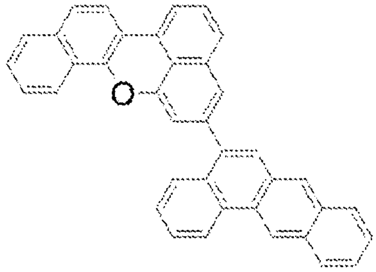

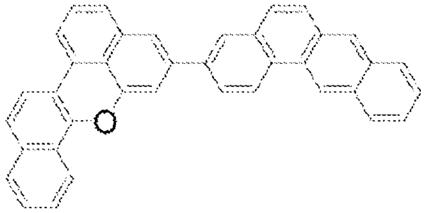

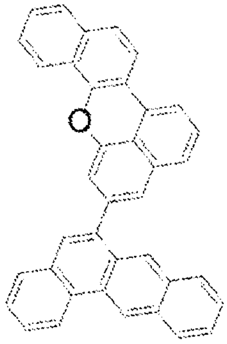

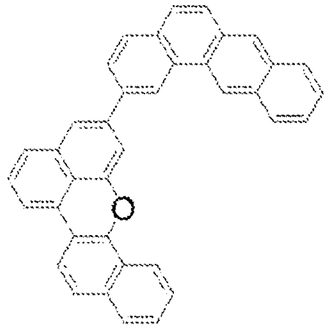

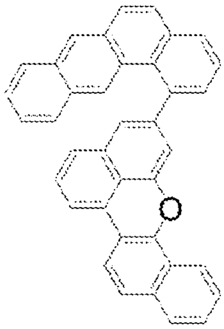

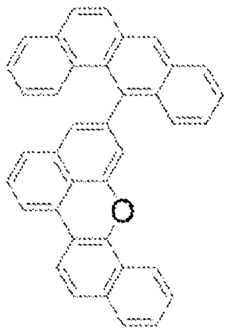

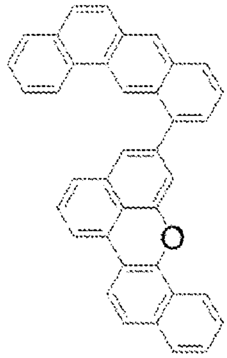

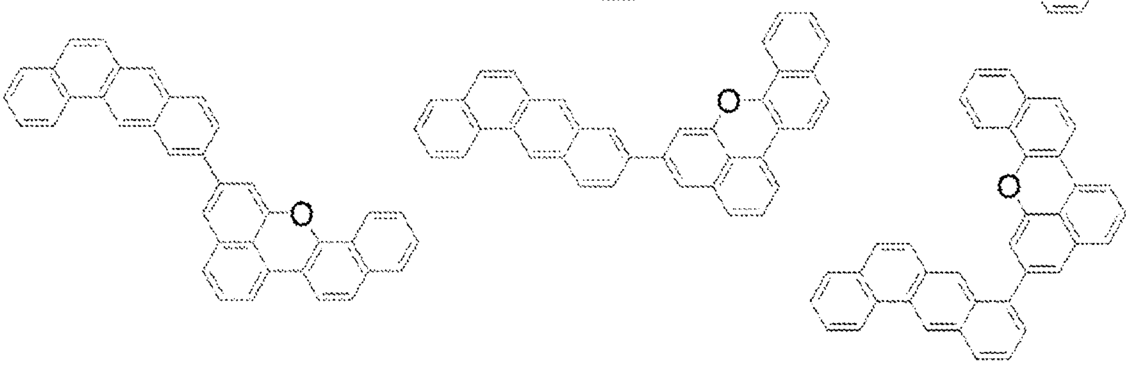

[Formula 150]

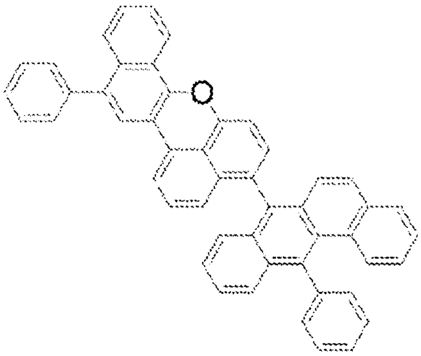

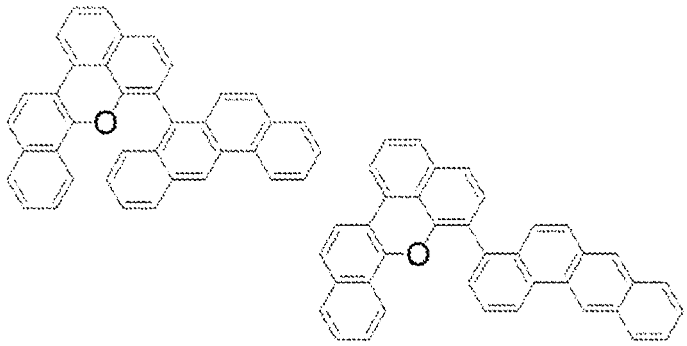

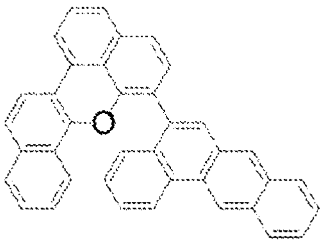

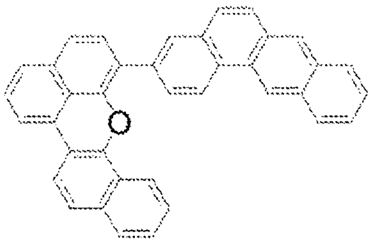

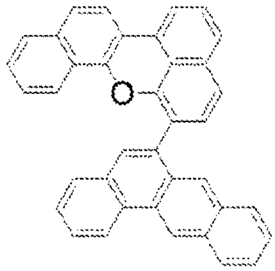

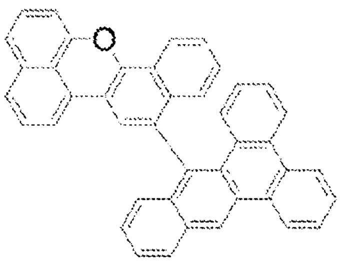

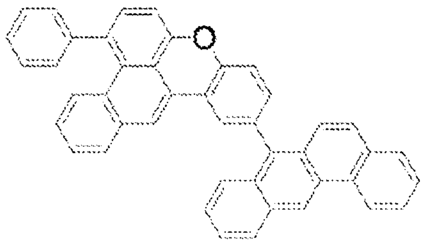

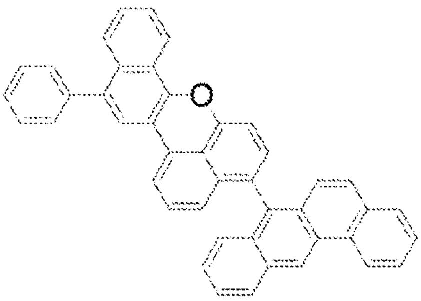

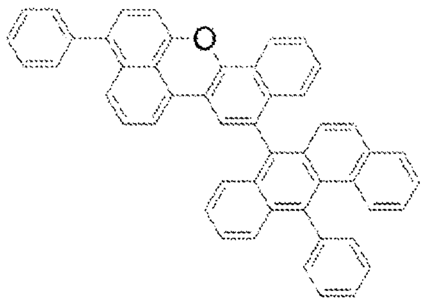

[Formula 151]

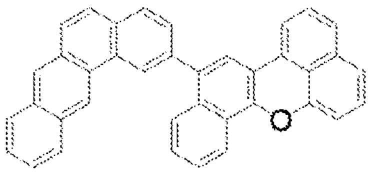

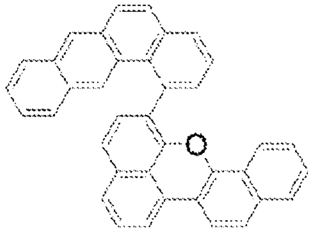

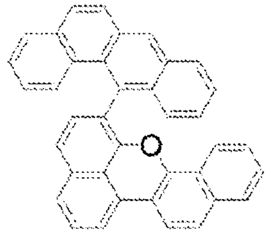

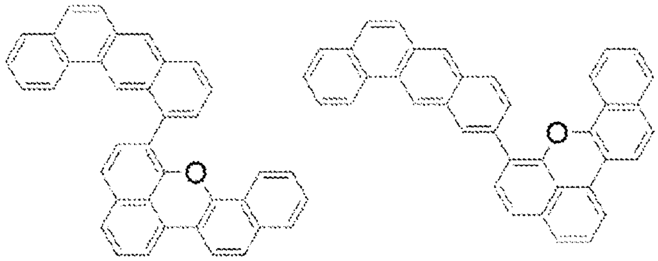

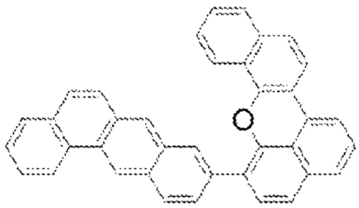

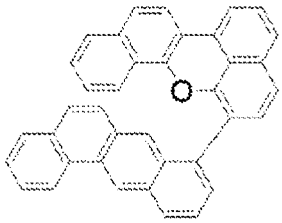

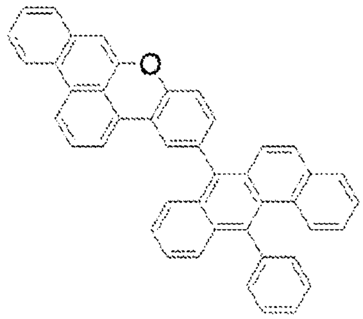

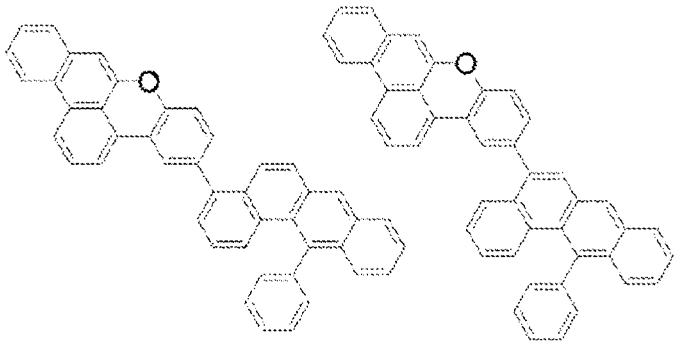

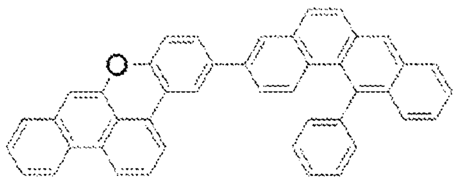

[Formula 152]

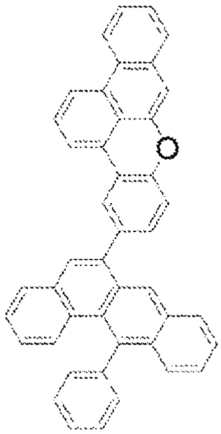
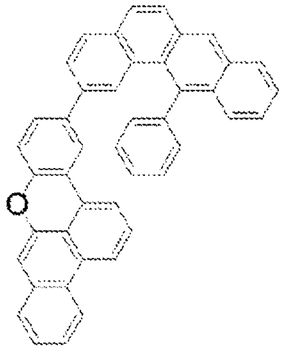
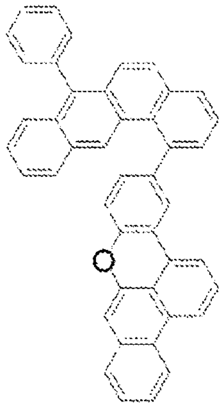
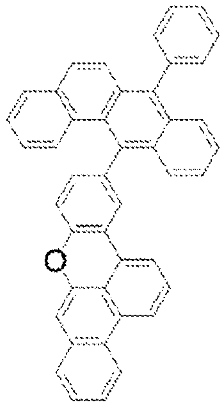
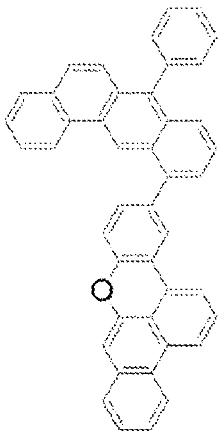
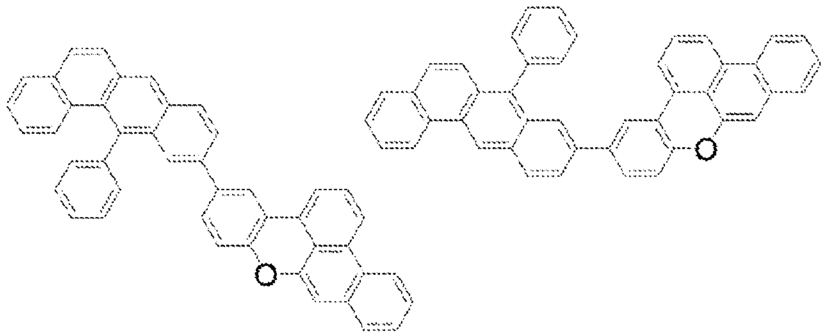
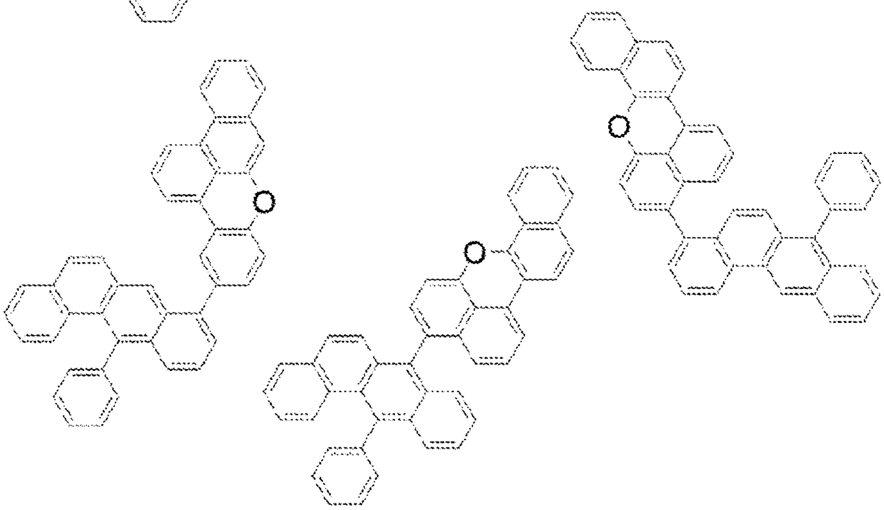
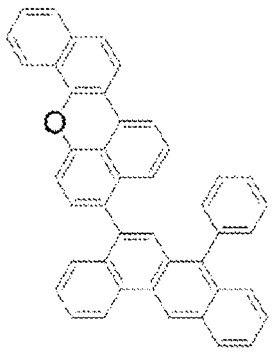

[Formula 153]

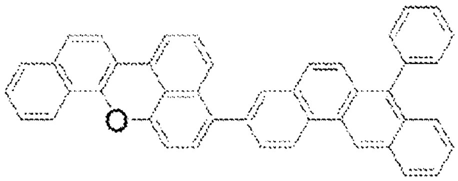

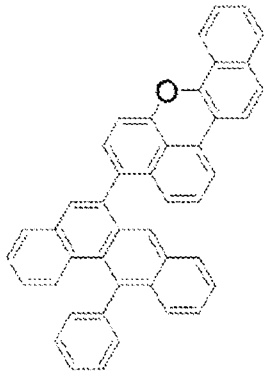

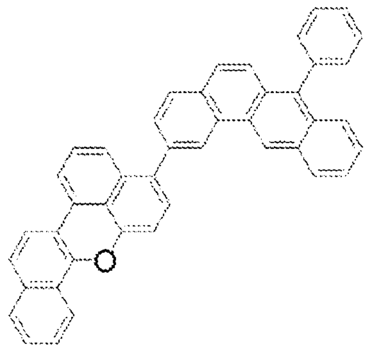

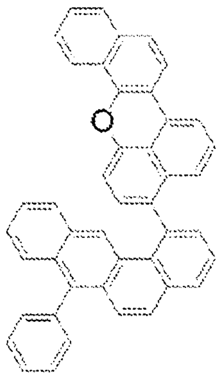

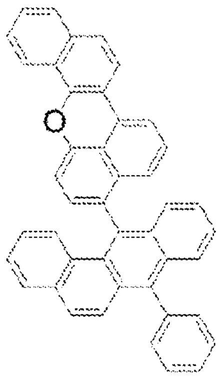

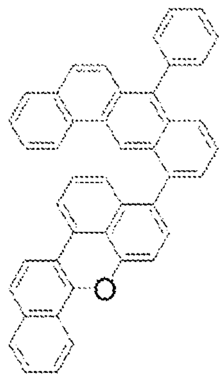

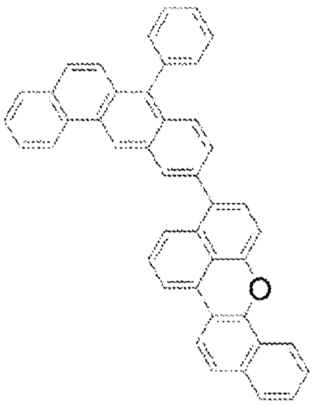

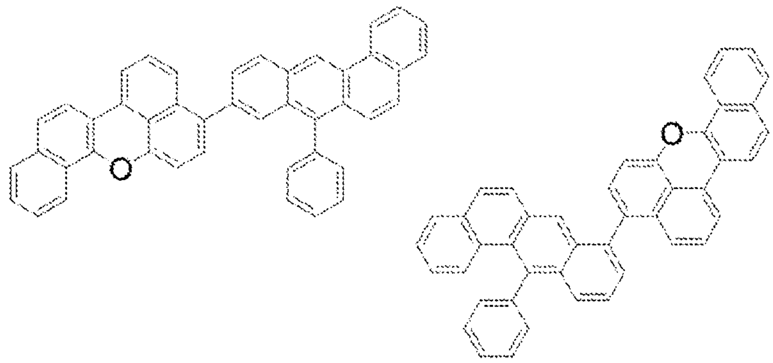

[Formula 154]

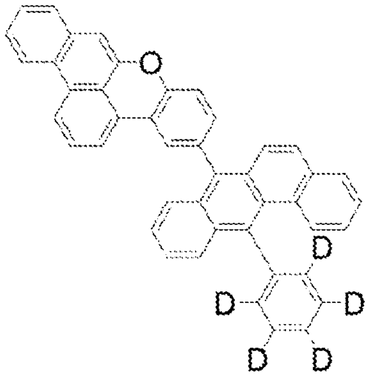

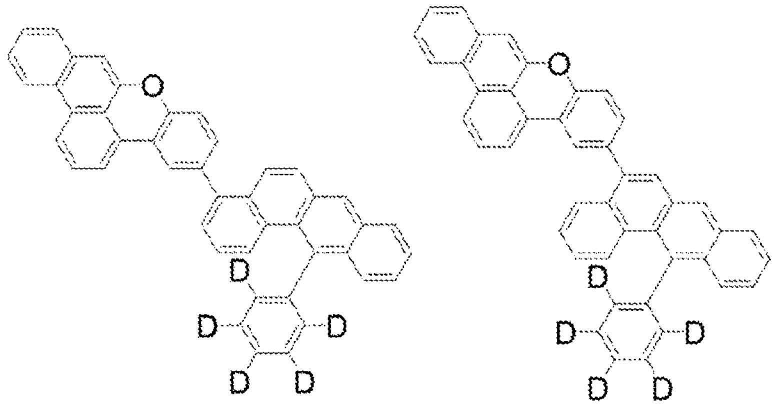

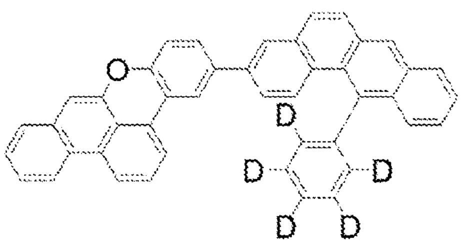

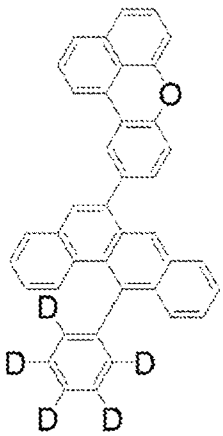

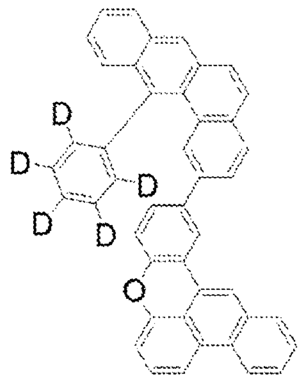

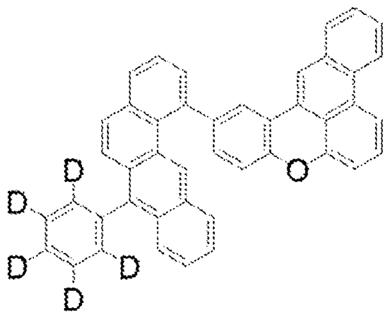

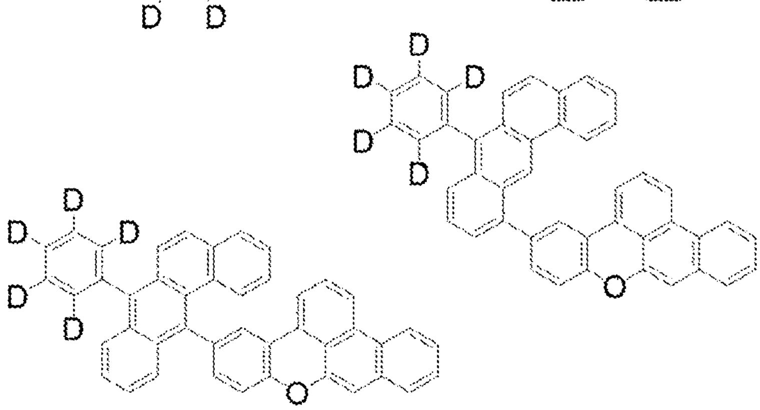

[Formula 155]

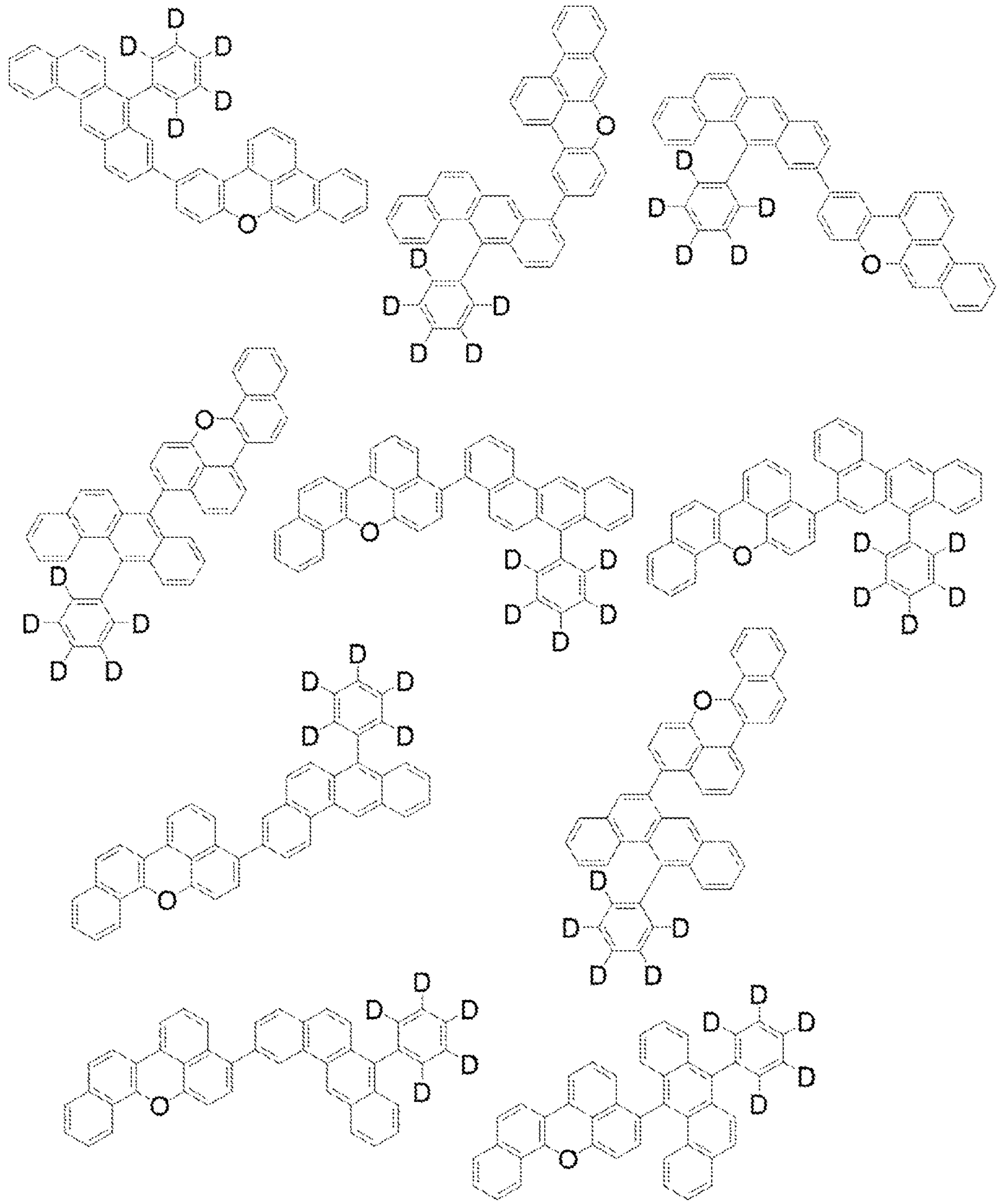

[Formula 156]

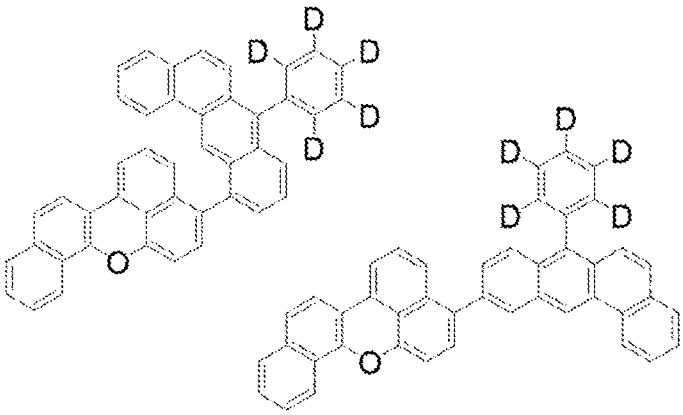

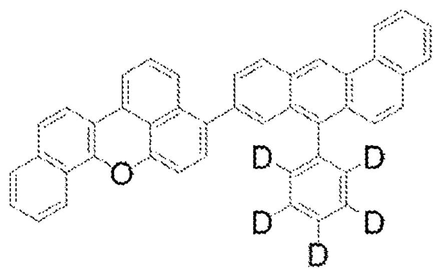

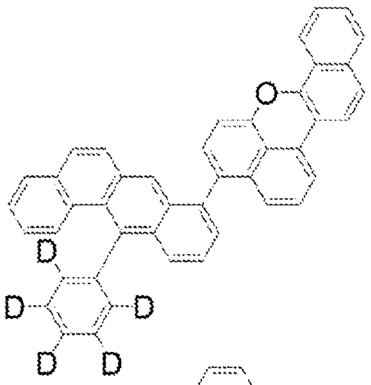

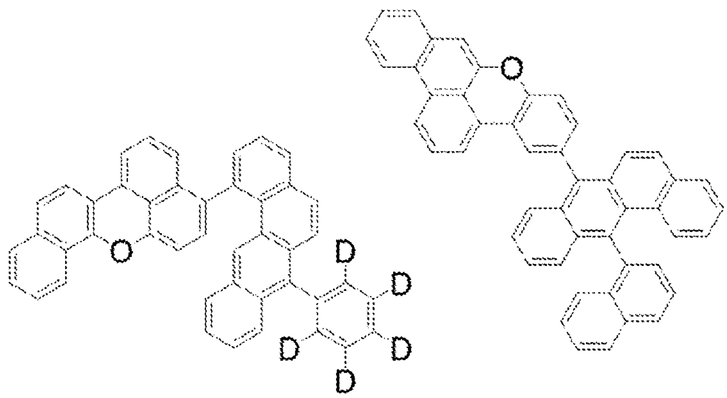

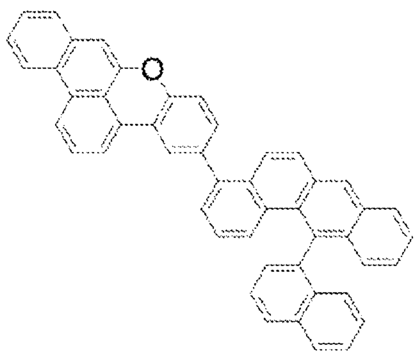

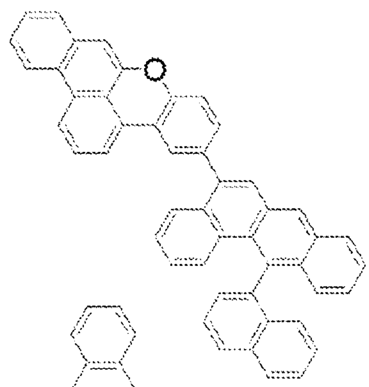

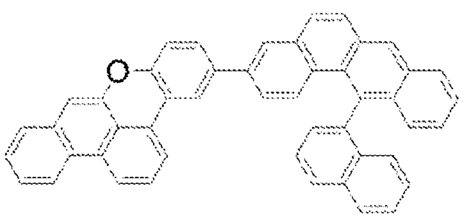

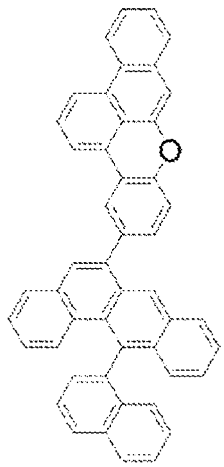

[Formula 157]

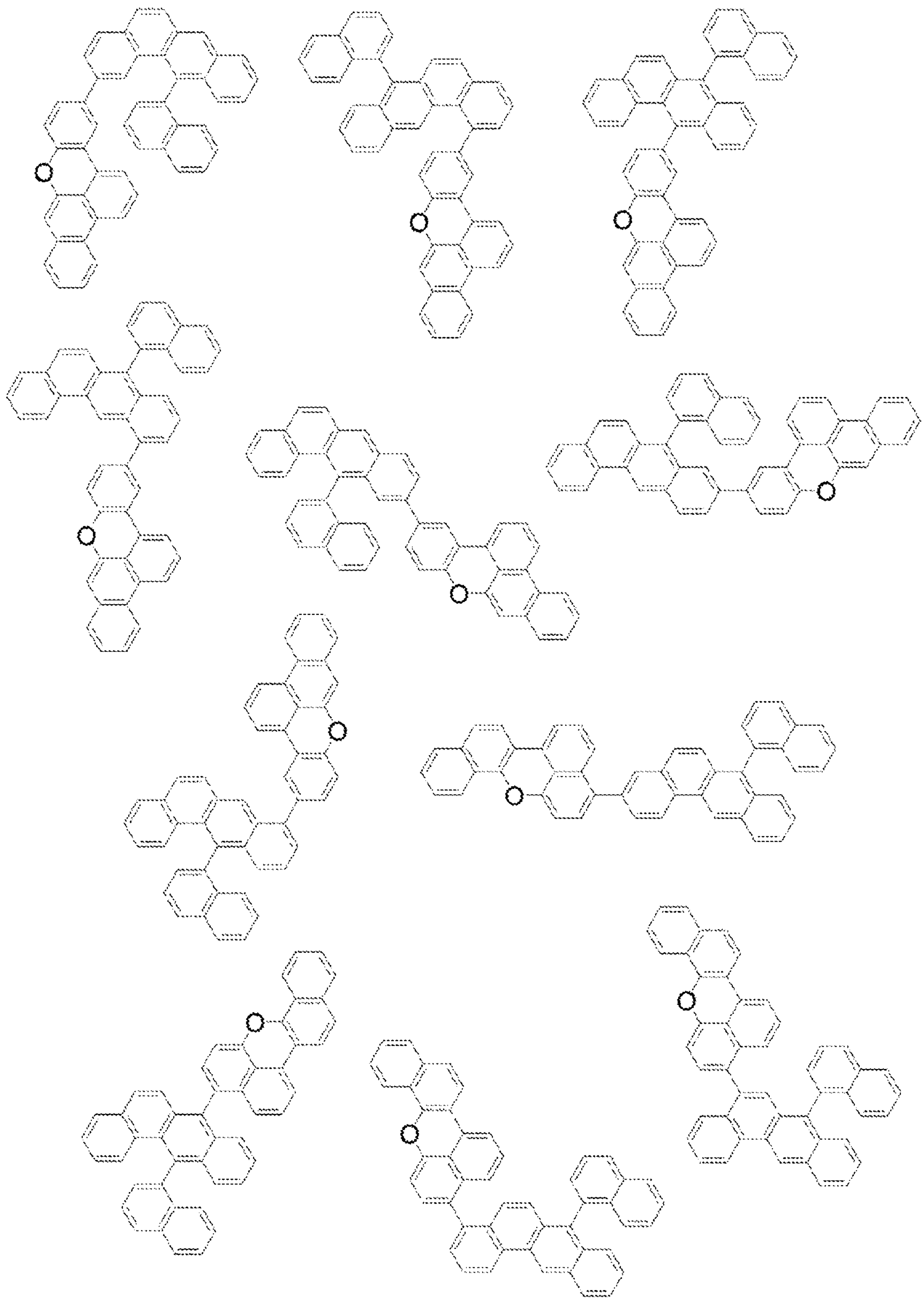

[Formula 158]

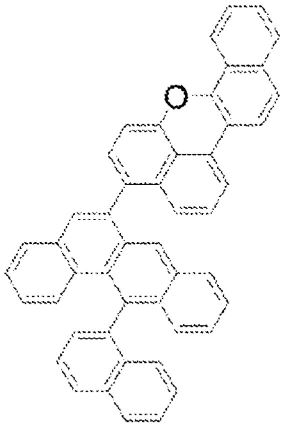

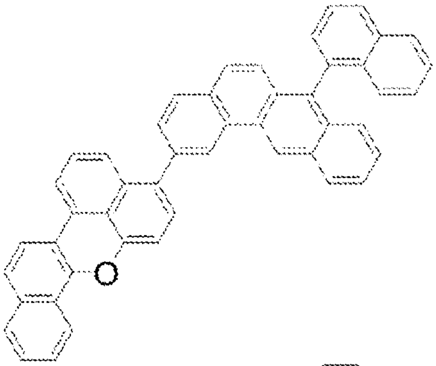

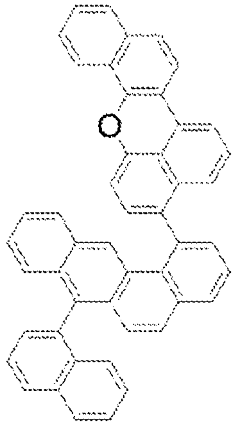

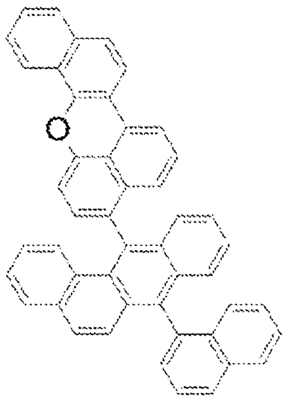

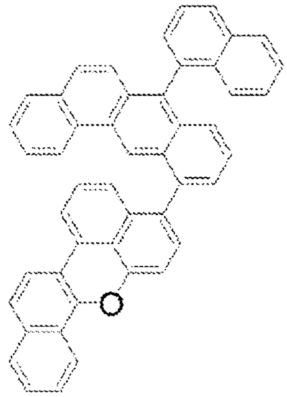

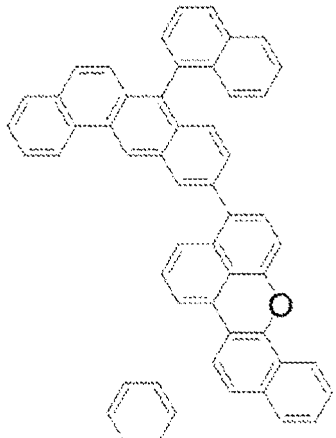

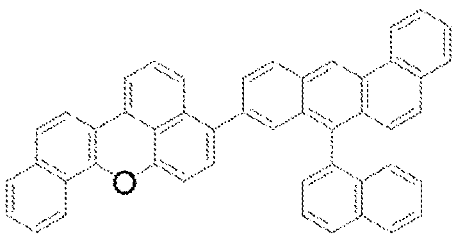

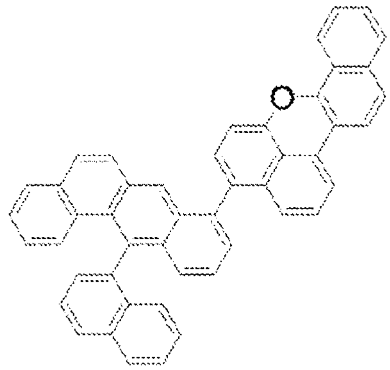

[Formula 159]

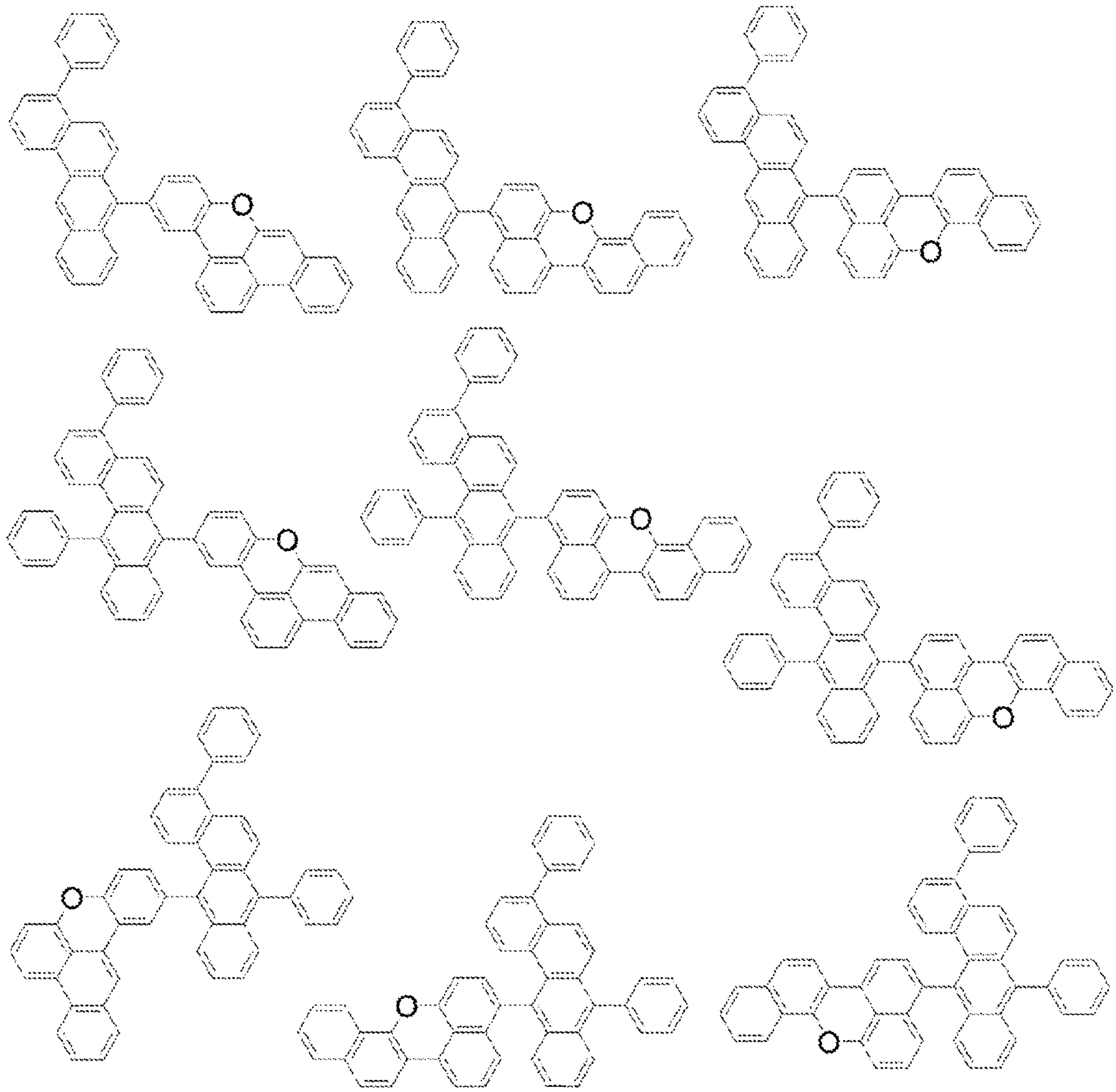

[Formula 160]

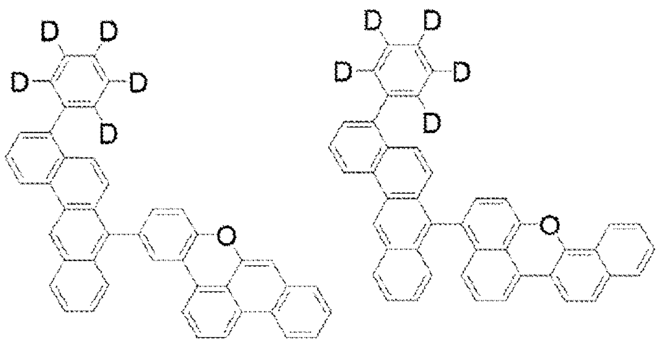

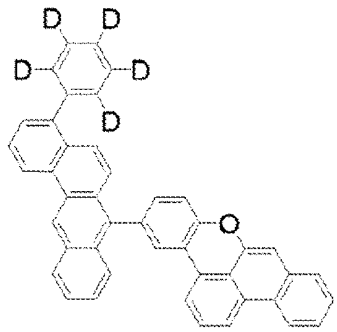

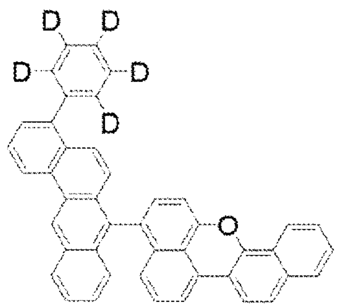

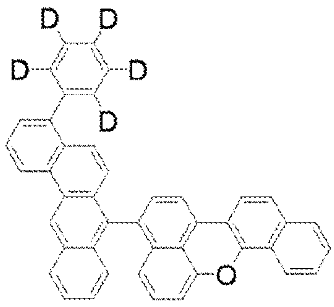

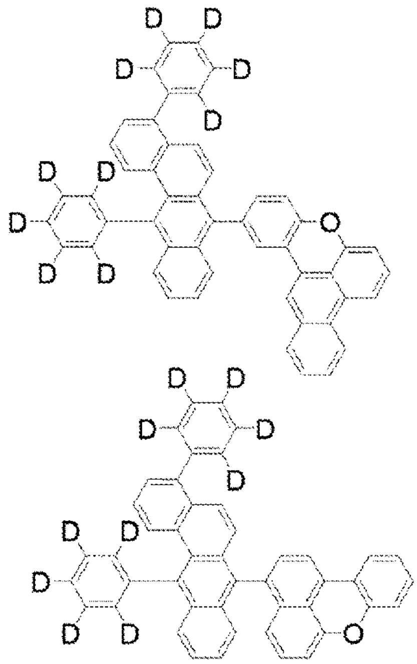

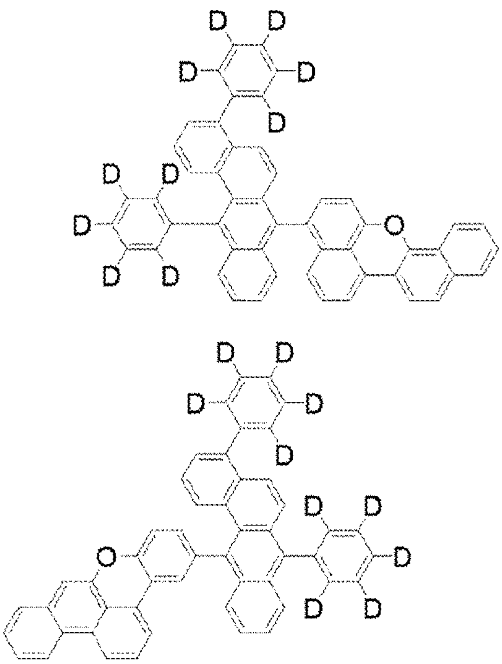

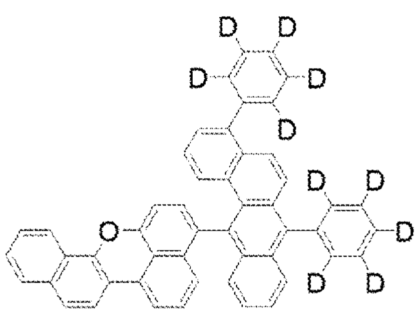

[Formula 161]

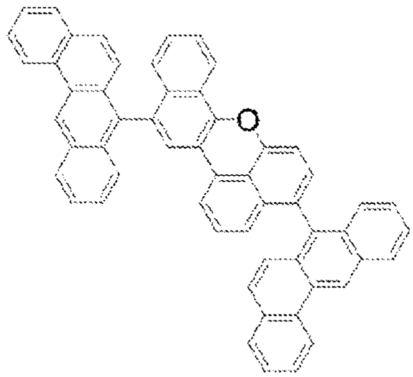
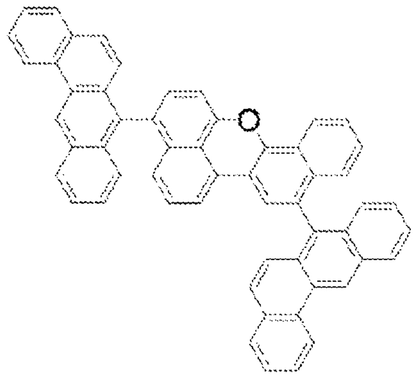
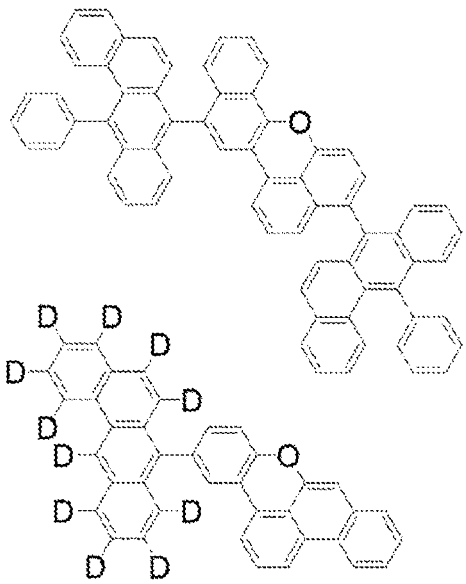
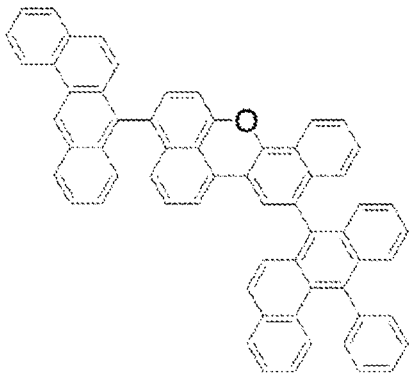
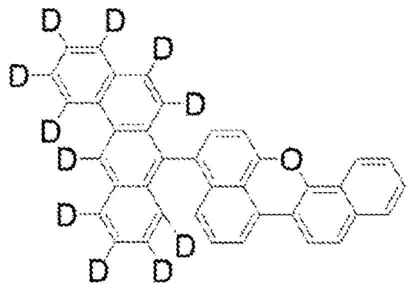
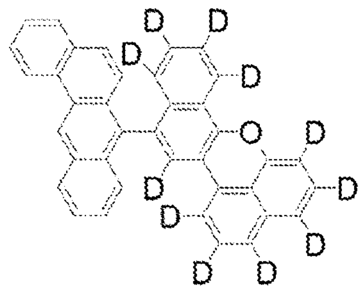
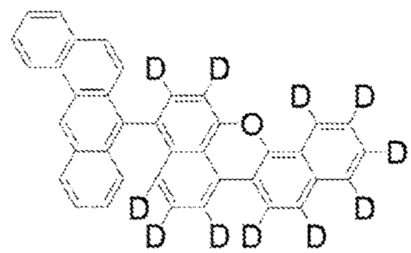
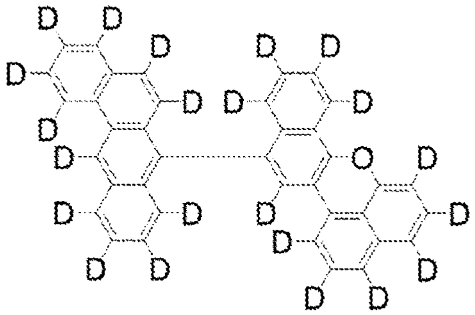
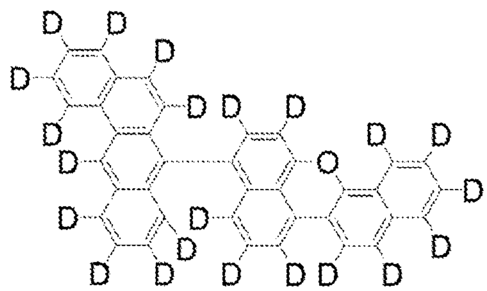

[Formula 162]

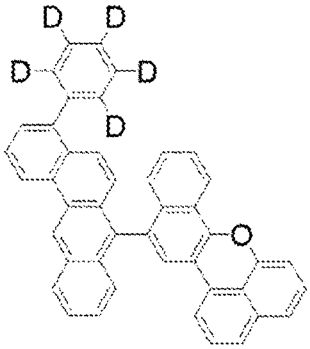
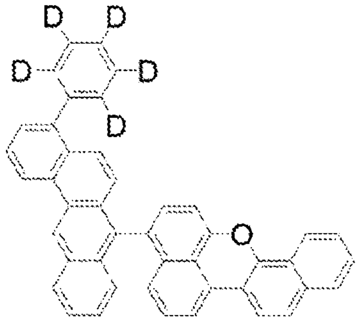
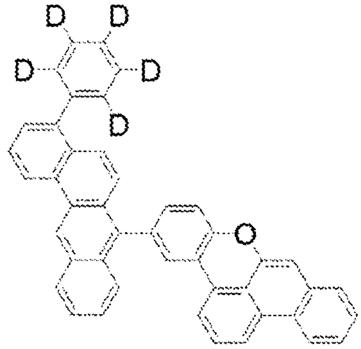
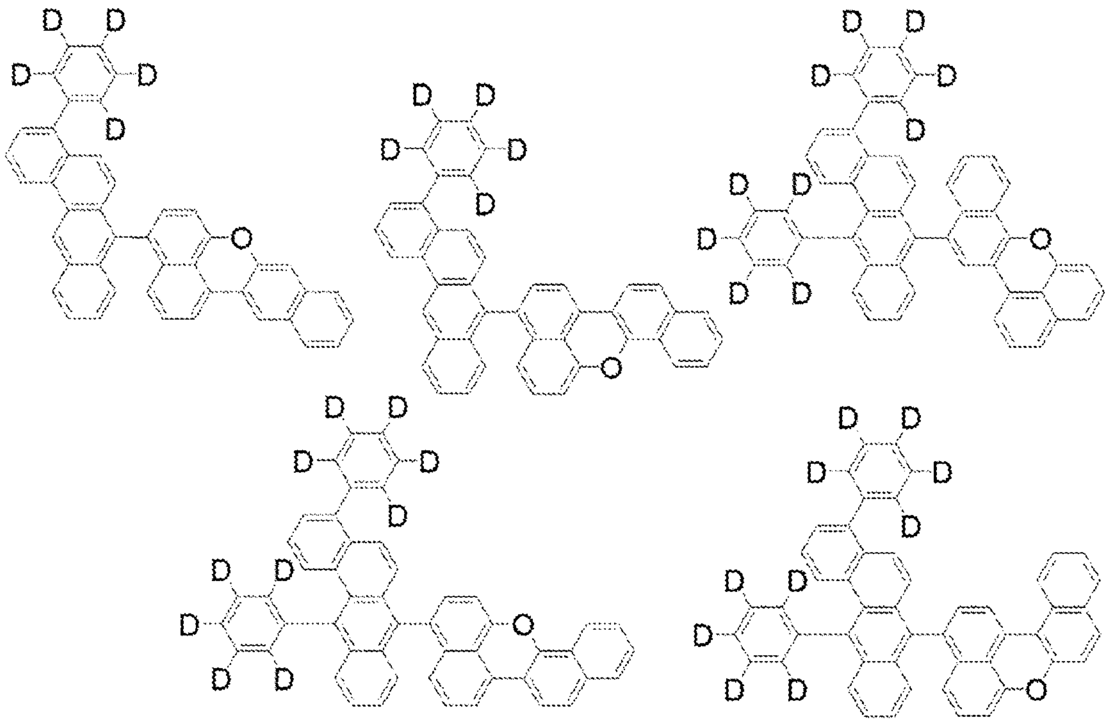
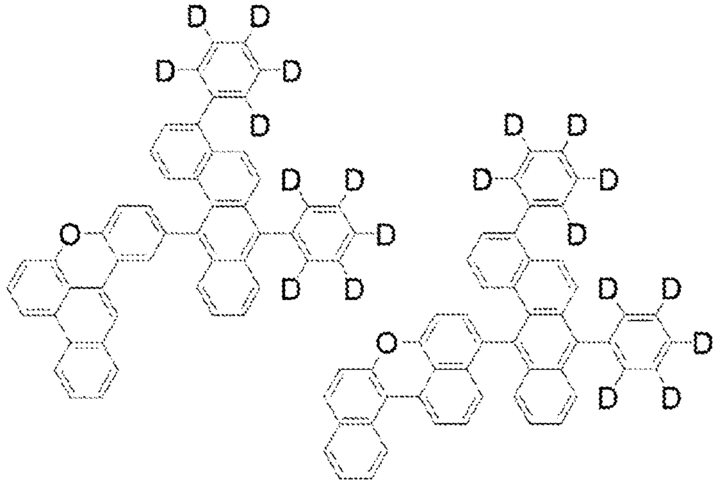
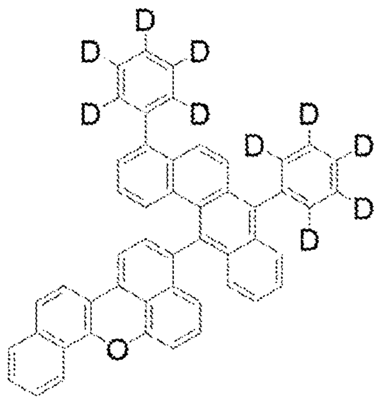

[Formula 163]

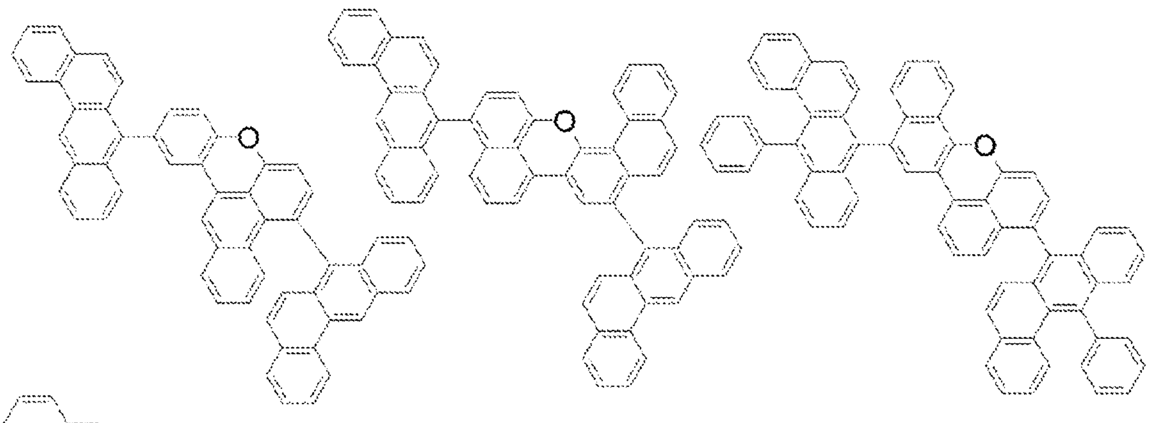

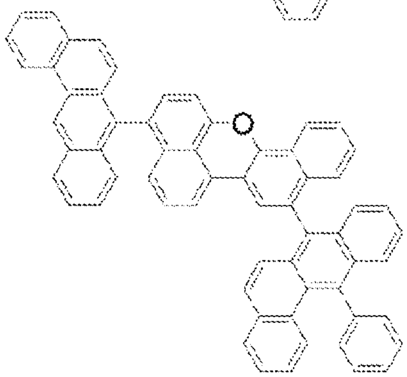

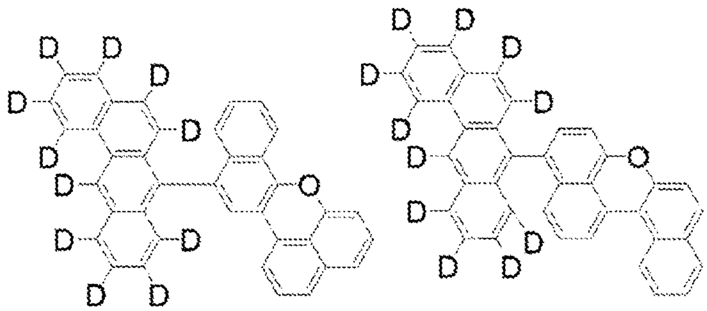

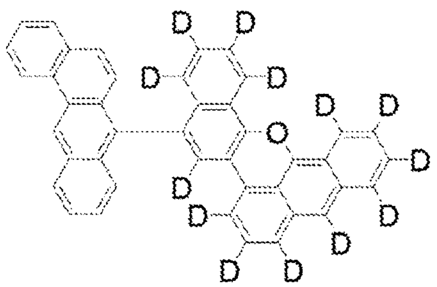

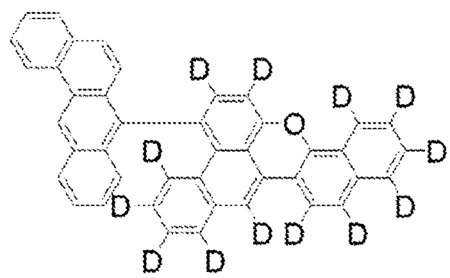

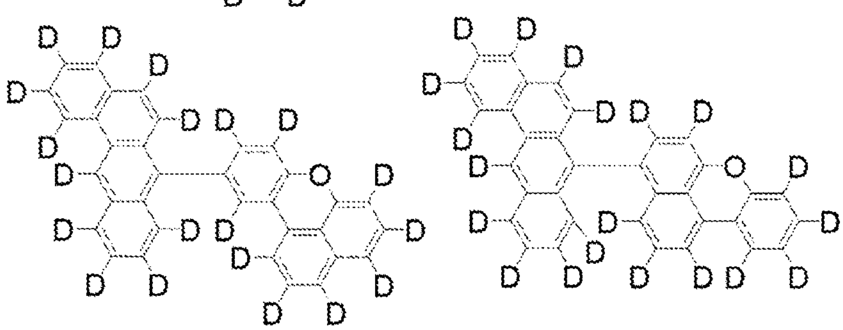

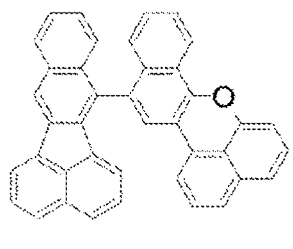

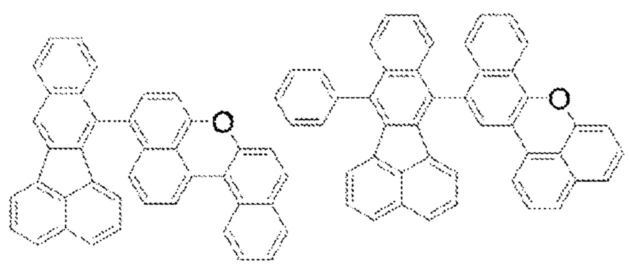

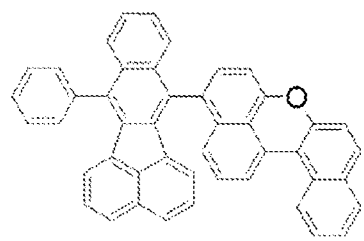

[Formula 164]

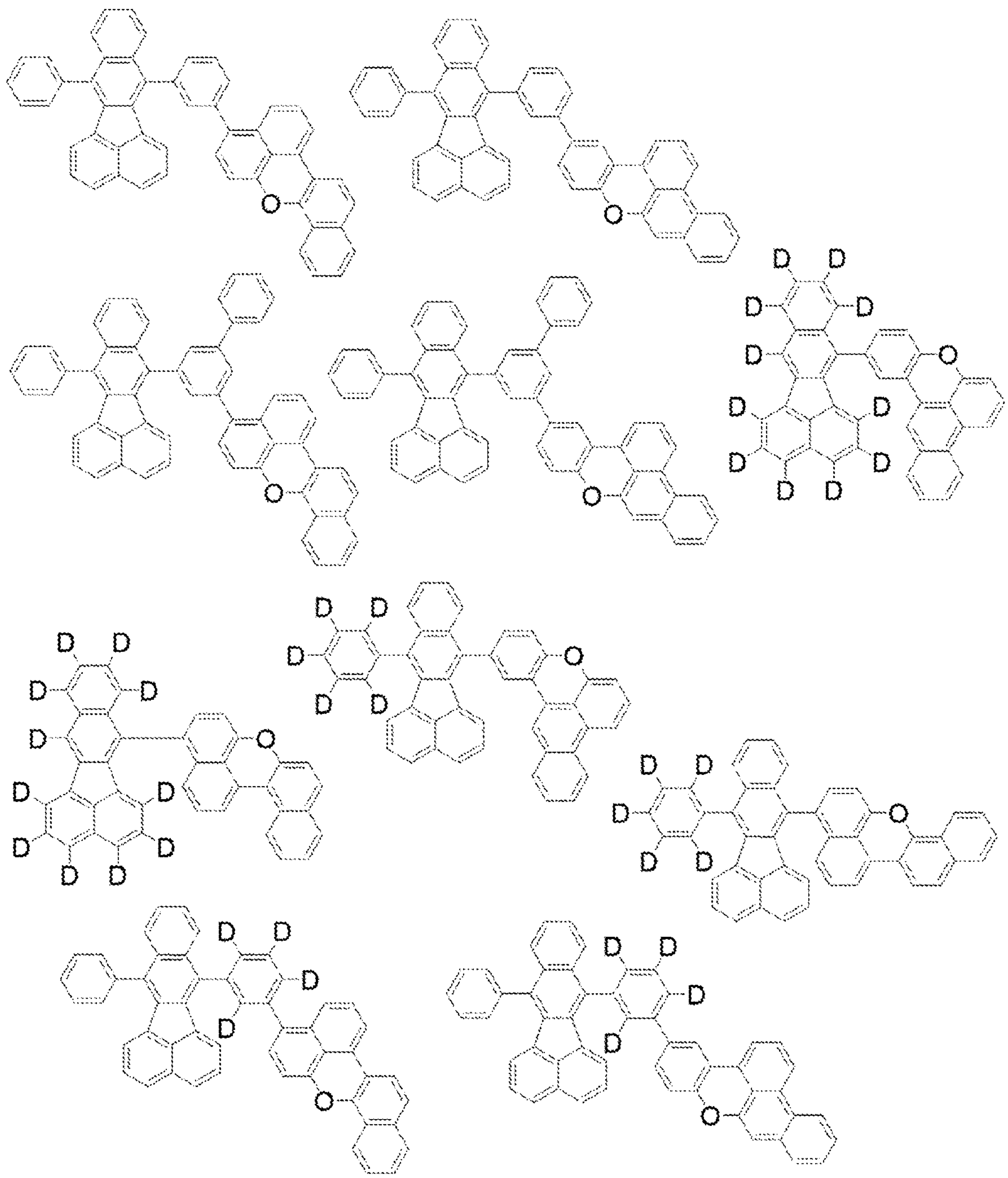

[Formula 165]

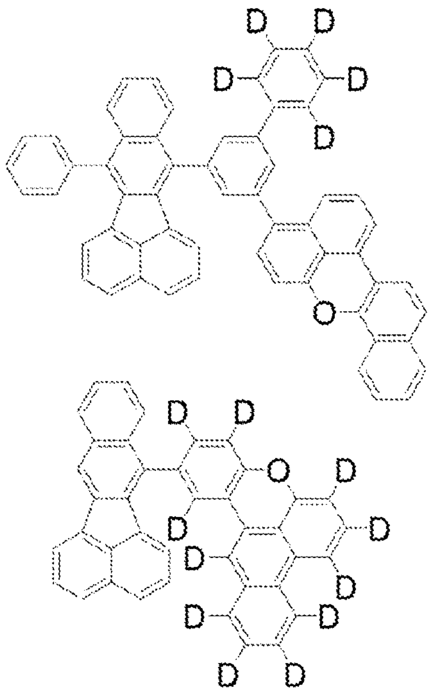

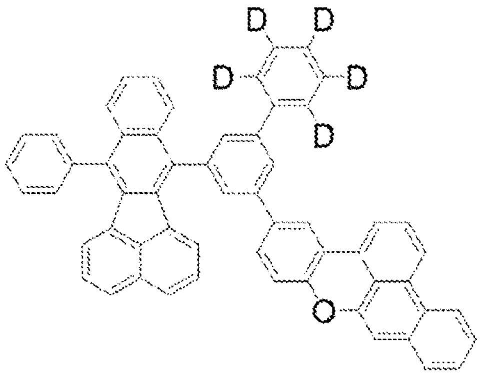

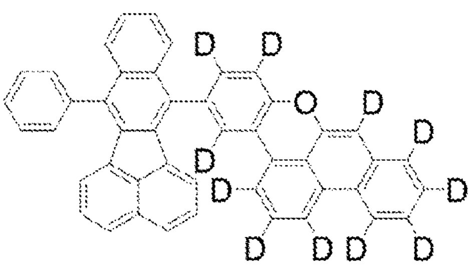

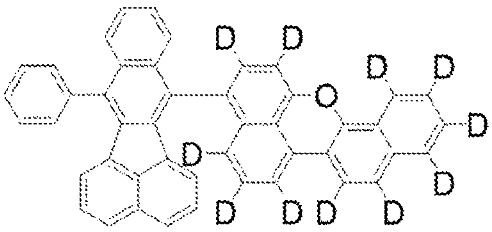

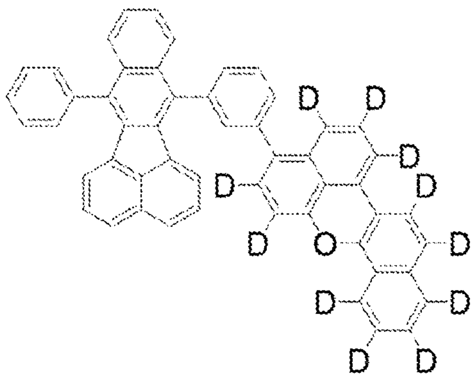

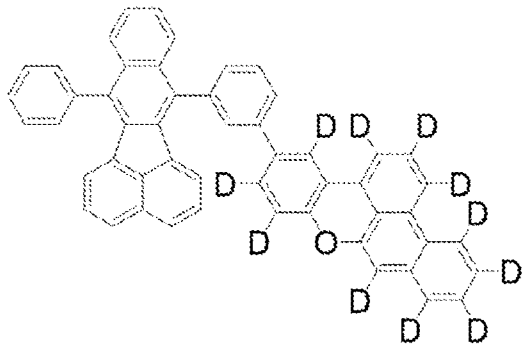

[Formula 166]

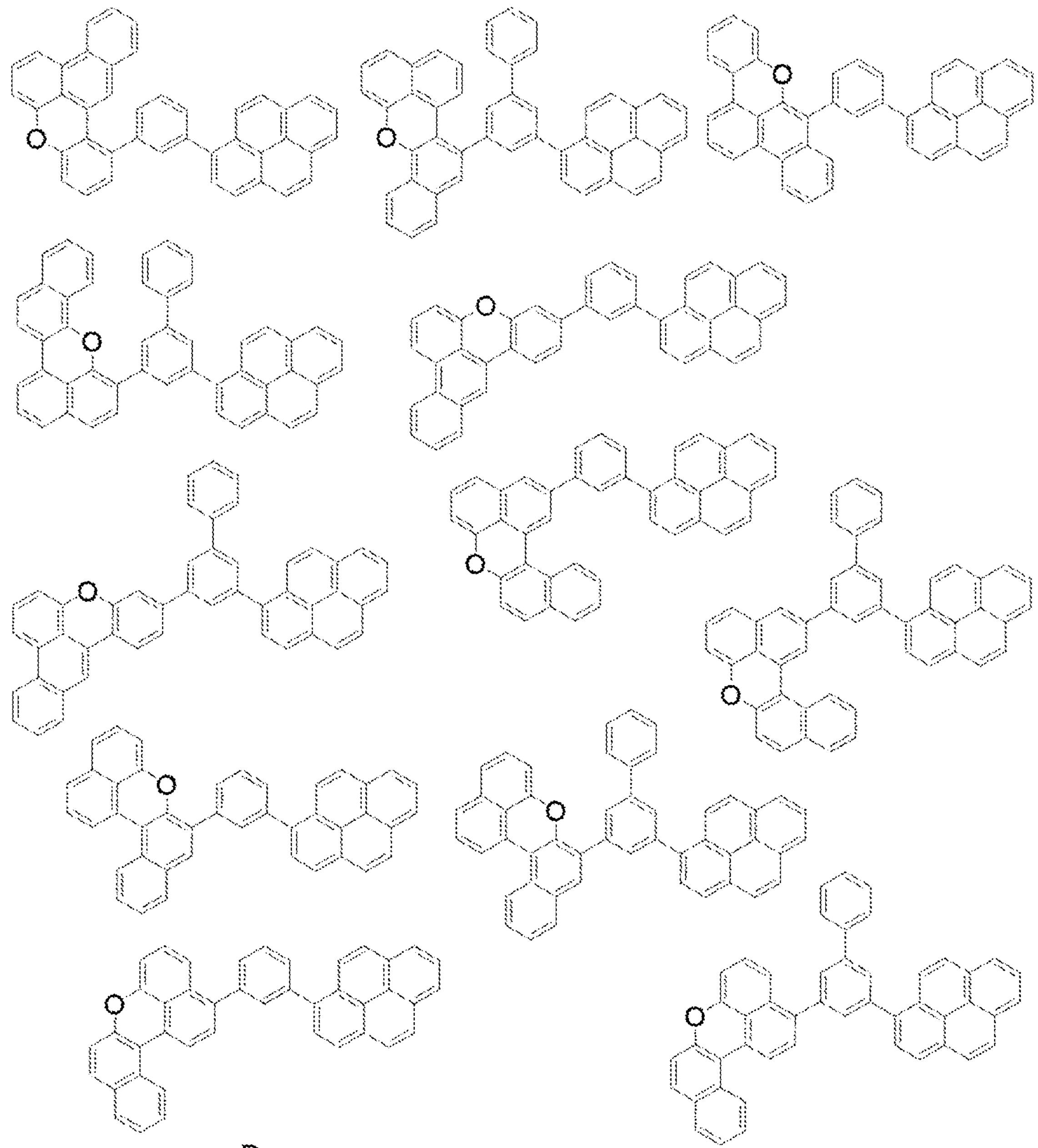

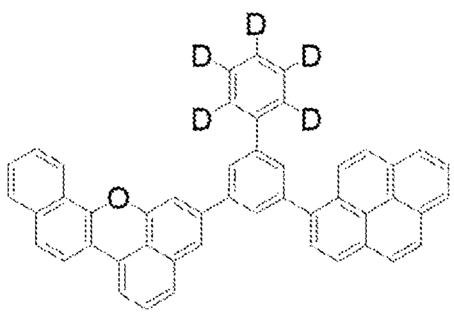

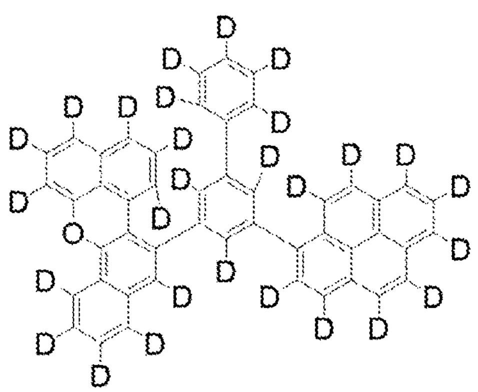

[Formula 167]

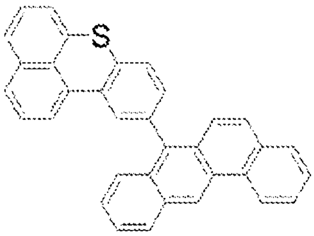
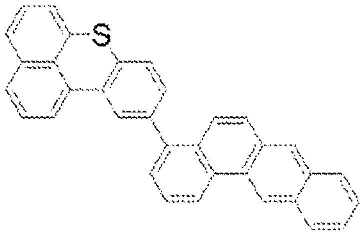
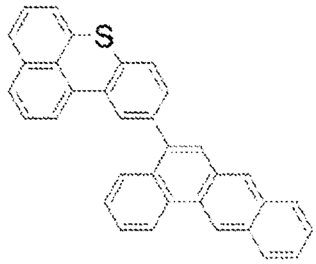
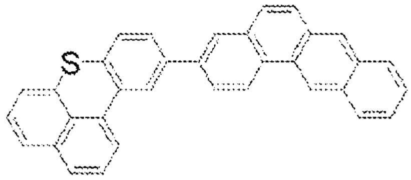
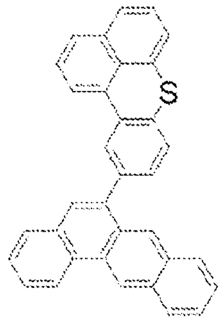
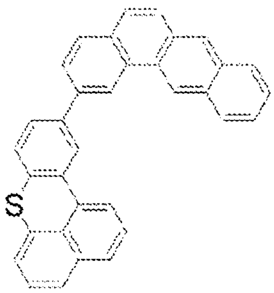
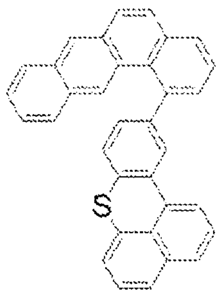
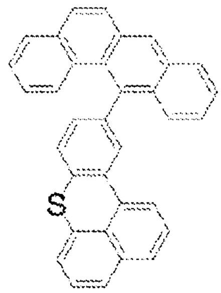
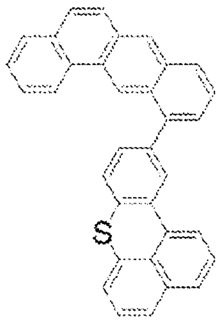
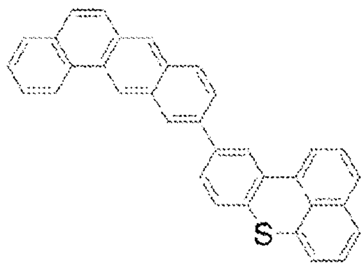

[Formula 168]

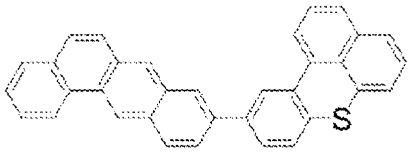

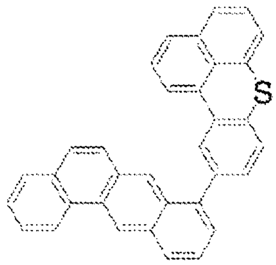

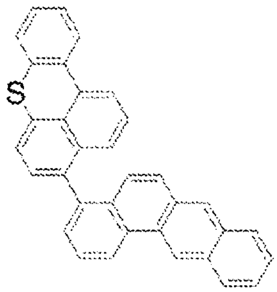

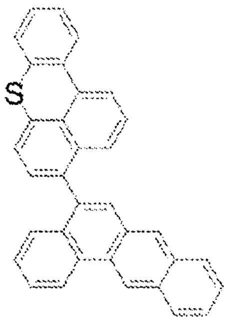

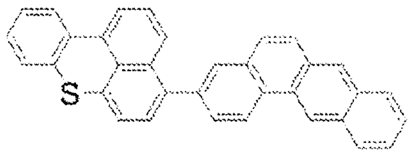

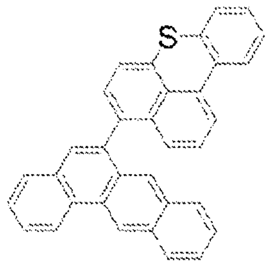

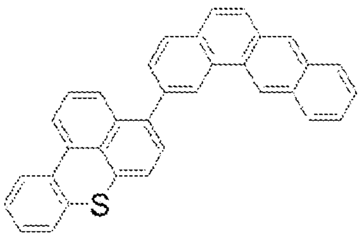

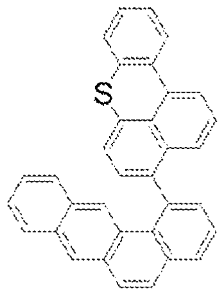

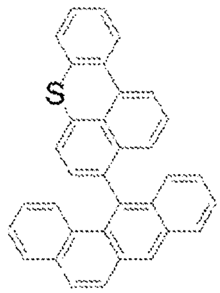

[Formula 169]

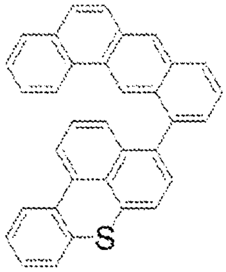
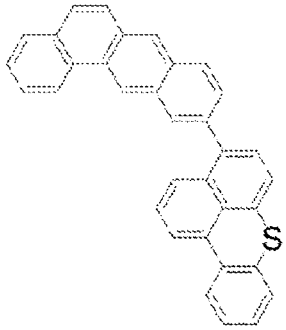
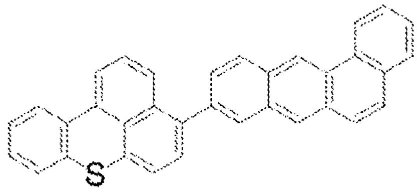
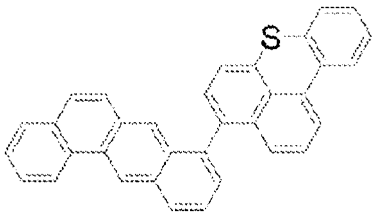
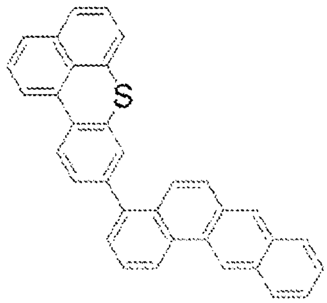
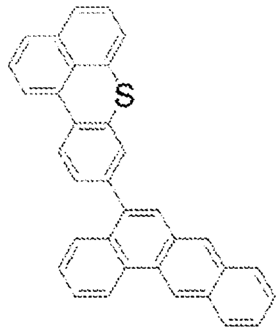
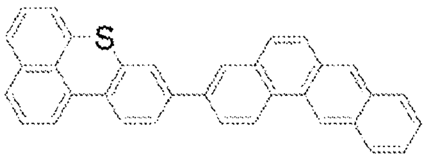
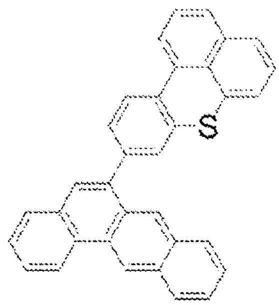

[Formula 170]

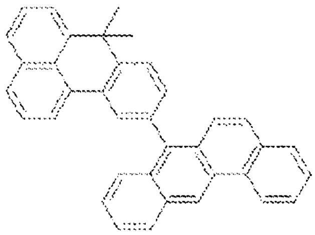

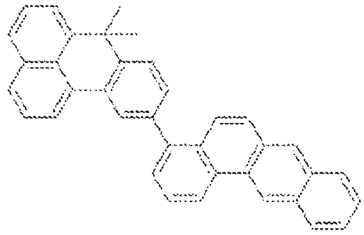

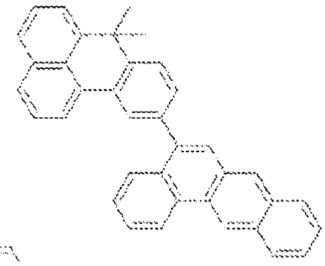

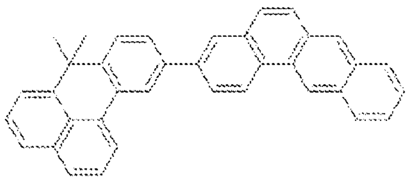

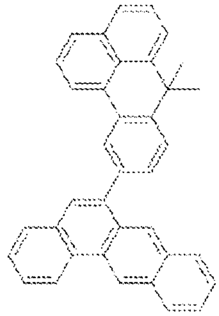

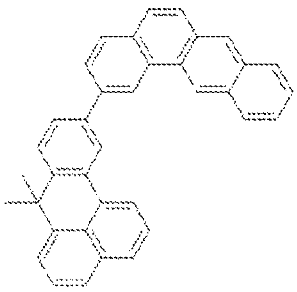

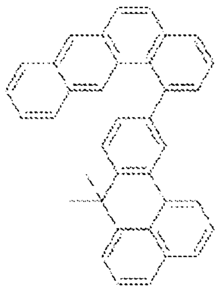

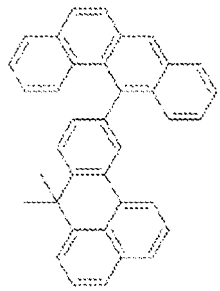

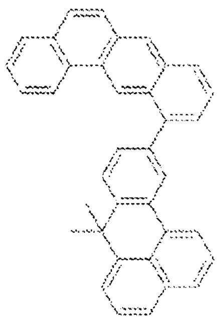

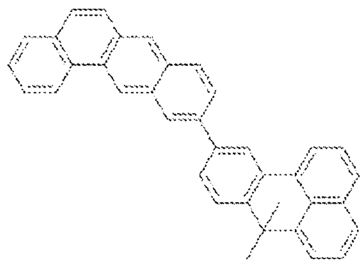

[Formula 171]

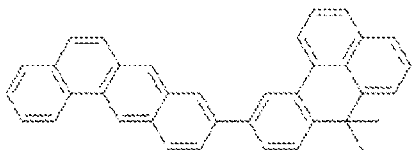
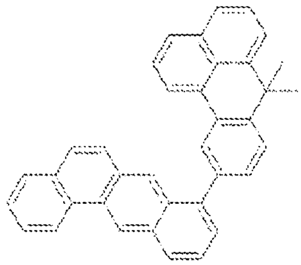
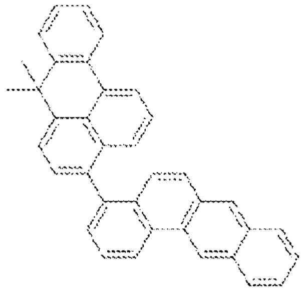
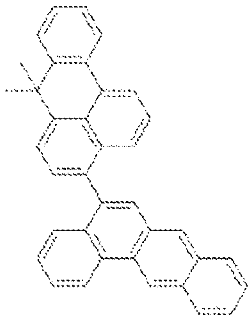
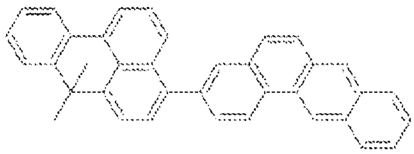
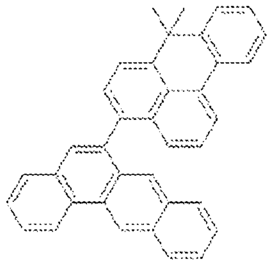
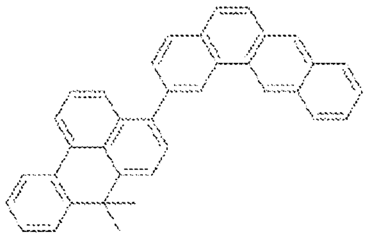
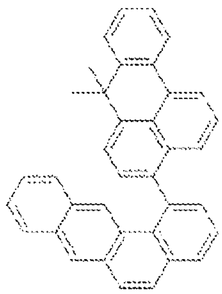
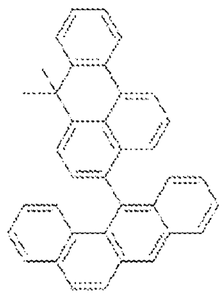

[Formula 172]

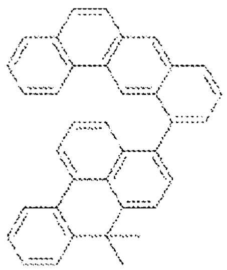
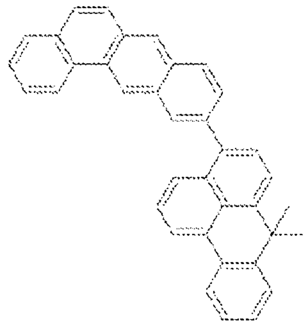
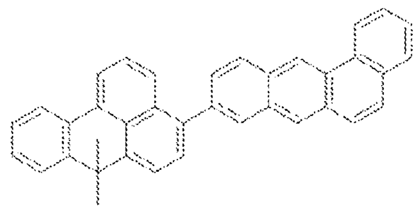
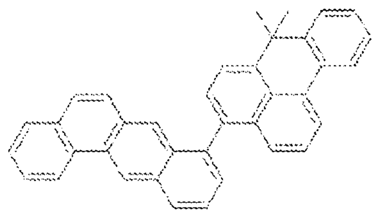
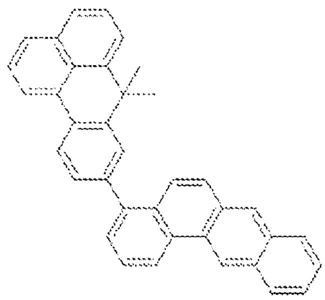
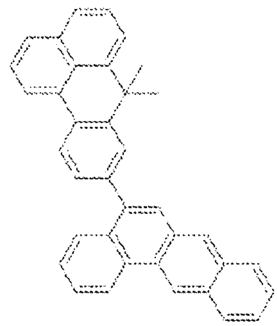
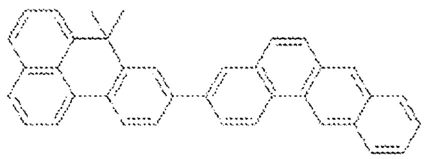
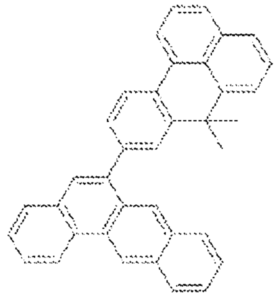

[Formula 173]

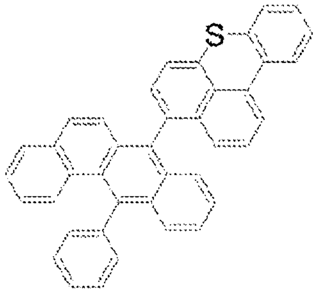
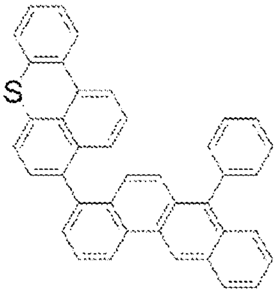
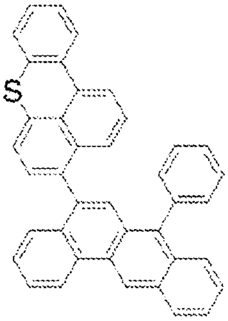
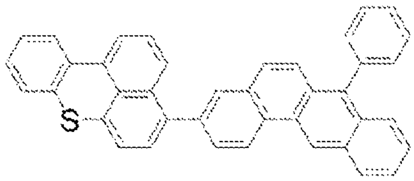
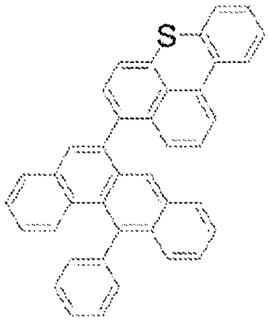
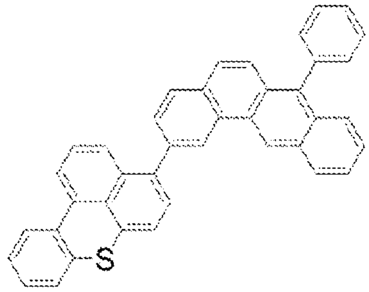
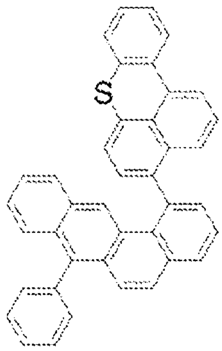
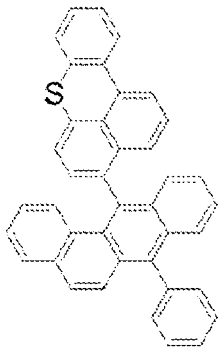
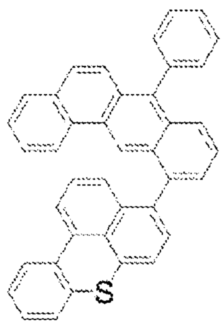
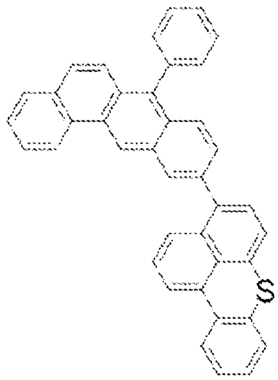
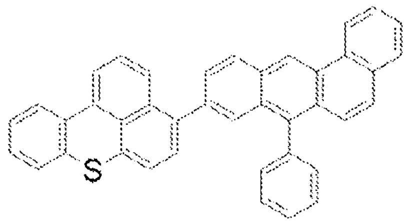
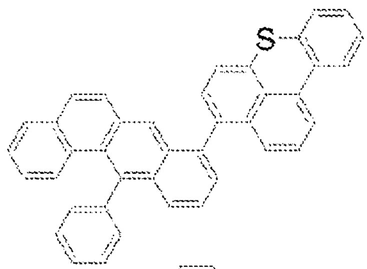
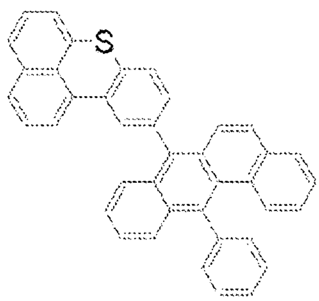
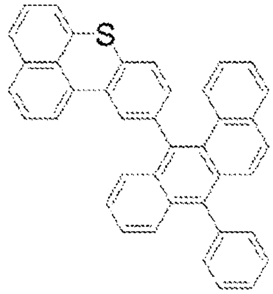

[Formula 174]

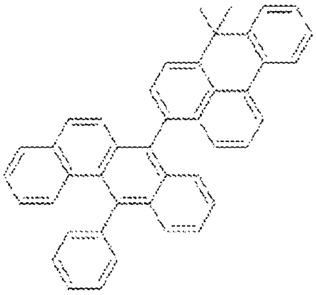
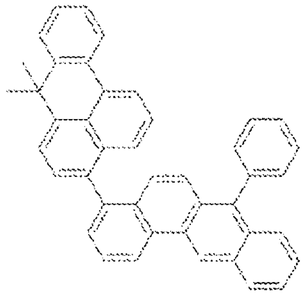
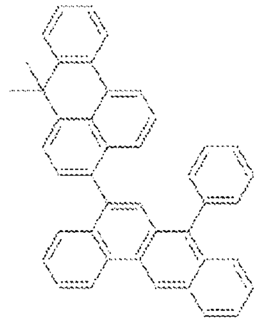
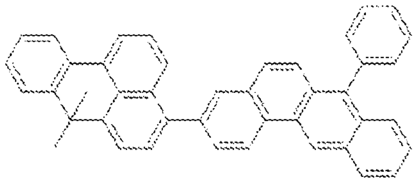
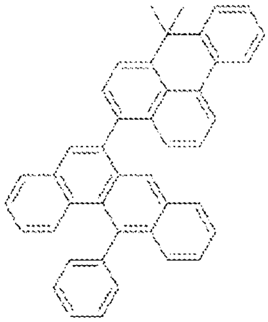
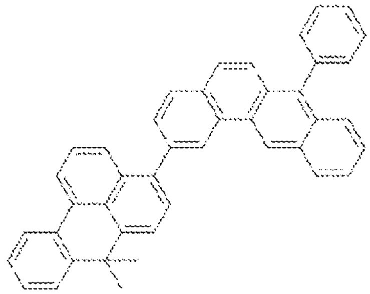
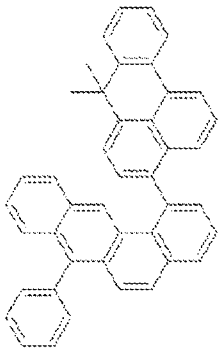
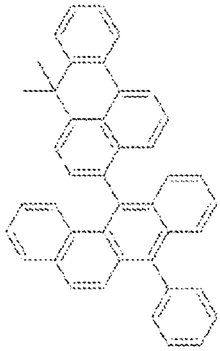
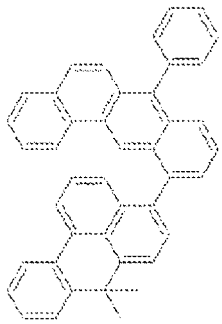
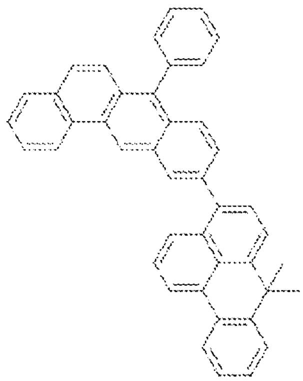
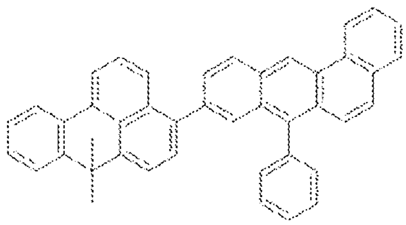
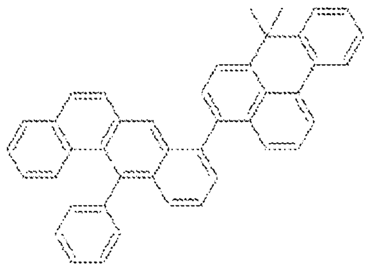
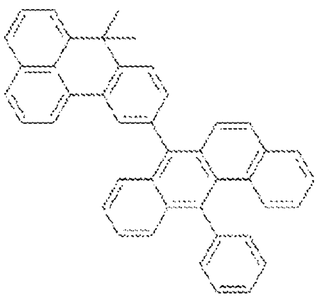
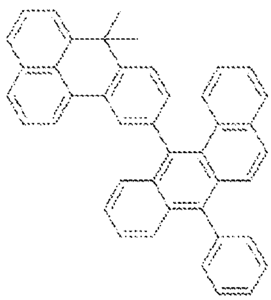

[Formula 175]

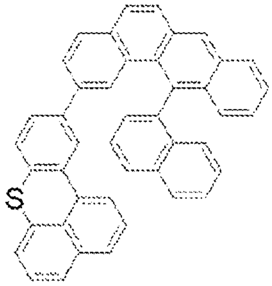
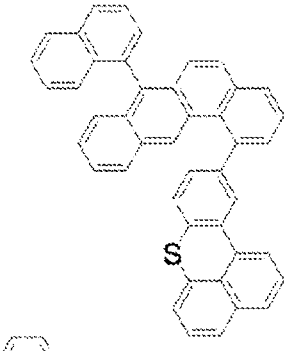
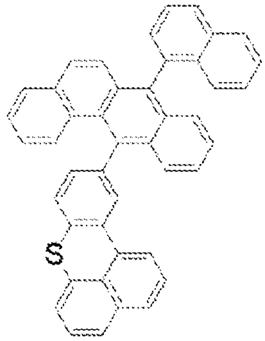
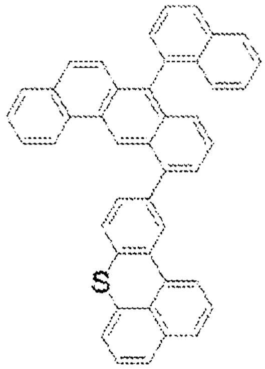
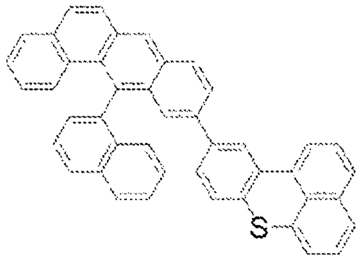
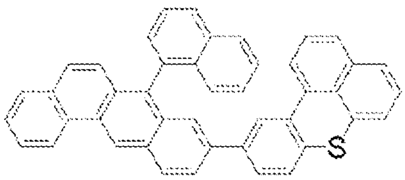
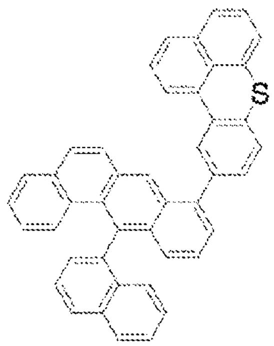
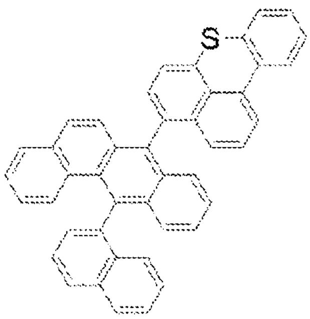
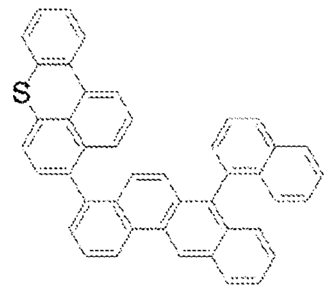
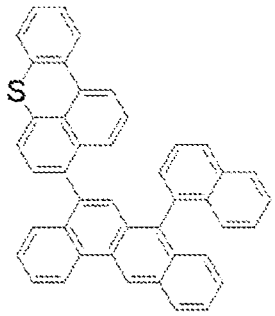

[Formula 176]

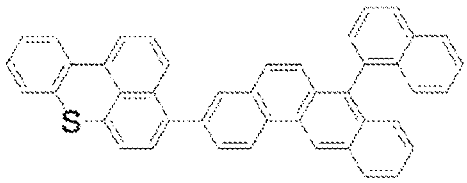

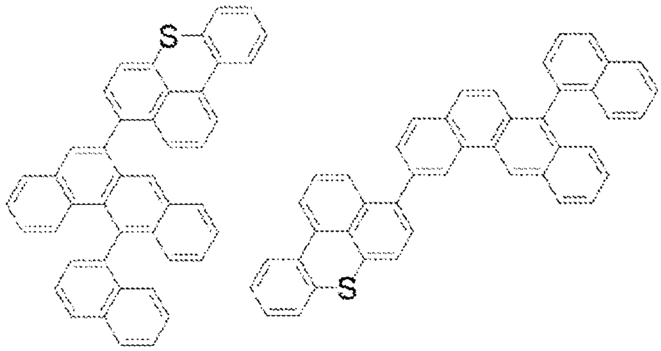

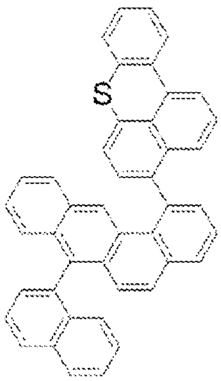

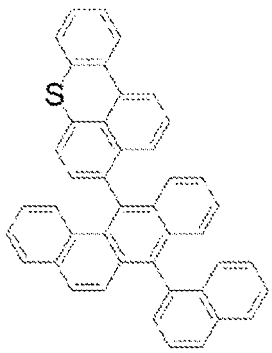

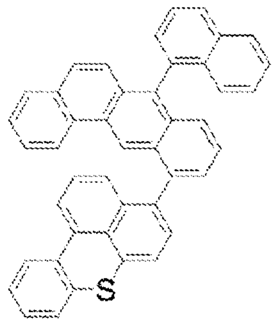

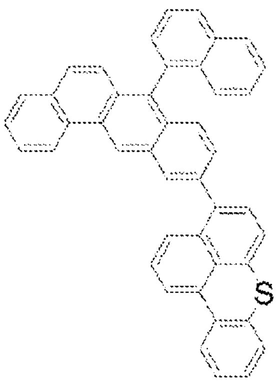

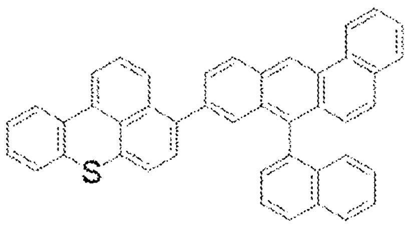

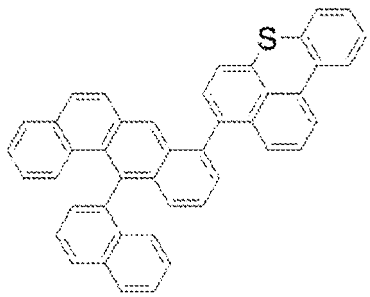

[Formula 177]

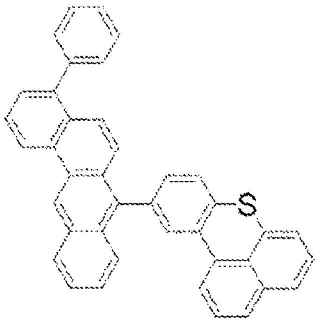
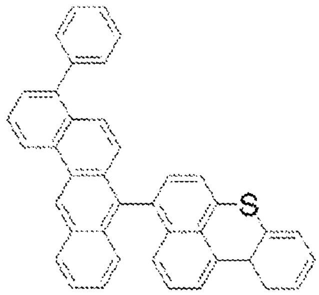
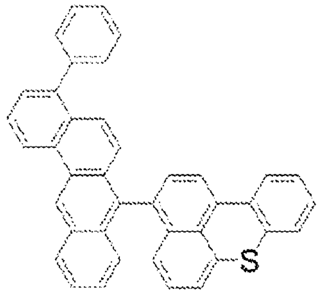
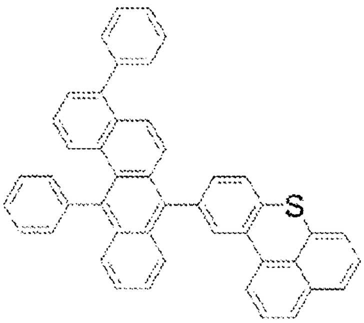
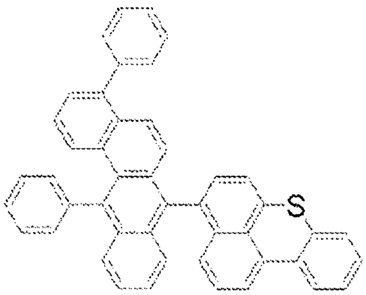
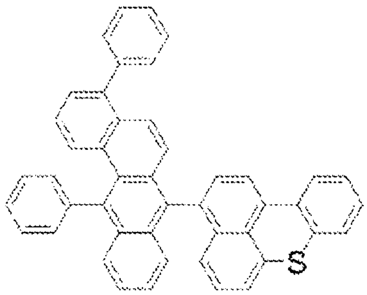
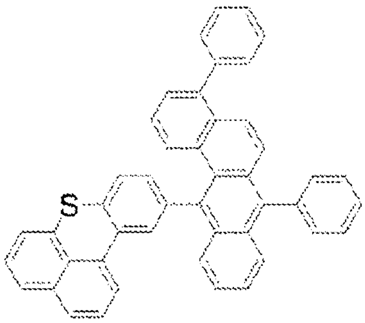
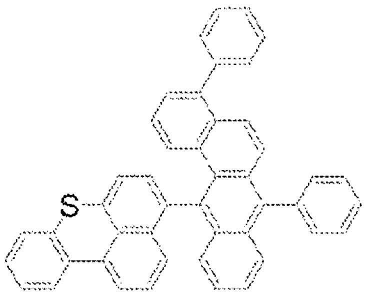
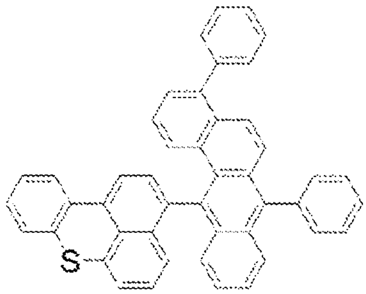

[Formula 178]

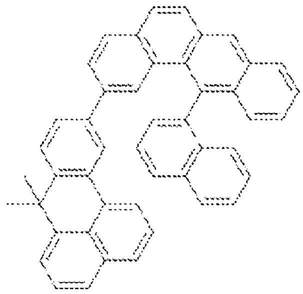

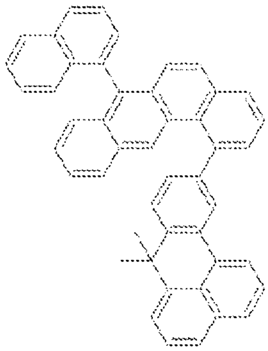

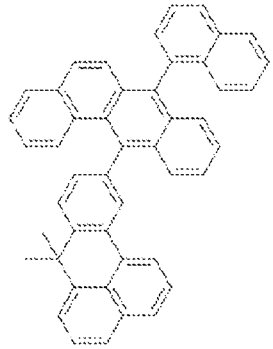

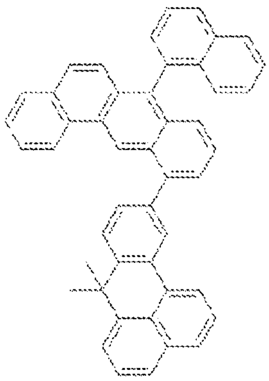

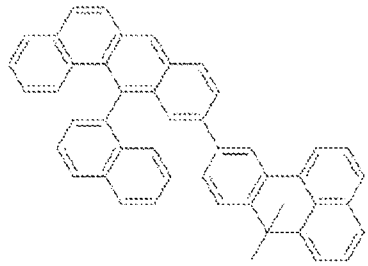

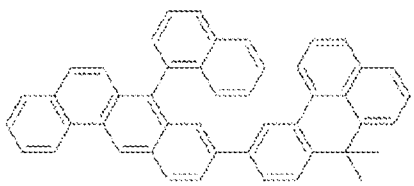

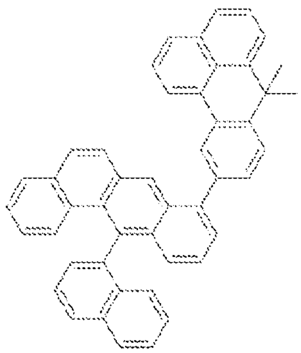

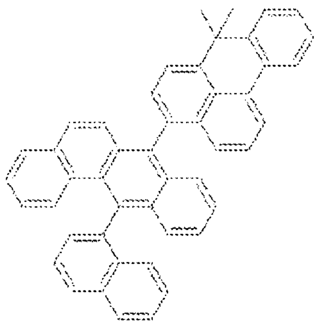

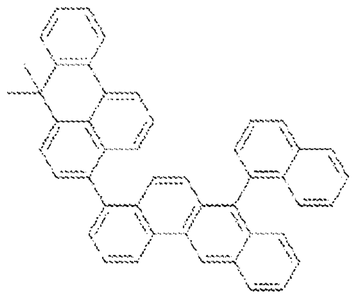

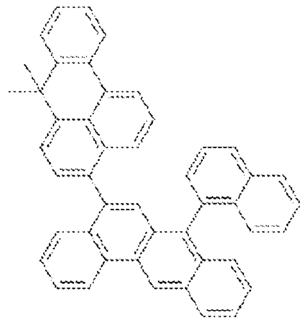

[Formula 179]

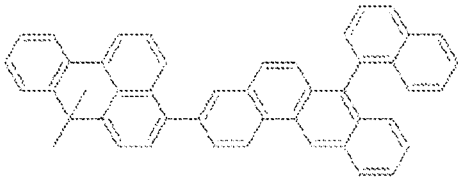
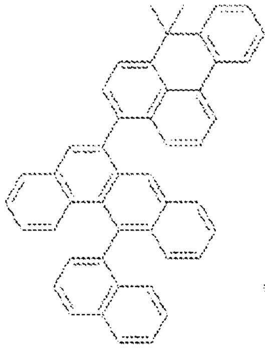
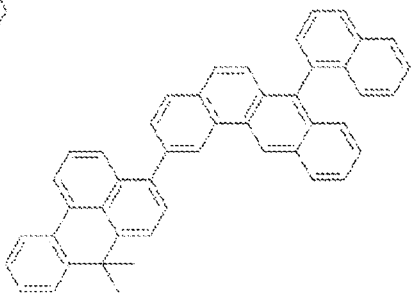
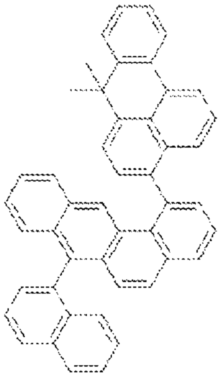
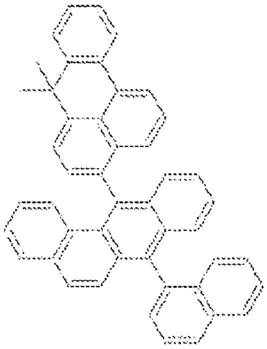
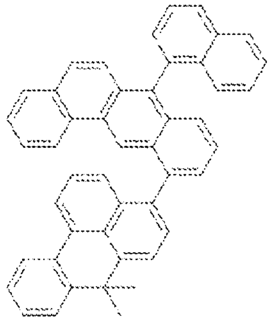
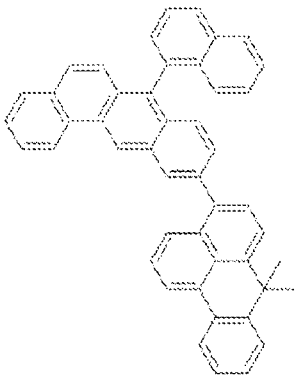
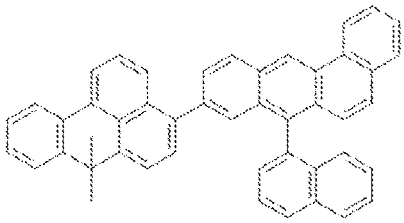
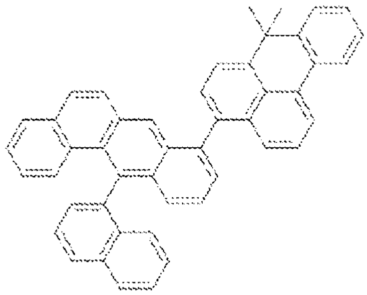

[Formula 180]

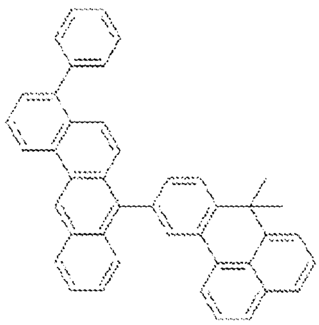
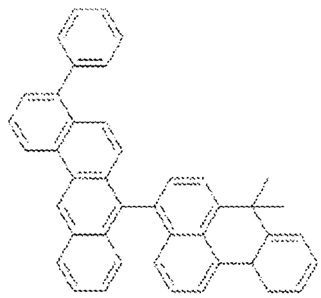
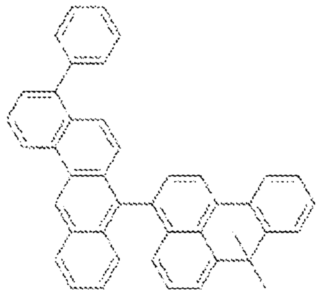
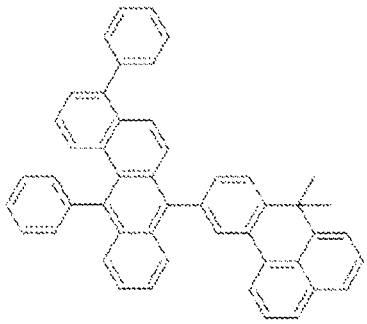
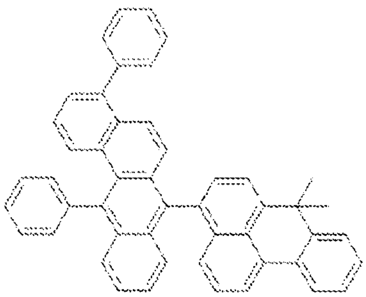
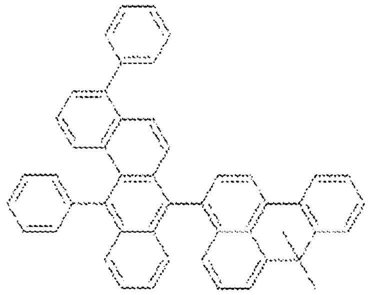
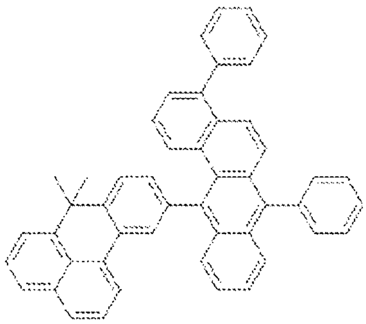
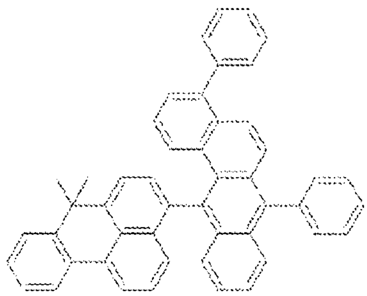
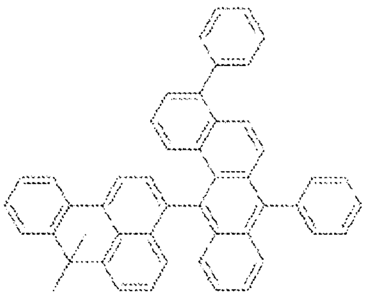

[Formula 181]

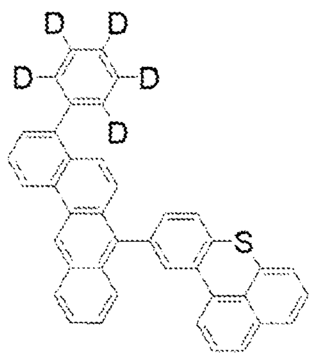

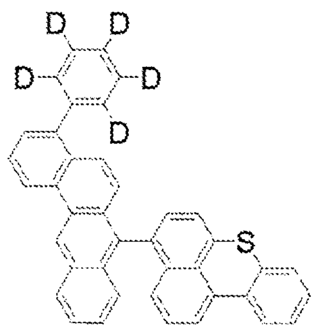

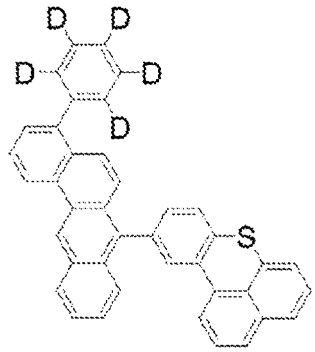

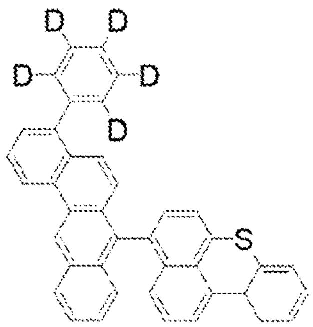

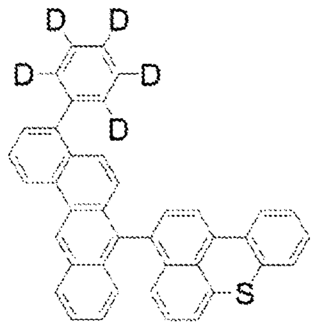

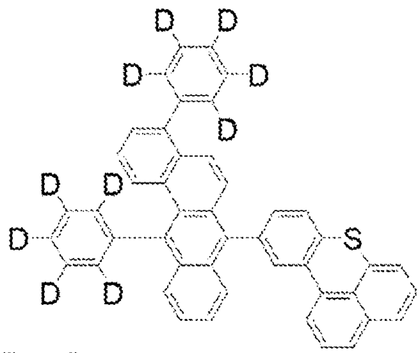

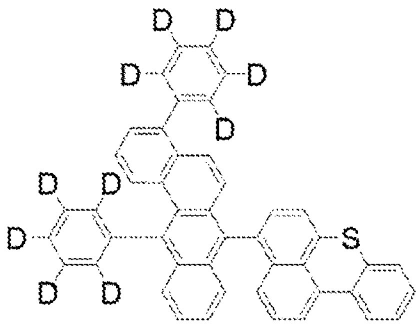

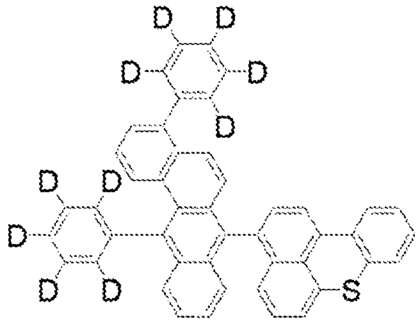

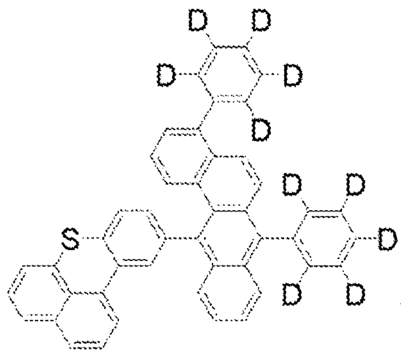

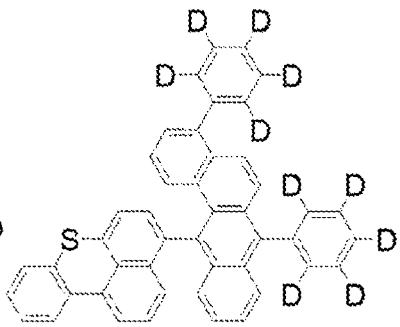

[Formula 182]

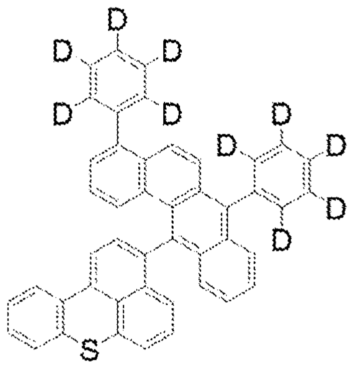

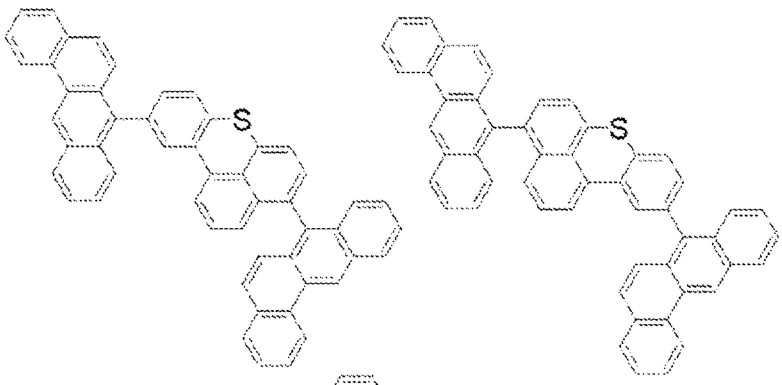

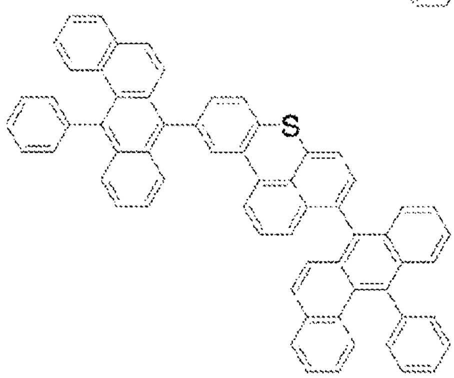

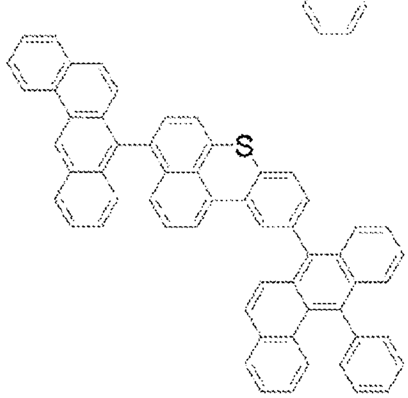

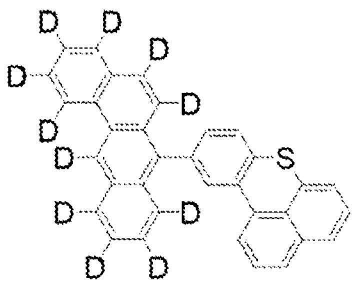

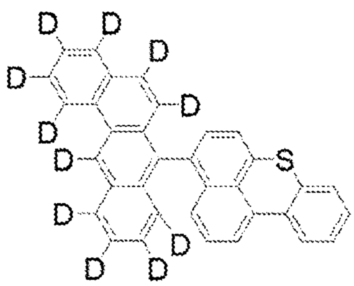

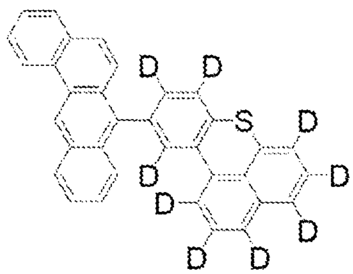

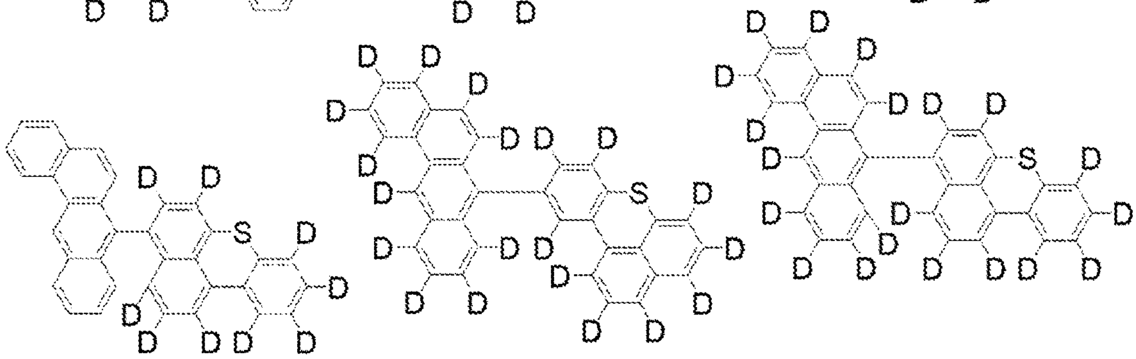

[Formula 183]

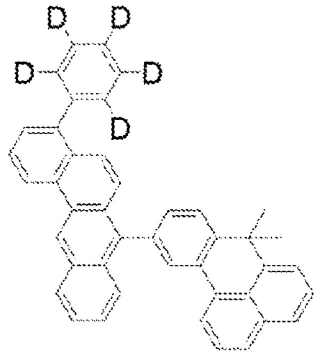
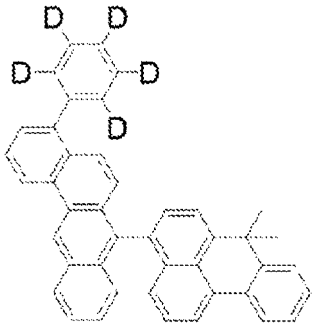
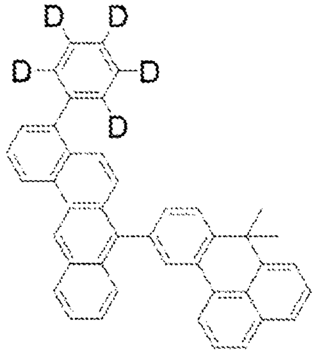
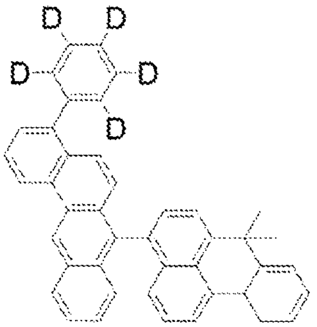
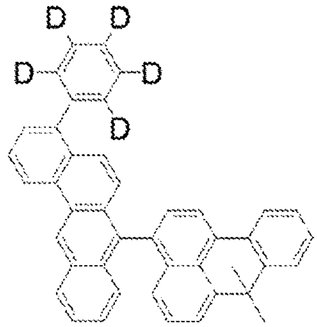
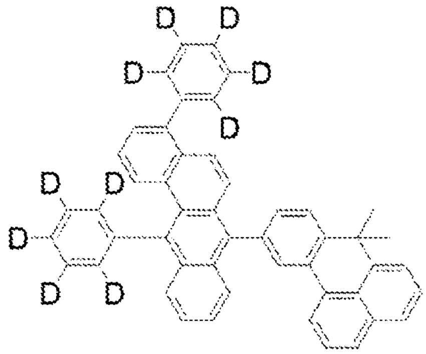
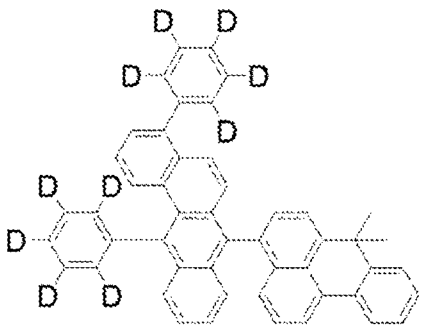
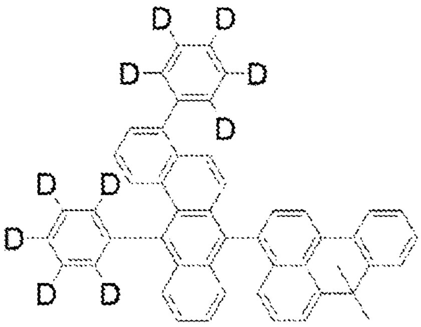
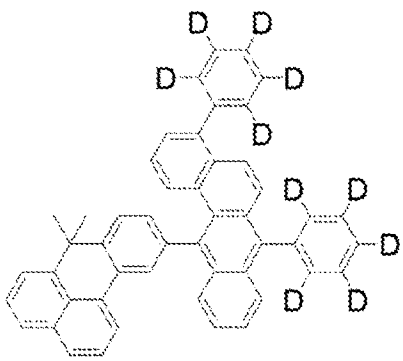
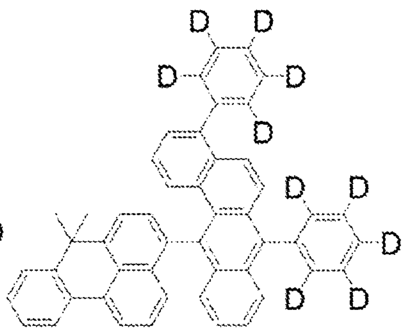

[Formula 184]

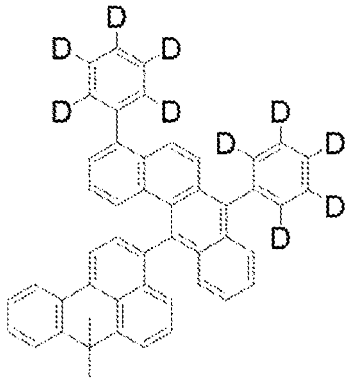

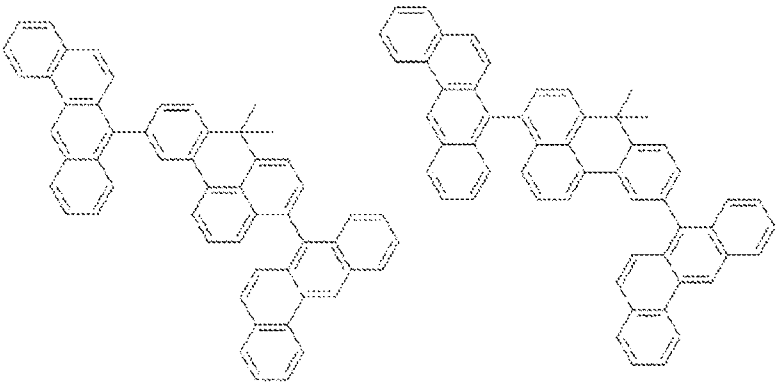

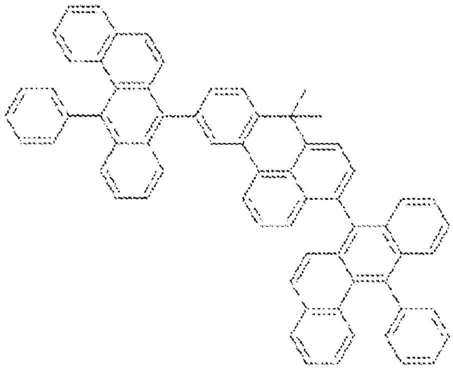

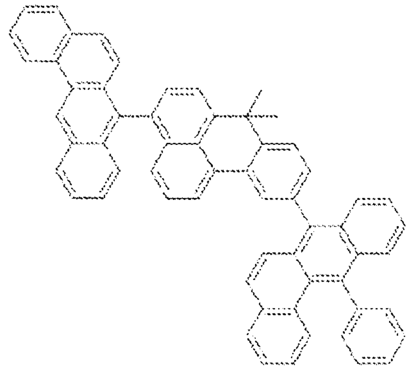

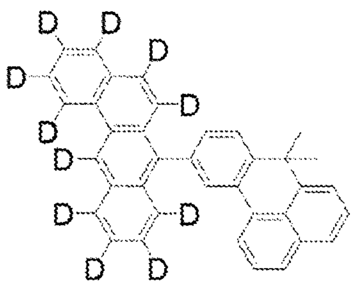

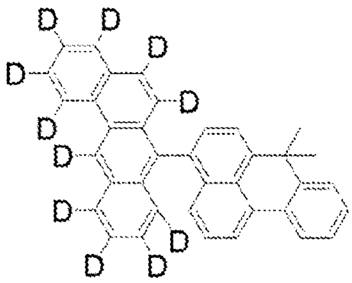

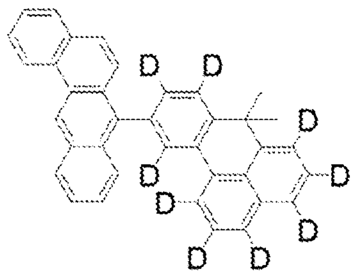

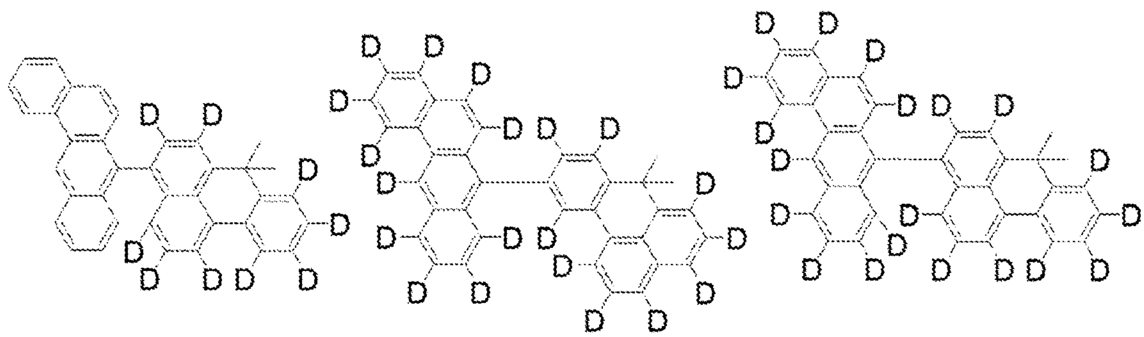

[Formula 185]

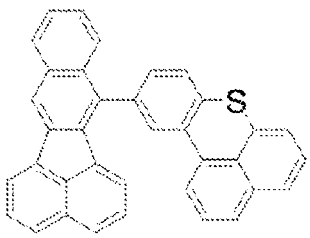
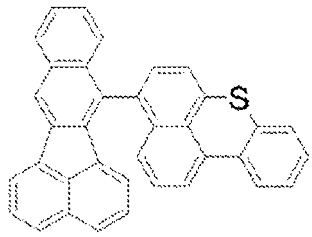
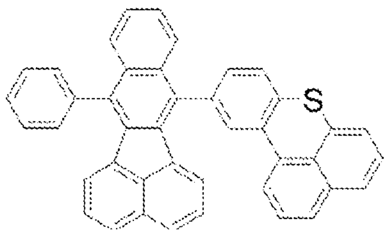
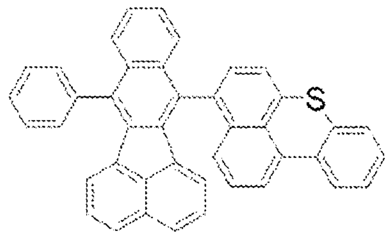

[Formula 186]

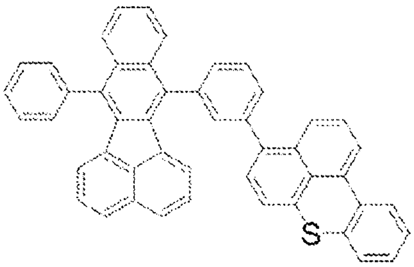

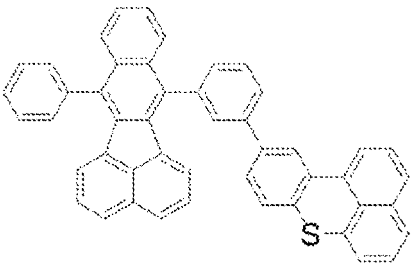

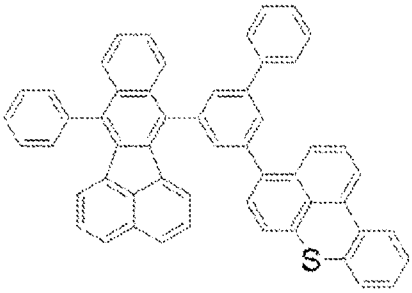

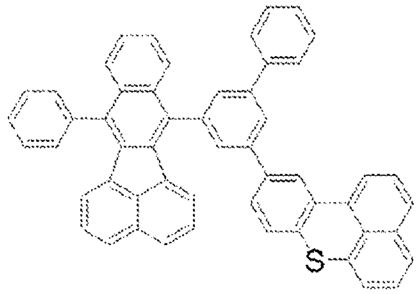

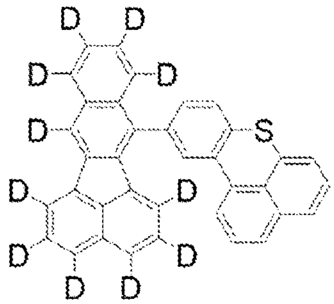

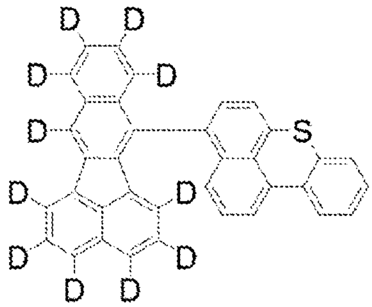

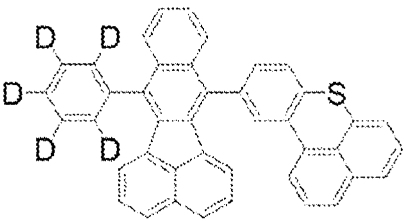

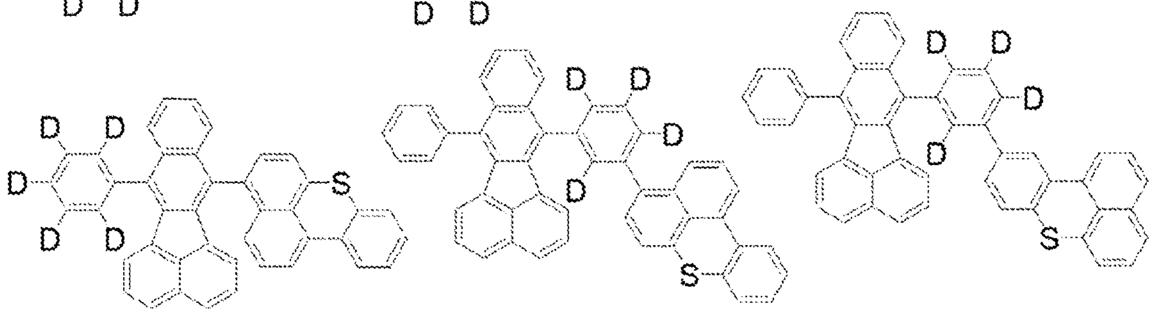

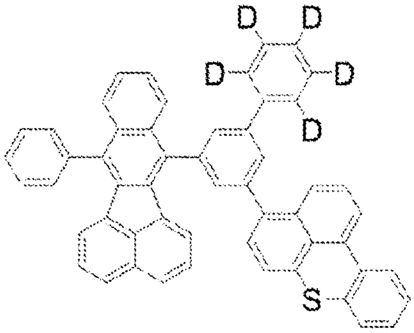

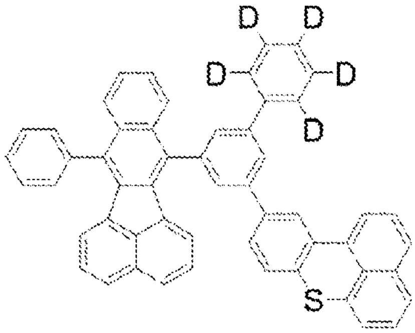

[Formula 187]

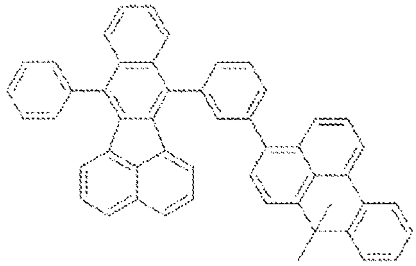
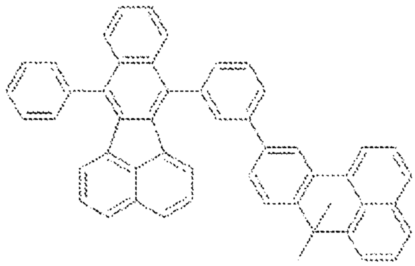

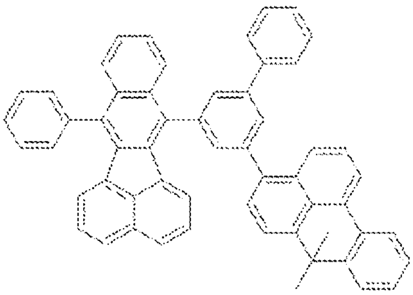
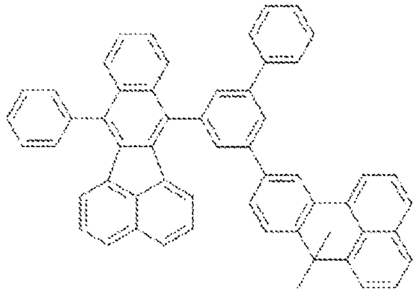

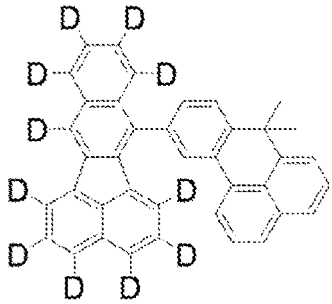
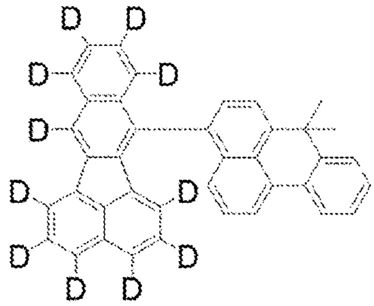
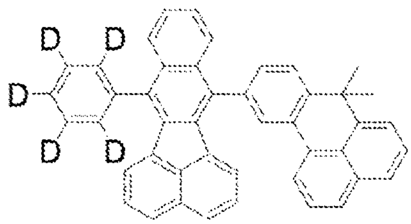

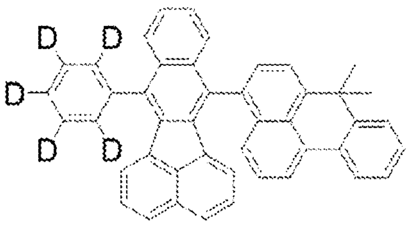
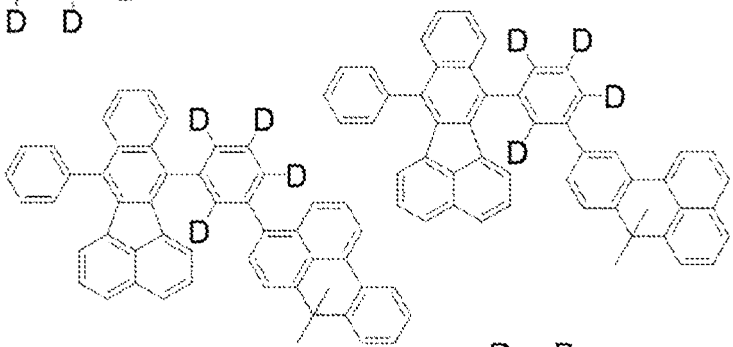

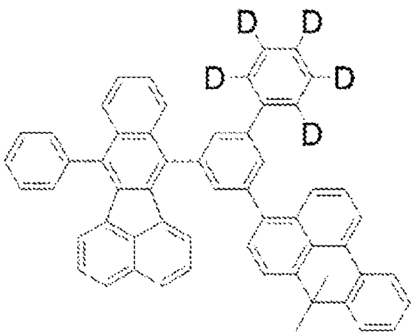
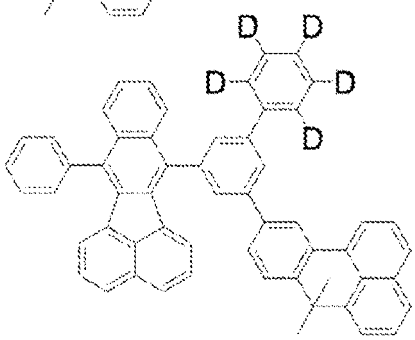

[Formula 188]

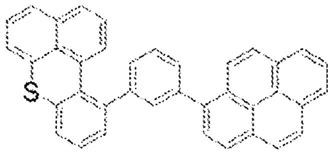 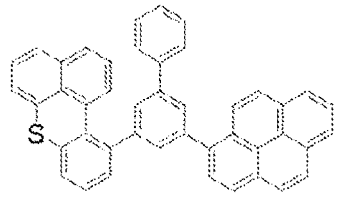 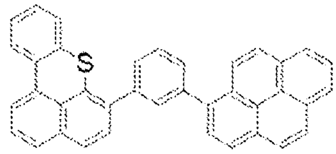

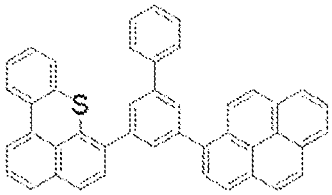 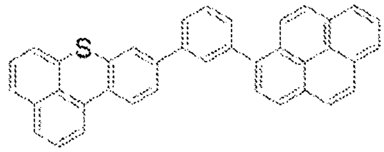 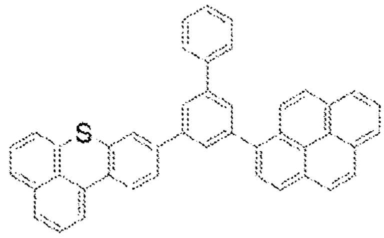

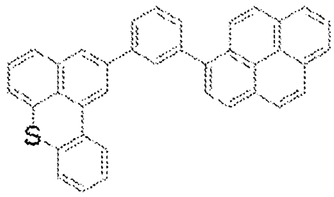 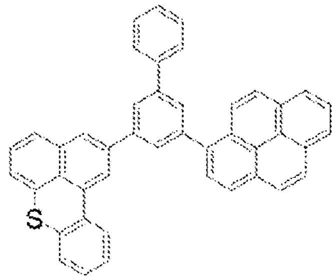 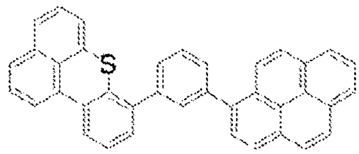

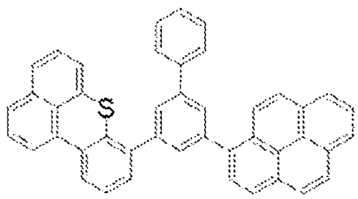 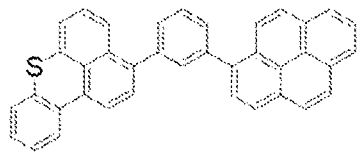 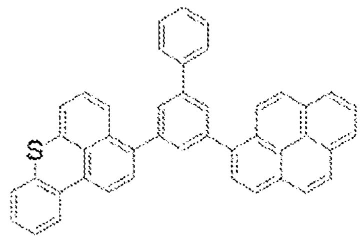

[Formula 189]

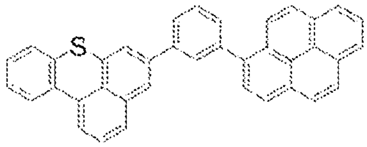
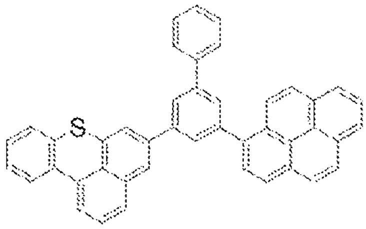
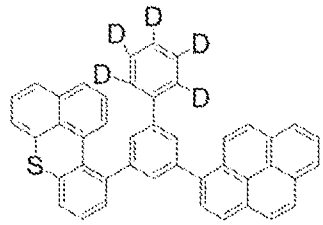
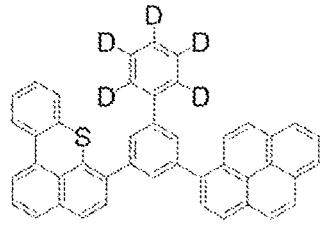
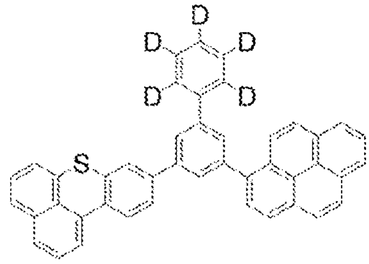
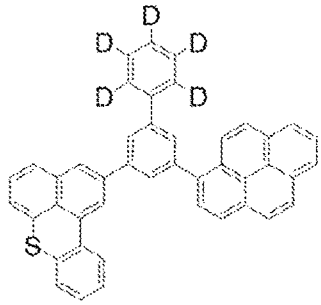
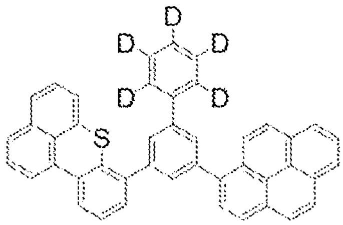
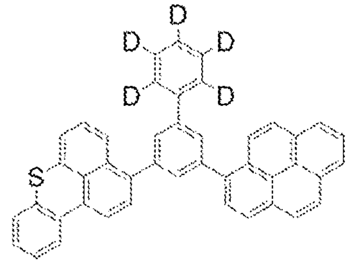
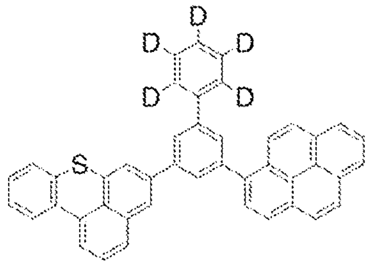

[Formula 190]

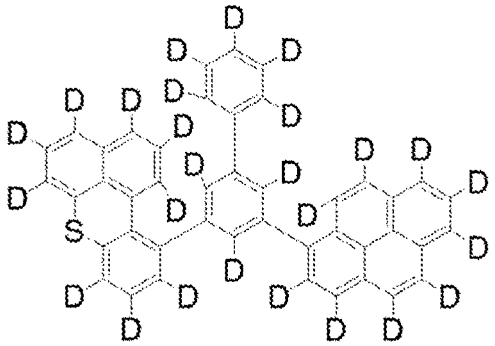

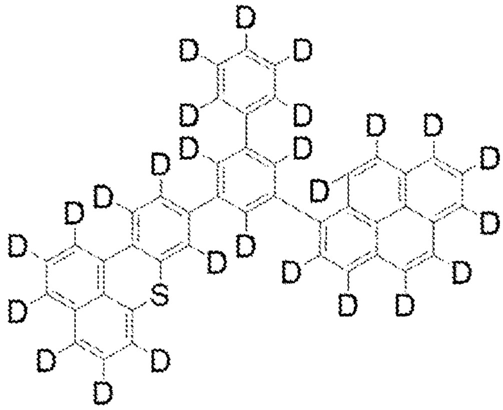

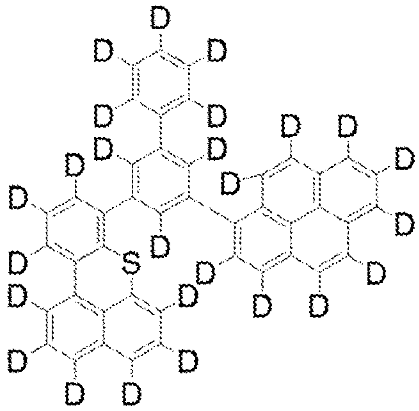

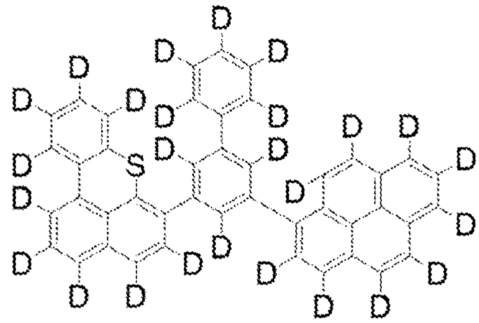

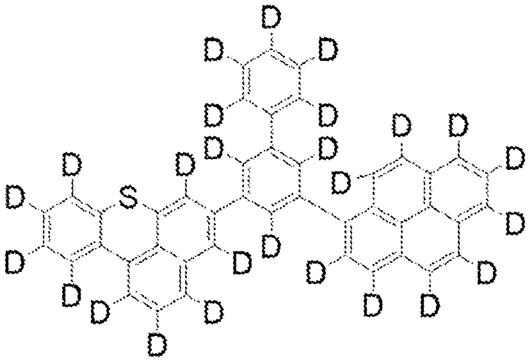

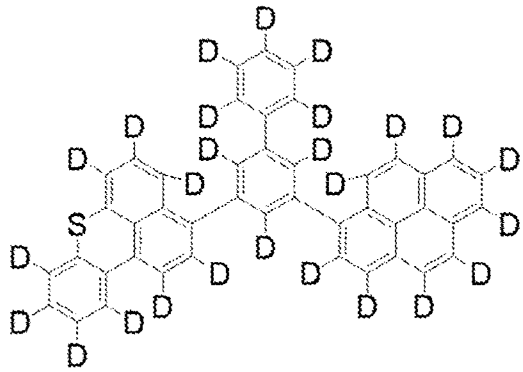

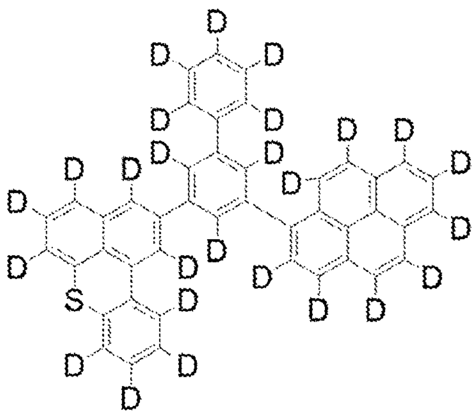

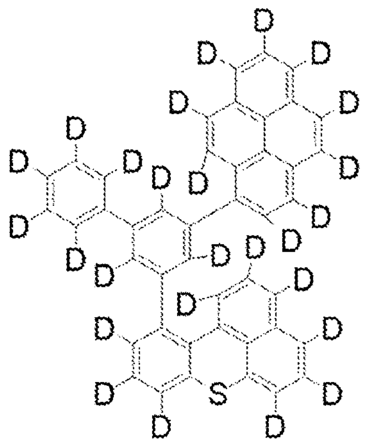

[Formula 191]

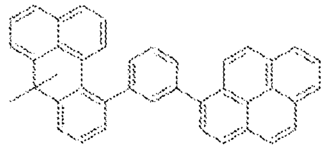 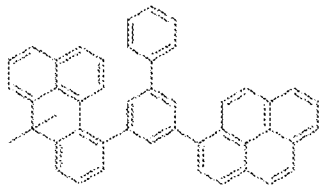 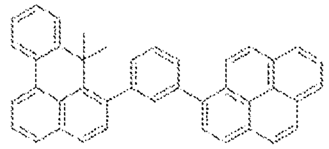

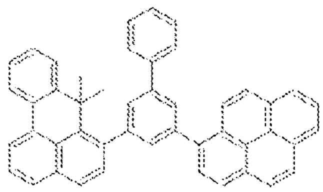 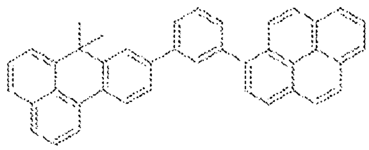 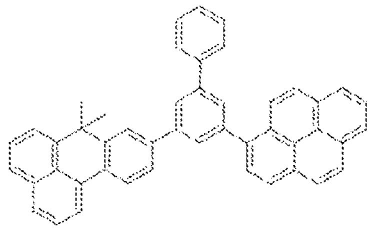

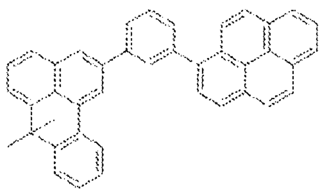 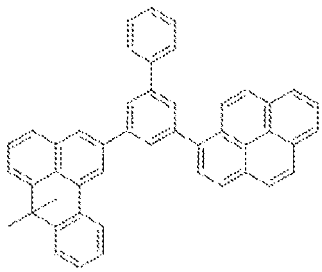 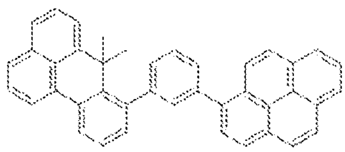

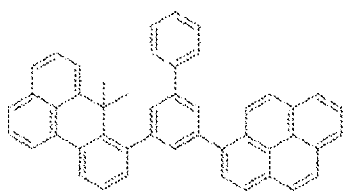 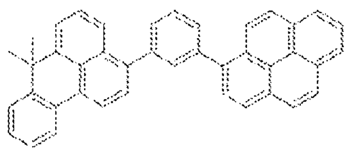 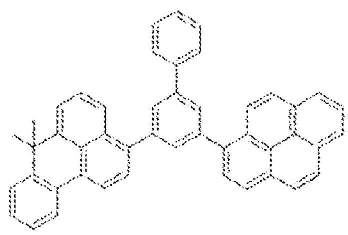

[Formula 192]

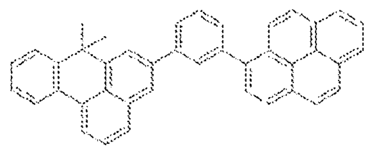
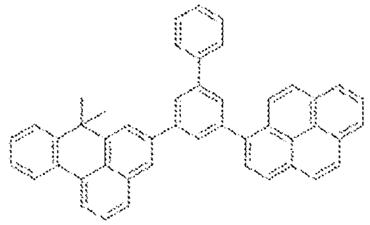
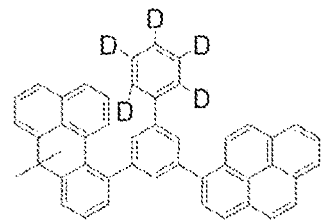

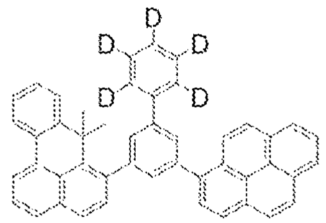
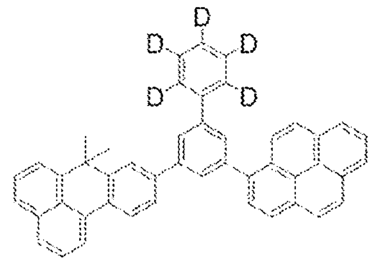
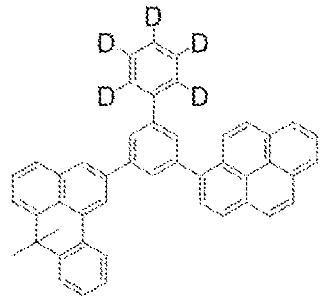

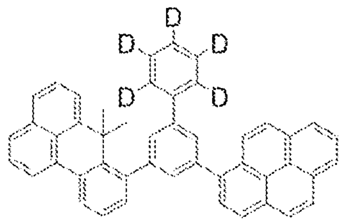
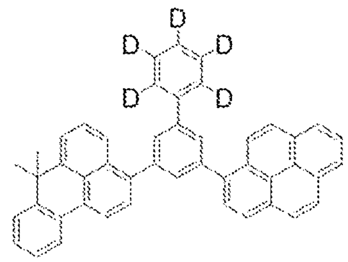
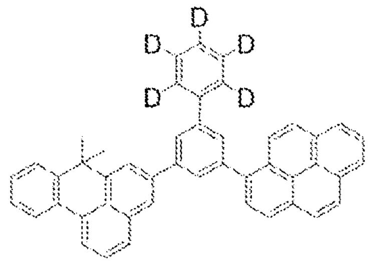

[Formula 193]

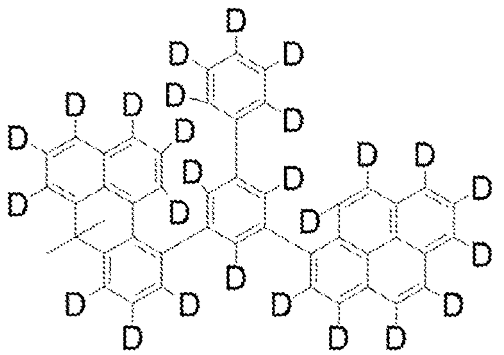

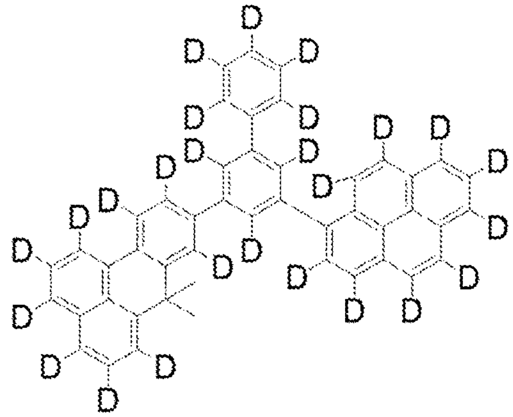

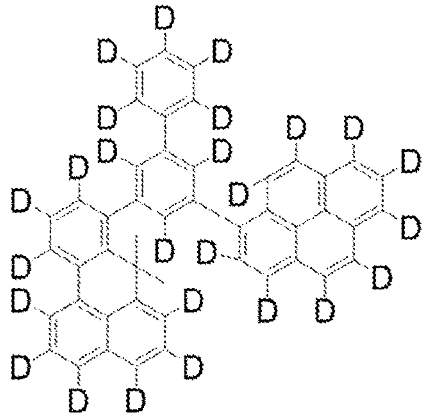

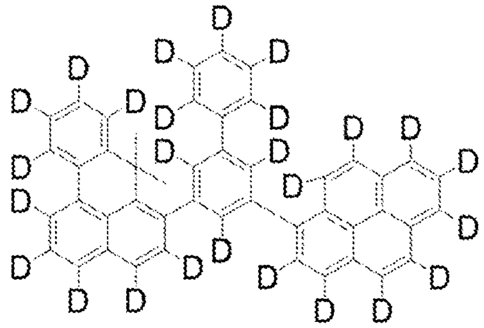

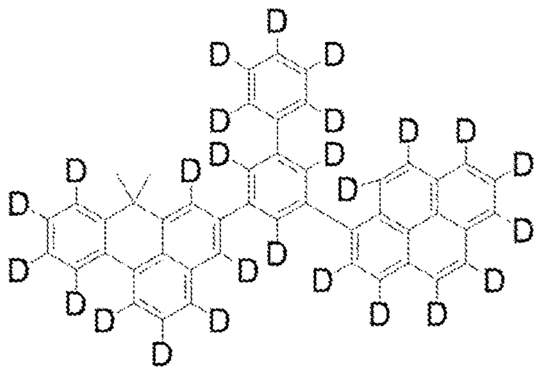

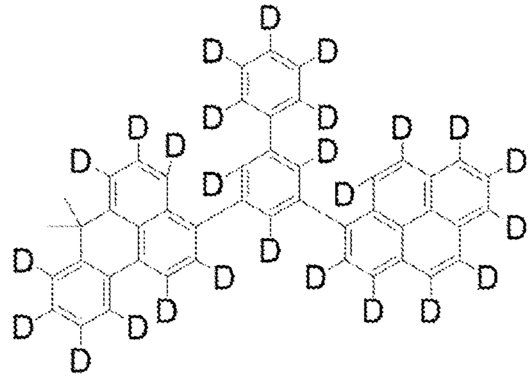

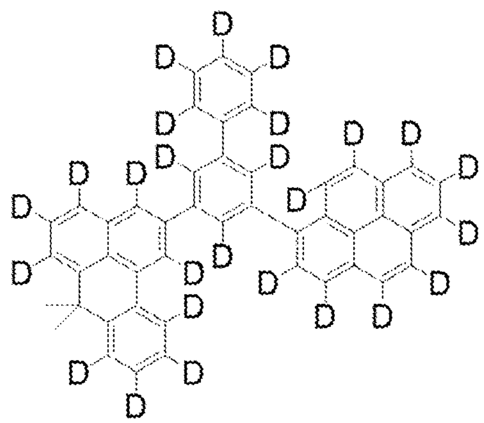

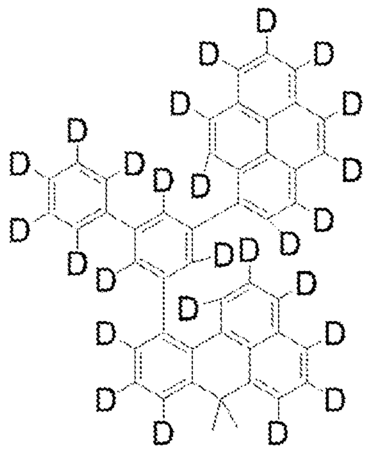

[Formula 194]

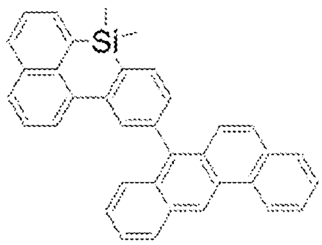
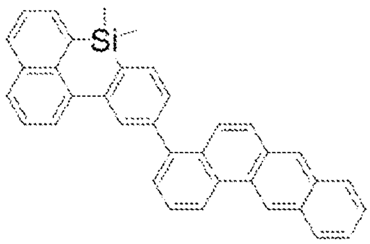
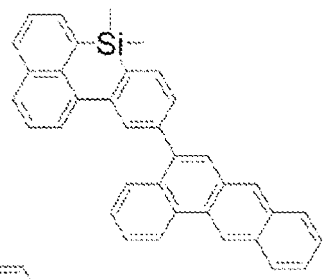

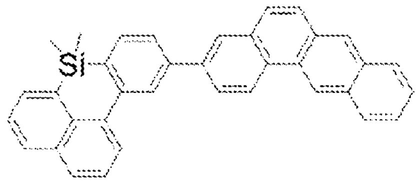
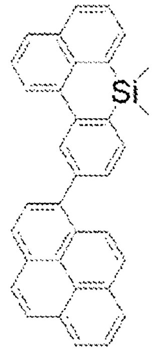

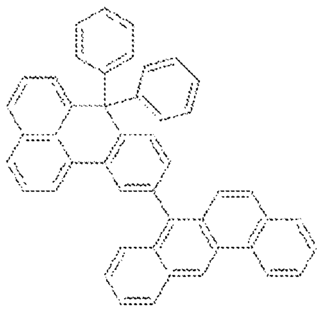
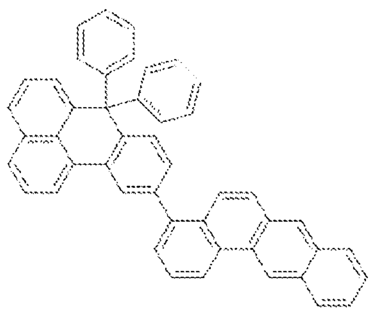
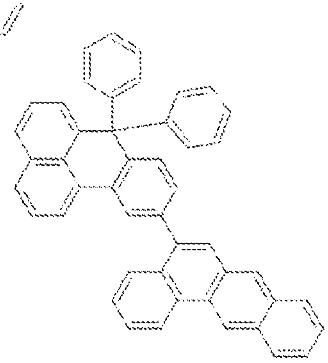

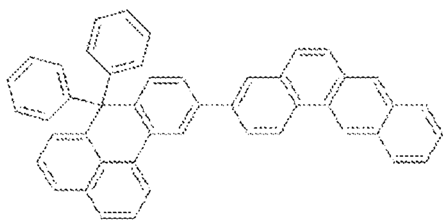
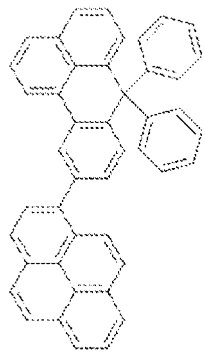

[Formula 195]

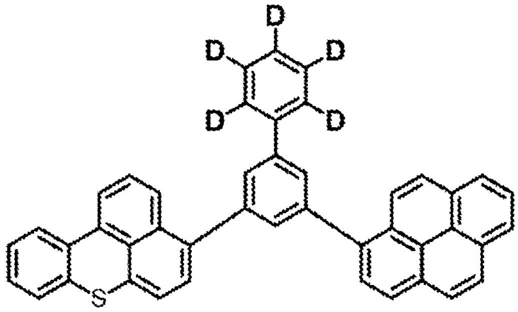

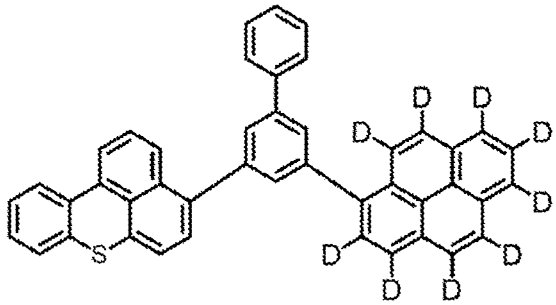

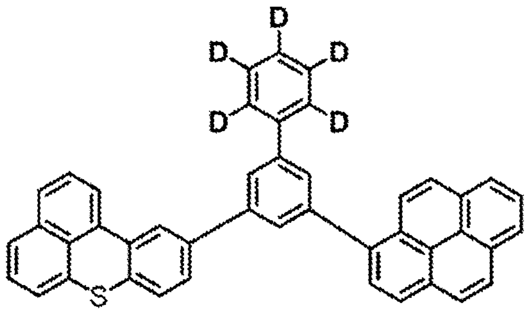

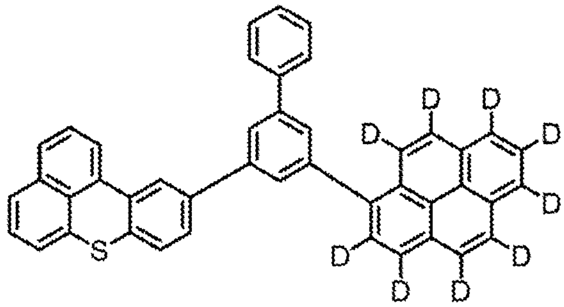

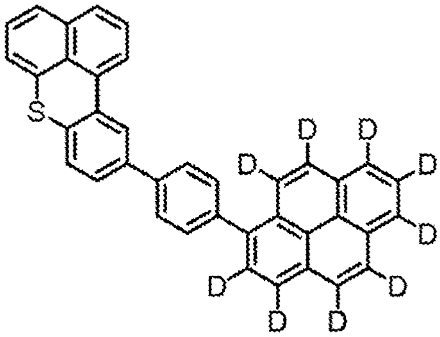

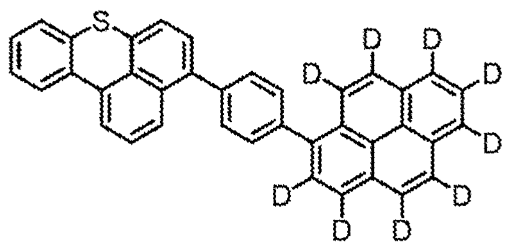

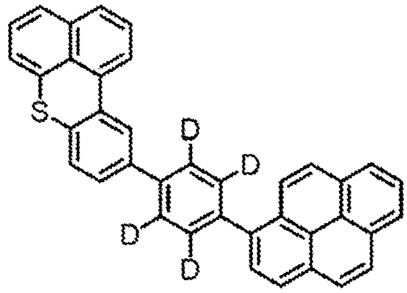

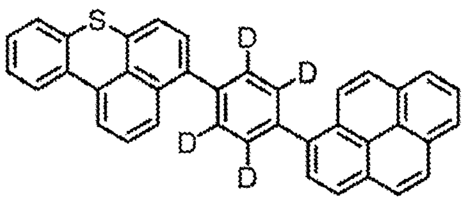

[Formula 196]

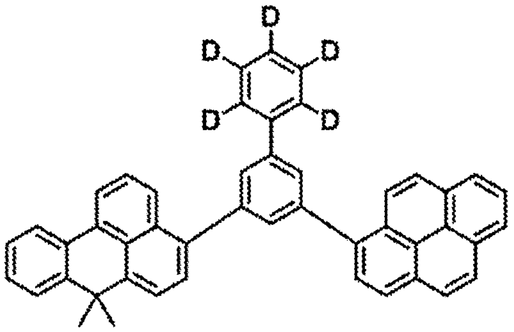

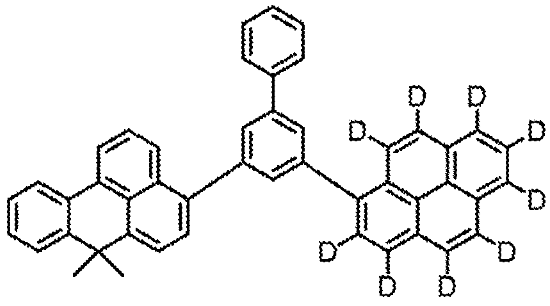

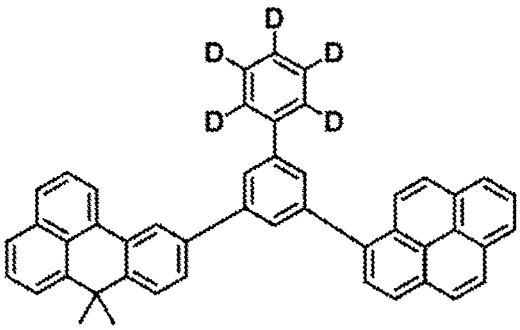

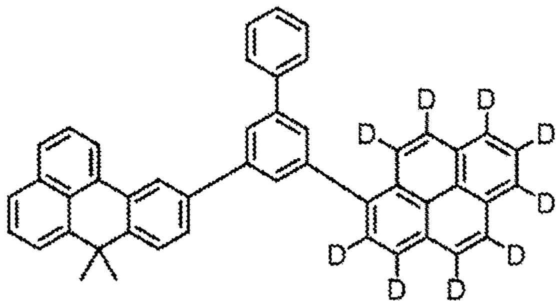

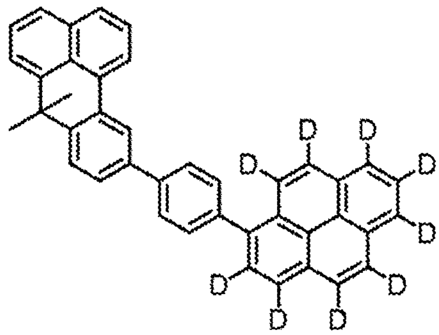

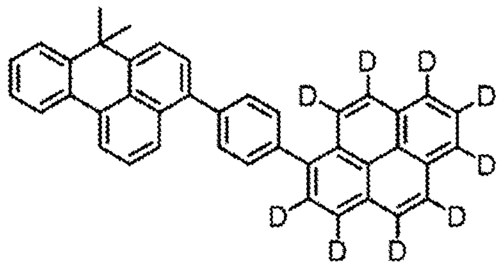

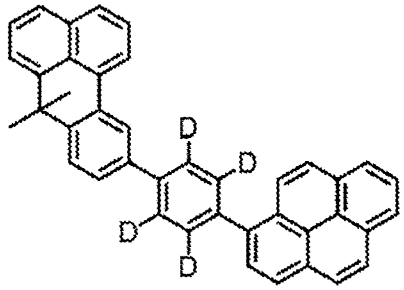

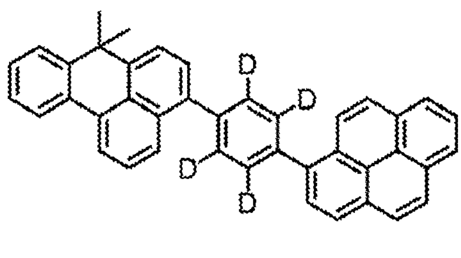

[Formula 197]

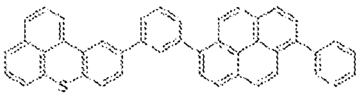 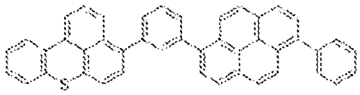 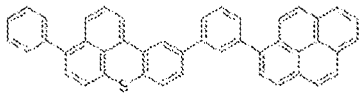

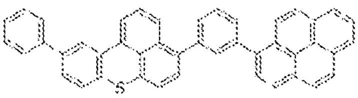 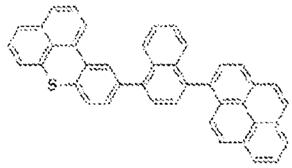 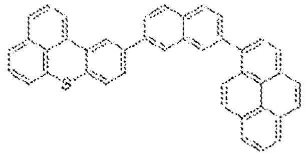

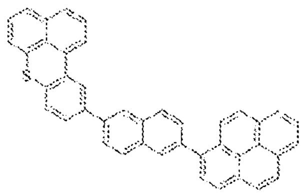 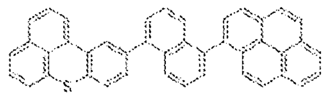 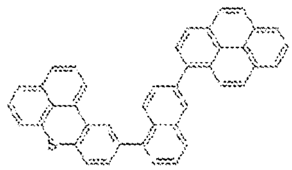

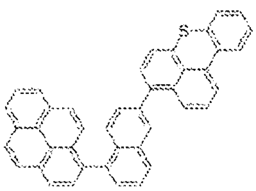 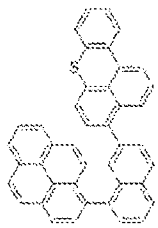 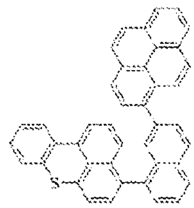

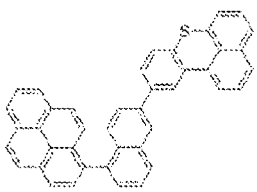 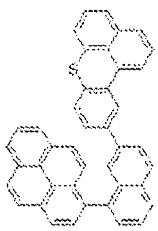 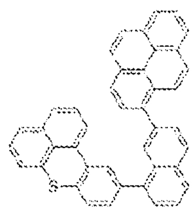

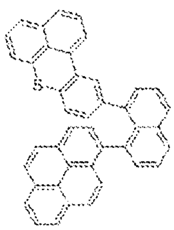 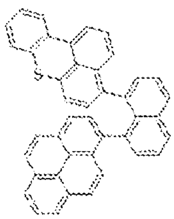 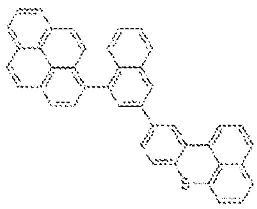 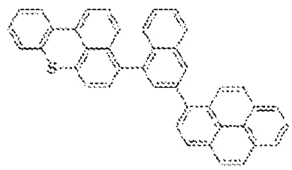

[Formula 198]

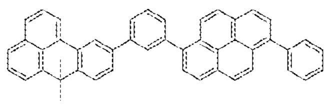
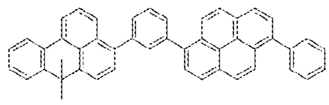
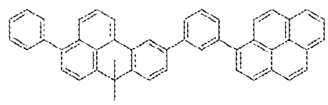

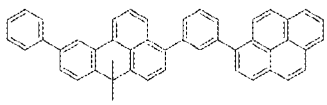
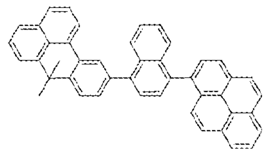
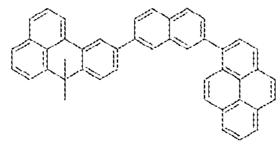

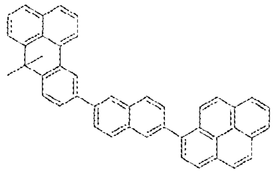
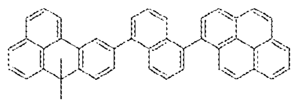
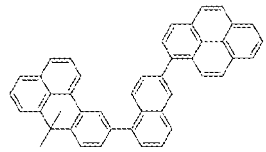

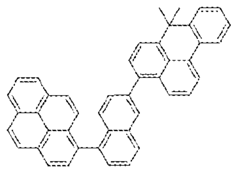
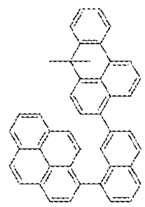
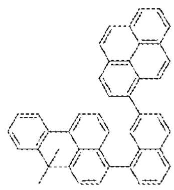

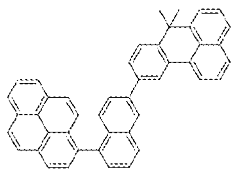
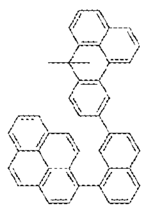
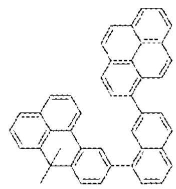

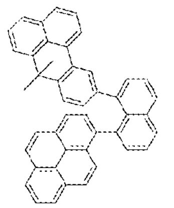
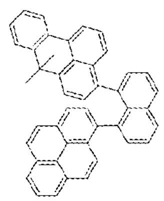
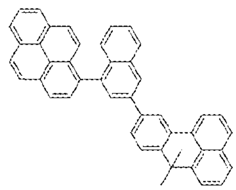
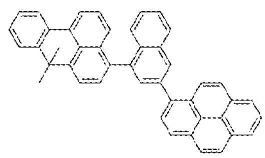

[Formula 199]

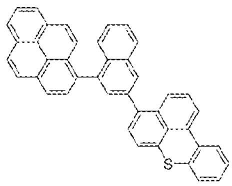
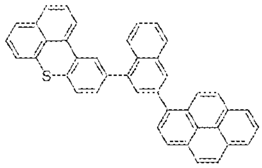
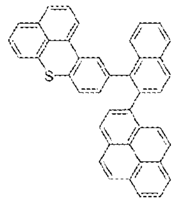
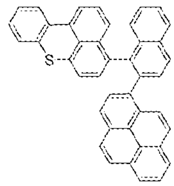
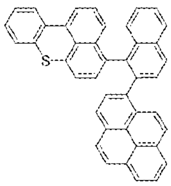
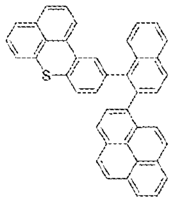
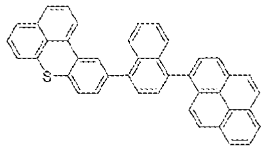
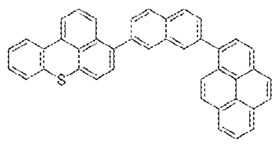
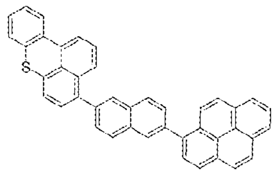
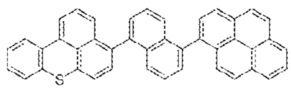
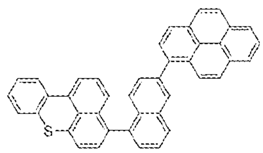
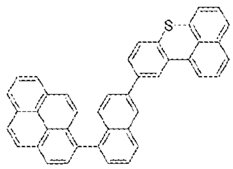

[Formula 200]

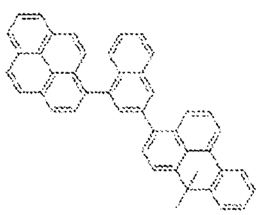

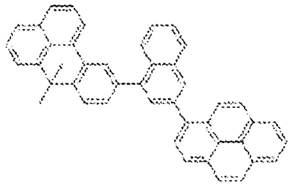

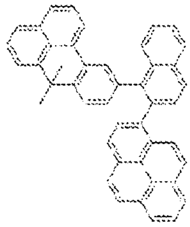

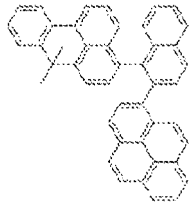

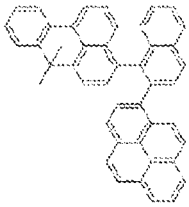

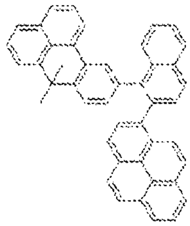

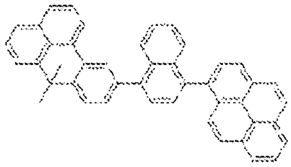

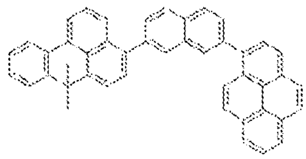

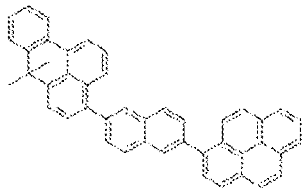

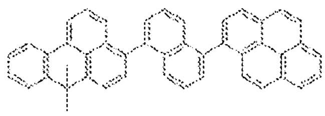

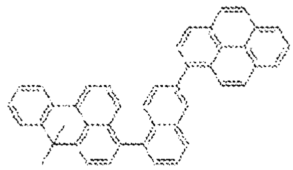

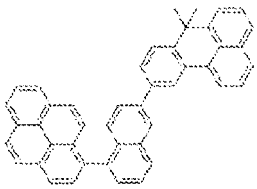

[Formula 201]

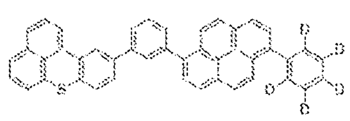
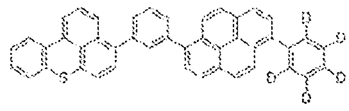
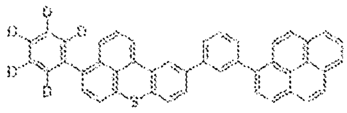
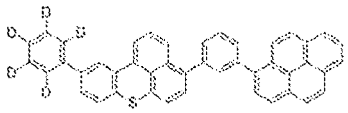
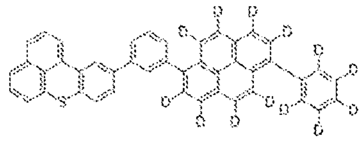
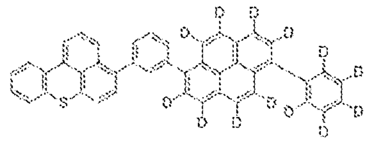
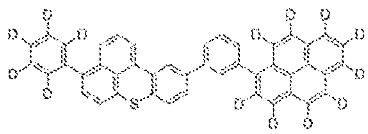
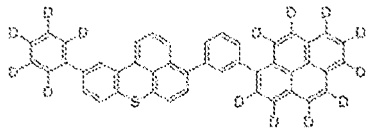
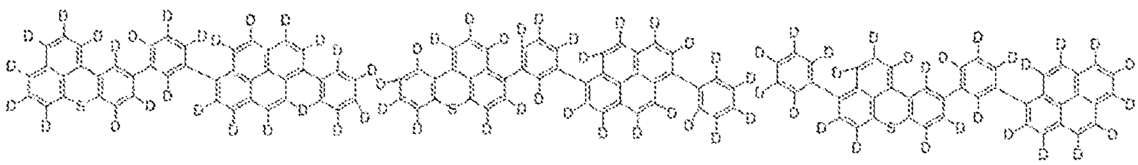
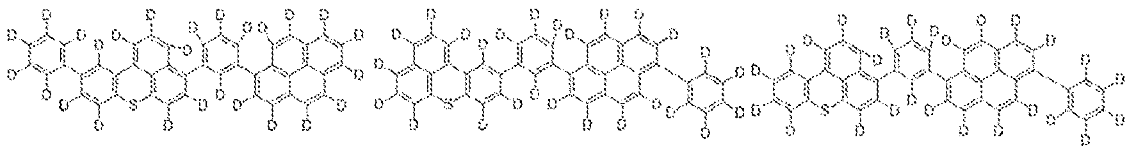
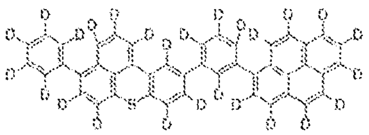
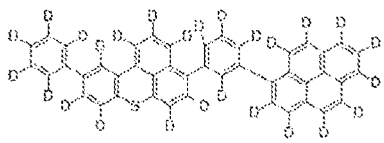

[Formula 202]

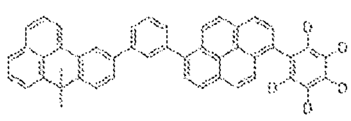
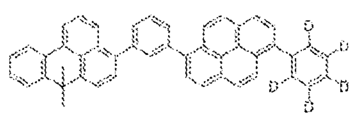
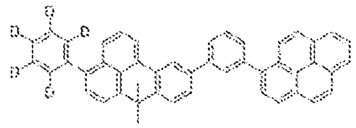
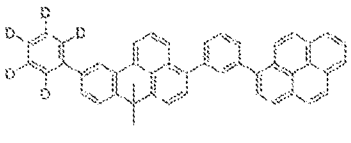
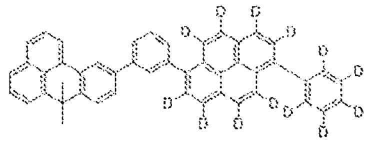
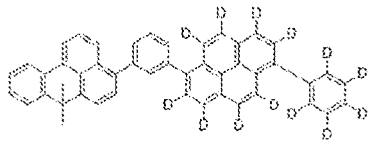
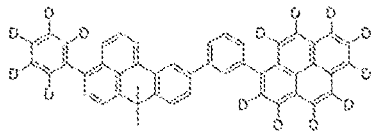
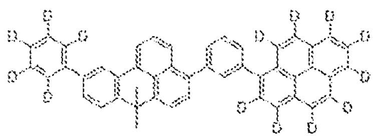
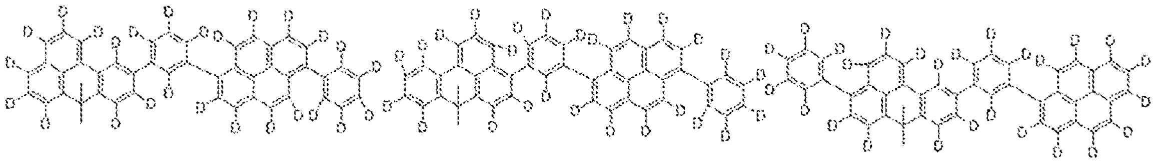
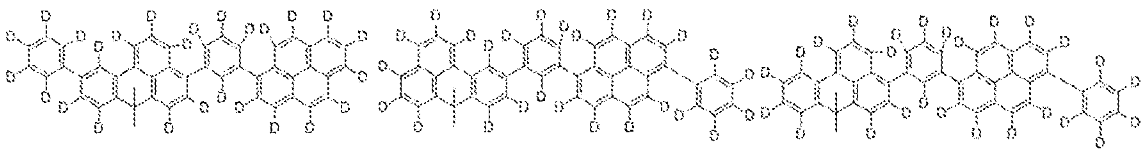
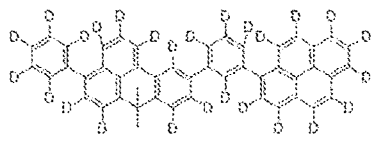
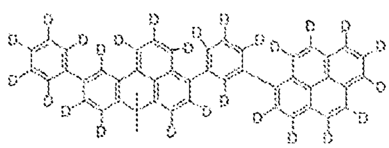

[Formula 203]

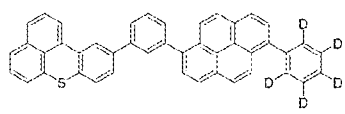
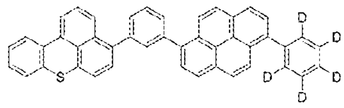
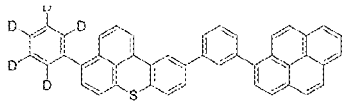

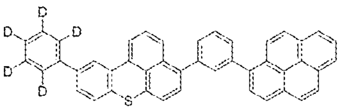
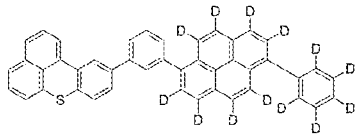
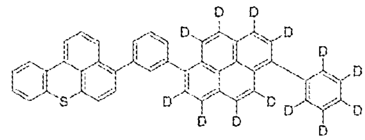

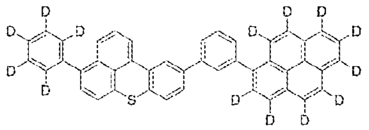
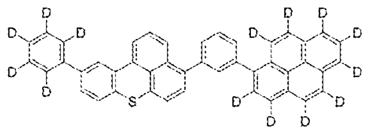

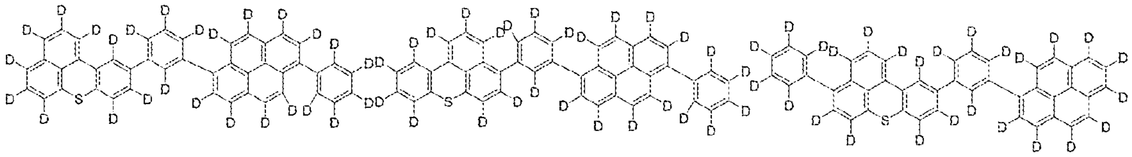

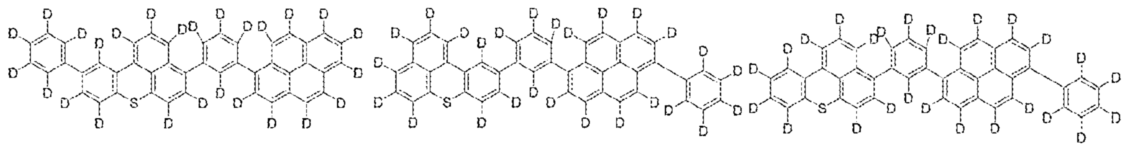

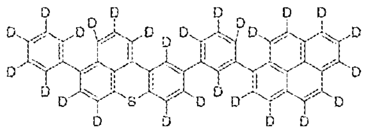
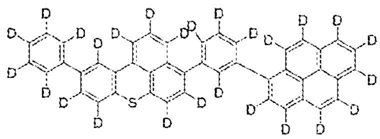

[Formula 204]

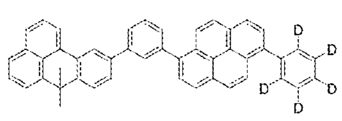
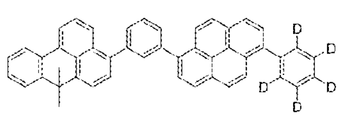
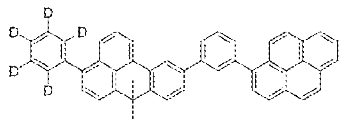
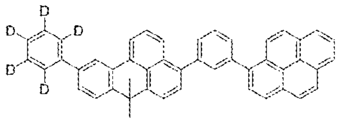
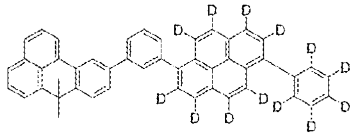
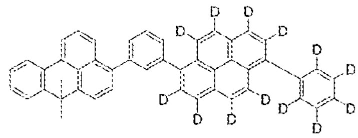
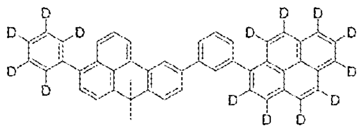
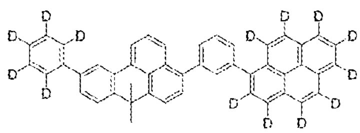
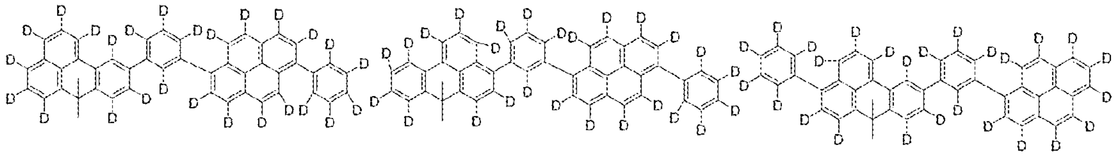
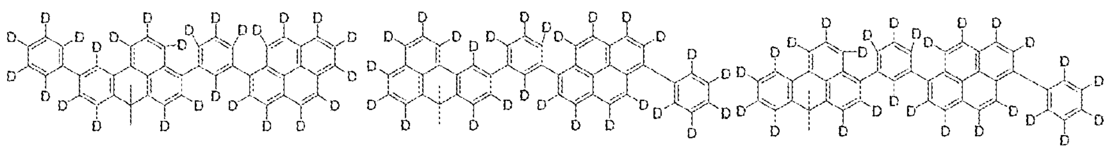
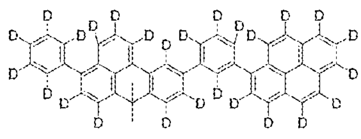
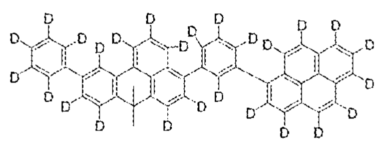

[Formula 205]

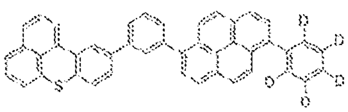
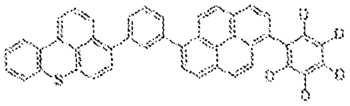
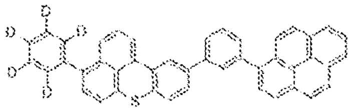
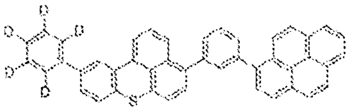
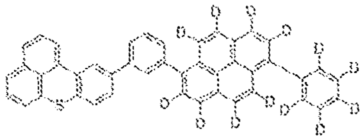
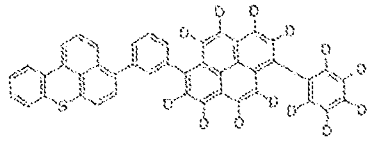
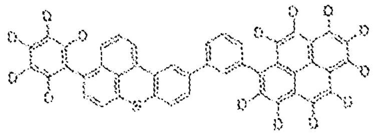
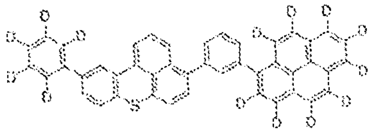
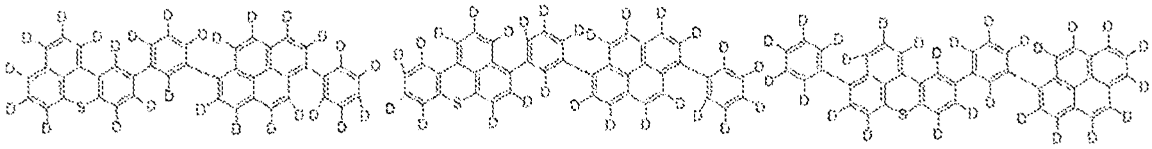
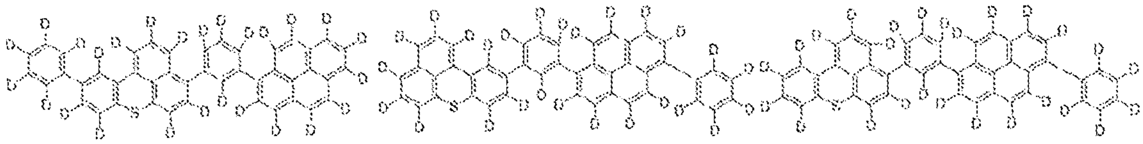
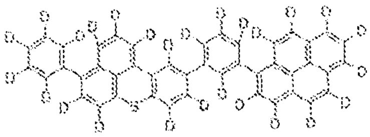
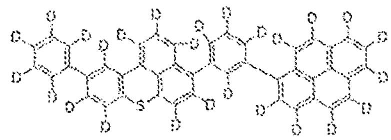

[Formula 206]

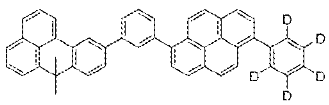
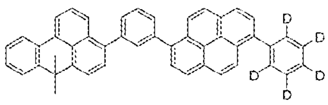
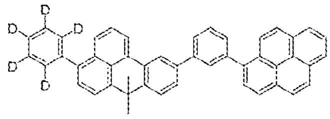
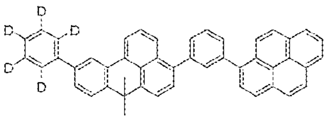
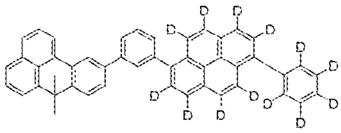
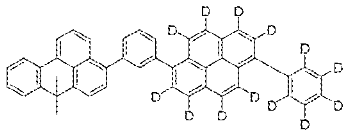
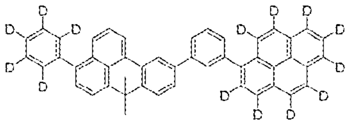
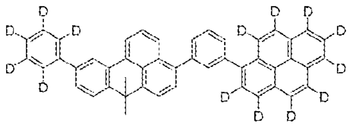
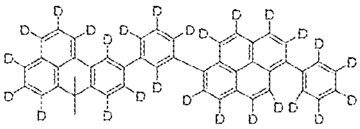
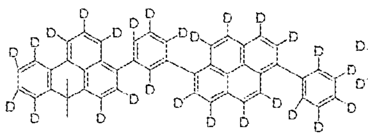
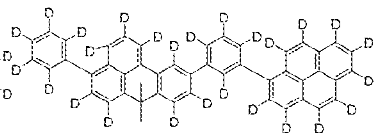
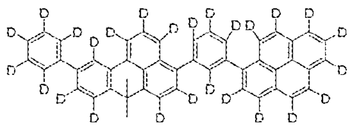
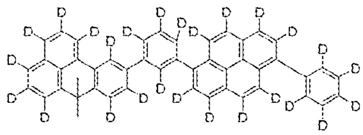
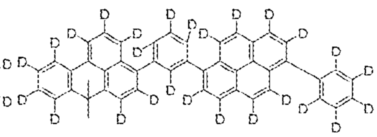
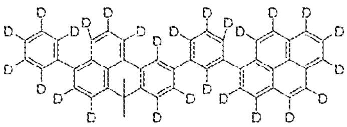
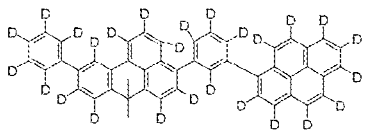

[Formula 207]

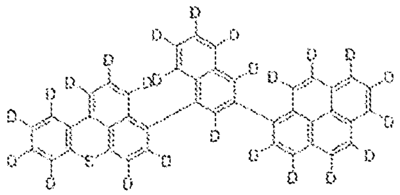
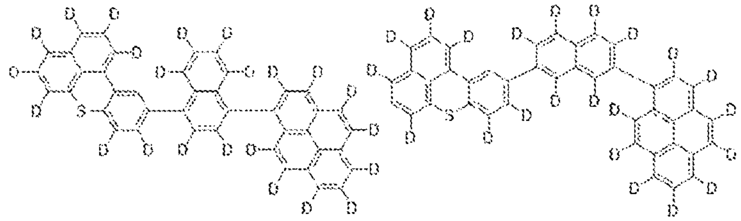
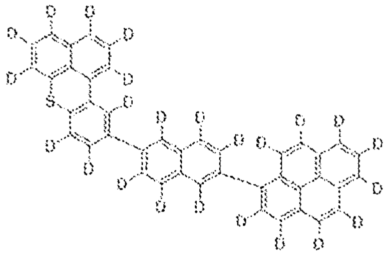
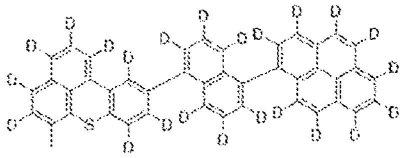
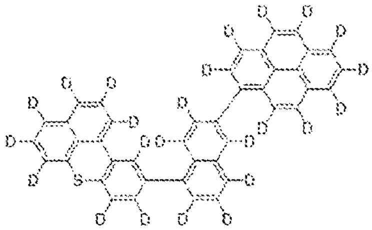
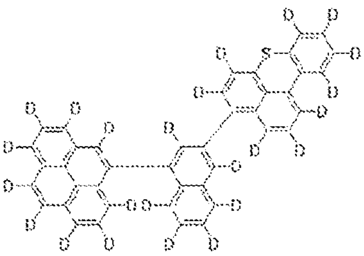
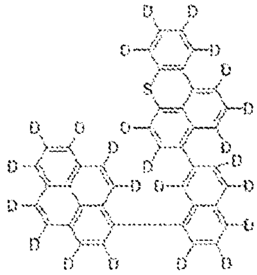
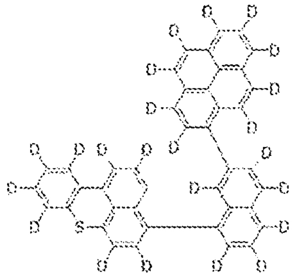
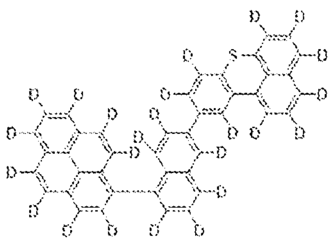
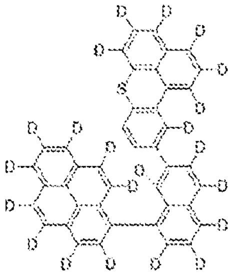
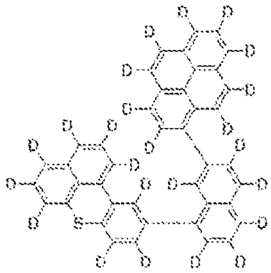
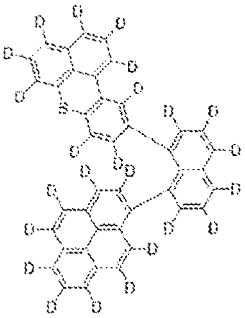
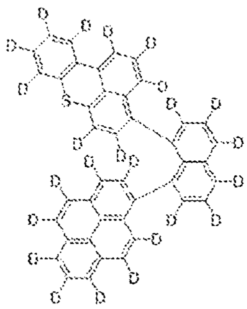
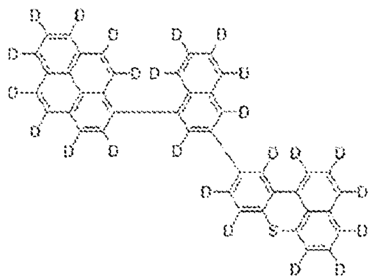

[Formula 208]

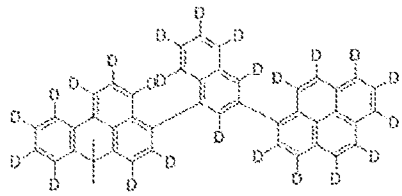

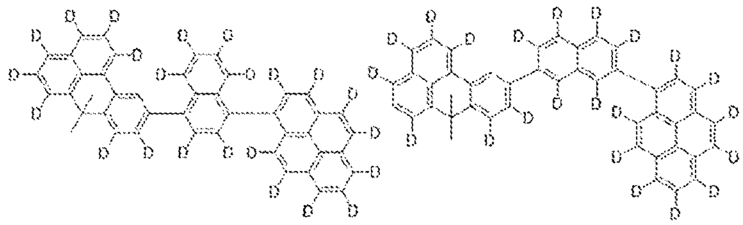

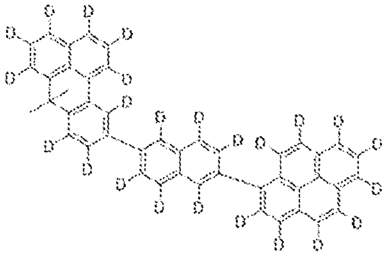

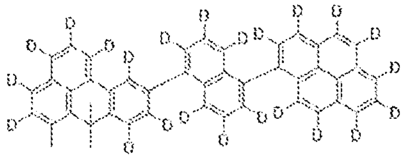

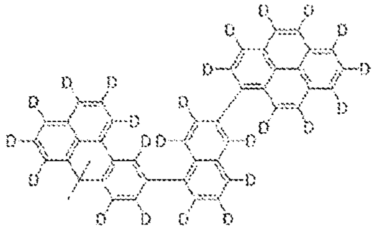

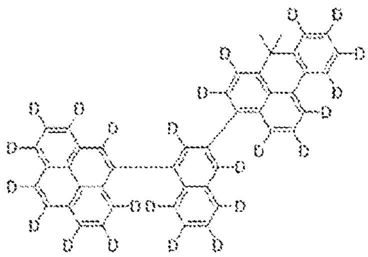

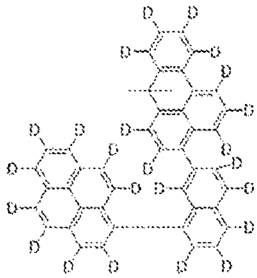

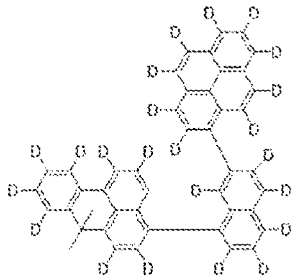

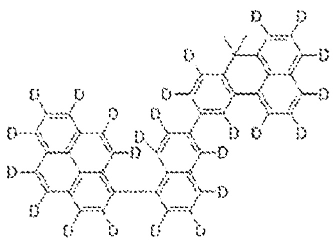

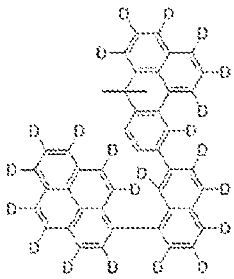

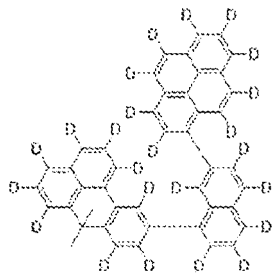

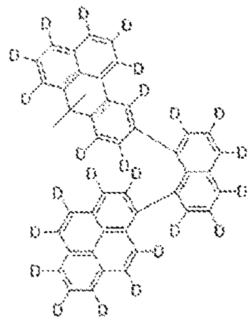

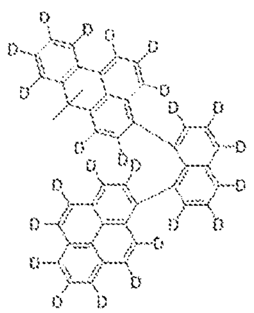

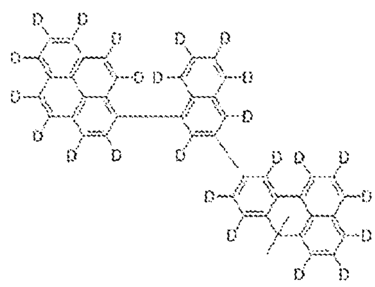

[Formula 209]

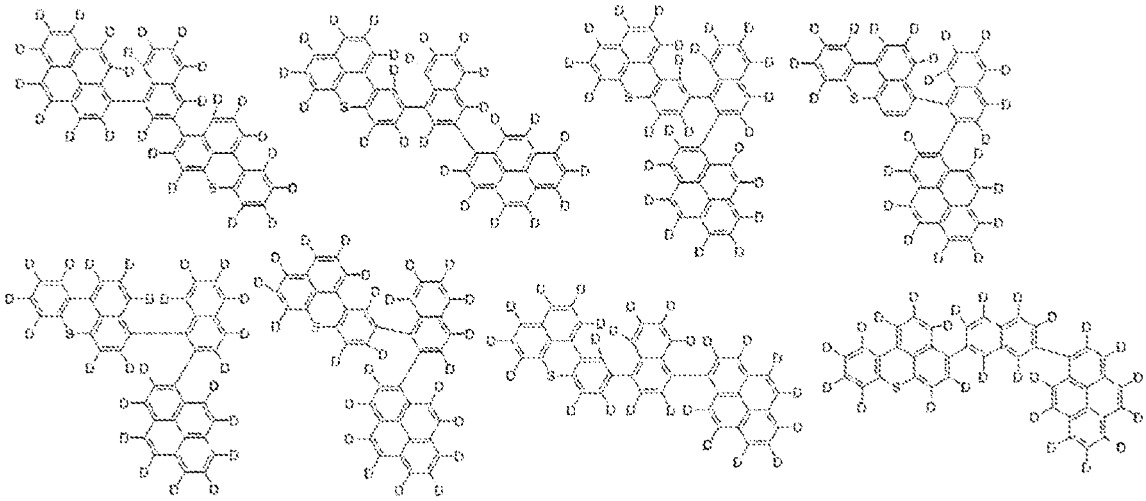

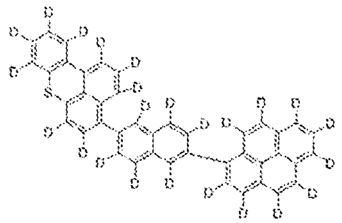

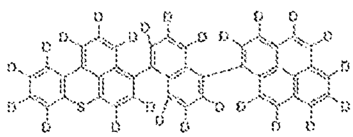

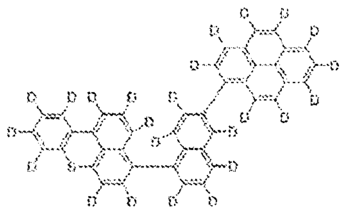

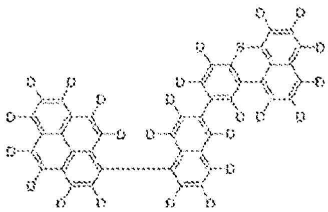

[Formula 210]

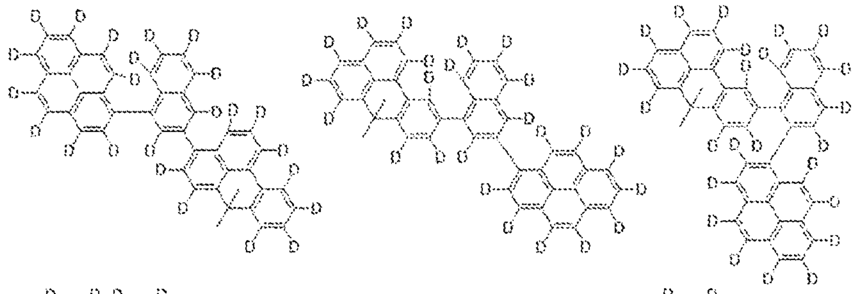

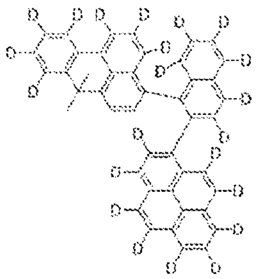

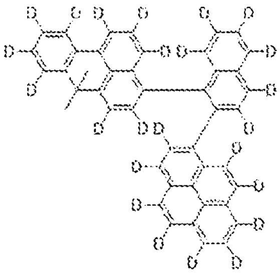

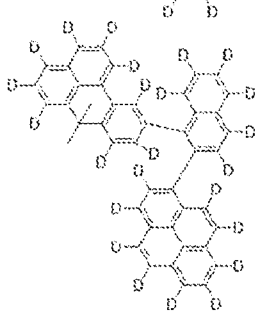

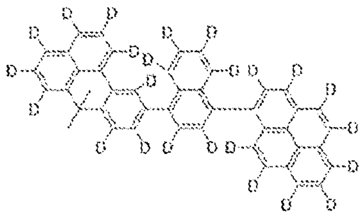

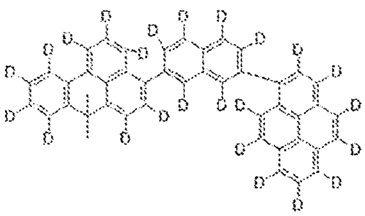

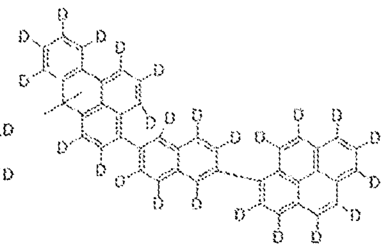

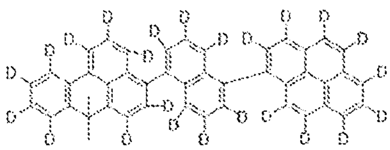

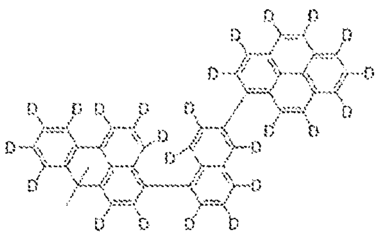

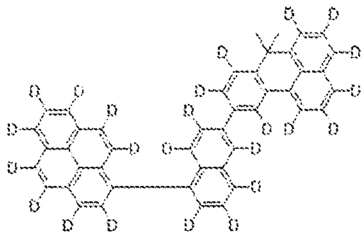

[Formula 211]

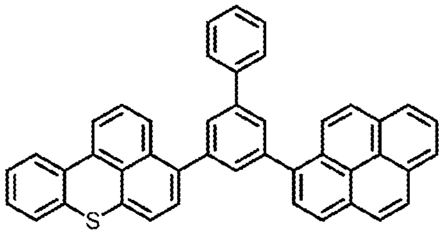
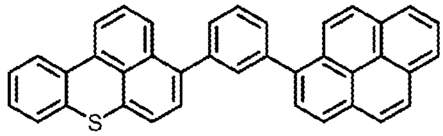

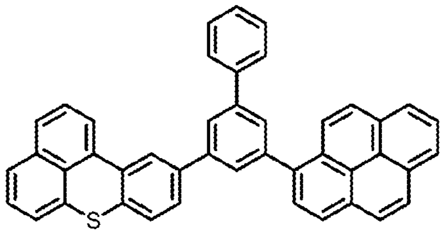
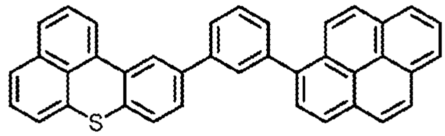

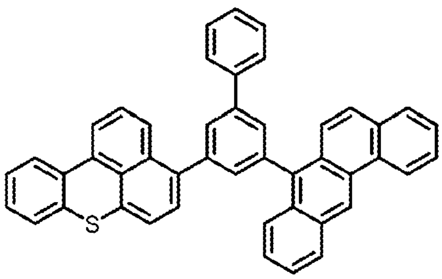
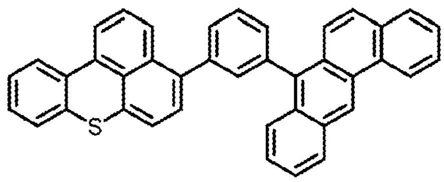

[Formula 212]

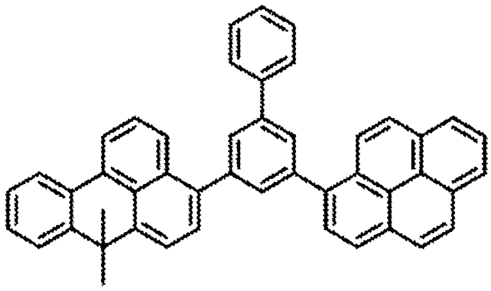

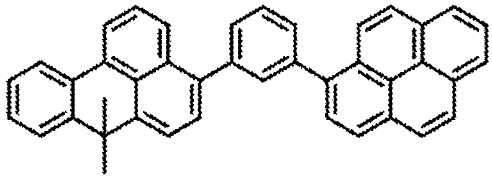

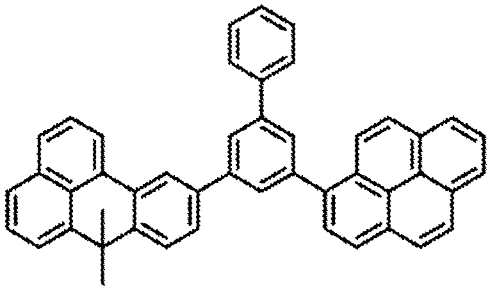

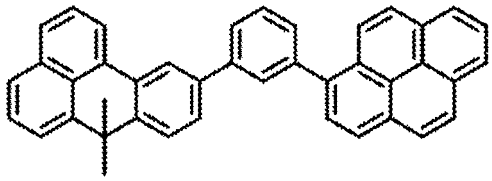

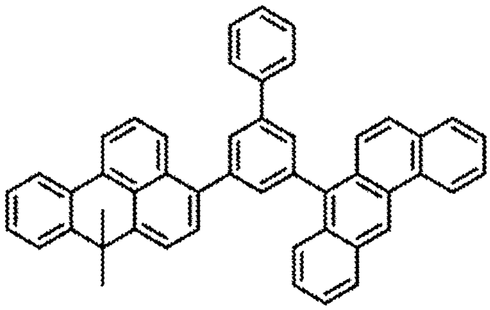

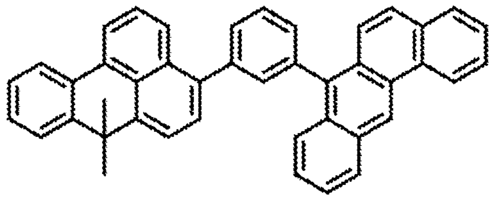

[Formula 213]

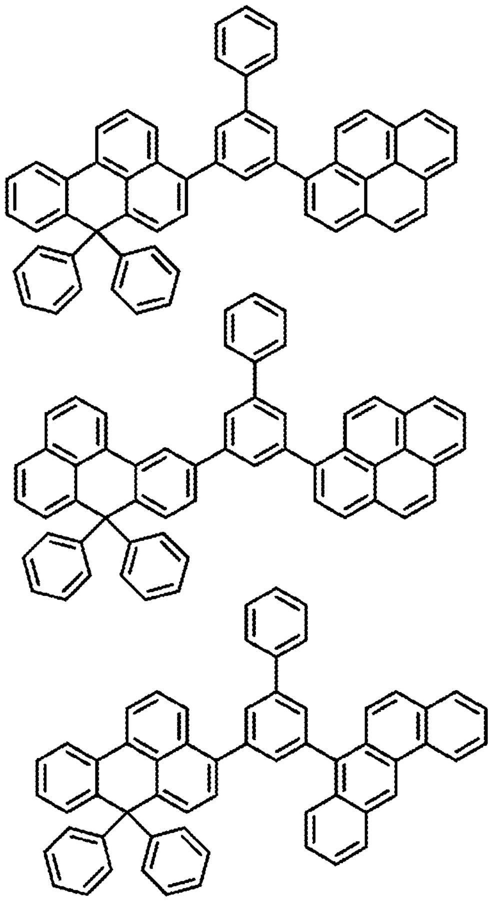

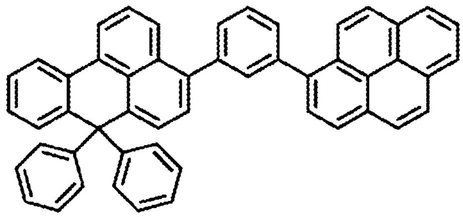

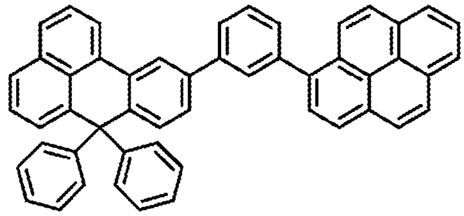

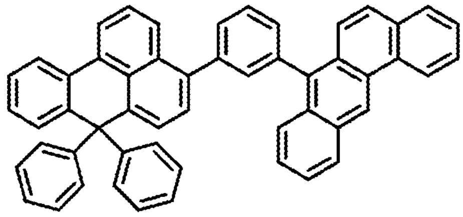

[Formula 214]

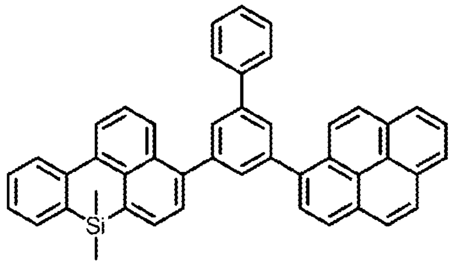
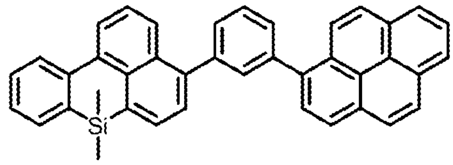

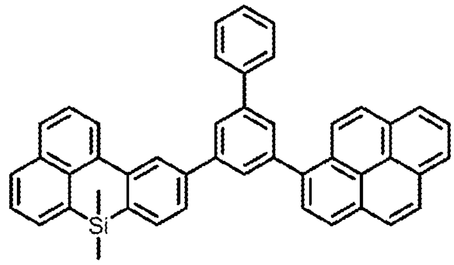
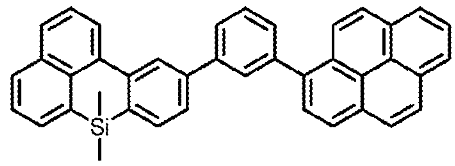

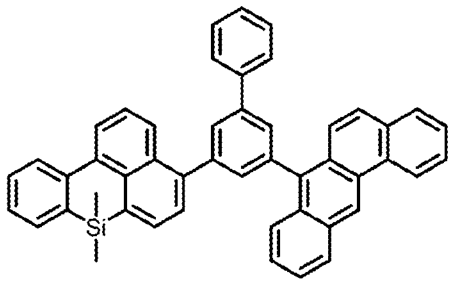
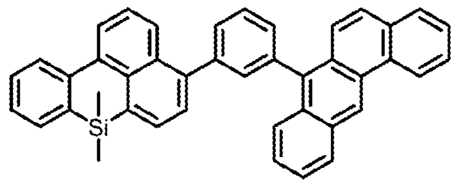

[Formula 215]

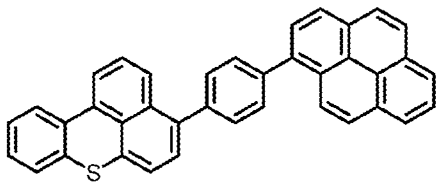
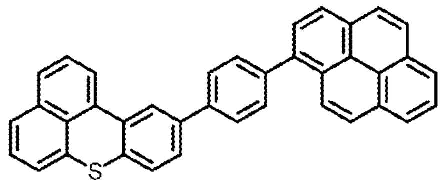

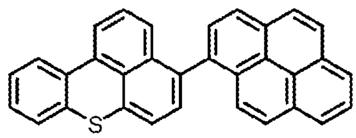
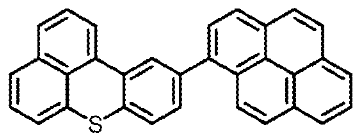

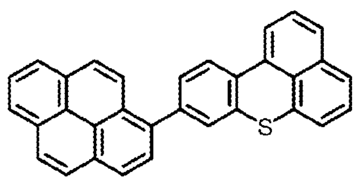

[Formula 216]

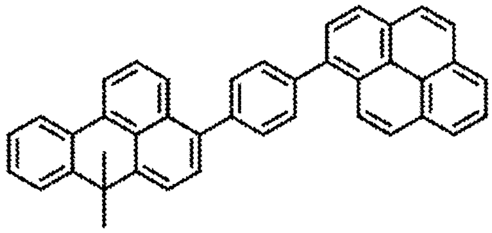

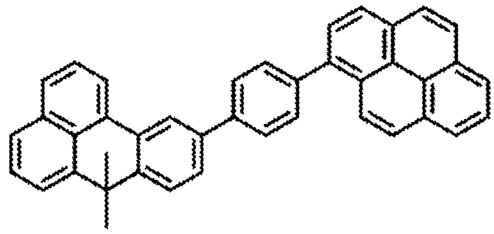

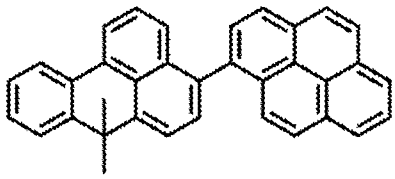

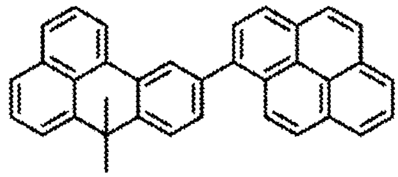

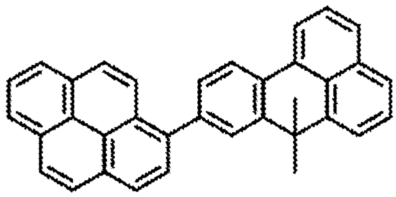

[Formula 217]

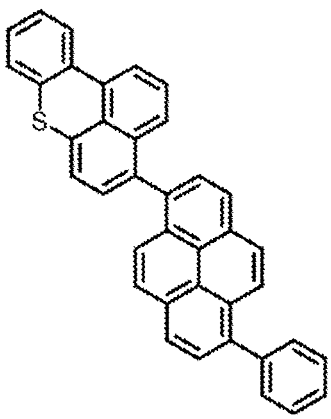

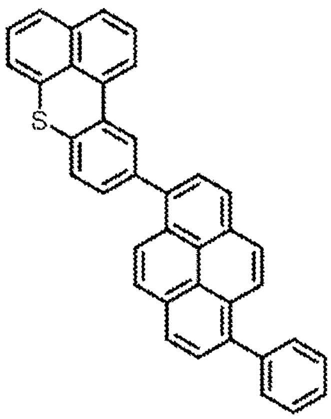

[Formula 218]

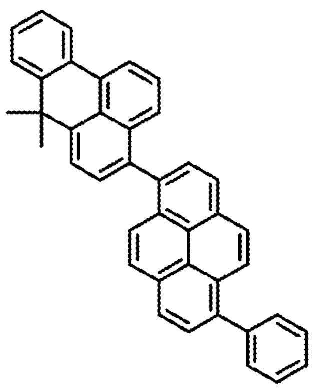

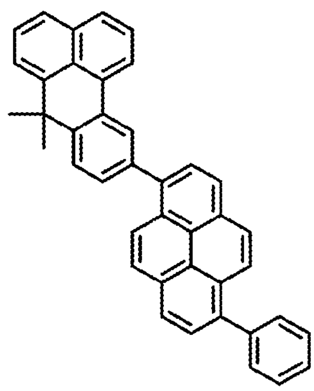

[Formula 219]

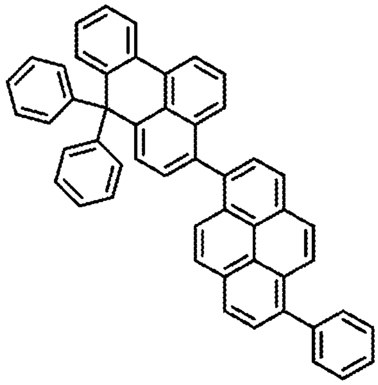

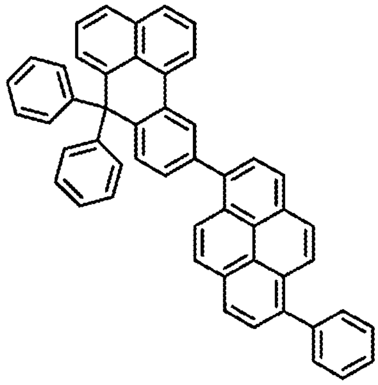

[Formula 220]

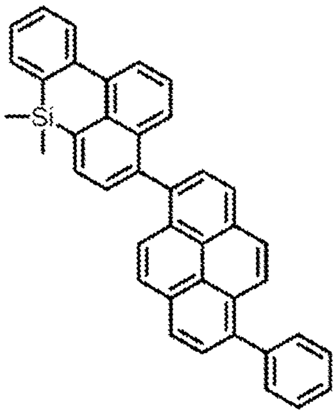

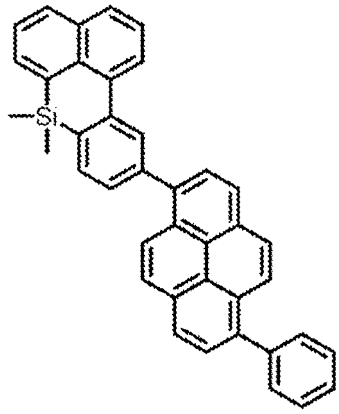

[Formula 221]

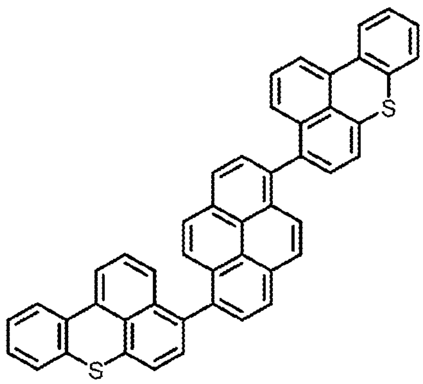

[Formula 222]

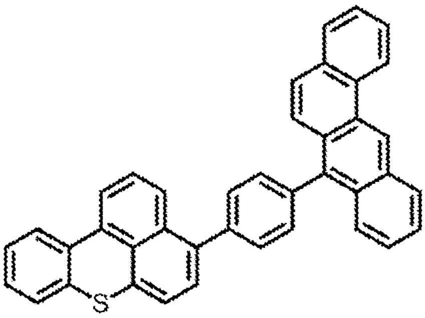

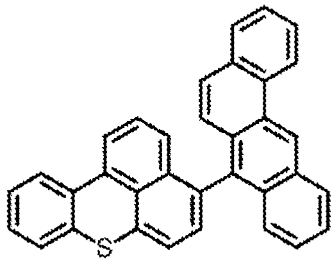

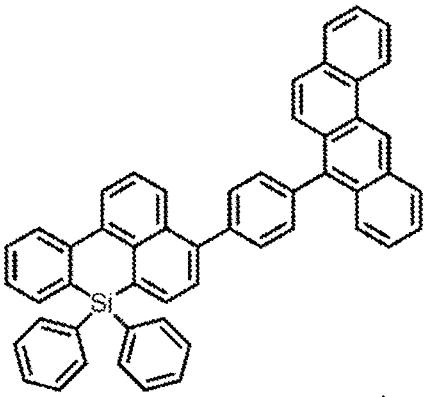

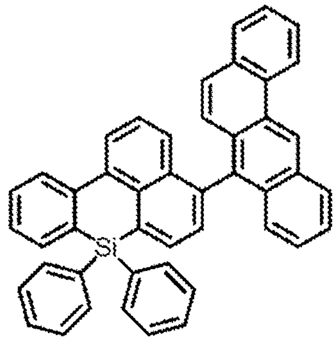

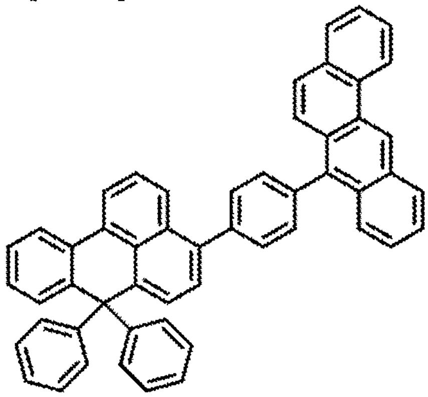

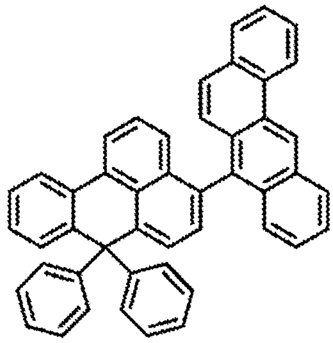

[Formula 223]

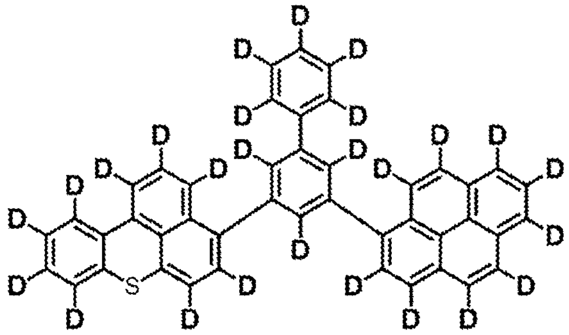

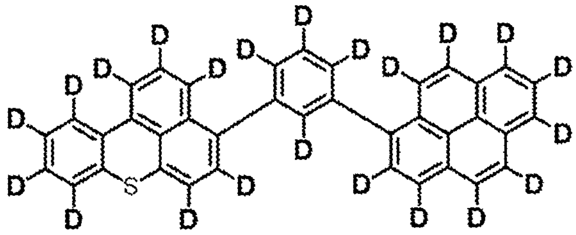

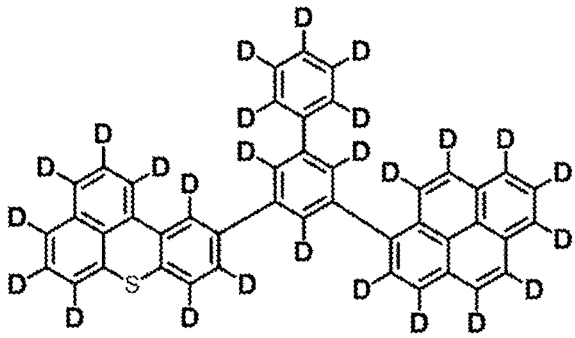

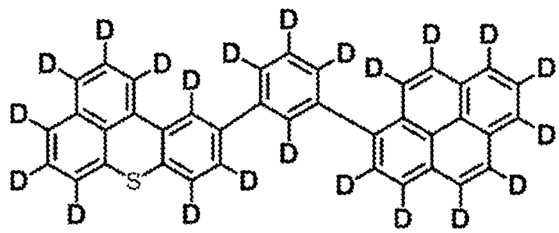

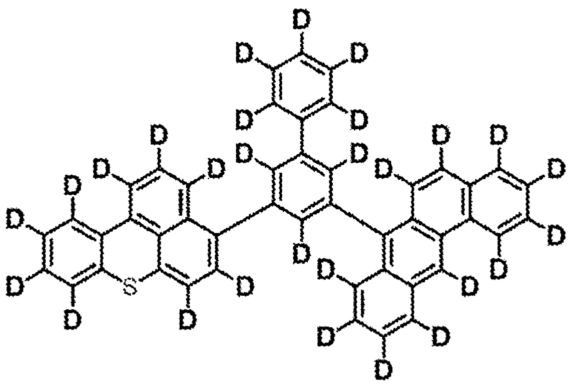

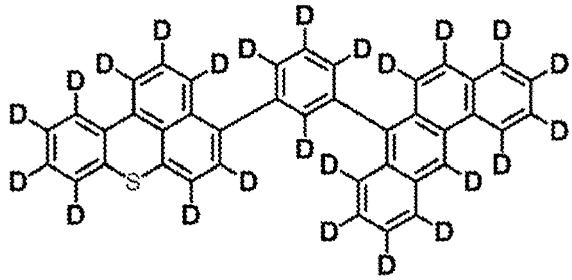

[Formula 224]

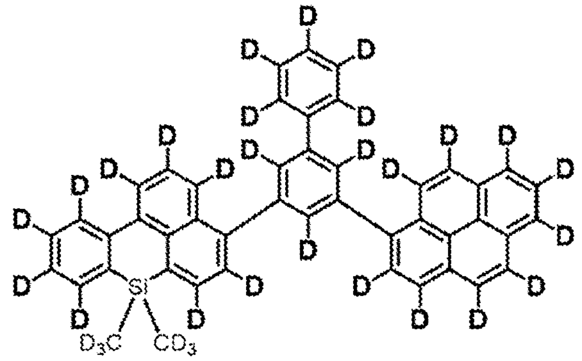

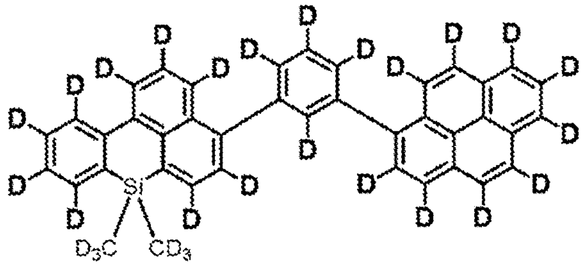

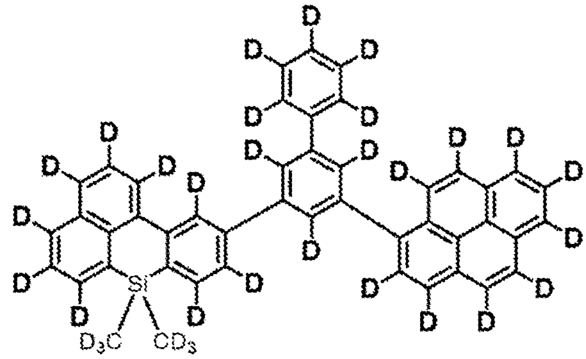

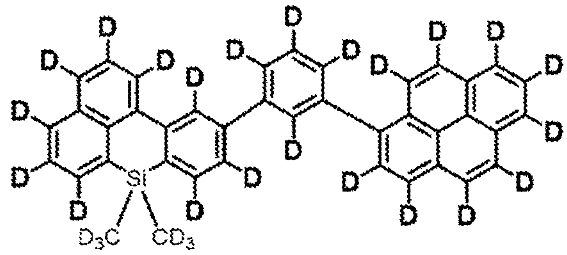

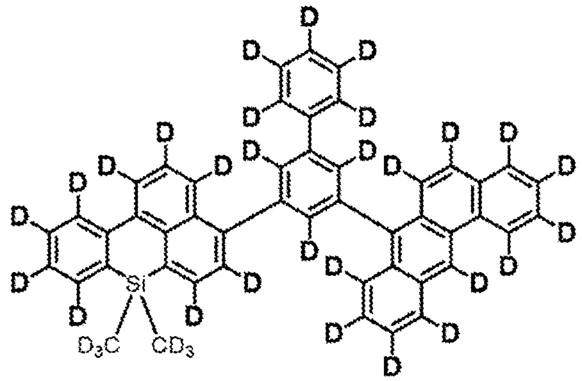

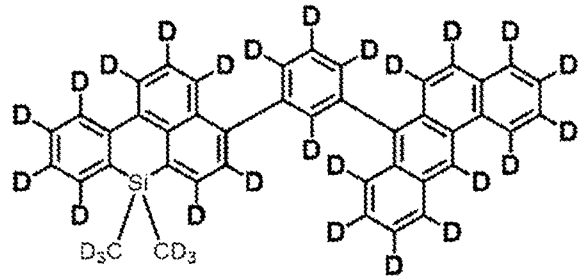

[Formula 225]

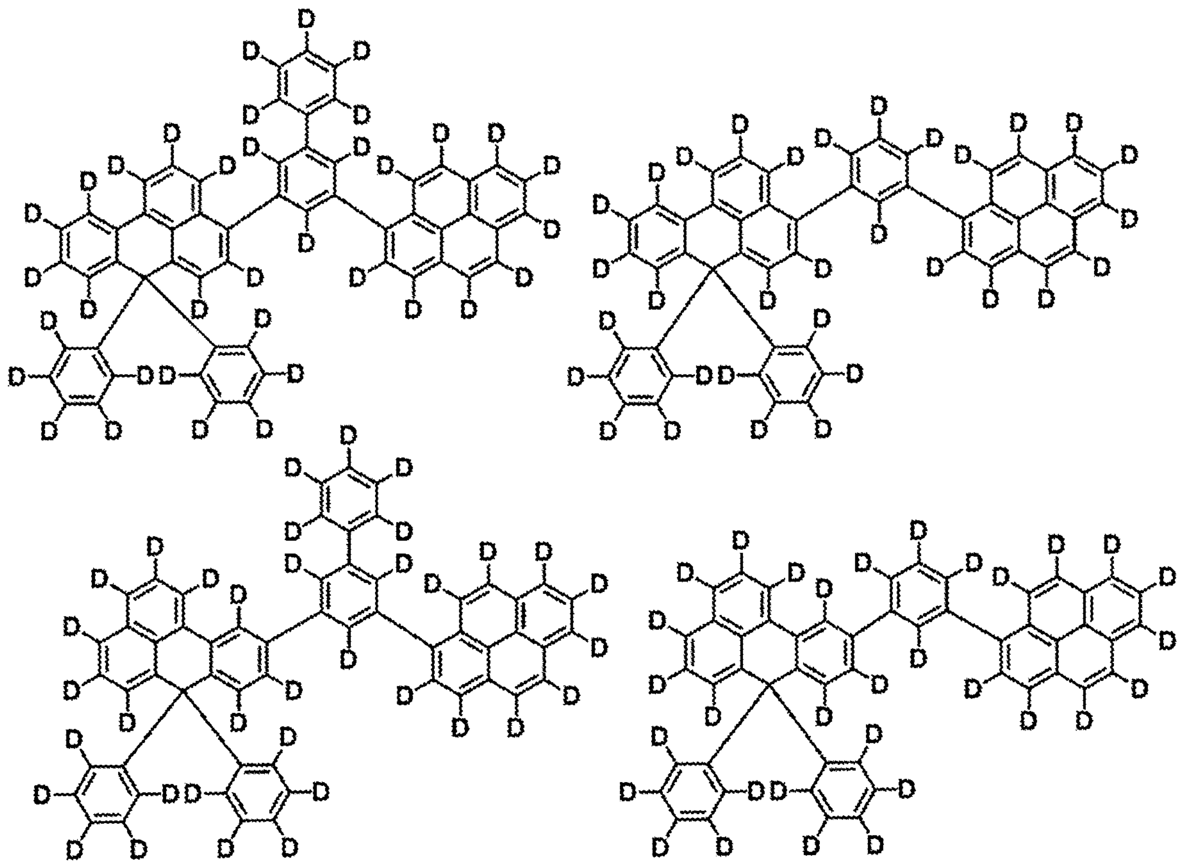

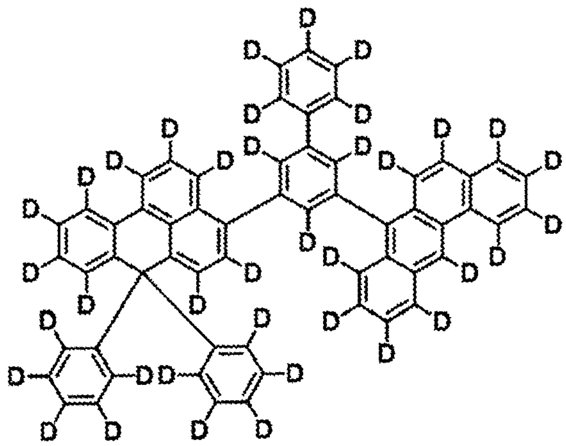

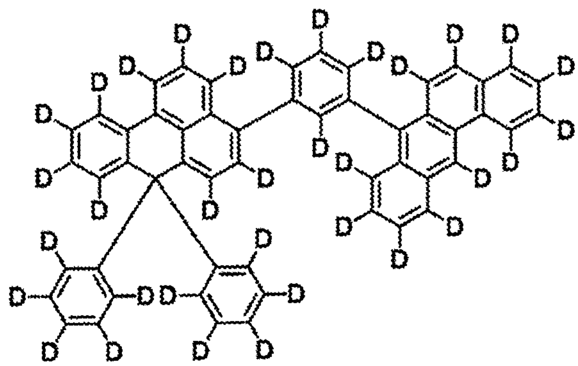

[Formula 226]

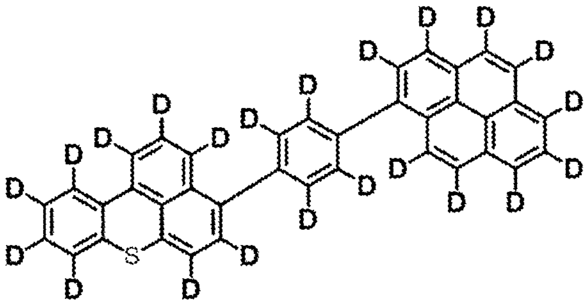

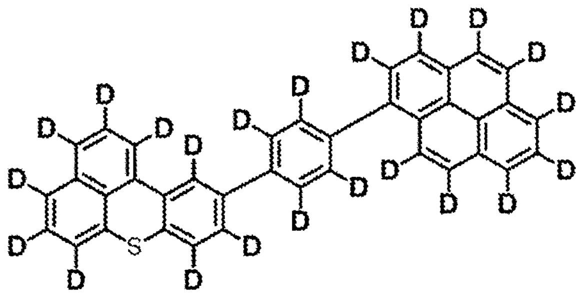

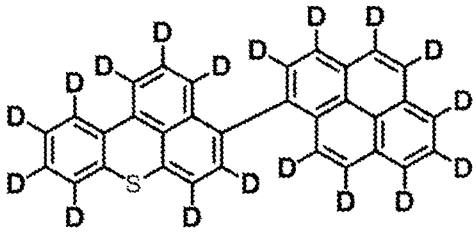

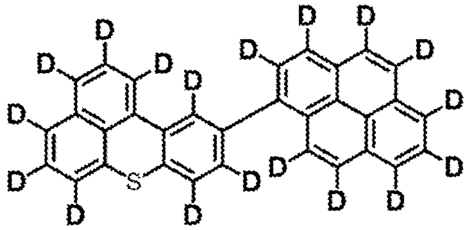

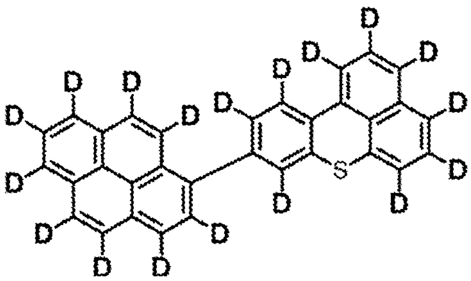

[Formula 227]

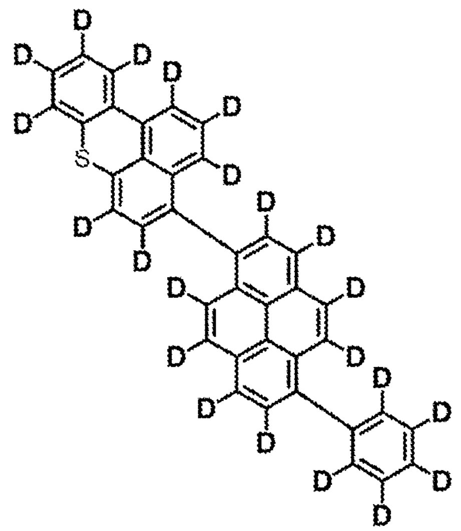

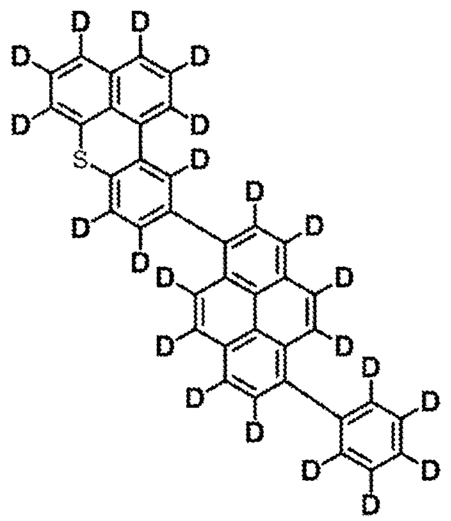

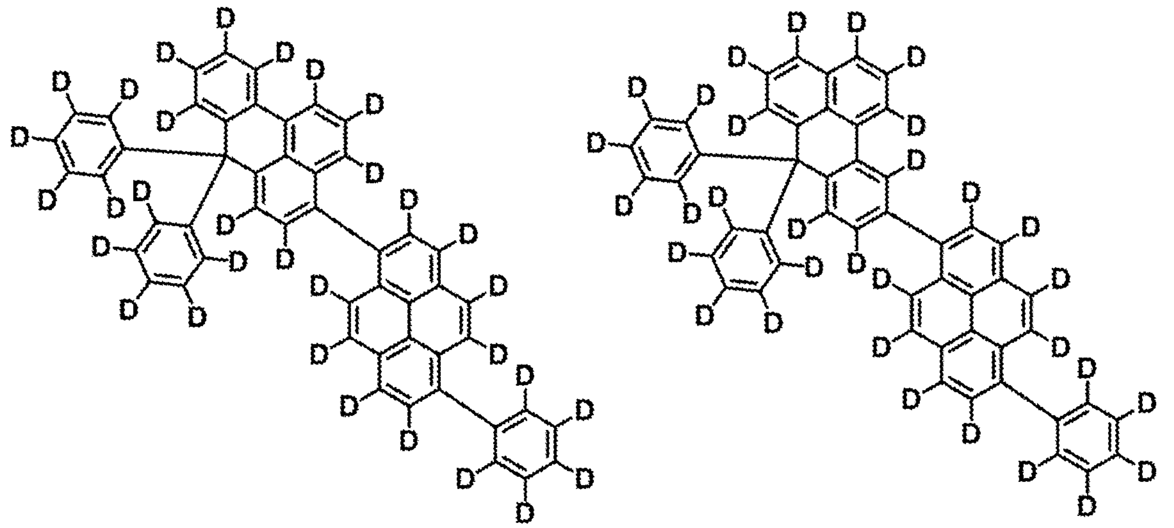

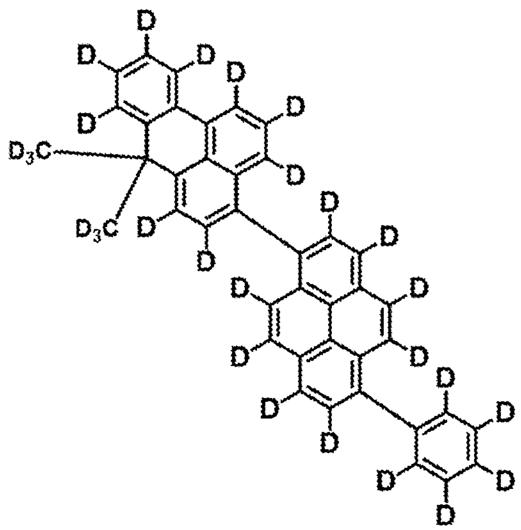

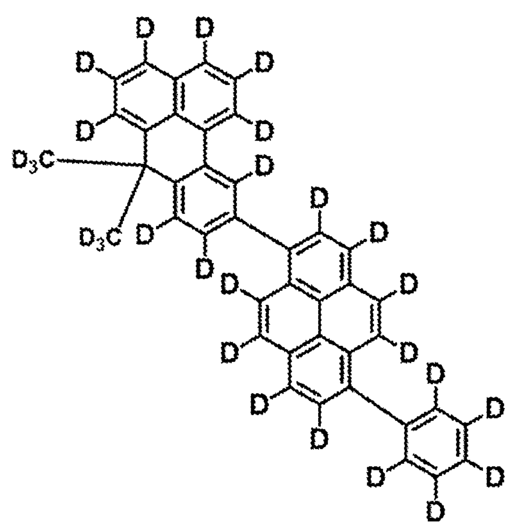

[Formula 228]

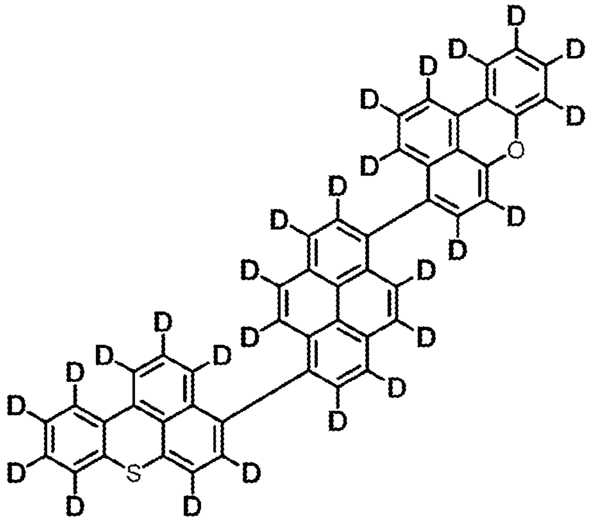

[Formula 229]

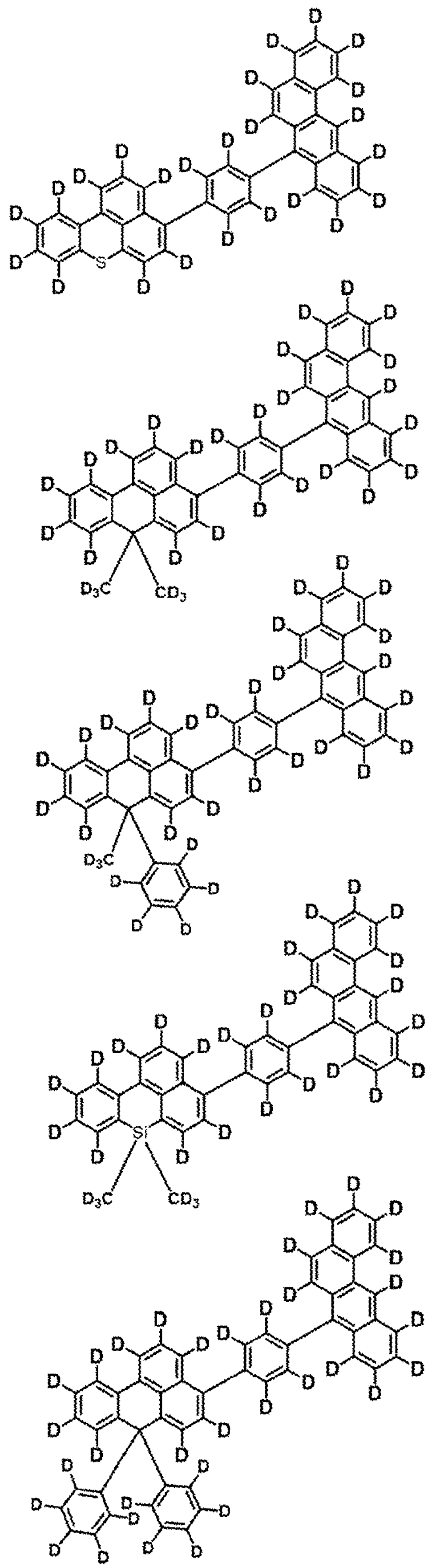

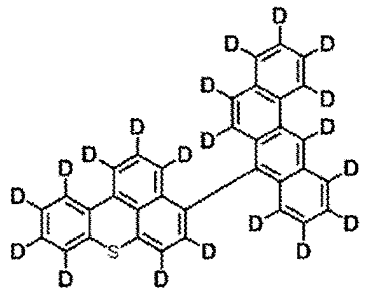

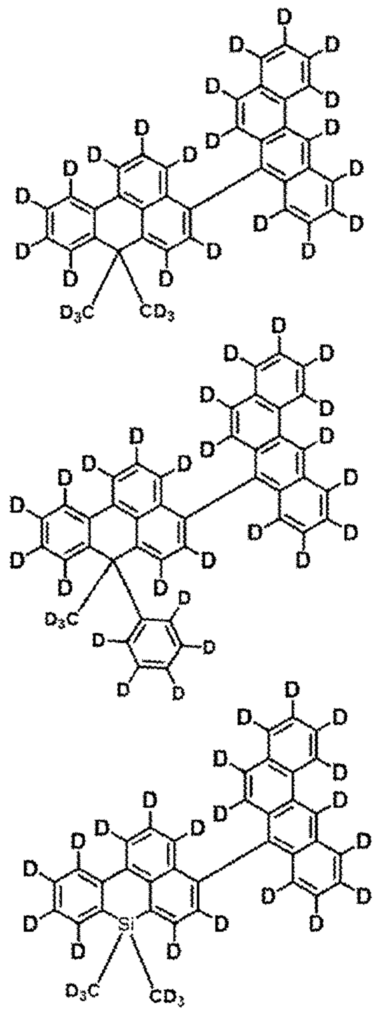

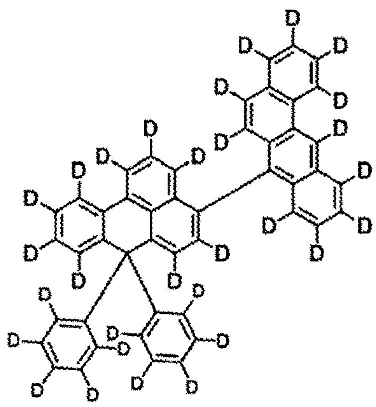

[Formula 230]

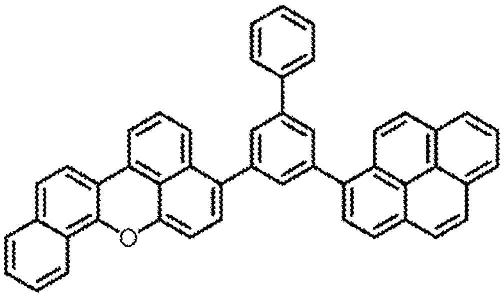
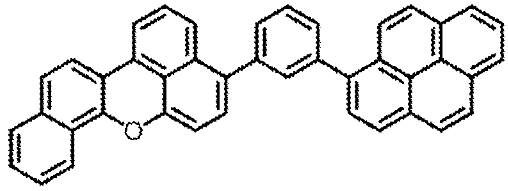
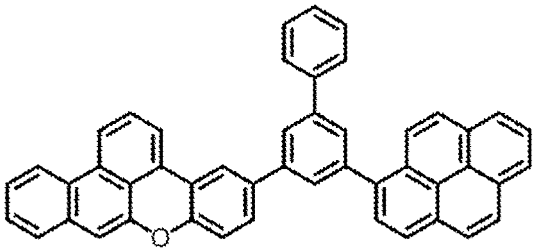
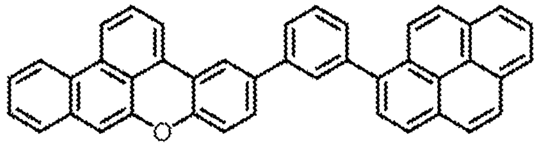
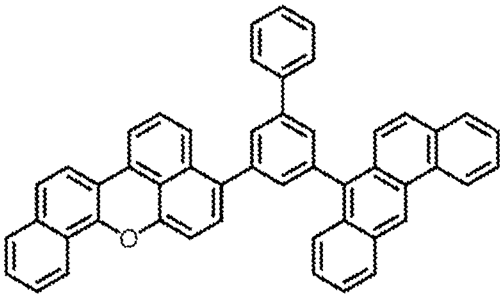
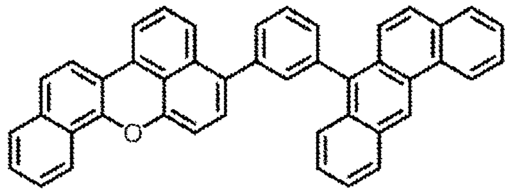

[Formula 231]

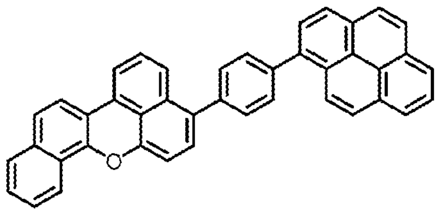
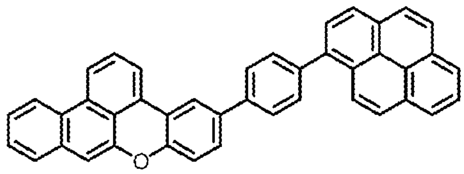
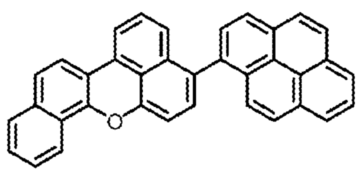
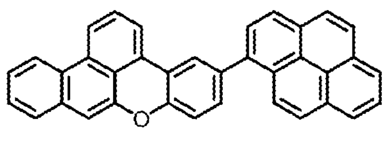
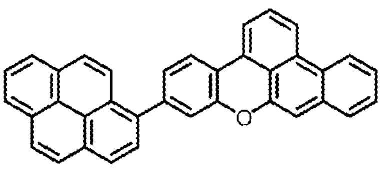

[Formula 232]

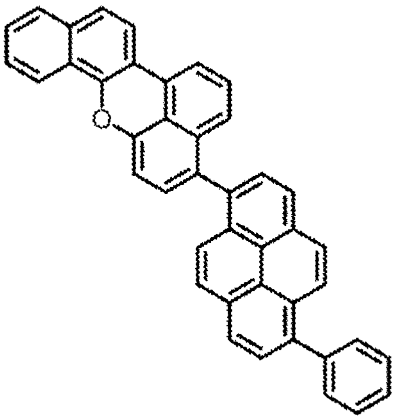

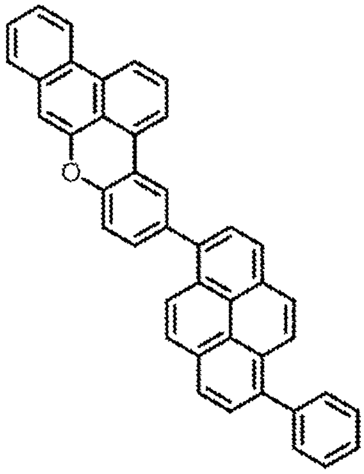

[Formula 233]

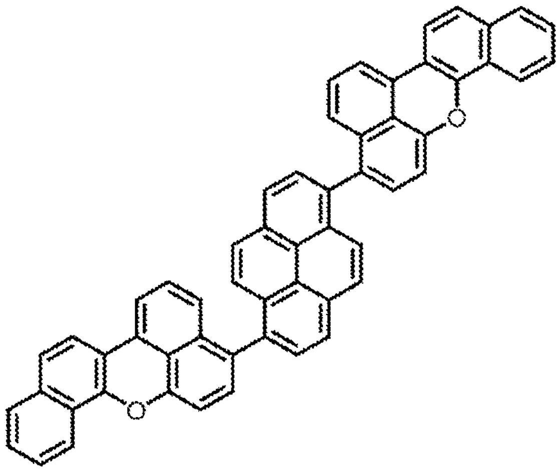

[Formula 234]

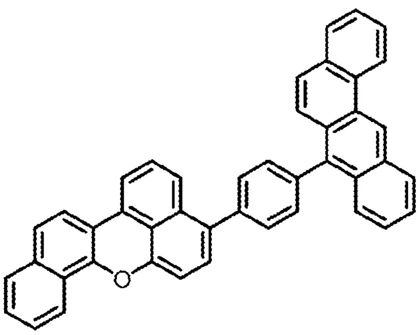

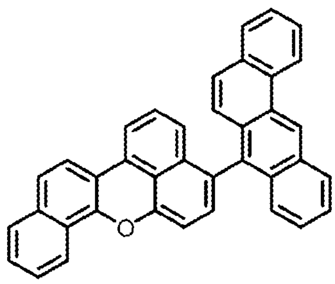

[Formula 235]

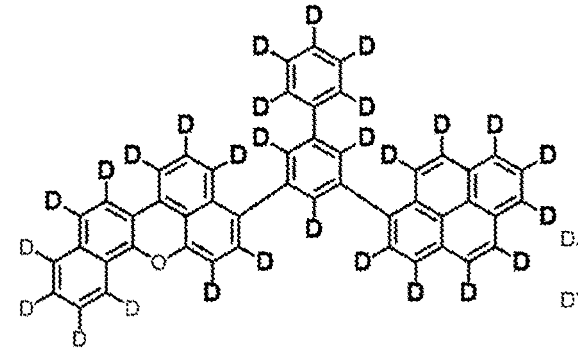

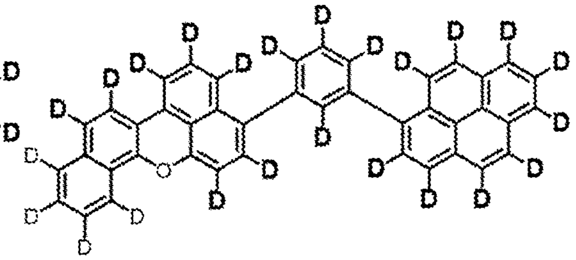

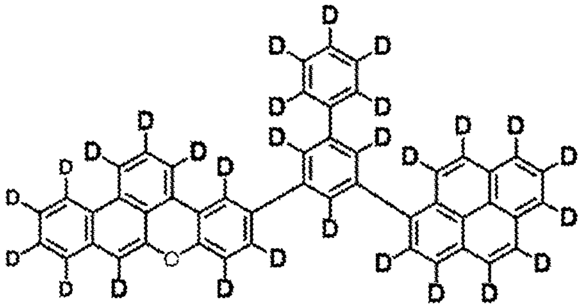

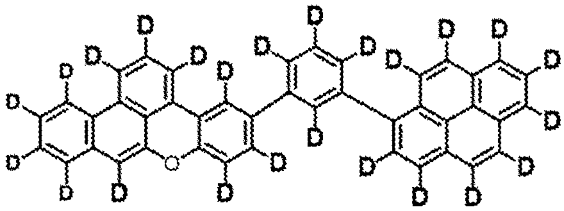

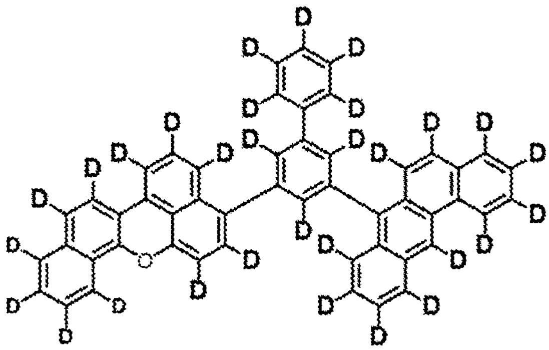

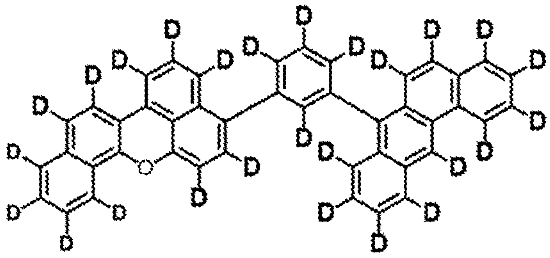

[Formula 236]

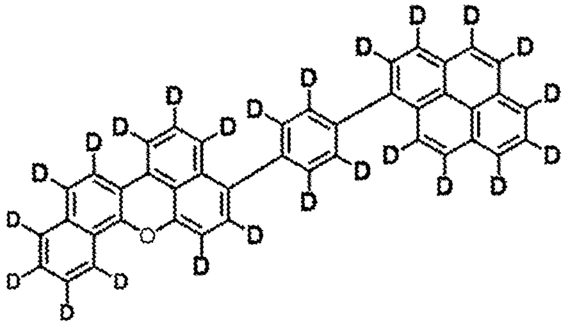

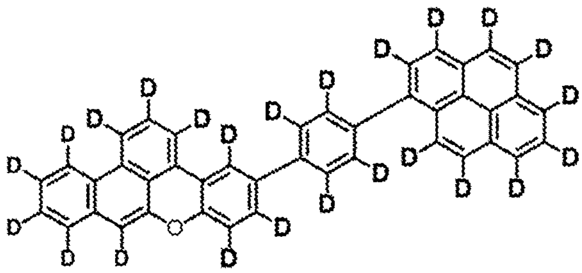

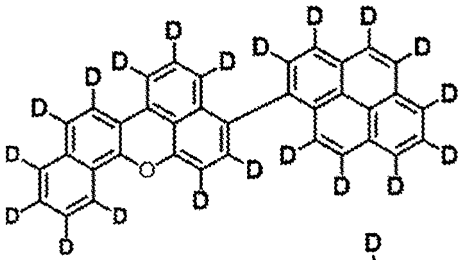

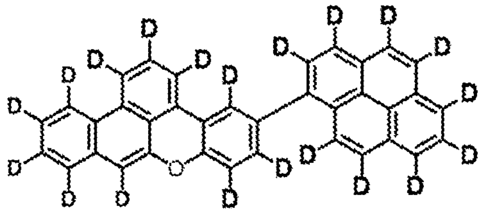

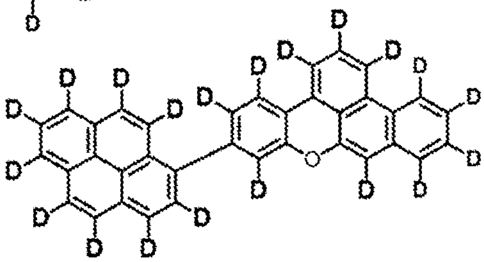

[Formula 237]

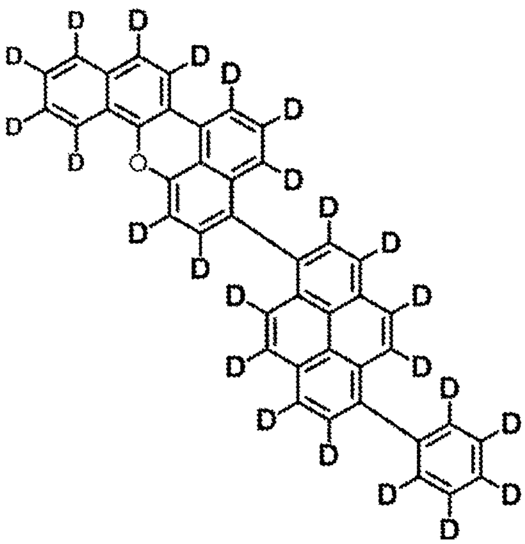

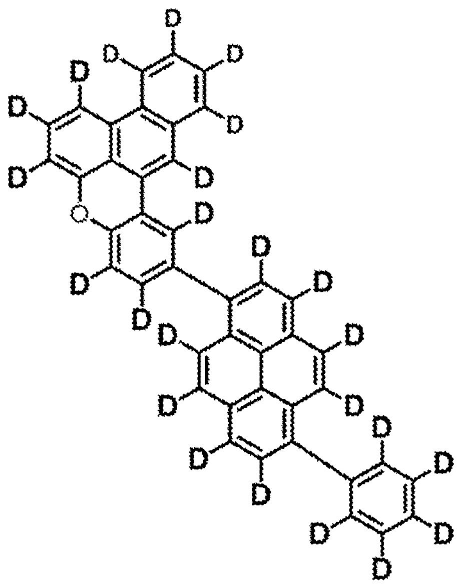

[Formula 238]

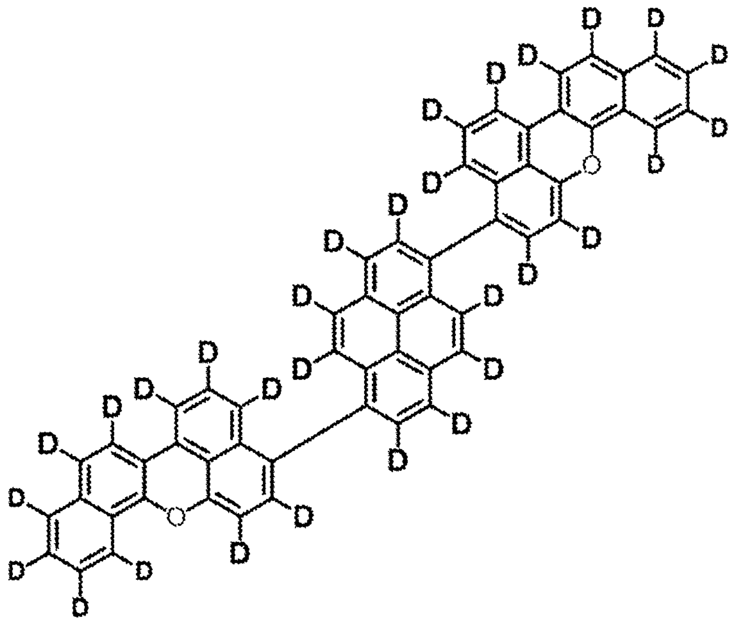

[Formula 239]

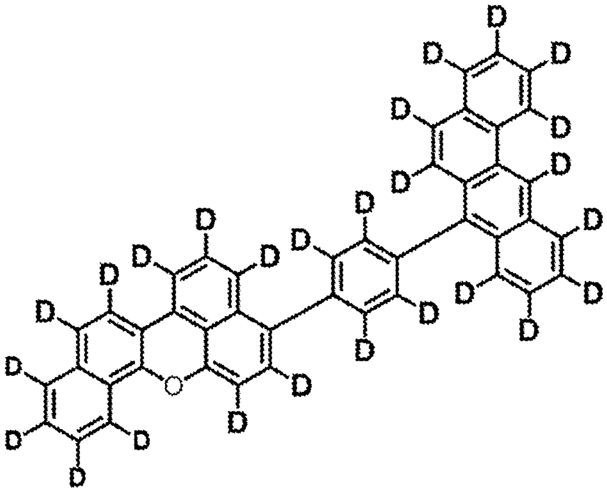

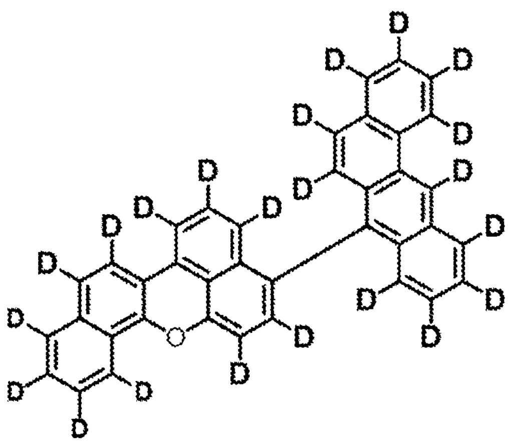

[Formula 240]

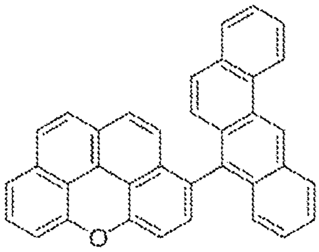
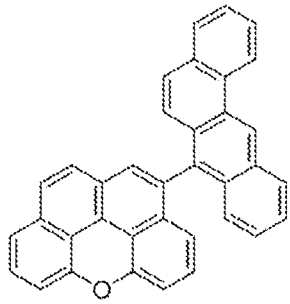
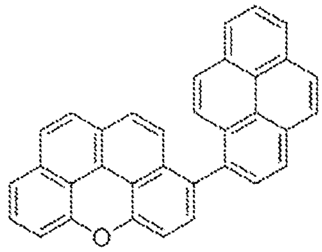

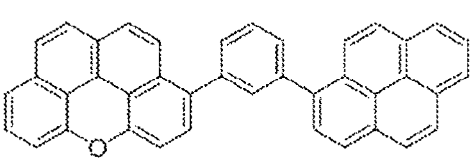
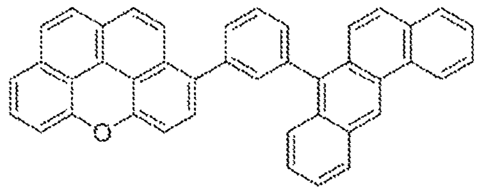

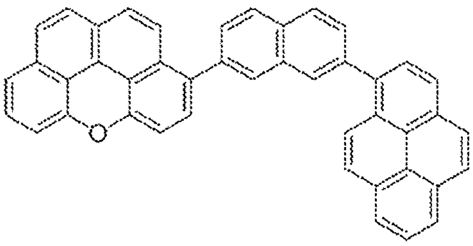
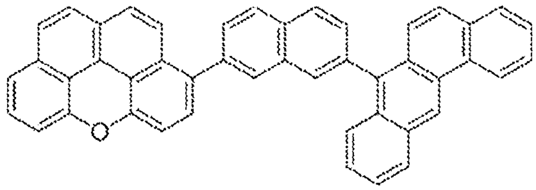

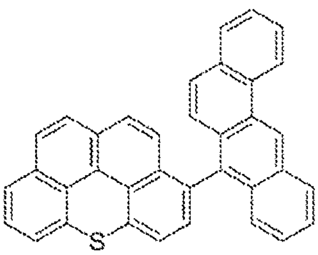
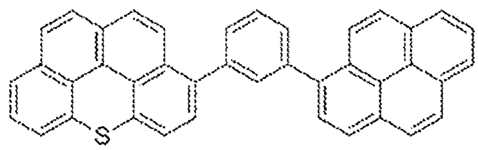

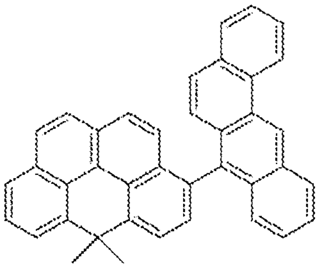
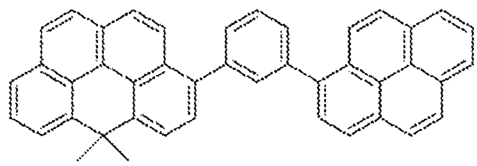

[Formula 241]

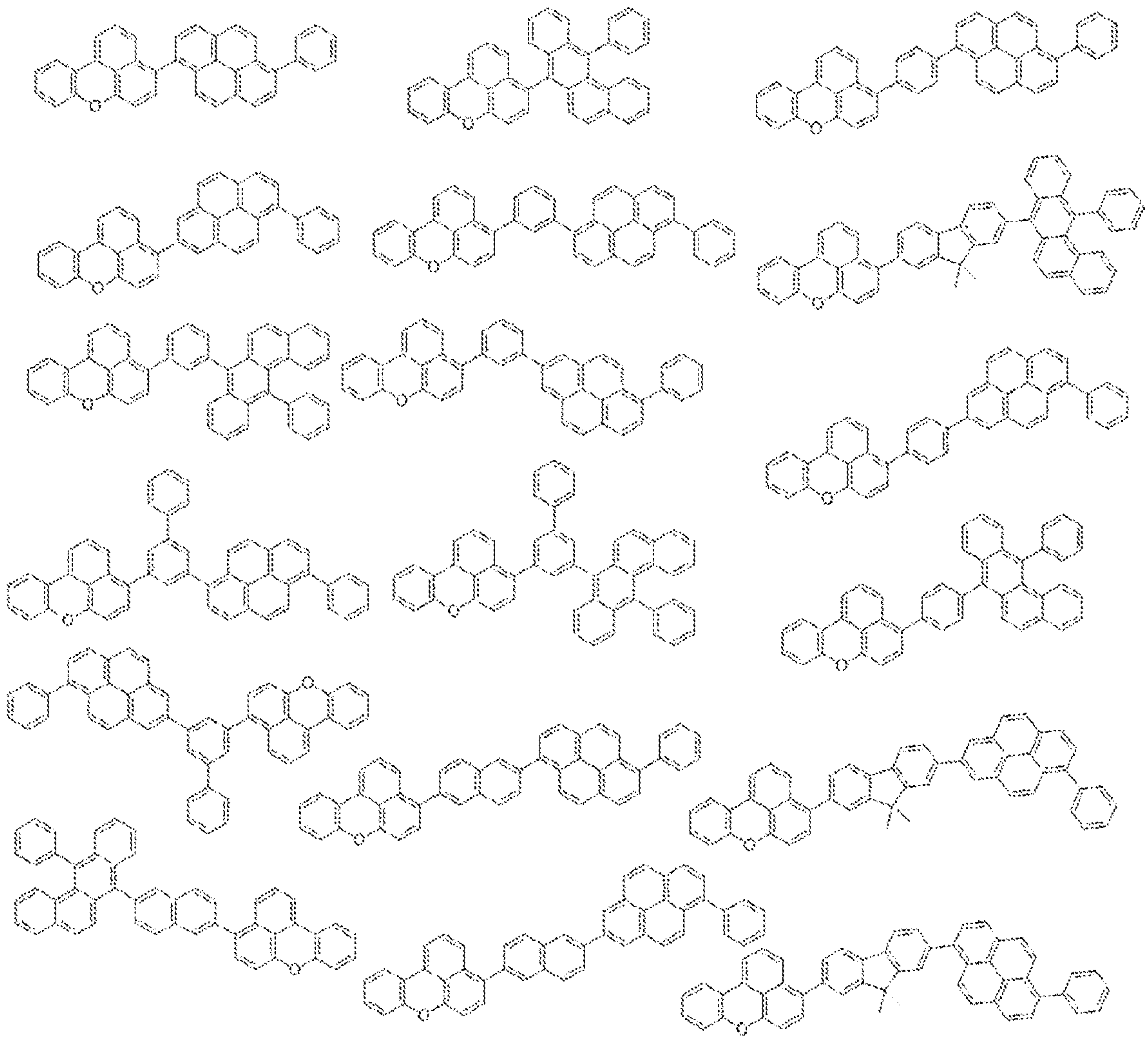

[Formula 242]

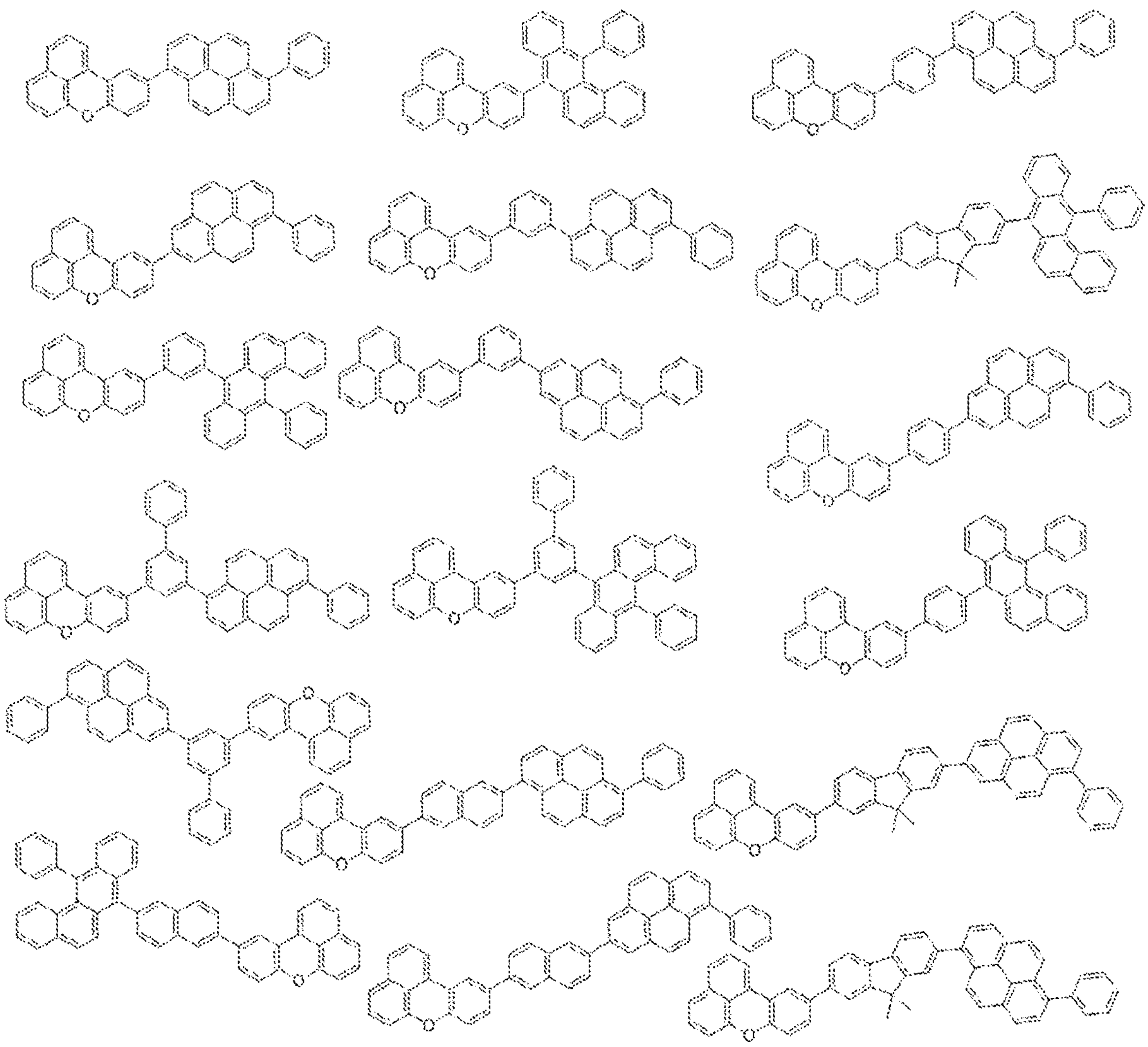

[Formula 243]

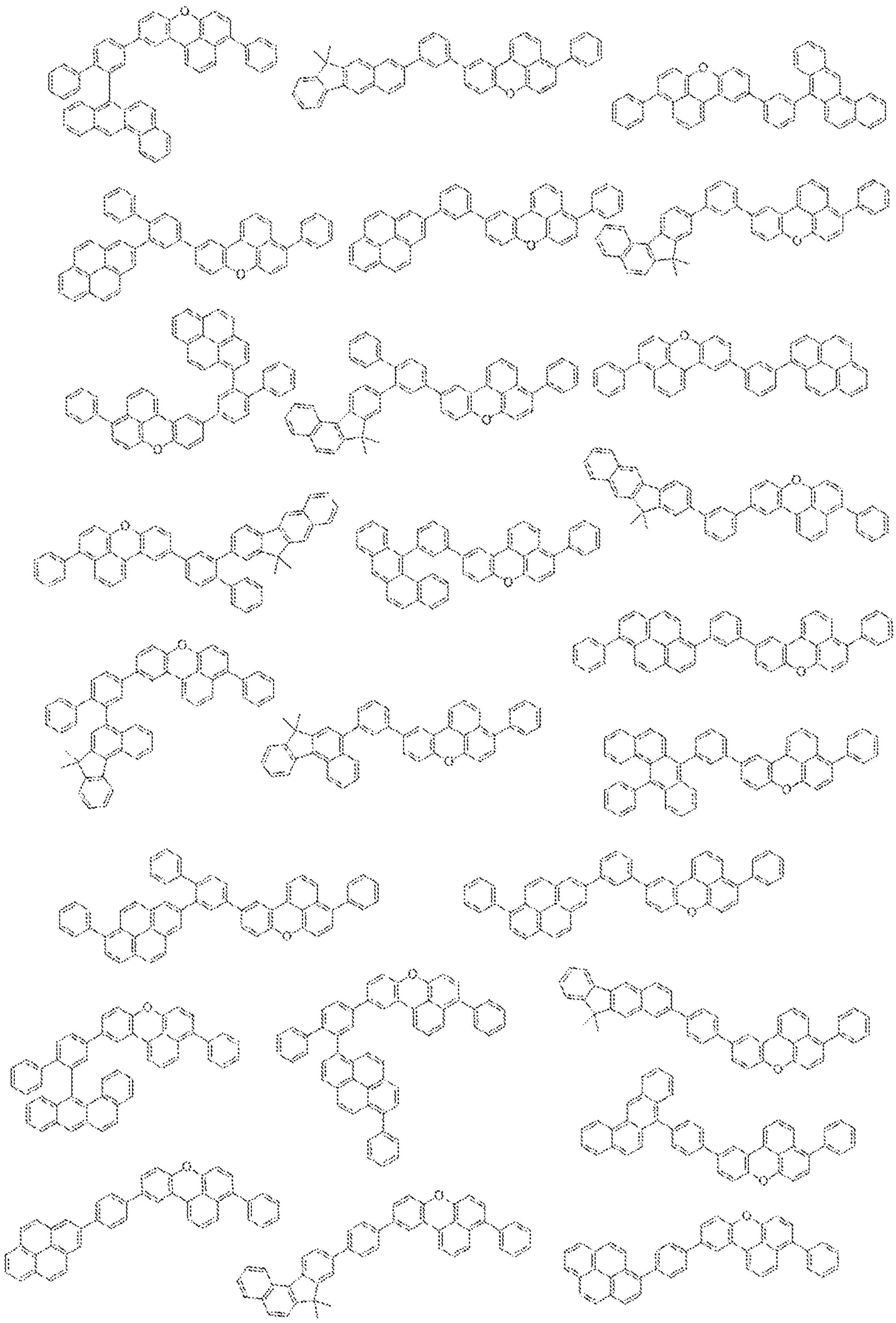

[Formula 244]

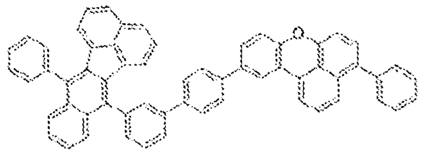

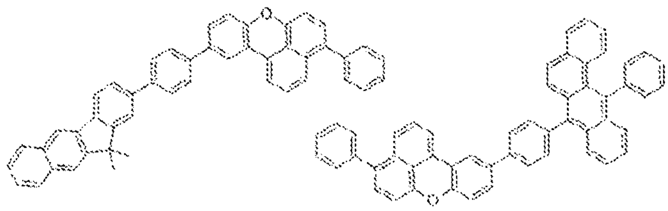

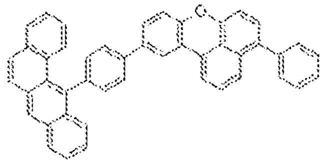

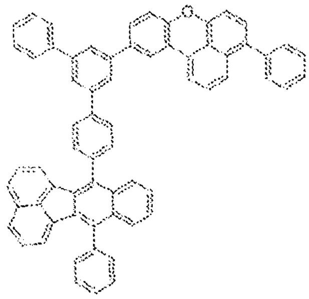

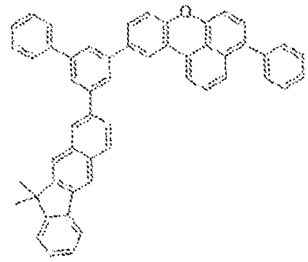

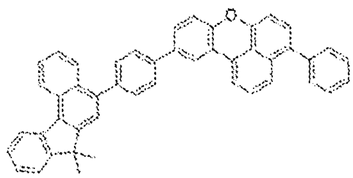

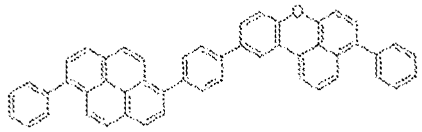

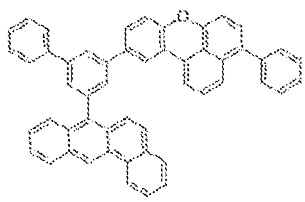

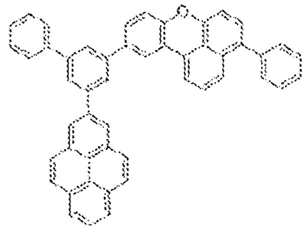

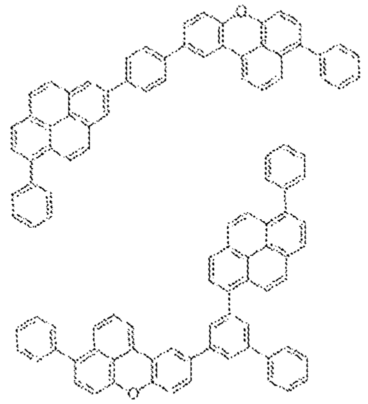

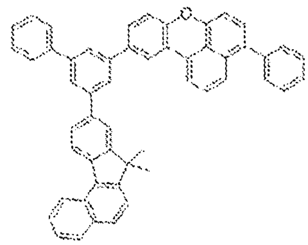

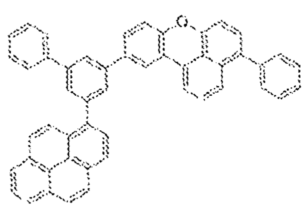

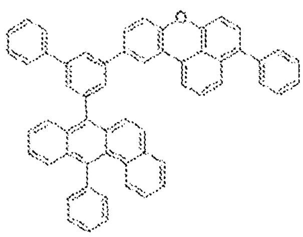

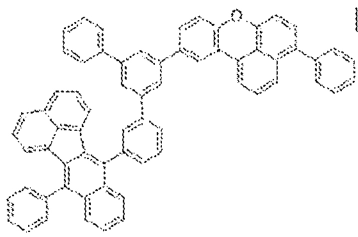

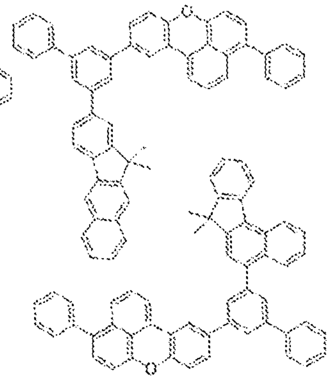

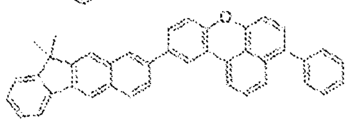

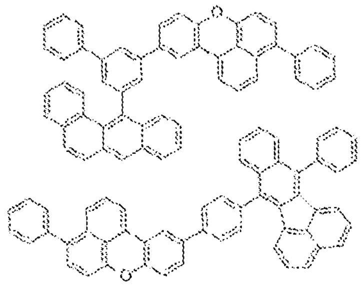

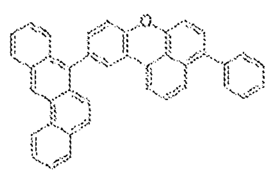

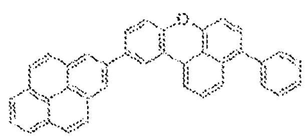

[Formula 245]

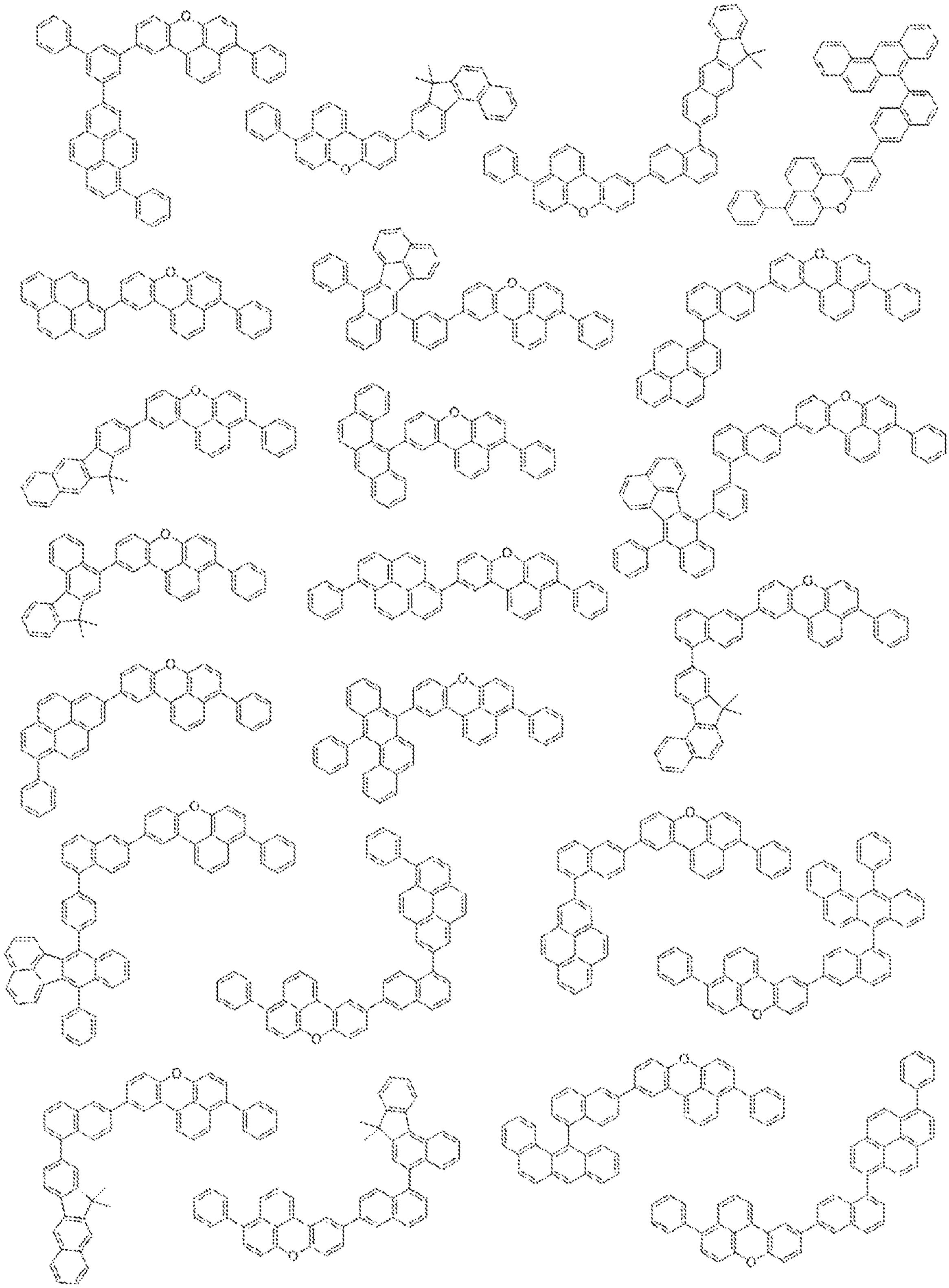

[Formula 246]

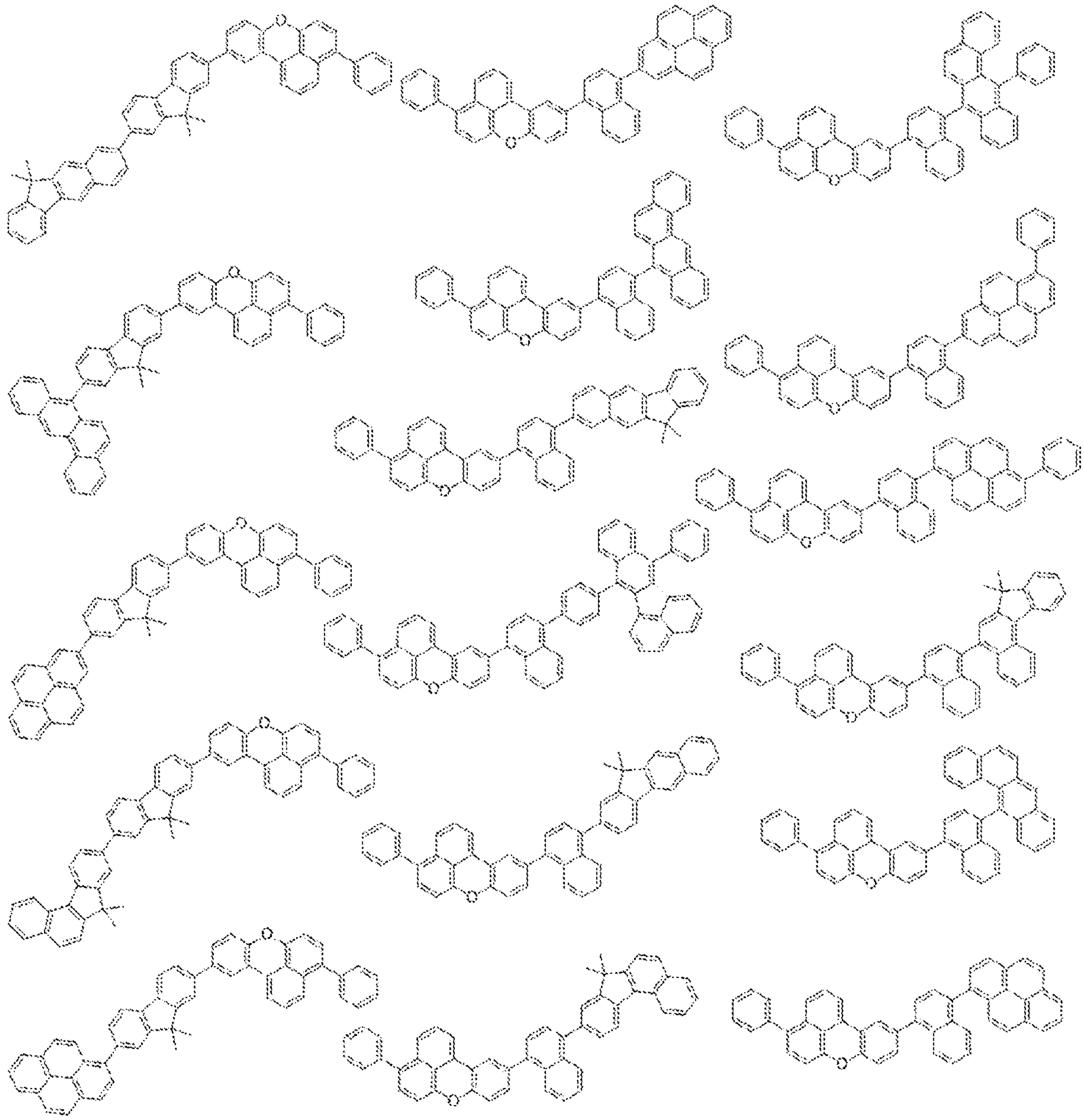

[Formula 247]

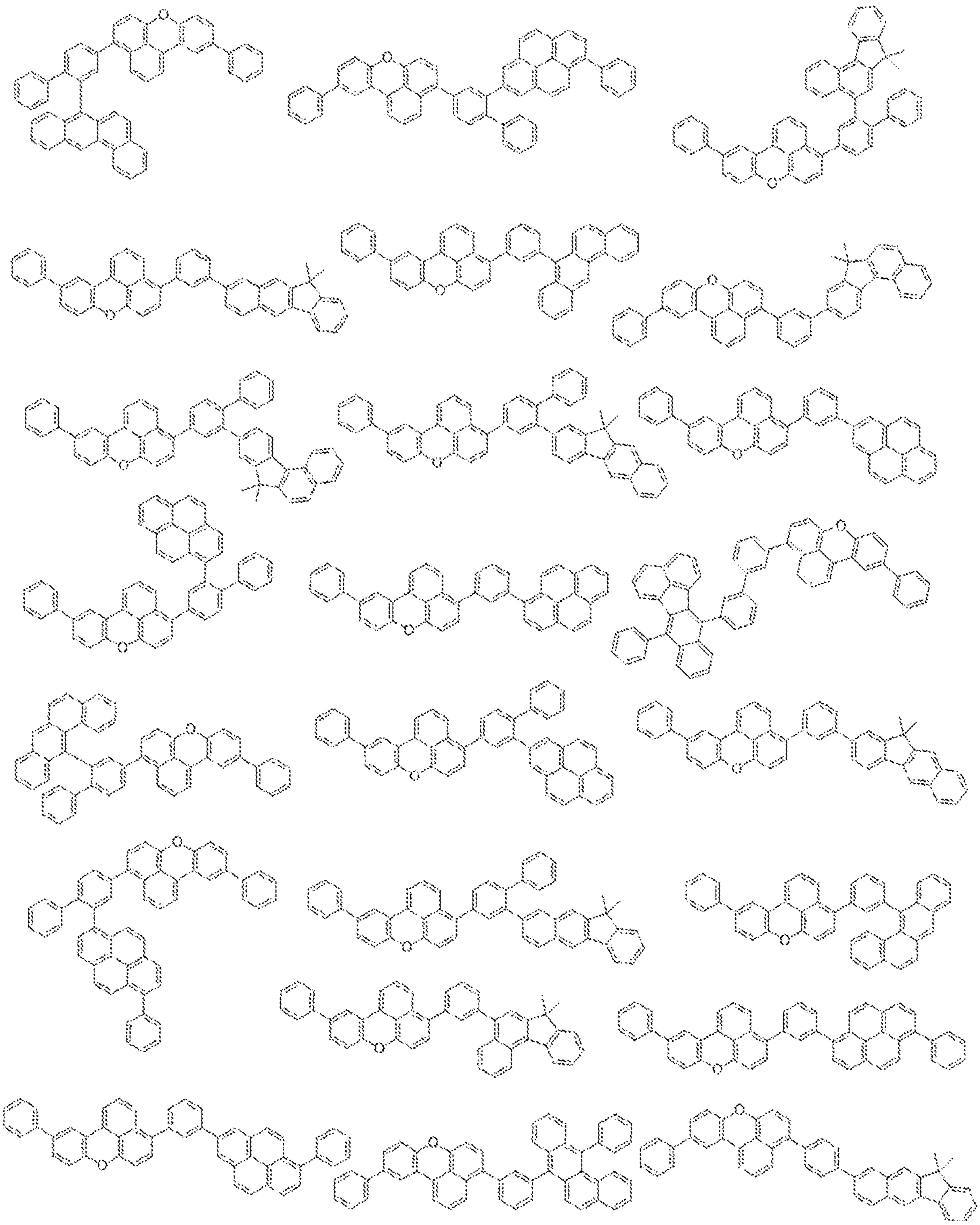

[Formula 248]

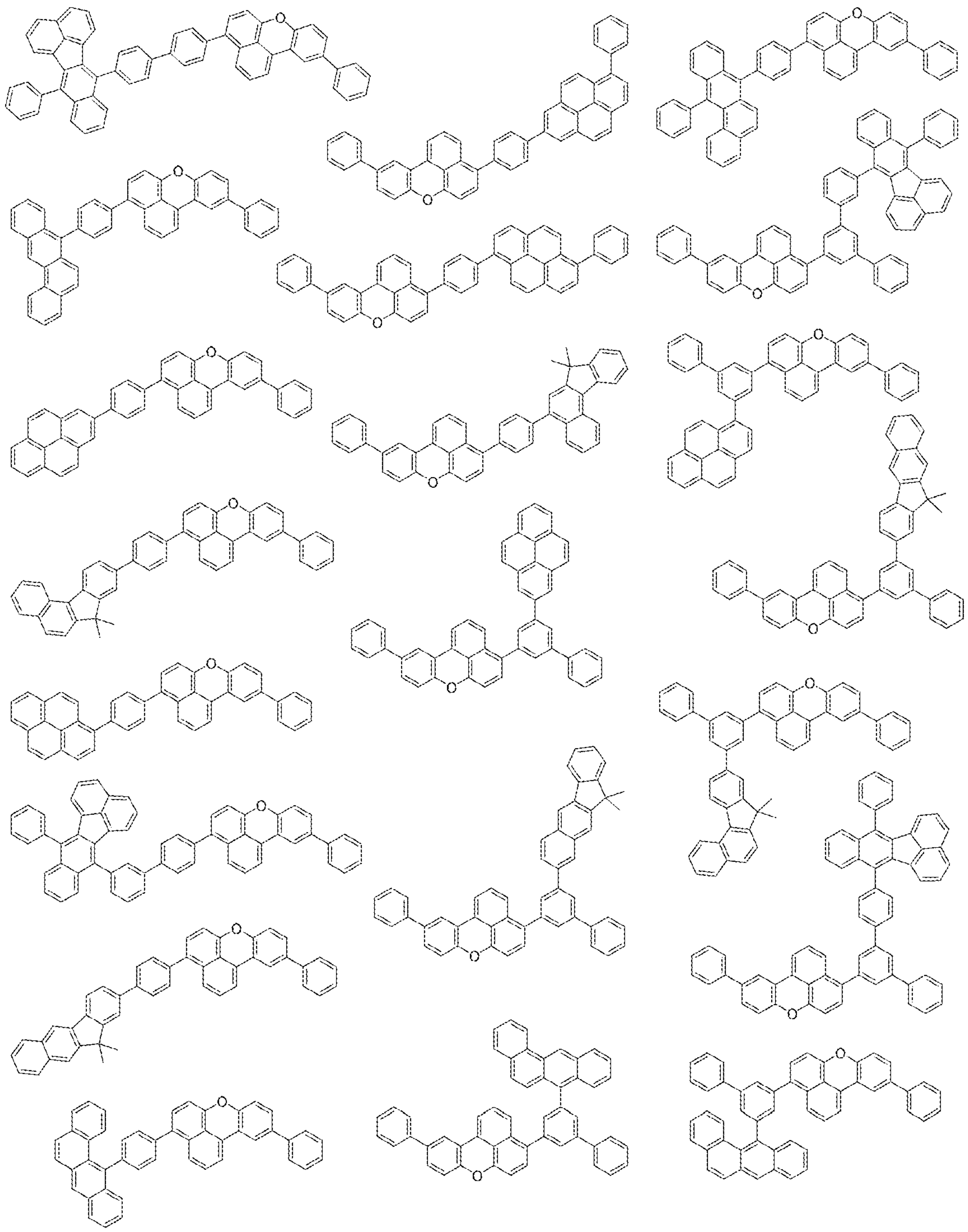

[Formula 249]

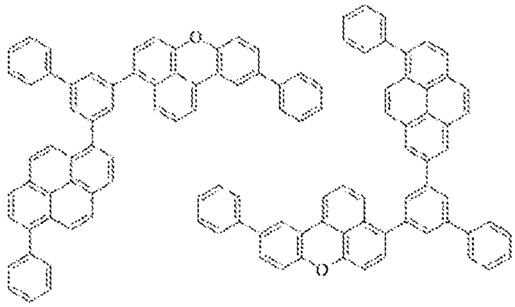
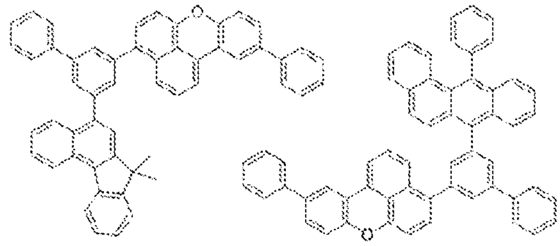
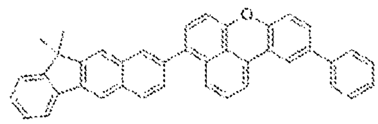
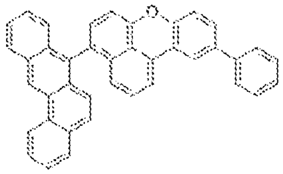
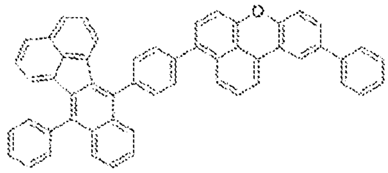
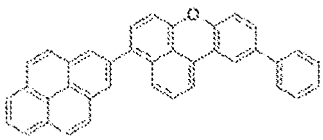
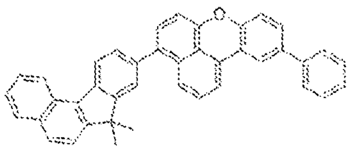
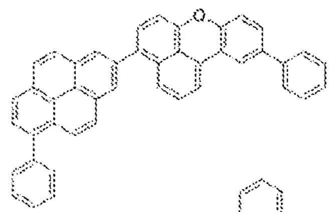
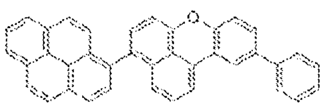
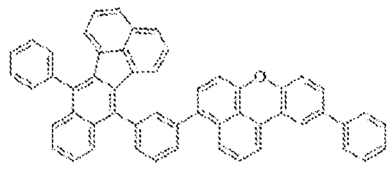
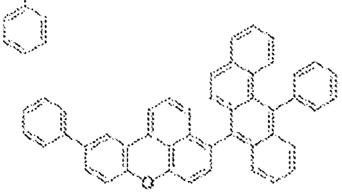
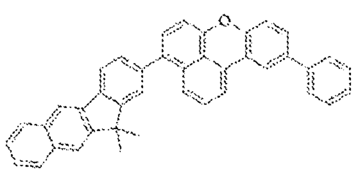
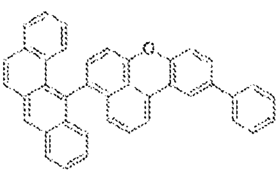
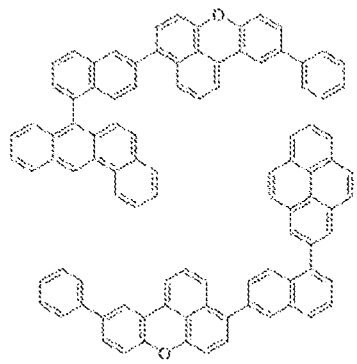
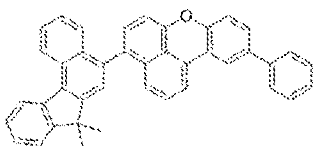
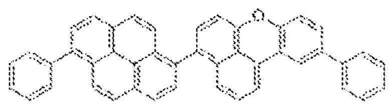
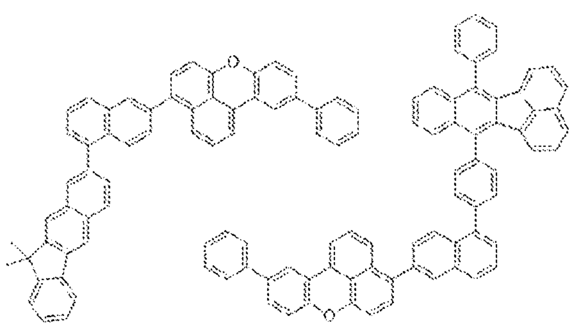
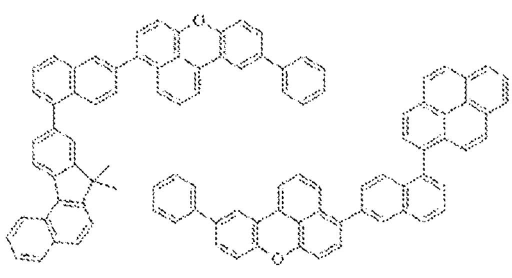

[Formula 250]

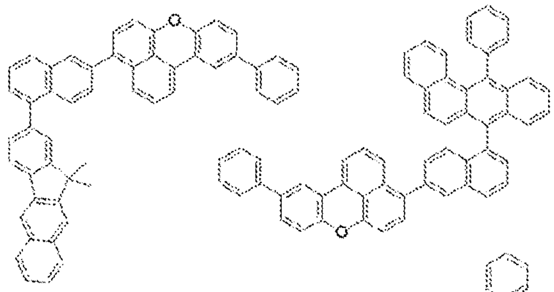

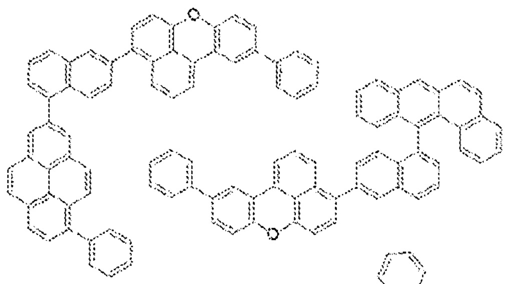

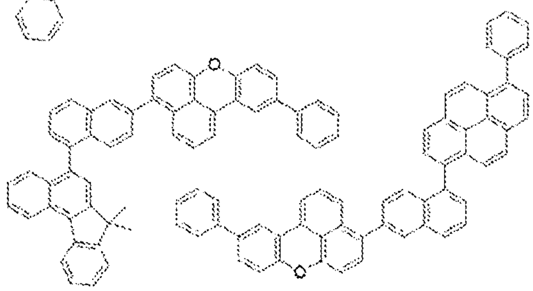

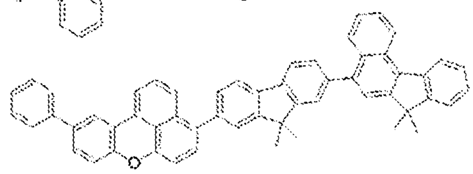

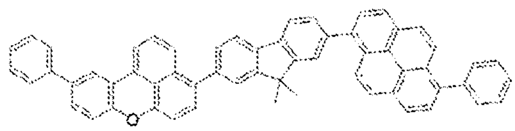

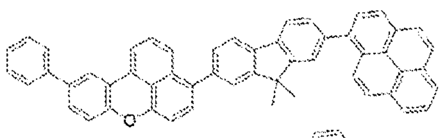

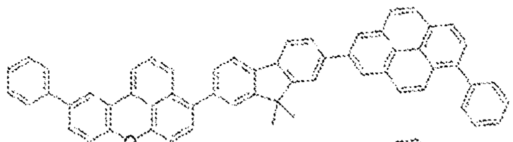

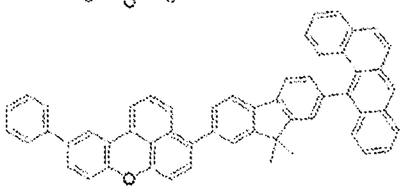

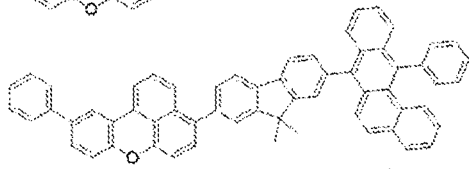

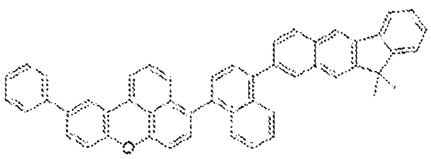

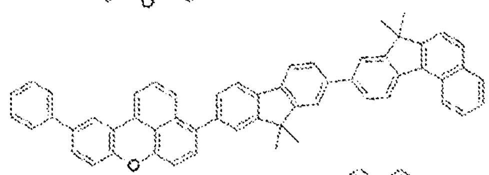

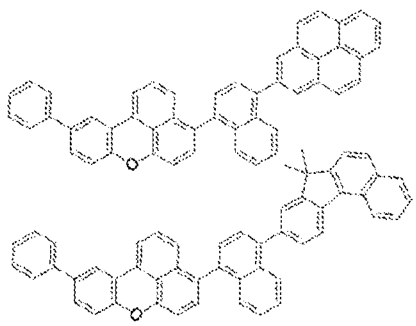

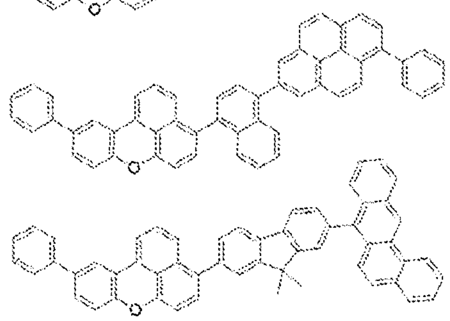

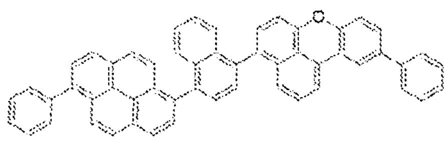

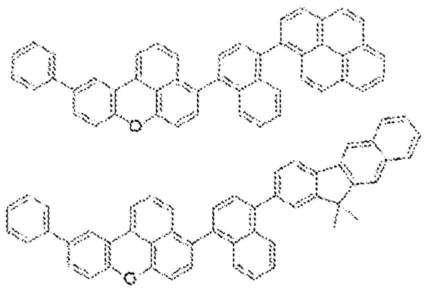

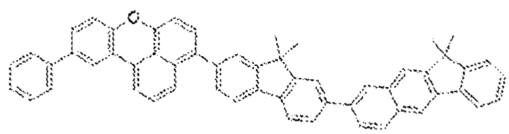

[Formula 251]

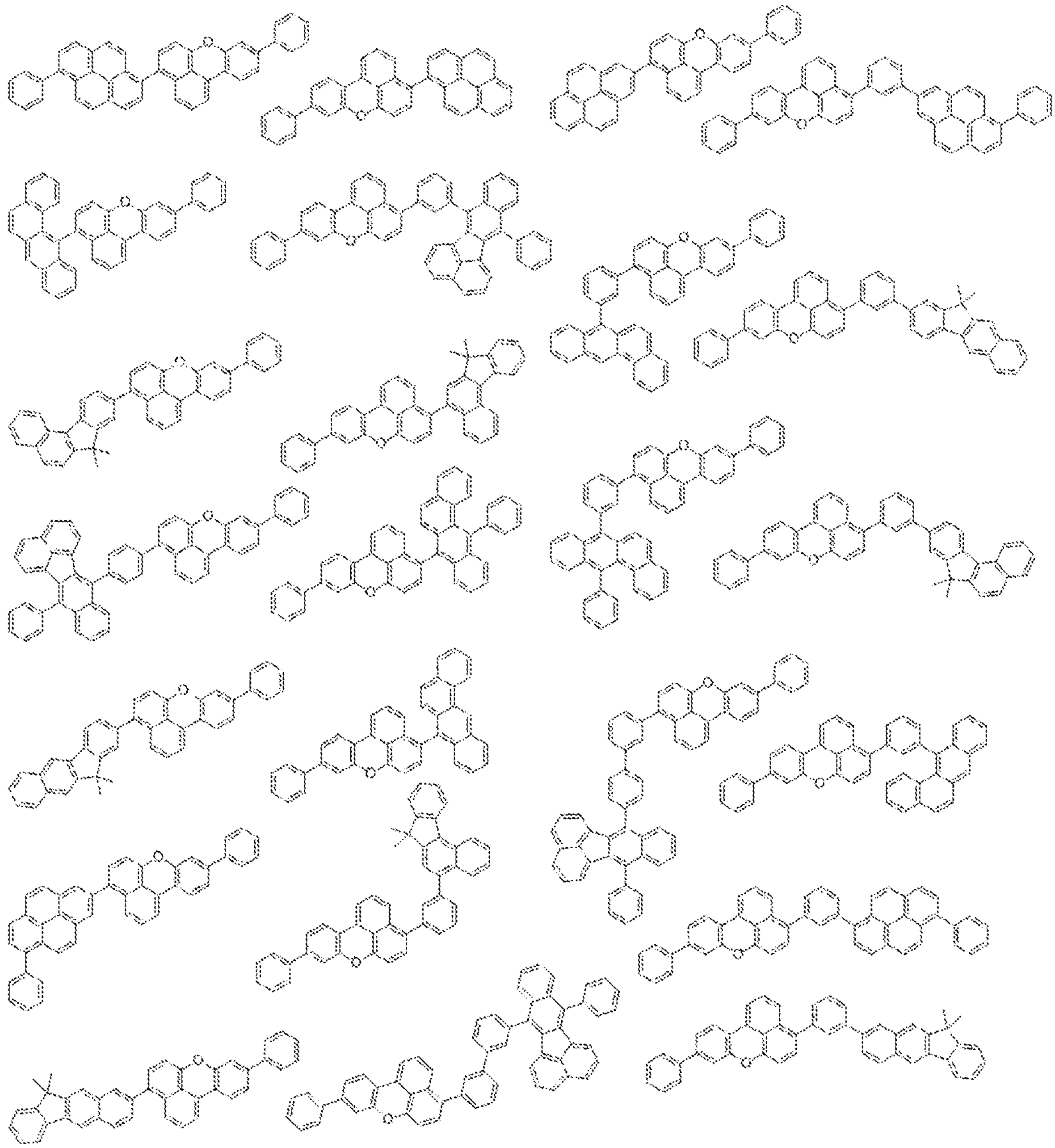

[Formula 252]

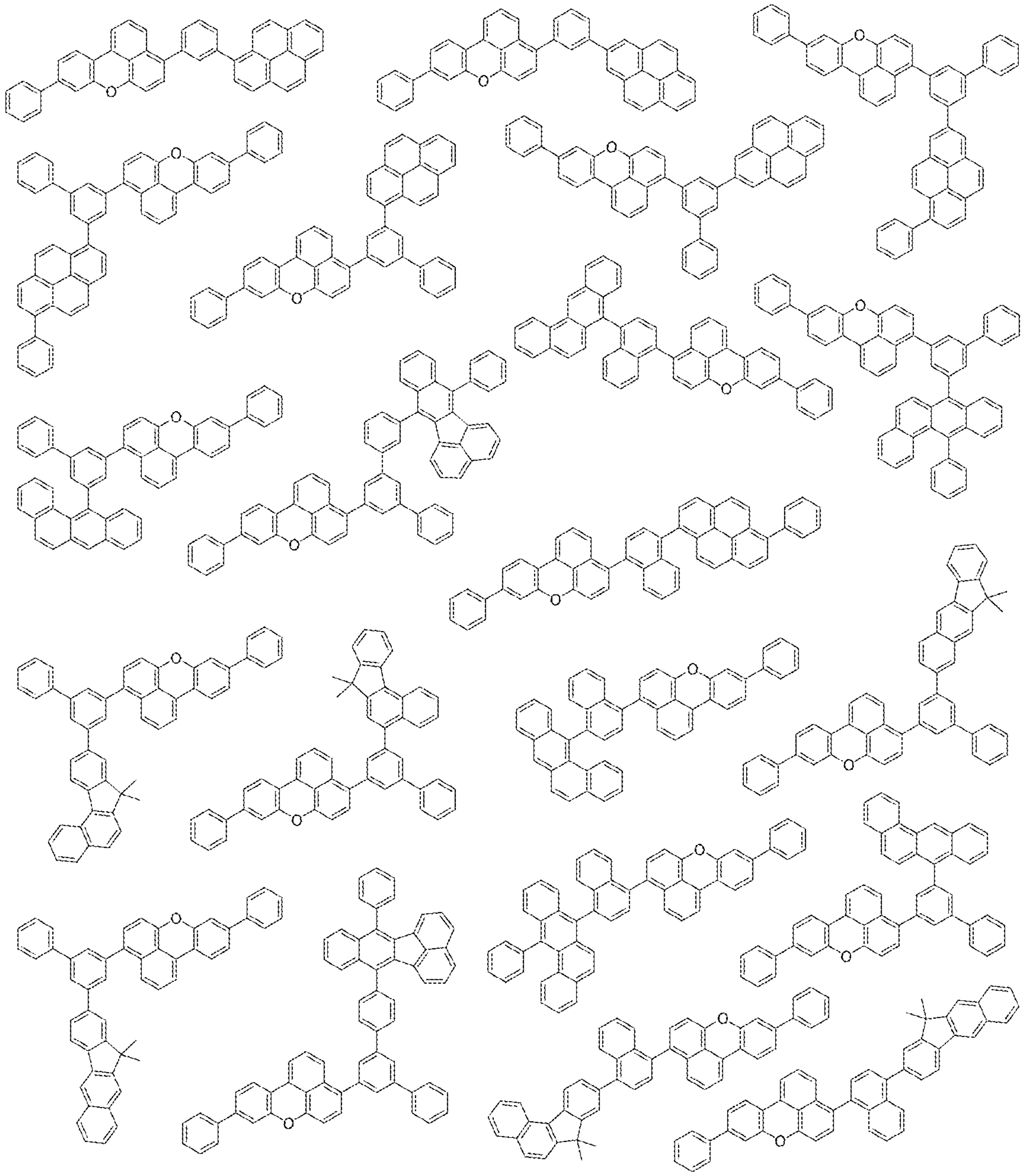

[Formula 253]

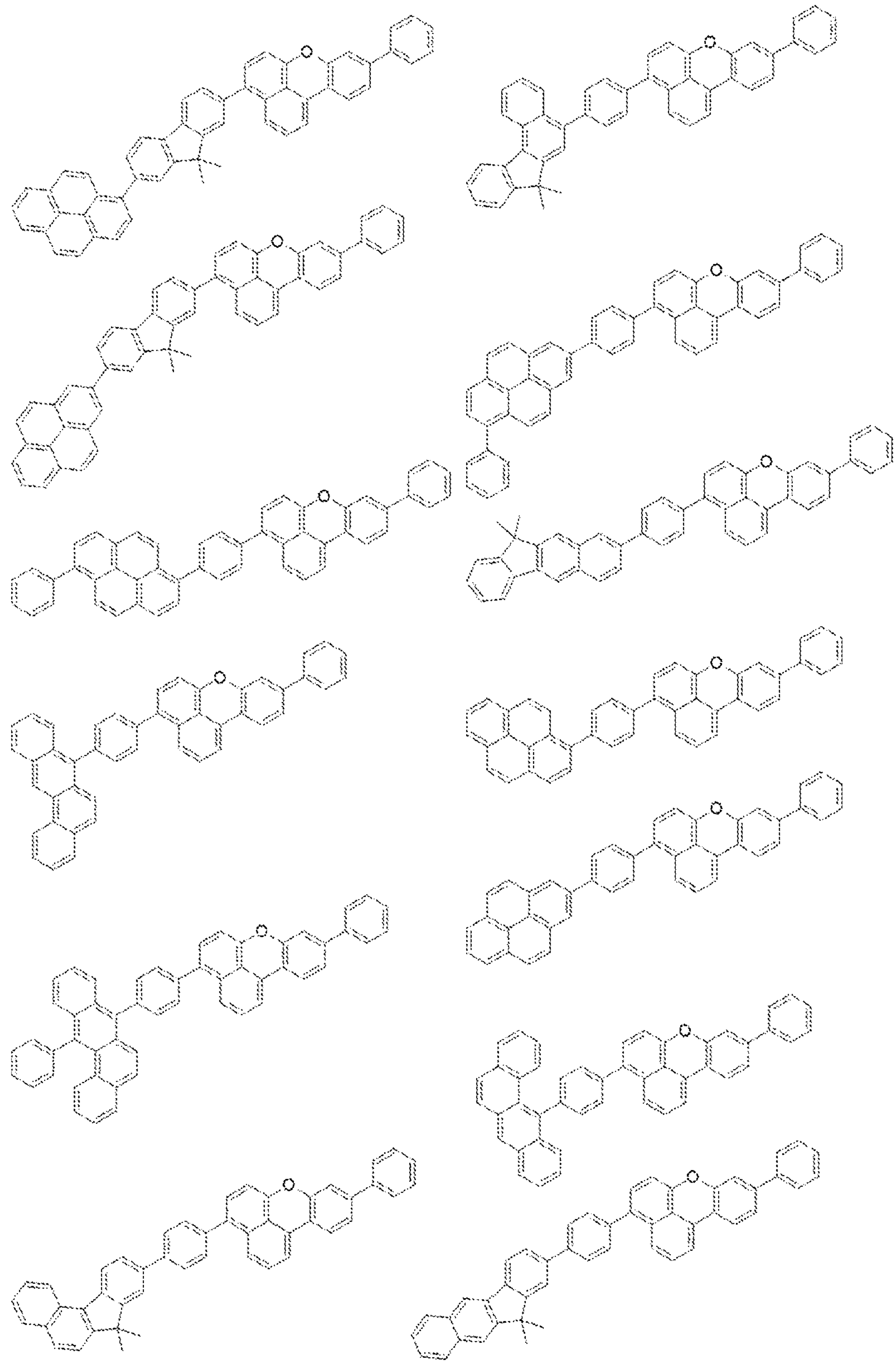

[Formula 254]

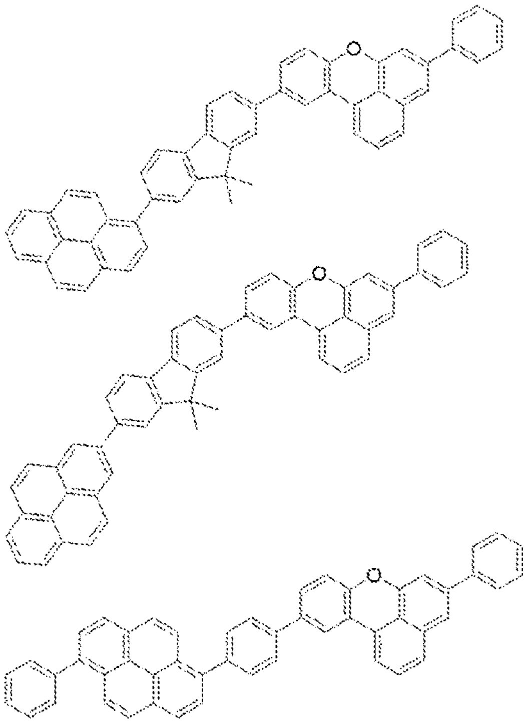

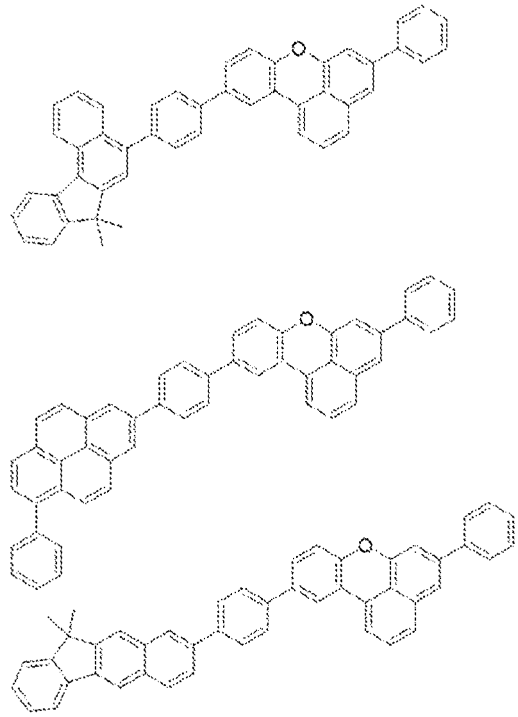

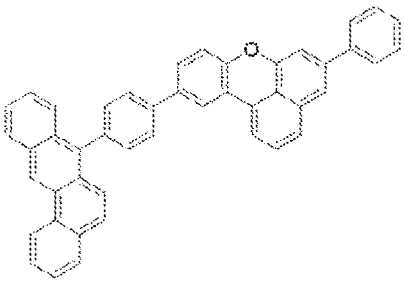

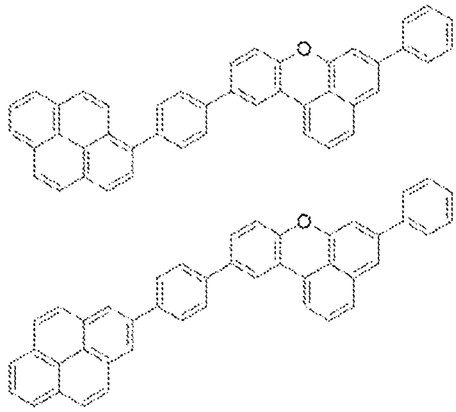

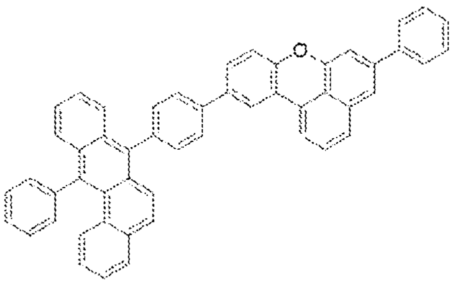

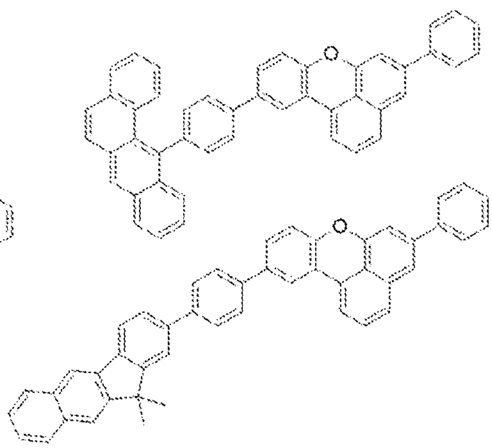

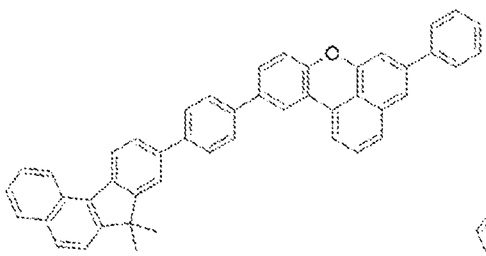

[Formula 255]

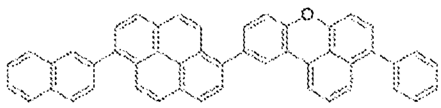
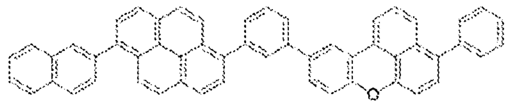
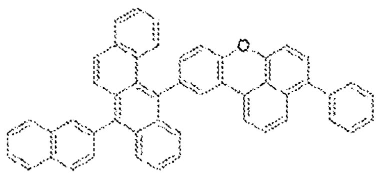
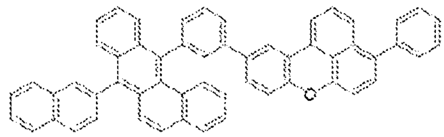
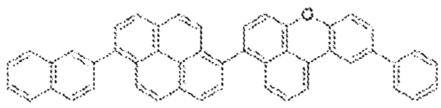
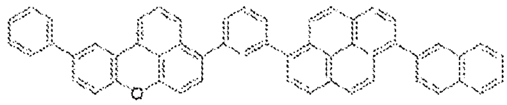
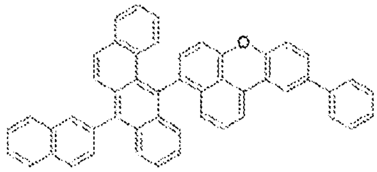
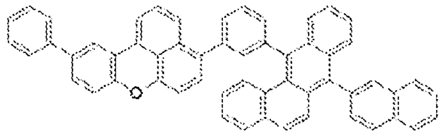
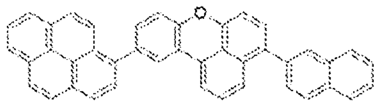
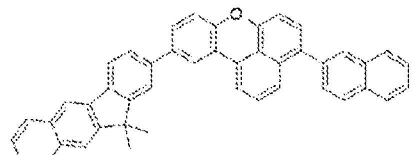
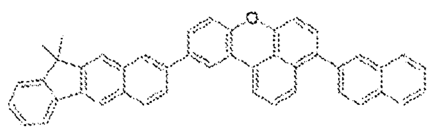
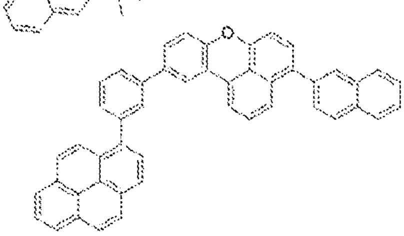
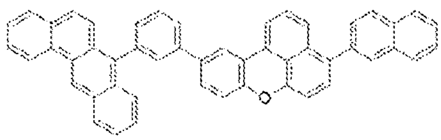
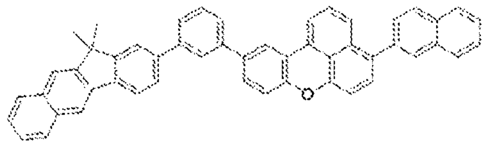
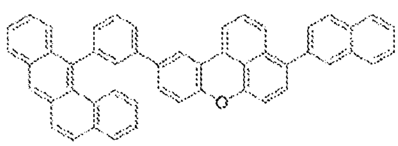
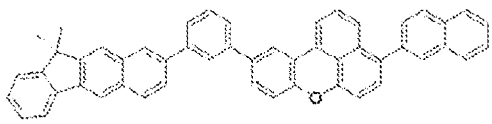
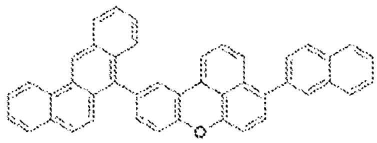
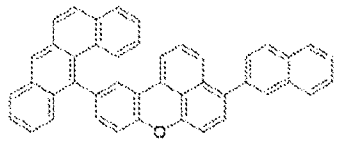

[Formula 256]

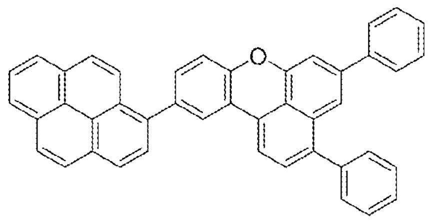

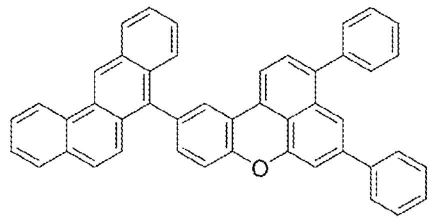

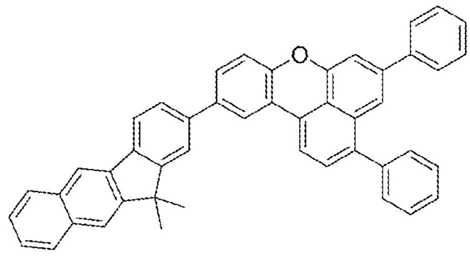

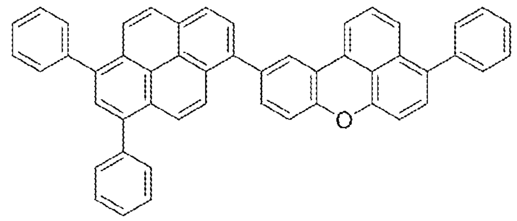

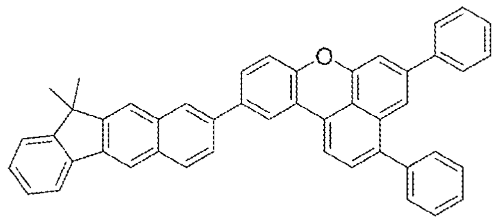

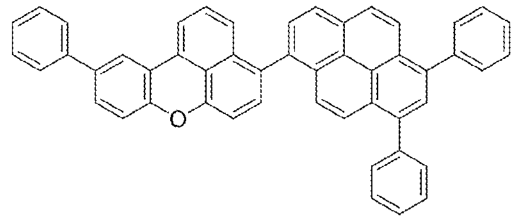

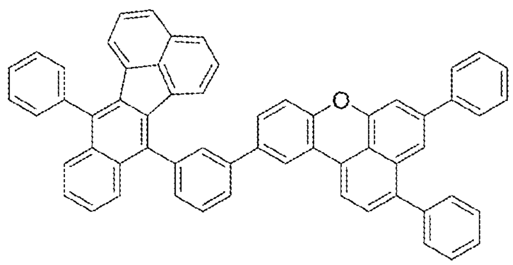

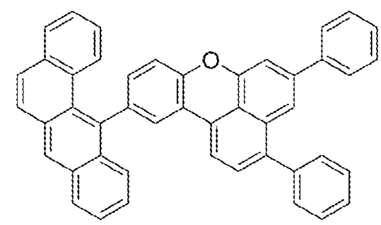

[Formula 257]

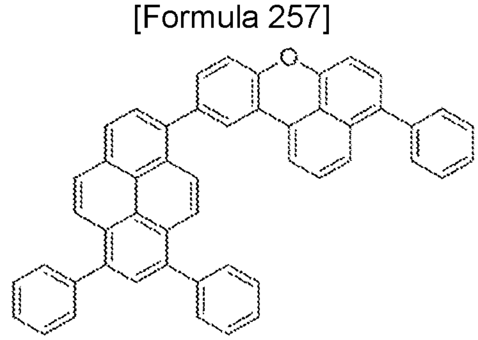

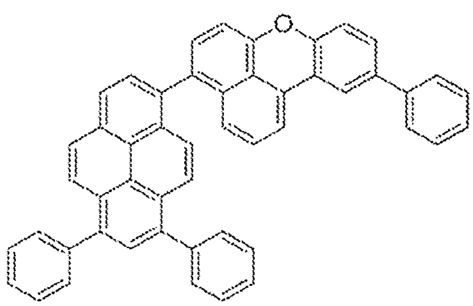

[Formula 258]

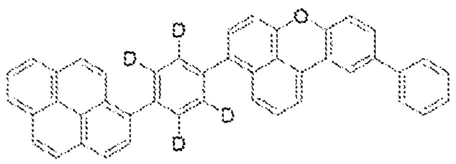
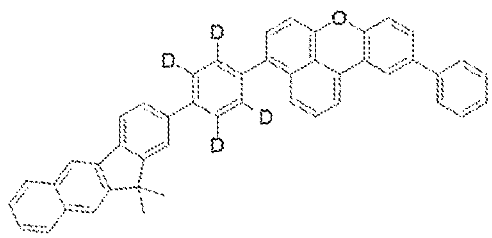
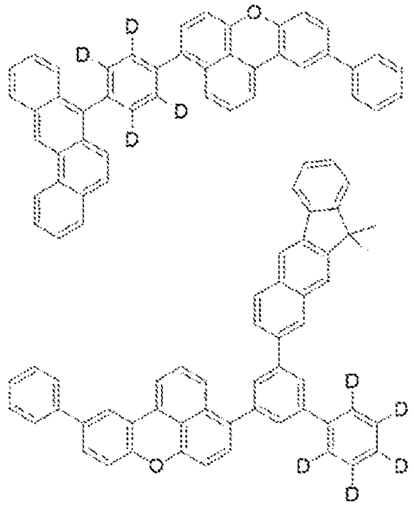
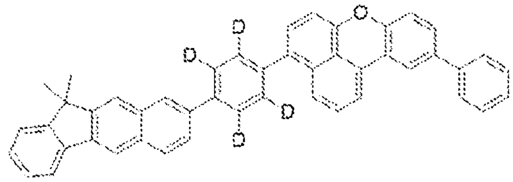
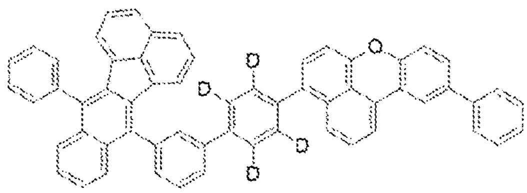
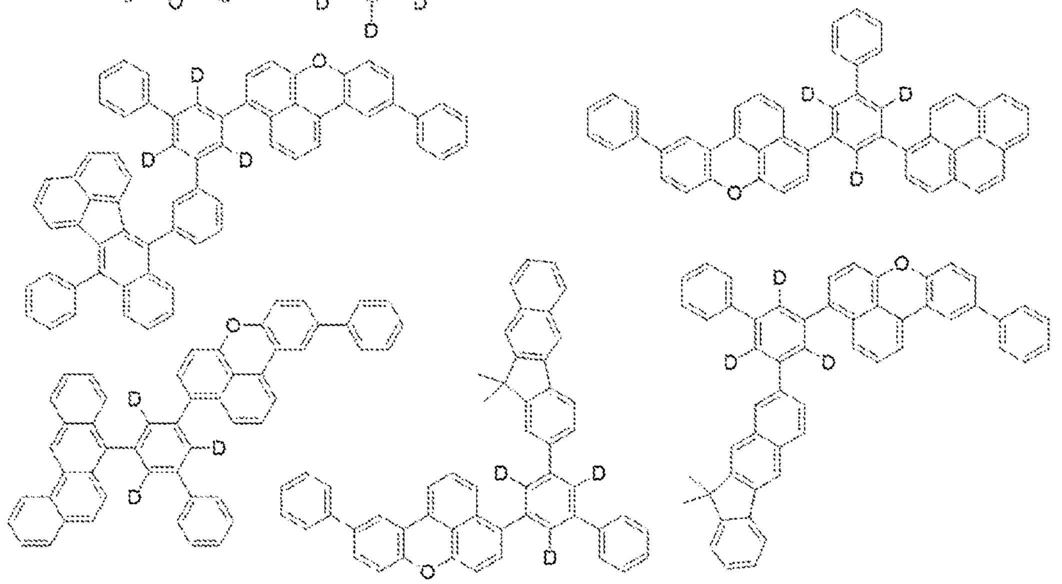
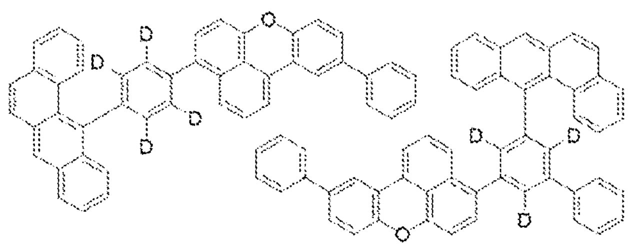
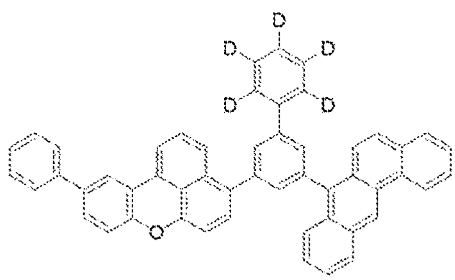

[Formula 259]

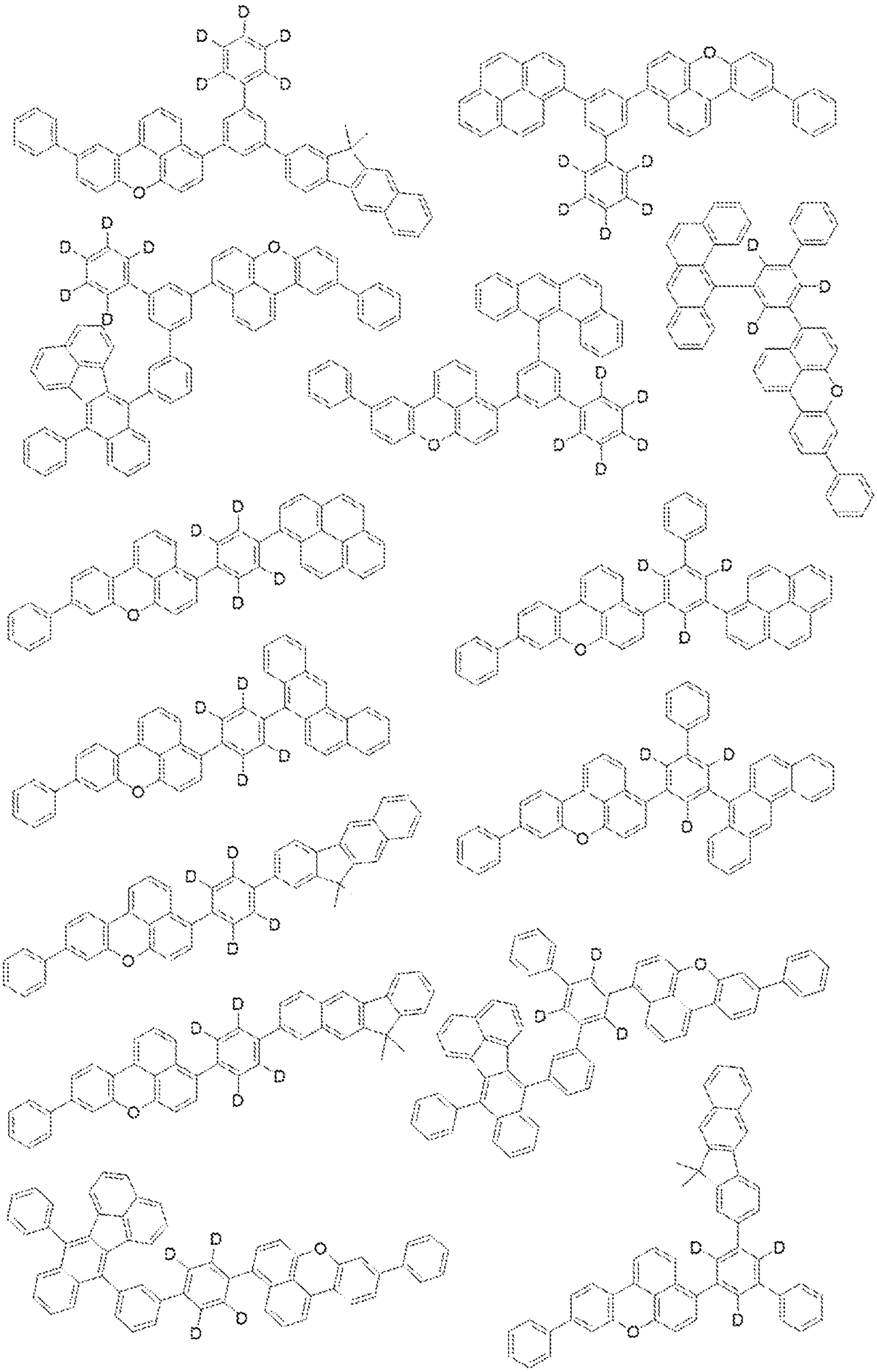

[Formula 260]

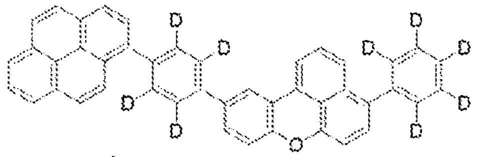
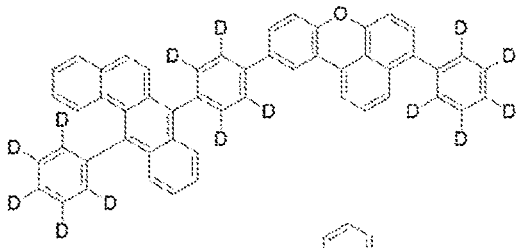
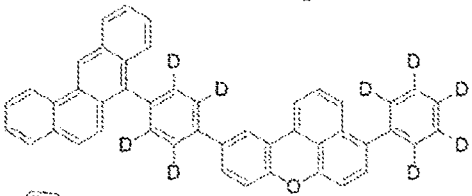
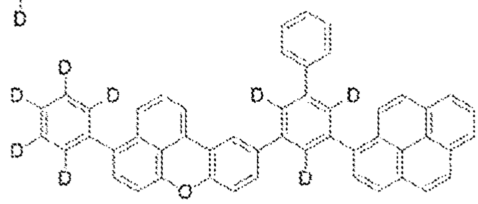
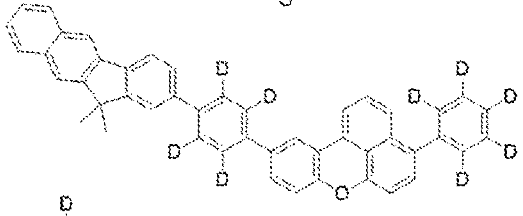
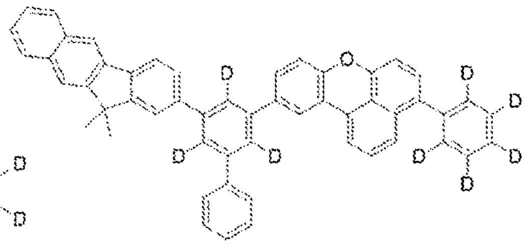
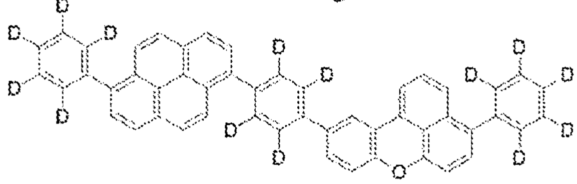
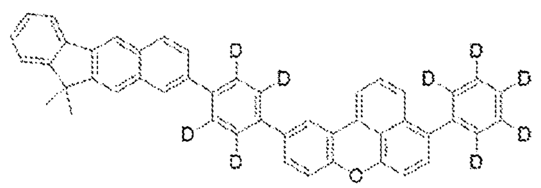
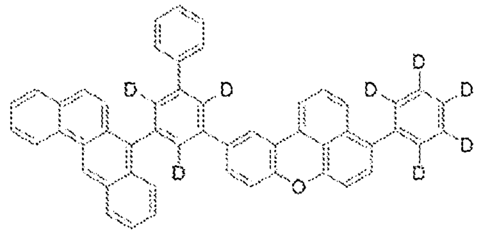
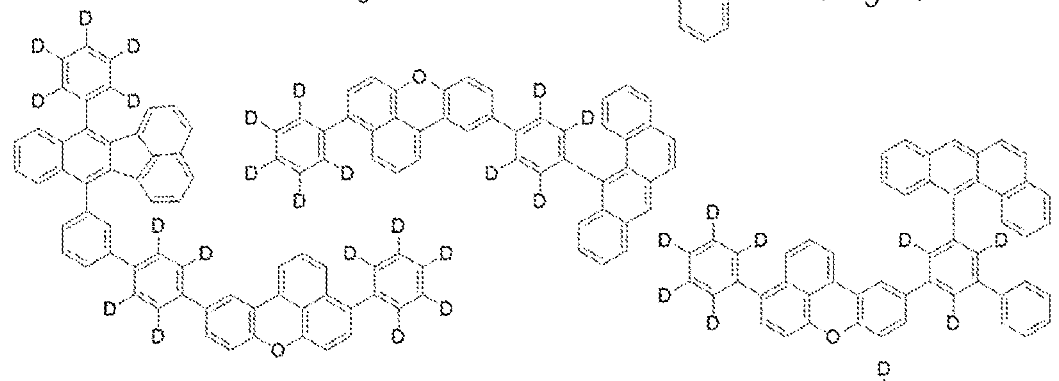
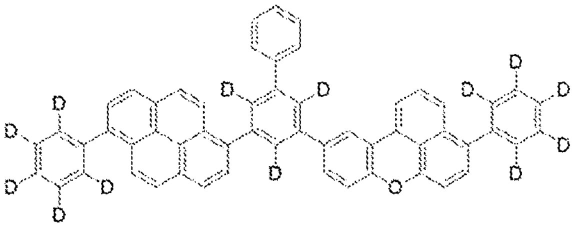
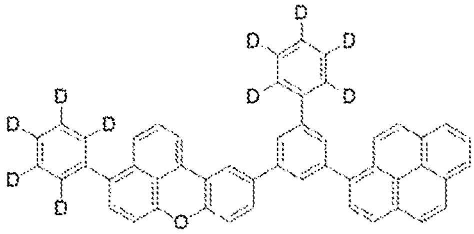

[Formula 261]

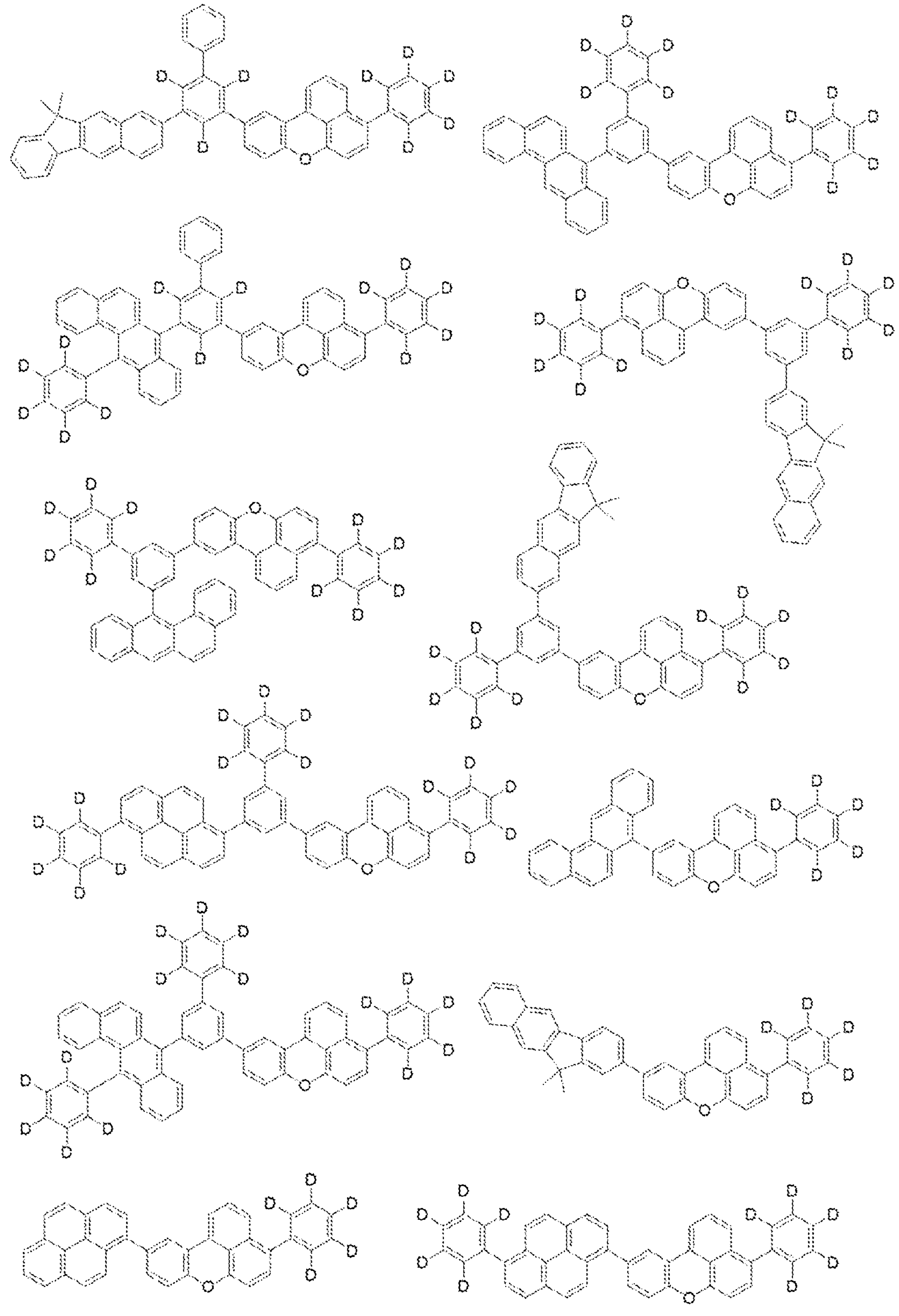

[Formula 262]

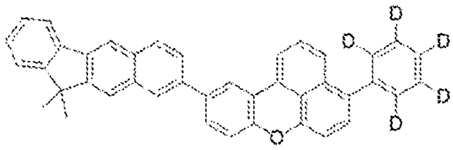
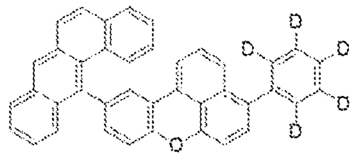
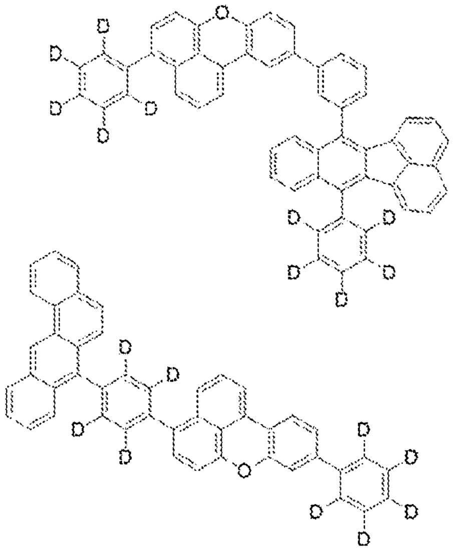
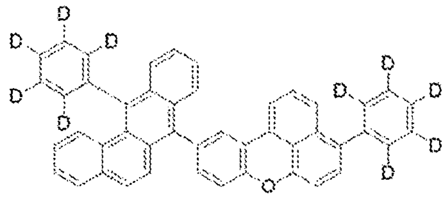
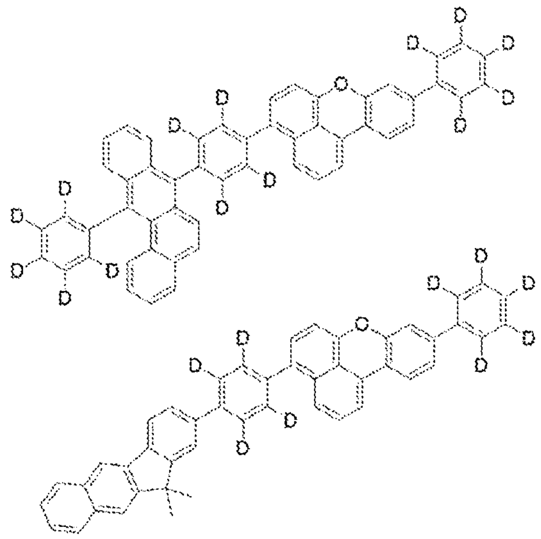
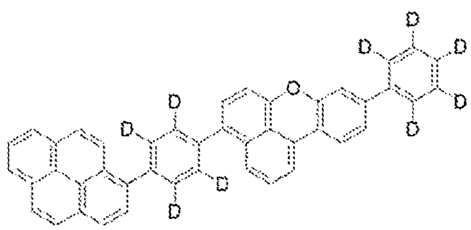
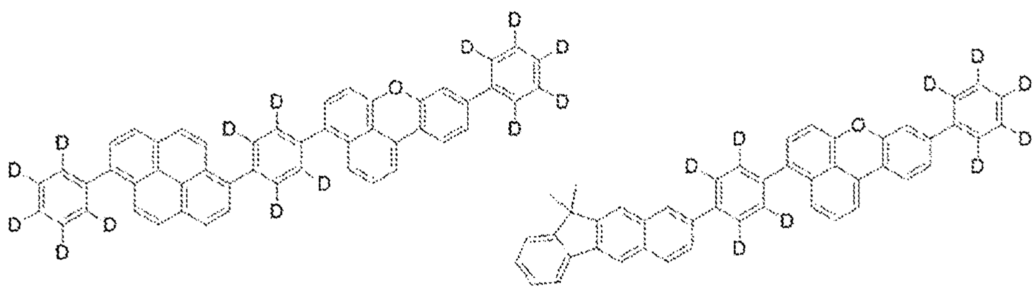
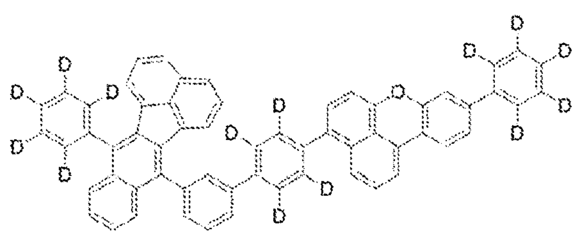
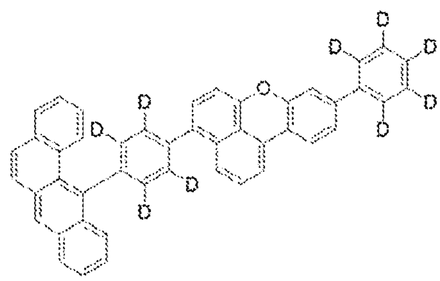

[Formula 263]

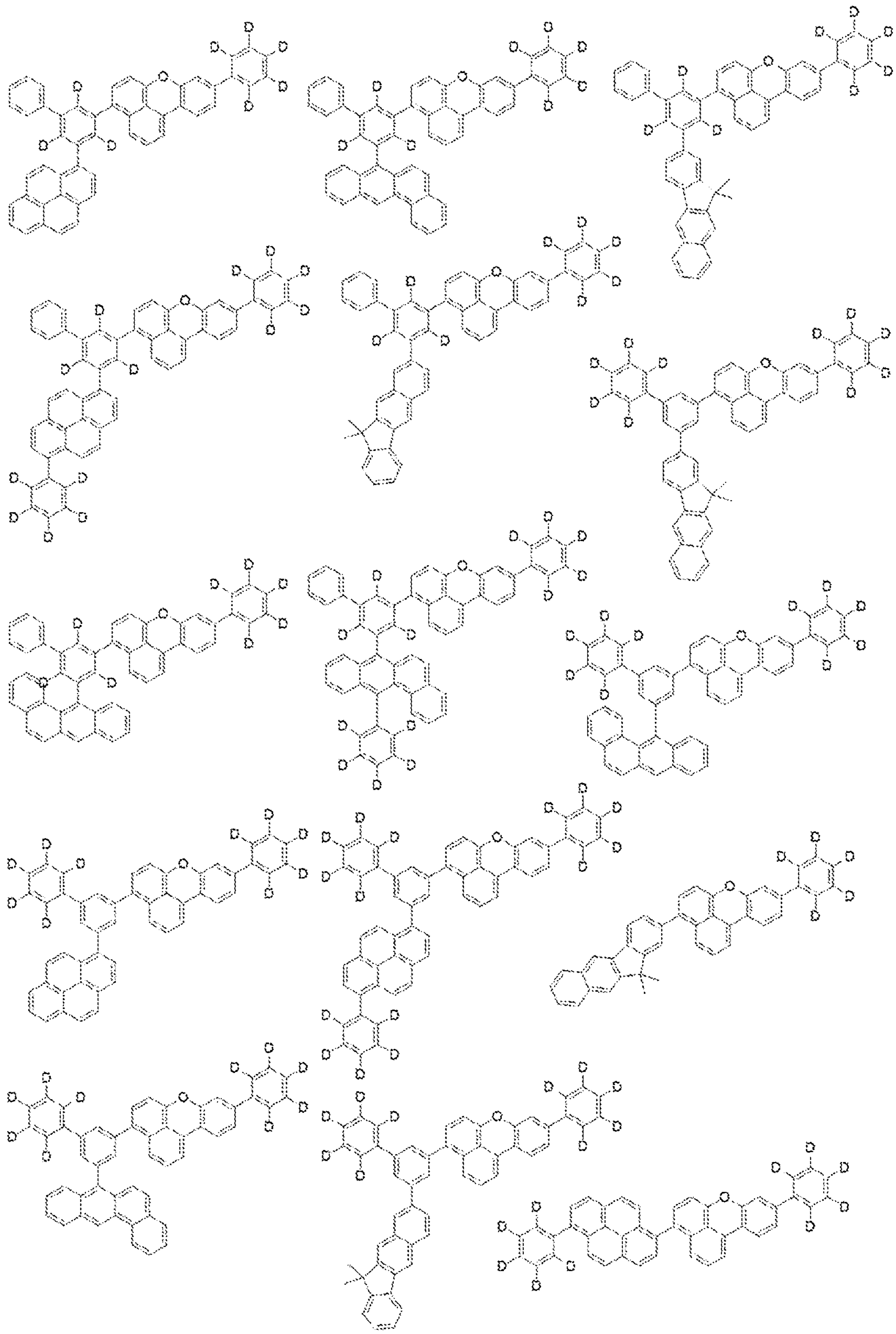

[Formula 264]

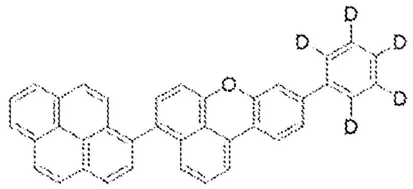

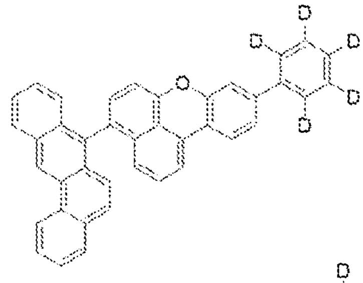

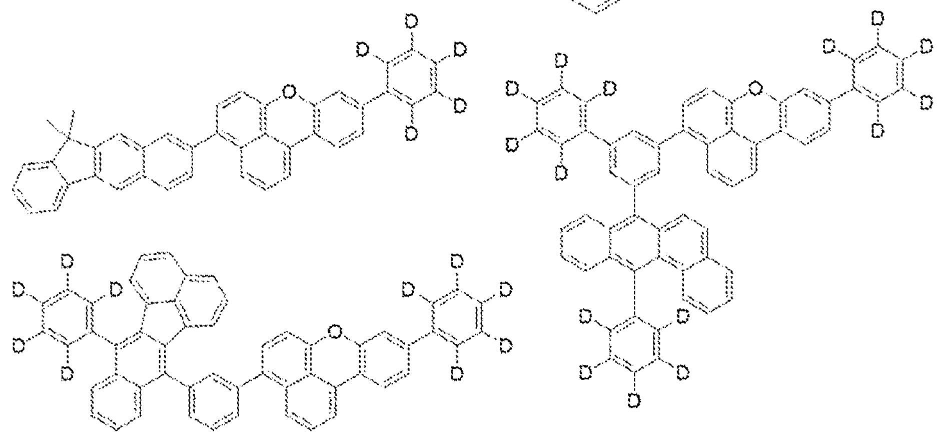

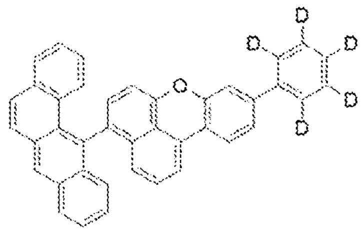

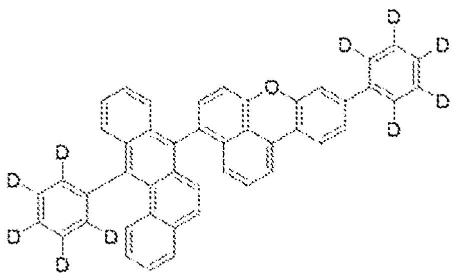

[Formula 265]

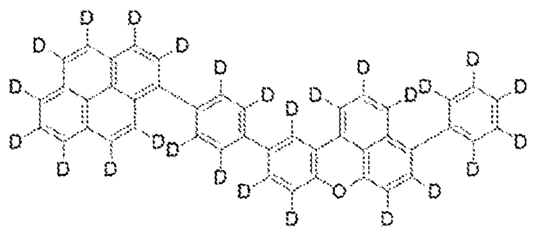

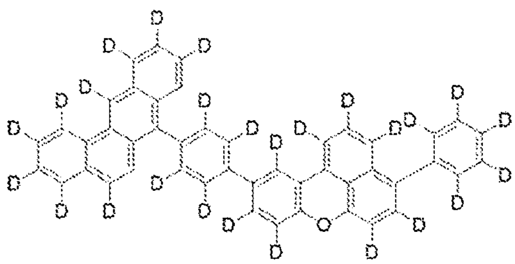

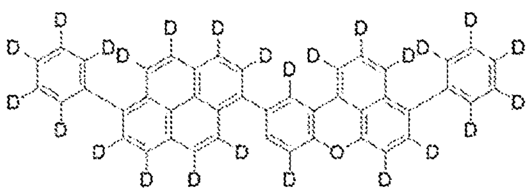

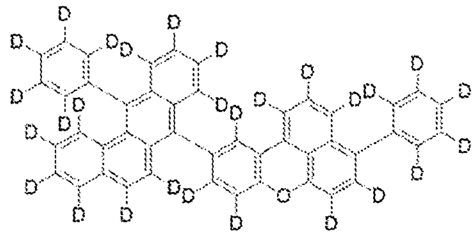

[Formula 266]

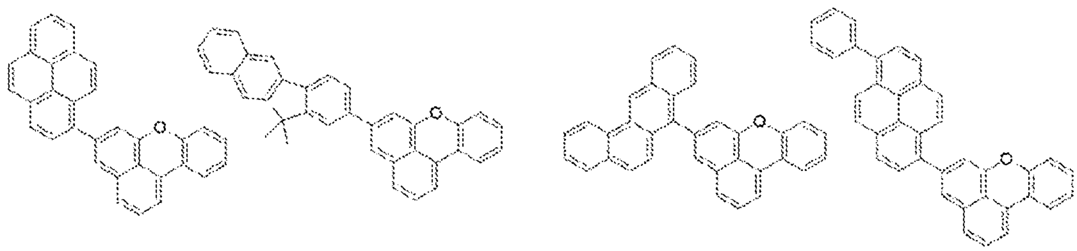

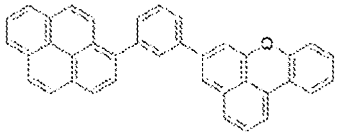

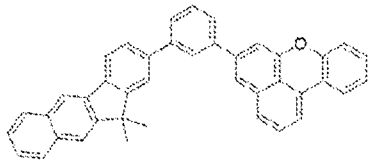

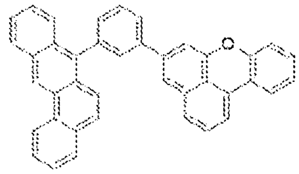

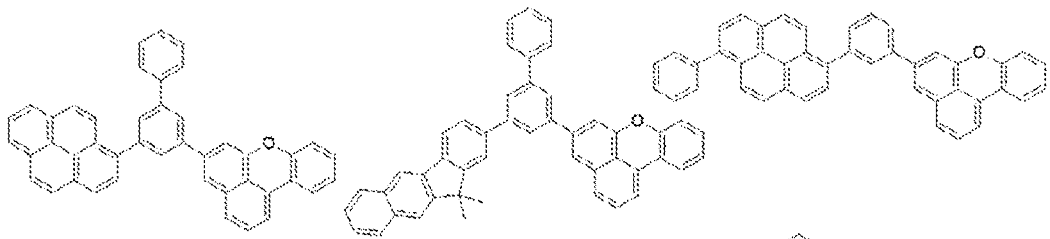

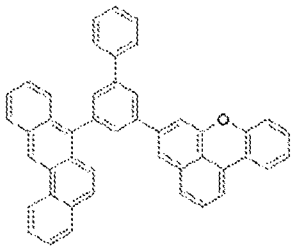

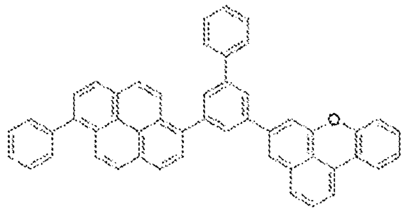

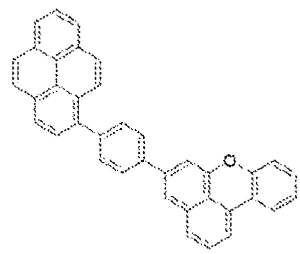

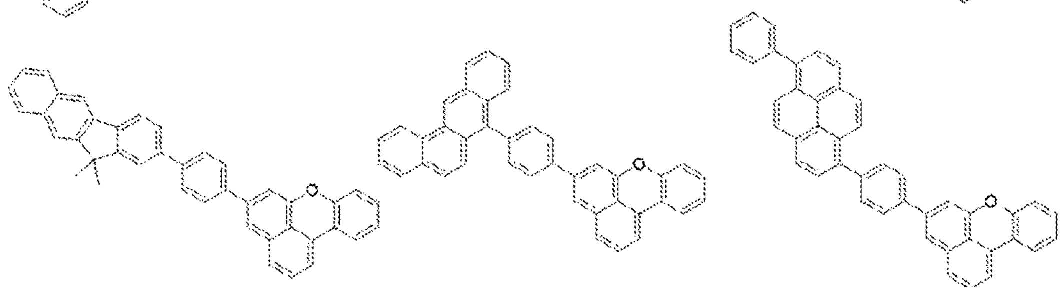

[Formula 267]

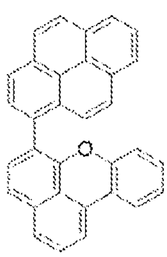

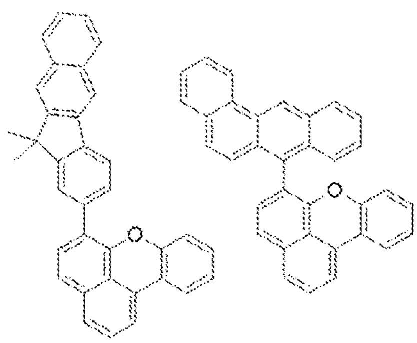

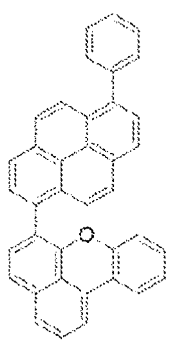

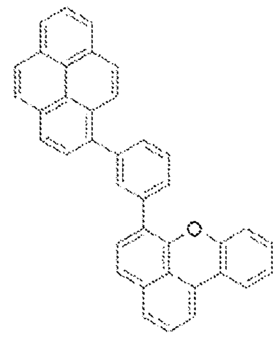

[Formula 268]

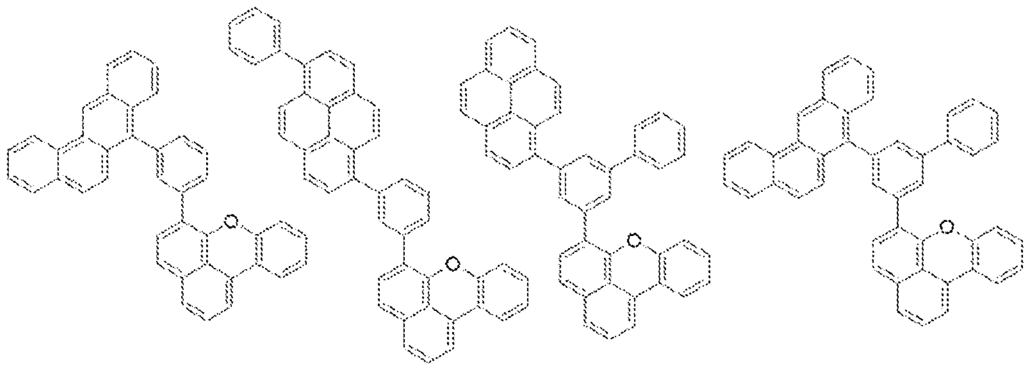

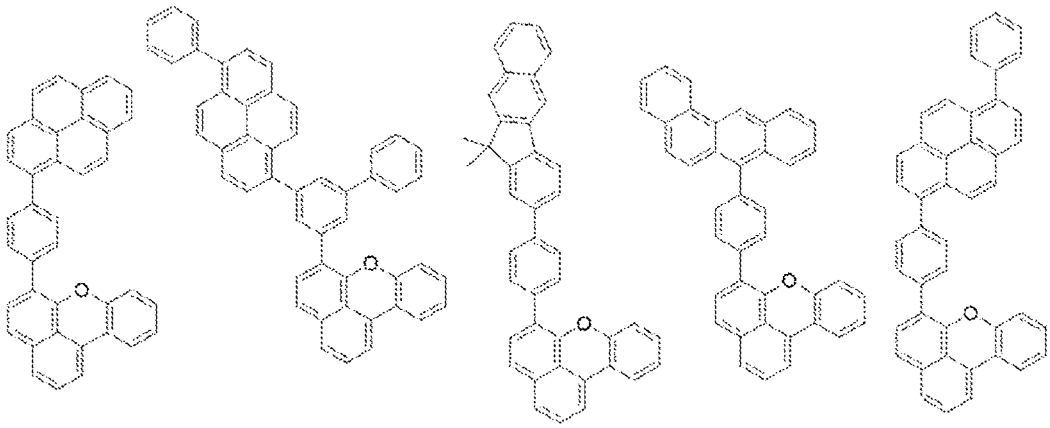

[Formula 269]

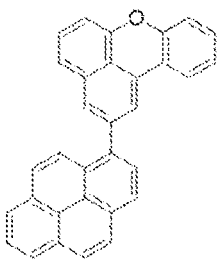

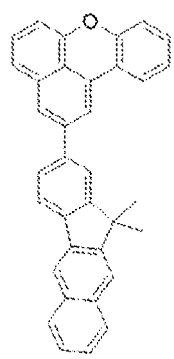

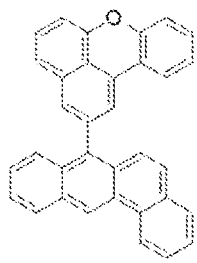

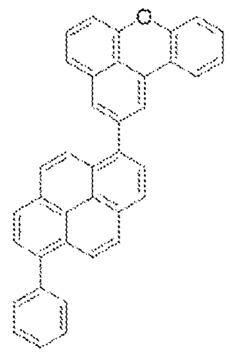

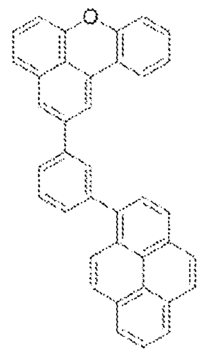

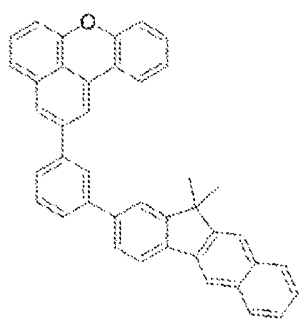

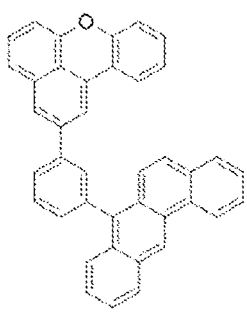

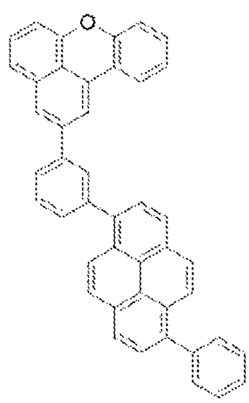

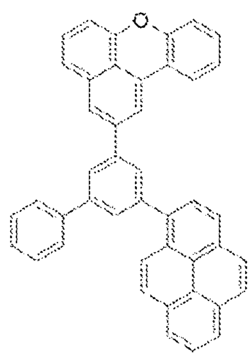

[Formula 270]

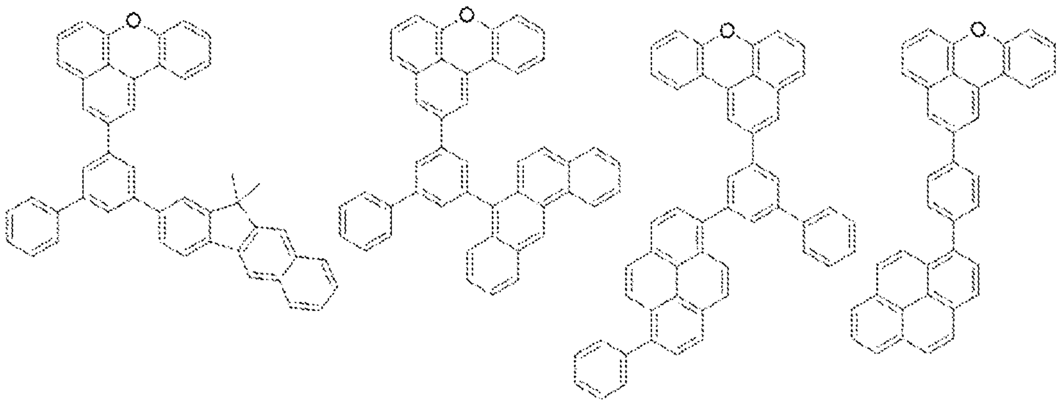

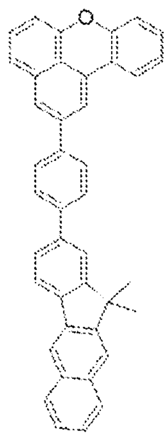

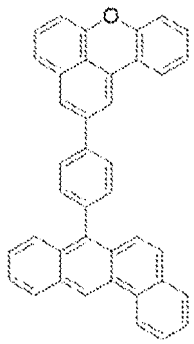

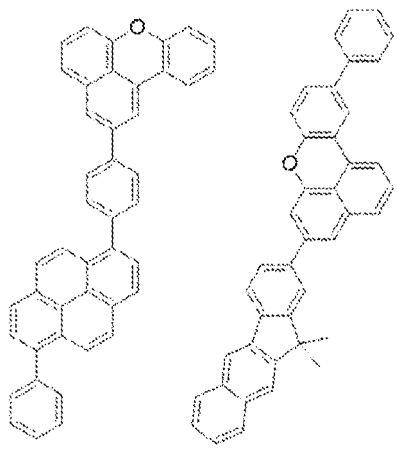

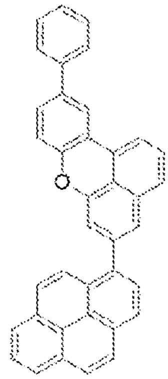

[Formula 271]

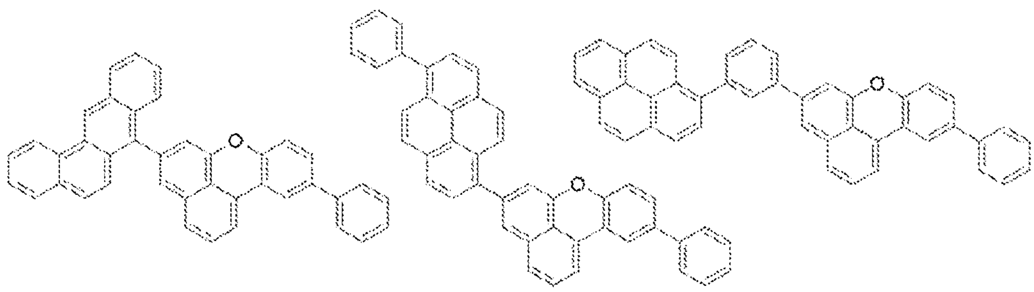

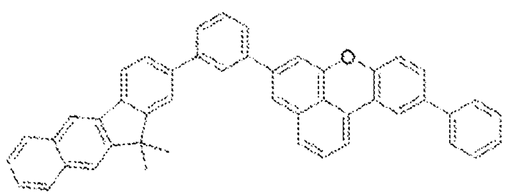

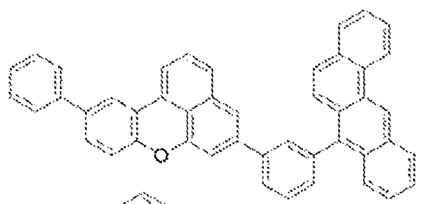

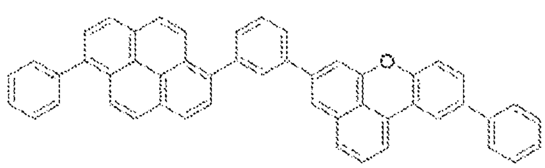

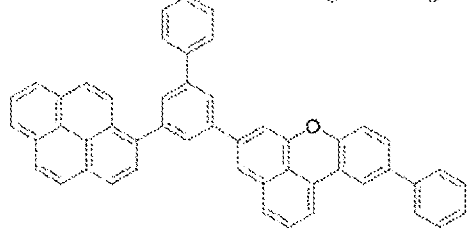

[Formula 272]

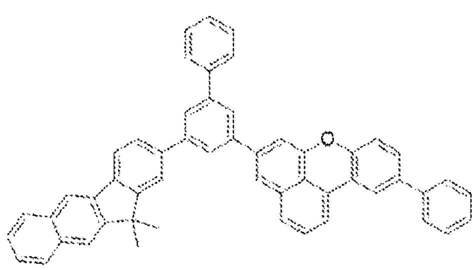

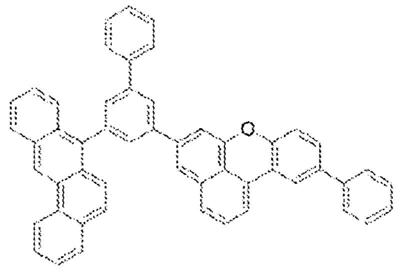

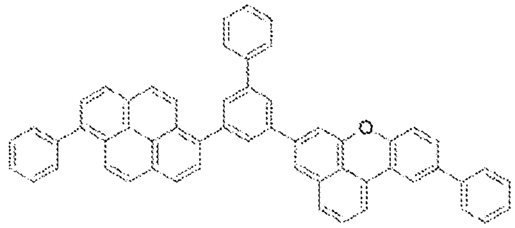

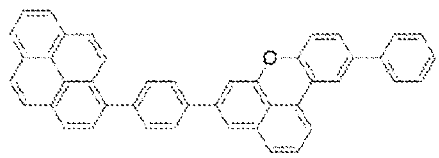

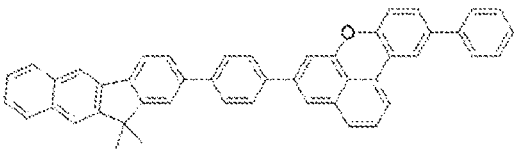

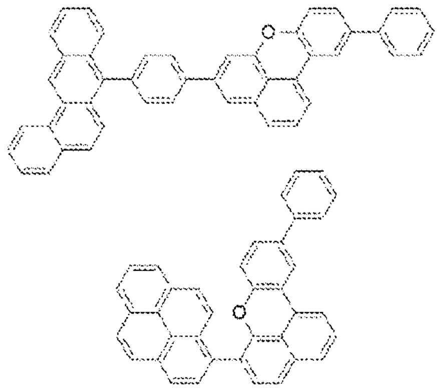

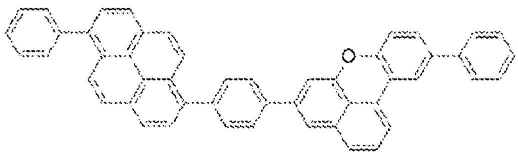

[Formula 273]

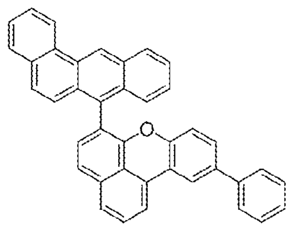

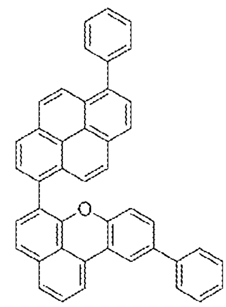

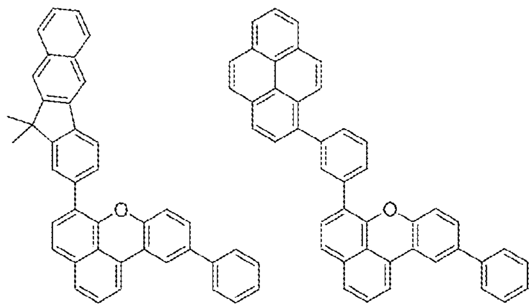

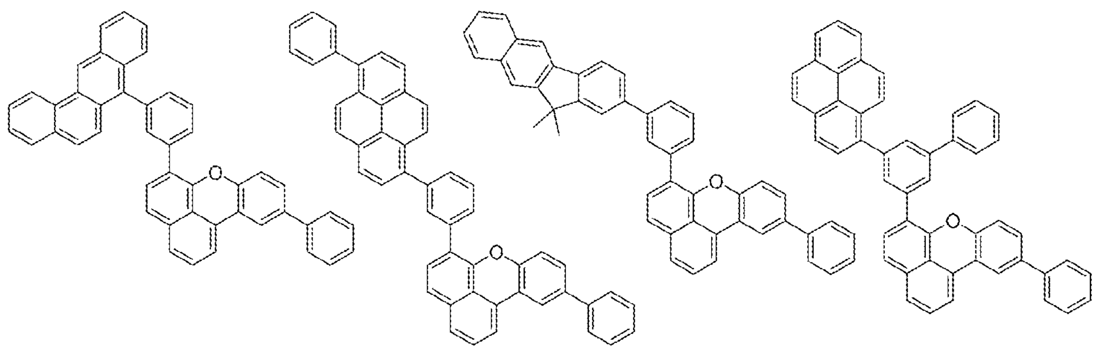

[Formula 274]

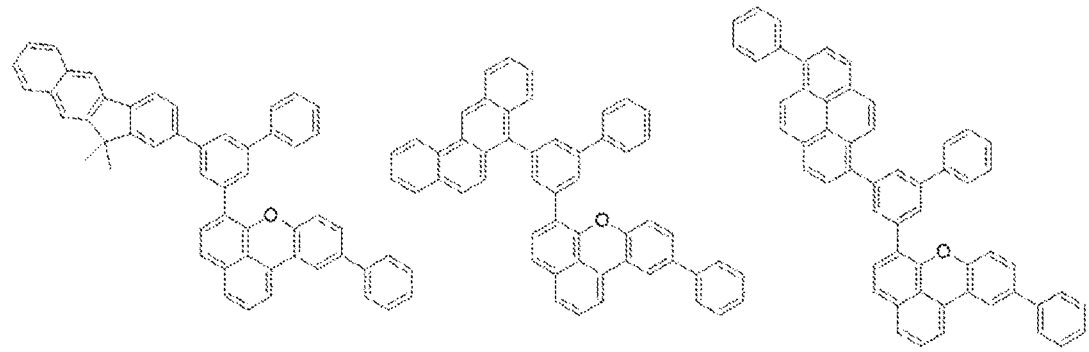

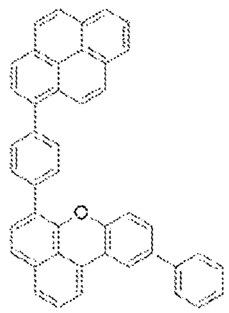

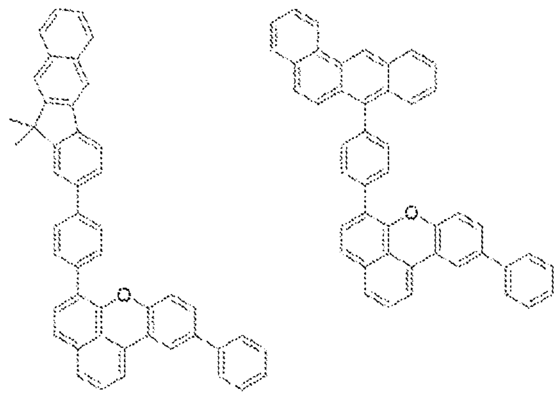

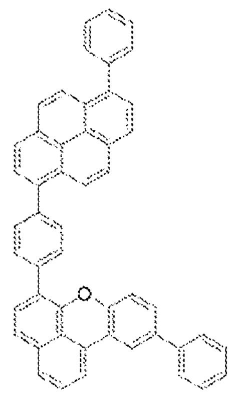

[Formula 275]

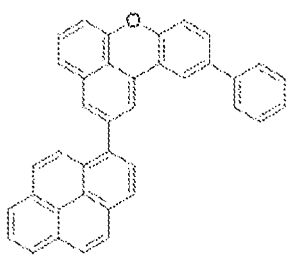

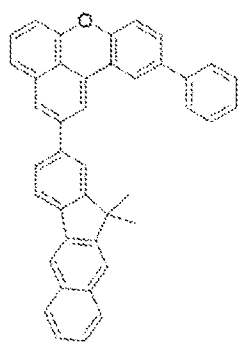

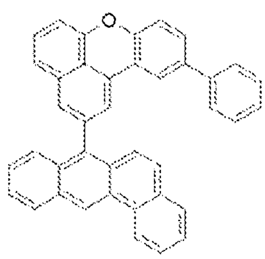

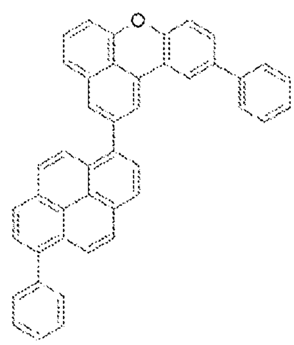

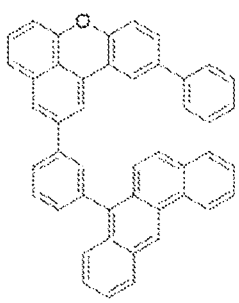

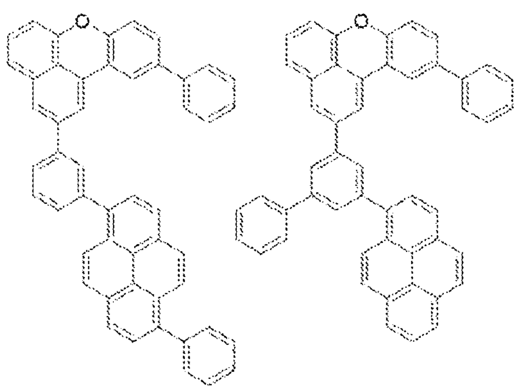

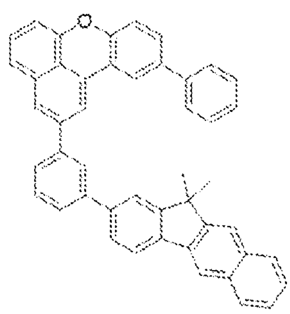

[Formula 276]

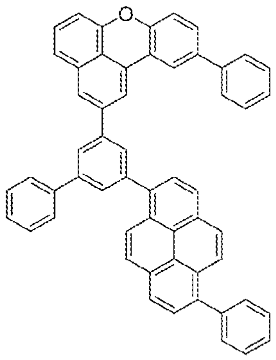
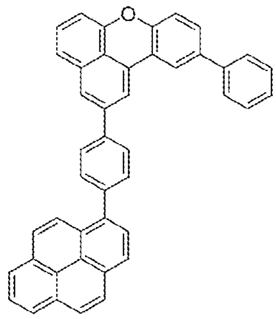
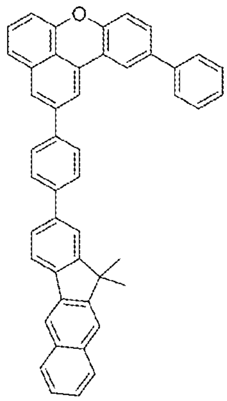
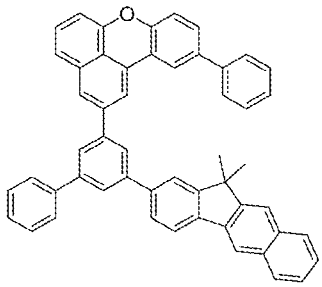
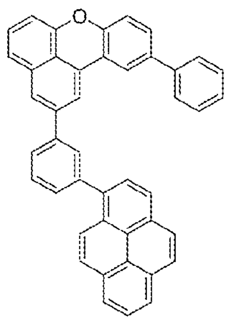
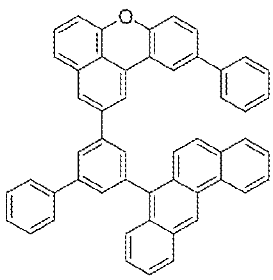
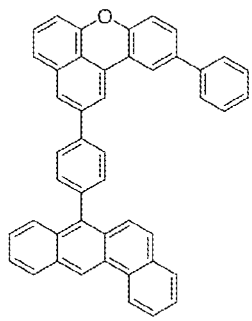
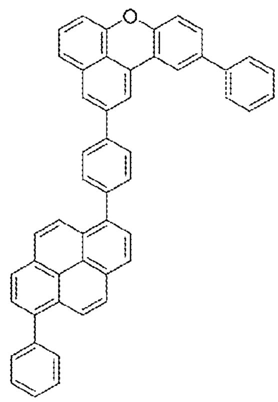

[Formula 277]

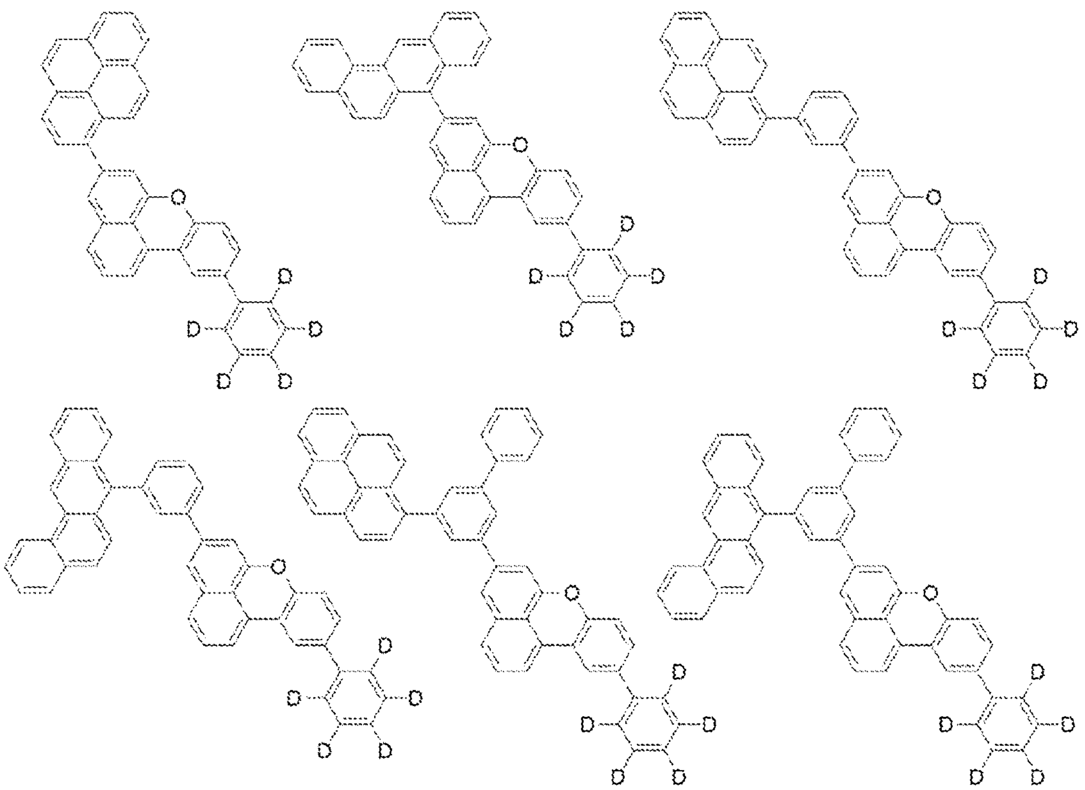

[Formula 278]

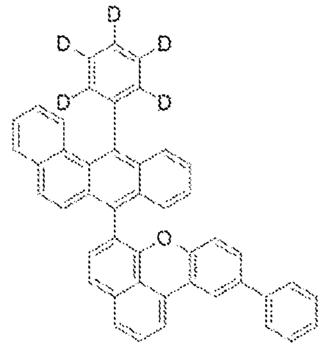
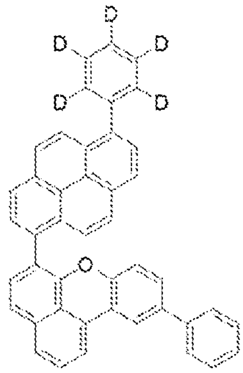
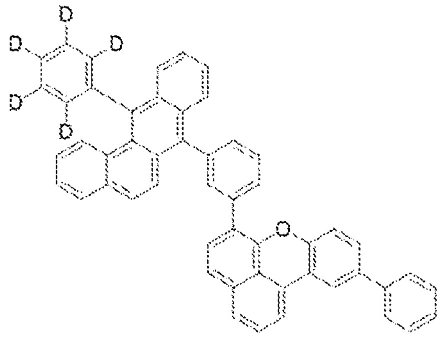
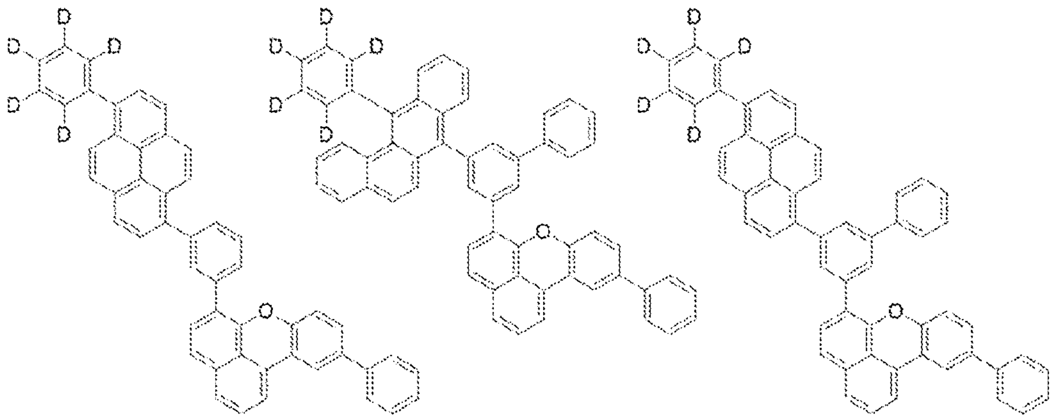

[Formula 279]

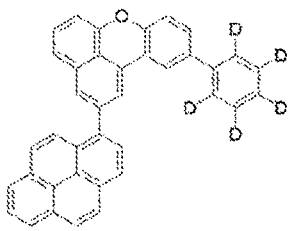
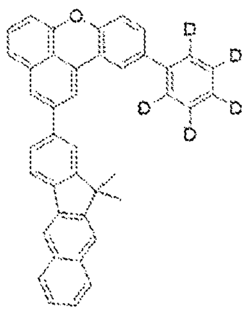
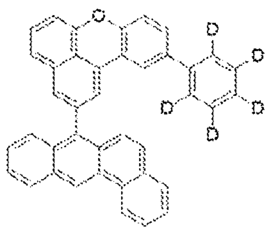
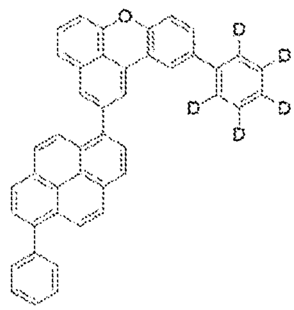
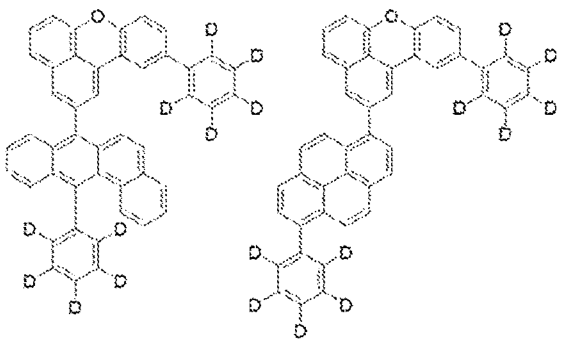
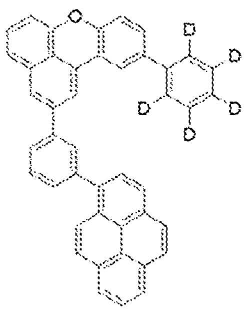
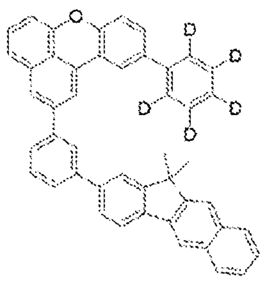

[Formula 280]

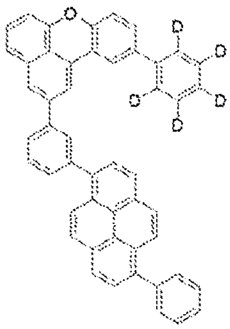

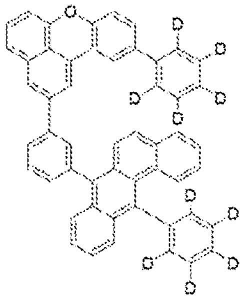

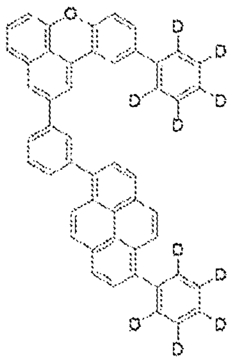

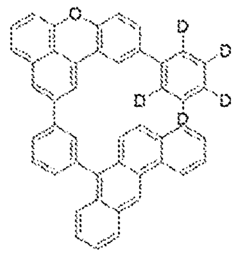

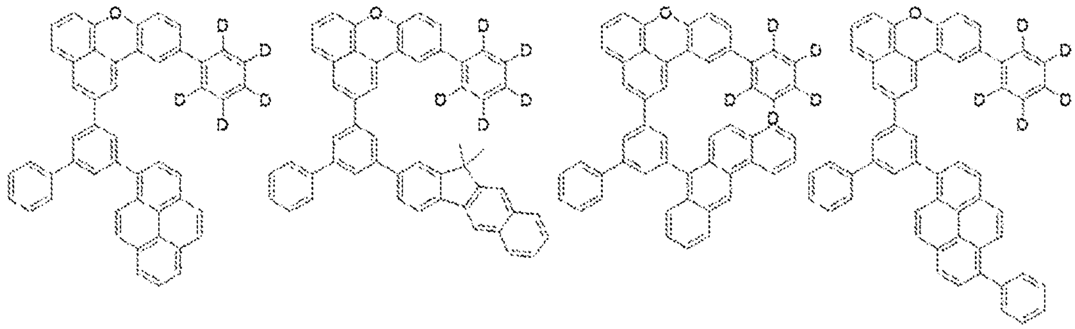

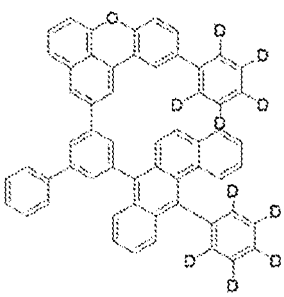

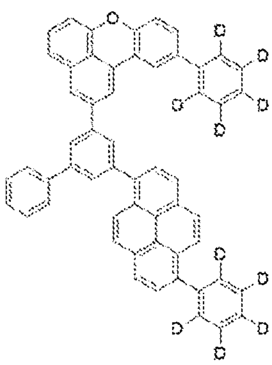

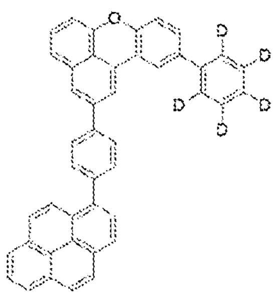

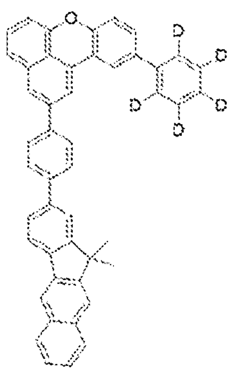

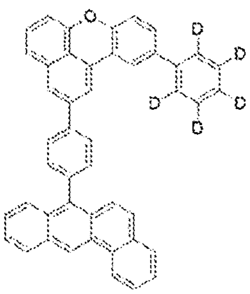

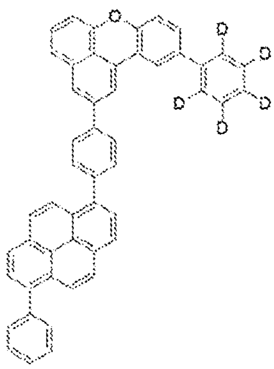

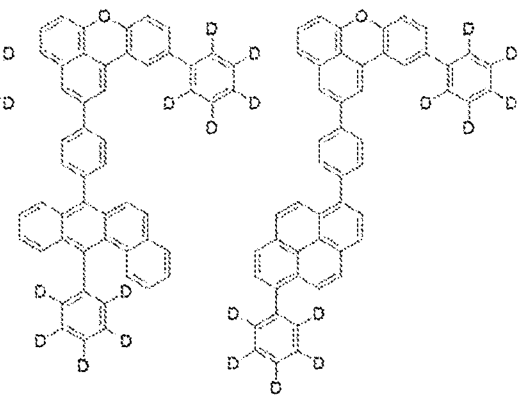

The compound according to the exemplary embodiment can improve performance of an organic EL device. Moreover, when the compound according to the exemplary embodiment is used in an emitting layer (first emitting layer) close to an anode of an organic EL device in which a plurality of emitting layers are layered, the organic EL device in a favorable balance between a luminous efficiency and a lifetime can be expected to be provided.

Second Exemplary Embodiment

Organic-Electroluminescence-Device Material

An organic-electroluminescence-device material according to a second exemplary embodiment contains the compound according to the first exemplary embodiment. As one example, the organic-electroluminescence-device material contains only the compound according to the first exemplary embodiment. As another example, the organic-electroluminescence-device material contains the compound according to the first exemplary embodiment and another compound(s) different from the compound according to the first exemplary embodiment.

In the organic-electroluminescence-device material according to the second exemplary embodiment, the compound according to the first exemplary embodiment is preferably a host material. In this case, the organic-electroluminescence-device material may contain the compound according to the first exemplary embodiment as the host material and another compound(s) such as a dopant material.

Third Exemplary Embodiment

Organic Electroluminescence Device

An organic EL device according to a third exemplary embodiment will be described.

The organic EL device according to the third exemplary embodiment includes an anode, a cathode, and at least one organic layer disposed between the anode and the cathode. The organic layer includes at least one layer formed of an organic compound. Alternatively, the organic layer includes a plurality of layers formed of an organic compound(s). The organic layer may further contain an inorganic compound.

In the organic EL device according to the third exemplary embodiment, at least one of the at least one organic layer contains the compound according to the first exemplary embodiment.

In the organic EL device according to the third exemplary embodiment, at least one of the at least one organic layer is preferably an emitting layer. In the organic EL device according to the third exemplary embodiment, the emitting layer preferably contains the compound according to the first exemplary embodiment. In an exemplary embodiment, the emitting layer contains a compound represented by the formula (100). In an exemplary embodiment, the emitting layer contains a compound represented by the formula (1). In an exemplary embodiment, the emitting layer contains a compound represented by the formula (100A).

In an exemplary embodiment, the emitting layer may contain a metal complex. In an exemplary embodiment, the emitting layer preferably does not contain a metal complex.

In an exemplary embodiment, the emitting layer preferably does not contain a phosphorescent material (dopant material).

In an exemplary embodiment, the emitting layer preferably does not contain a heavy metal complex and a phosphorescent rare-metal complex. Examples of the heavy metal complex include an iridium complex, osmium complex, and platinum complex.

For instance, the organic layer may consist of a single emitting layer or may further include at least one layer usable in organic EL devices. Examples of the layer usable in the organic EL device, which are not particularly limited, include at least one layer selected from the group consisting of a hole injecting layer, hole transporting layer, electron injecting layer, electron transporting layer, and blocking layer.

The organic EL device according to the exemplary embodiment preferably includes a hole transporting layer between the anode and the emitting layer.

The organic EL device according to the exemplary embodiment preferably includes an electron transporting layer between the cathode and the emitting layer.

In the organic EL device according to the exemplary embodiment, the organic layer may be provided by a plurality of emitting layers. For instance, the organic layer may be provided by two emitting layers, that is, a first emitting layer and a second emitting layer. The organic EL device according to the exemplary embodiment may include at least one organic layer in addition to the first and second emitting layers. For instance, the organic EL device according to the exemplary embodiment may further include at least one layer selected from the group consisting of a hole injecting layer, hole transporting layer, electron injecting layer, electron transporting layer, hole blocking layer, and electron blocking layer.

In the organic EL device according to the exemplary embodiment, the organic layer also preferably has the first and second emitting layers.

An exemplary arrangement is assumed in which the first emitting layer is disposed between the anode and the cathode and the second emitting layer is disposed between the first emitting layer and the cathode. Another exemplary arrangement is assumed in which the second emitting layer is disposed between the anode and the cathode and the

first emitting layer is disposed between the second emitting layer and the cathode. In the organic EL device according to the exemplary embodiment, the first and second emitting layers can be disposed in any one of the above exemplary arrangements.

In the organic EL device according to the exemplary embodiment, the first and second emitting layers may be in direct contact with each other or may not be in direct contact with each other.

An organic EL device according to an exemplary embodiment includes: the anode, the cathode, the first emitting layer disposed between the anode and the cathode, and the second emitting layer disposed between the first emitting layer and the cathode, in which the first emitting layer contains a first compound, the first compound is a compound represented by the formula (100) and having at least one group represented by the formula (110), and the first and second emitting layers are in direct contact with each other.

In a case where the organic layer has the emitting layer in the organic EL device according to the exemplary embodiment, the organic EL device preferably has the electron transporting layer between the cathode and the emitting layer. In a case where the emitting layer has the first emitting layer and the second emitting layer, the organic EL device may have the electron transporting layer between the cathode and the second emitting layer in an exemplary arrangement.

In a case where the organic layer has the emitting layer, the organic EL device according to the exemplary embodiment preferably has the hole transporting layer between the anode and the emitting layer. In a case where the emitting layer has the first emitting layer and the second emitting layer, the organic EL device may have the hole transporting layer between the anode and the first emitting layer in another exemplary arrangement.

In the organic EL device according to the exemplary embodiment, a non-doped region, which does not contain a third compound that fluoresces, may also be provided on a side of the first emitting layer close to the anode.

The organic EL device according to the exemplary embodiment may have the first emitting layer, the second emitting layer, and the non-doped region disposed between the anode and the first emitting layer. The non-doped region may be, for instance, a non-doped organic layer provided on a side of the first emitting layer close to the anode and being in direct contact with the first emitting layer.

The non-doped organic layer does not contain a metal atom. A content ratio of each of all materials forming the non-doped organic layer with respect to the non-doped organic layer is preferably 10 mass% or more, more preferably more than 10 mass%. The non-doped organic layer preferably contains the compound according to the first exemplary embodiment. The non-doped organic layer also preferably consists of the compound according to the first exemplary embodiment.

The non-doped organic layer is, for instance, a second hole transporting layer or an electron blocking layer.

Fig. 1 schematically shows an exemplary arrangement of the organic EL device of the exemplary embodiment.

An organic EL device 1 includes a light-transmissive substrate 2, an anode 3, a cathode 4, and an organic layer 10 provided between the anode 3 and the cathode 4. The organic layer 10 includes a hole injecting layer 6, a hole transporting layer 7, a first emitting layer 51, a second emitting layer 52, an electron transporting layer 8, and an electron injecting layer 9, which are sequentially layered on the anode 3.

First Emitting Layer

In the organic EL device according to the exemplary embodiment, the first emitting layer preferably contains the first compound. The first compound is preferably a first host material.

In the organic EL device according to the exemplary embodiment, it is also preferable that the first emitting layer further contains a third compound that emits fluorescence. The first emitting layer also preferably contains the first compound as the fist host material and the third compound as a first dopant material.

Herein, the "host material" refers to, for instance, a material that accounts for "50 mass% or more of the layer." Accordingly, for instance, the first emitting layer contains 50 mass% or more of the first compound with respect to a total mass of the first emitting layer. Moreover, for instance, the "host material" may account for 60 mass% or more of the layer, 70 mass% or more of the layer, 80 mass% or more of the layer, 90 mass% or more of the layer, or 95 mass% or more of the layer.

First Compound

In the organic EL device according to the exemplary embodiment, the first compound is the compound according to the first exemplary embodiment.

In an organic EL device according to an exemplary embodiment, the first compound is a compound represented by the formula (1000B) and having at least one group represented by the formula (110).

In an organic EL device according to an exemplary embodiment, the first compound is a compound having at least one group represented by the formula (11) and a single benz[de]anthracene derivative skeleton represented by the formula (1000) in a molecule.

In an organic EL device according to an exemplary embodiment, the first compound is a compound having at least one group represented by the formula (110A) and having a single benz[de]anthracene derivative skeleton represented by the formula (1000A) in a molecule.

Second Emitting Layer

In the organic EL device according to the exemplary embodiment, the second emitting layer preferably contains a second compound. The second compound is preferably a second host material.

In the organic EL device according to the exemplary embodiment, it is also preferable that the second emitting layer further contains a fourth compound that emits fluorescence. The second emitting layer also preferably contains the second compound as the second host material and the fourth compound as a second dopant material.

When the first emitting layer contains the third compound and the the second emitting layer contains the fourth compound, the third compound and the fourth compound are mutually the same or different.

Second Compound

In the organic EL device according to the exemplary embodiment, the second compound is not particularly limited. In the organic EL device according to the exemplary embodiment, the second compound as the host material is exemplified by a heterocyclic compound and a fused aromatic compound. Examples of the fused aromatic compound include an anthracene derivative, a pyrene derivative, and a benzanthracene derivative.

The second compound is also preferably, for instance, a compound represented by a formula (2).

[Formula 281]

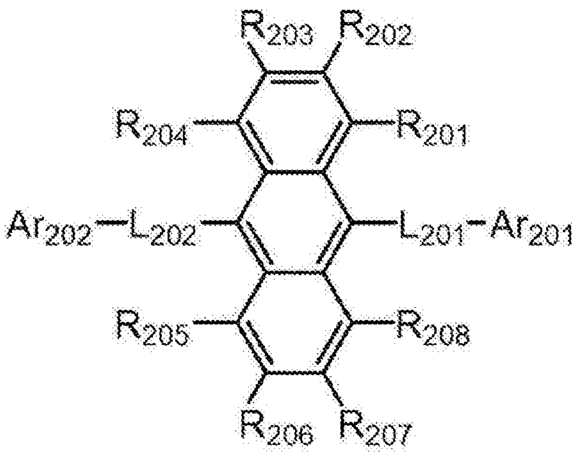

(2)

In the formula (2):

$R_{201}$ to $R_{208}$ are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by $-C(=O)R_{801}$, a group represented by $-COOR_{802}$, a halogen atom, a nitro group, a substituted or unsubstituted aryl group having 6 to 50 ring carbon atoms, or a substituted or unsubstituted heterocyclic group having 5 to 50 ring atoms;

$L_{201}$ and $L_{202}$ are each independently a single bond, a substituted or unsubstituted arylene group having 6 to 50 ring carbon atoms, or a substituted or unsubstituted divalent heterocyclic group having 5 to 50 ring atoms;

$Ar_{201}$ and $Ar_{202}$ are each independently a substituted or unsubstituted aryl group having 6 to 50 ring carbon atoms, or a substituted or unsubstituted heterocyclic group having 5 to 50 ring atoms.

In the second compound according to the exemplary embodiment, $R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{801}$ are present, the plurality of $R_{801}$ are mutually the same or different; and
when a plurality of $R_{802}$ are present, the plurality of $R_{802}$ are mutually the same or different.

In the organic EL device according to the exemplary embodiment, it is preferable that $R_{201}$ to $R_{208}$ are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by $-C(=O)R_{801}$, a group represented by $-COOR_{802}$, a halogen atom, or a nitro group;

$L_{201}$ and $L_{202}$ are each independently a single bond, a substituted or unsubstituted arylene group having 6 to 50 ring carbon atoms, or a substituted or unsubstituted divalent heterocyclic group having 5 to 50 ring atoms;

$Ar_{201}$ and $Ar_{202}$ are each independently a substituted or unsubstituted aryl group having 6 to 50 ring carbon atoms, or a substituted or unsubstituted heterocyclic group having 5 to 50 ring atoms.

In the second compound, $R_{201}$ to $R_{208}$ also preferably each independently a hydrogen atom, a substituted or unsubstituted alkyl group having 1 to 50 carbon atoms, a substituted or unsubstituted cycloalkyl having 3 to 50 ring carbon atoms, or a group represented by $-Si(R_{901})(R_{902})(R_{903})$.

In the second compound, $R_{201}$ to $R_{208}$ are also preferably each a hydrogen atom.

In the organic EL device according to the exemplary embodiment, it is preferable that $L_{201}$ and $L_{202}$ are each independently a single bond or a substituted or unsubstituted arylene group having 6 to 50 ring carbon atoms and $Ar_{201}$ and $Ar_{202}$ are each independently a substituted or unsubstituted aryl group having 6 to 50 ring carbon atoms.

In the organic EL device according to the exemplary embodiment, $Ar_{201}$ and $Ar_{202}$ are preferably each independently phenyl group, a naphthyl group, phenanthryl group, a biphenyl group, a terphenyl group, a diphenylfluorenyl group, a dimethylfluorenyl group, a benzodiphenylfluorenyl group, a benzodimethylfluorenyl group, dibenzofuranyl group, a dibenzothienyl group, a benzoxanthenyl group, a naphthobenzofuranyl group, or a naphthobenzothienyl group.

In the organic EL device according to the exemplary embodiment, the second compound represented by the formula (2) is preferably a compound represented by a formula (201), (202), (203), (204), (205), (206), (207), (208) or (209) below.

[Formula 282]

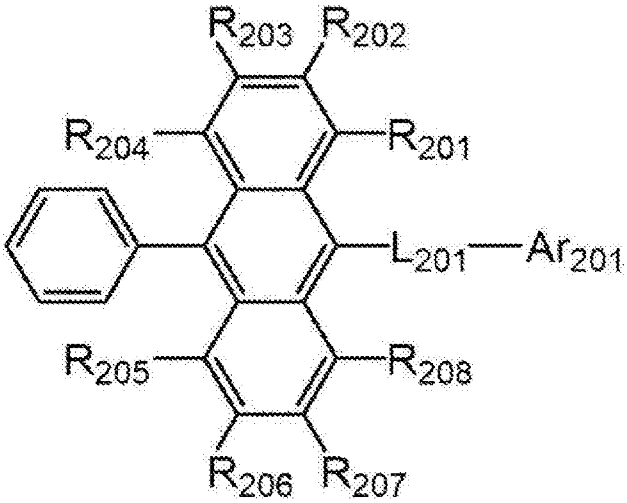

(201)

[Formula 283]

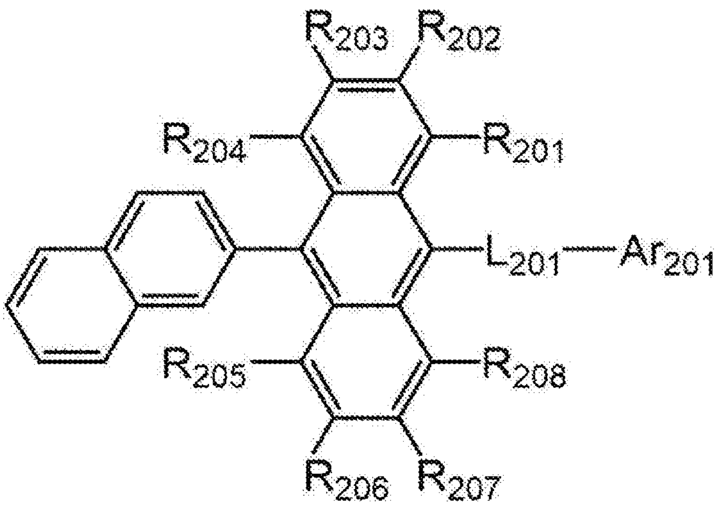

(202)

[Formula 284]

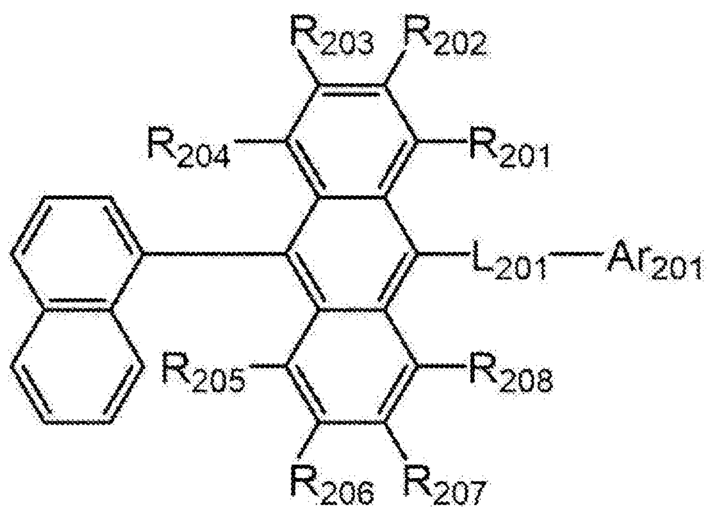

(203)

[Formula 285]

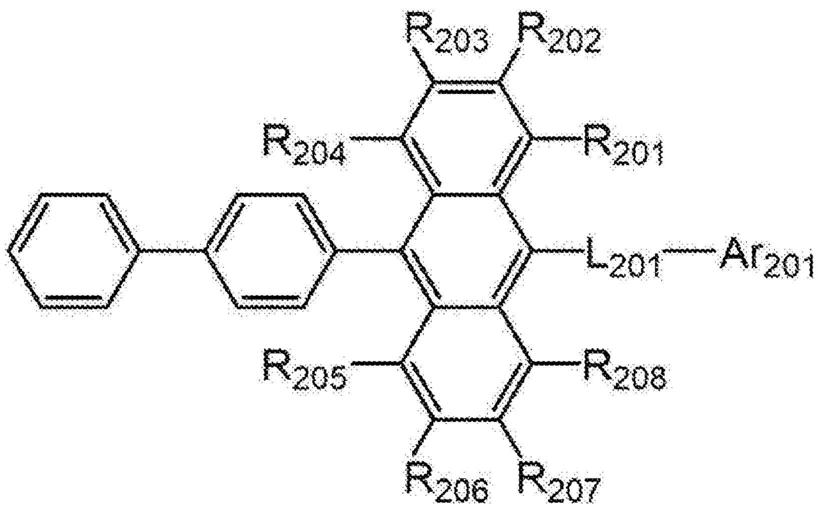

(204)

[Formula 286]

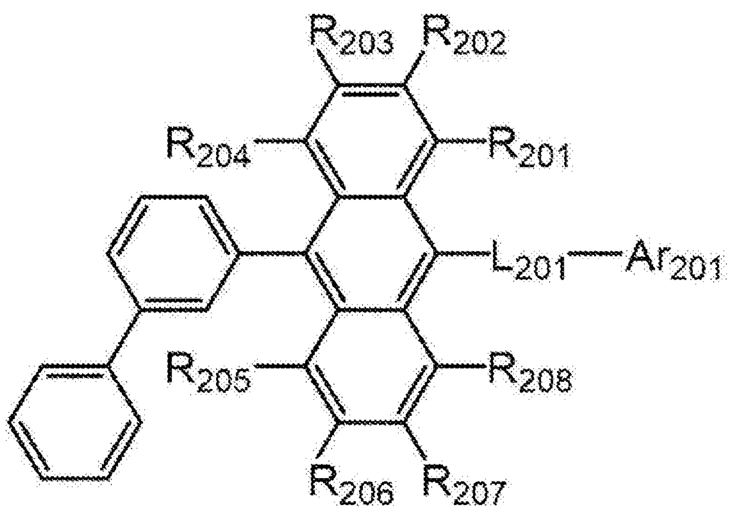

(205)

[Formula 287]

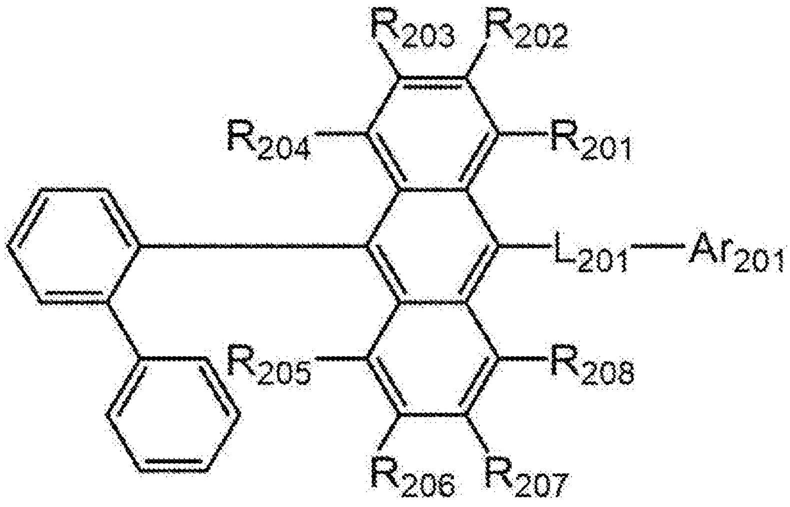

(206)

[Formula 288]

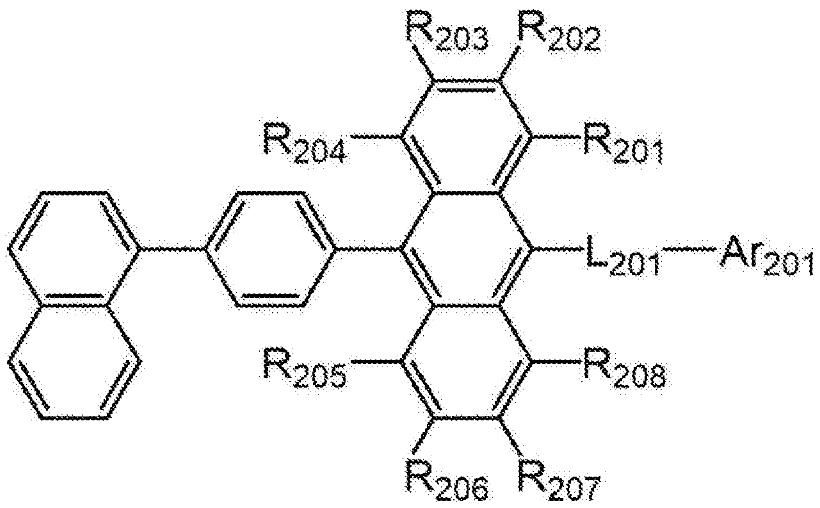

(207)

[Formula 289]

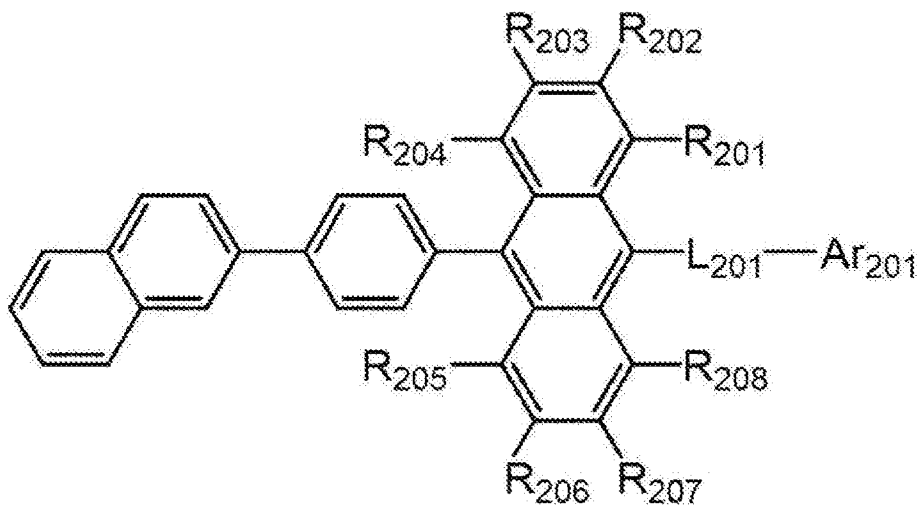

(208)

[Formula 290]

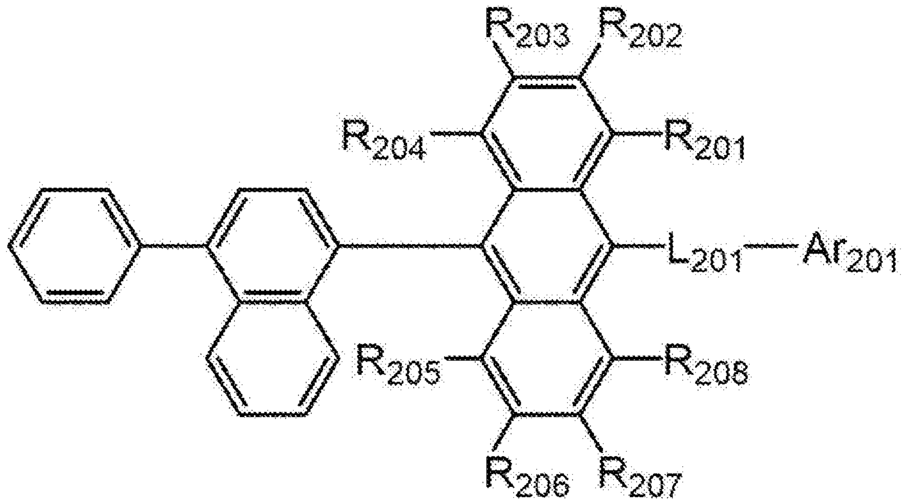

(209)

In the formulae (201) to (209), $L_{201}$ and $Ar_{201}$ represent the same as $L_{201}$ and $Ar_{201}$ in the formula (2), and $R_{201}$ to $R_{208}$ each independently represent the same as $R_{201}$ to $R_{208}$ in the formula (2).

The second compound represented by the formula (2) is also preferably a compound represented by a formula (221), (222), (223), (224), (225), (226), (227), (228) or (229) below.

[Formula 291]

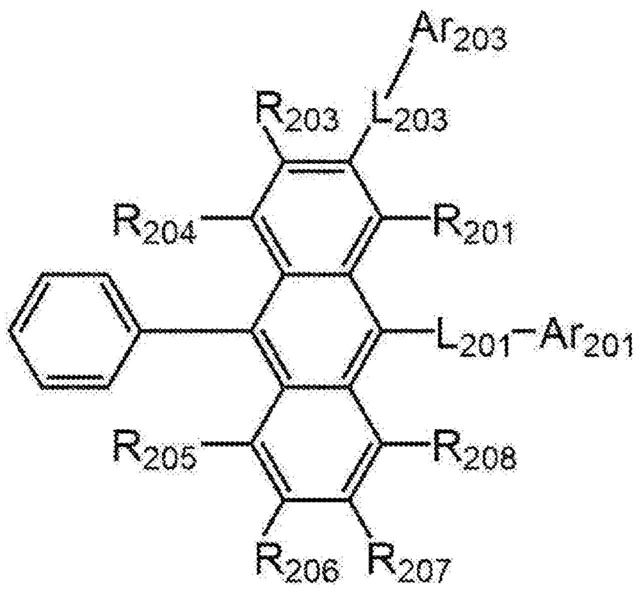

(221)

[Formula 292]

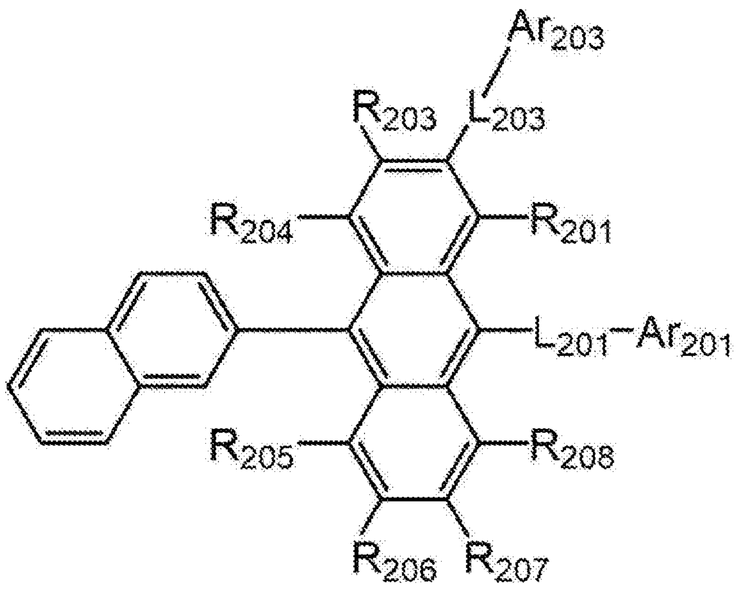

(222)

[Formula 293]

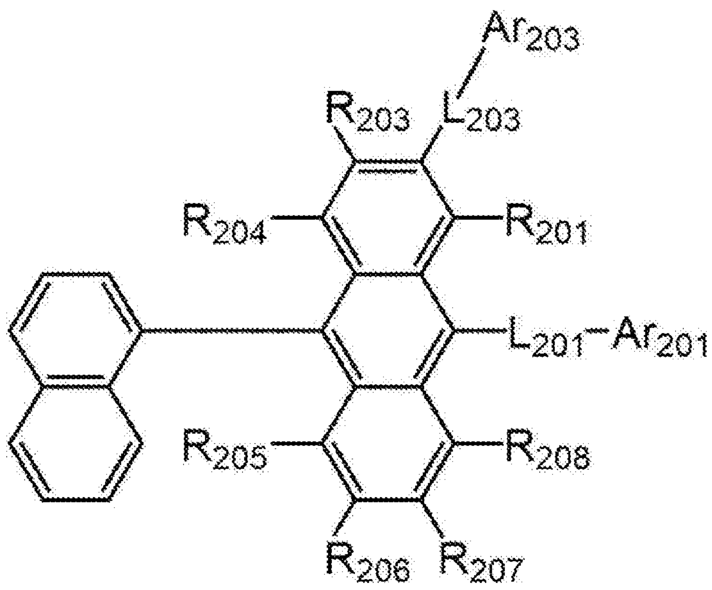

(223)

[Formula 294]

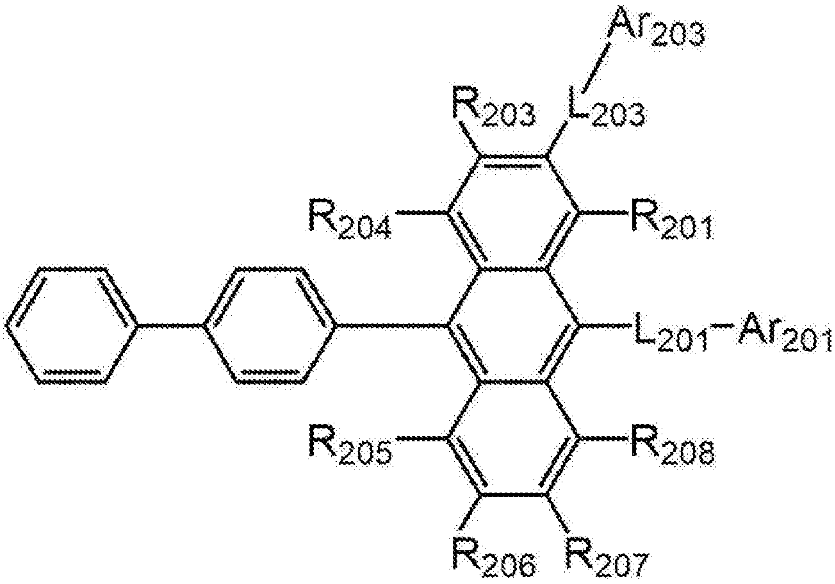

(224)

[Formula 295]

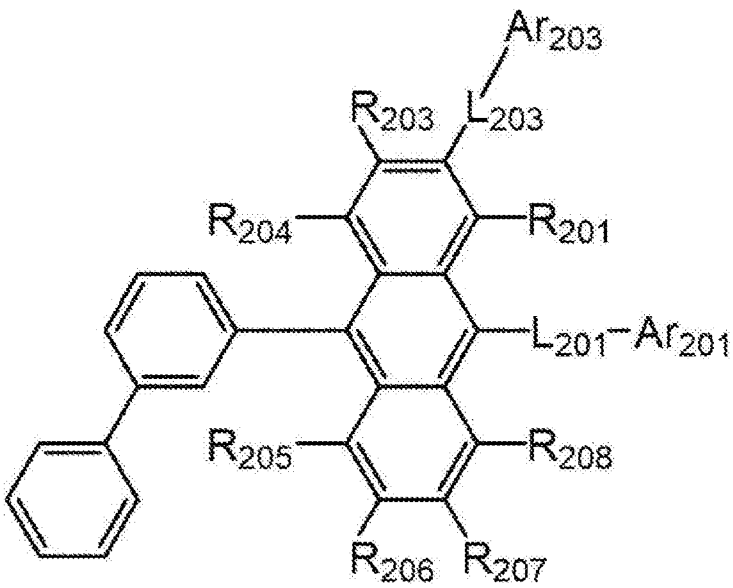

(225)

[Formula 296]

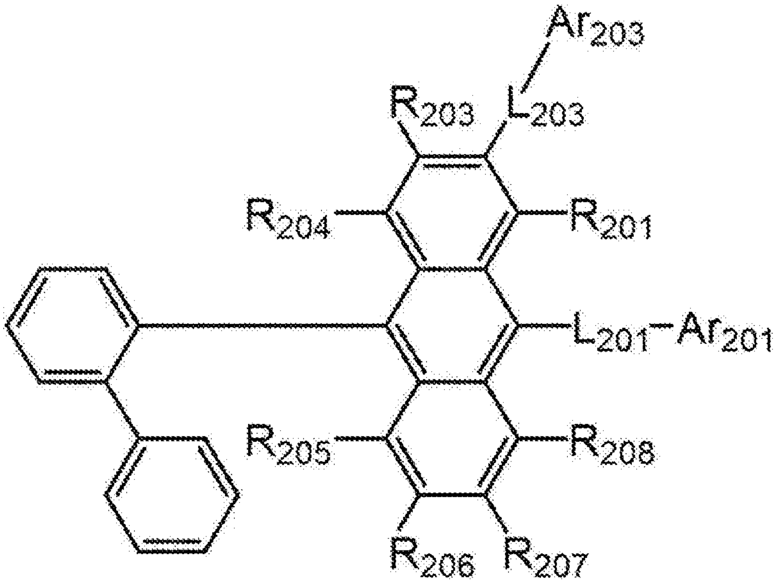

(226)

[Formula 297]

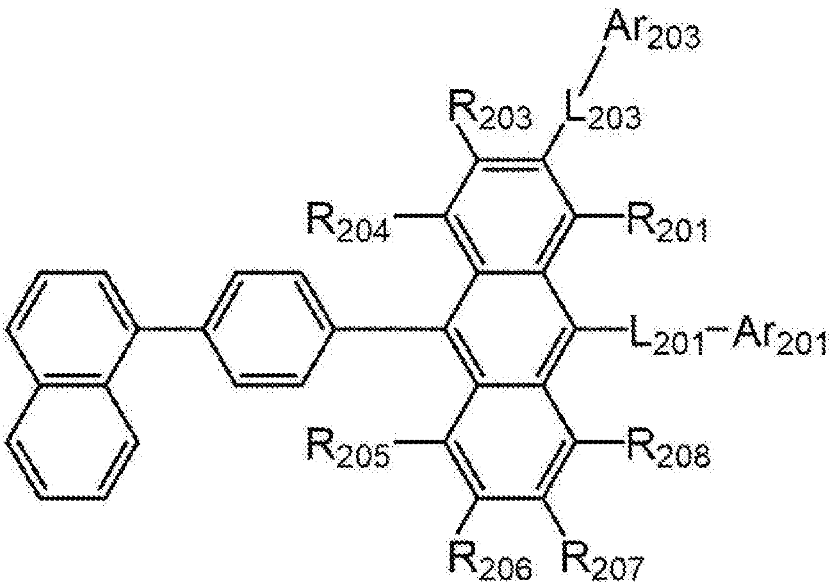

(227)

[Formula 298]

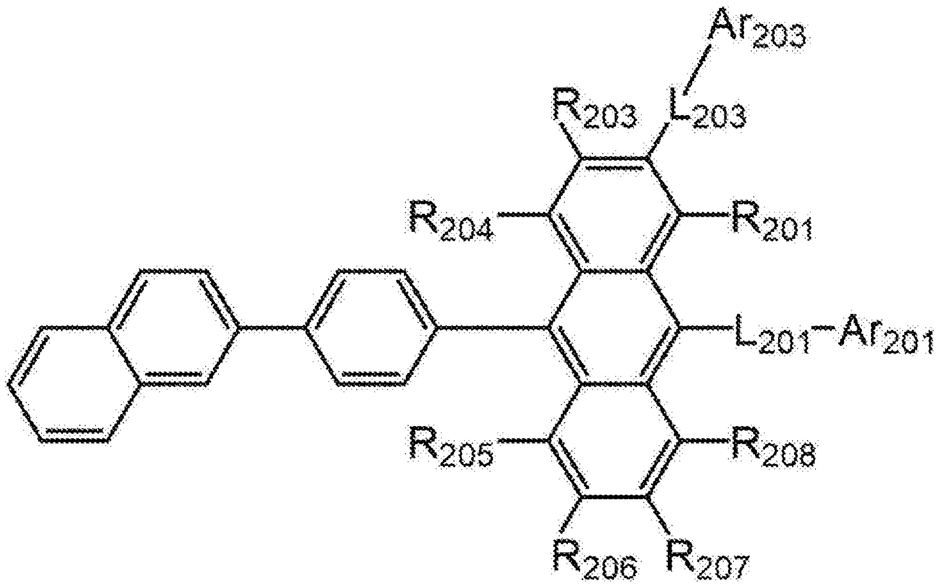

(228)

[Formula 299]

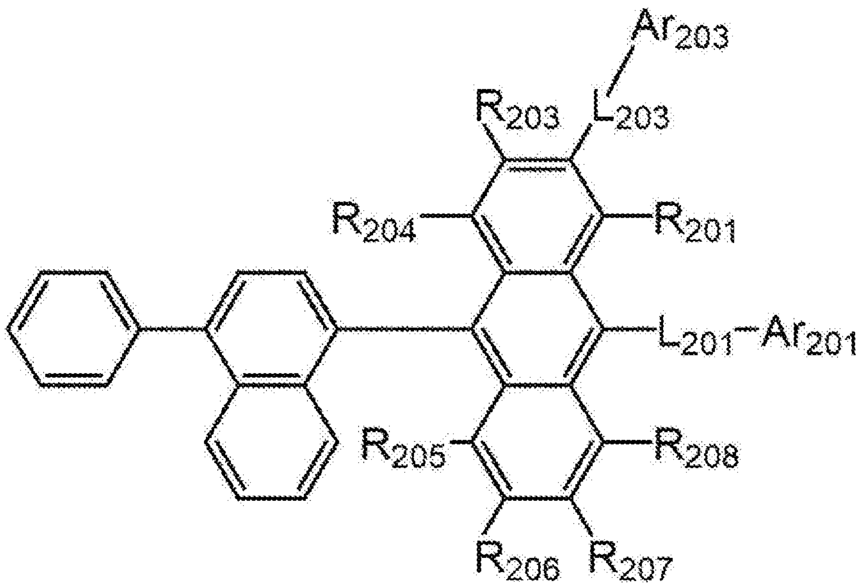

(229)

In the formulae (221), (222), (223), (224), (225), (226), (227), (228) and (229):

$R_{201}$ and $R_{203}$ to $R_{208}$ each independently represent the same as $R_{201}$ and $R_{203}$ to $R_{208}$ in the formula (2);
$L_{201}$ and $Ar_{201}$ respectively represent the same as $L_{201}$ and $Ar_{201}$ in the formula (2);
$L_{203}$ represents the same as $L_{201}$ in the formula (2);
$L_{203}$ and $L_{201}$ are mutually the same or different;
$Ar_{203}$ represents the same as $Ar_{201}$ in the formula (2); and
$Ar_{203}$ and $Ar_{201}$ are mutually the same or different.

The second compound represented by the formula (2) is also preferably a compound represented by a formula (241), (242), (243), (244), (245), (246), (247), (248) or (249) below.

[Formula 300]

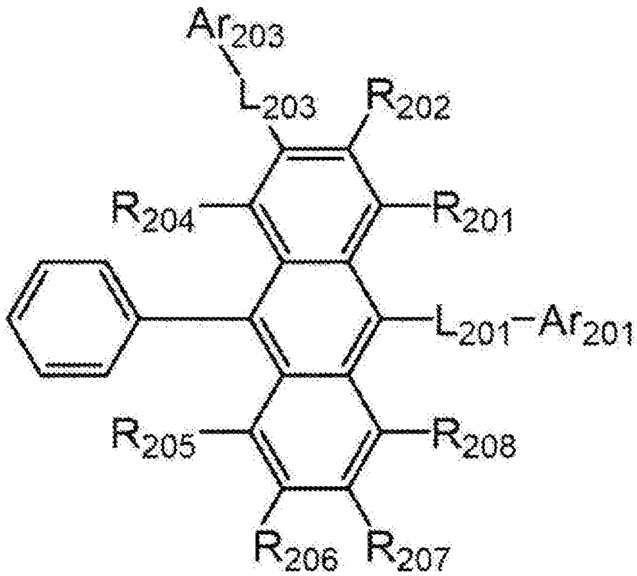

(241)

[Formula 301]

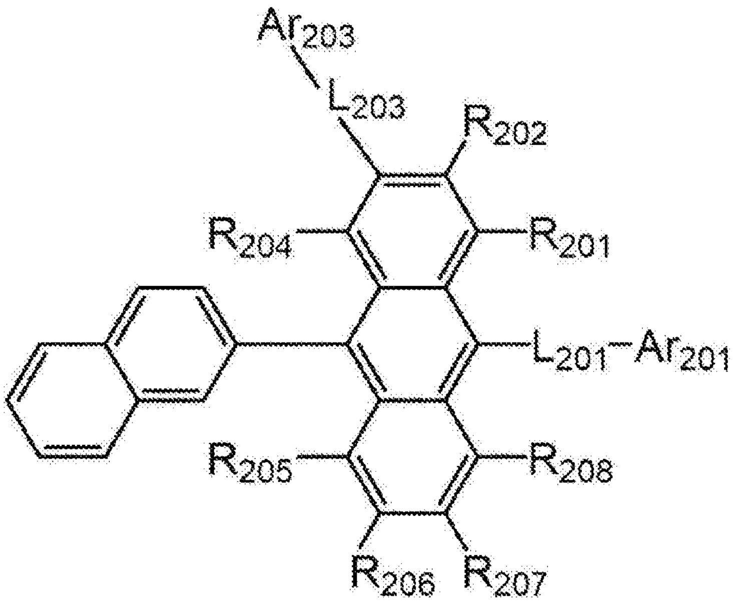

(242)

[Formula 302]

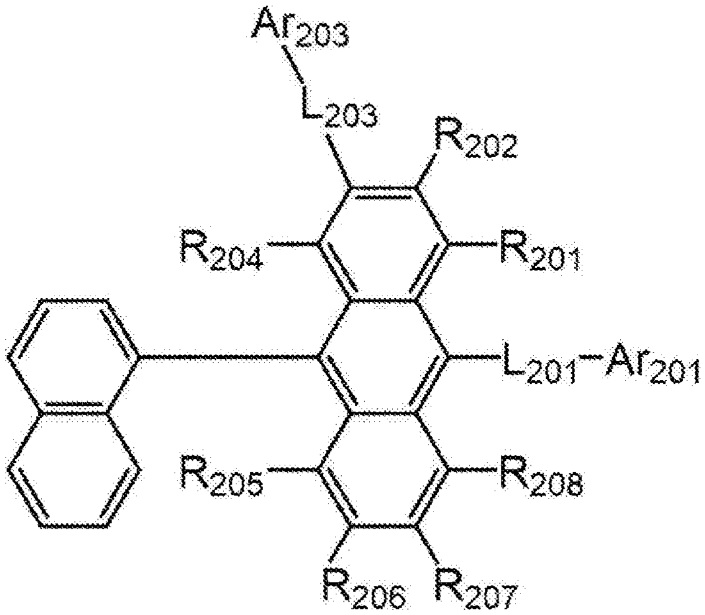

(243)

[Formula 303]

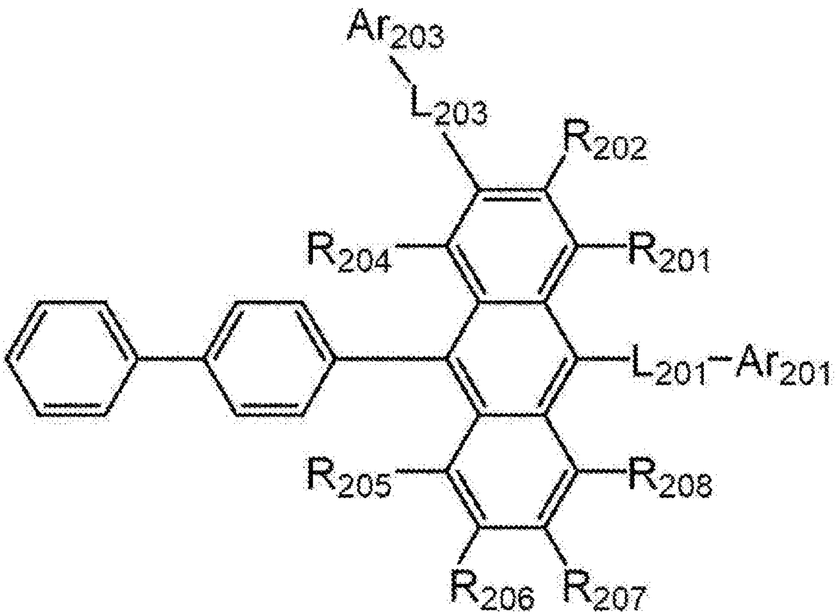

(244)

[Formula 304]

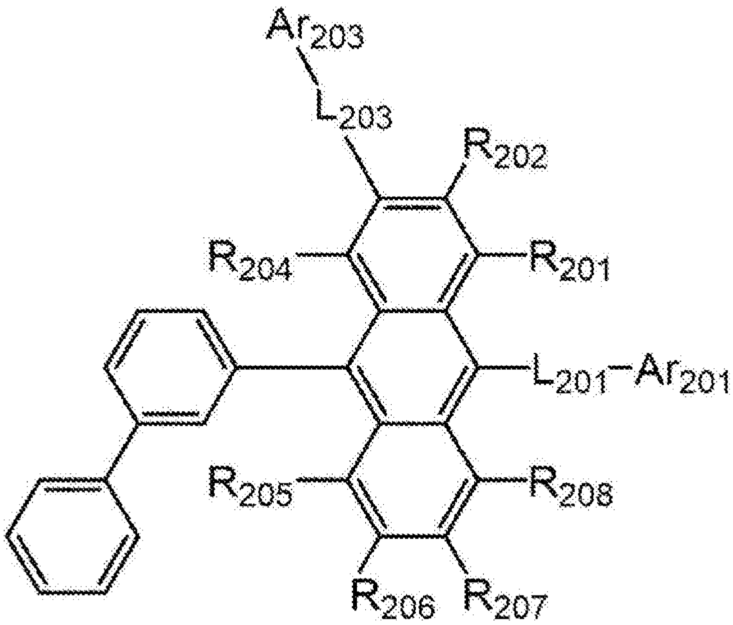

(245)

[Formula 305]

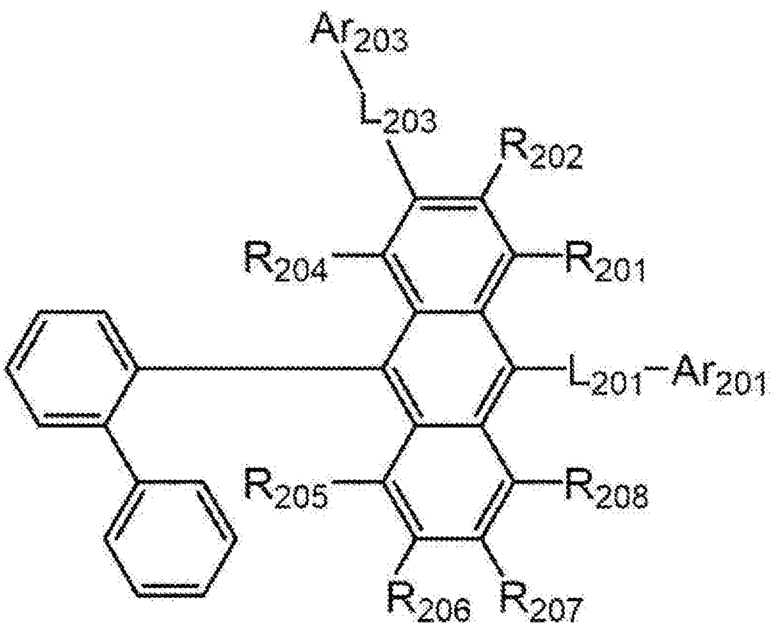

(246)

[Formula 306]

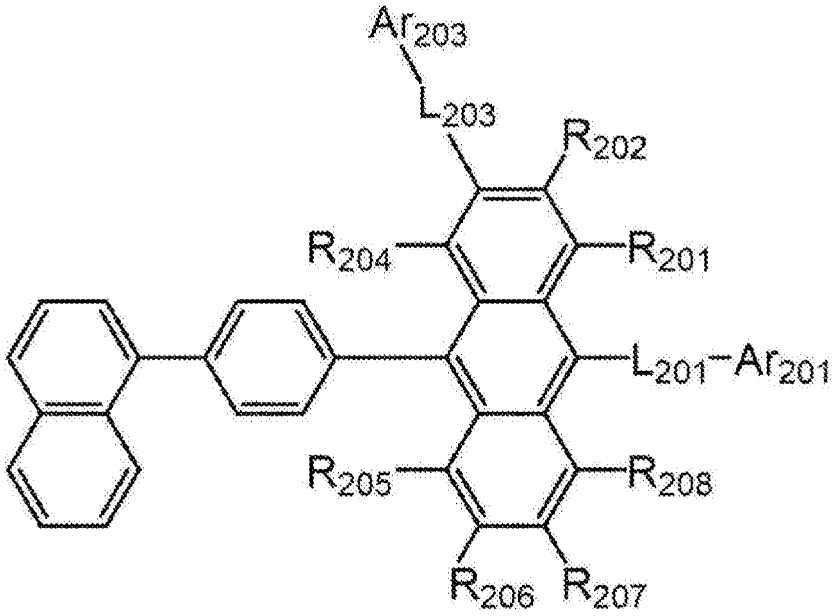

(247)

[Formula 307]

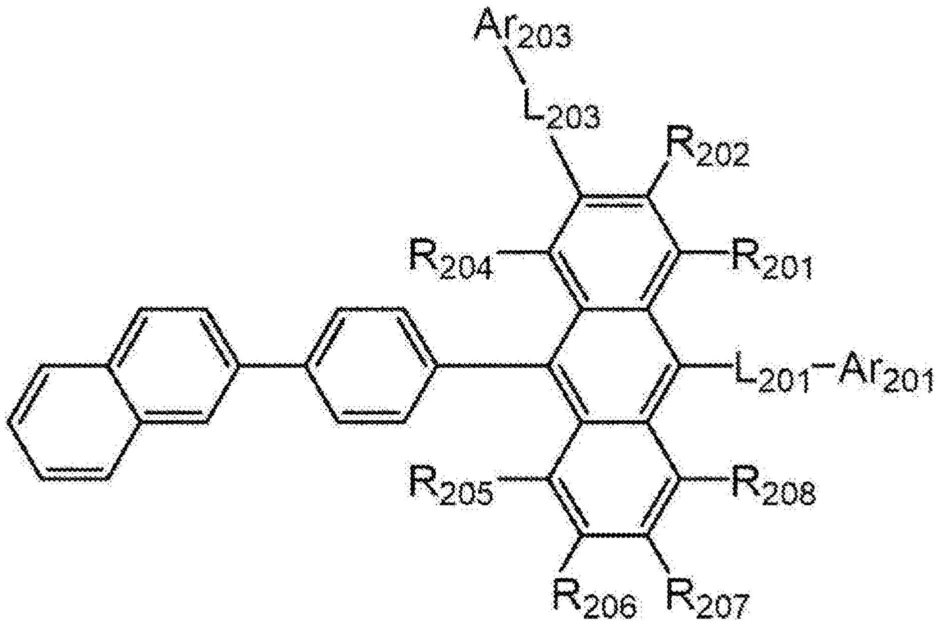

(248)

[Formula 308]

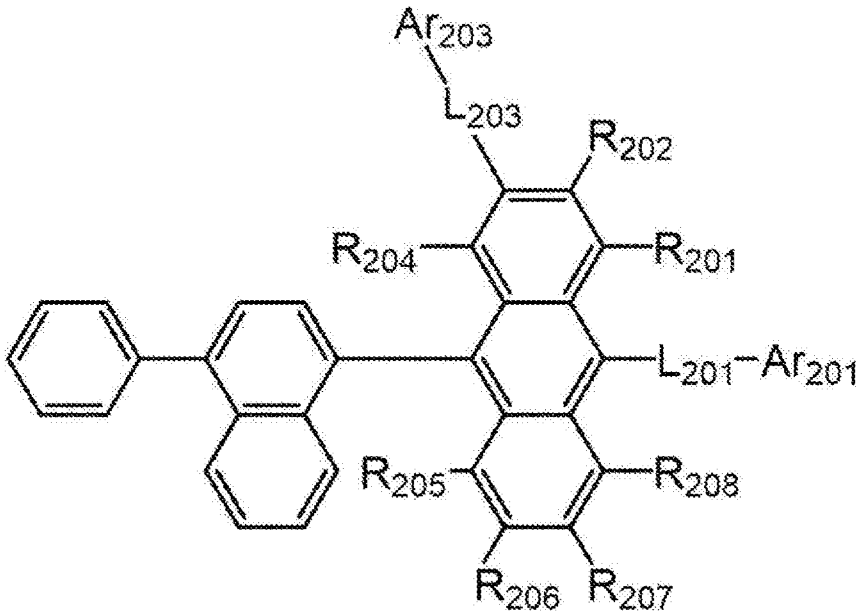

(249)

In the formulae (241), (242), (243), (244), (245), (246), (247), (248) and (249):

$R_{201}$, $R_{202}$ and $R_{204}$ to $R_{208}$ each independently represent the same as $R_{201}$, $R_{202}$ and $R_{204}$ to $R_{208}$ in the formula (2);
$L_{201}$ and $Ar_{201}$ respectively represent the same as $L_{201}$ and $Ar_{201}$ in the formula (2);
$L_{203}$ represents the same as $L_{201}$ in the formula (2);
$L_{203}$ and $L_{201}$ are mutually the same or different;
$Ar_{203}$ represents the same as $Ar_{201}$ in the formula (2); and
$Ar_{203}$ and $Ar_{201}$ are mutually the same or different.

$R_{201}$ to $R_{208}$ in the second compound represented by the formula (2) are preferably each independently a hydrogen atom, a substituted or unsubstituted alkyl group having 1 to 50 carbon atoms, a substituted or unsubstituted cycloalkyl group having 3 to 50 ring carbon atoms, or a group represented by -Si($R_{901}$)($R_{902}$)($R_{903}$).

$L_{101}$ is preferably a single bond, or an unsubstituted arylene group having 6 to 22 ring carbon atoms.

$Ar_{101}$ is preferably a substituted or unsubstituted aryl group having 6 to 22 ring carbon atoms.

In the organic EL device according to the exemplary embodiment, $R_{201}$ to $R_{208}$ that are substituents on an anthracene skeleton in the second compound represented by the formula (2) are preferably hydrogen atoms in terms of preventing inhibition of intermolecular interaction to inhibit a decrease in electron mobility. However, $R_{201}$ to $R_{208}$ may be a substituted or unsubstituted aryl group having 6 to 50 ring carbon atoms or a substituted or unsubstituted heterocyclic group having 5 to 50 ring atoms.

Assuming that $R_{201}$ to $R_{208}$ each are a bulky substituent such as an alkyl group and a cycloalkyl group, intermolecular interaction may be inhibited to decrease the electron mobility of the second compound relative to that of the first compound, so that a relationship of $\mu H2 > \mu H1$ shown by a numerical formula below (Numerical Formula 3) may not be satisfied. When the second compound is used in the second emitting layer, it can be expected that satisfying the relationship of $\mu H2 > \mu H1$ inhibits a decrease in a recombination ability between holes and electrons in the first emitting layer and a decrease in a luminous efficiency. It should be noted that substituents, namely, a haloalkyl group, alkenyl group, alkynyl group, group represented by - Si($R_{901}$)($R_{902}$)($R_{903}$), group represented by -O-($R_{904}$), group represented by -S-($R_{905}$), group represented by -N($R_{906}$)($R_{907}$), aralkyl group, group represented by -C(=O)$R_{801}$, group represented by -COO$R_{802}$, halogen atom, cyano group, and nitro group are likely to be bulky, and an alkyl group and cycloalkyl group are likely to be further bulky.

In the second compound represented by the formula (2), $R_{201}$ to $R_{208}$ being the substituents on the anthracene skeleton are each preferably not a bulky substituent and preferably not an alkyl group and cycloalkyl group. More preferably, $R_{201}$ to $R_{208}$ are not an alkyl group, cycloalkyl group, haloalkyl group, alkenyl group, alkynyl group, group represented by -Si($R_{901}$)($R_{902}$)($R_{903}$), group represented by -O-($R_{904}$), group represented by -S-($R_{905}$), group represented by -N($R_{906}$)($R_{907}$), aralkyl group, group represented by C(=O)$R_{801}$, group represented by -COO$R_{802}$, halogen atom, cyano group, and nitro group.

In the second compound represented by the formula (2) in the organic EL device according to the exemplary embodiment, $R_{201}$ to $R_{208}$ also preferably each independently a hydrogen atom, a substituted or unsubstituted alkyl

group having 1 to 50 carbon atoms, a substituted or unsubstituted cycloalkyl having 3 to 50 ring carbon atoms, or a group represented by -Si($R_{901}$)($R_{902}$)($R_{903}$).

In the second compound represented by the formula (2) in the organic EL device according to the exemplary embodiment, $R_{201}$ to $R_{208}$ are preferably each a hydrogen atom.

In the second compound, it is also preferable that examples of a substituent for a "substituted or unsubstituted group" on $R_{201}$ to $R_{208}$ do not include the above-described substituent that is likely to be bulky, especially a substituted or unsubstituted alkyl group and a substituted or unsubstituted cycloalkyl group. Since the examples of the substituent for a "substituted or unsubstituted" group on $R_{201}$ to $R_{208}$ do not include a substituted or unsubstituted alkyl group and a substituted or unsubstituted cycloalkyl group, inhibition of intermolecular interaction to be caused by presence of a bulky substituent such as an alkyl group and a cycloalkyl group can be prevented, thereby preventing a decrease in the electron mobility. Moreover, when the second compound described above is used in the second emitting layer, a decrease in a recombination ability between holes and electrons in the first emitting layer and a decrease in the luminous efficiency can be inhibited.

It is more preferable that $R_{201}$ to $R_{208}$ being the substituents on the anthracene skeleton are not bulky substituents, and $R_{201}$ to $R_{208}$ as substituents are unsubstituted. Assuming that $R_{201}$ to $R_{208}$ being the substituents on the anthracene skeleton are not bulky substituents and substituents are bonded to $R_{201}$ to $R_{208}$ which are the not-bulky substituents, the substituents bonded to $R_{201}$ to $R_{208}$ are preferably not the bulky substituents; the substituents bonded to $R_{201}$ to $R_{208}$ serving as substituents are preferably not an alkyl group and cycloalkyl group, more preferably not an alkyl group, cycloalkyl group, haloalkyl group, alkenyl group, alkynyl group, group represented by -Si($R_{901}$)($R_{902}$)($R_{903}$), group represented by -O-($R_{904}$), group represented by -S-($R_{905}$), group represented by -N($R_{906}$)($R_{907}$), aralkyl group, group represented by -C(=O)$R_{801}$, group represented by -COO$R_{802}$, halogen atom, cyano group, and nitro group.

In the first compound and the second compound, it is also preferable that all of the "substituted or unsubstituted" groups are "unsubstituted" groups.

In the organic EL device according to the exemplary embodiment, for instance, $Ar_{201}$ in the second compound represented by the formula (2) is a substituted or unsubstituted dibenzofuranyl group.

In the organic EL device according to the exemplary embodiment, for instance, $Ar_{201}$ in the second compound represented by the formula (2) is an unsubstituted dibenzofuranyl group.

In the organic EL device according to the exemplary embodiment, for instance, the second compound represented by the formula (2) has at least one hydrogen atom, the hydrogen atom including at least one deuterium atom.

In the organic EL device according to the exemplary embodiment, for instance, $L_{201}$ in the second compound represented by the formula (2) is one of TEMP-63 to TEMP-68.

[Formula 309]

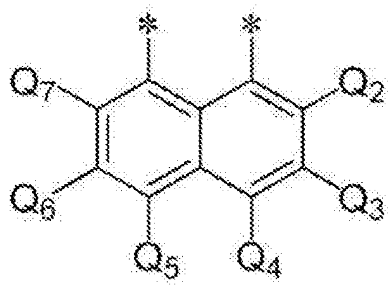
(TEMP-63)

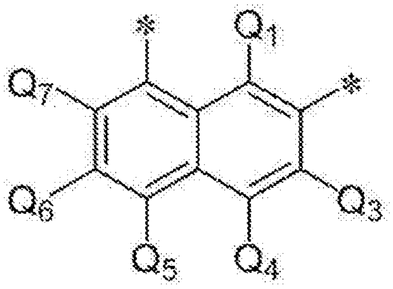
(TEMP-64)

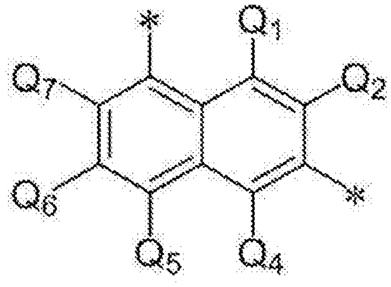
(TEMP-65)

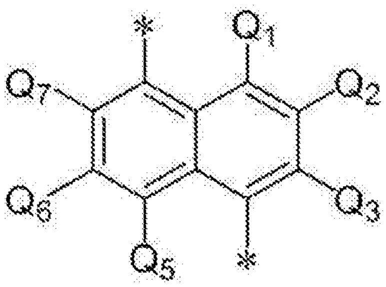
(TEMP-66)

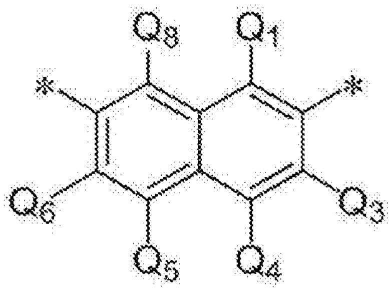
(TEMP-67)

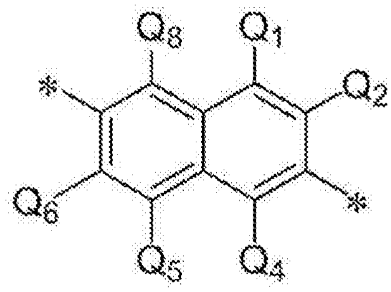
(TEMP-68)

In the formulae (TEMP-63) to (TEMP-68), * represents a bonding position.

In the second compound represented by the formula (2) in the organic EL device according to the exemplary embodiment, $Ar_{201}$ is at least one group selected from the group consisting of a substituted or unsubstituted anthryl group, benzanthryl group, phenanthryl group, benzophenanthryl group, phenalenyl group, pyrenyl group, chrysenyl group, benzochrysenyl group, triphenylenyl group, benzotriphenylenyl group, tetracenyl group, pentacenyl group, fluoranthenyl group, benzofluoranthenyl group, and perylenyl group.

In the organic EL device according to the exemplary embodiment, for instance, $Ar_{201}$ in the second compound represented by the formula (2) is a substituted or unsubstituted fluorenyl group.

In the organic EL device according to the exemplary embodiment, for instance, $Ar_{201}$ in the second compound represented by the formula (2) is a substituted or unsubstituted xanthenyl group.

In the organic EL device according to the exemplary embodiment, for instance, $Ar_{201}$ in the second compound represented by the formula (2) is a benzoxanthenyl group.

Manufacturing Method of Second Compound

The second compound can be manufactured by a known method. The second compound can also be manufactured based on a known method through a known alternative reaction using a known material(s) tailored for the target compound.

Specific Examples of Second Compound

Examples of the second compound include compounds below. It should however be noted that the invention is not limited to the specific examples of the second compound.

[Formula 310]

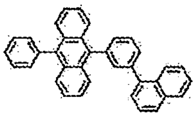
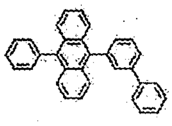
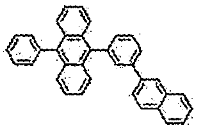
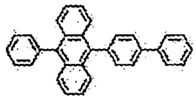
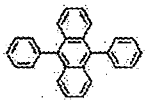
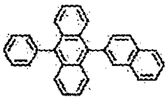
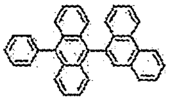
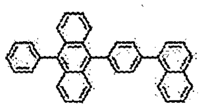
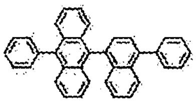
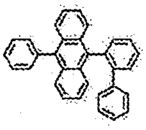
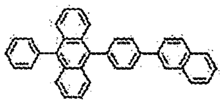
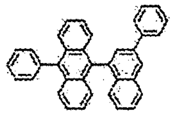
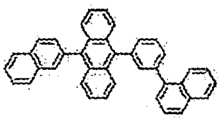
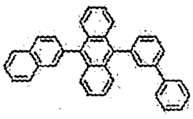
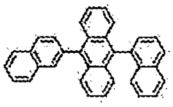
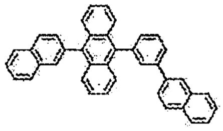
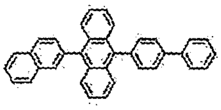
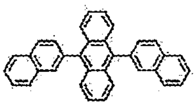
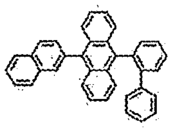
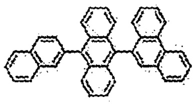
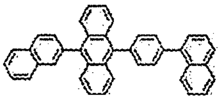
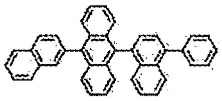
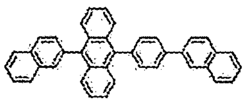
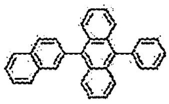
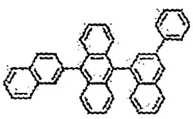
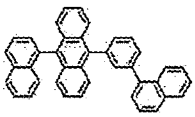
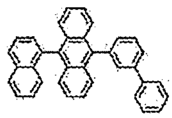
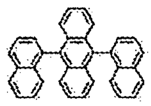
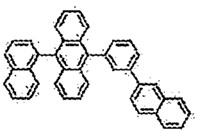
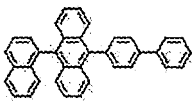
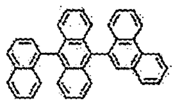
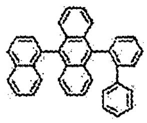
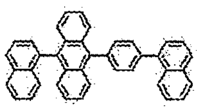
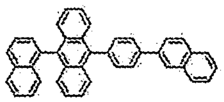
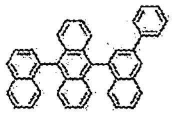
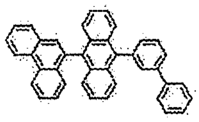
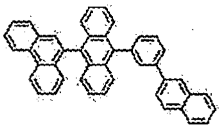
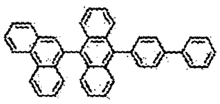
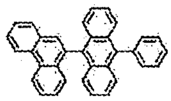
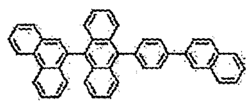

[Formula 311]

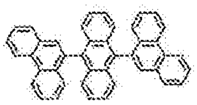
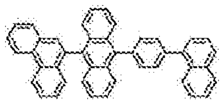
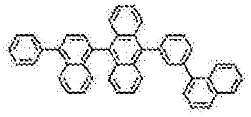
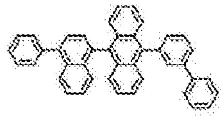
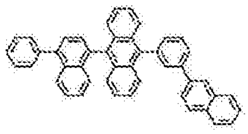
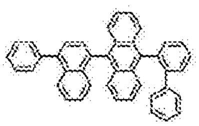
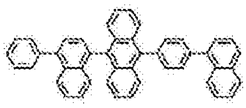
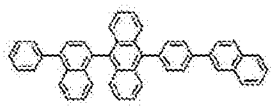
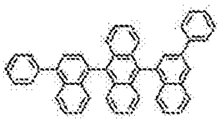
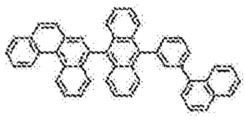
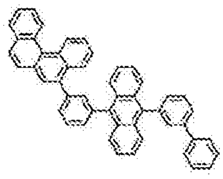
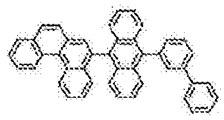
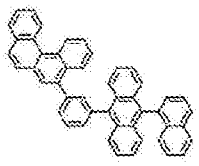
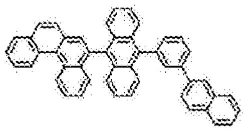
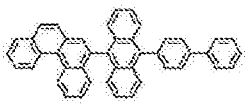
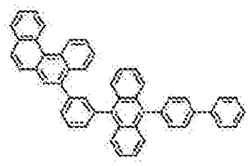
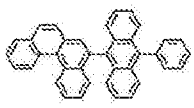
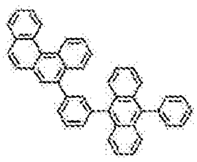
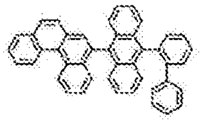
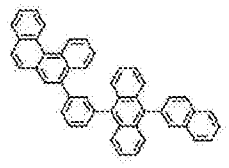
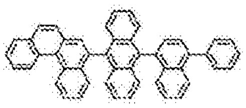
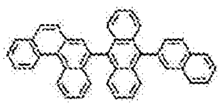
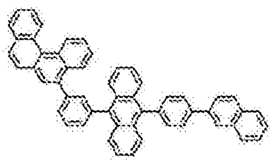
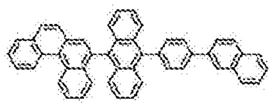
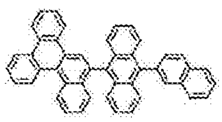
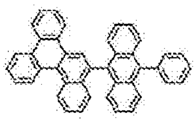
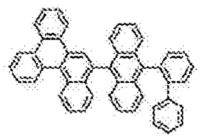
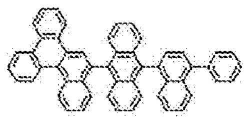
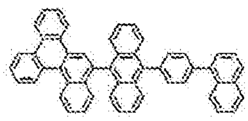
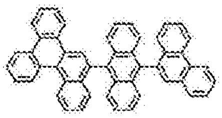
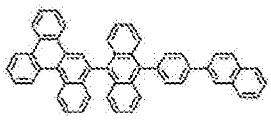
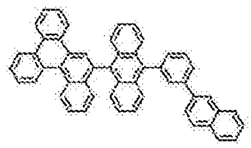
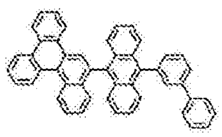
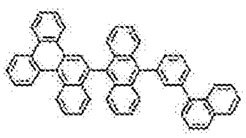
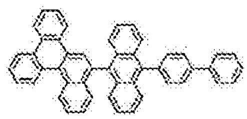
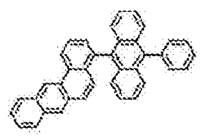
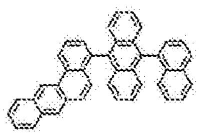
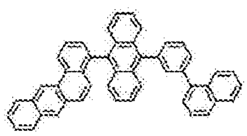
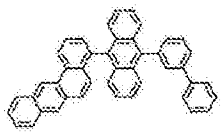
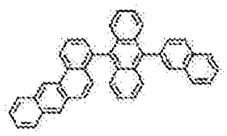

[Formula 312]

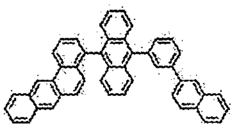 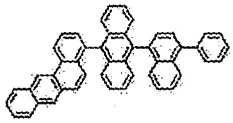 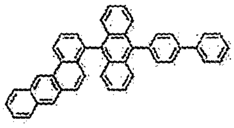 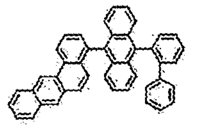

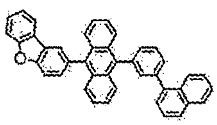 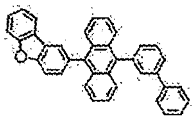 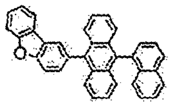 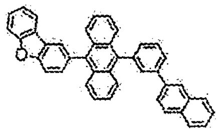

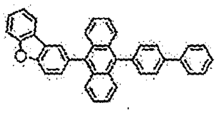 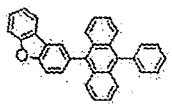 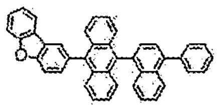 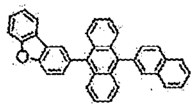

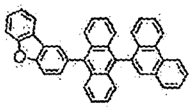 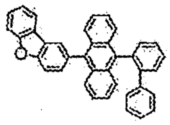 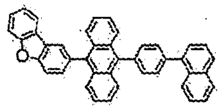 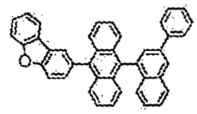

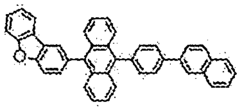 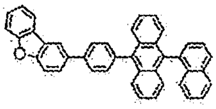 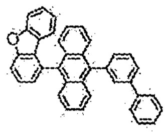 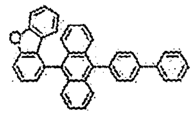

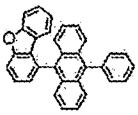 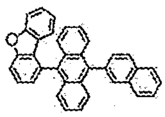 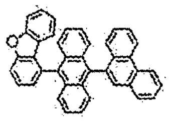 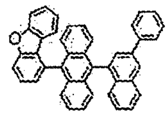

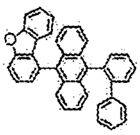 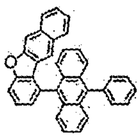 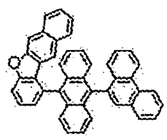 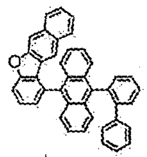

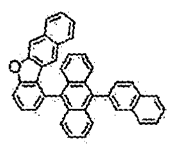 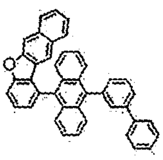 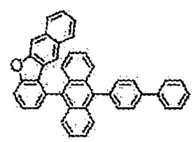 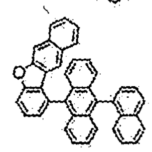

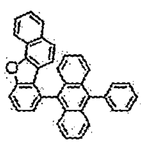 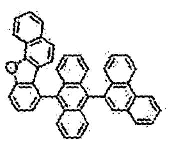 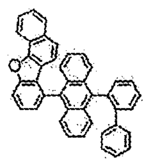 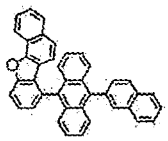

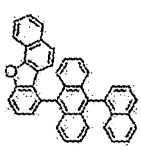 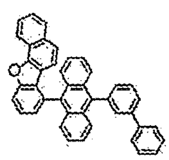 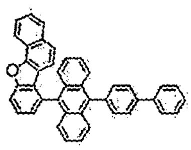 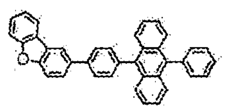

[Formula 313]

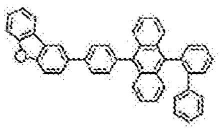 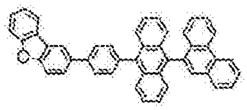 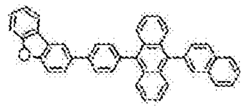 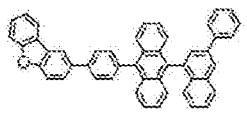

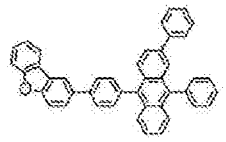 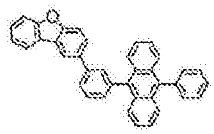 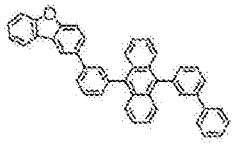 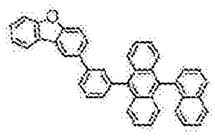

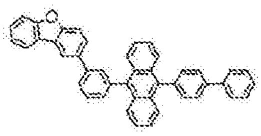 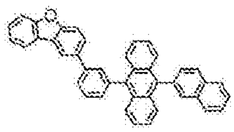 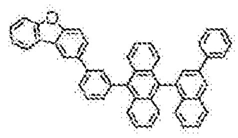 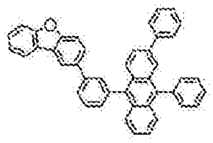

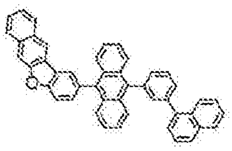 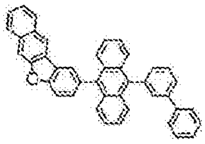 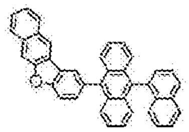 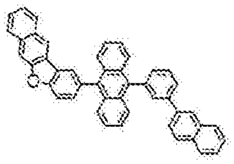

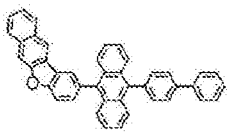 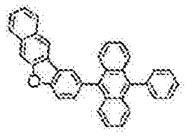 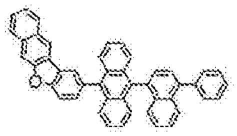 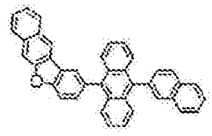

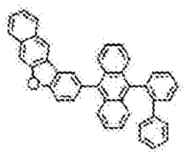 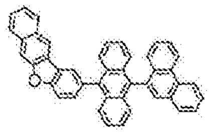 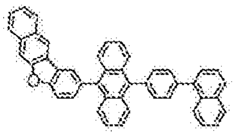 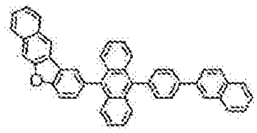

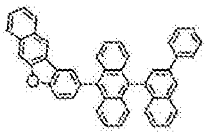 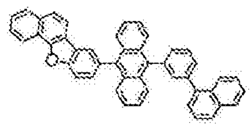 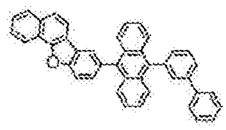 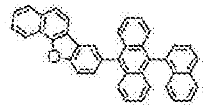

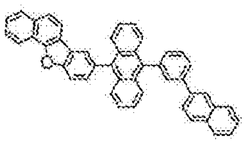 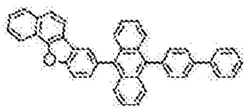 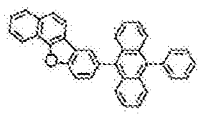 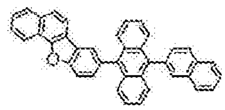

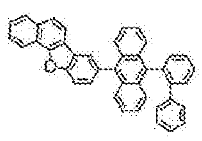 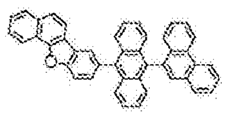 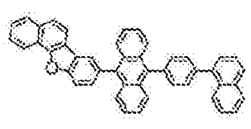 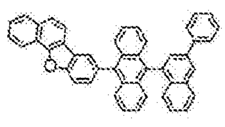

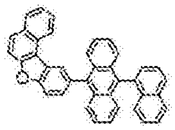 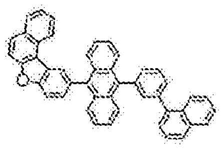 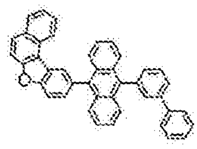 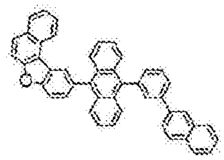

[Formula 314]

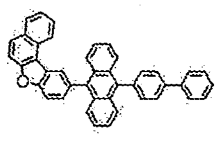 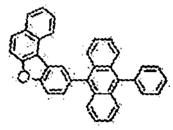 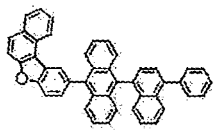 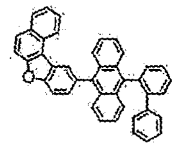

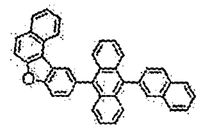 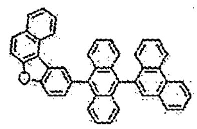 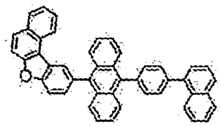 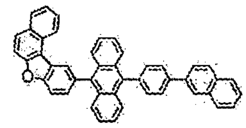

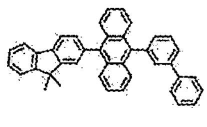 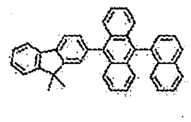 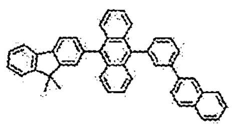 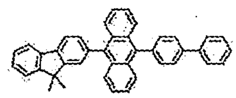

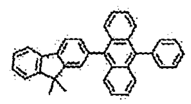 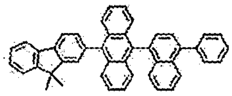 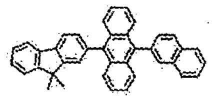 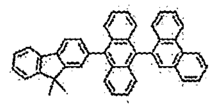

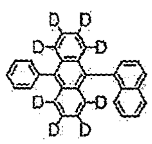 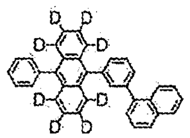 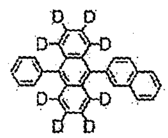 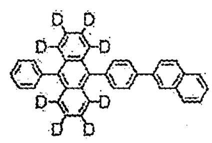

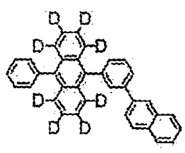 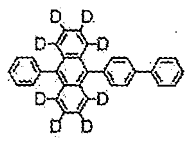 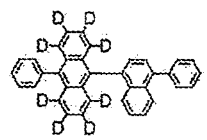 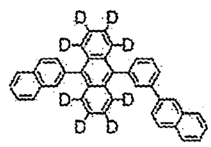

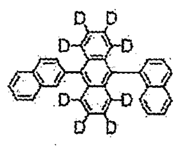 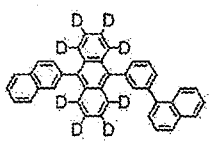 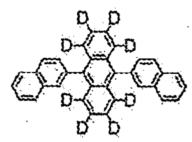 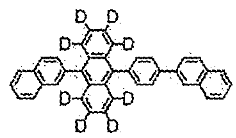

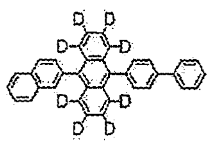 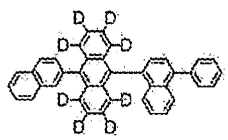 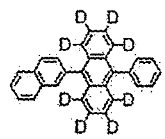 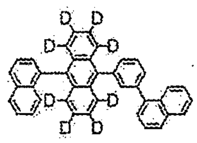

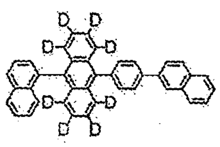 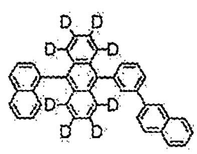 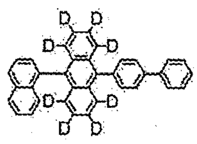 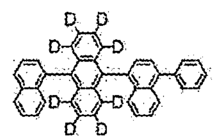

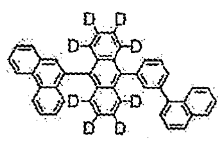 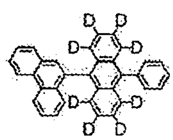 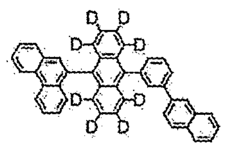 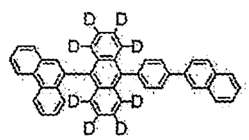

[Formula 315]

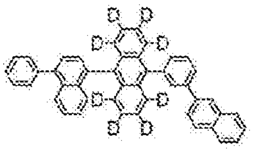 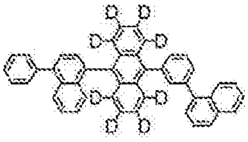 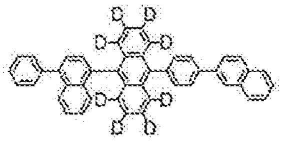 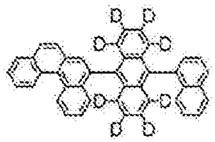

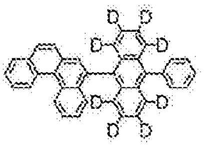 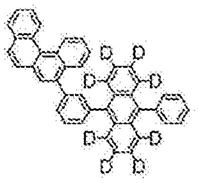 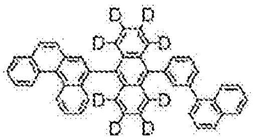 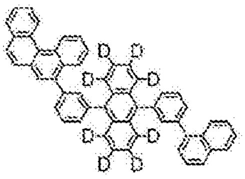

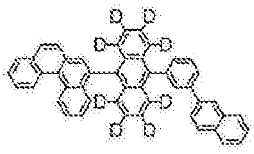 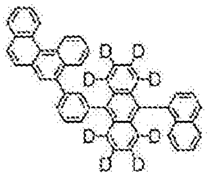 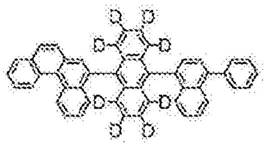 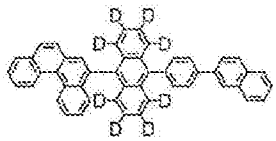

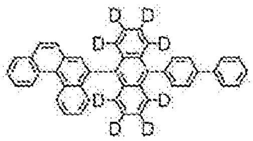 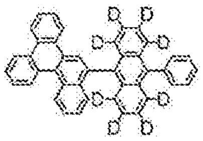 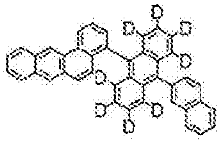 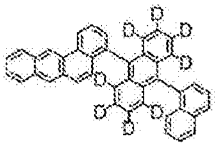

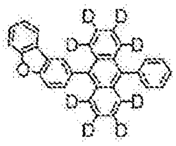 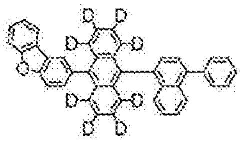 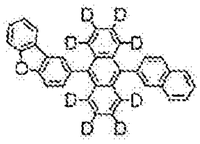 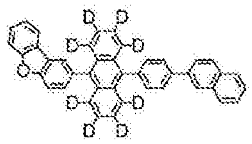

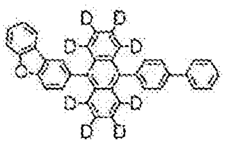 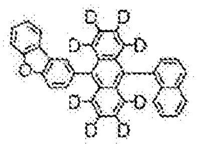 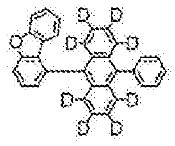 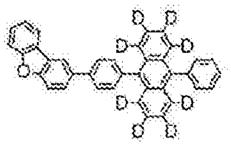

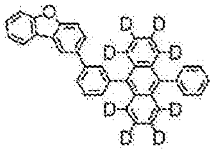 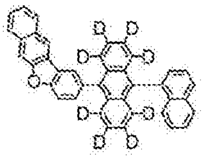 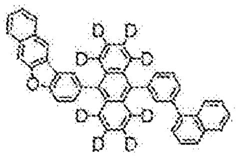 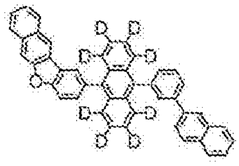

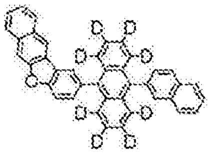 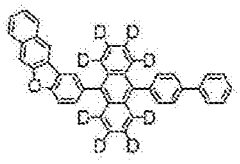 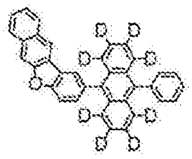 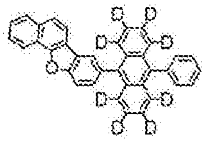

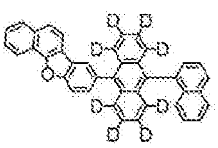 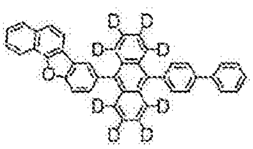 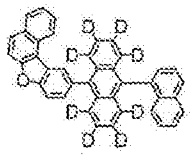 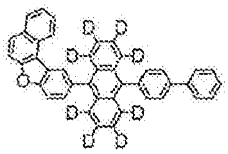

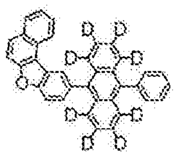 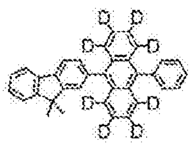 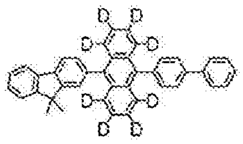 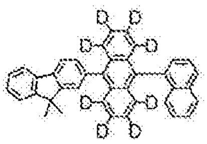

[Formula 316]

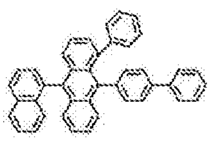
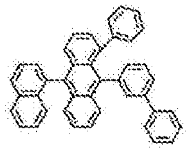
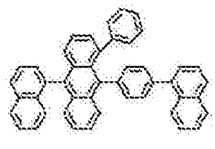
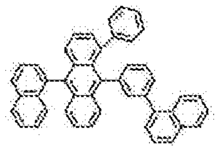

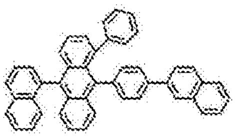
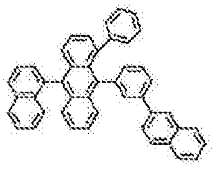
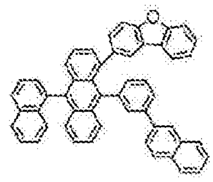
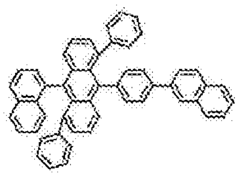

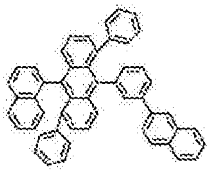
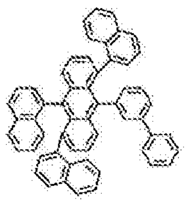
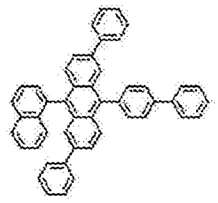
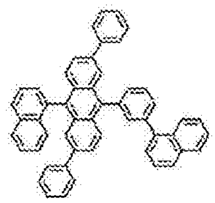

[Formula 317]

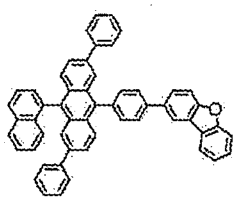
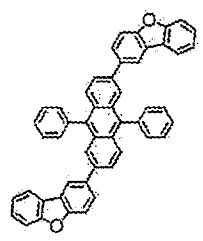
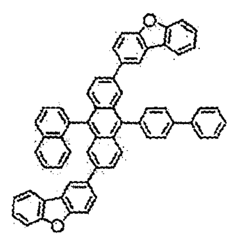
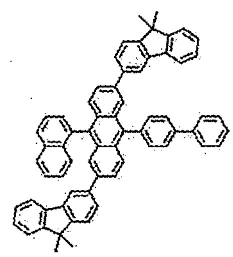

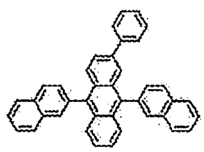
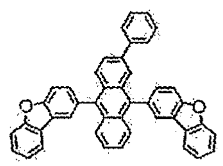
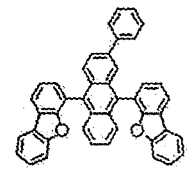
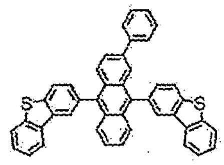

[Formula 318]

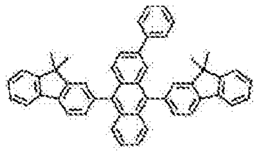 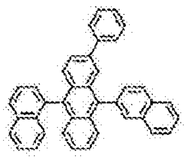 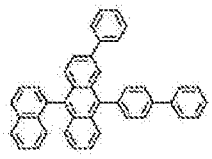 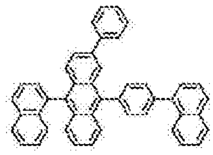

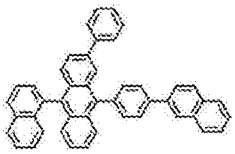 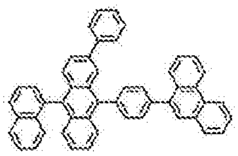 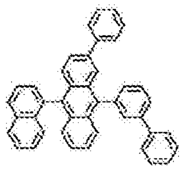 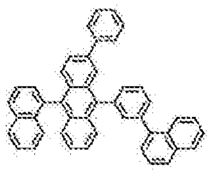

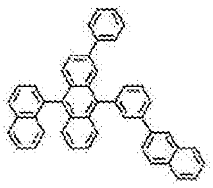 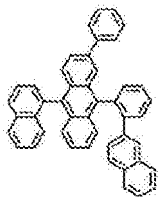 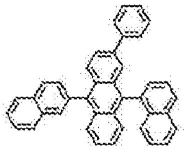 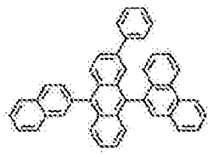

[Formula 319]

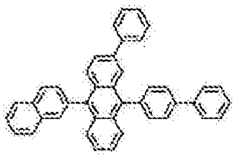 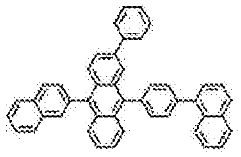 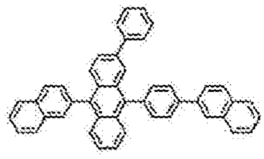 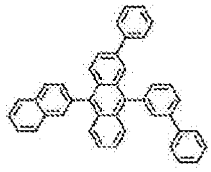

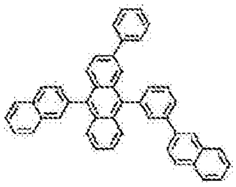 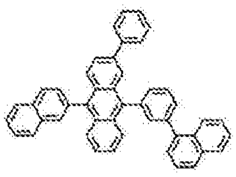 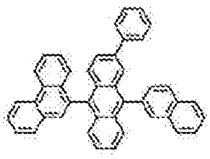 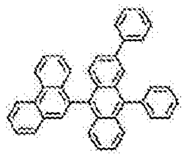

[Formula 320]

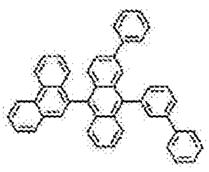 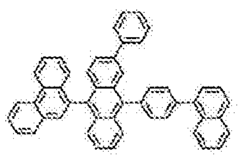 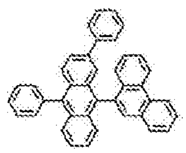 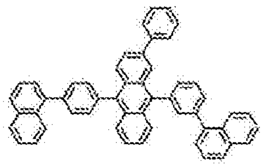

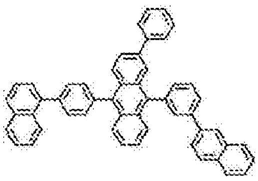 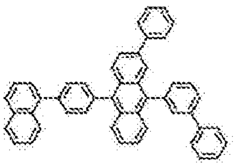 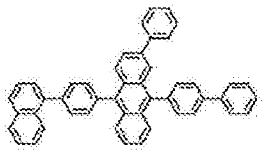 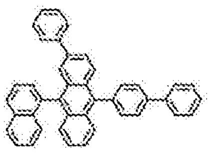

[Formula 321]

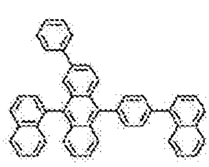
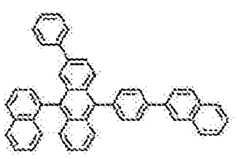
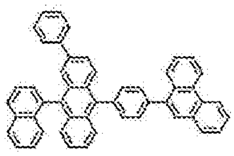
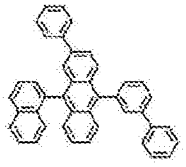
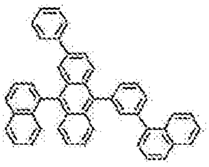
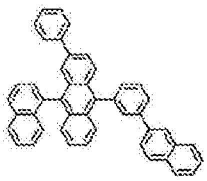
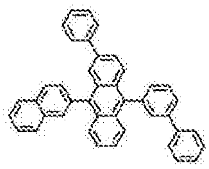
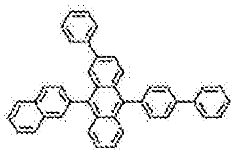

[Formula 322]

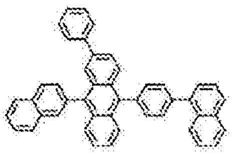
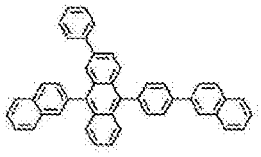
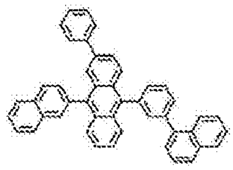
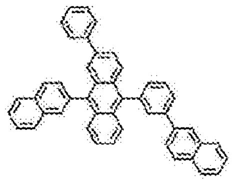
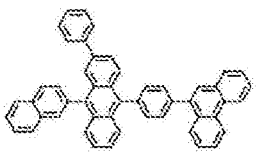
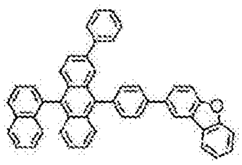
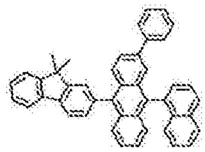
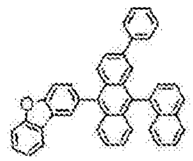

[Formula 323]

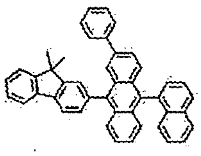
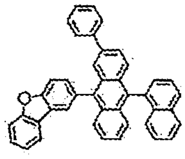
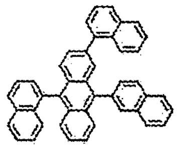
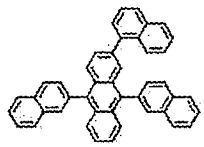
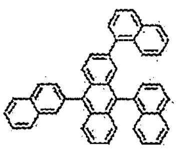
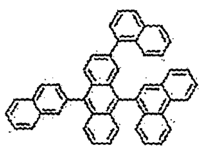
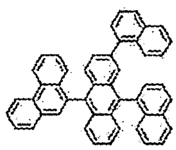
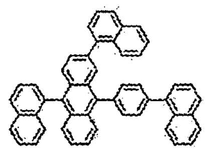

[Formula 324]

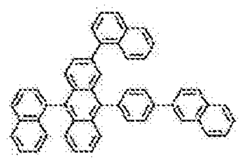 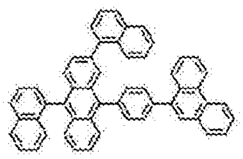 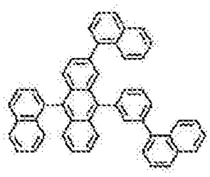 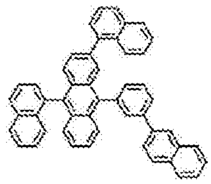

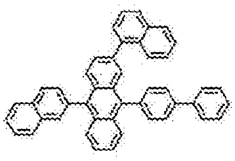 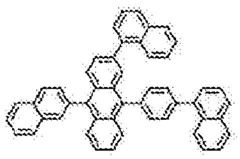 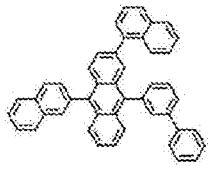 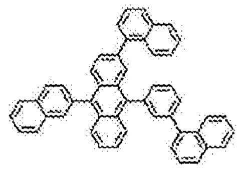

[Formula 325]

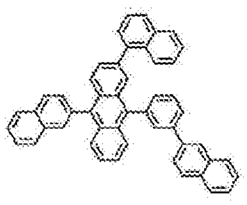 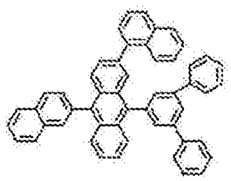 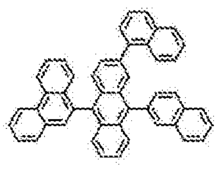 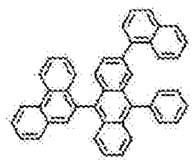

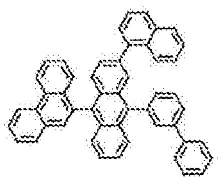 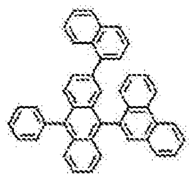 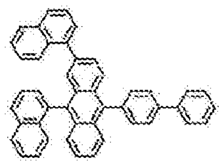 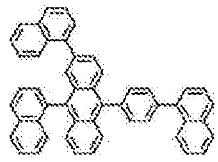

[Formula 326]

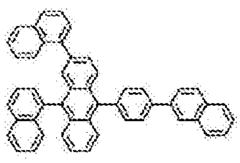 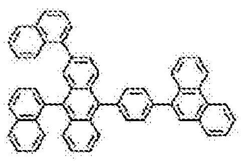 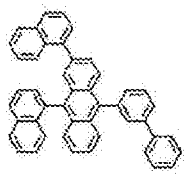 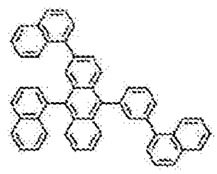

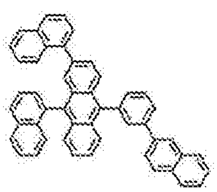 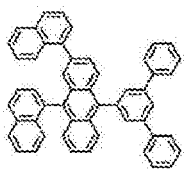 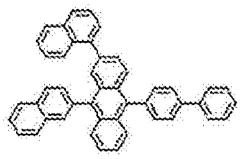 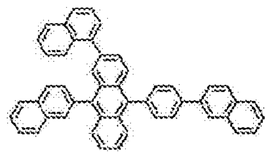

[Formula 327]

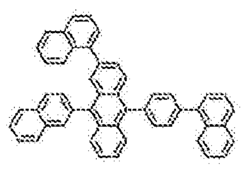
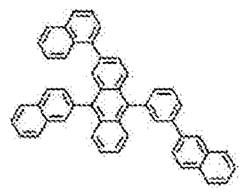
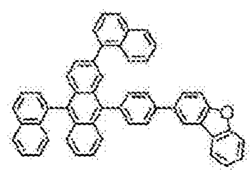
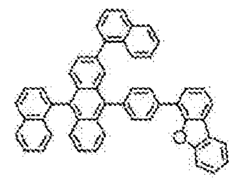
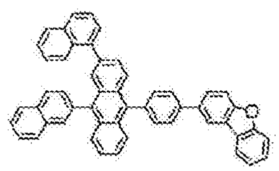
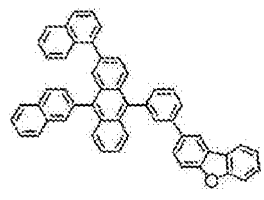
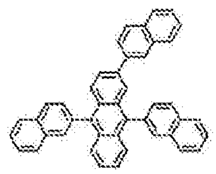
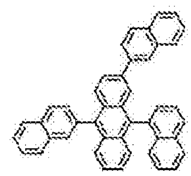

[Formula 328]

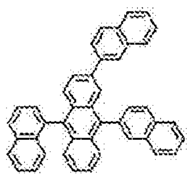
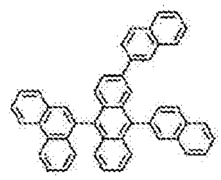
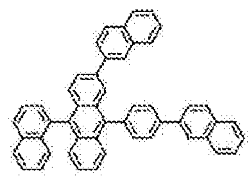
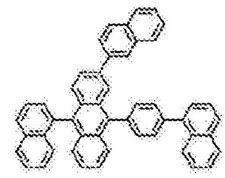
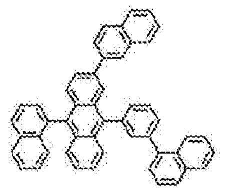
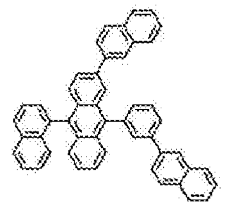
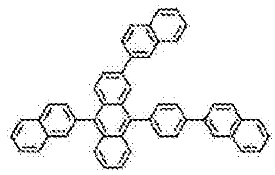
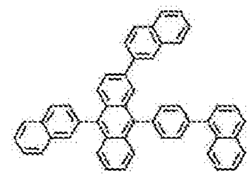

[Formula 329]

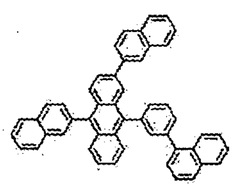
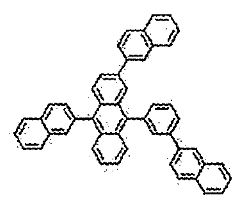
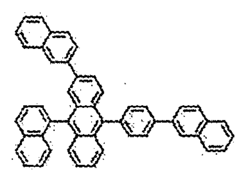
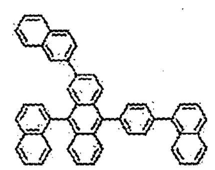
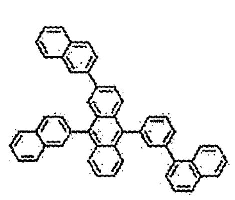
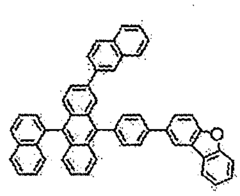
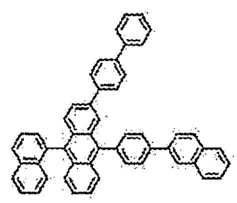
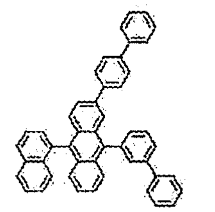

[Formula 330]

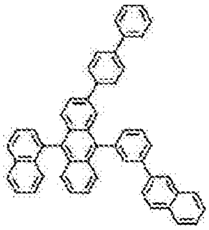 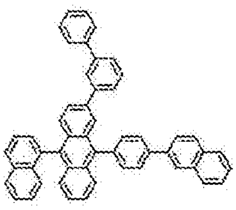 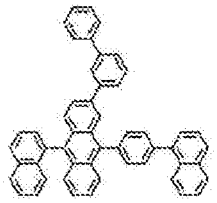 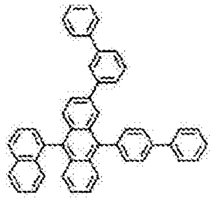

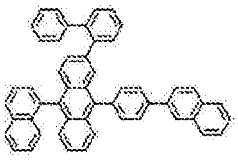 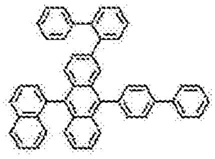 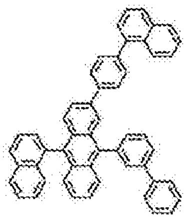 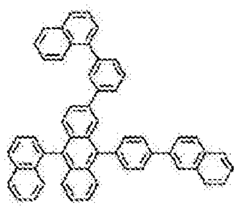

[Formula 331]

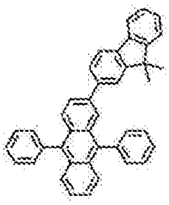 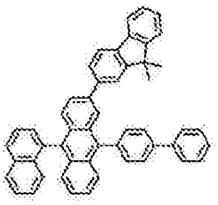 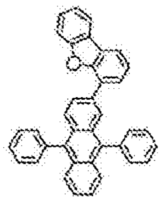 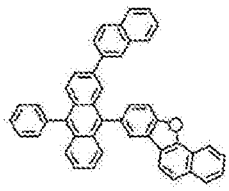

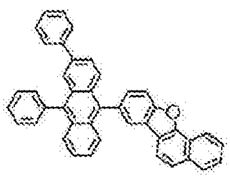 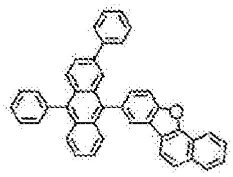 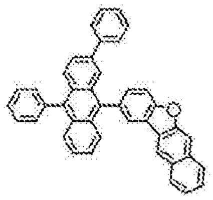 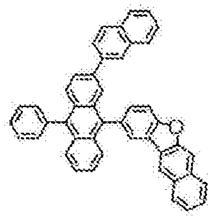

[Formula 332]

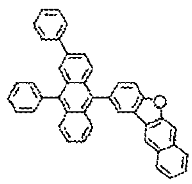 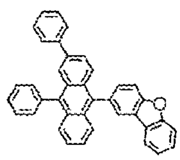 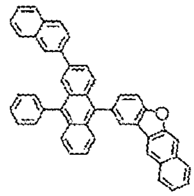 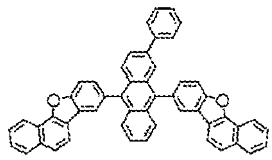

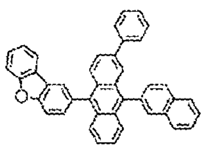 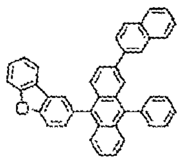 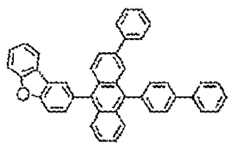 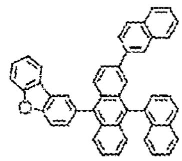

[Formula 333]

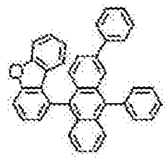
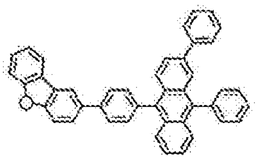
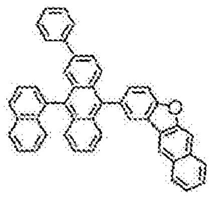
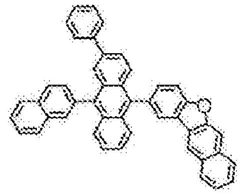

[Formula 334]

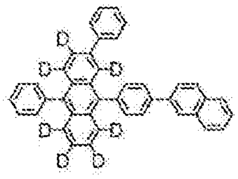
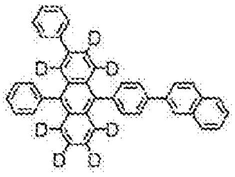
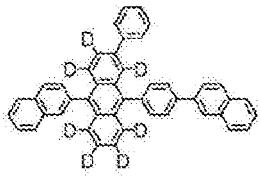
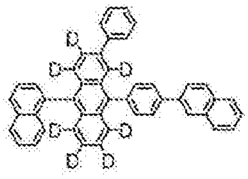
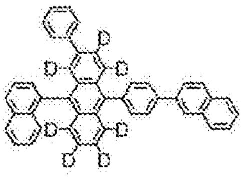
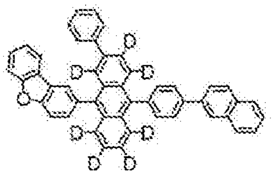
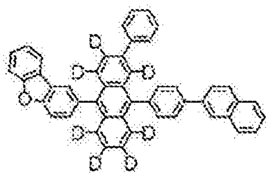
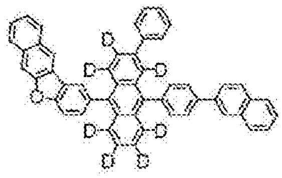

[Formula 335]

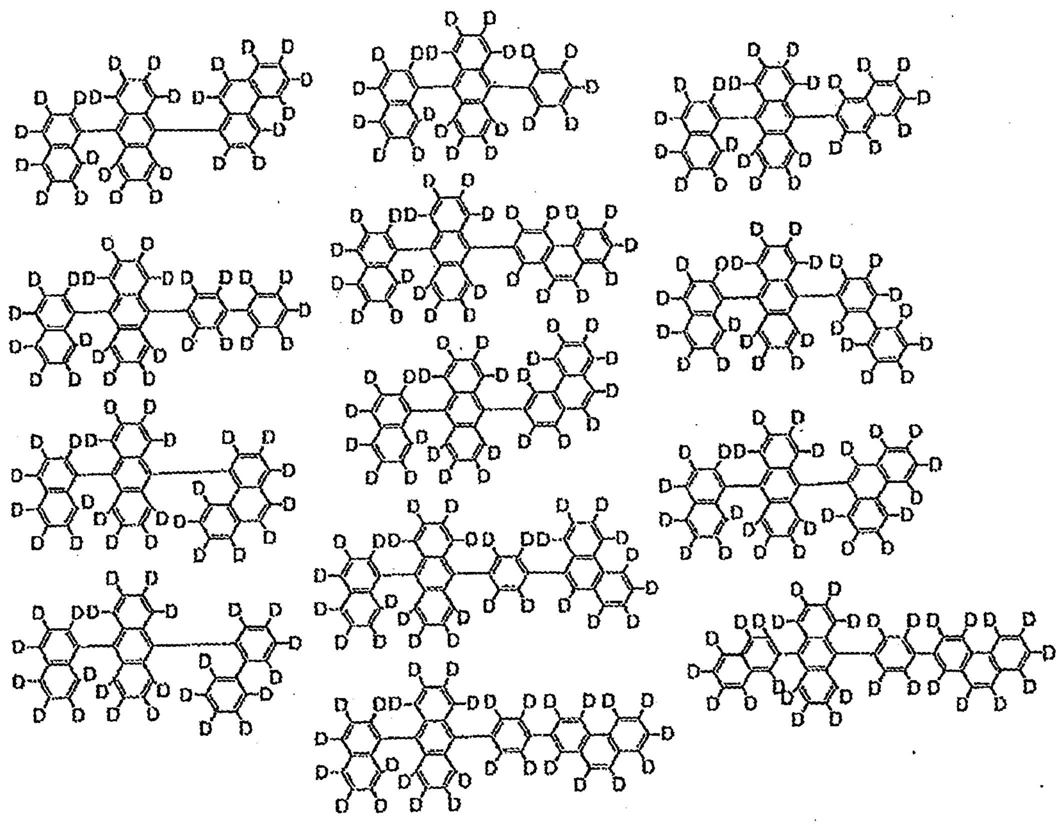

[Formula 336]

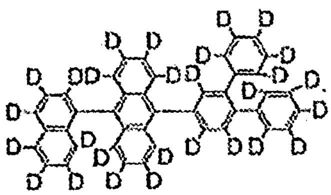
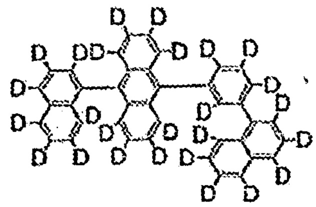
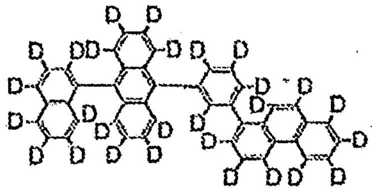
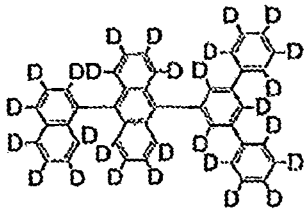
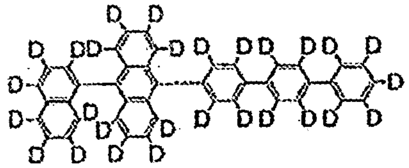
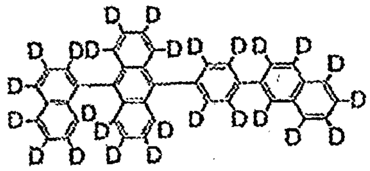
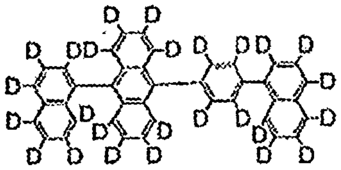
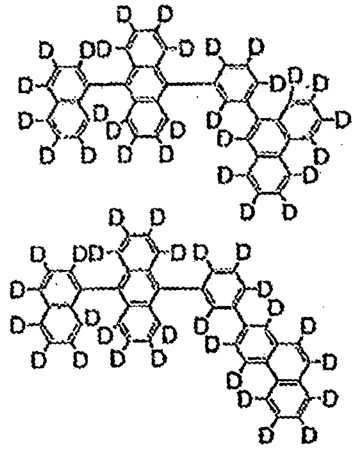
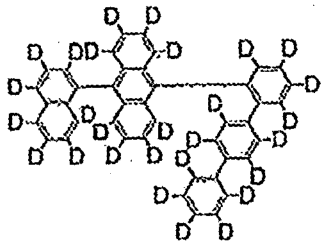
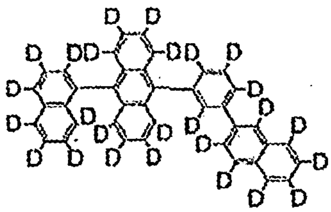
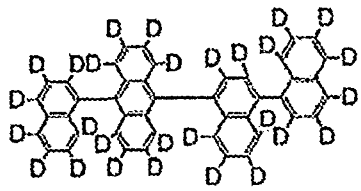
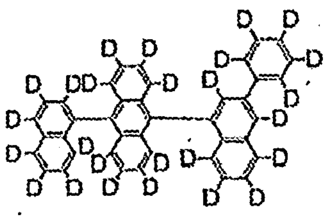
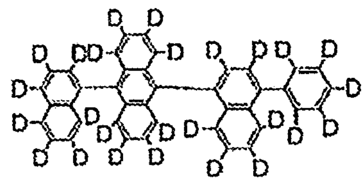
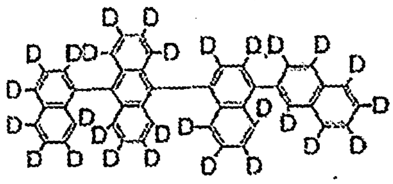
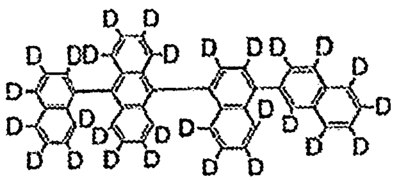

[Formula 337]

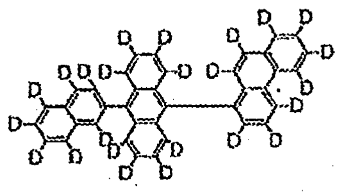
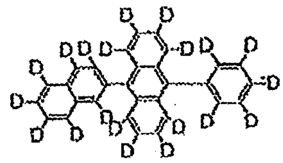
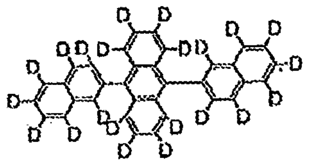
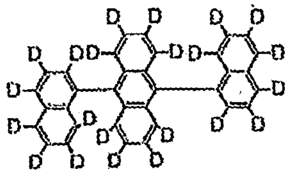
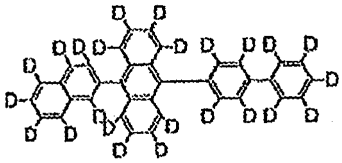
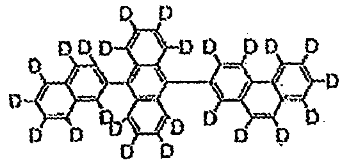
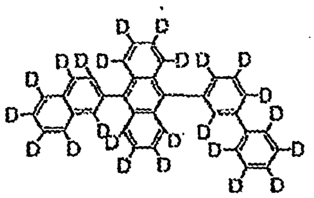
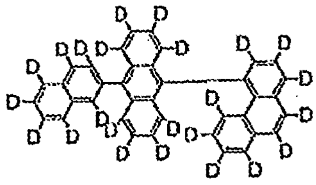
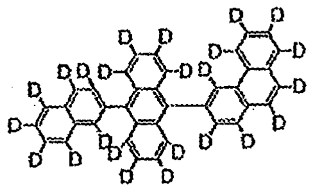
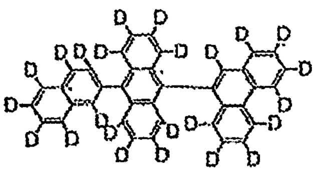
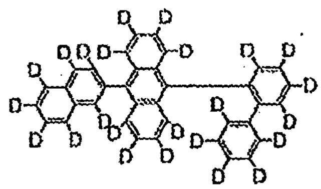
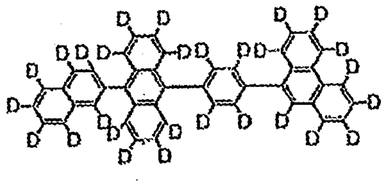

[Formula 338]

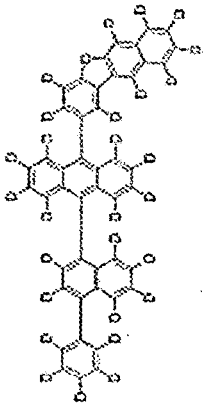
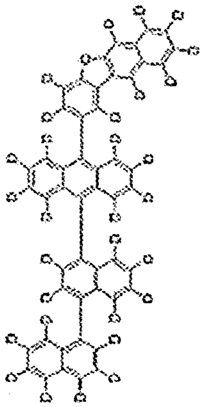
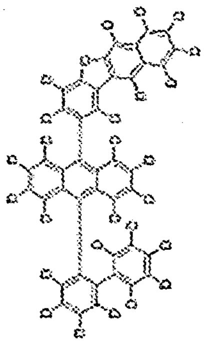
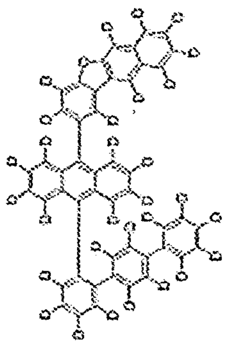
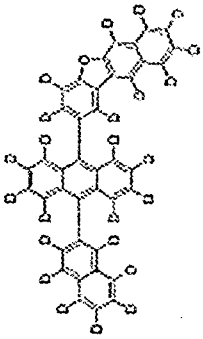
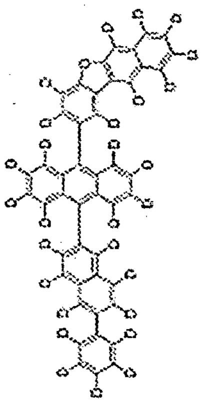
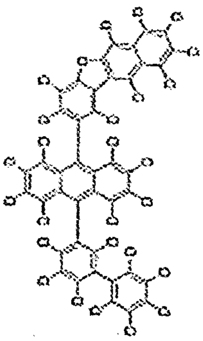
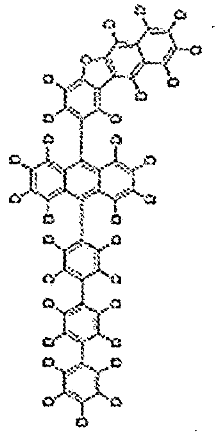
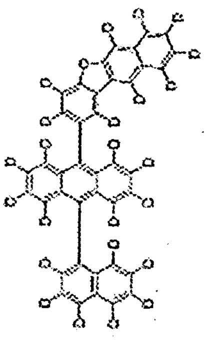
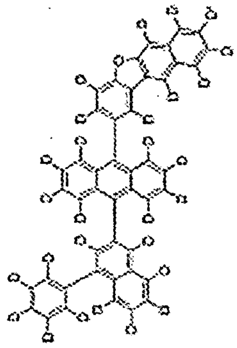
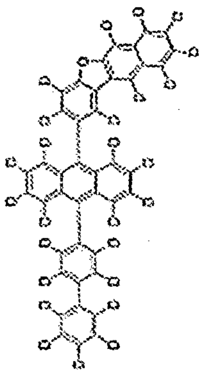
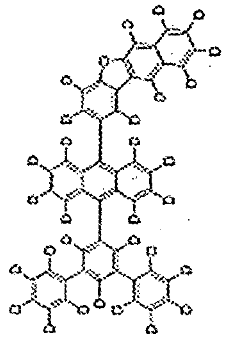
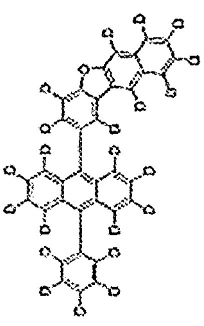
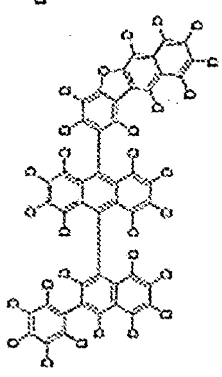
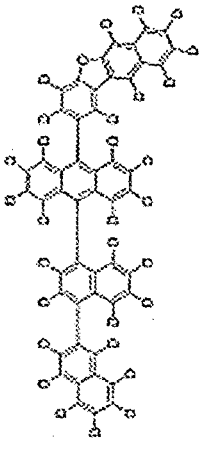
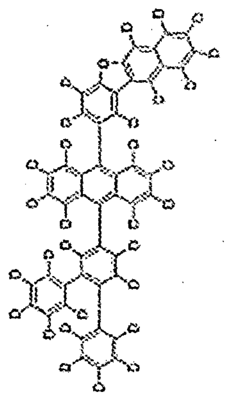

[Formula 339]

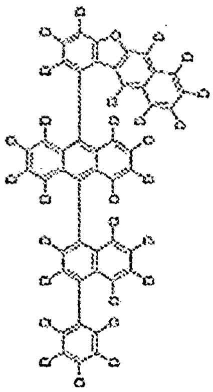
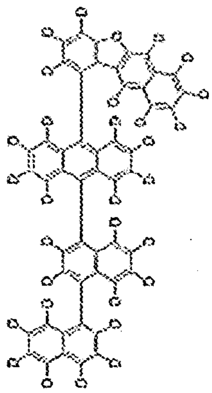
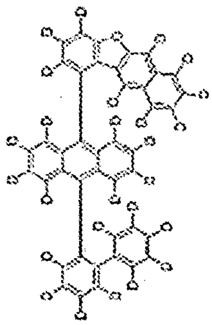
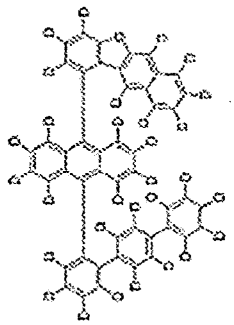
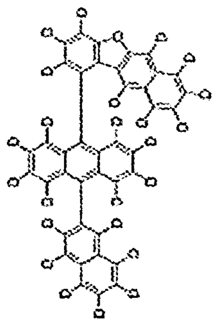
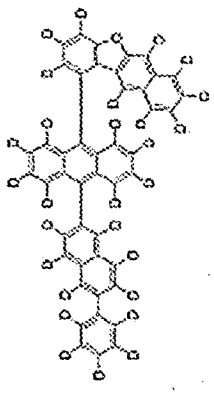
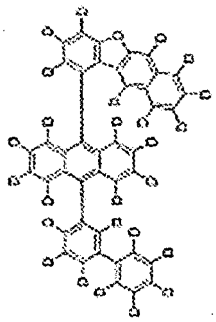
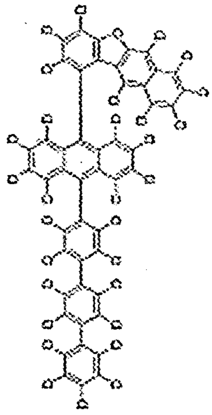
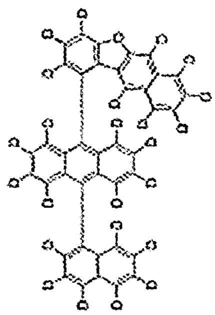
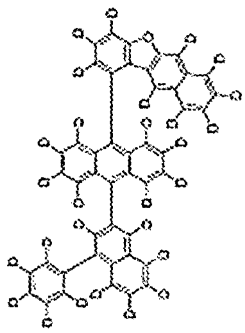
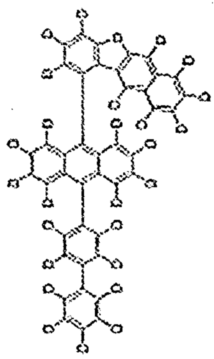
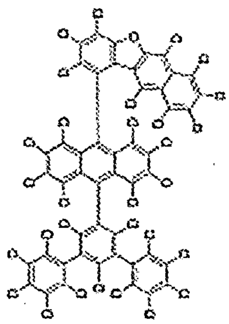
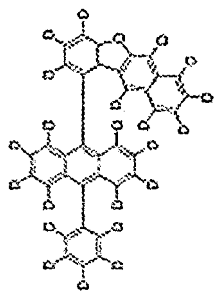
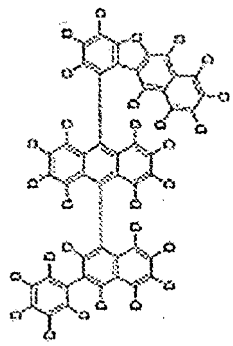
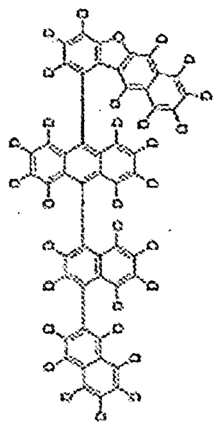
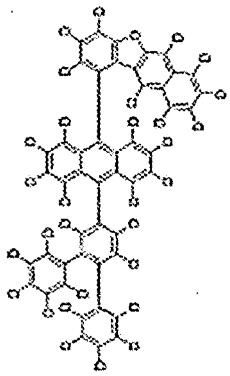

[Formula 340]

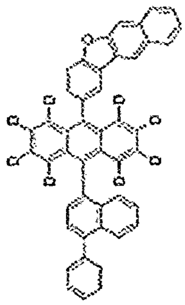 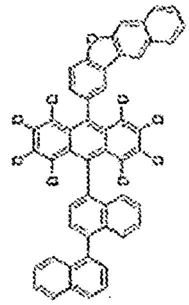 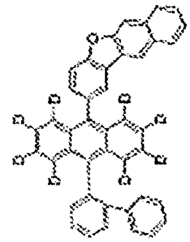 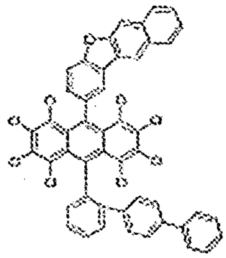

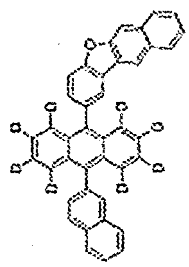 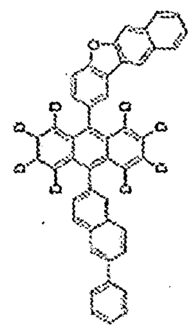 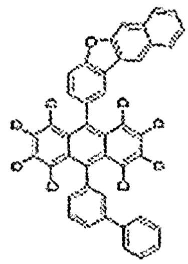 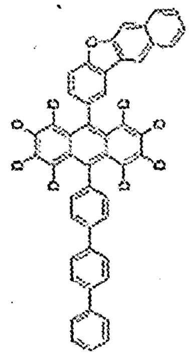

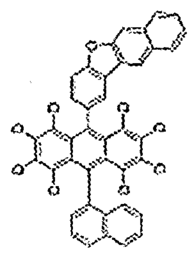 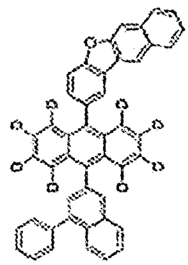 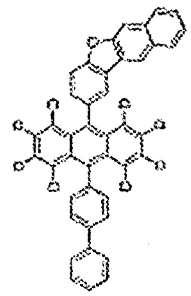 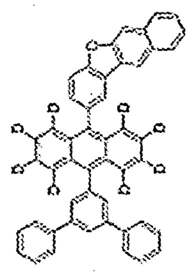

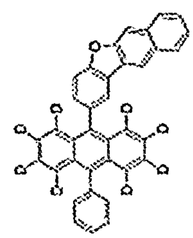 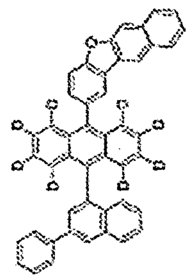 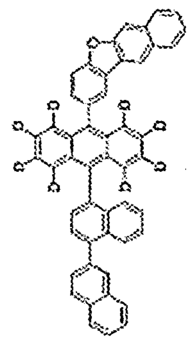 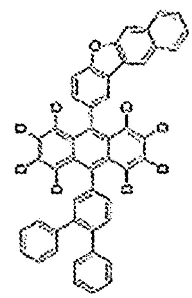

[Formula 341]

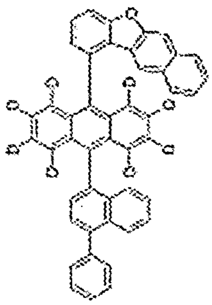
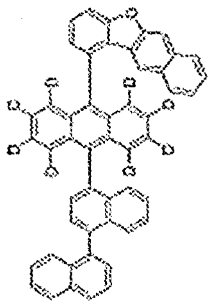
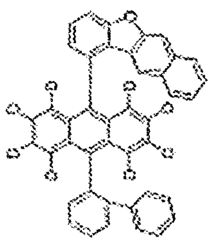
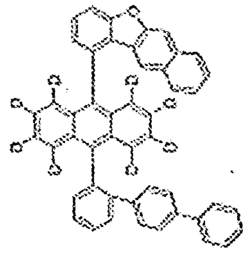

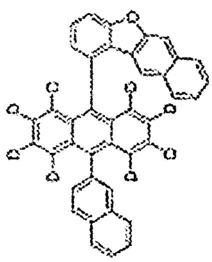
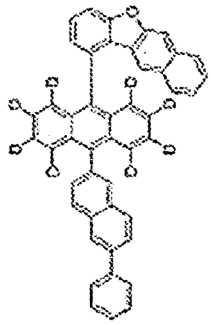
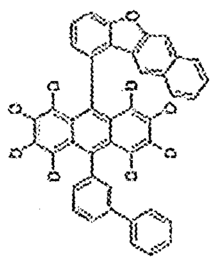
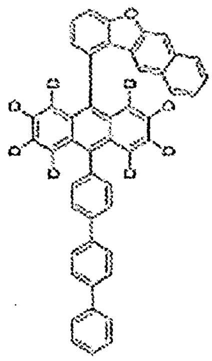

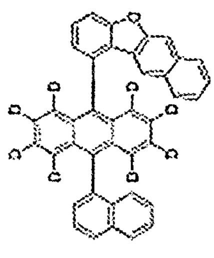
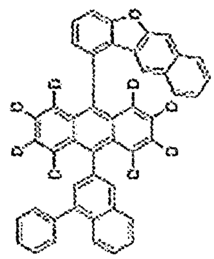
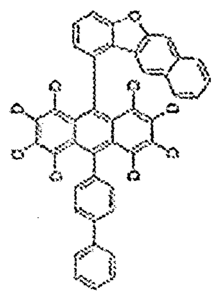
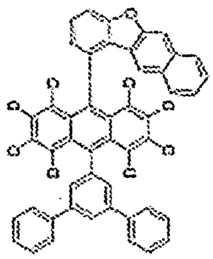

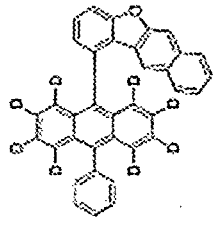
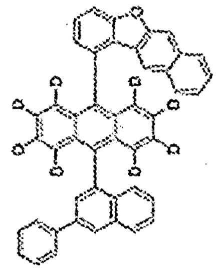
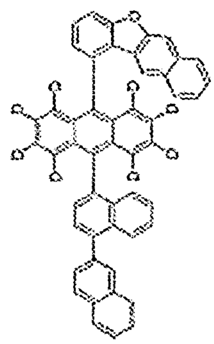
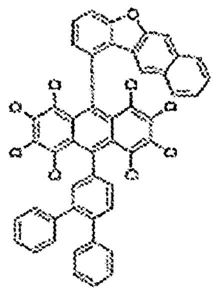

[Formula 342]

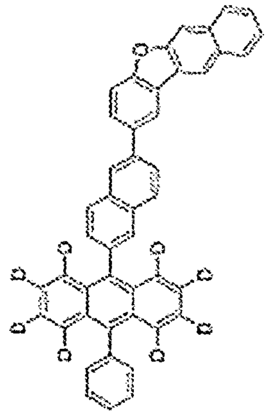
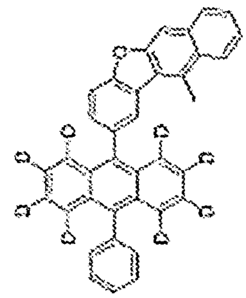
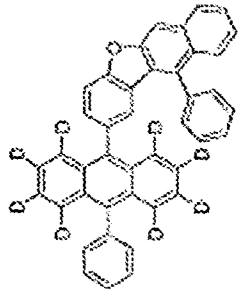
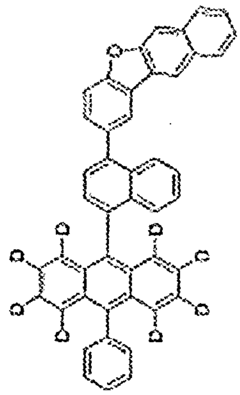
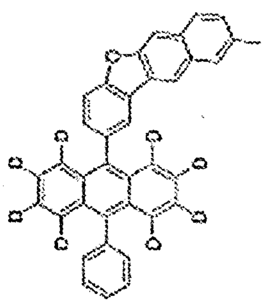
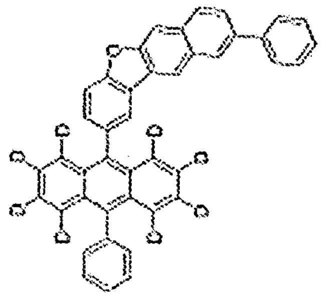
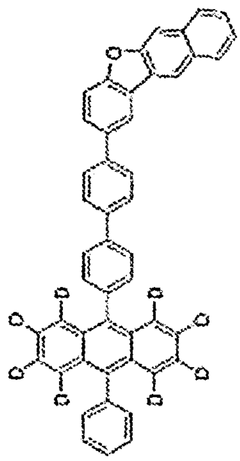
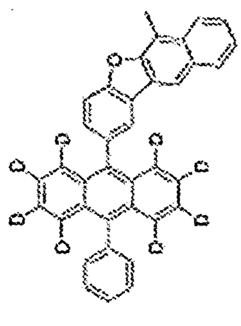
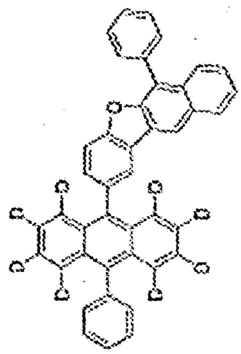
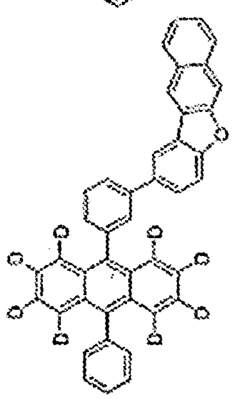
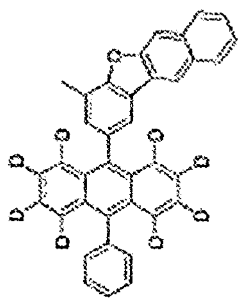
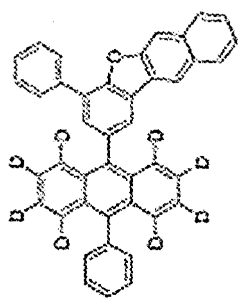

[Formula 343]

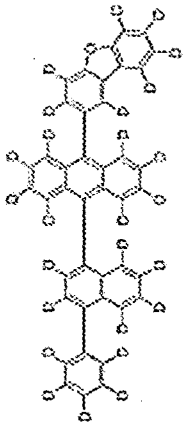
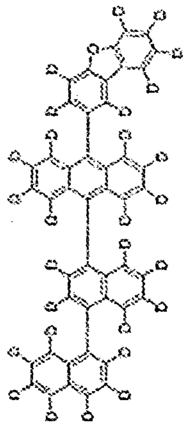
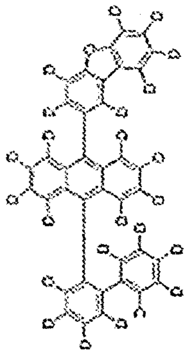
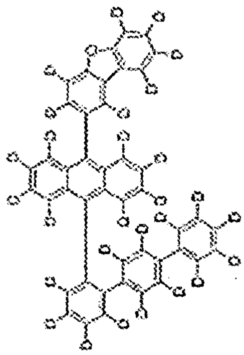
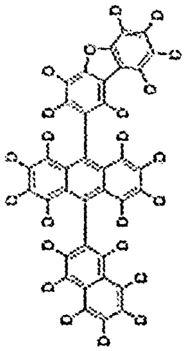
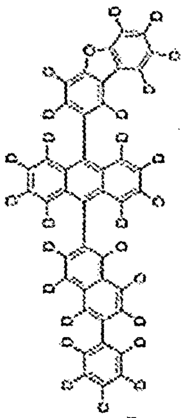
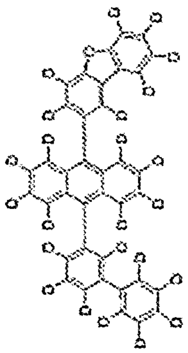
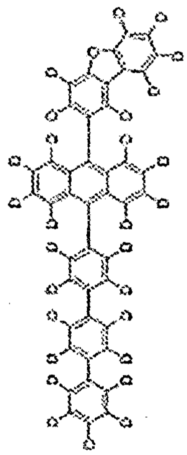
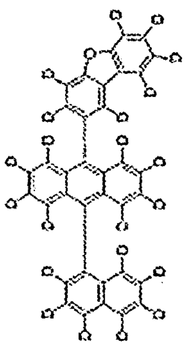
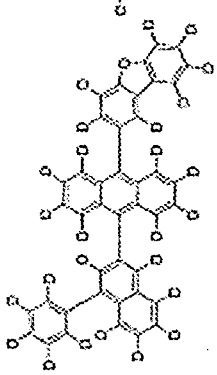
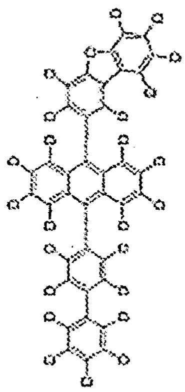
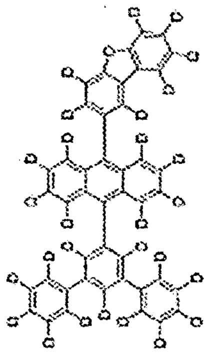
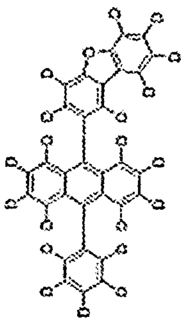
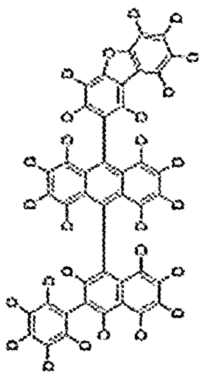
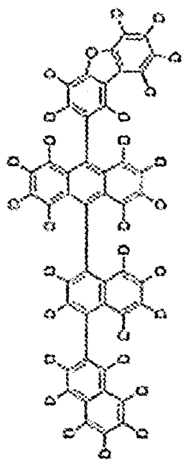
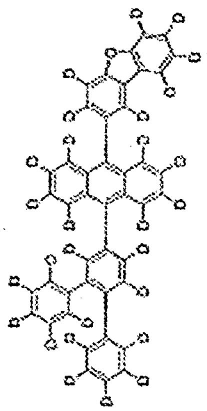

[Formula 344]

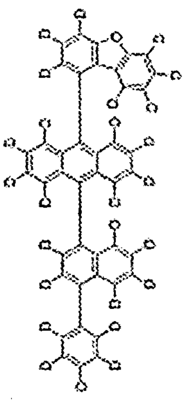
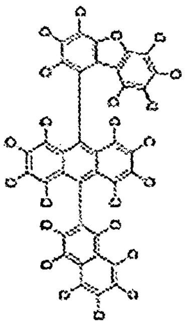
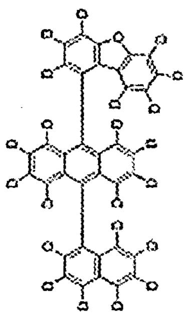
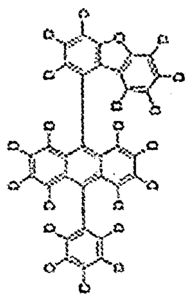
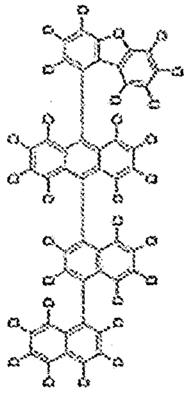
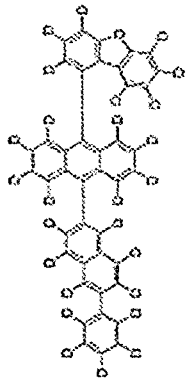
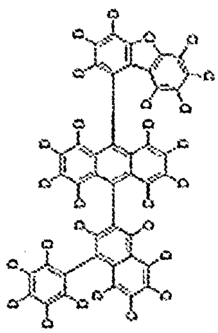
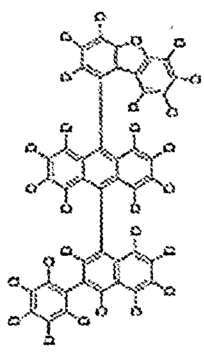
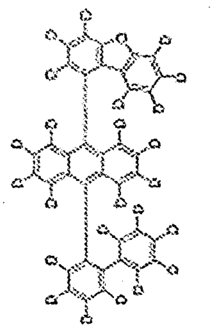
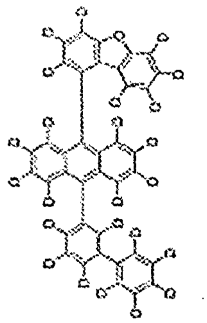
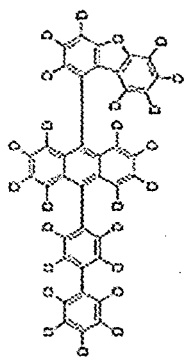
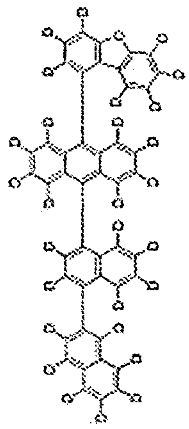
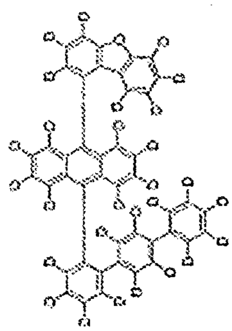
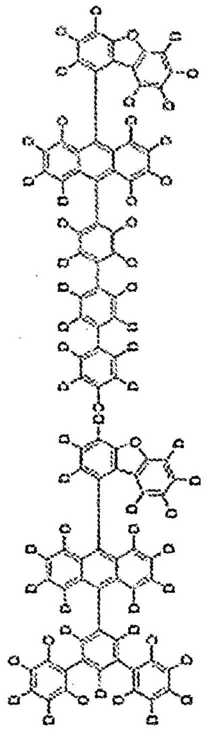
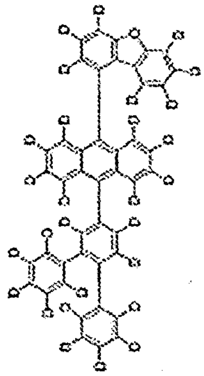

[Formula 345]

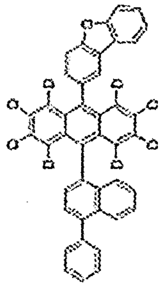 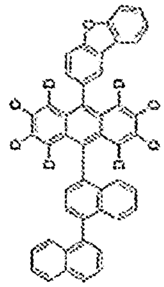 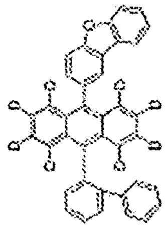 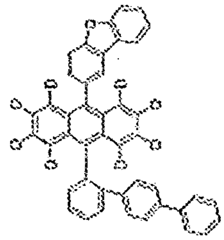

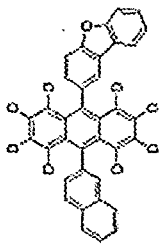 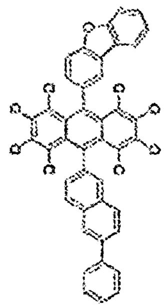 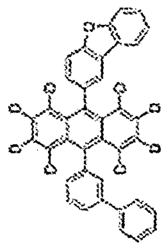 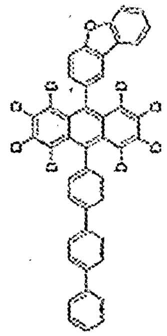

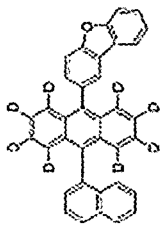 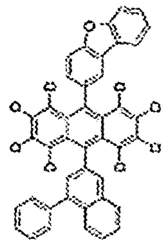 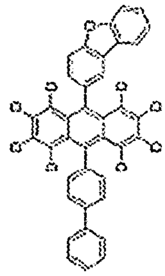 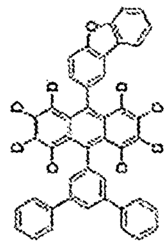

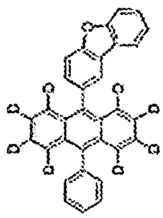 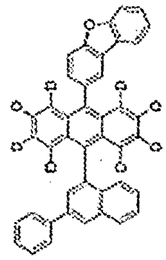 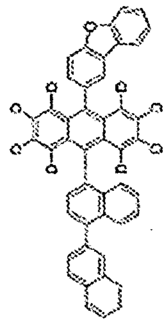 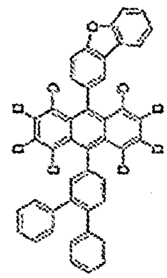

[Formula 346]

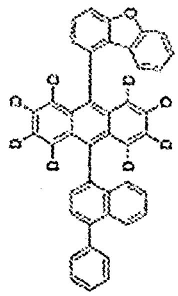
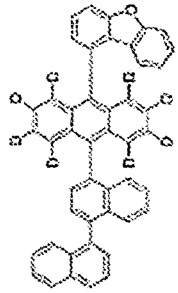
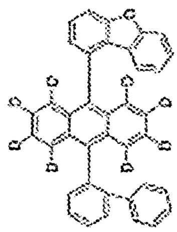
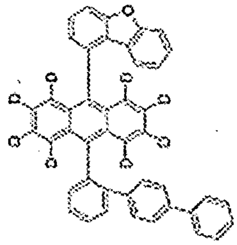

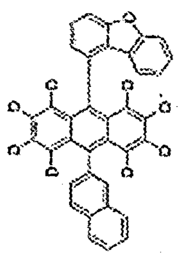
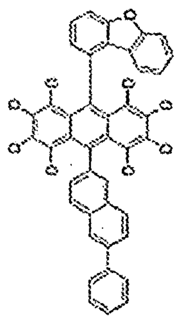
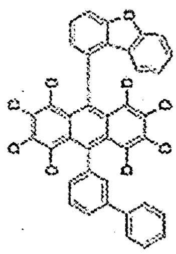
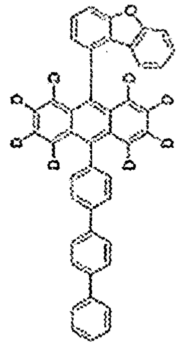

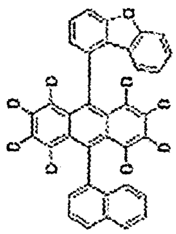
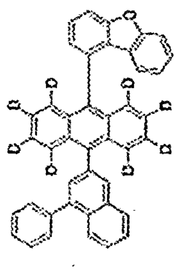
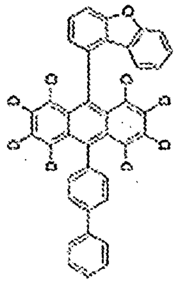
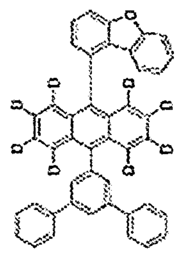

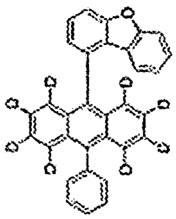
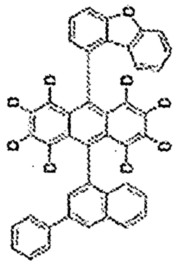
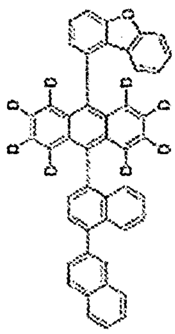
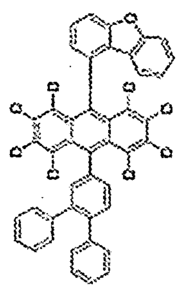

[Formula 347]

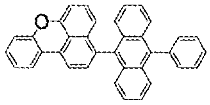
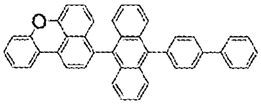
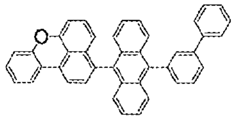
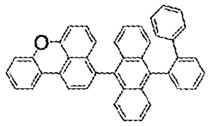
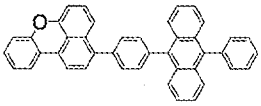
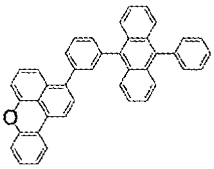
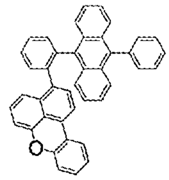
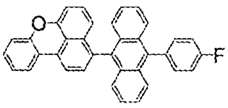
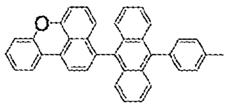
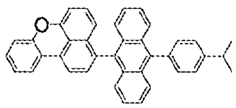
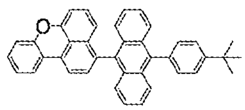
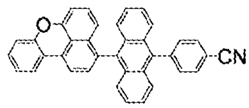
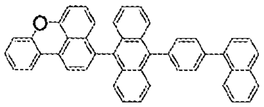
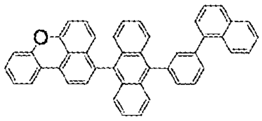
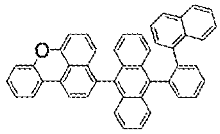
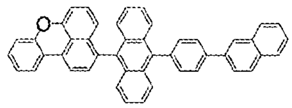
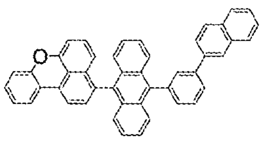
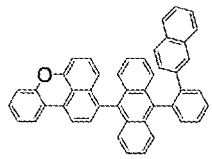
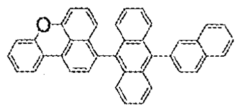
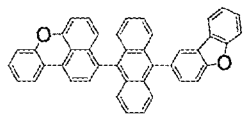
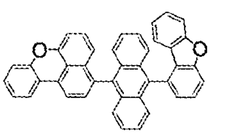
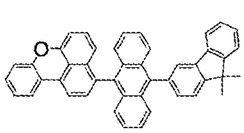
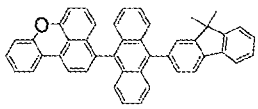
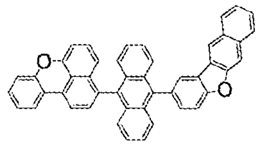
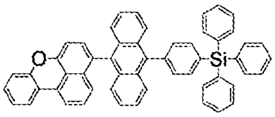
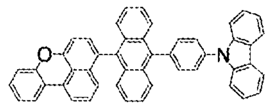
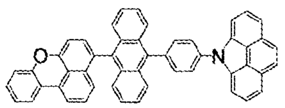
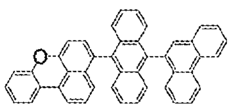
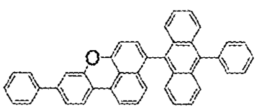
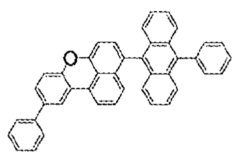
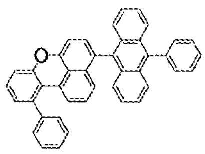
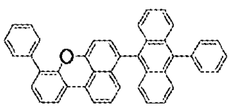
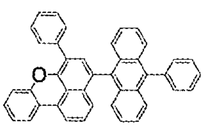
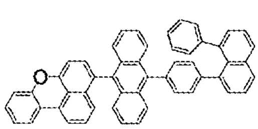
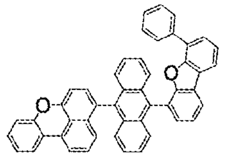
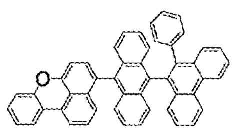

[Formula 348]

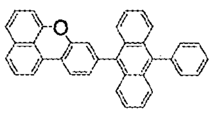
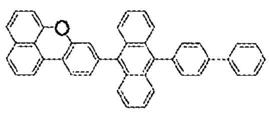
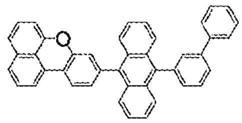
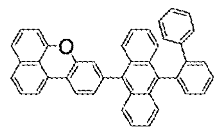
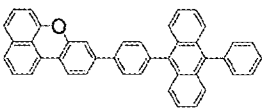
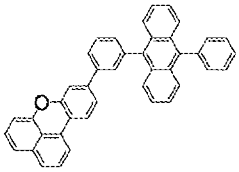
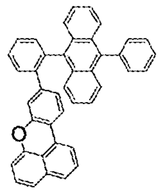
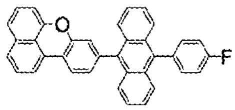
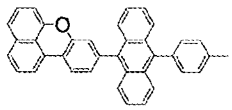
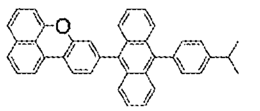
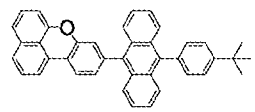
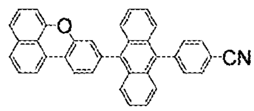
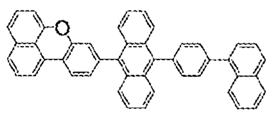
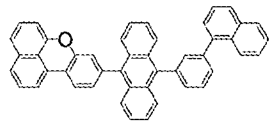
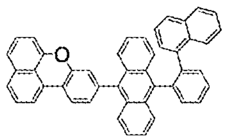
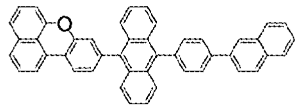
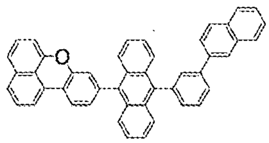
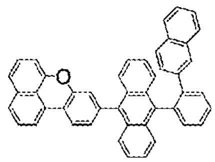
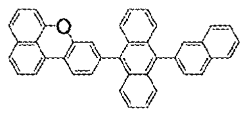
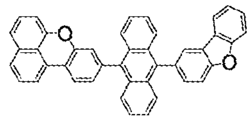
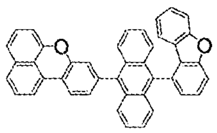
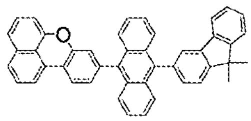
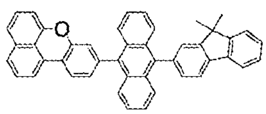
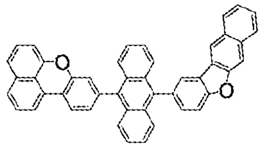

[Formula 349]

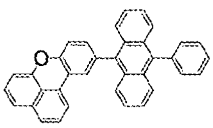 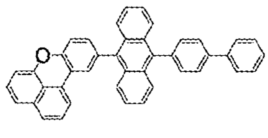 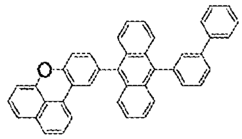 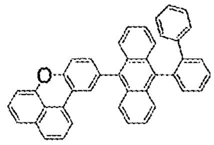

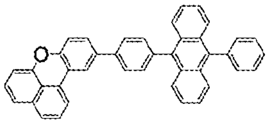 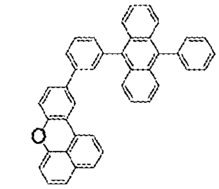 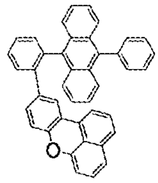 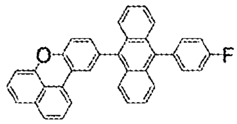

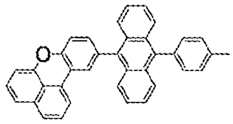 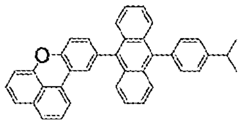 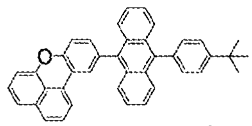 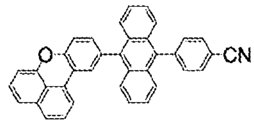

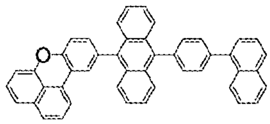 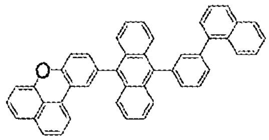 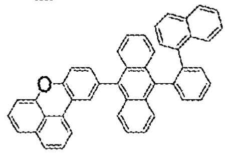 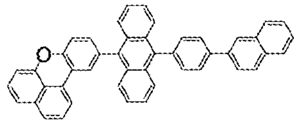

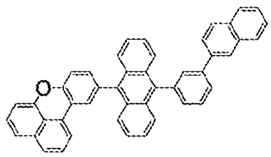 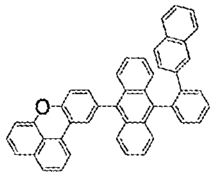 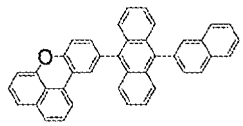 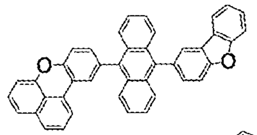

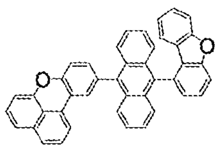 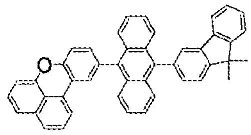 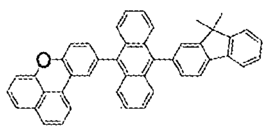 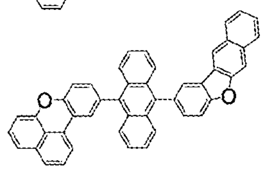

[Formula 350]

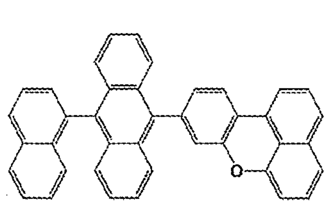
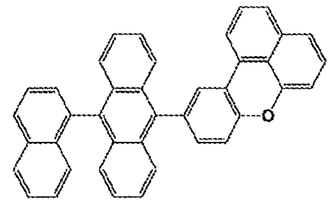
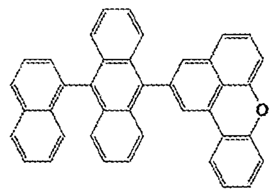
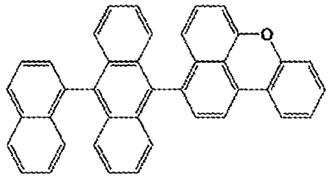
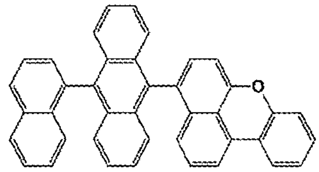
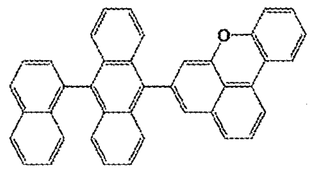
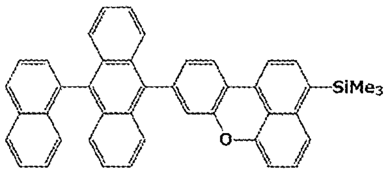
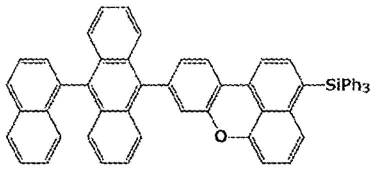
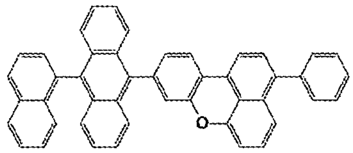
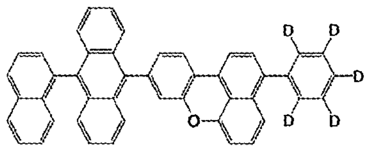
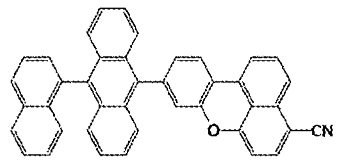
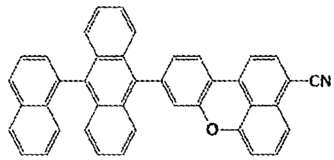
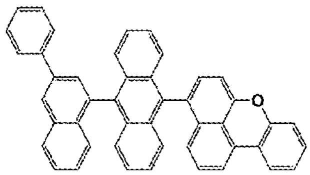
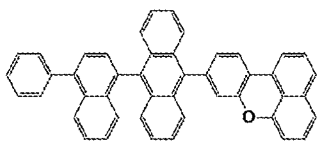
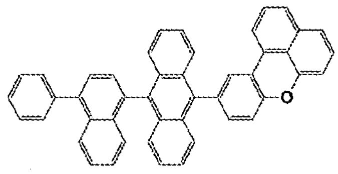
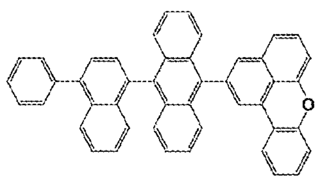
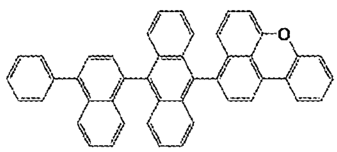
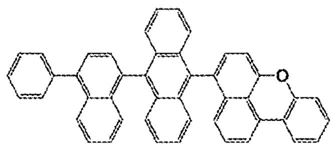
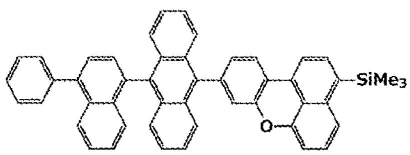
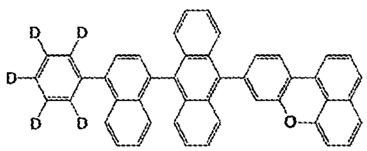
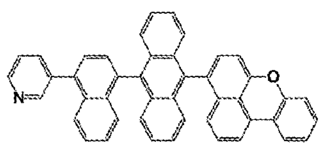
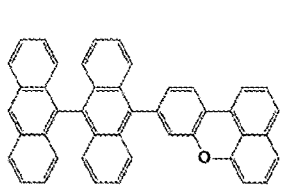
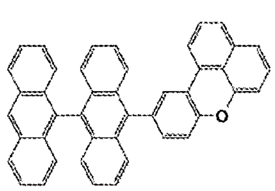
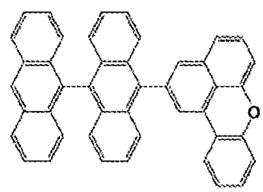

[Formula 351]

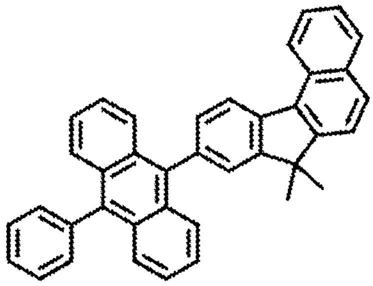
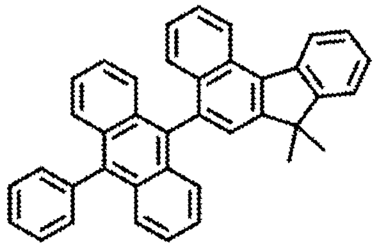
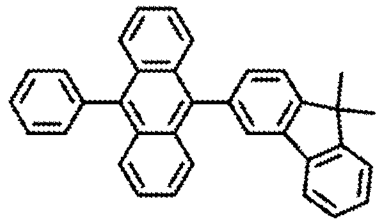
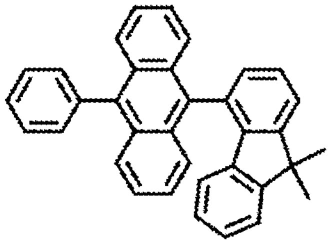
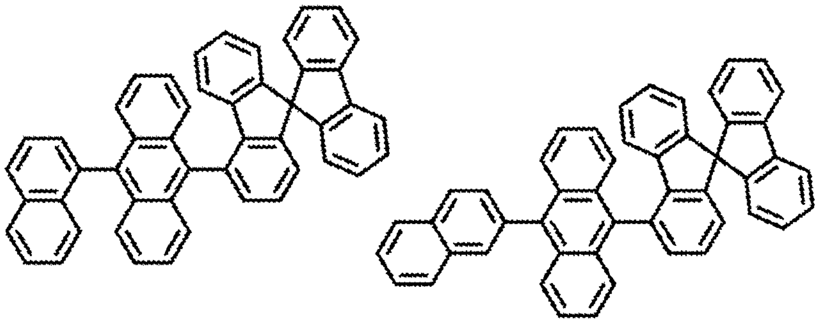
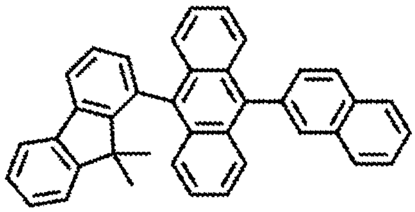

[Formula 352]

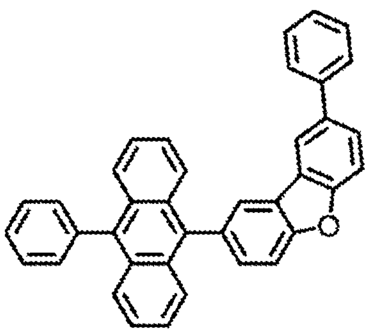
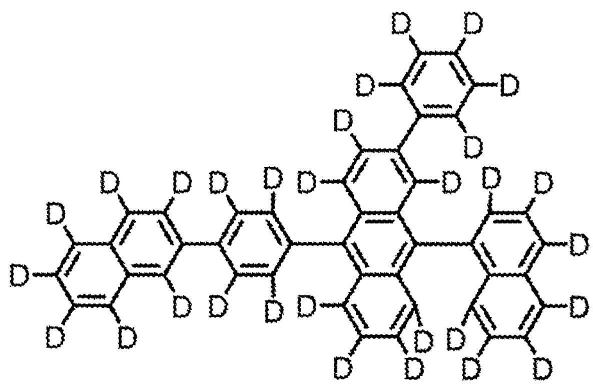

Third Compound and Fourth Compound

The third compound and the fourth compound are each independently at least one compound selected from the group consisting of a compound represented by a formula (3) below, a compound represented by a formula (4) below, a compound represented by a formula (5) below, a compound represented by a formula (6) below, a compound represented by a formula (7) below, a compound represented by a formula (8) below, a compound represented by a formula (9) below, and a compound represented by a formula (10) below.

Compound Represented by Formula (3)

The compound represented by the formula (3) will be described.

[Formula 353]

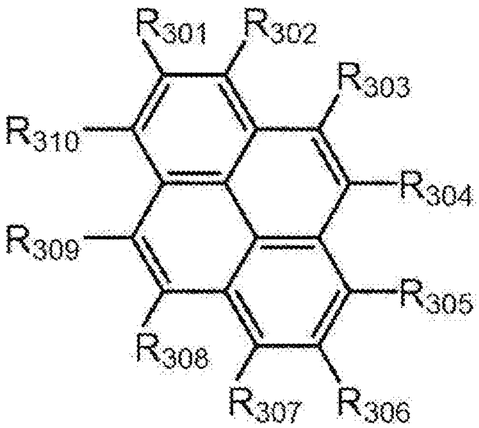

(3)

In the formula (3):

at least one combination of adjacent two or more of $R_{301}$ to $R_{310}$ are mutually bonded to form a substituted or unsubstituted monocyclic ring, mutually bonded to form a substituted or unsubstituted fused ring, or not mutually bonded;

at least one of $R_{301}$ to $R_{310}$ is a monovalent group represented by a formula (31) below; and

$R_{301}$ to $R_{310}$ forming neither the substituted or unsubstituted monocyclic ring nor the substituted or unsubstituted fused ring and not being a monovalent group represented by the formula (31) below are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

[Formula 354]

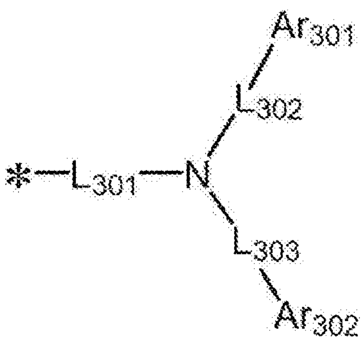

(31)

In the formula (31):

$Ar_{301}$ and $Ar_{302}$ are each independently a substituted or unsubstituted aryl group having 6 to 50 ring carbon atoms, or a substituted or unsubstituted heterocyclic group having 5 to 50 ring atoms;

$L_{301}$ to $L_{303}$ are each independently a single bond, a substituted or unsubstituted arylene group having 6 to 30 ring carbon atoms, or a substituted or unsubstituted divalent heterocyclic group having 5 to 30 ring atoms; and

* represents a bonding position in a pyrene ring in the formula (3).

In the third and fourth compounds, $R_{901}$, $R_{902}$, $R_{903}$, $R_{904}$, $R_{905}$, $R_{906}$, and $R_{907}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different; and
when a plurality of $R_{907}$ are present, the plurality of $R_{907}$ are mutually the same or different.

In the formula (3), two of $R_{301}$ to $R_{310}$ are each preferably a group represented by the formula (31).
In an exemplary embodiment, the compound represented by the formula (3) is a compound represented by a formula (33) below.

[Formula 355]

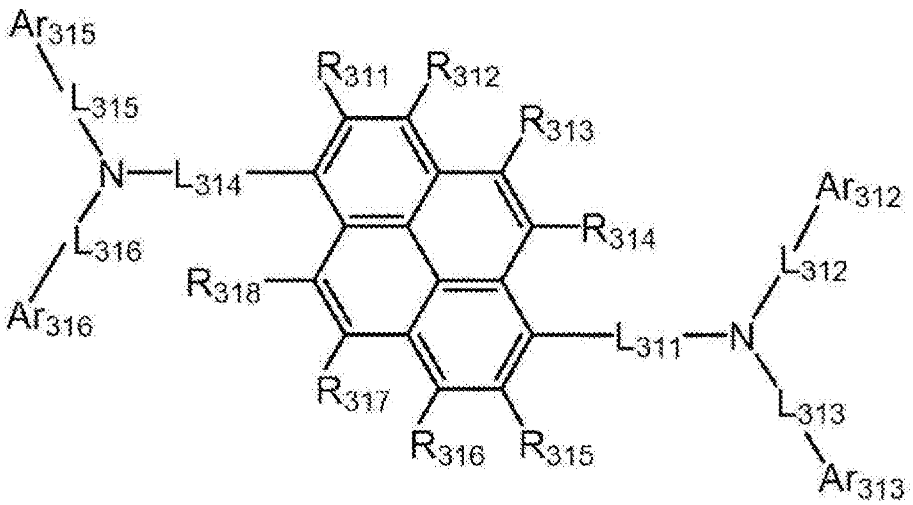

(33)

In the formula (33):

$R_{311}$ to $R_{318}$ each independently represent the same as $R_{301}$ to $R_{310}$ in the formula (3) that are not the monovalent group represented by the formula (31);
$L_{311}$ to $L_{316}$ are each independently a single bond, a substituted or unsubstituted arylene group having 6 to 30 ring carbon atoms, or a substituted or unsubstituted divalent heterocyclic group having 5 to 30 ring atoms;
$Ar_{312}$, $Ar_{313}$, $Ar_{315}$, and $Ar_{316}$ are each independently a substituted or unsubstituted aryl group having 6 to 50 ring carbon atoms, or a substituted or unsubstituted heterocyclic group having 5 to 50 ring atoms.

In the formula (31), $L_{301}$ is preferably a single bond, and $L_{302}$ and $L_{303}$ are each preferably a single bond.
In an exemplary embodiment, the compound represented by the formula (3) is represented by a formula (34) or a formula (35) below.

[Formula 356]

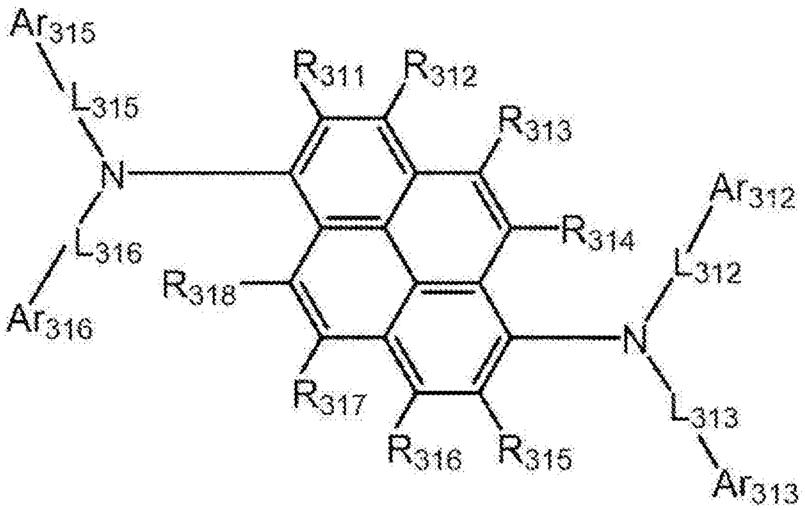

(34)

In the formula (34):

$R_{311}$ to $R_{318}$ each independently represent the same as $R_{301}$ to $R_{310}$ in the formula (3) that are not the monovalent group represented by the formula (31);
$L_{312}$, $L_{313}$, $L_{315}$ and $L_{316}$ each independently represent the same as $L_{312}$, $L_{313}$, $L_{315}$ and $L_{316}$ in the formula (33); and
$Ar_{312}$, $Ar_{313}$, $Ar_{315}$ and $Ar_{316}$ each independently represent the same as $Ar_{312}$, $Ar_{313}$, $Ar_{315}$ and $Ar_{316}$ in the formula (33).

[Formula 357]

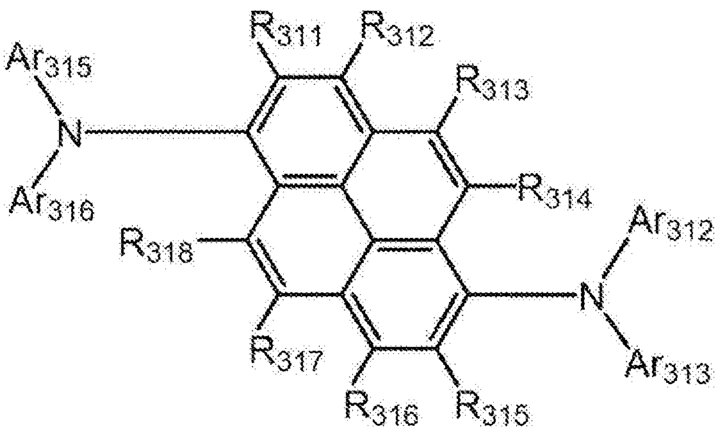

(35)

In the formula (35):

$R_{311}$ to $R_{318}$ each independently represent the same as $R_{301}$ to $R_{310}$ in the formula (3) that are not the monovalent group represented by the formula (31); and
$Ar_{312}$, $Ar_{313}$, $Ar_{315}$ and $Ar_{316}$ each independently represent the same as $Ar_{312}$, $Ar_{313}$, $Ar_{315}$ and $Ar_{316}$ in the formula (33).

In the formula (31), at least one of $Ar_{301}$ or $Ar_{302}$ is preferably a group represented by a formula (36) below.
In the formulae (33) to (35), at least one of $Ar_{312}$ or $Ar_{313}$ is preferably a group represented by the formula (36) below.
In the formulae (33) to (35), at least one of $Ar_{315}$ or $Ar_{316}$ is preferably a group represented by the formula (36) below.

[Formula 358]

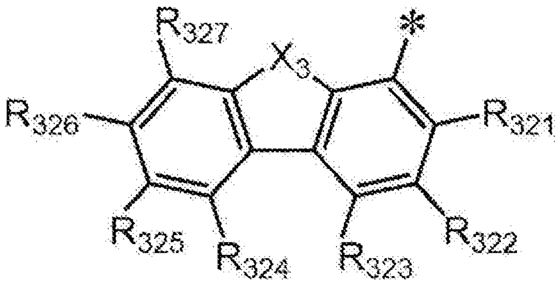

(36)

In the formula (36):

$X_3$ represents an oxygen atom or a sulfur atom;

at least one combination of adjacent two or more of $R_{321}$ to $R_{327}$ are mutually bonded to form a substituted or unsubstituted monocyclic ring, mutually bonded to form a substituted or unsubstituted fused ring, or not mutually bonded;

$R_{321}$ to $R_{327}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms; and

* represents a bonding position to $L_{302}$, $L_{303}$, $L_{312}$, $L_{313}$, $L_{315}$ or $L_{316}$.

$X_3$ is preferably an oxygen atom.

At least one of $R_{321}$ to $R_{327}$ is preferably a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms.

In the formula (31), it is preferable that $Ar_{301}$ is a group represented by the formula (36) and $Ar_{302}$ is a substituted or unsubstituted aryl group having 6 to 50 ring carbon atoms.

In the formulae (33) to (35), it is preferable that $Ar_{312}$ is a group represented by the formula (36) and $Ar_{313}$ is a substituted or unsubstituted aryl group having 6 to 50 ring carbon atoms.

In the formulae (33) to (35), it is preferable that $Ar_{315}$ is a group represented by the formula (36) and $Ar_{316}$ is a substituted or unsubstituted aryl group having 6 to 50 ring carbon atoms.

In an exemplary embodiment, the compound represented by the formula (3) is represented by a formula (37) below.

[Formula 359]

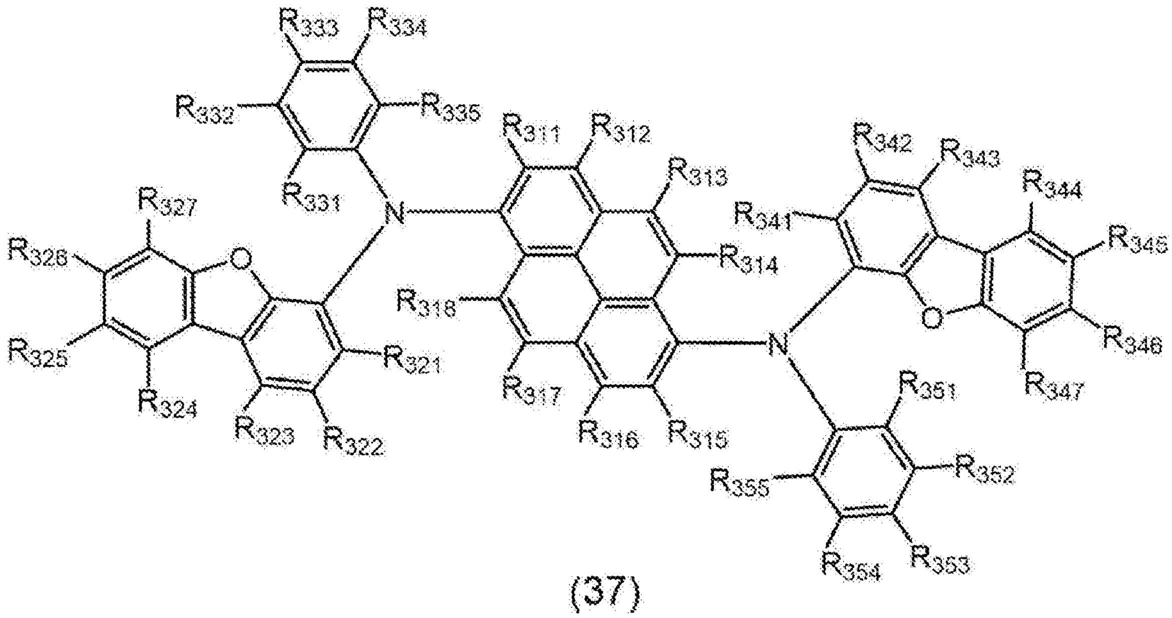

(37)

In the formula (37):

$R_{311}$ to $R_{318}$ each independently represent the same as $R_{301}$ to $R_{310}$ in the formula (3) that are not the monovalent group represented by the formula (31);

at least one combination of adjacent two or more of $R_{321}$ to $R_{327}$ are mutually bonded to form a substituted or unsubstituted monocyclic ring, mutually bonded to form a substituted or unsubstituted fused ring, or not mutually bonded;

at least one combination of adjacent two or more of $R_{341}$ to $R_{347}$ are mutually bonded to form a substituted or unsubstituted monocyclic ring, mutually bonded to form a substituted or unsubstituted fused ring, or not mutually bonded;

$R_{321}$ to $R_{327}$ and $R_{341}$ to $R_{347}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms;

$R_{331}$ to $R_{335}$ and $R_{351}$ to $R_{355}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $- S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

Specific examples of the compound represented by the formula (3) include compounds shown below.

[Formula 360]

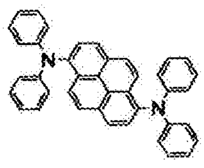
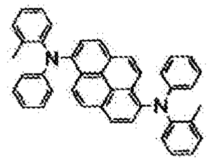
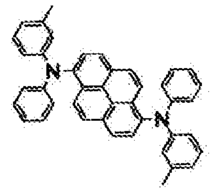
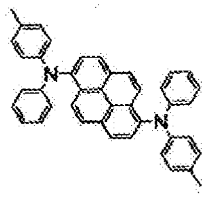
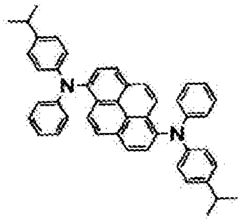
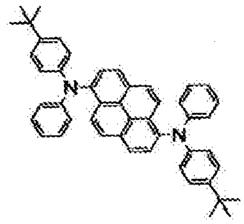
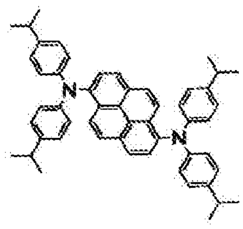
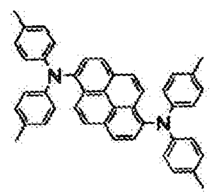
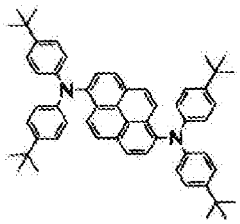
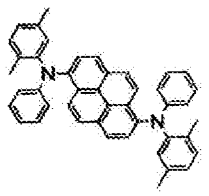
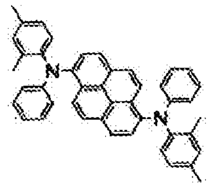
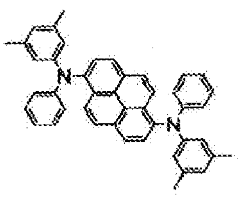
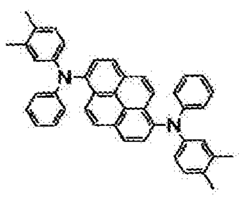
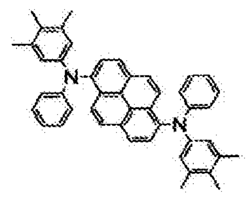
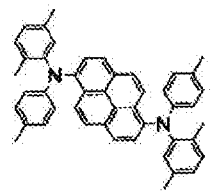
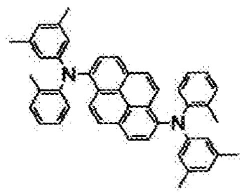
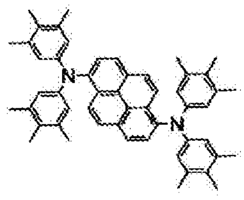
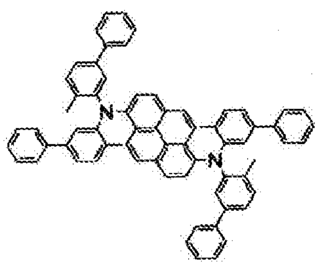
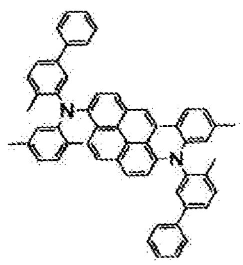
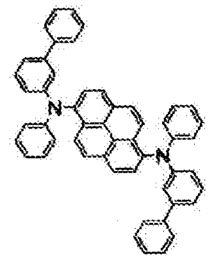

[Formula 361]

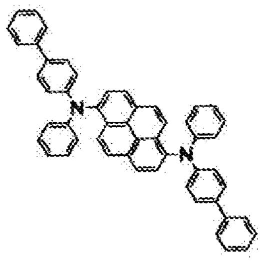
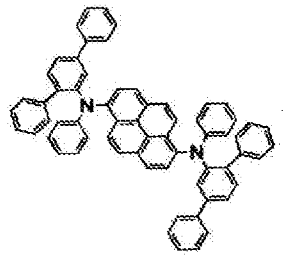
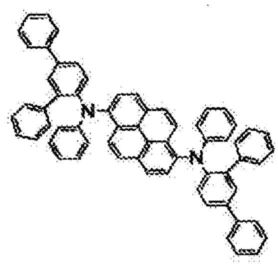
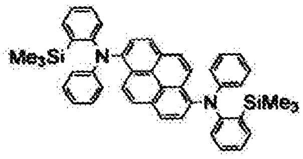
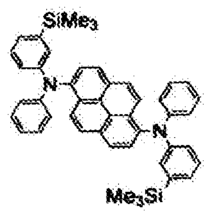
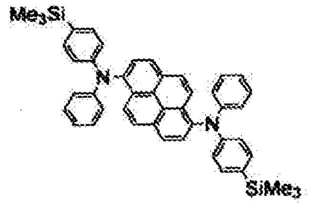
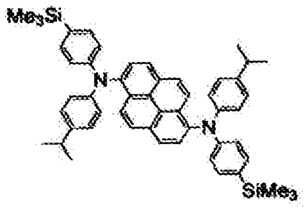
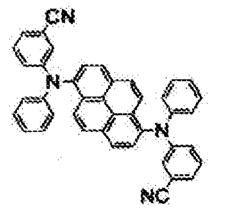
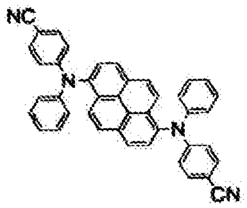
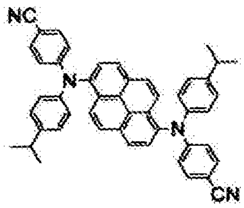
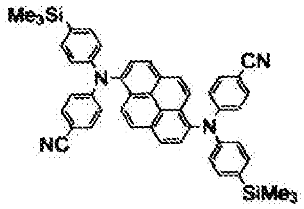
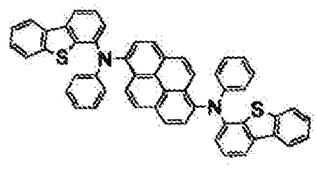
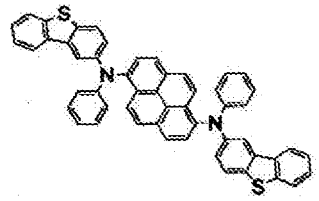
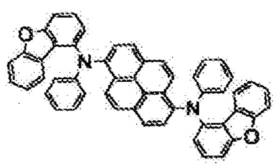
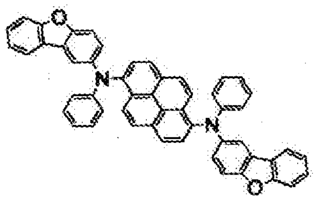
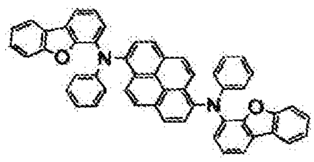

[Formula 362]

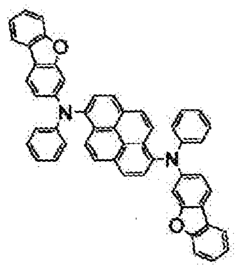
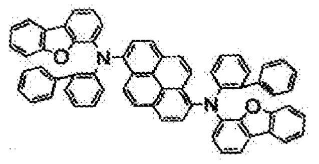
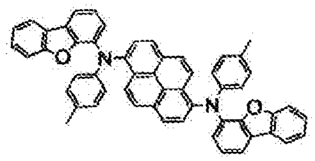
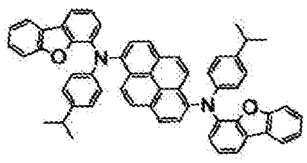
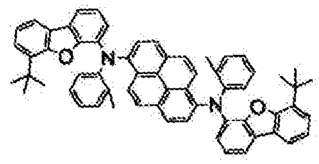
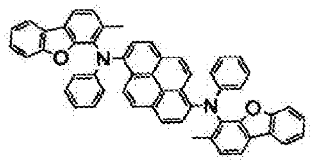
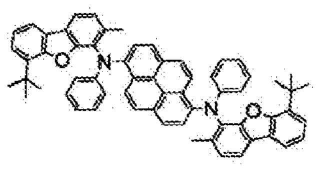
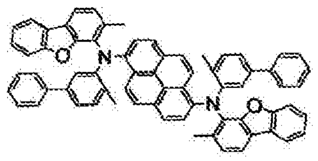
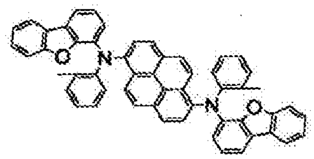
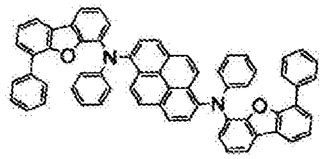
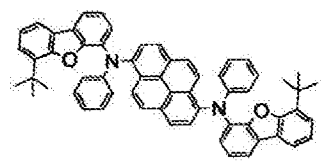
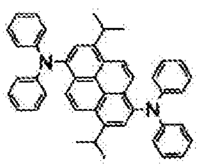
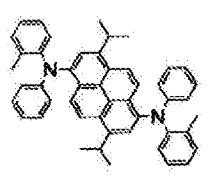
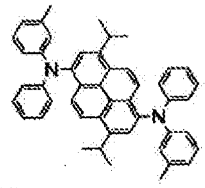
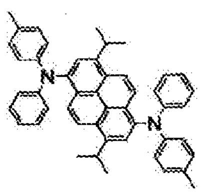
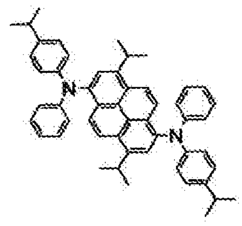
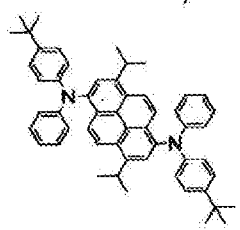
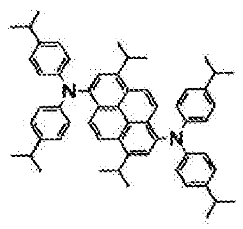
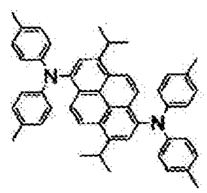

[Formula 363]

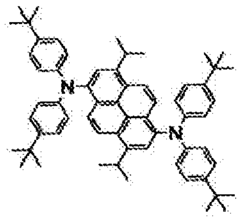 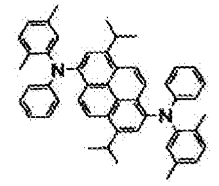 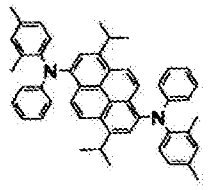

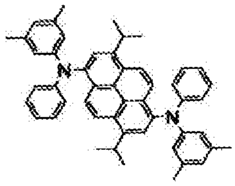 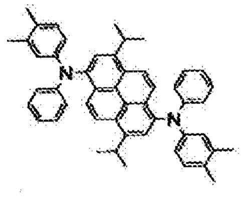 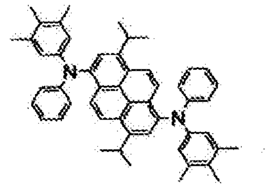

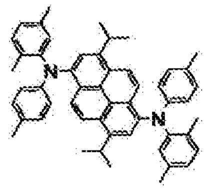 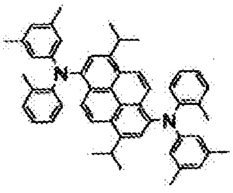 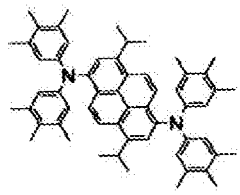

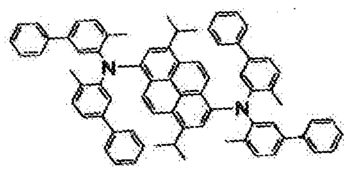 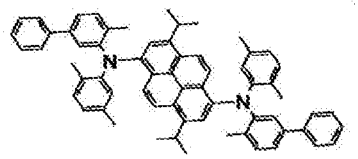 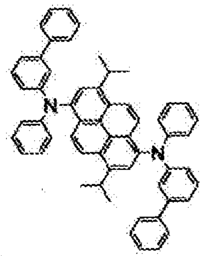

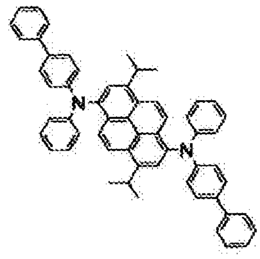 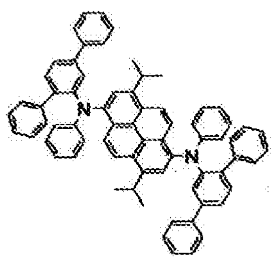 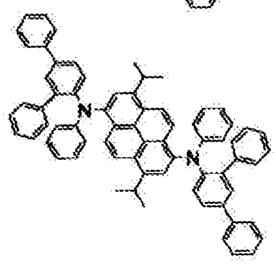

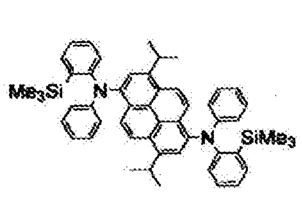 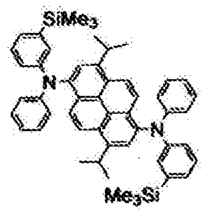 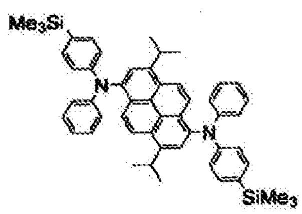

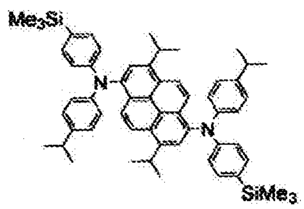 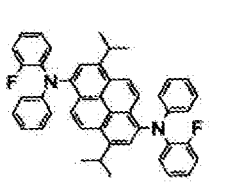 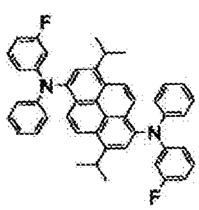

[Formula 364]

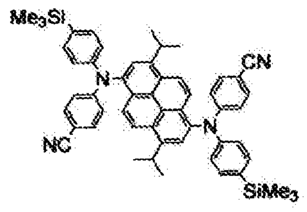
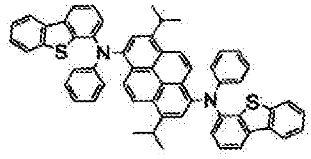
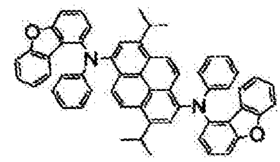
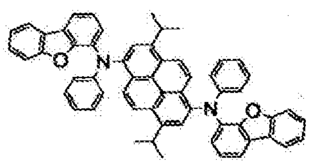
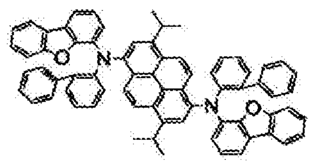
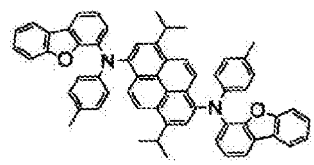
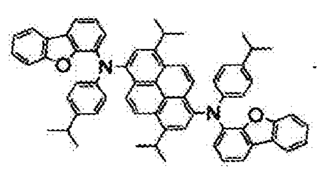
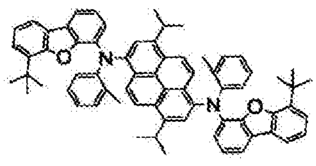
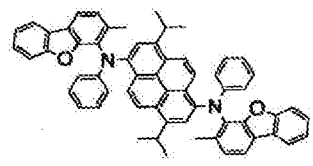
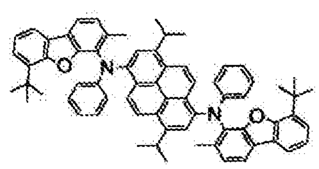
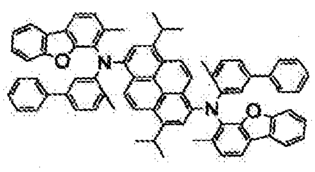
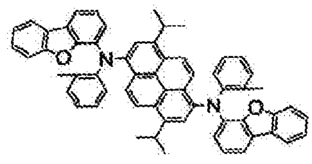
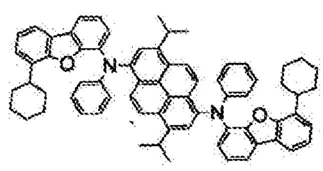
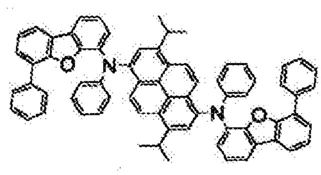
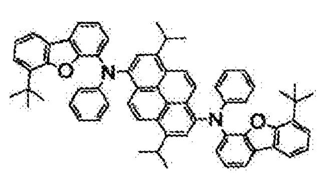

Compound Represented by Formula (4)

The compound represented by the formula (4) will be described.

[Formula 365]

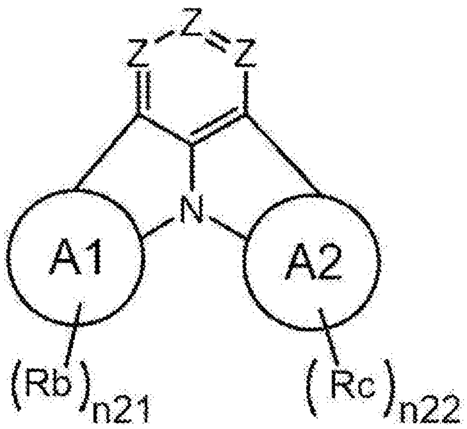

(4)

In the formula (4):

Z is each independently CRa or a nitrogen atom;
A1 ring and A2 ring are each independently a substituted or unsubstituted aromatic hydrocarbon ring having 6 to 50 ring carbon atoms, or a substituted or unsubstituted heterocycle having 5 to 50 ring atoms;
when a plurality of Ra are present, at least one combination of adjacent two or more of the plurality of Ra are mutually bonded to form a substituted or unsubstituted monocyclic ring, mutually bonded to form a substituted or unsubstituted fused ring, or not mutually bonded;
n21 and n22 are each independently 0, 1, 2, 3, or 4;
when a plurality of Rb are present, at least one combination of adjacent two or more of the plurality of Rb are mutually bonded to form a substituted or unsubstituted monocyclic ring, mutually bonded to form a substituted or unsubstituted fused ring, or not mutually bonded;
when a plurality of Rc are present, at least one combination of adjacent two or more of the plurality of Rc are mutually bonded to form a substituted or unsubstituted monocyclic ring, mutually bonded to form a substituted or unsubstituted fused ring, or not mutually bonded; and
Ra, Rb and Rc forming neither the substituted or unsubstituted monocyclic ring nor the substituted or unsubstituted fused ring are each independently a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

The "aromatic hydrocarbon ring" for the A1 ring and A2 ring has the same structure as a compound formed by introducing a hydrogen atom to the "aryl group" described above.
Ring atoms of the "aromatic hydrocarbon ring" for the A1 ring and the A2 ring include two carbon atoms on a fused bicyclic structure at the center of the formula (4).
Specific examples of the "substituted or unsubstituted aromatic hydrocarbon ring having 6 to 50 ring carbon atoms" include a compound formed by introducing a hydrogen atom to the "aryl group" described in the specific example group G1.
The "heterocycle" for the A1 ring and A2 ring has the same structure as a compound formed by introducing a hydrogen atom to the "heterocyclic group" described above.
Ring atoms of the "heterocycle" for the A1 ring and the A2 ring include two carbon atoms on a fused bicyclic structure at the center of the formula (4).
Specific examples of the "substituted or unsubstituted heterocycle having 5 to 50 ring atoms" include a compound formed by introducing a hydrogen atom to the "heterocyclic group" described in the specific example group G2.
Rb is bonded to any one of carbon atoms forming the aromatic hydrocarbon ring for the A1 ring or any one of the atoms forming the heterocycle for the A1 ring.
Rc is bonded to any one of carbon atoms forming the aromatic hydrocarbon ring for the A2 ring or any one of the atoms forming the heterocycle for the A2 ring.
At least one of Ra, Rb, or Rc is preferably a group represented by a formula (4a) below. More preferably, at least two of Ra, Rb, and Rc are groups represented by the formula (4a).
[Formula 366]

$*-L_{401}-Ar_{401}$ (4a)

In the formula (4a):

$L_{401}$ is a single bond, a substituted or unsubstituted arylene group having 6 to 30 ring carbon atoms, or a substituted or unsubstituted divalent heterocyclic group having 5 to 30 ring atoms; and
$Ar_{401}$ is a substituted or unsubstituted aryl group having 6 to 50 ring carbon atoms, a substituted or unsubstituted heterocyclic group having 5 to 50 ring atoms, or a group represented by the formula (4b).

[Formula 367]

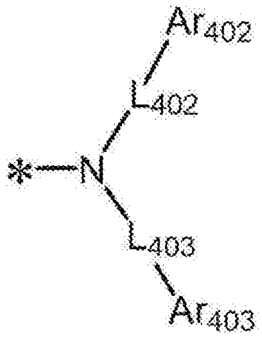

(4b)

In the formula (4b):

$L_{402}$ and $L_{403}$ are each independently a single bond, a substituted or unsubstituted arylene group having 6 to 30 ring carbon atoms, or a substituted or unsubstituted divalent heterocyclic group having 5 to 30 ring atoms;
a combination of $Ar_{402}$ and $Ar_{403}$ are mutually bonded to form a substituted or unsubstituted monocyclic ring, mutually bonded to form a substituted or unsubstituted fused ring, or not mutually bonded; and
$Ar_{402}$ and $Ar_{403}$ forming neither the substituted or unsubstituted monocyclic ring nor the substituted or unsubstituted fused ring are each independently a substituted or unsubstituted aryl group having 6 to 50 ring carbon atoms, or a substituted or unsubstituted heterocyclic group having 5 to 50 ring atoms.

In an exemplary embodiment, the compound represented by the formula (4) is represented by a formula (42) below.

[Formula 368]

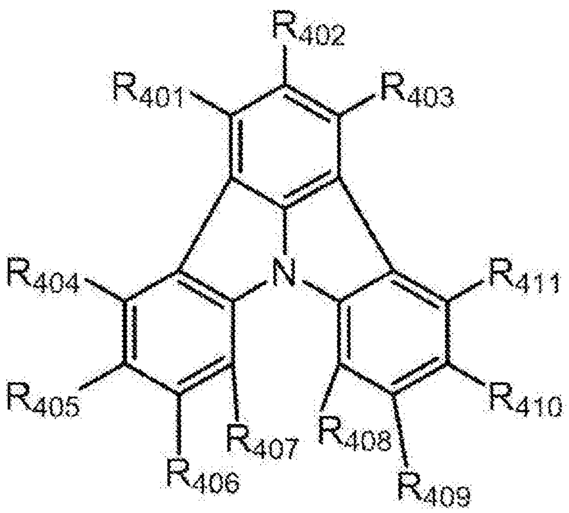

(42)

In the formula (42):

at least one combination of adjacent two or more of $R_{401}$ to $R_{411}$ are mutually bonded to form a substituted or unsubstituted monocyclic ring, mutually bonded to form a substituted or unsubstituted fused ring, or not mutually bonded; and

$R_{401}$ to $R_{411}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

At least one of $R_{401}$ to $R_{411}$ is preferably a group represented by the formula (4a). More preferably, at least two of $R_{401}$ to $R_{411}$ are each a group represented by the formula (4a).

Preferably, $R_{404}$ and $R_{411}$ are each a group represented by the formula (4a).

In an exemplary embodiment, the compound represented by the formula (4) is a compound formed by bonding a structure represented by a formula (4-1) or a formula (4-2) below to the A1 ring.

Further, in an exemplary embodiment, the compound represented by the formula (42) is a compound formed by bonding a structure represented by the formula (4-1) or the formula (4-2) to the ring bonded with $R_{404}$ to $R_{407}$.

[Formula 369]

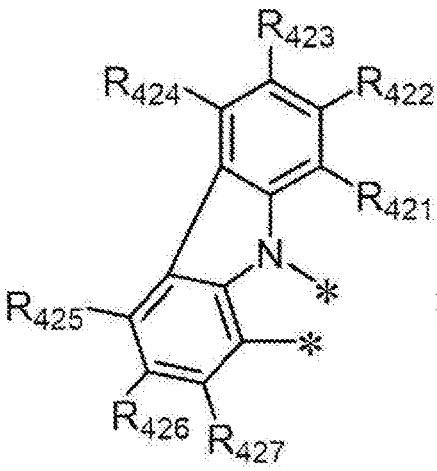

(4-1)

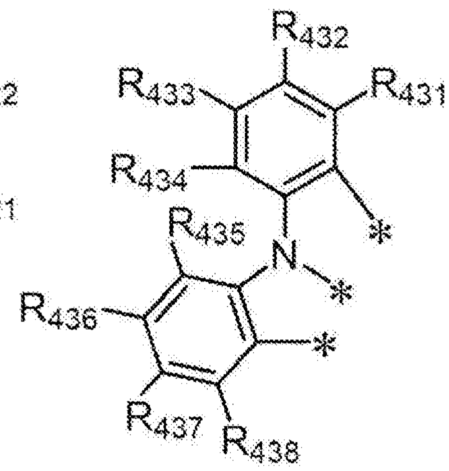

(4-2)

In the formula (4-1), two bonds * are each independently bonded to a ring-forming carbon atom of the aromatic hydrocarbon ring or a ring atom of the heterocycle for the A1 ring in the formula (4) or bonded to one of $R_{404}$ to $R_{407}$ in the formula (42);

in the formula (4-2), three bonds * are each independently bonded to a ring-forming carbon atom of the aromatic hydrocarbon ring or a ring atom of the heterocycle for the A1 ring in the formula (4) or bonded to one of $R_{404}$ to $R_{407}$ in the formula (42);

at least one combination of adjacent two or more of $R_{421}$ to $R_{427}$ are mutually bonded to form a substituted or unsubstituted monocyclic ring, mutually bonded to form a substituted or unsubstituted fused ring, or not mutually bonded;

at least one combination of adjacent two or more of $R_{431}$ to $R_{438}$ are mutually bonded to form a substituted or unsubstituted monocyclic ring, mutually bonded to form a substituted or unsubstituted fused ring, or not mutually bonded; and

$R_{421}$ to $R_{427}$ and $R_{431}$ to $R_{438}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having

3 to 50 ring carbon atoms, a group represented by -Si($R_{901}$)($R_{902}$)($R_{903}$), a group represented by -O-($R_{904}$), a group represented by -S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms.

In an exemplary embodiment, the compound represented by the formula (4) is a compound represented by a formula (41-3), a formula (41-4), or a formula (41-5) below.

[Formula 370]

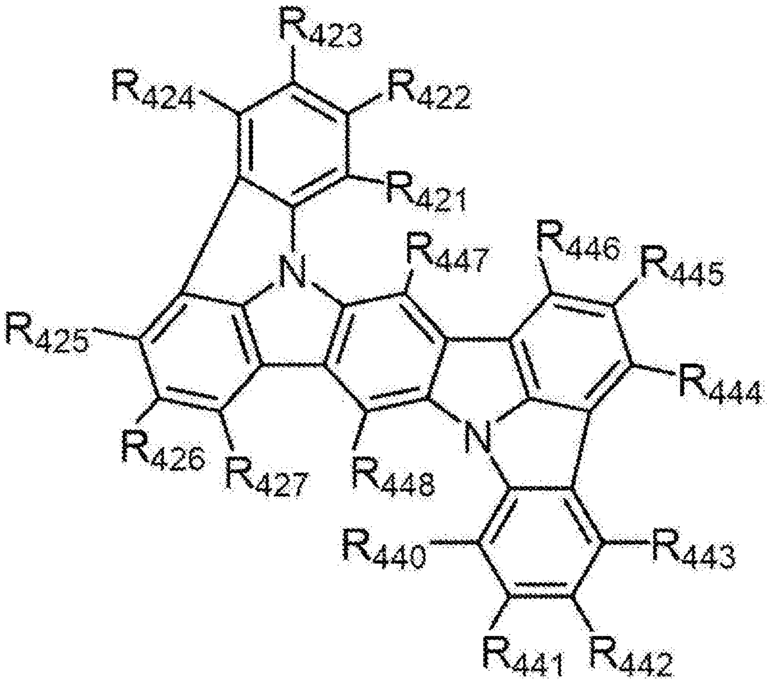

(41-3)

[Formula 371]

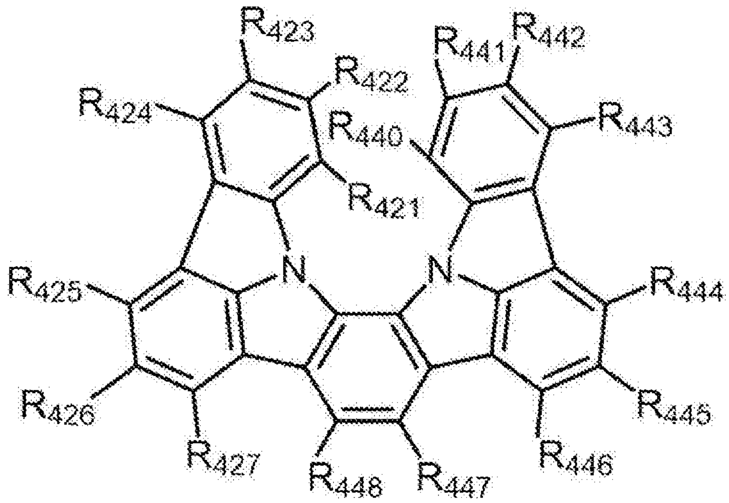

(41-4)

[Formula 372]

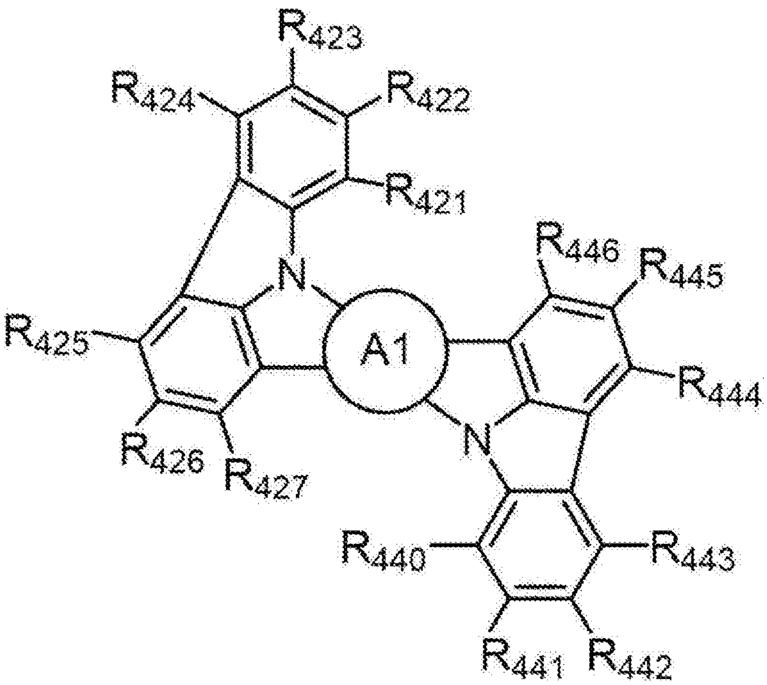

(41-5)

In the formulae (41-3), (41-4) and (41-5):

A1 ring is as defined for the formula (4);
$R_{421}$ to $R_{427}$ each independently represent the same as $R_{421}$ to $R_{427}$ in the formula (4-1); and
$R_{440}$ to $R_{448}$ each independently represent the same as $R_{401}$ to $R_{411}$ in the formula (42).

In an exemplary embodiment, a substituted or unsubstituted aromatic hydrocarbon ring having 6 to 50 ring carbon atoms for the A1 ring in the formula (41-5) is a substituted or unsubstituted naphthalene ring, or a substituted or unsubstituted fluorene ring.

In an exemplary embodiment, a substituted or unsubstituted heterocycle having 5 to 50 ring atoms for the A1 ring in the formula (41-5) is a substituted or unsubstituted dibenzofuran ring, a substituted or unsubstituted carbazole ring, or a substituted or unsubstituted dibenzothiophene ring.

In an exemplary embodiment, the compound represented by the formula (4) or the formula (42) is selected from the group consisting of compounds represented by formulae (461) to (467) below.

[Formula 373]

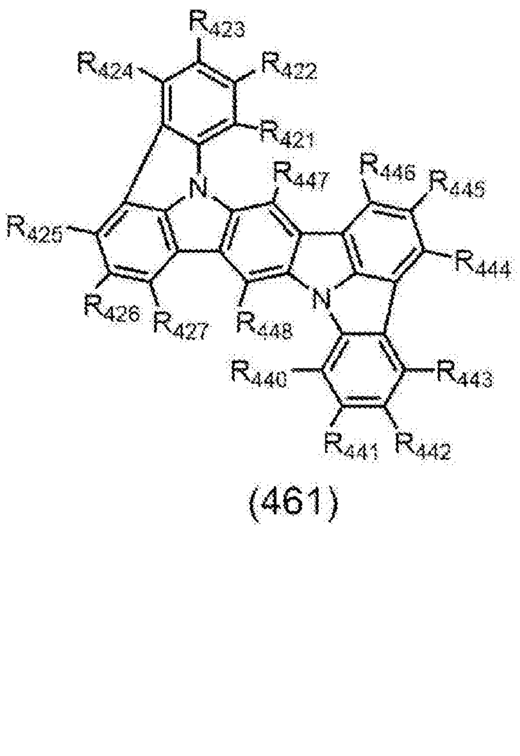

(461)

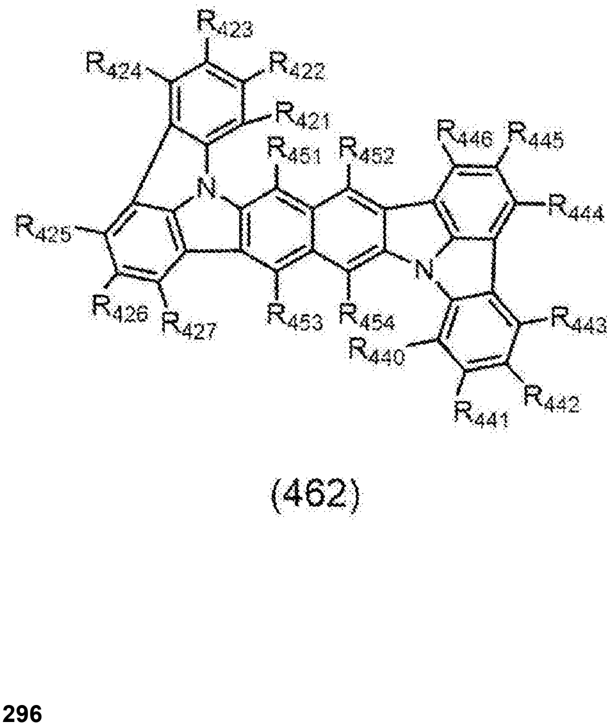

(462)

[Formula 374]

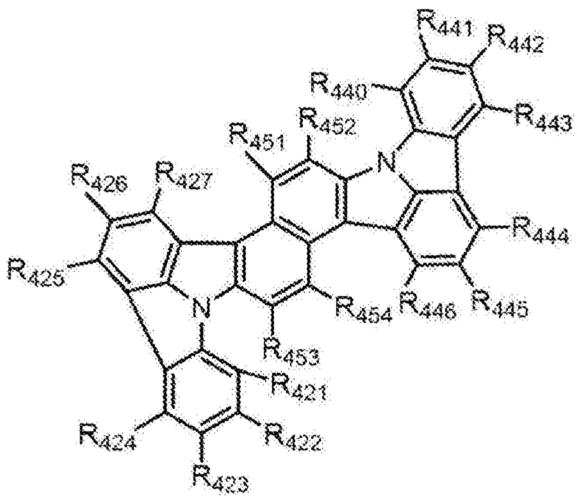

(463)

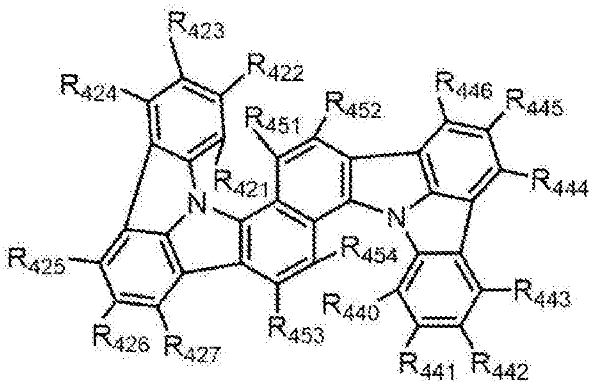

(464)

[Formula 375]

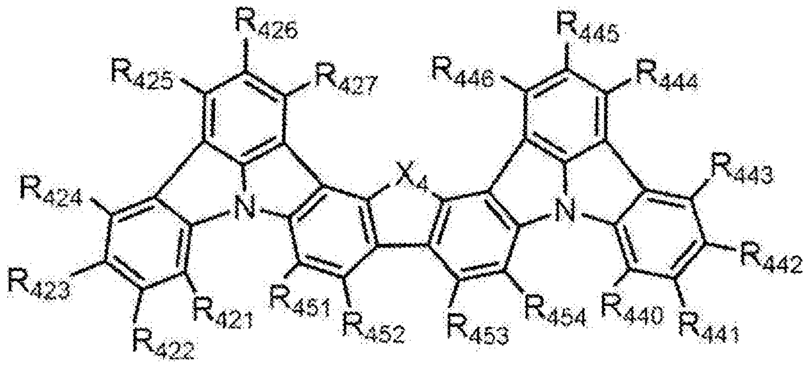

(465)

[Formula 376]

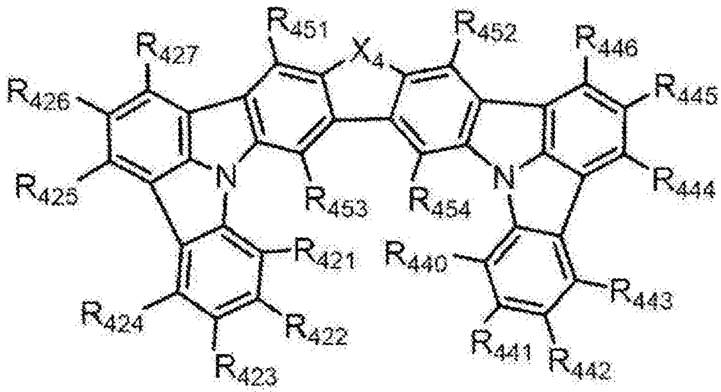

(466)

[Formula 377]

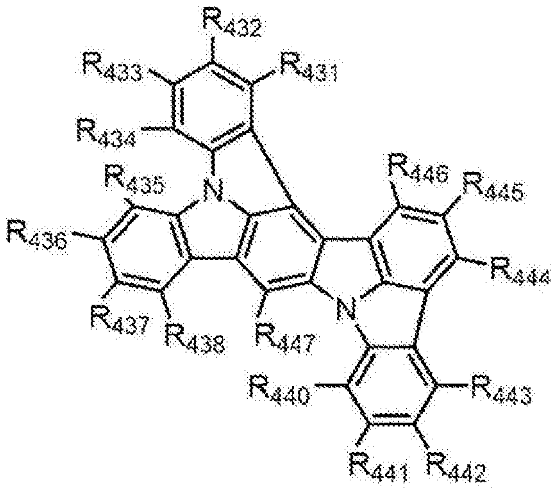

(467)

In the formulae (461), (462), (463), (464), (465), (466) and (467):

$R_{421}$ to $R_{427}$ each independently represent the same as $R_{421}$ to $R_{427}$ in the formula (4-1);
$R_{431}$ to $R_{438}$ each independently represent the same as $R_{431}$ to $R_{438}$ in the formula (4-2);
$R_{440}$ to $R_{448}$ and $R_{451}$ to $R_{454}$ each independently represent the same as $R_{401}$ to $R_{411}$ in the formula (42);
$X_4$ is an oxygen atom, $NR_{801}$, or $C(R_{802})(R_{803})$;
$R_{801}$, $R_{802}$, and $R_{803}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;
a substituted or unsubstituted alkyl group having 1 to 50 carbon atoms, or a substituted or unsubstituted aryl group having 6 to 50 ring carbon atoms;
when a plurality of $R_{801}$ are present, the plurality of $R_{801}$ are mutually the same or different;
when a plurality of $R_{802}$ are present, the plurality of $R_{802}$ are mutually the same or different; and
when a plurality of $R_{803}$ are present, the plurality of $R_{803}$ are mutually the same or different.

In an exemplary embodiment, in a compound represented by the formula (42), at least one combination of adjacent two or more of $R_{401}$ to $R_{411}$ are mutually bonded to form a substituted or unsubstituted monocyclic ring or a substituted or unsubstituted fused ring. The compound represented by the formula (42) in the exemplary embodiment is described in detail as a compound represented by a formula (45) below.

Compound Represented by Formula (45)

The compound represented by the formula (45) will be described.

[Formula 378]

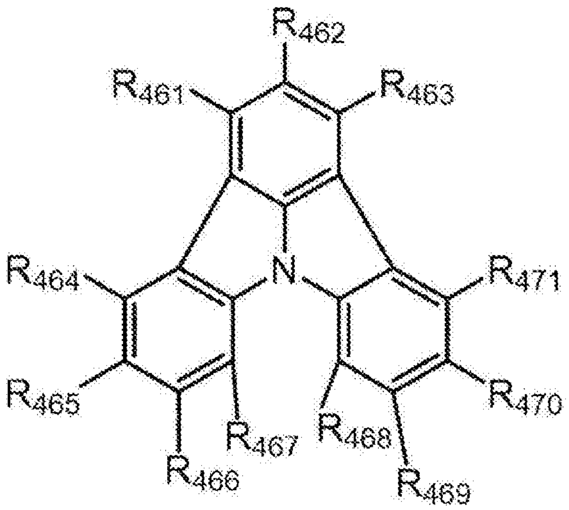

(45)

In the formula (45): two or more of combinations selected from the group consisting of a combination of $R_{461}$ and $R_{462}$, a combination of $R_{462}$ and $R_{463}$, a combination of $R_{464}$ and $R_{465}$, a combination of $R_{465}$ and $R_{466}$, a combination of $R_{466}$ and $R_{467}$, a combination of $R_{468}$ and $R_{469}$, a combination of $R_{469}$ and $R_{470}$, and a combination of $R_{470}$ and $R_{471}$ are mutually bonded to form a substituted or unsubstituted monocyclic ring or a substituted or unsubstituted fused ring.

However, the combination of $R_{461}$ and $R_{462}$ and the combination of $R_{462}$ and $R_{463}$; the combination of $R_{464}$ and $R_{465}$ and the combination of $R_{465}$ and $R_{466}$; the combination of $R_{465}$ and $R_{466}$ and the combination of $R_{466}$ and $R_{467}$; the combination of $R_{468}$ and $R_{469}$ and the combination of $R_{469}$ and $R_{470}$; and the combination of $R_{469}$ and $R_{470}$ and the combination of $R_{470}$ and $R_{471}$ do not form a ring at the same time.

At least two rings formed by $R_{461}$ to $R_{471}$ are mutually the same or different.

$R_{461}$ to $R_{471}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $- S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

In the formula (45), $R_n$ and $R_{n+1}$ (n being an integer selected from 461, 462, 464 to 466, and 468 to 470) are mutually bonded to form a substituted or unsubstituted monocyclic ring or fused ring together with two ring-forming carbon atoms bonded to $R_n$ and $R_{n+1}$. The ring is preferably formed of atoms selected from the group consisting of a carbon atom, an oxygen atom, a sulfur atom, and a nitrogen atom, and is preferably made of 3 to 7, more preferably 5 or 6 atoms.

The number of the above cyclic structures in the compound represented by the formula (45) is, for instance, 2, 3, or 4. The two or more of the cyclic structures may be present on the same benzene ring on the basic skeleton represented by the formula (45) or may be present on different benzene rings. For instance, when three cyclic structures are present, each of the cyclic structures may be present on corresponding one of the three benzene rings of the formula (45).

Examples of the above cyclic structures in the compound represented by the formula (45) include structures represented by formulae (451) to (460) below.

[Formula 379]

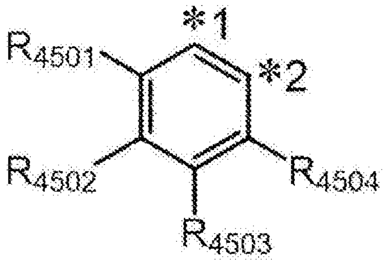

(451)

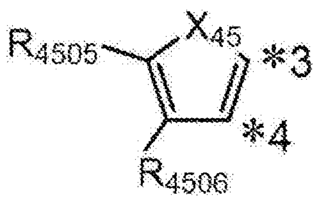

(452)

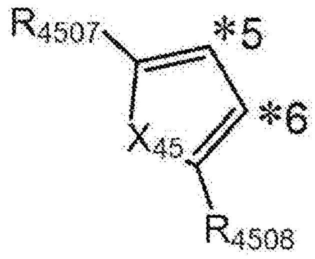

(453)

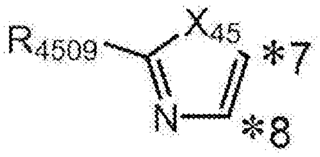

(454)

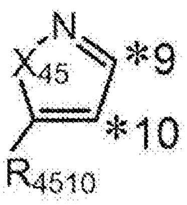

(455)

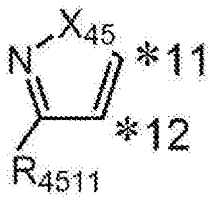

(456)

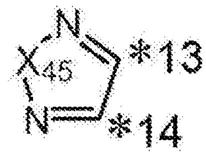

(457)

In the formulae (451) to (457):

each combination of *1 and *2, *3 and *4, *5 and *6, *7 and *8, *9 and *10, *11 and *12, and *13 and *14 represent the two ring-forming carbon atoms bonded to $R_n$ and $R_{n+1}$;
the ring-forming carbon atom bonded to $R_n$ may be any one of the two ring-forming carbon atoms represented by *1 and *2, *3 and *4, *5 and *6, *7 and *8, *9 and *10, *11 and *12, and *13 and *14;
$X_{45}$ is $C(R_{4512})(R_{4513})$, $NR_{4514}$, an oxygen atom, or a sulfur atom;
at least one combination of adjacent two or more of $R_{4501}$ to $R_{4506}$ and $R_{4512}$ to $R_{4513}$ are mutually bonded to form a substituted or unsubstituted monocyclic ring, mutually bonded to form a substituted or unsubstituted fused ring, or not mutually bonded; and
$R_{4501}$ to $R_{4514}$ forming neither the substituted or unsubstituted monocyclic ring nor the substituted or unsubstituted fused ring each independently represent the same as $R_{461}$ to $R_{471}$ in the formula (45).

[Formula 380]

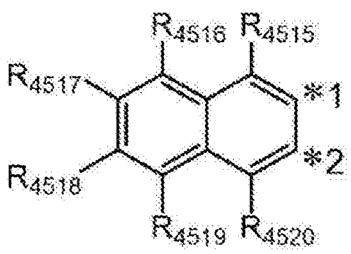

(458)

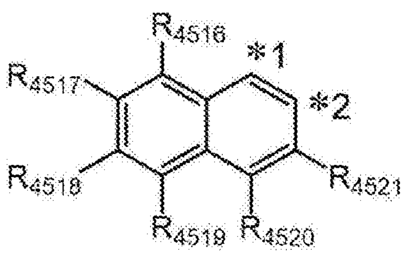

(459)

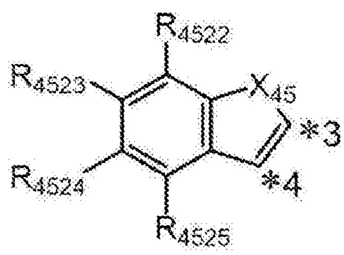

(460)

In the formulae (458) to (460):

each combination of *1 and *2, and *3 and *4 represent the two ring-forming carbon atoms bonded to $R_n$ and $R_{n+1}$;
the ring-forming carbon atom bonded to $R_n$ may be any one of the two ring-forming carbon atoms represented by

*1 and *2, or *3 and *4;

$X_{45}$ is $C(R_{4512})(R_{4513})$, $NR_{4514}$, an oxygen atom, or a sulfur atom;

at least one combination of adjacent two or more of $R_{4512}$ to $R_{4513}$ and $R_{4515}$ to $R_{4525}$ are mutually bonded to form a substituted or unsubstituted monocyclic ring, mutually bonded to form a substituted or unsubstituted fused ring, or not mutually bonded; and

$R_{4512}$ to $R_{4513}$, $R_{4515}$ to $R_{4521}$ and $R_{4522}$ to $R_{4525}$ forming neither the substituted or unsubstituted monocyclic ring nor the substituted or unsubstituted fused ring, and $R_{4514}$ each independently represent the same as $R_{461}$ to $R_{471}$ in the formula (45).

In the formula (45), it is preferable that at least one of $R_{462}$, $R_{464}$, $R_{465}$, $R_{470}$ or $R_{471}$ (preferably, at least one of $R_{462}$, $R_{465}$, or $R_{470}$, more preferably $R_{462}$) is a group forming no cyclic structure.

(i) In the formula (45), a substituent, if present, for a cyclic structure formed by $R_n$ and $R_{n+1}$,

(ii) in the formula (45), $R_{461}$ to $R_{471}$ forming no cyclic structure, and

(iii) $R_{4501}$ to $R_{4514}$ and $R_{4515}$ to $R_{4525}$ in the formulae (451) to (460) are preferably each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-N(R_{906})(R_{907})$, a substituted or unsubstituted aryl group having 6 to 50 ring carbon atoms, a substituted or unsubstituted heterocyclic group having 5 to 50 ring atoms, or groups represented by formulae (461) to (464).

[Formula 381]

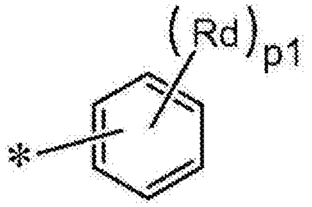

(461)

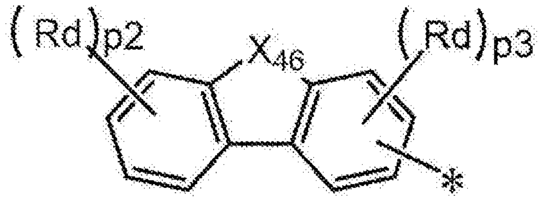

(462)

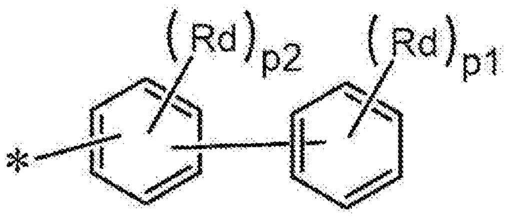

(463)

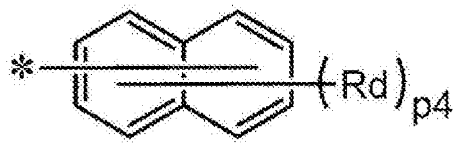

(464)

In the formulae (461) to (464):

$R_d$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms;

$X_{46}$ is $C(R_{801})(R_{802})$, $NR_{803}$, an oxygen atom, or a sulfur atom;

$R_{801}$, $R_{802}$, and $R_{803}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

a substituted or unsubstituted alkyl group having 1 to 50 carbon atoms, or a substituted or unsubstituted aryl group having 6 to 50 ring carbon atoms;

when a plurality of $R_{801}$ are present, the plurality of $R_{801}$ are mutually the same or different;

when a plurality of $R_{802}$ are present, the plurality of $R_{802}$ are mutually the same or different;

when a plurality of $R_{803}$ are present, the plurality of $R_{803}$ are mutually the same or different;

p1 is 5;

p2 is 4;

p3 is 3;

p4 is 7; and

* in the formulae (461) to (464) each independently represents a bonding position to a cyclic structure.

In the third and fourth compounds, $R_{901}$ to $R_{907}$ represent the same as those as described above.

In an exemplary embodiment, the compound represented by the formula (45) is represented by one of formulae (45-1) to (45-6) below.

[Formula 382]

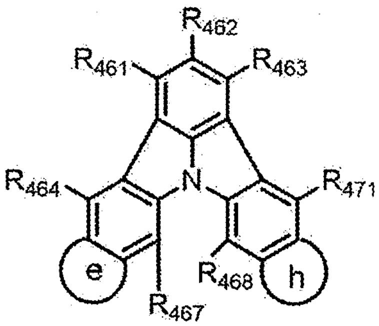

(45-1)

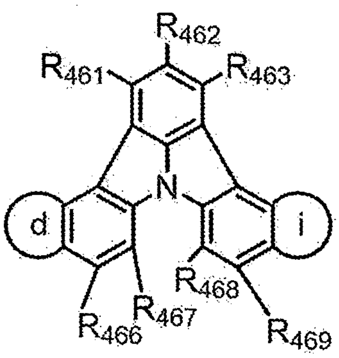

(45-2)

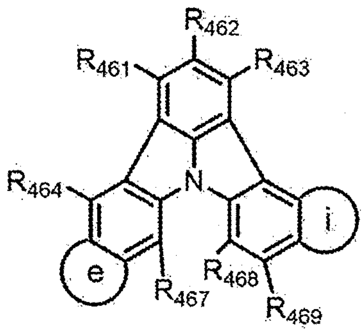

(45-3)

[Formula 383]

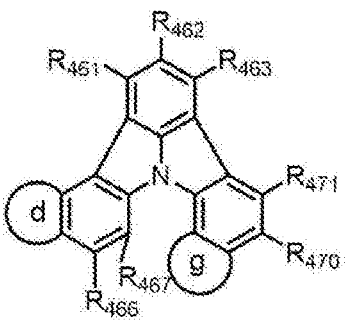

(45-4)

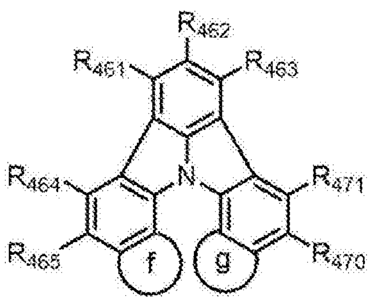

(45-5)

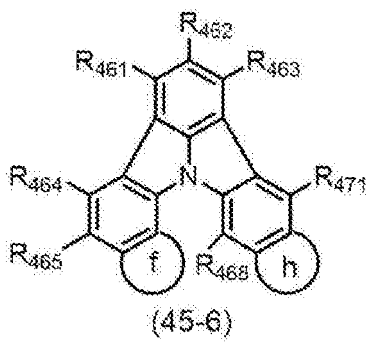

(45-6)

In the formulae (45-1) to (45-6):

rings d to i are each independently a substituted or unsubstituted monocyclic ring or a substituted or unsubstituted fused ring; and

$R_{461}$ to $R_{471}$ each independently represent the same as $R_{461}$ to $R_{471}$ in the formula (45).

In an exemplary embodiment, the compound represented by the formula (45) is represented by one of formulae (45-7) to (45-12) below.

[Formula 384]

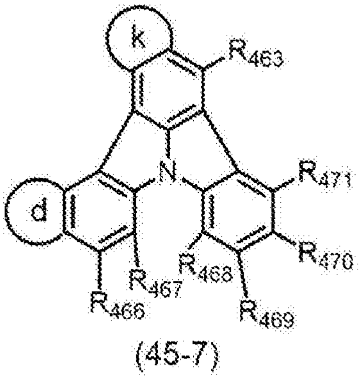

(45-7)

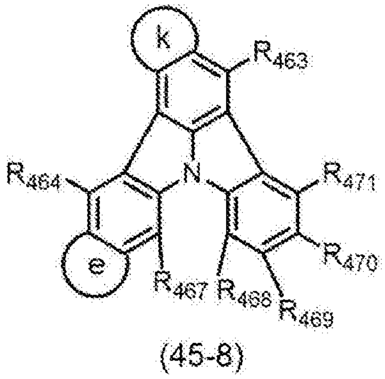

(45-8)

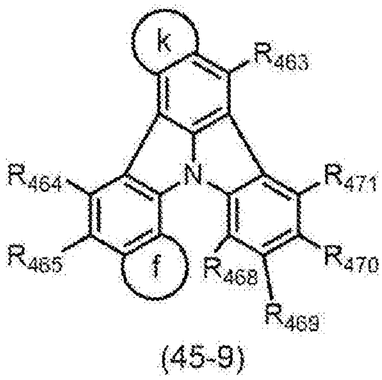

(45-9)

[Formula 385]

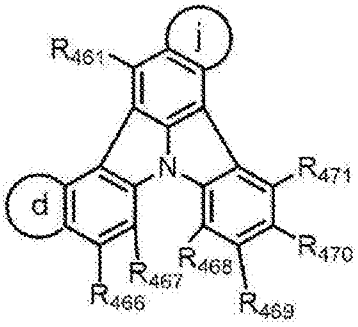

(45-10)

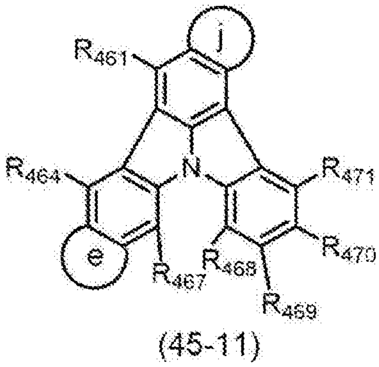

(45-11)

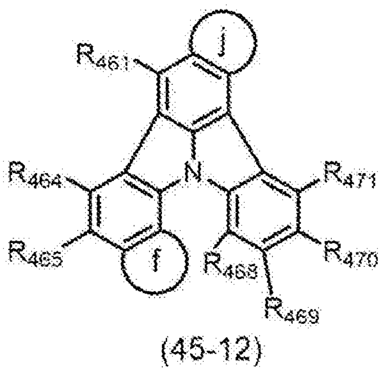

(45-12)

In the formulae (45-7) to (45-12):

rings d to f, k, and j are each independently a substituted or unsubstituted monocyclic ring or a substituted or unsubstituted fused ring; and
$R_{461}$ to $R_{471}$ each independently represent the same as $R_{461}$ to $R_{471}$ in the formula (45).

In an exemplary embodiment, the compound represented by the formula (45) is represented by one of formulae (45-13) to (45-21) below.

[Formula 386]

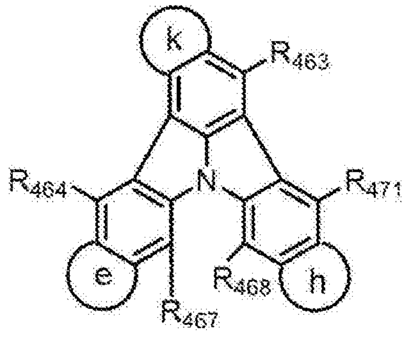

(45-13)

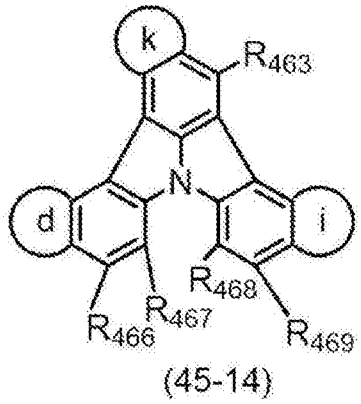

(45-14)

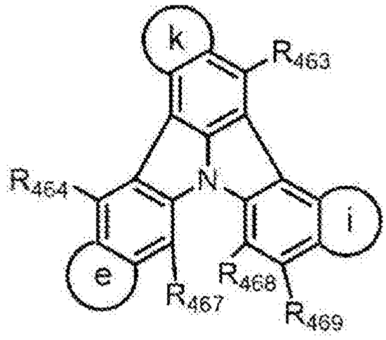

(45-15)

[Formula 387]

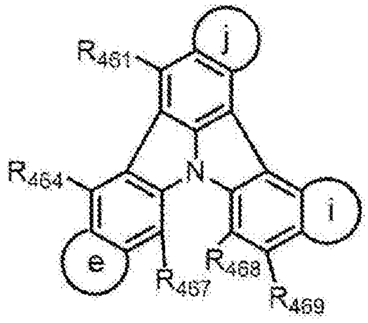

(45-16)

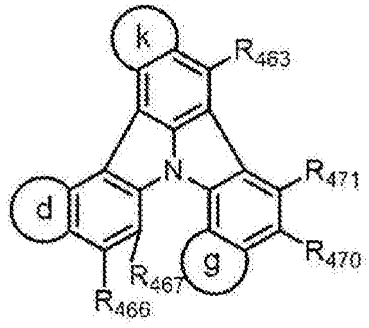

(45-17)

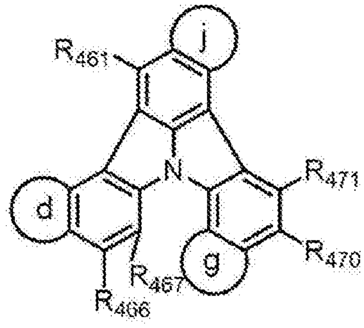

(45-18)

[Formula 388]

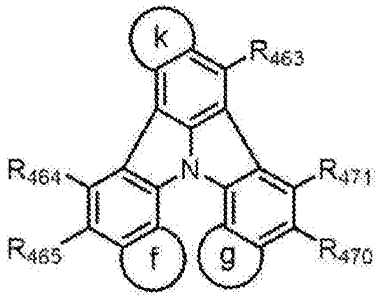

(45-19)

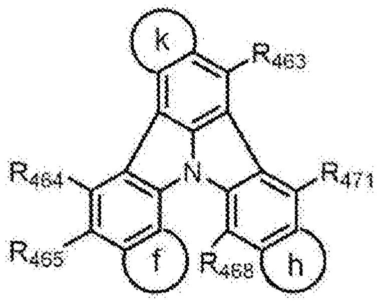

(45-20)

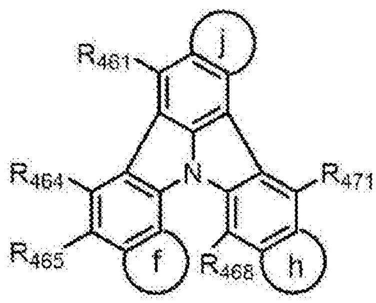

(45-21)

In the formulae (45-13) to (45-21):

rings d to k are each independently a substituted or unsubstituted monocyclic ring or a substituted or unsubstituted fused ring; and

$R_{461}$ to $R_{471}$ each independently represent the same as $R_{461}$ to $R_{471}$ in the formula (45).

When the ring g or the ring h further has a substituent, examples of the substituent include a substituted or unsubstituted alkyl group having 1 to 50 carbon atoms, a substituted or unsubstituted aryl group having 6 to 50 ring carbon atoms, a group represented by the formula (461), a group represented by the formula (463), and a group represented by the formula (464).

In an exemplary embodiment, the compound represented by the formula (45) is represented by one of formulae (45-22) to (45-25) below.

[Formula 389]

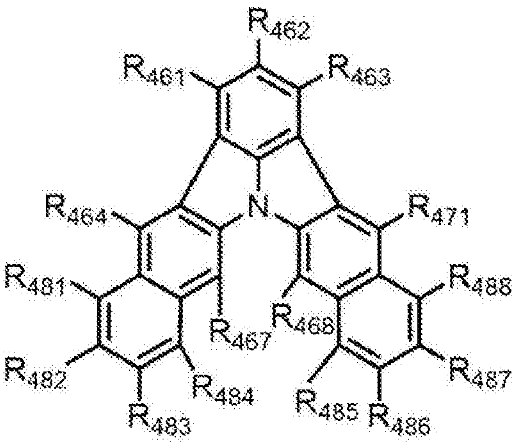

(45-22)

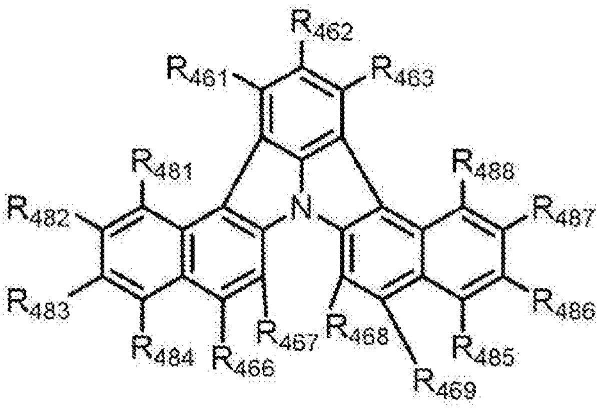

(45-23)

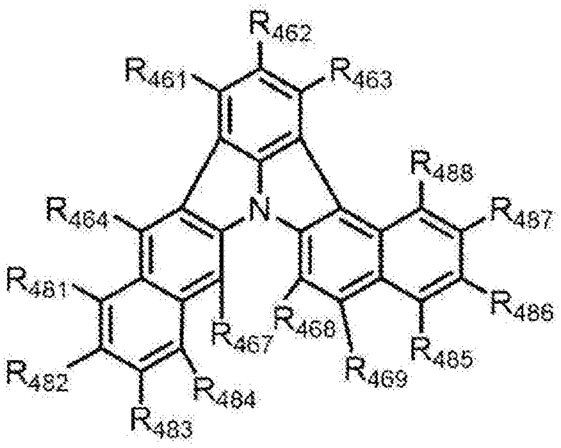

(45-24)

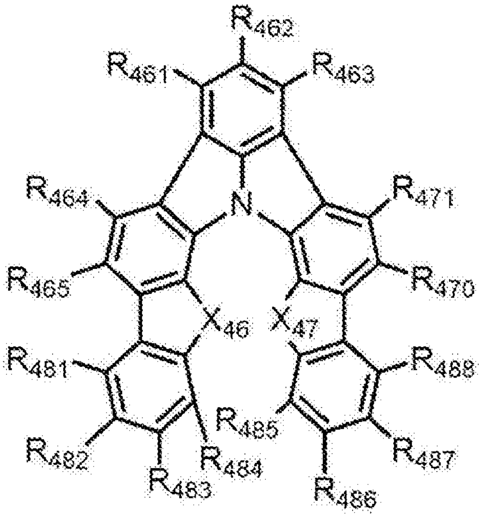

(45-25)

In the formulae (45-22) to (45-25):

$X_{46}$ and $X_{47}$ are each independently $C(R_{801})(R_{802})$, $NR_{803}$, an oxygen atom, or a sulfur atom;
$R_{461}$ to $R_{471}$ and $R_{481}$ to $R_{488}$ each independently represent the same as $R_{461}$ to $R_{471}$ of the formula (45);
$R_{801}$, $R_{802}$, and $R_{803}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;
a substituted or unsubstituted alkyl group having 1 to 50 carbon atoms, or a substituted or unsubstituted aryl group having 6 to 50 ring carbon atoms;
when a plurality of $R_{801}$ are present, the plurality of $R_{801}$ are mutually the same or different;
when a plurality of $R_{802}$ are present, the plurality of $R_{802}$ are mutually the same or different; and
when a plurality of $R_{803}$ are present, the plurality of $R_{803}$ are mutually the same or different.

In an exemplary embodiment, the compound represented by the formula (45) is represented by a formula

(45-26) below.

[Formula 390]

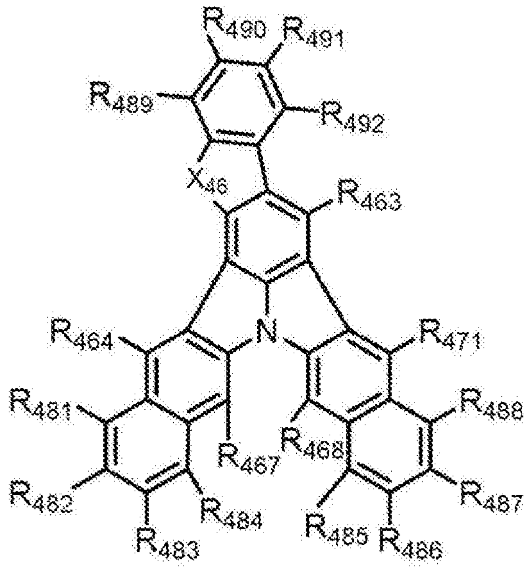

(45-26)

In the formula (45-26):

$X_{46}$ is $C(R_{801})(R_{802})$, $NR_{803}$, an oxygen atom, or a sulfur atom;
$R_{463}$, $R_{464}$, $R_{467}$, $R_{468}$, $R_{471}$, and $R_{481}$ to $R_{492}$ each independently represent the same as $R_{461}$ to $R_{471}$ in the formula (45);
$R_{801}$, $R_{802}$, and $R_{803}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;
a substituted or unsubstituted alkyl group having 1 to 50 carbon atoms, or a substituted or unsubstituted aryl group having 6 to 50 ring carbon atoms;
when a plurality of $R_{801}$ are present, the plurality of $R_{801}$ are mutually the same or different;
when a plurality of $R_{802}$ are present, the plurality of $R_{802}$ are mutually the same or different; and
when a plurality of $R_{803}$ are present, the plurality of $R_{803}$ are mutually the same or different.

Specific examples of the compound represented by the formula (4) include compounds shown below. In the specific examples below, Ph represents a phenyl group, and D represents a deuterium atom.

[Formula 391]

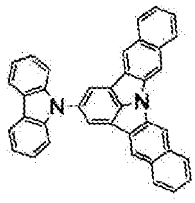
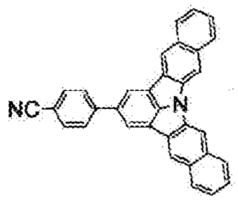
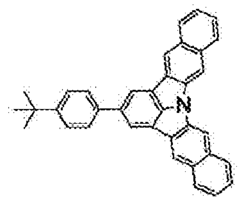

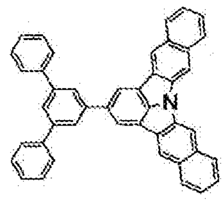
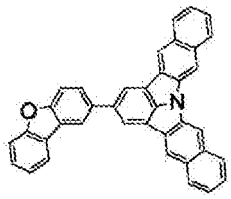
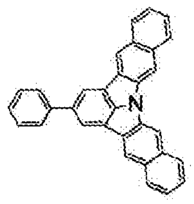

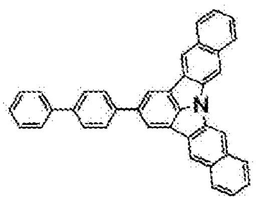
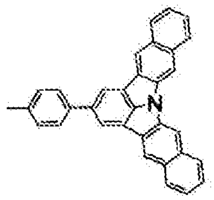
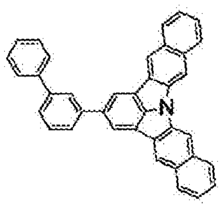

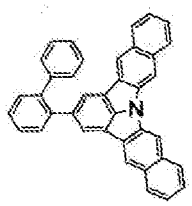
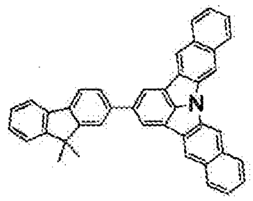
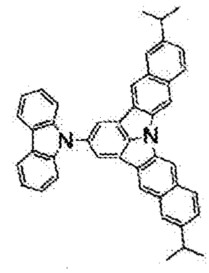

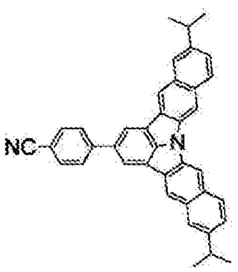
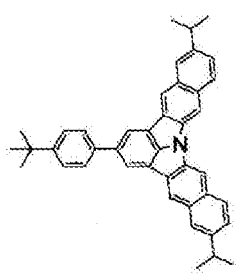
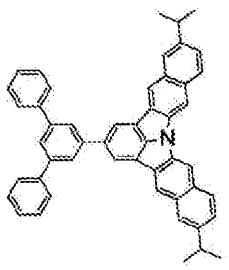

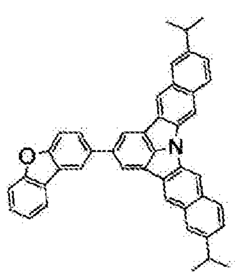
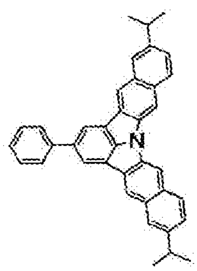
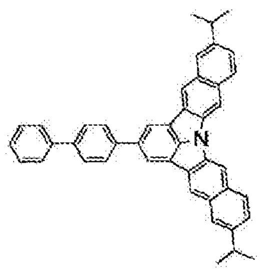

[Formula 392]

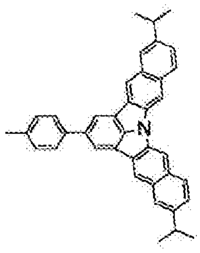
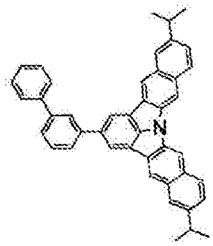
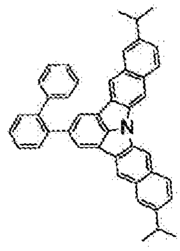
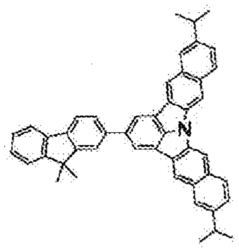
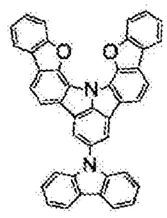
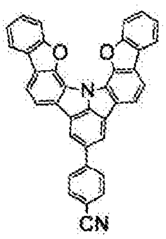
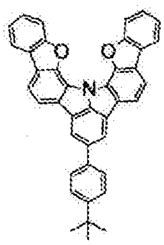
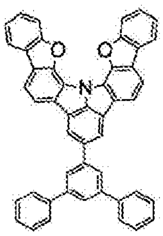
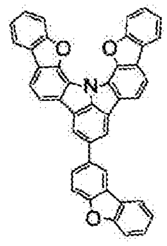
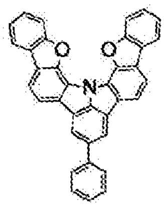
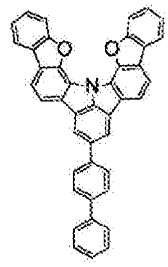
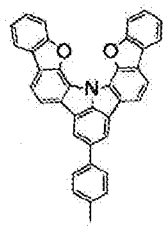
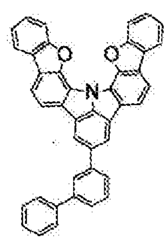
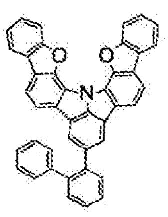
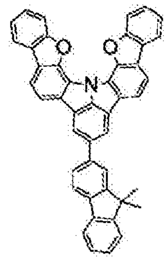
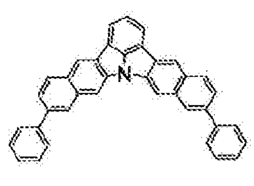
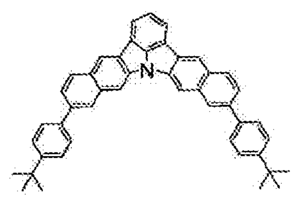
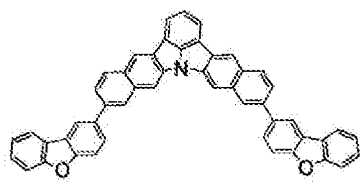

[Formula 393]

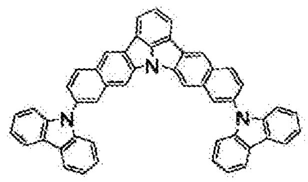
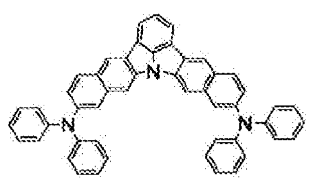
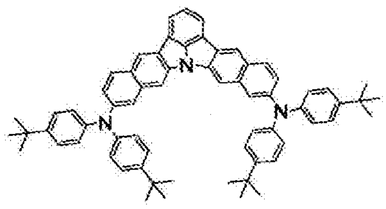

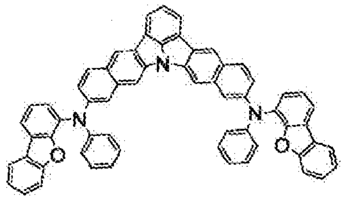

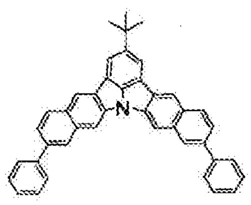
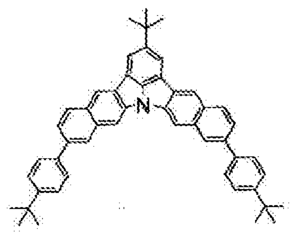
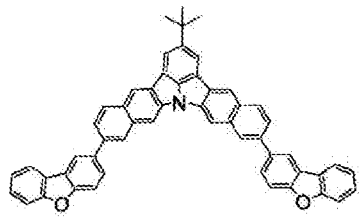

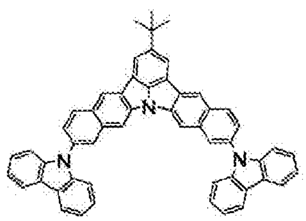
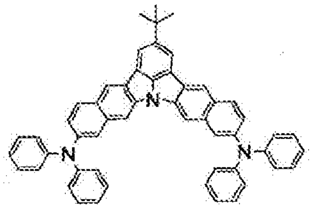
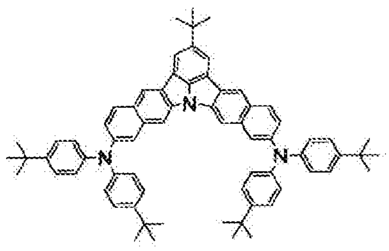

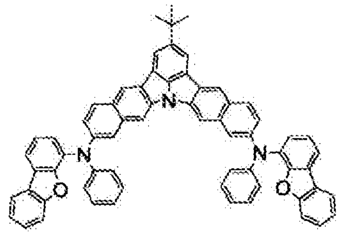

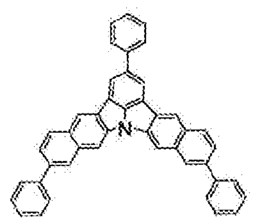
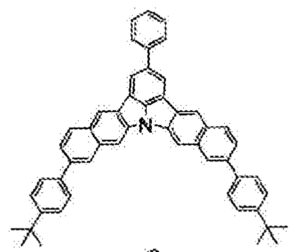
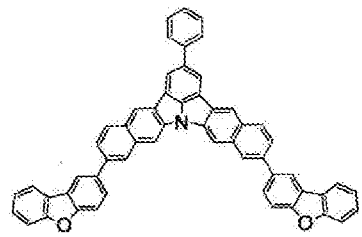

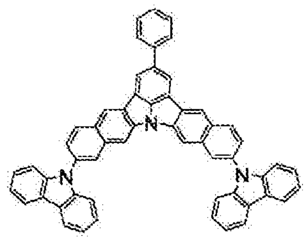
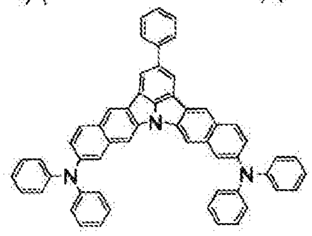
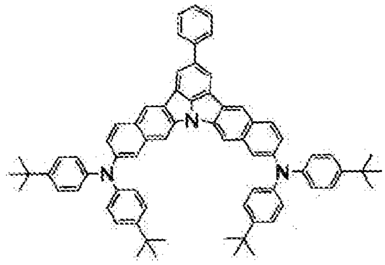

[Formula 394]

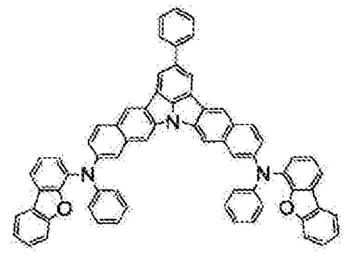

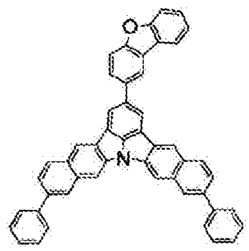

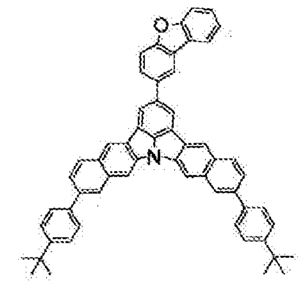

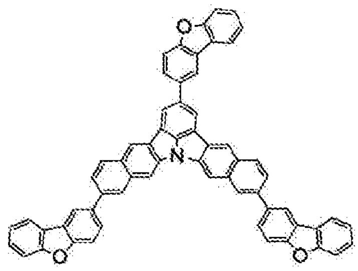

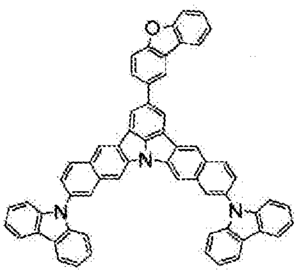

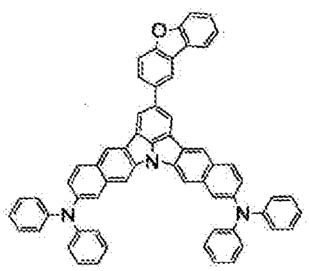

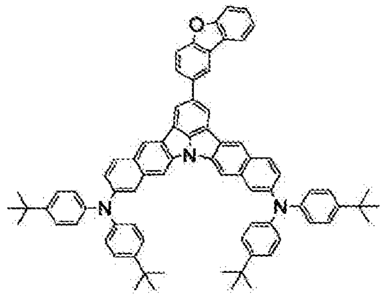

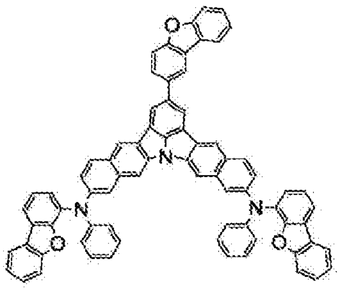

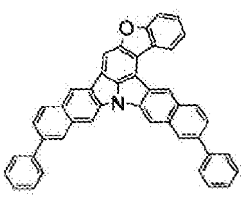

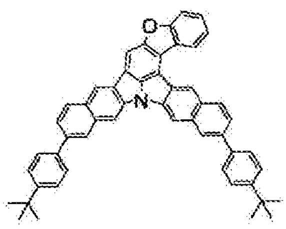

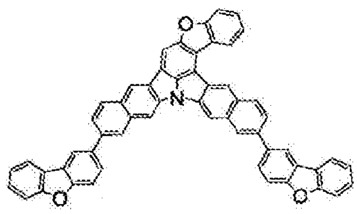

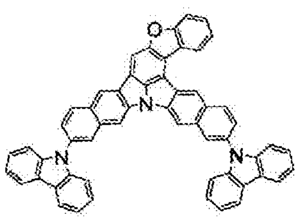

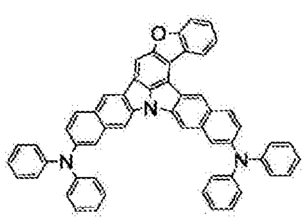

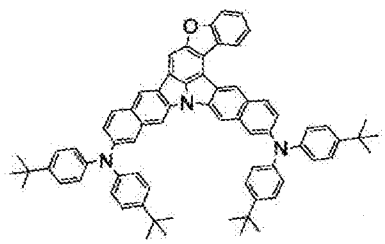

[Formula 395]

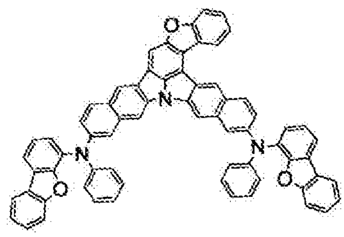

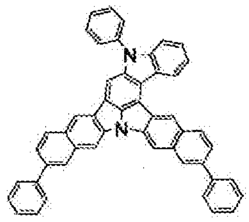

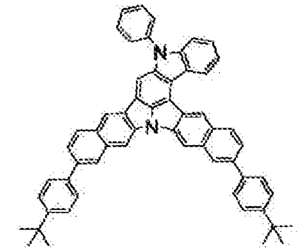

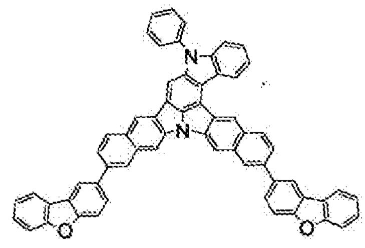

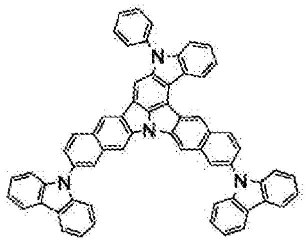

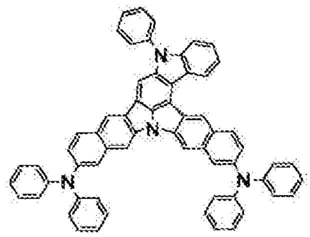

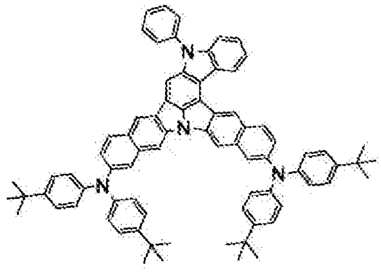

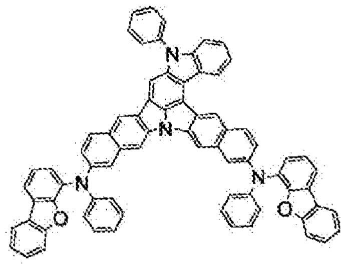

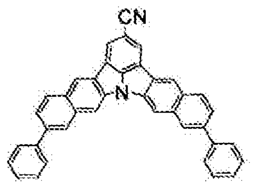

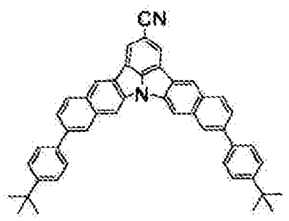

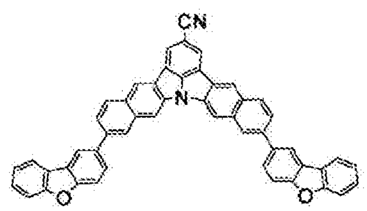

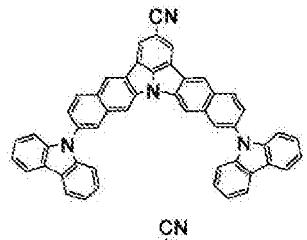

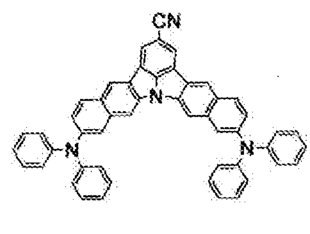

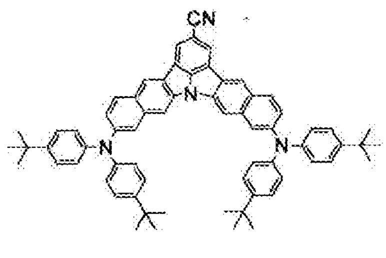

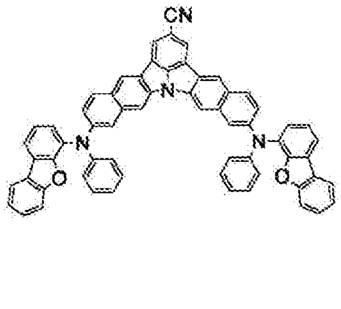

[Formula 396]

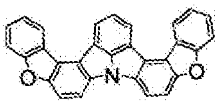
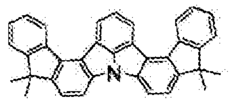
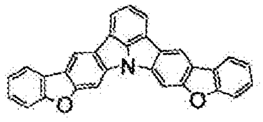
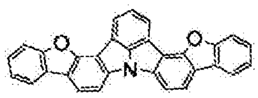
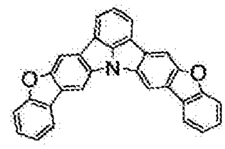
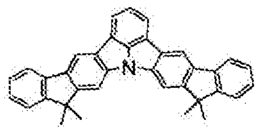
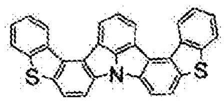
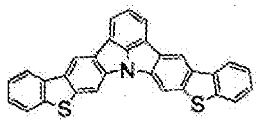
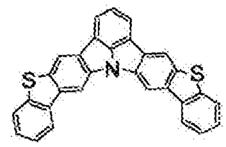
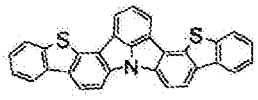
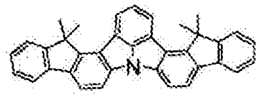
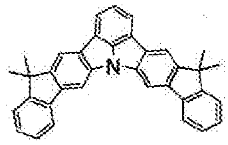
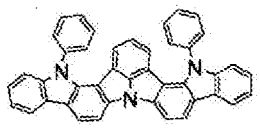
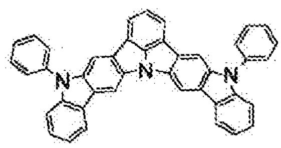
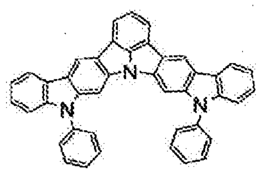
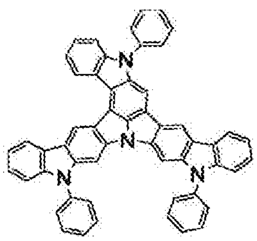
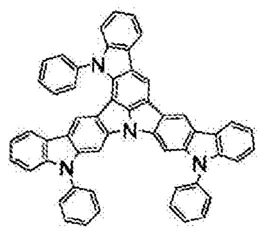
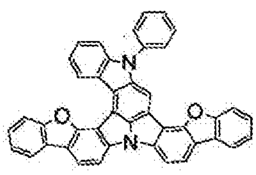
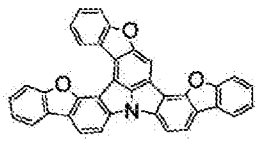
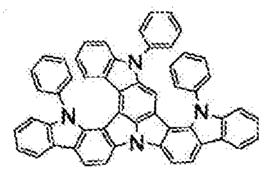
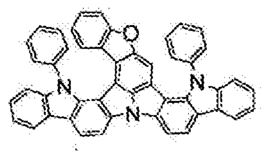
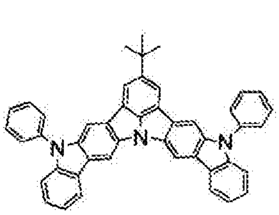
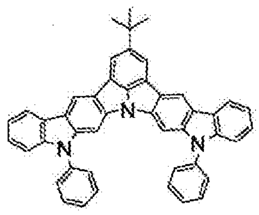

[Formula 397]

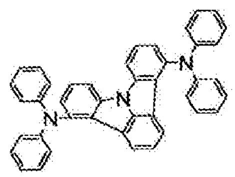
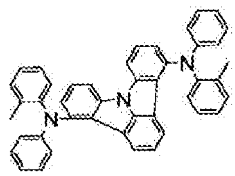
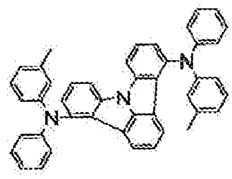
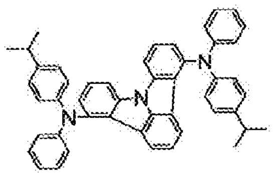
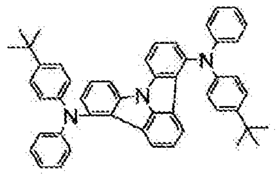
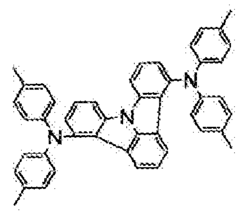
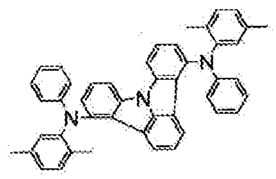
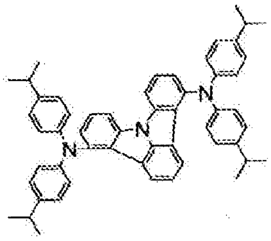
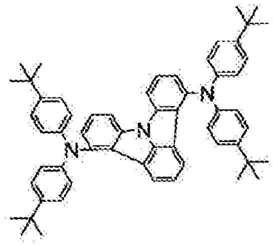
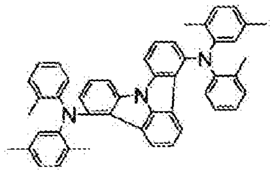
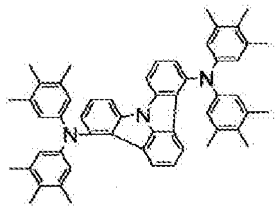
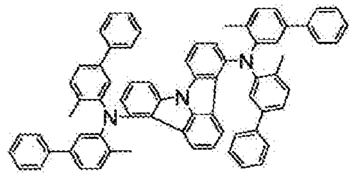
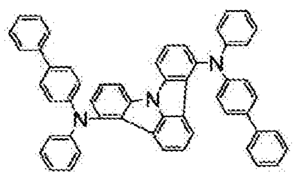
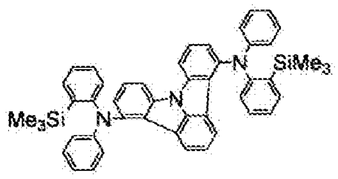
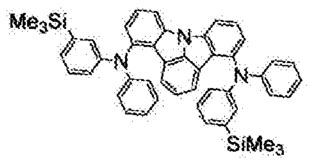
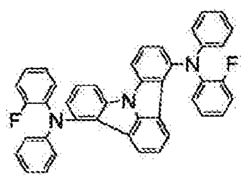
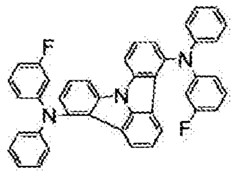
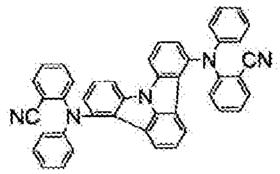
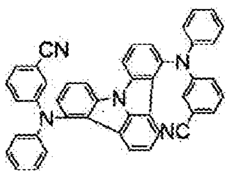
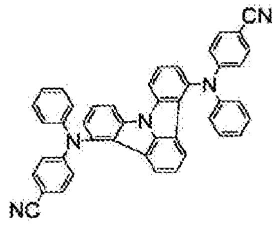
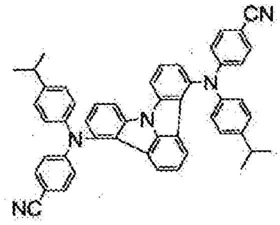

[Formula 398]

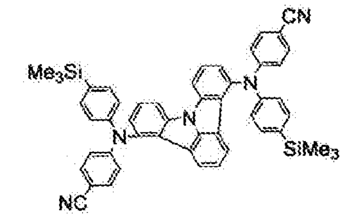
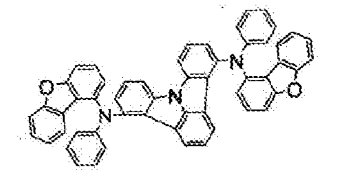
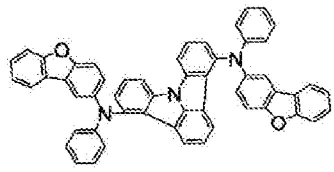
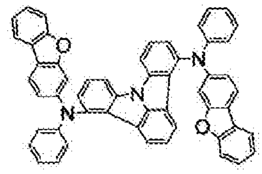
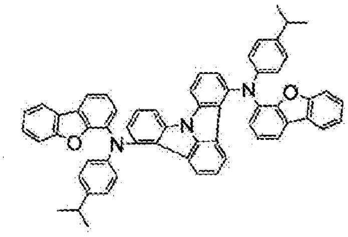
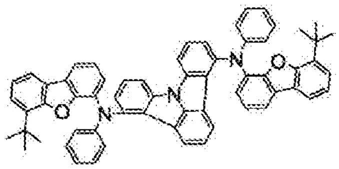
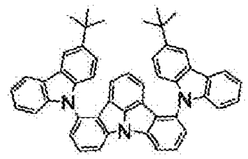
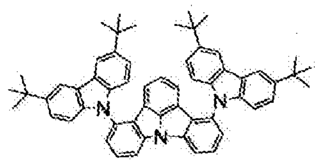
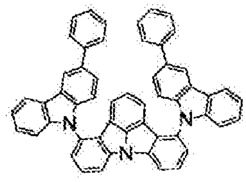
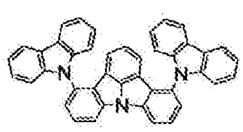
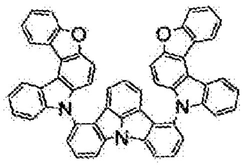
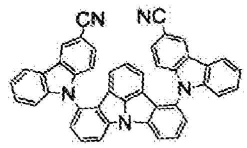
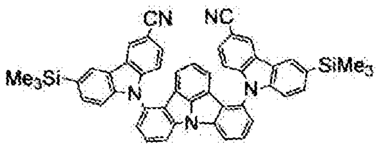
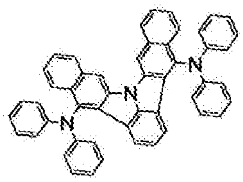
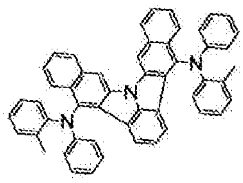
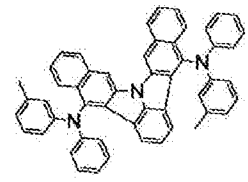
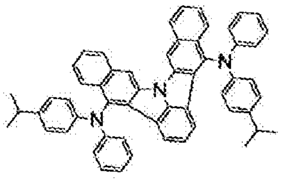
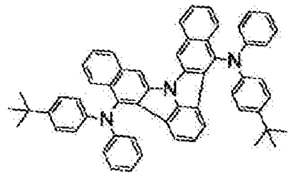
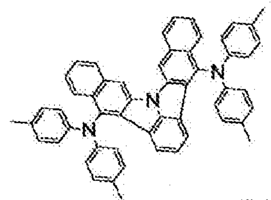
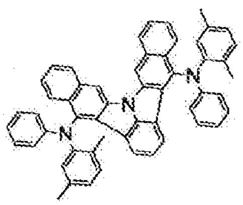
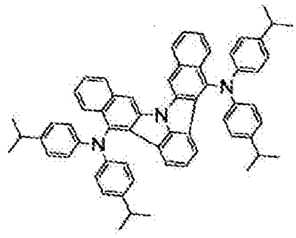
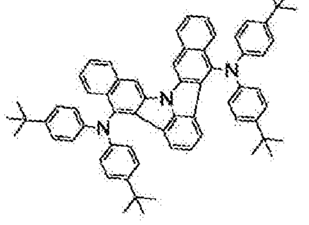

[Formula 399]

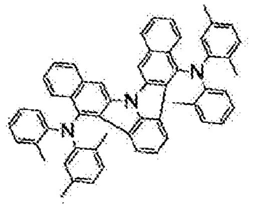
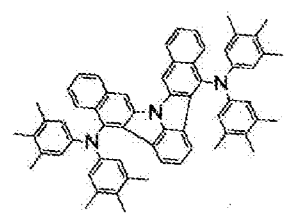
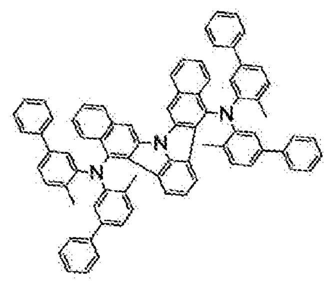

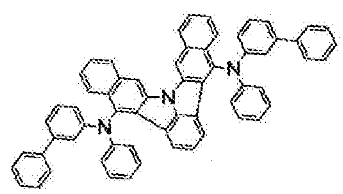
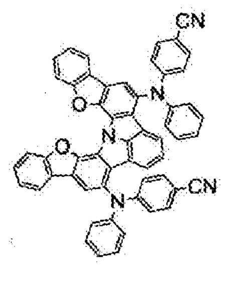
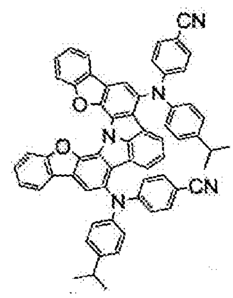

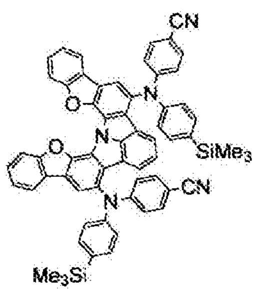
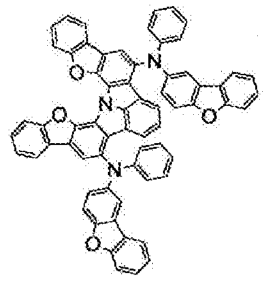
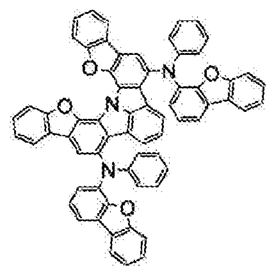

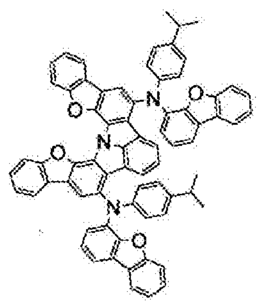
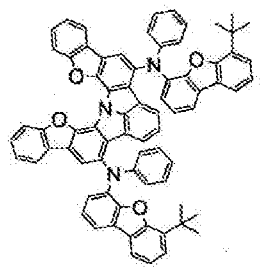
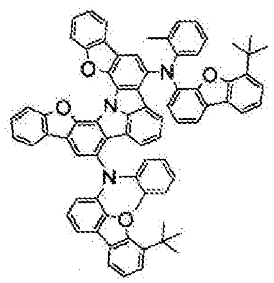

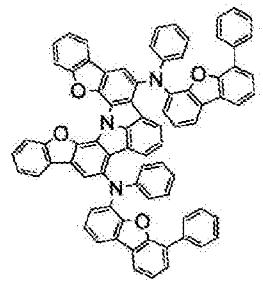
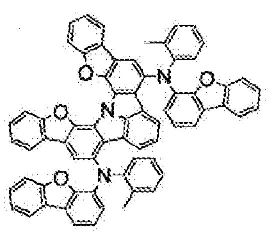

[Formula 400]

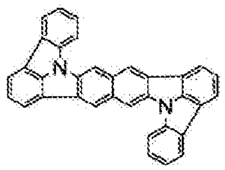
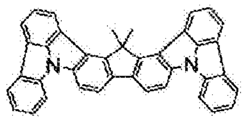
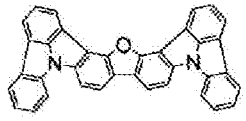
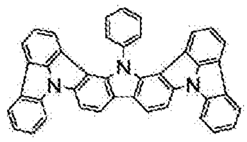
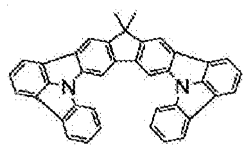
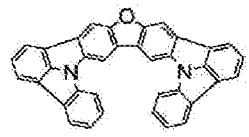
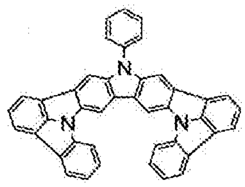
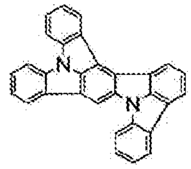
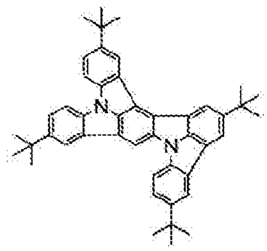
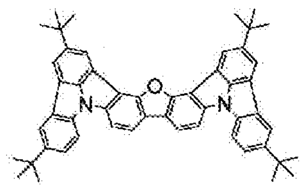

Compound Represented by Formula (5)

The compound represented by the formula (5) will be described. The compound represented by the formula (5) corresponds to a compound represented by the formula (41-3).

[Formula 401]

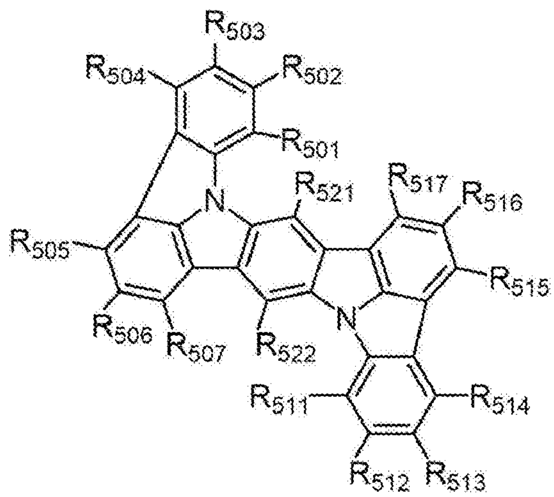

(5)

In the formula (5):

at least one combination of adjacent two or more of $R_{501}$ to $R_{507}$ and $R_{511}$ to $R_{517}$ are mutually bonded to form a substituted or unsubstituted monocyclic ring, mutually bonded to form a substituted or unsubstituted fused ring, or

not mutually bonded;

$R_{501}$ to $R_{507}$ and $R_{511}$ to $R_{517}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group Represented by -O-($R_{904}$), a group represented by -S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms; and

$R_{521}$ and $R_{522}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group Represented by -O-($R_{904}$), a group represented by -S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms.

"A combination of adjacent two or more of $R_{501}$ to $R_{507}$ and $R_{511}$ to $R_{517}$" refers to, for instance, a combination of $R_{501}$ and $R_{502}$, a combination of $R_{502}$ and $R_{503}$, a combination of $R_{503}$ and $R_{504}$, a combination of $R_{505}$ and $R_{506}$, a combination of $R_{506}$ and $R_{507}$, and a combination of $R_{501}$, $R_{502}$, and $R_{503}$.

In an exemplary embodiment, at least one, preferably two of $R_{501}$ to $R_{507}$ and $R_{511}$ to $R_{517}$ are groups represented by -N($R_{906}$)($R_{907}$).

In an exemplary embodiment, $R_{501}$ to $R_{507}$ and $R_{511}$ to $R_{517}$ are each independently a hydrogen atom, a substituted or unsubstituted aryl group having 6 to 50 ring carbon atoms, or a substituted or unsubstituted heterocyclic group having 5 to 50 ring atoms.

In an exemplary embodiment, a compound represented by the formula (5) is a compound represented by a formula (52) below.

[Formula 402]

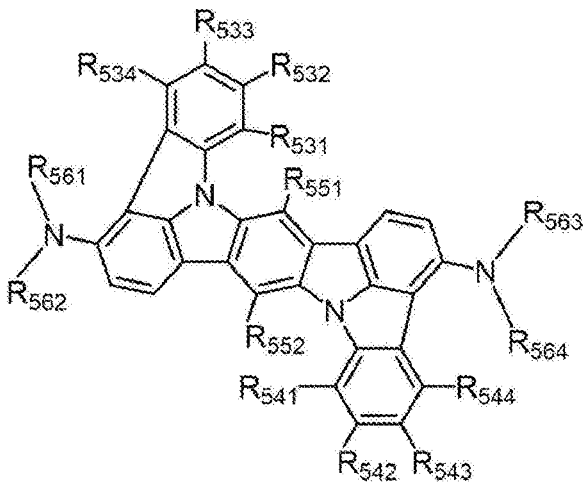

(52)

In the formula (52):

at least one combination of adjacent two or more of $R_{531}$ to $R_{534}$ and $R_{541}$ to $R_{544}$ are mutually bonded to form a substituted or unsubstituted monocyclic ring, mutually bonded to form a substituted or unsubstituted fused ring, or not mutually bonded;

$R_{531}$ to $R_{534}$ and $R_{541}$ to $R_{544}$ forming neither the substituted or unsubstituted monocyclic ring nor the substituted or unsubstituted fused ring, and $R_{551}$ and $R_{552}$ are each independently a hydrogen atom, a substituted or unsubstituted aryl group having 6 to 50 ring carbon atoms, or a substituted or unsubstituted heterocyclic group having 5 to 50 ring atoms; and

$R_{561}$ to $R_{564}$ are each independently a substituted or unsubstituted aryl group having 6 to 50 ring carbon atoms, or a substituted or unsubstituted heterocyclic group having 5 to 50 ring atoms.

In an exemplary embodiment, a compound represented by the formula (5) is a compound represented by a formula (53) below.

[Formula 403]

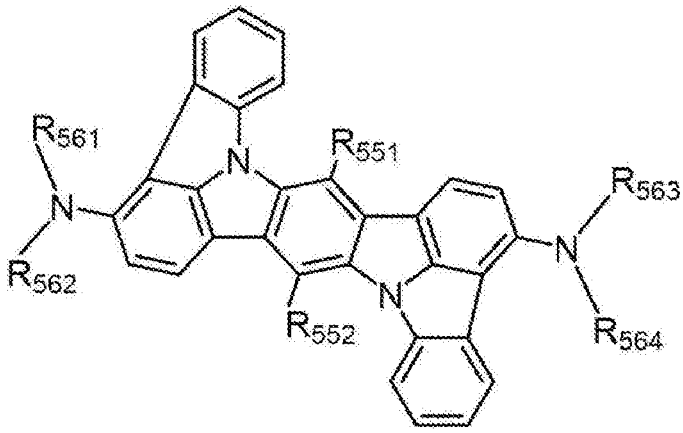

(53)

In the formula (53), $R_{551}$, $R_{552}$ and $R_{561}$ to $R_{564}$ each independently represent the same as $R_{551}$, $R_{552}$ and $R_{561}$ to $R_{564}$ in the formula (52).

In an exemplary embodiment, $R_{561}$ to $R_{564}$ in the formulae (52) and (53) are each independently a substituted or unsubstituted aryl group having 6 to 50 ring carbon atoms (preferably a phenyl group).

In an exemplary embodiment, $R_{521}$ and $R_{522}$ in the formula (5) and $R_{551}$ and $R_{552}$ in the formulae (52) and (53) are hydrogen atoms.

In an exemplary embodiment, the substituent for "substituted or unsubstituted" in the formulae (5), (52) and (53) is a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms.

Specific examples of a compound represented by the formula (5) include compounds shown below.

[Formula 404]

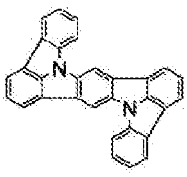
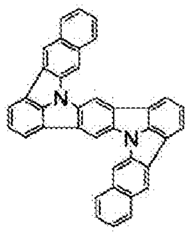
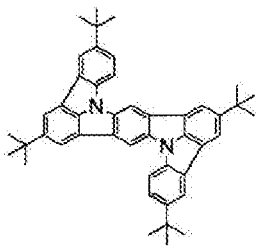
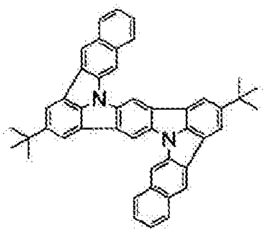
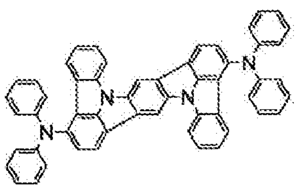
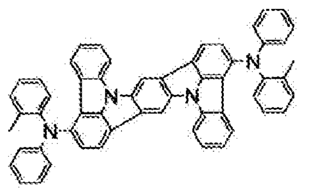
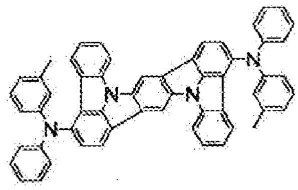
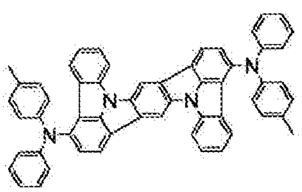
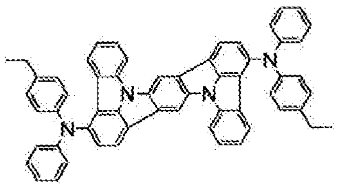
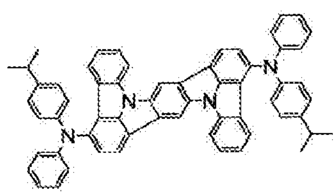

[Formula 405]

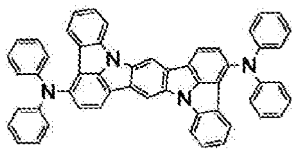
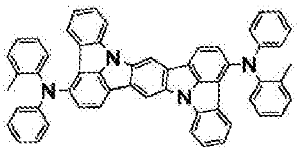
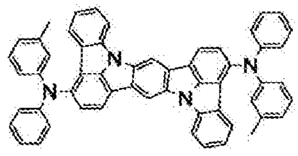
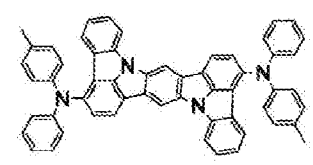
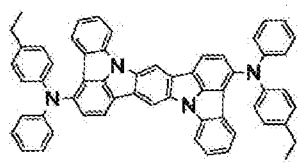
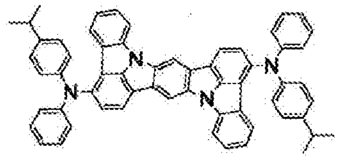
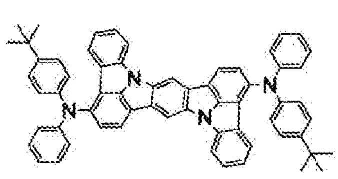
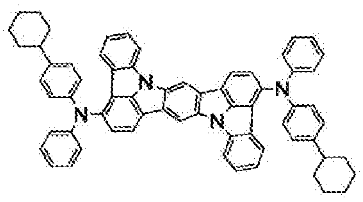
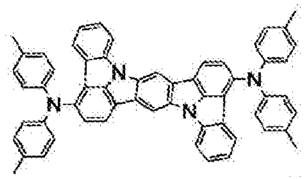
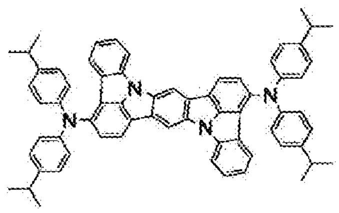
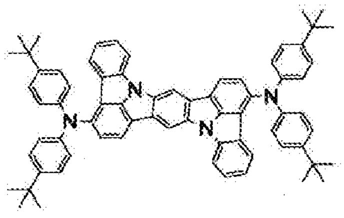
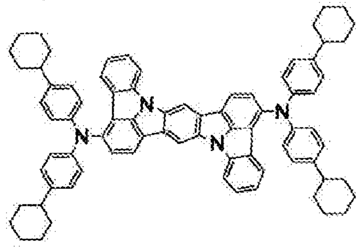
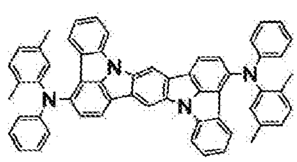
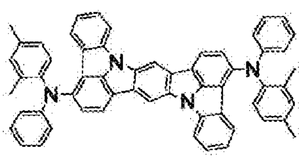
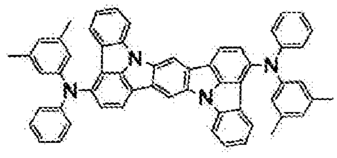
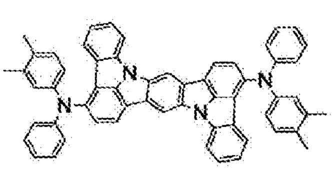
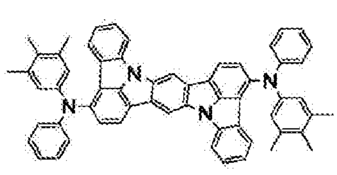
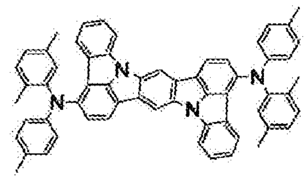
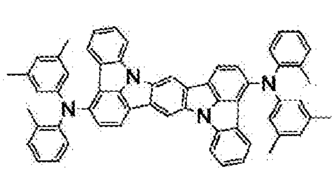
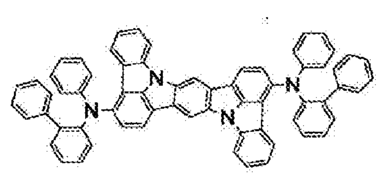
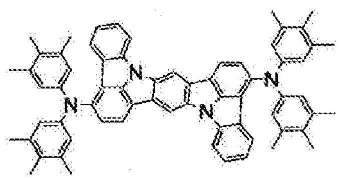
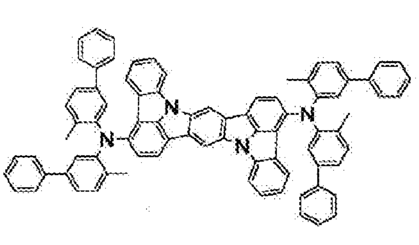
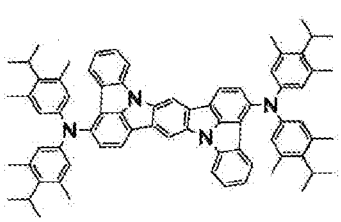
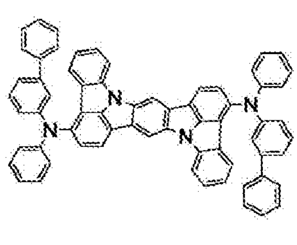

[Formula 406]

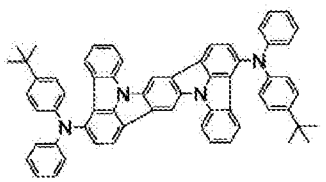
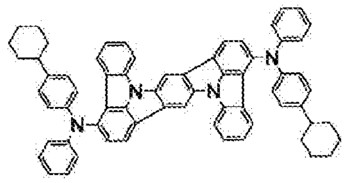
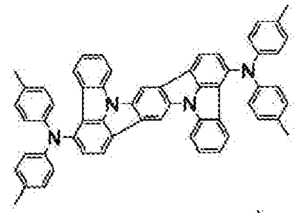
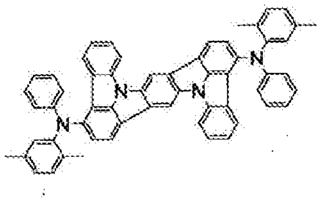
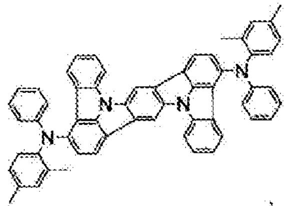
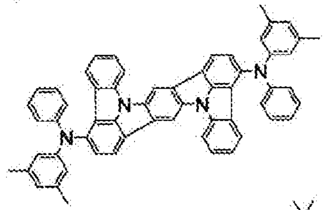
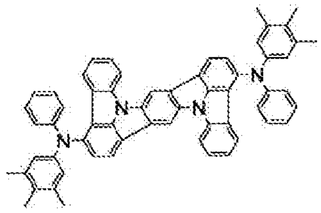
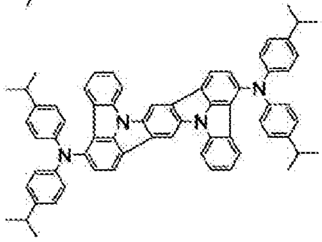
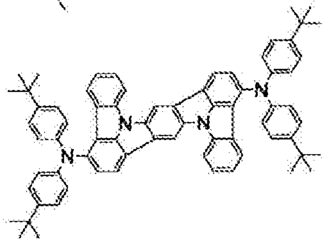
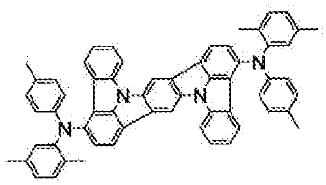
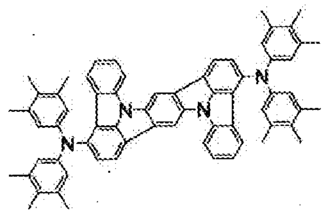
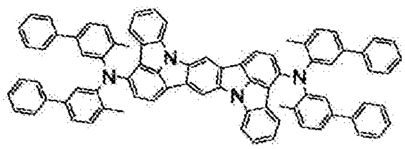
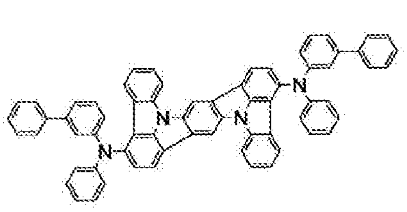
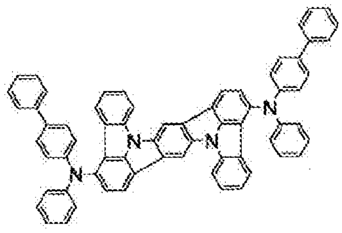
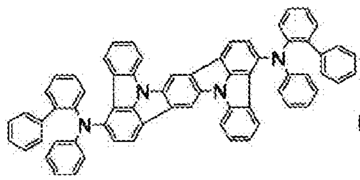
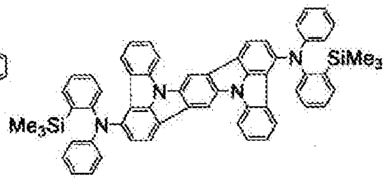
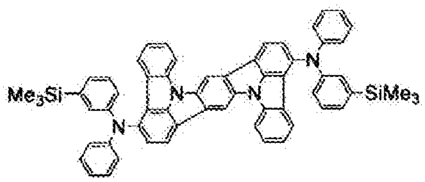
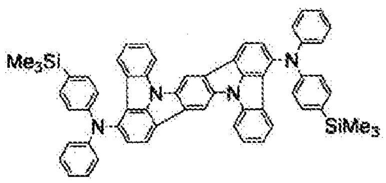
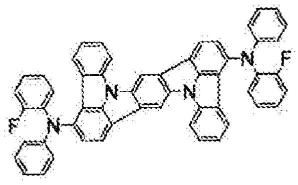
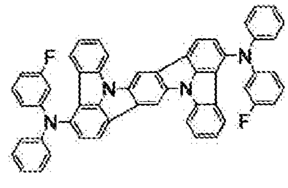

[Formula 407]

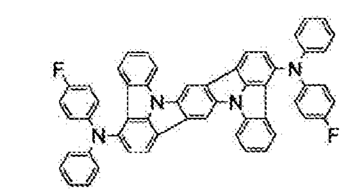
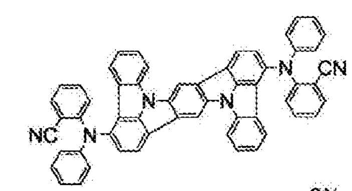
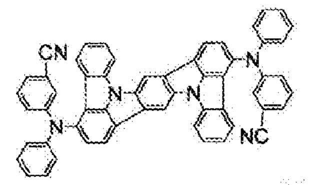
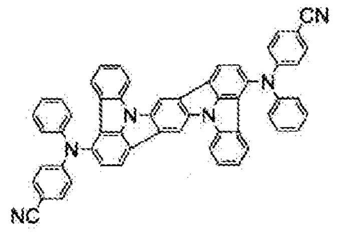
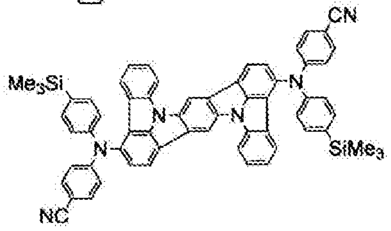
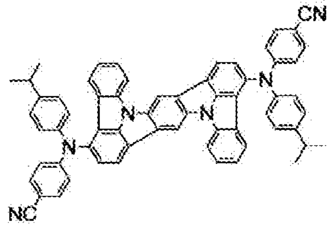
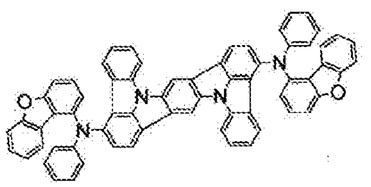
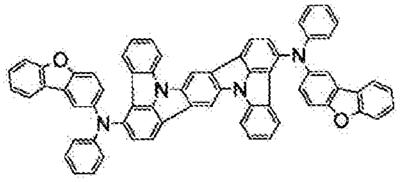
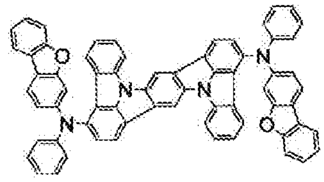
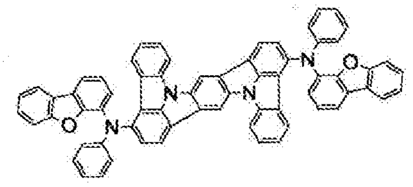
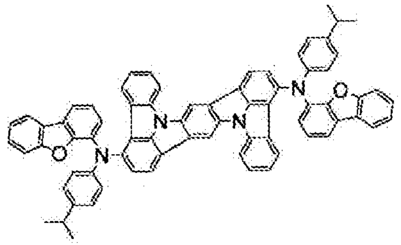
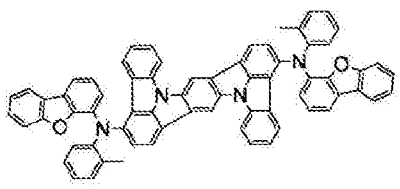
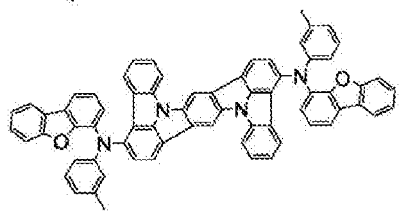
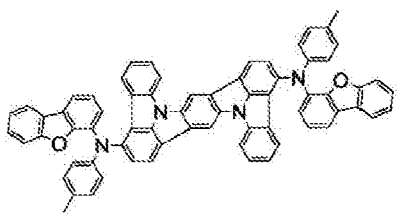
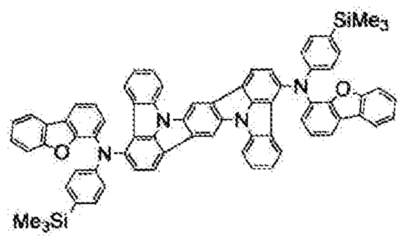
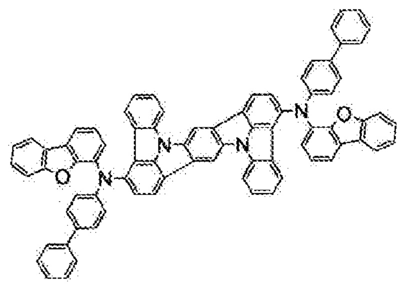
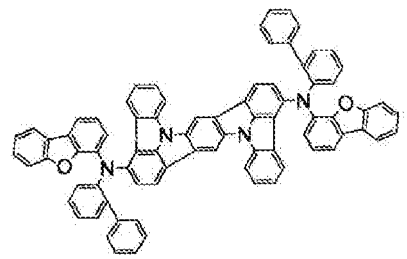

[Formula 408]

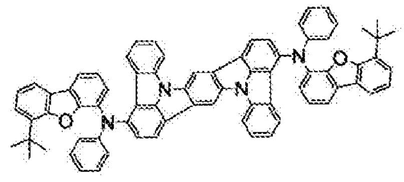
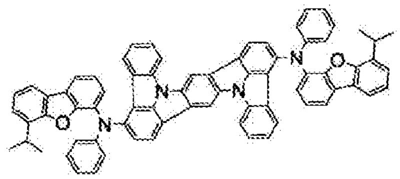
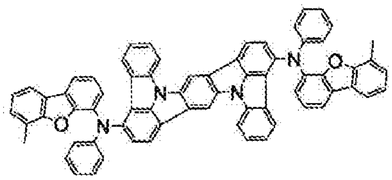
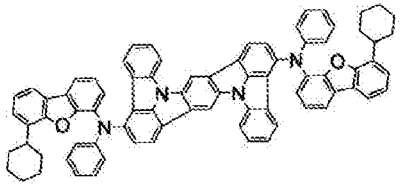
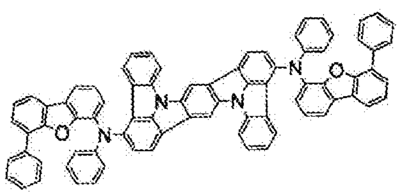
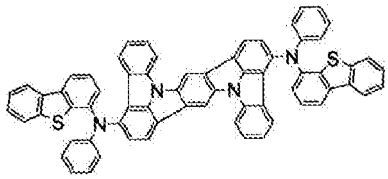
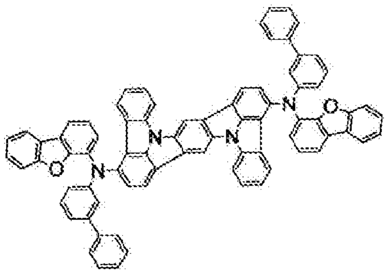
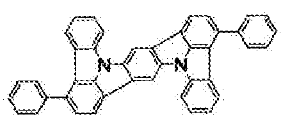
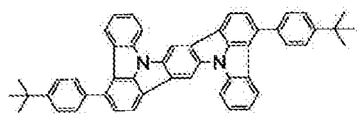
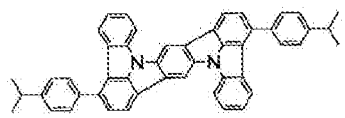
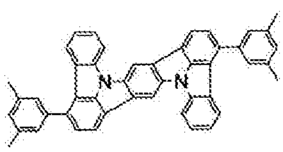
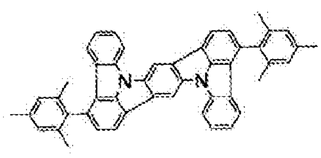
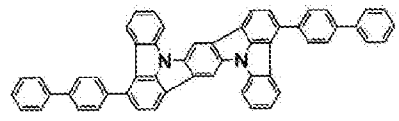
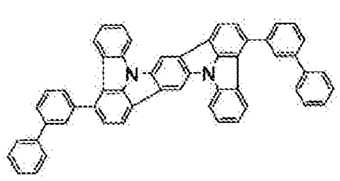
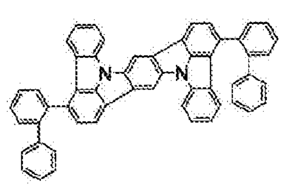
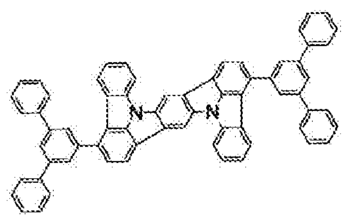
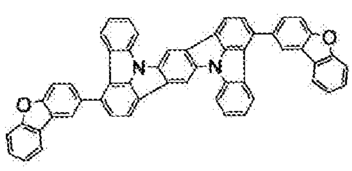
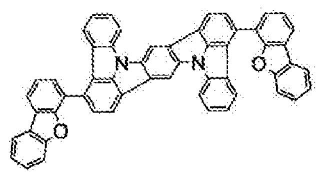
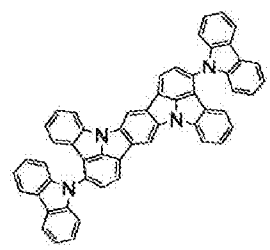

[Formula 409]

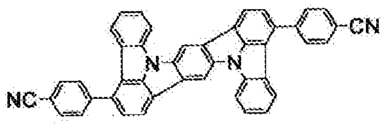

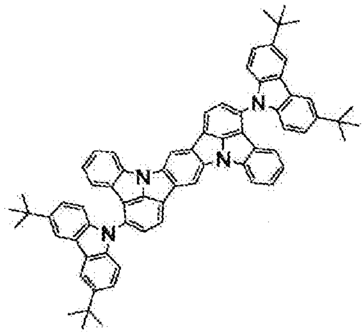

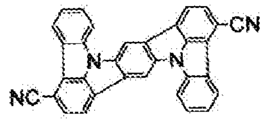

[Formula 410]

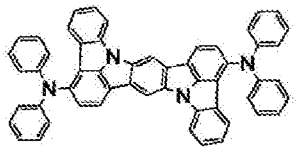
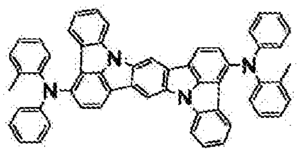
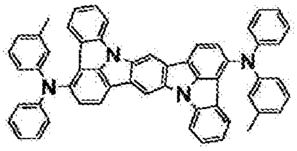
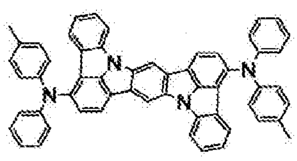
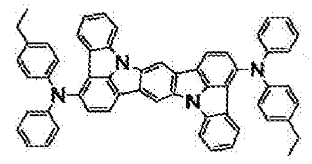
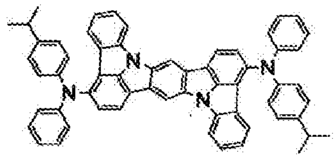
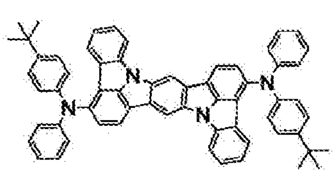
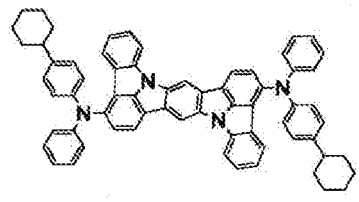
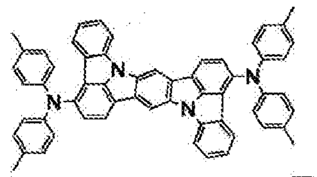
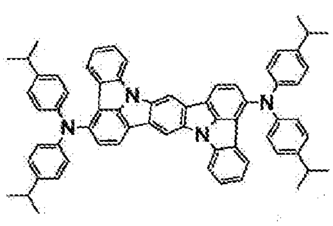
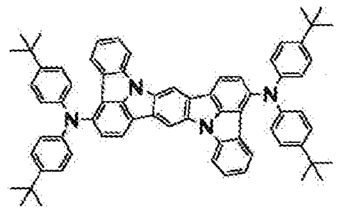
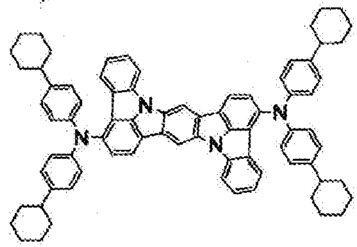
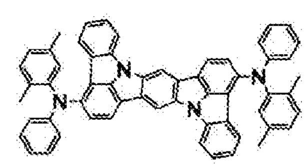
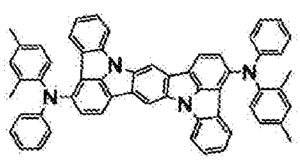
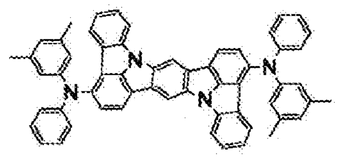
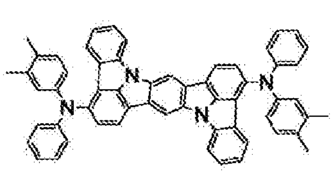
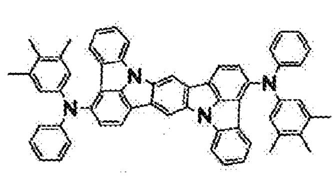
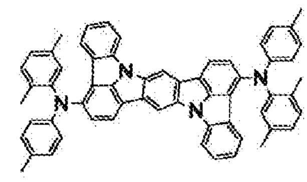
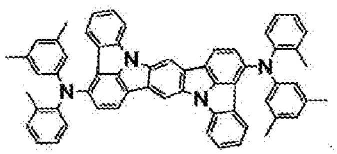
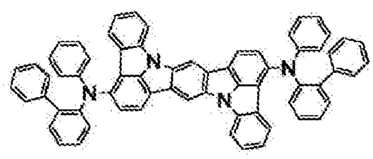
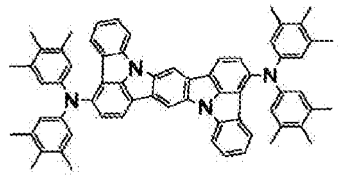
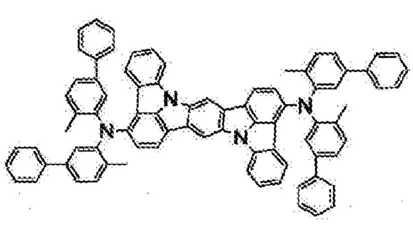
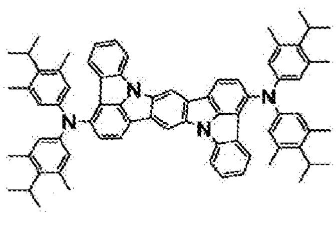
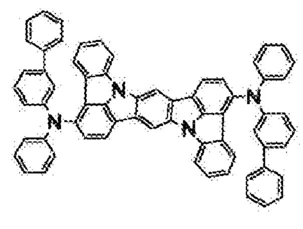

[Formula 411]

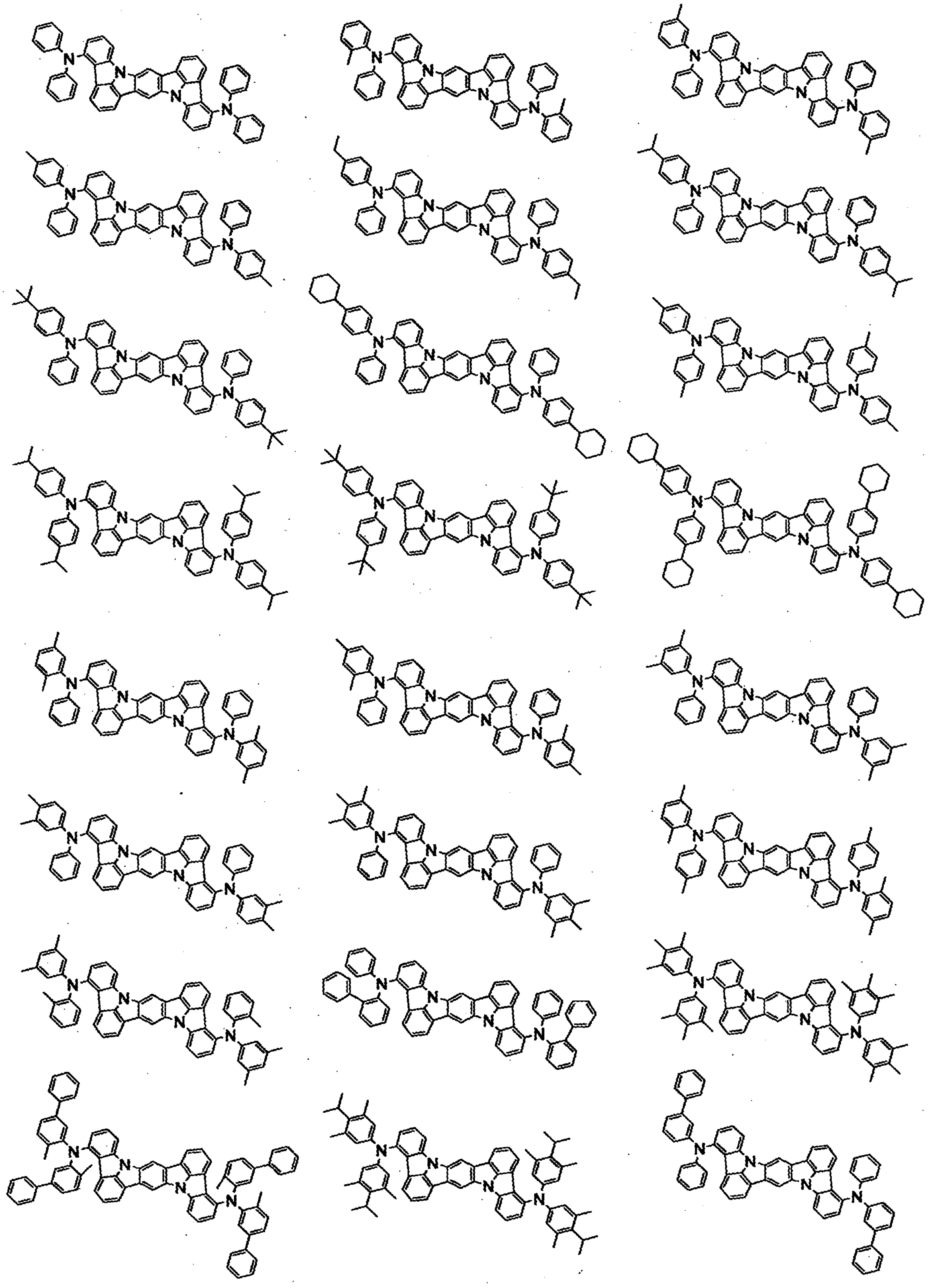

[Formula 412]

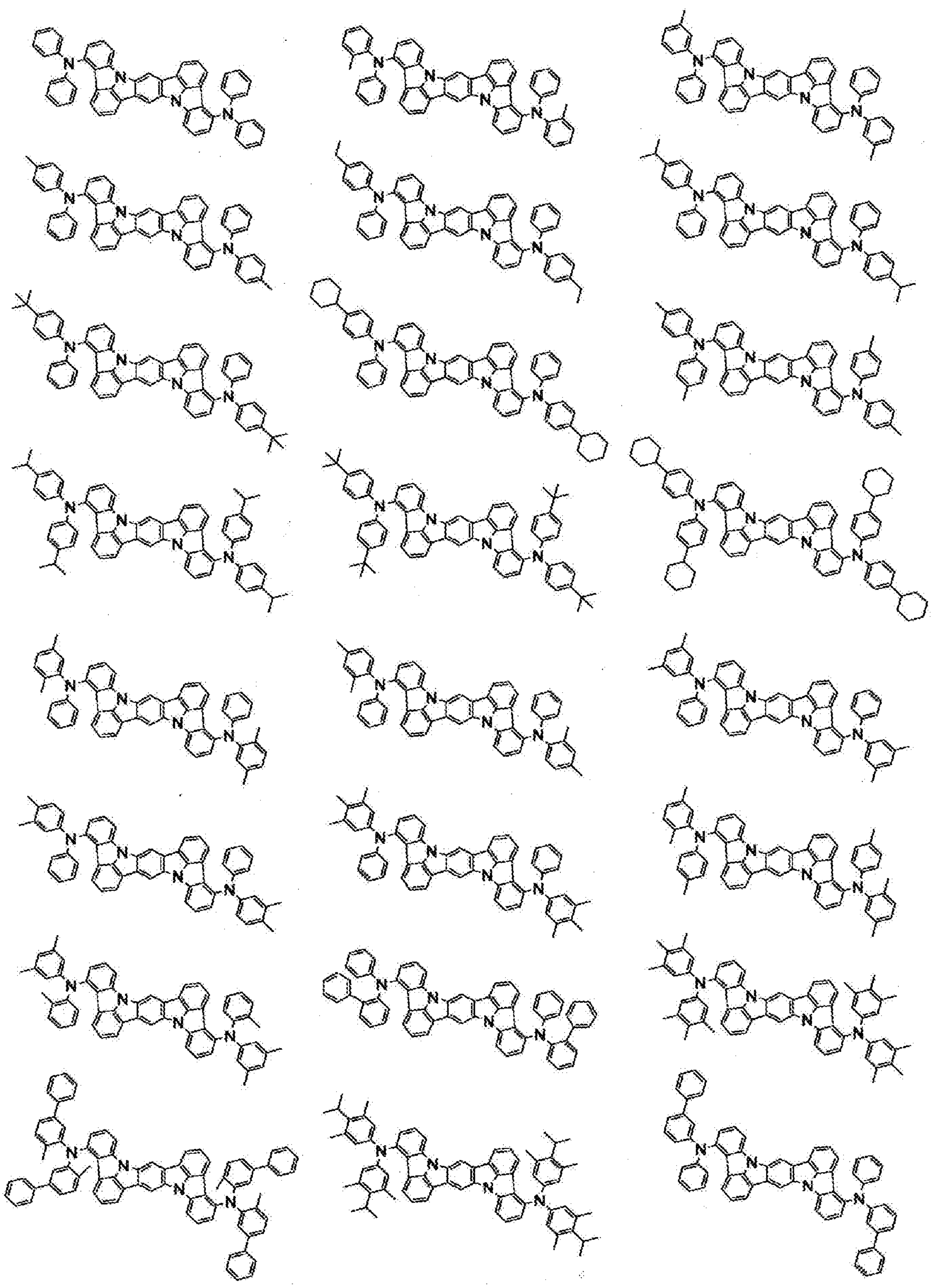

[Formula 413]

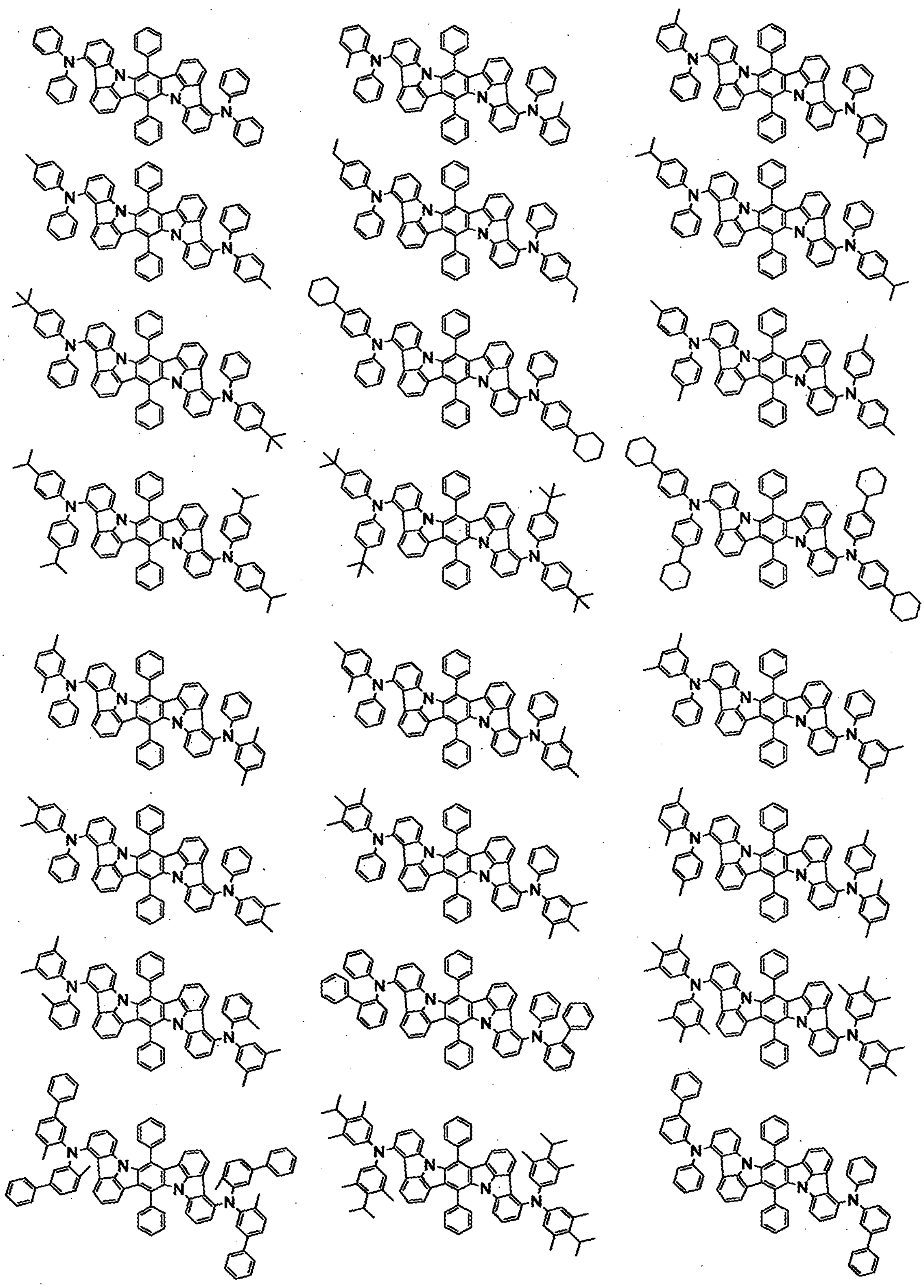

[Formula 414]

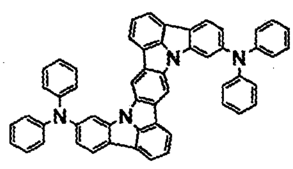
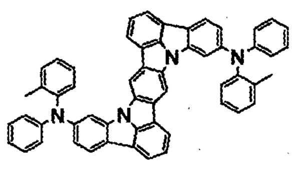
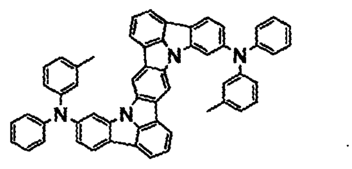
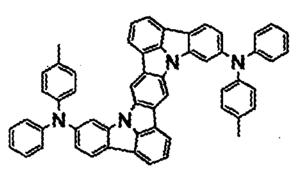
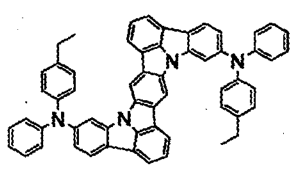
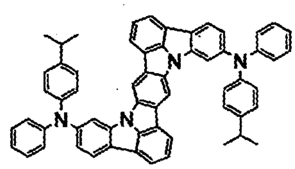
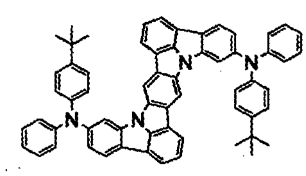
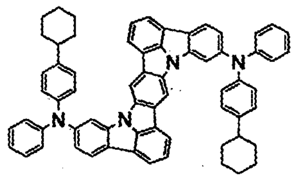
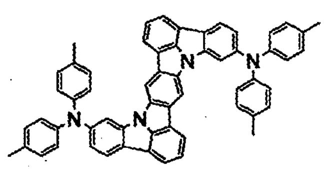
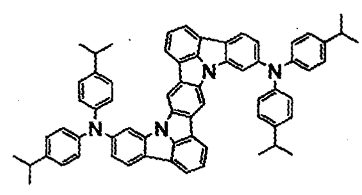
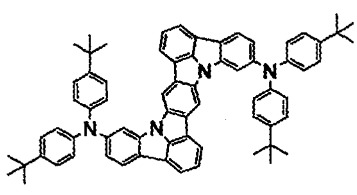
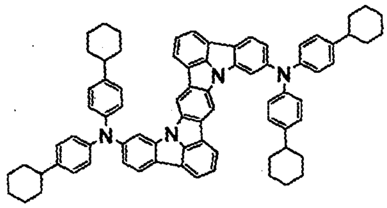
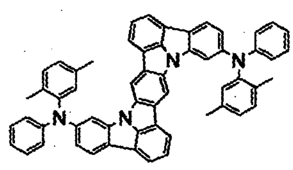
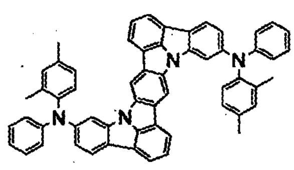
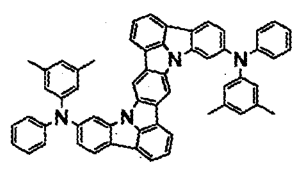
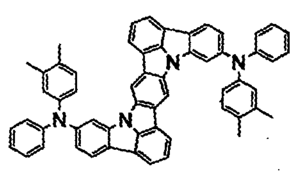
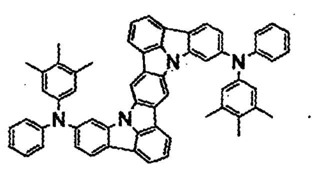
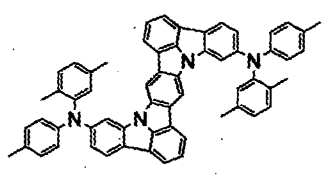
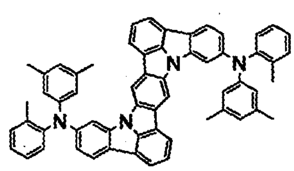
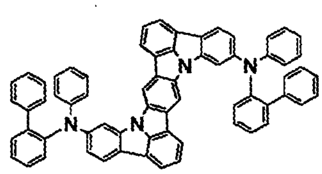
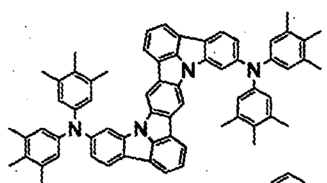
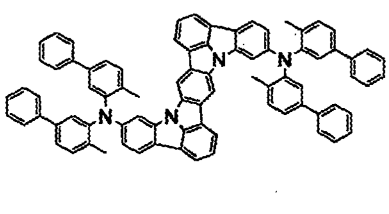
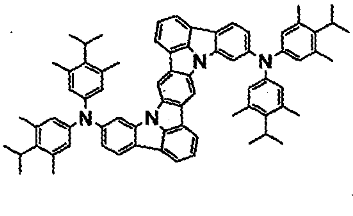
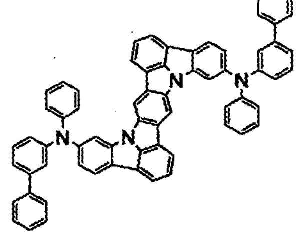

[Formula 415]

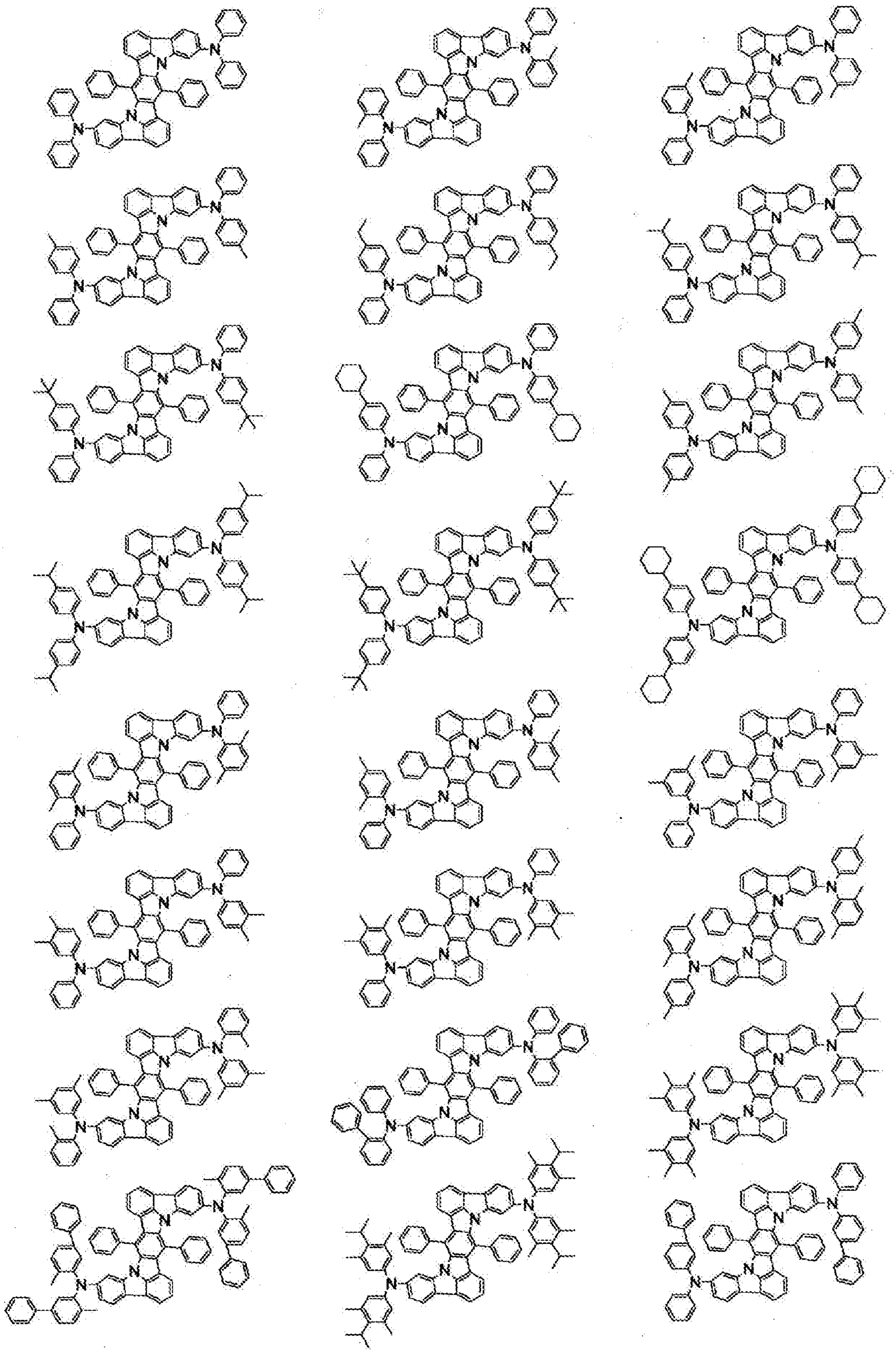

[Formula 416]

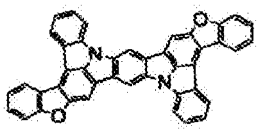
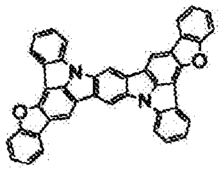
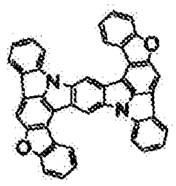
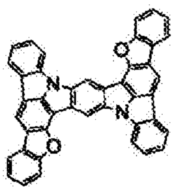

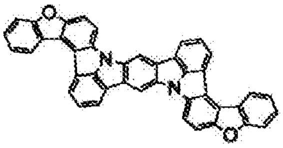
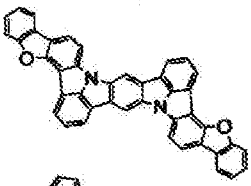
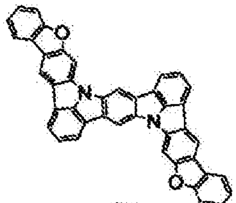
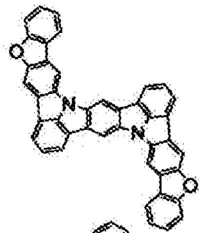

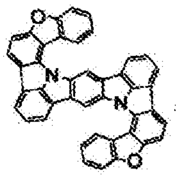
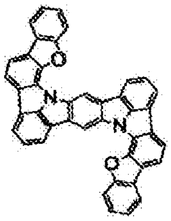
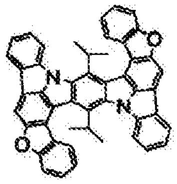
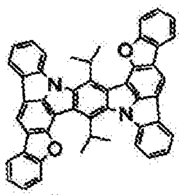

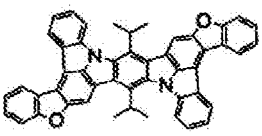
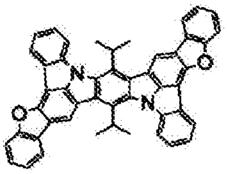
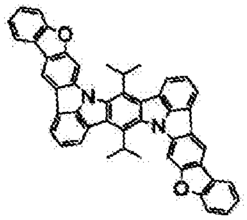
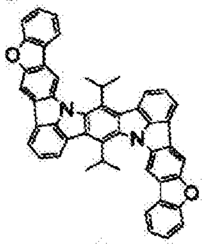

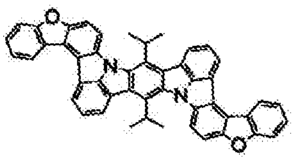
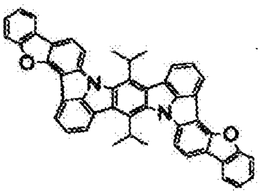
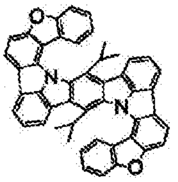
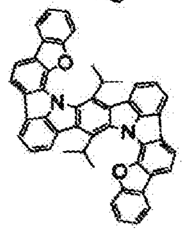

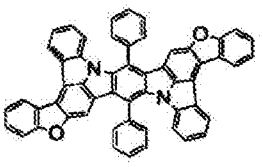
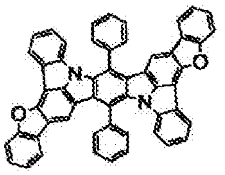
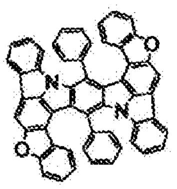
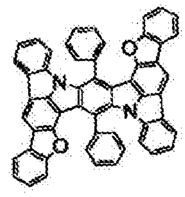

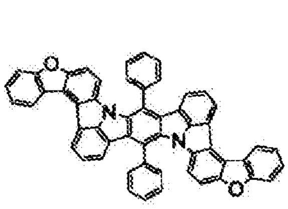
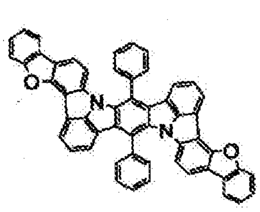
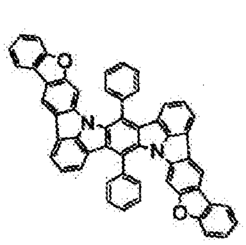
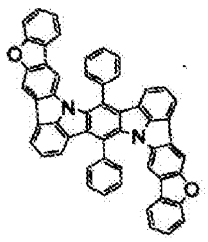

[Formula 417]

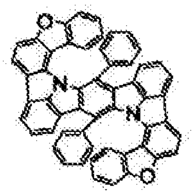
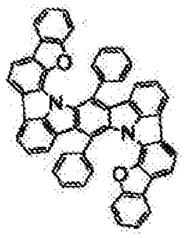
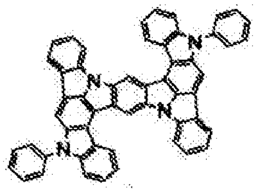
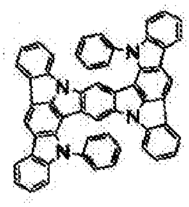
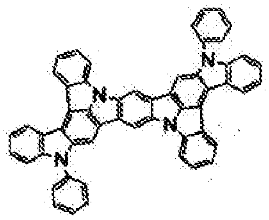
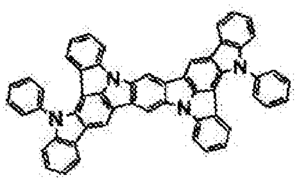
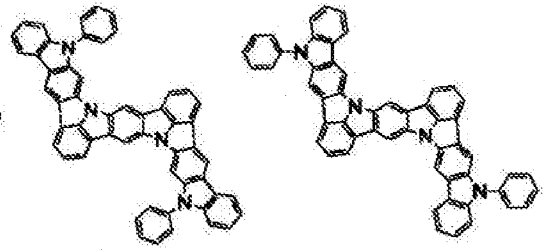
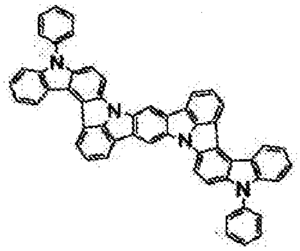
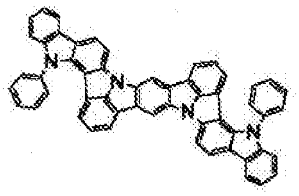
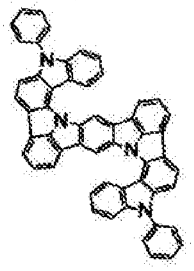
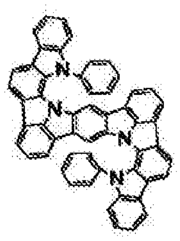
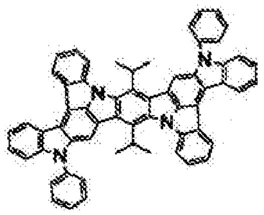
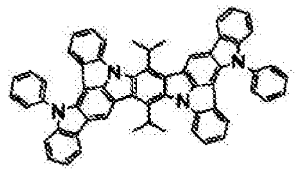
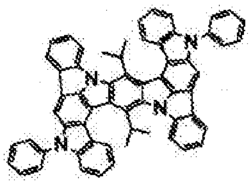
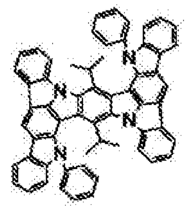
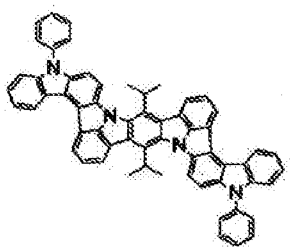
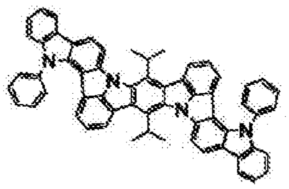
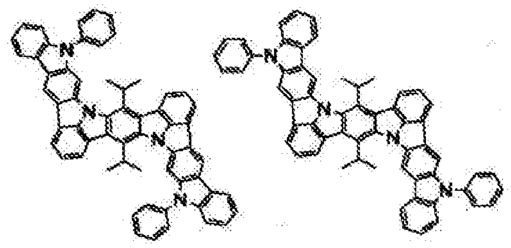
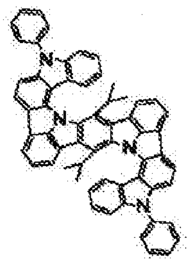
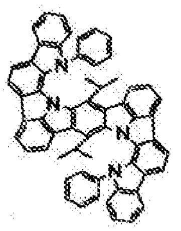
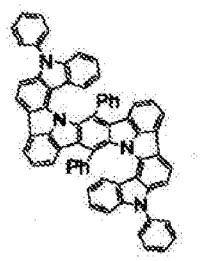
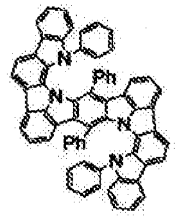

In the formulae, Ph represents a phenyl group.

[Formula 418]

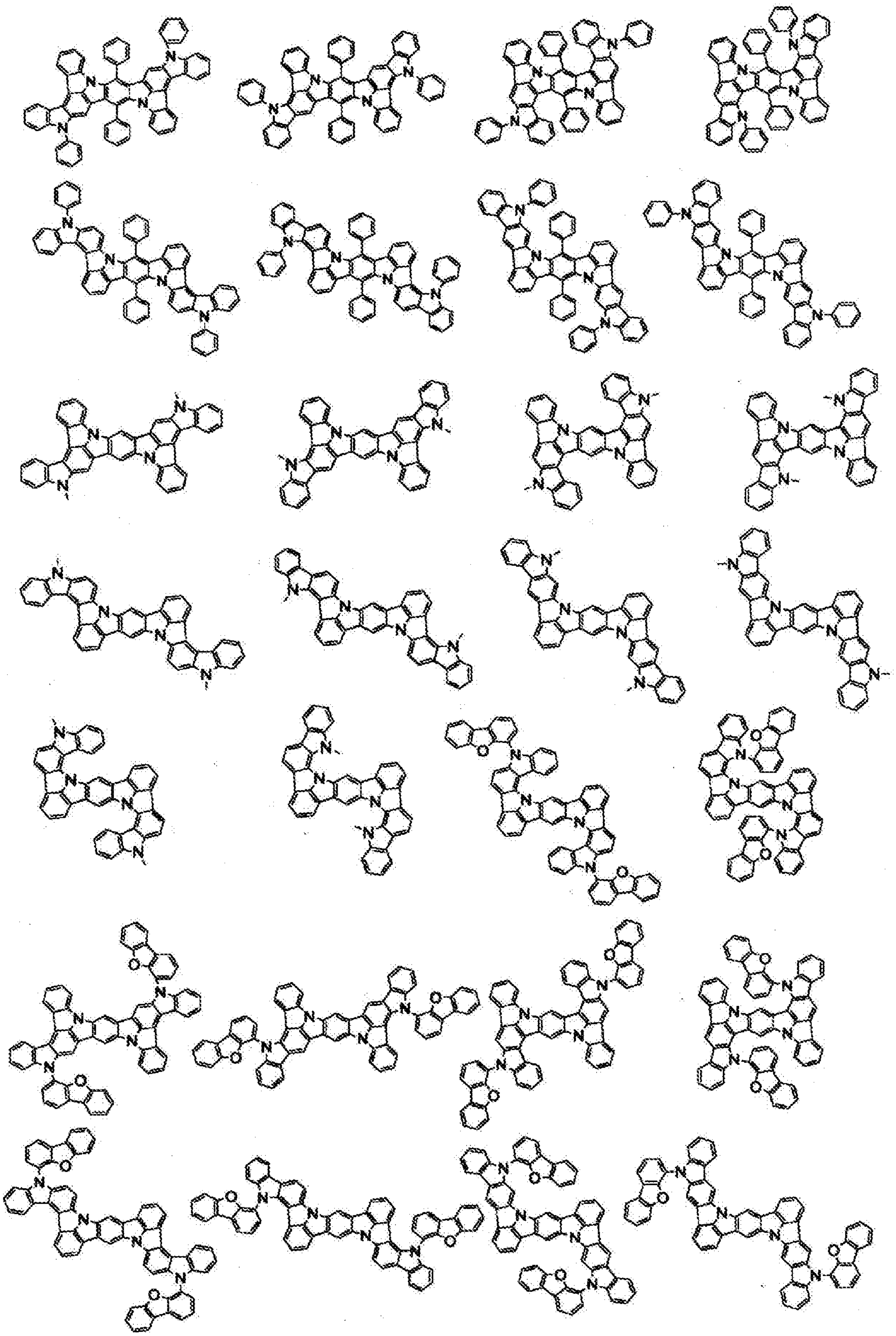

[Formula 419]

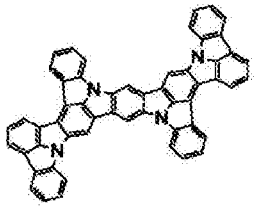
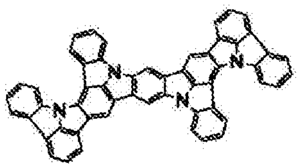
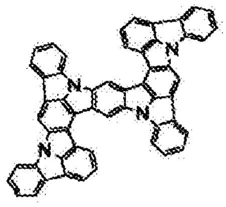
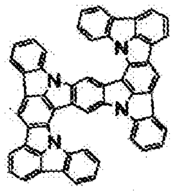
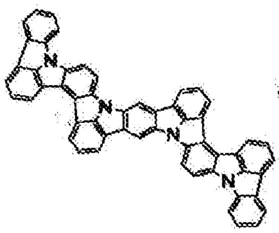
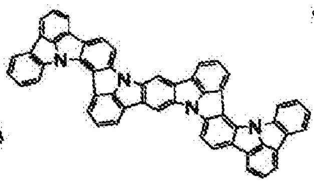
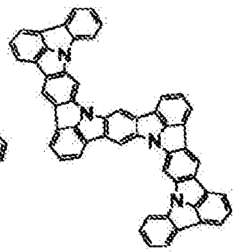
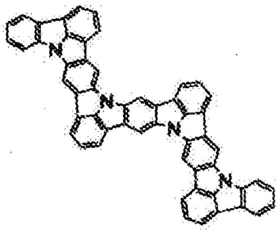
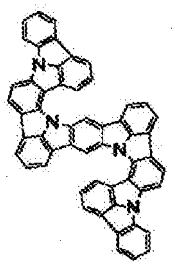
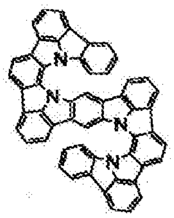
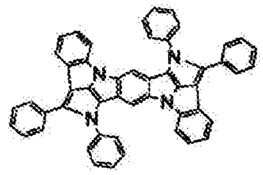
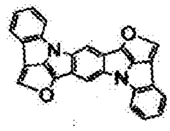
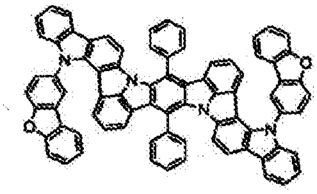
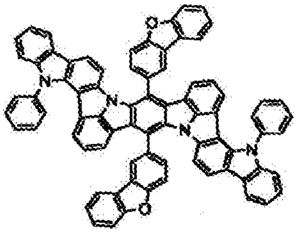
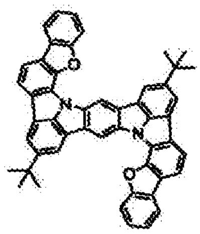
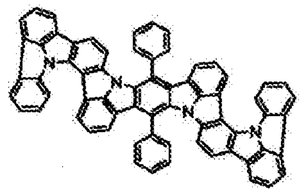
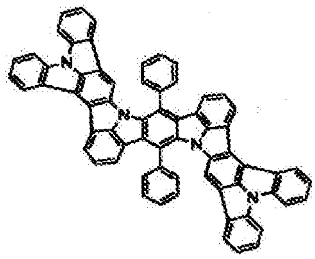

[Formula 420]

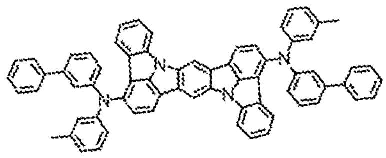
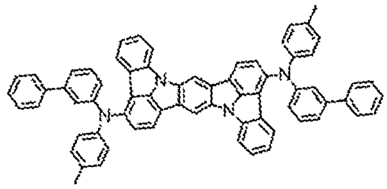
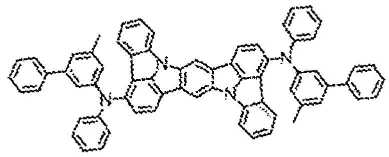

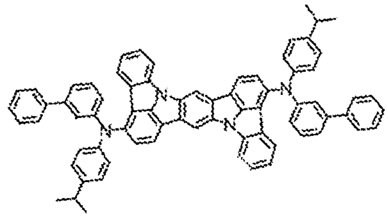
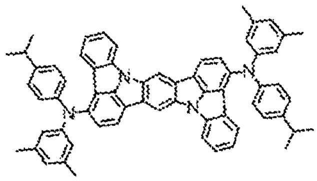
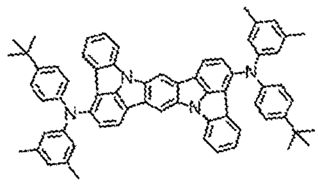

Compound Represented by Formula (6)

A compound represented by the formula (6) will be described.

[Formula 421]

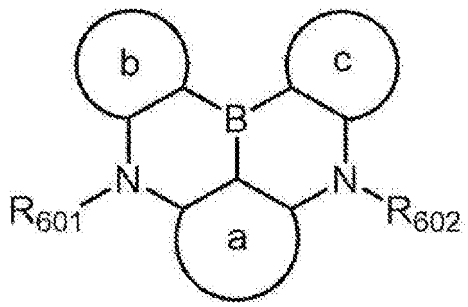

(6)

In the formula (6):

a ring, b ring and c ring are each independently a substituted or unsubstituted aromatic hydrocarbon ring having 6 to 50 ring carbon atoms, or a substituted or unsubstituted heterocycle having 5 to 50 ring atoms;

$R_{601}$ and $R_{602}$ are each independently bonded to the a ring, b ring or c ring to form a substituted or unsubstituted heterocycle, or not bonded thereto to form no substituted or unsubstituted heterocycle; and

$R_{601}$ and $R_{602}$ not forming the substituted or unsubstituted heterocycle are each independently a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms.

The a ring, b ring and c ring are each a ring (a substituted or unsubstituted aromatic hydrocarbon ring having 6 to 50 ring carbon atoms, or a substituted or unsubstituted heterocycle having 5 to 50 ring atoms) fused with a fused bicyclic structure formed of a boron atom and two nitrogen atoms at the center of the formula (6).

The "aromatic hydrocarbon ring" for the a, b, and c rings has the same structure as a compound formed by introducing a hydrogen atom to the "aryl group" described above.

Ring atoms of the "aromatic hydrocarbon ring" for the a ring include three carbon atoms on the fused bicyclic structure at the center of the formula (6).

Ring atoms of the "aromatic hydrocarbon ring" for the b ring and c ring include two carbon atoms on the fused bicyclic structure at the center of the formula (6).

Specific examples of the "substituted or unsubstituted aromatic hydrocarbon ring having 6 to 50 ring carbon atoms" include a compound formed by introducing a hydrogen atom to the "aryl group" described in the specific example

group G1.

The "heterocycle" for the a, b, and c rings has the same structure as a compound formed by introducing a hydrogen atom to the "heterocyclic group" described above.

Ring atoms of the "heterocycle" for the a ring include three carbon atoms on the fused bicyclic structure at the center of the formula (6). Ring atoms of the "heterocycle" for the b ring and c ring include two carbon atoms on the fused bicyclic structure at the center of the formula (6). Specific examples of the "substituted or unsubstituted heterocycle having 5 to 50 ring atoms" include a compound formed by introducing a hydrogen atom to the "heterocyclic group" described in the specific example group G2.

$R_{601}$ and $R_{602}$ are optionally each independently bonded with the a ring, b ring, or c ring to form a substituted or unsubstituted heterocycle. The "heterocycle" in this arrangement includes a nitrogen atom on the fused bicyclic structure at the center of the formula (6). The heterocycle in the above arrangement optionally includes a hetero atom other than the nitrogen atom. $R_{601}$ and $R_{602}$ bonded with the a ring, b ring, or c ring specifically means that atoms forming $R_{601}$ and $R_{602}$ are bonded with atoms forming the a ring, b ring, or c ring. For instance, $R_{601}$ may be bonded with the a ring to form a bicyclic (or tri-or-more cyclic) fused nitrogen-containing heterocycle, in which the ring including $R_{601}$ and the a ring are fused. Specific examples of the nitrogen-containing heterocycle include a compound corresponding to a nitrogen-containing bi(or-more)cyclic fused heterocyclic group in the specific example group G2.

The same applies to $R_{601}$ bonded with the b ring, $R_{602}$ bonded with the a ring, and $R_{602}$ bonded with the c ring.

In an exemplary embodiment, the a ring, b ring and c ring in the formula (6) are each independently a substituted or unsubstituted aromatic hydrocarbon ring having 6 to 50 ring carbon atoms.

In an exemplary embodiment, the a ring, b ring and c ring in the formula (6) are each independently a substituted or unsubstituted benzene ring or a substituted or unsubstituted naphthalene ring.

In an exemplary embodiment, $R_{601}$ and $R_{602}$ in the formula (6) are each independently a substituted or unsubstituted aryl group having 6 to 50 ring carbon atoms, or a substituted or unsubstituted heterocyclic group having 5 to 50 ring atoms, preferably a substituted or unsubstituted aryl group having 6 to 50 ring carbon atoms.

In an exemplary embodiment, a compound represented by the formula (6) is a compound represented by a formula (62) below.

[Formula 422]

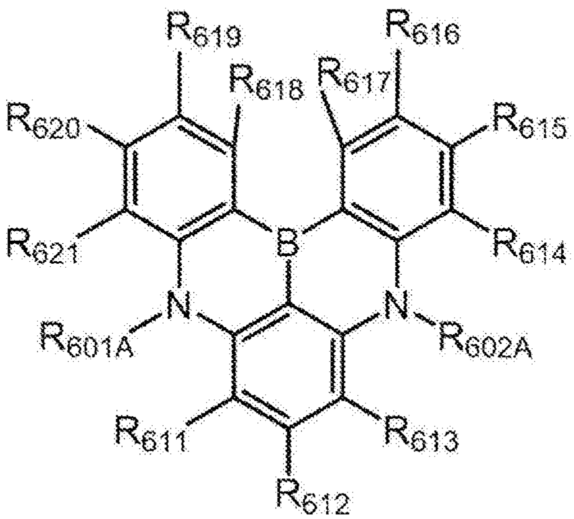

(62)

In the formula (62):

$R_{601A}$ is bonded with at least one of $R_{611}$ or $R_{621}$ to form a substituted or unsubstituted heterocycle, or not bonded therewith to form no substituted or unsubstituted heterocycle;

$R_{602A}$ is bonded with at least one of $R_{613}$ or $R_{614}$ to form a substituted or unsubstituted heterocycle, or not bonded therewith to form no substituted or unsubstituted heterocycle;

$R_{601A}$ and $R_{602A}$ not forming the substituted or unsubstituted heterocycle are each independently a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

at least one combination of adjacent two or more of $R_{611}$ to $R_{621}$ may be mutually bonded to form a substituted or

unsubstituted monocyclic ring, mutually bonded to form a substituted or unsubstituted fused ring, or not mutually bonded; and

$R_{611}$ to $R_{621}$ not forming the substituted or unsubstituted heterocycle, the substituted or unsubstituted monocyclic ring, and the substituted or unsubstituted fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group Represented by -O-($R_{904}$), a group represented by -S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms.

$R_{601A}$ and $R_{602A}$ in the formula (62) are groups corresponding to $R_{601}$ and $R_{602}$ in the formula (6), respectively.

For instance, $R_{601A}$ and $R_{611}$ are optionally bonded with each other to form a bicyclic (or tri-or-more cyclic) fused nitrogen-containing heterocycle, in which the ring including $R_{601A}$ and $R_{611}$ and a benzene ring corresponding to the a ring are fused. Specific examples of the nitrogen-containing heterocycle include a compound corresponding to a nitrogen-containing bi(or-more)cyclic fused heterocyclic group in the specific example group G2. The same applies to $R_{601A}$ bonded with $R_{621}$, $R_{602A}$ bonded with $R_{613}$, and $R_{602A}$ bonded with $R_{614}$.

At least one combination of adjacent two or more of $R_{611}$ to $R_{621}$ may be mutually bonded to form a substituted or unsubstituted monocyclic ring, or mutually bonded to form a substituted or unsubstituted fused ring.

For instance, $R_{611}$ and $R_{612}$ are optionally mutually bonded to form a structure in which a benzene ring, indole ring, pyrrole ring, benzofuran ring, benzothiophene ring or the like is fused to the six-membered ring bonded with $R_{611}$ and $R_{612}$, the resultant fused ring forming a naphthalene ring, carbazole ring, indole ring, dibenzofuran ring, or dibenzothiophene ring, respectively.

In an exemplary embodiment, $R_{611}$ to $R_{621}$ not contributing to ring formation are each independently a hydrogen atom, a substituted or unsubstituted alkyl group having 1 to 50 carbon atoms, a substituted or unsubstituted aryl group having 6 to 50 ring carbon atoms, or a substituted or unsubstituted heterocyclic group having 5 to 50 ring atoms.

In an exemplary embodiment, $R_{611}$ to $R_{621}$ not contributing to ring formation are each independently a hydrogen atom, a substituted or unsubstituted aryl group having 6 to 50 ring carbon atoms, or a substituted or unsubstituted heterocyclic group having 5 to 50 ring atoms.

In an exemplary embodiment, $R_{611}$ to $R_{621}$ not contributing to ring formation are each independently a hydrogen atom, or a substituted or unsubstituted alkyl group having 1 to 50 carbon atoms.

In an exemplary embodiment, $R_{611}$ to $R_{621}$ not contributing to ring formation are each independently a hydrogen atom, or a substituted or unsubstituted alkyl group having 1 to 50 carbon atoms, and

at least one of $R_{611}$ to $R_{621}$ is a substituted or unsubstituted alkyl group having 1 to 50 carbon atoms.

In an exemplary embodiment, a compound represented by the formula (62) is a compound represented by a formula (63) below.

[Formula 423]

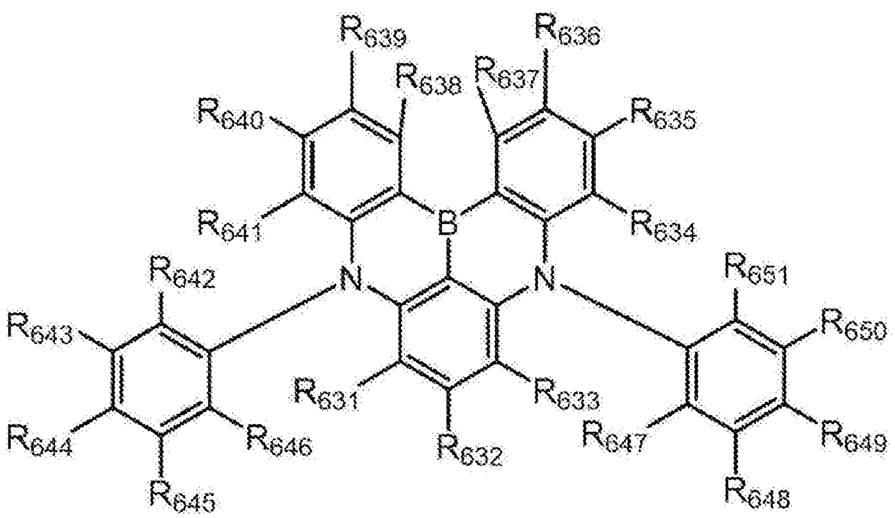

(63)

In the formula (63):

$R_{631}$ is bonded with $R_{646}$ to form a substituted or unsubstituted heterocycle, or not bonded therewith to form no substituted or unsubstituted heterocycle;

$R_{633}$ is bonded with $R_{647}$ to form a substituted or unsubstituted heterocycle, or not bonded therewith to form no substituted or unsubstituted heterocycle;

$R_{634}$ is bonded with $R_{651}$ to form a substituted or unsubstituted heterocycle, or not bonded therewith to form no substituted or unsubstituted heterocycle;

$R_{641}$ is bonded with $R_{642}$ to form a substituted or unsubstituted heterocycle, or not bonded therewith to form no substituted or unsubstituted heterocycle;

at least one combination of adjacent two or more of $R_{631}$ to $R_{651}$ are mutually bonded to form a substituted or unsubstituted monocyclic ring, mutually bonded to form a substituted or unsubstituted fused ring, or not mutually bonded;

$R_{631}$ to $R_{651}$ not forming the substituted or unsubstituted heterocycle, the substituted or unsubstituted monocyclic ring, and the substituted or unsubstituted fused ring are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group Represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

$R_{631}$ are optionally bonded with $R_{646}$ to form a substituted or unsubstituted heterocycle. For instance, $R_{631}$ and $R_{646}$ are optionally bonded with each other to form a tri-or-more cyclic fused nitrogen-containing heterocycle, in which a benzene ring bonded with $R_{646}$, a ring including a nitrogen atom, and a benzene ring corresponding to the a ring are fused. Specific examples of the nitrogen-containing heterocycle include a compound corresponding to a nitrogen-containing tri(-or-more)cyclic fused heterocyclic group in the specific example group G2. The same applies to $R_{633}$ bonded with $R_{647}$, $R_{634}$ bonded with $R_{651}$, and $R_{641}$ bonded with $R_{642}$.

In an exemplary embodiment, $R_{631}$ to $R_{651}$ not contributing to ring formation are each independently a hydrogen atom, a substituted or unsubstituted alkyl group having 1 to 50 carbon atoms, a substituted or unsubstituted aryl group having 6 to 50 ring carbon atoms, or a substituted or unsubstituted heterocyclic group having 5 to 50 ring atoms.

In an exemplary embodiment, $R_{631}$ to $R_{651}$ not contributing to ring formation are each independently a hydrogen atom, a substituted or unsubstituted aryl group having 6 to 50 ring carbon atoms, or a substituted or unsubstituted heterocyclic group having 5 to 50 ring atoms.

In an exemplary embodiment, $R_{631}$ to $R_{651}$ not contributing to ring formation are each independently a hydrogen atom, or a substituted or unsubstituted alkyl group having 1 to 50 carbon atoms.

In an exemplary embodiment, $R_{631}$ to $R_{651}$ not contributing to ring formation are each independently a hydrogen atom, or a substituted or unsubstituted alkyl group having 1 to 50 carbon atoms, and

at least one of $R_{631}$ to $R_{651}$ is a substituted or unsubstituted alkyl group having 1 to 50 carbon atoms.

In an exemplary embodiment, a compound represented by the formula (63) is a compound represented by a formula (63A) below.

[Formula 424]

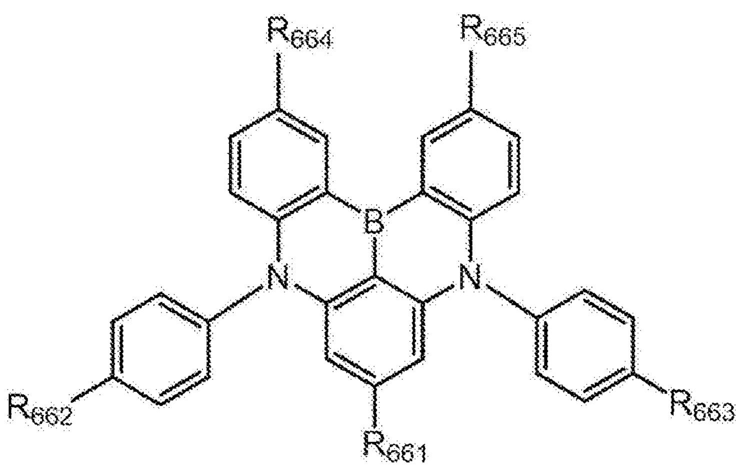

(63A)

In the formula (63A):

$R_{661}$ is a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, or a substituted or unsubstituted aryl group having 6 to 50 ring carbon atoms; and

$R_{662}$ to $R_{665}$ are each independently a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, or a substituted or unsubstituted aryl group having 6 to 50 ring carbon atoms.

In an exemplary embodiment, $R_{661}$ to $R_{665}$ are each independently a substituted or unsubstituted alkyl group having 1 to 50 carbon atoms, or a substituted or unsubstituted aryl group having 6 to 50 ring carbon atoms.

In an exemplary embodiment, $R_{661}$ to $R_{665}$ are each independently a substituted or unsubstituted alkyl group having 1 to 50 carbon atoms.

In an exemplary embodiment, a compound represented by the formula (63) is a compound represented by a formula (63B) below.

[Formula 425]

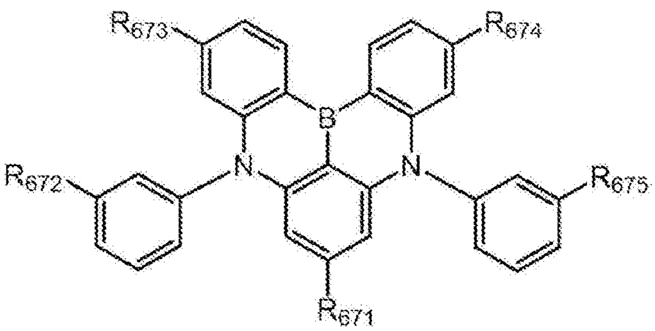

(63B)

In the formula (63B):

$R_{671}$ and $R_{672}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-N(R_{906})(R_{907})$, or a substituted or unsubstituted aryl group having 6 to 50 ring carbon atoms; and

$R_{673}$ to $R_{675}$ are each independently a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-N(R_{906})(R_{907})$, or a substituted or unsubstituted aryl group having 6 to 50 ring carbon atoms.

In an exemplary embodiment, a compound represented by the formula (63) is a compound represented by a formula (63B') below.

[Formula 426]

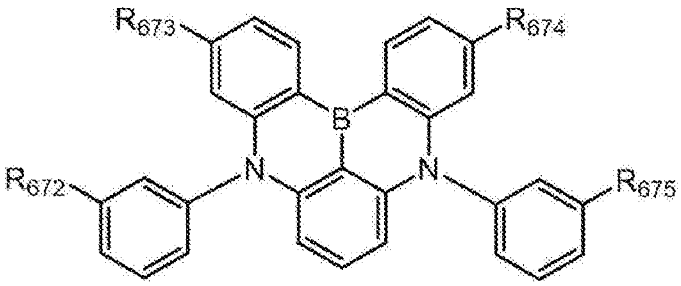

(63B')

In the formula (63B'), $R_{672}$ to $R_{675}$ each independently represent the same as $R_{672}$ to $R_{675}$ in the formula (63B).

In an exemplary embodiment, at least one of $R_{671}$ to $R_{675}$ is a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -N($R_{906}$)($R_{907}$), or a substituted or unsubstituted aryl group having 6 to 50 ring carbon atoms.

In an exemplary embodiment: $R_{672}$ is a hydrogen atom, a substituted or unsubstituted alkyl group having 1 to 50 carbon atoms, a group represented by-N($R_{906}$)($R_{907}$), or a substituted or unsubstituted aryl group having 6 to 50 ring carbon atoms; and

$R_{671}$ and $R_{673}$ to $R_{675}$ are each independently a substituted or unsubstituted alkyl group having 1 to 50 carbon atoms, a group represented by -N($R_{906}$)($R_{907}$), or a substituted or unsubstituted aryl group having 6 to 50 ring carbon atoms.

In an exemplary embodiment, a compound represented by the formula (63) is a compound represented by a formula (63C) below.

[Formula 427]

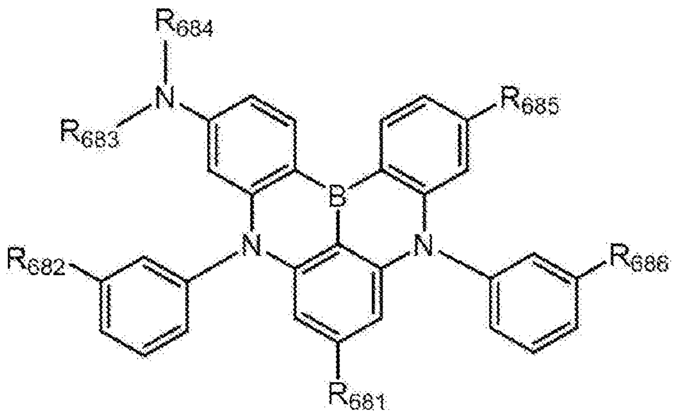

(63C)

In the formula (63C): $R_{631}$ and $R_{682}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, or a substituted or unsubstituted aryl group having 6 to 50 ring carbon atoms; and

$R_{683}$ to $R_{686}$ are each independently a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, or a substituted or unsubstituted aryl group having 6 to 50 ring carbon atoms.

In an exemplary embodiment, a compound represented by the formula (63) is a compound represented by a formula (63C') below.

[Formula 428]

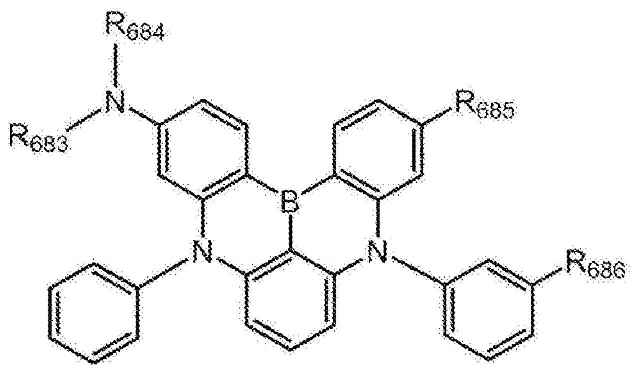

(63C')

In the formula (63C'), $R_{683}$ to $R_{686}$ each independently represent the same as $R_{683}$ to $R_{686}$ in the formula (63C).

In an exemplary embodiment, $R_{631}$ to $R_{686}$ are each independently a substituted or unsubstituted alkyl group having 1 to 50 carbon atoms, or a substituted or unsubstituted aryl group having 6 to 50 ring carbon atoms.

In an exemplary embodiment, $R_{631}$ to $R_{686}$ are each independently a substituted or unsubstituted aryl group having 6 to 50 ring carbon atoms.

A compound represented by the formula (6) is producible by initially bonding the a ring, b ring and c ring with linking groups (a group including N-$R_{601}$ and a group including N-$R_{602}$) to form an intermediate (first reaction), and bonding the a ring, b ring and c ring with a linking group (a group including a boron atom) to form a final product (second reaction). In the first reaction, an amination reaction (e.g. Buchwald-Hartwig reaction) is applicable. In the second reaction, Tandem Hetero-Friedel-Crafts Reactions or the like is applicable.

Specific examples of a compound represented by the formula (6) are shown below. It should however be noted that these specific examples are merely exemplary and do not limit a compound represented by the formula (6).

[Formula 429]

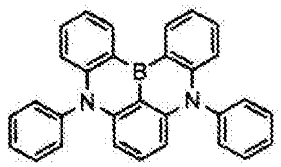
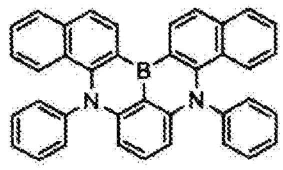
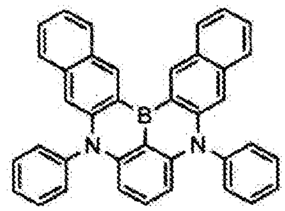
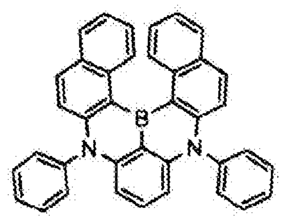
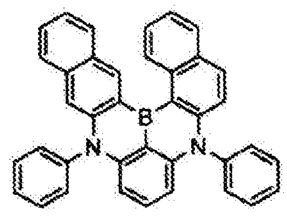
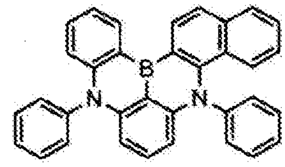
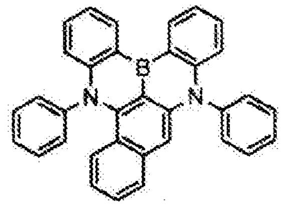
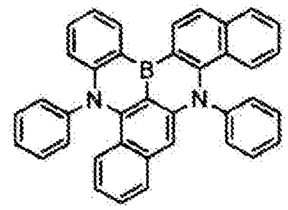
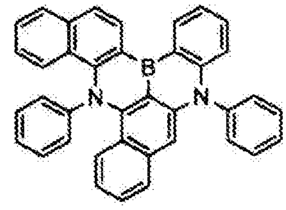
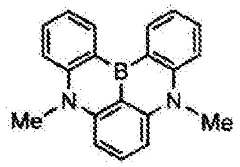
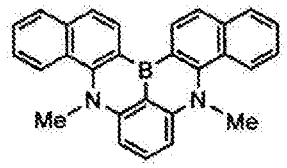
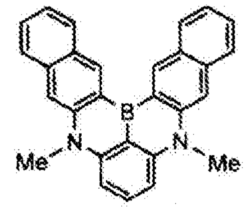
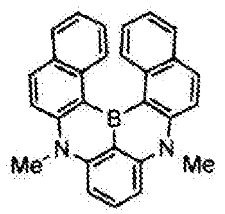
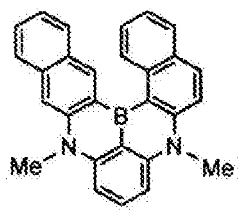
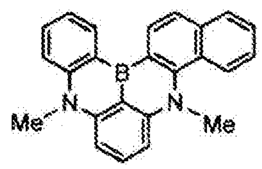
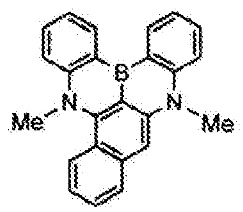
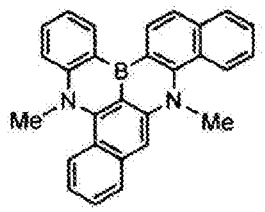
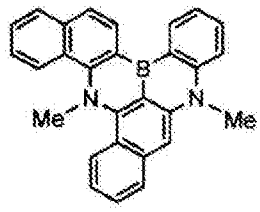

[Formula 430]

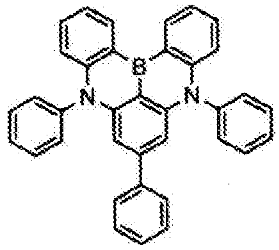
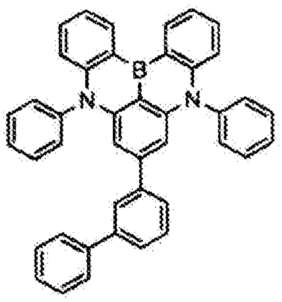
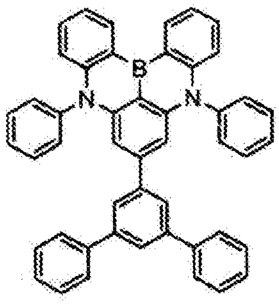
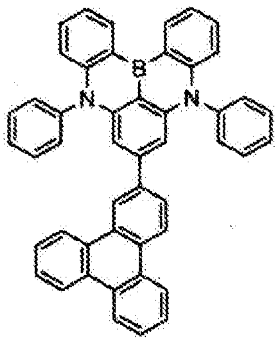
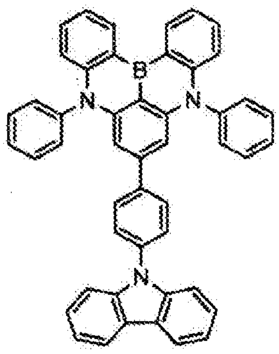
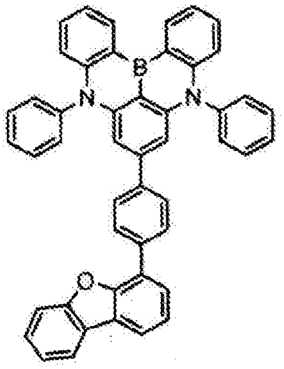
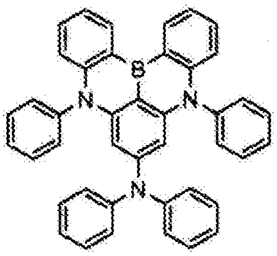
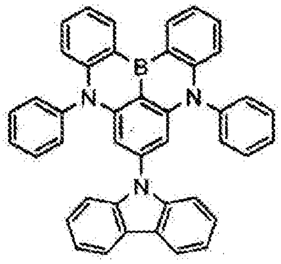
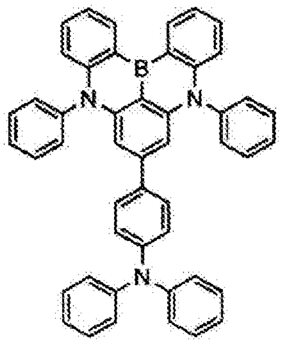

[Formula 431]

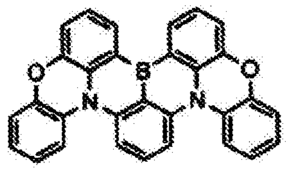

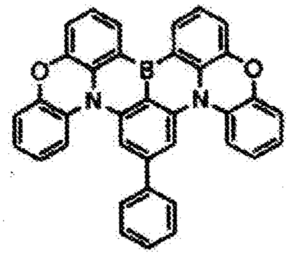

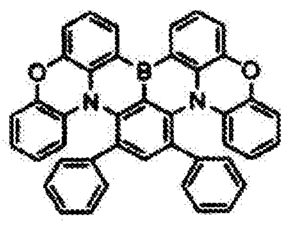

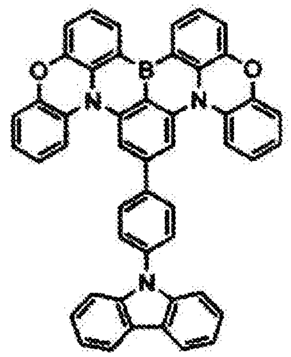

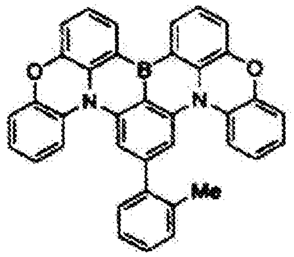

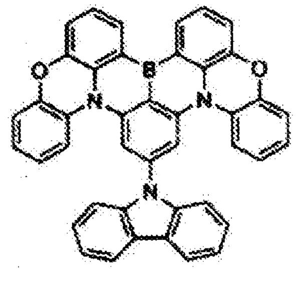

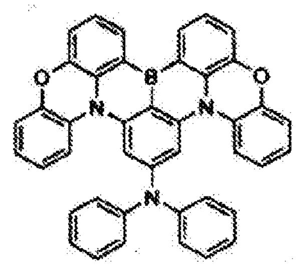

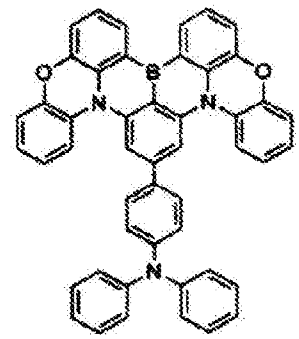

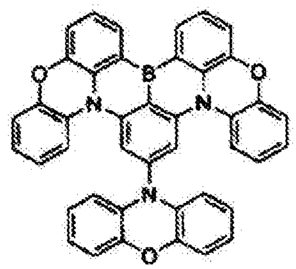

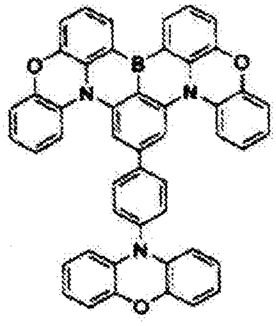

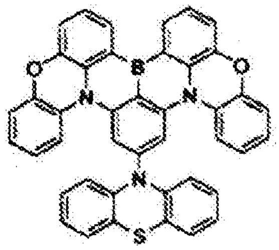

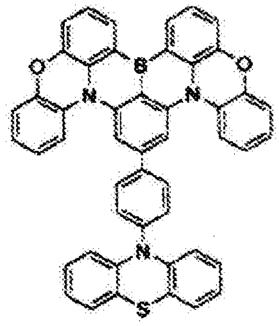

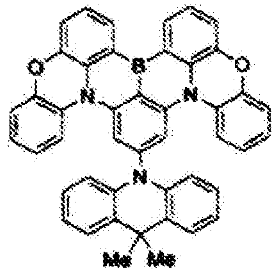

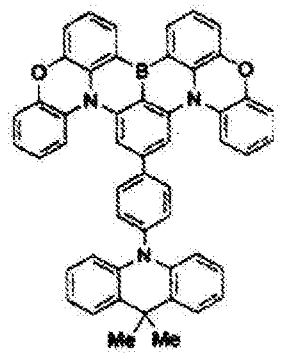

[Formula 432]

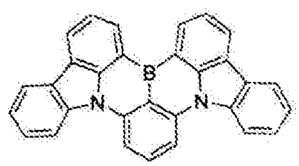
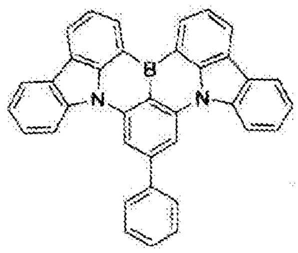
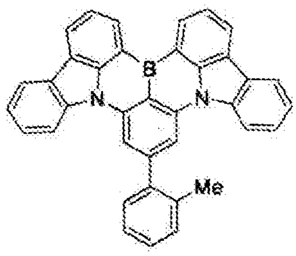
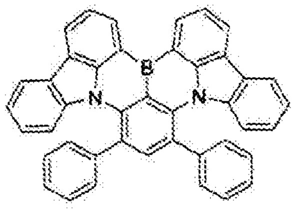
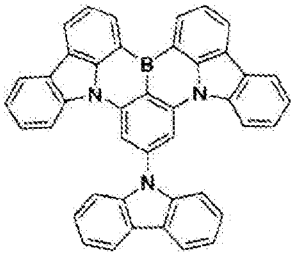
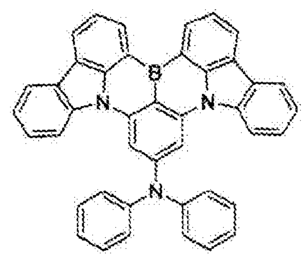
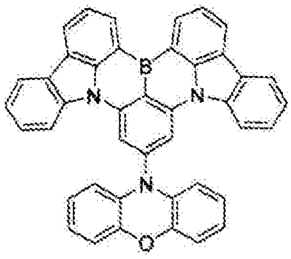
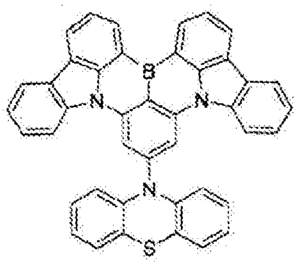
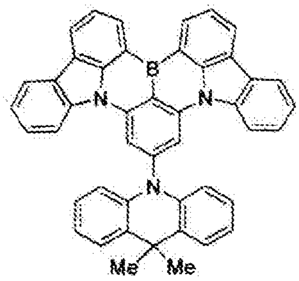
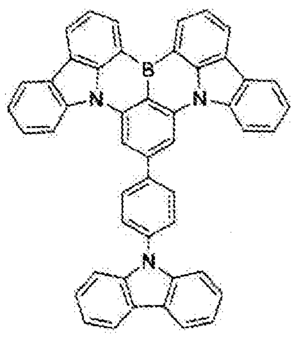
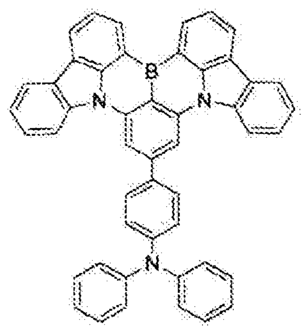
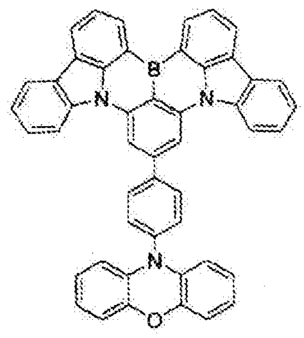
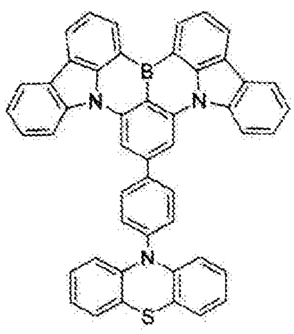
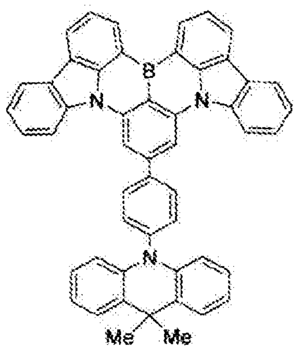

[Formula 433]

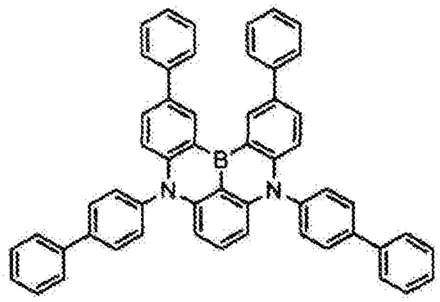

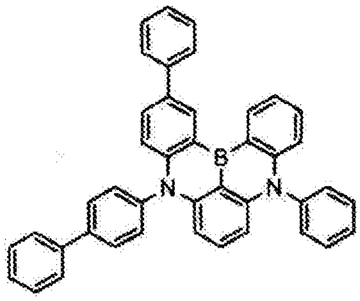

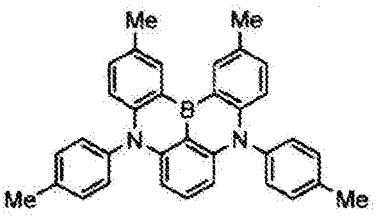

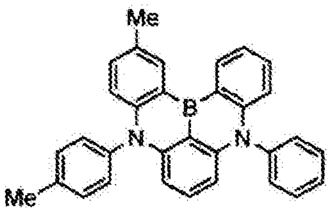

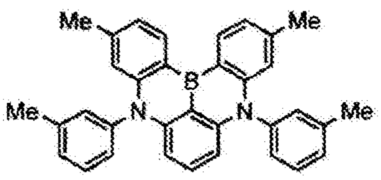

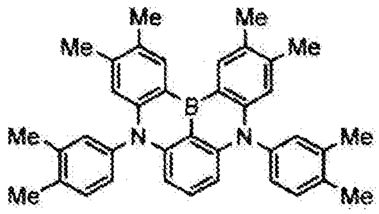

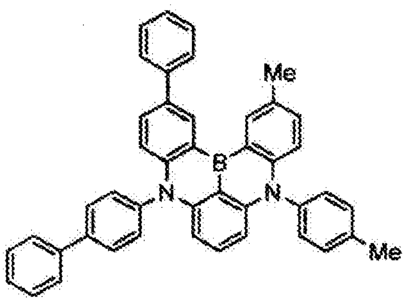

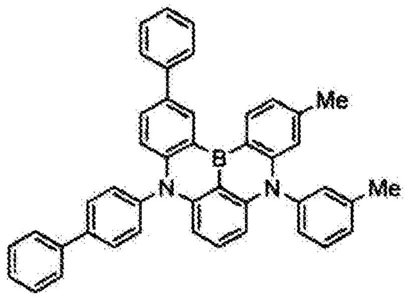

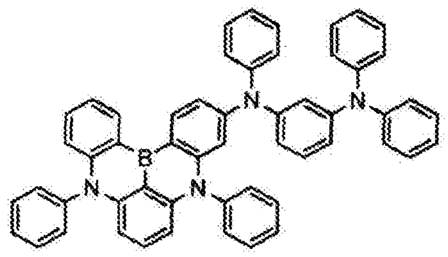

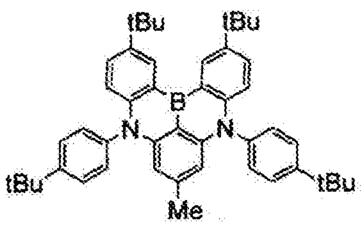

[Formula 434]

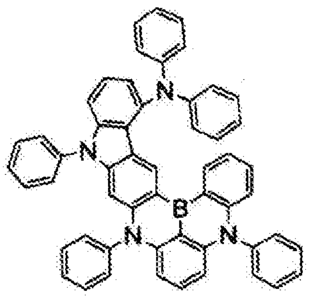
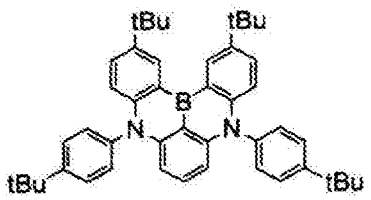
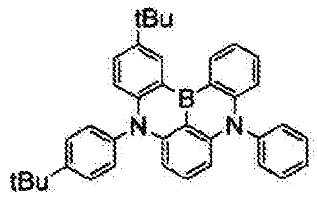

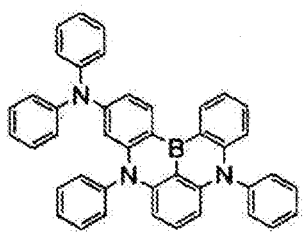
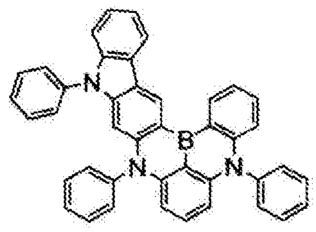
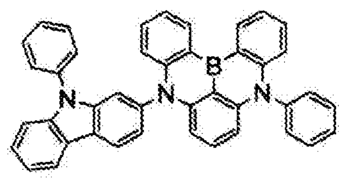

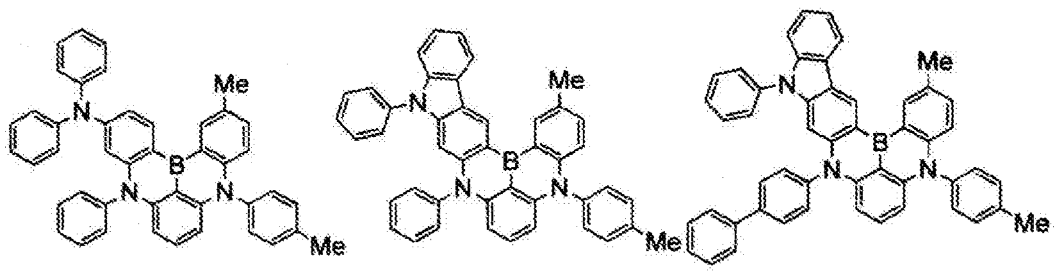

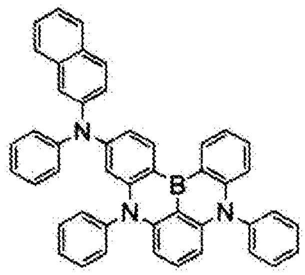
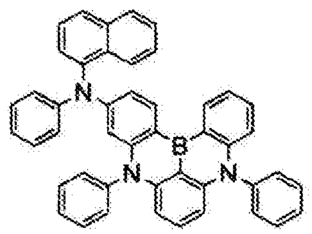
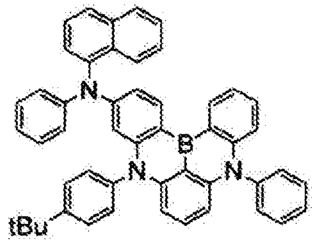

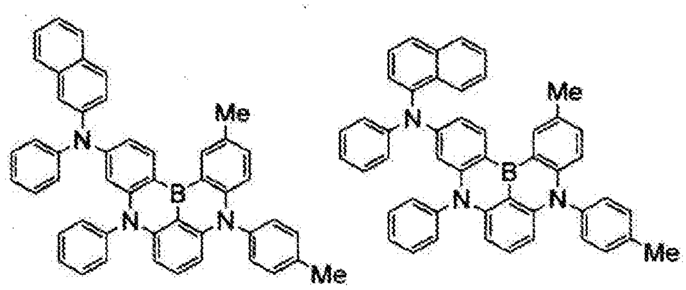
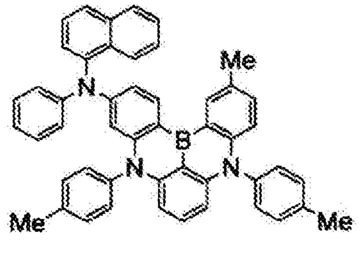

[Formula 435]

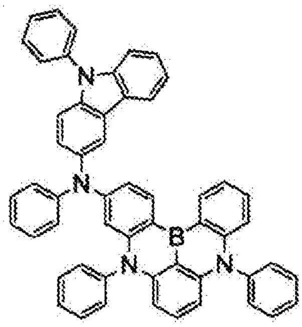
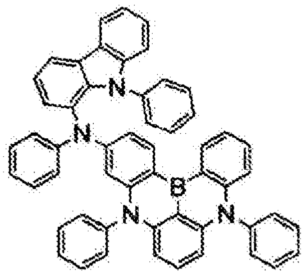
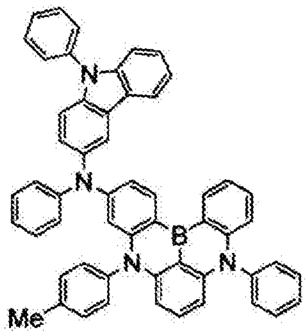

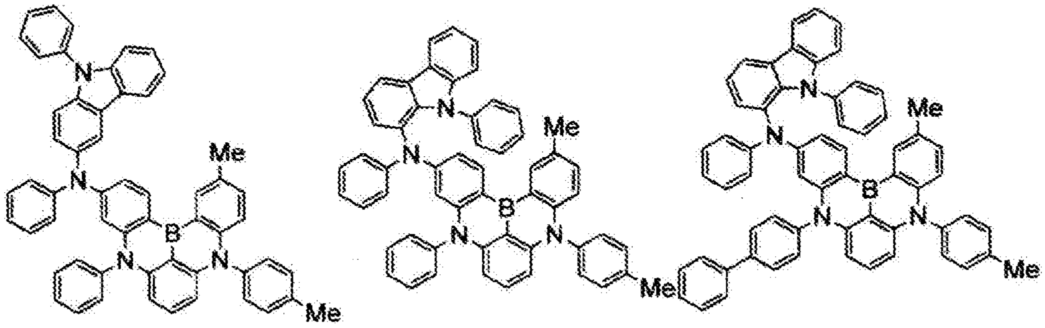

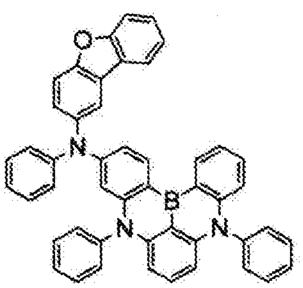
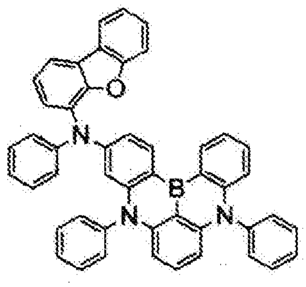
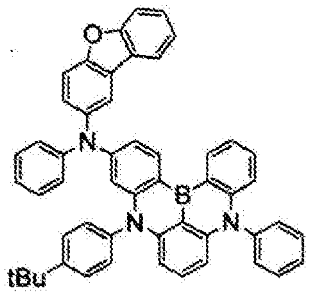

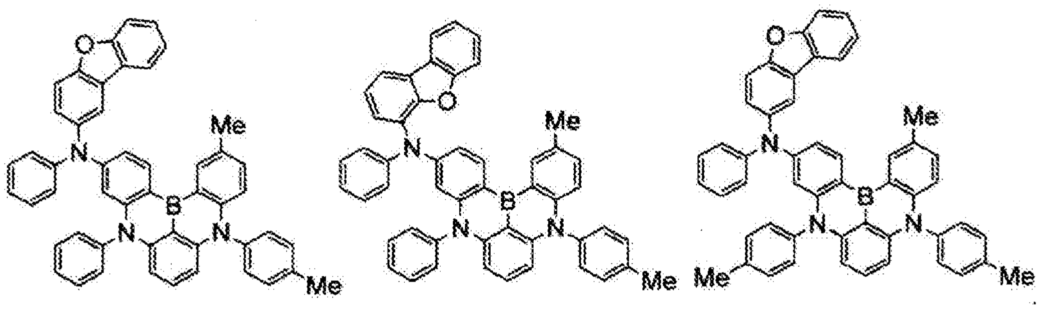

[Formula 436]

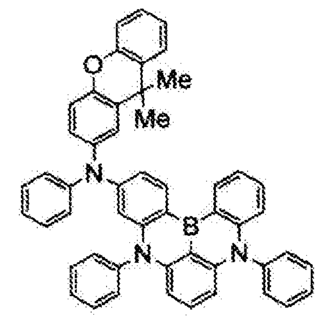
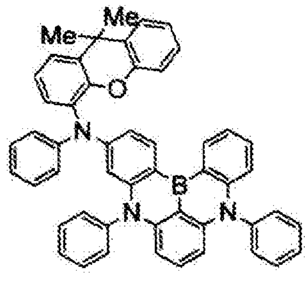
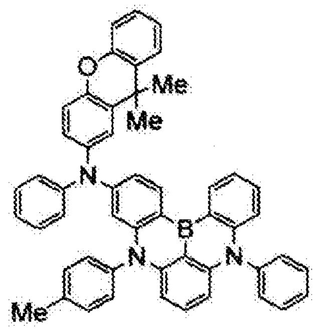

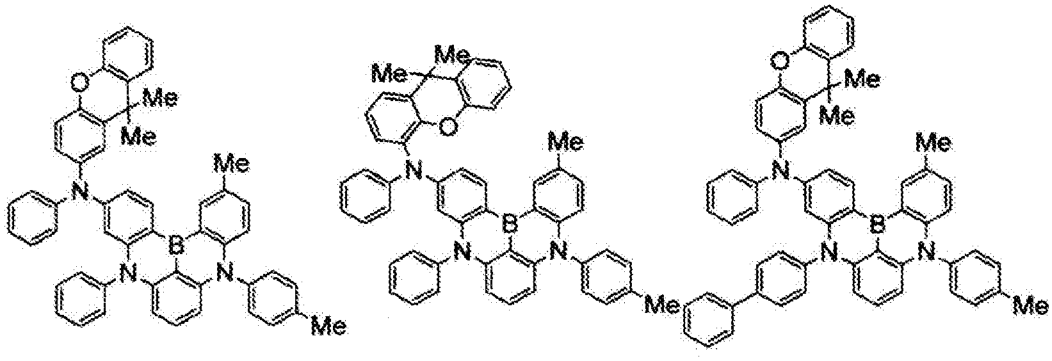

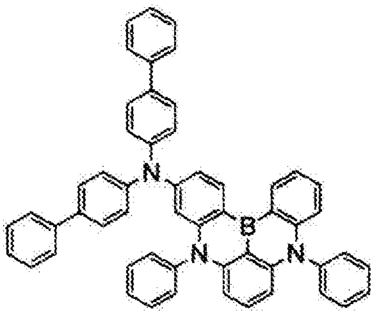
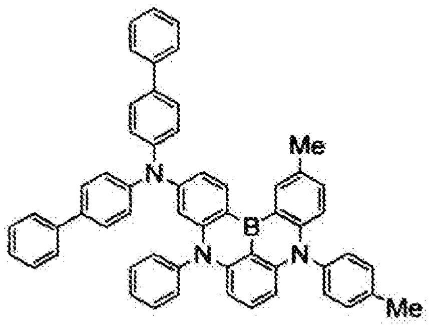

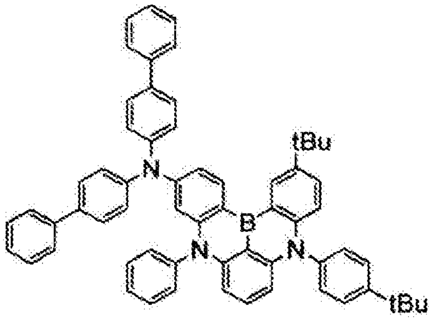
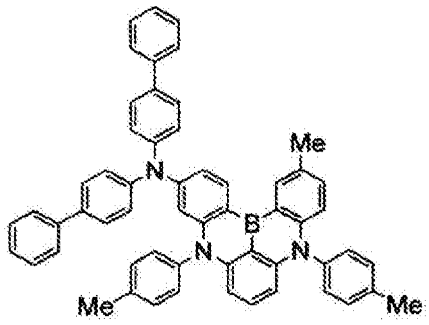

[Formula 437]

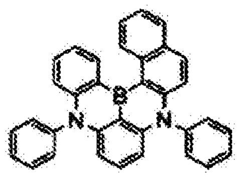
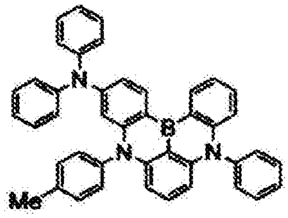
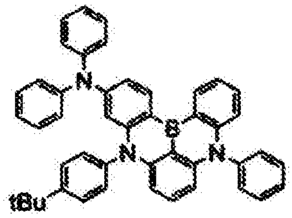
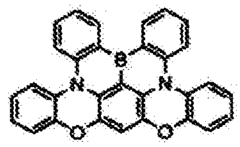

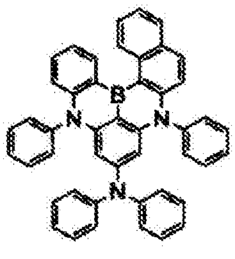
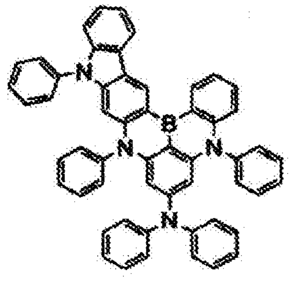
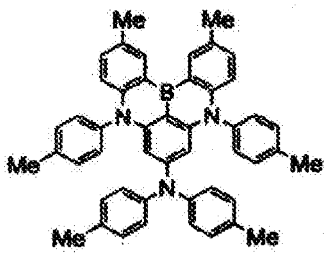
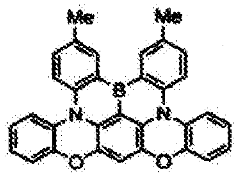

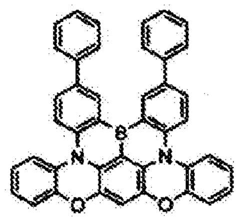

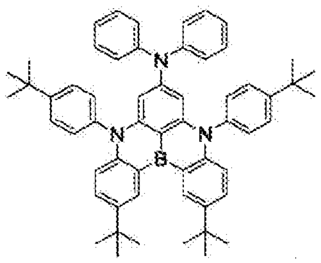

[Formula 438]

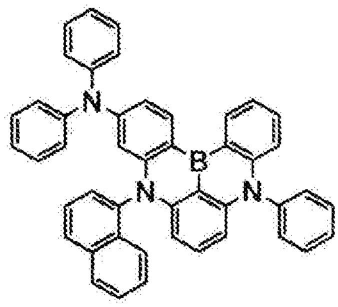
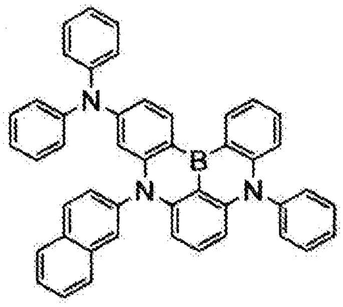
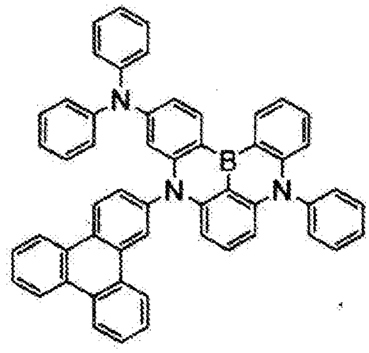

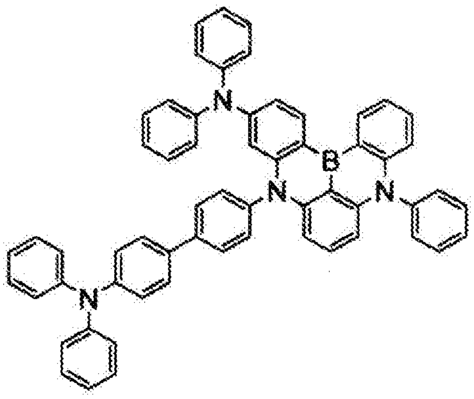
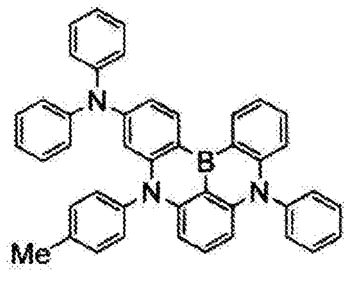

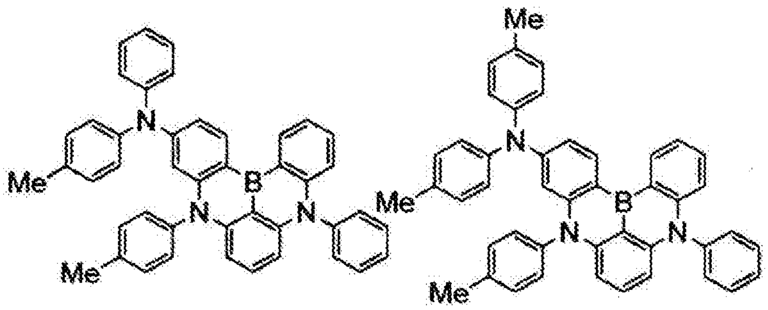
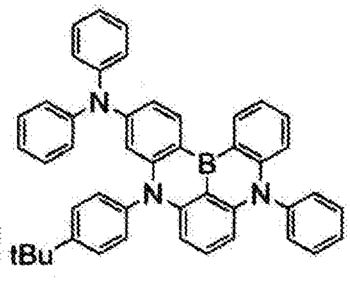

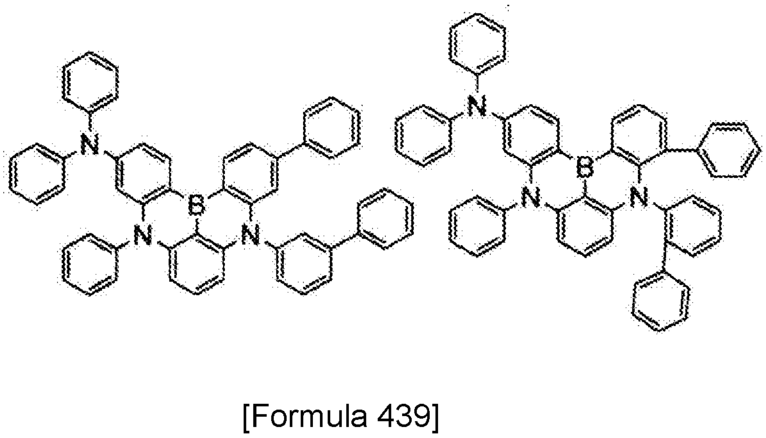

[Formula 439]

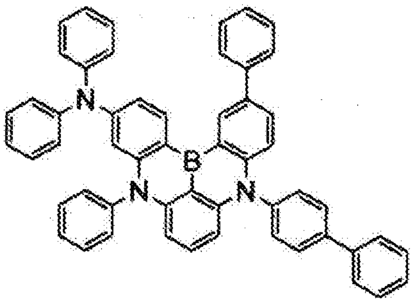
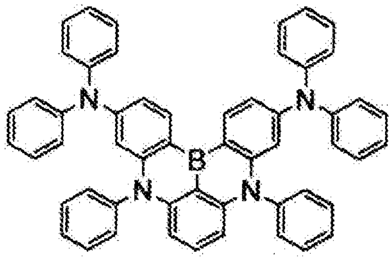
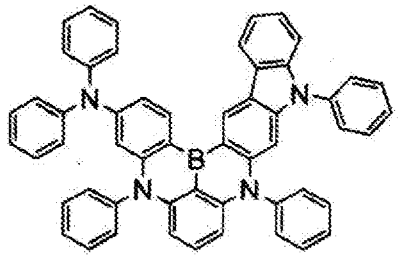
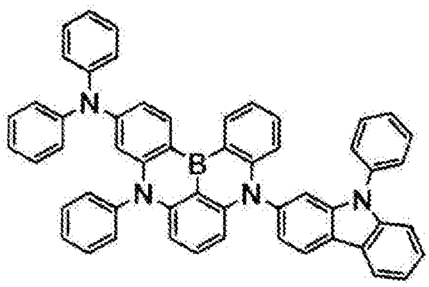
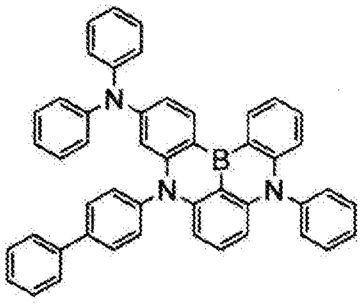
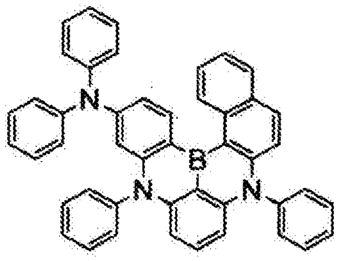
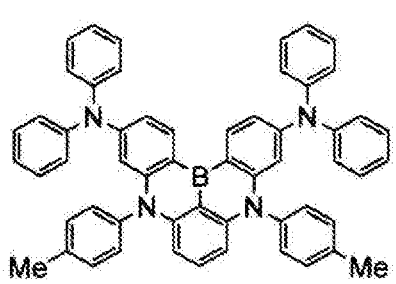
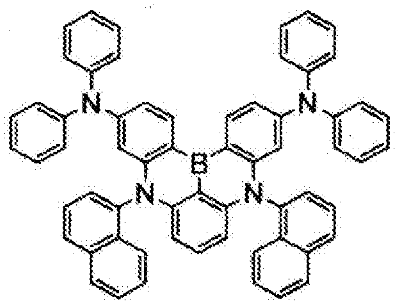

[Formula 440]

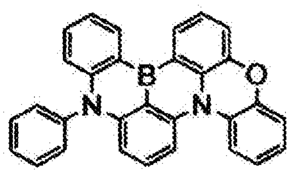

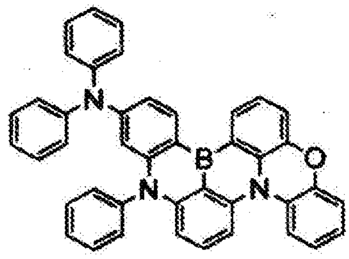

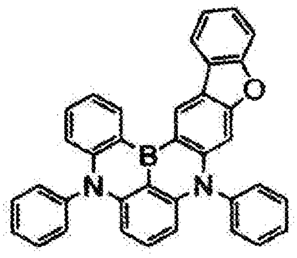

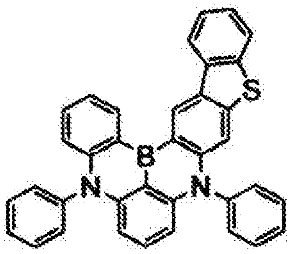

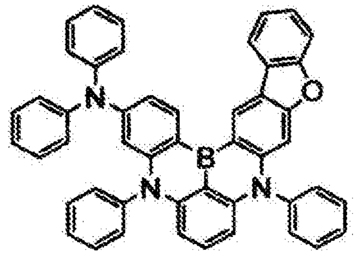

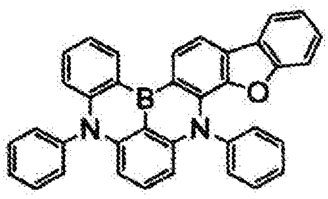

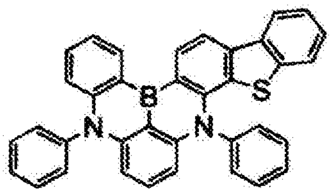

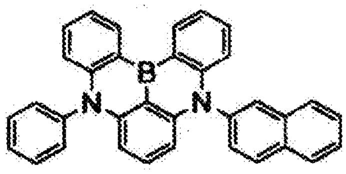

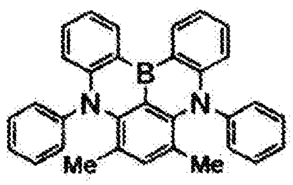

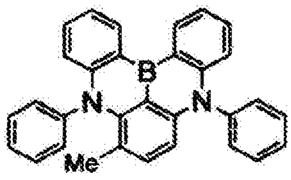

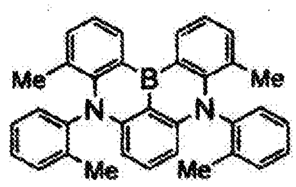

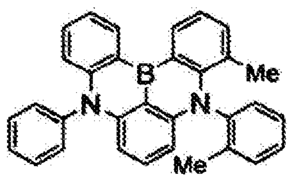

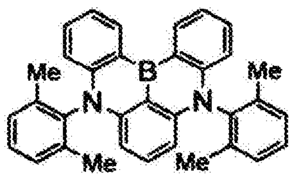

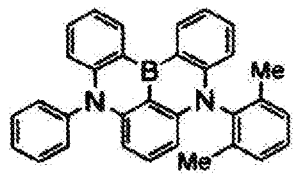

Compound Represented by Formula (7)

A compound represented by the formula (7) will be described below.

[Formula 441]

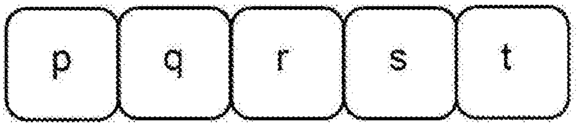

(7)

[Formula 442]

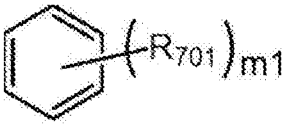

(72)

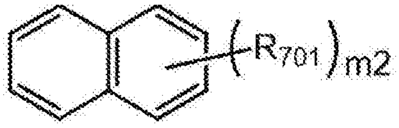

(73)

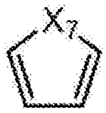

(74)

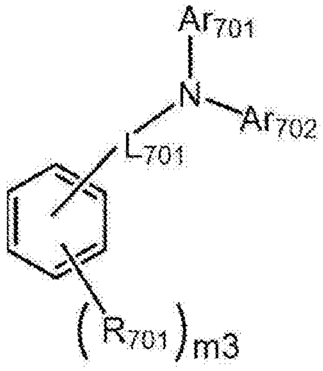

(75)

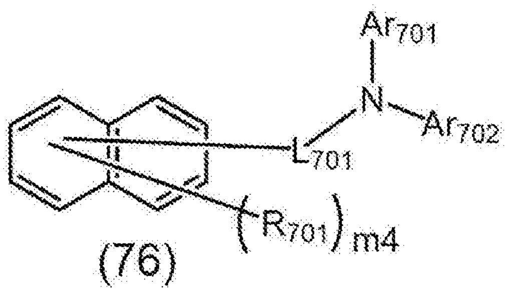

(76)

In the formula (7): r ring is a ring represented by the formula (72) or the formula (73), the r ring being fused with adjacent ring(s) at any position(s);

q ring and s ring are each independently a ring represented by the formula (74) and fused with adjacent ring(s) at any position(s);

p ring and t ring are each independently a structure represented by the formula (75) or the formula (76) and fused with adjacent ring(s) at any position(s);

$X_7$ is an oxygen atom, a sulfur atom, or $NR_{702}$;

when a plurality of $R_{701}$ are present, adjacent ones of the plurality of $R_{701}$ are mutually bonded to form a substituted or unsubstituted monocyclic ring, mutually bonded to form a substituted or unsubstituted fused ring, or not mutually bonded;

$R_{701}$ and $R_{702}$ forming neither the substituted or unsubstituted monocyclic ring nor the substituted or unsubstituted fused ring are each independently a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group Represented by $-O-(R_{904})$, a group represented by$-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms;

$Ar_{701}$ and $Ar_{702}$ are each independently a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

$L_{701}$ is a substituted or unsubstituted alkylene group having 1 to 50 carbon atoms, a substituted or unsubstituted alkenylene group having 2 to 50 carbon atoms, a substituted or unsubstituted alkynylene group having 2 to 50 carbon atoms, a substituted or unsubstituted cycloalkylene group having 3 to 50 ring carbon atoms, a substituted or unsubstituted arylene group having 6 to 50 ring carbon atoms, or a substituted or unsubstituted divalent heterocyclic

group having 5 to 50 ring atoms;
m1 is 0, 1, or 2;
m2 is 0, 1, 2, 3, or 4;
m3 is each independently 0, 1, 2, 3 or 3;
m4 is each independently 0, 1, 2, 3, 4, or 5;
when a plurality of $R_{701}$ are present, the plurality of $R_{701}$ are mutually the same or different;
when a plurality of $X_7$ are present, the plurality of $X_7$ are mutually the same or different;
when a plurality of $R_{702}$ are present, the plurality of $R_{702}$ are mutually the same or different;
when a plurality of $Ar_{701}$ are present, the plurality of $Ar_{701}$ are mutually the same or different;
when a plurality of $Ar_{702}$ are present, the plurality of $Ar_{702}$ are mutually the same or different; and
when a plurality of $L_{701}$ are present, the plurality of $L_{701}$ are mutually the same or different.

In the formula (7), each of the p ring, q ring, r ring, s ring, and t ring is fused with an adjacent ring(s) sharing two carbon atoms. The fused position and orientation are not limited but may be defined as required.
In an exemplary embodiment, in the formula (72) or the formula (73) representing the r ring, m1 = 0 or m2 = 0 is satisfied.
In an exemplary embodiment, a compound represented by the formula (7) is represented by any one of formulae (71-1) to (71-6) below.

[Formula 443]

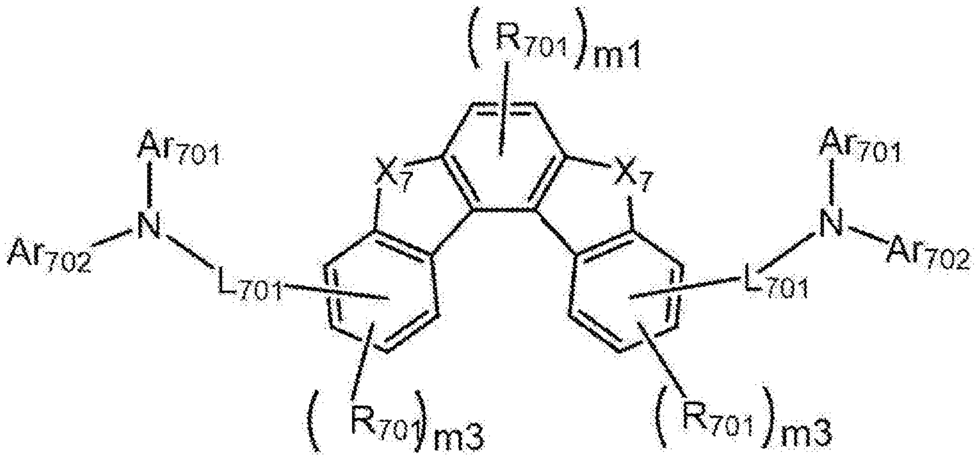

(71-1)

[Formula 444]

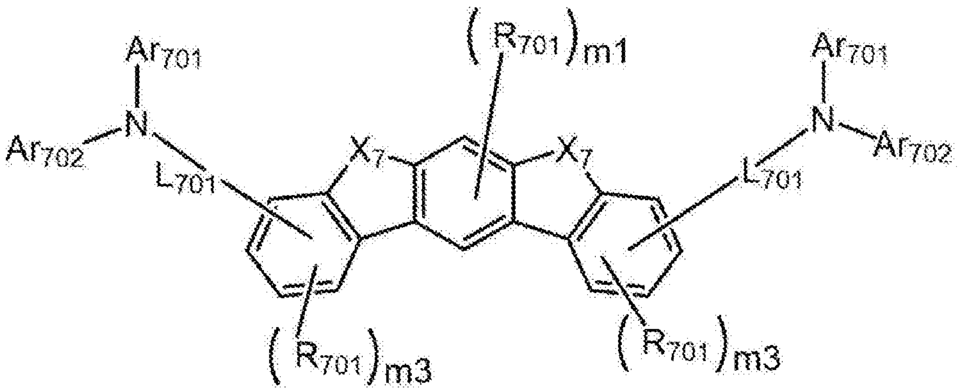

(71-2)

[Formula 445]

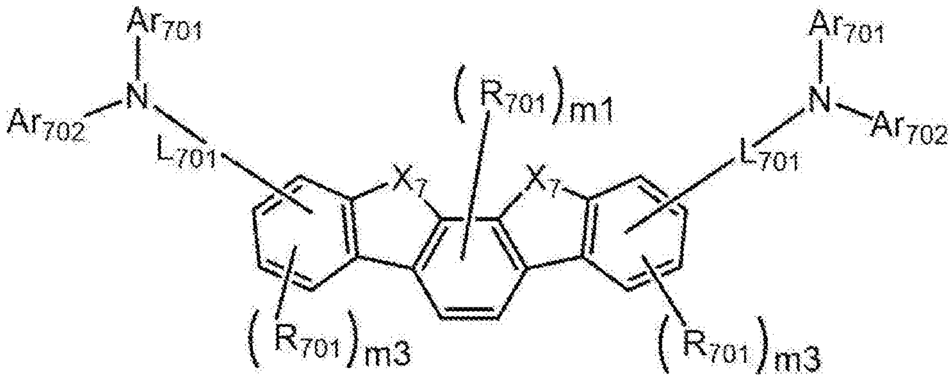

(71-3)

[Formula 446]

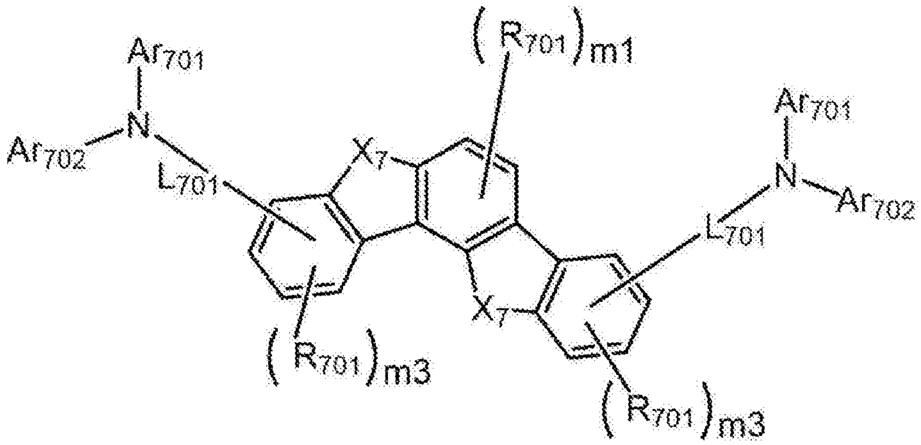

(71-4)

[Formula 447]

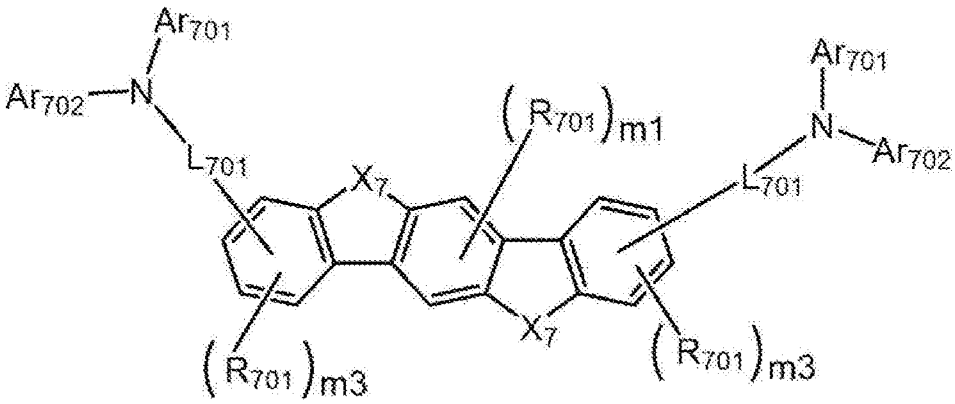

(71-5)

[Formula 448]

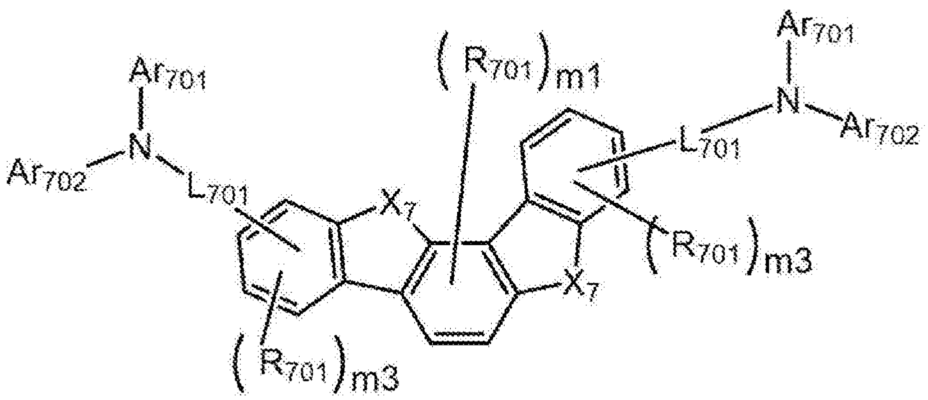

(71-6)

In the formulae (71-1) to (71-6), $R_{701}$, $X_7$, $Ar_{701}$, $Ar_{702}$, $L_{701}$, m1 and m3 respectively represent the same as $R_{701}$, $X_7$, $Ar_{701}$, $Ar_{702}$, $L_{701}$, m1 and m3 in the formula (7).

In an exemplary embodiment, a compound represented by the formula (7) is represented by any one of formulae (71-11) to (71-13) below.

[Formula 449]

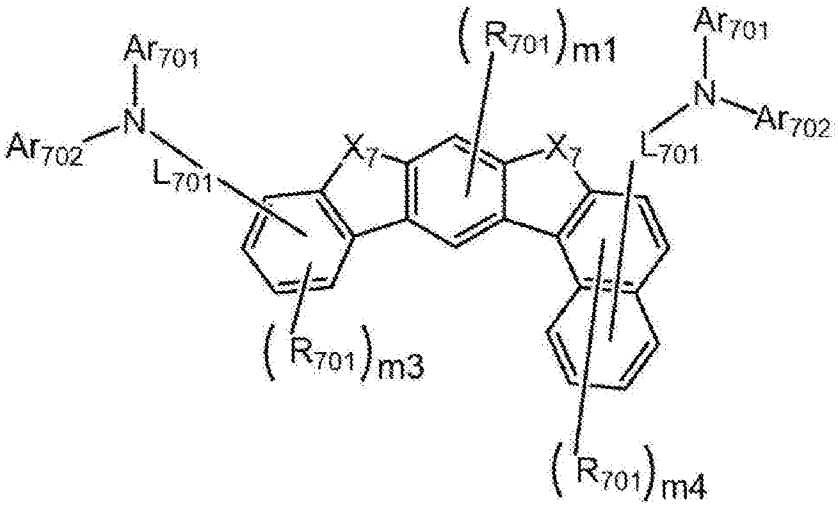

(71-11)

[Formula 450]

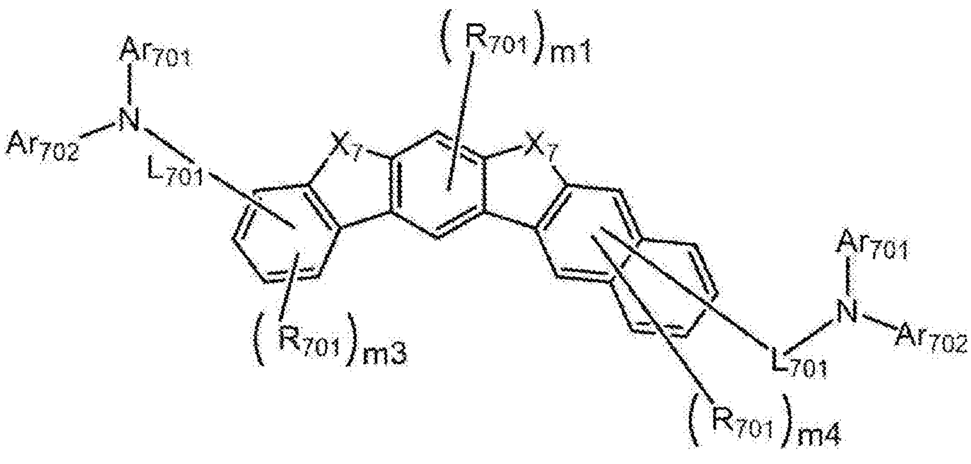

(71-12)

[Formula 451]

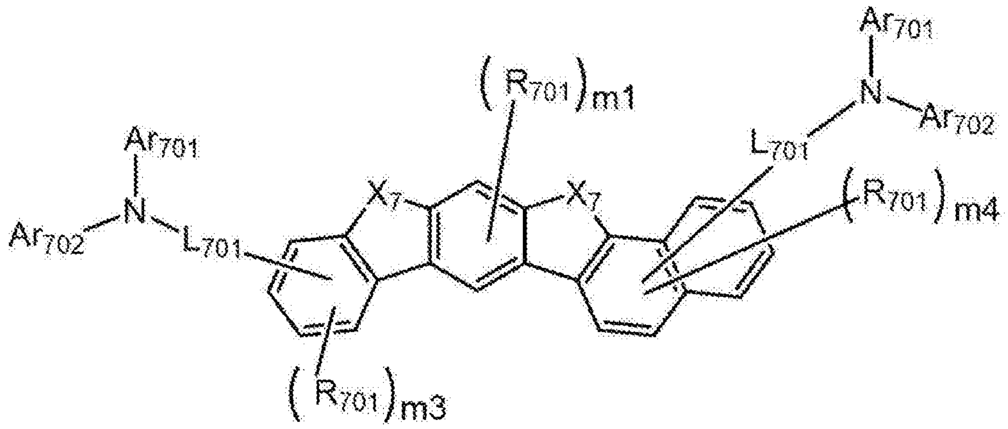

(71-13)

In the formulae (71-11) to (71-13), $R_{701}$, $X_7$, $Ar_{701}$, $Ar_{702}$, $L_{701}$, m1, m3 and m4 respectively represent the same as $R_{701}$, $X_7$, $Ar_{701}$, $Ar_{702}$, $L_{701}$, m1, m3 and m4 in the formula (7).

In an exemplary embodiment, a compound represented by the formula (7) is represented by any one of formulae (71-21) to (71-25) below.

[Formula 452]

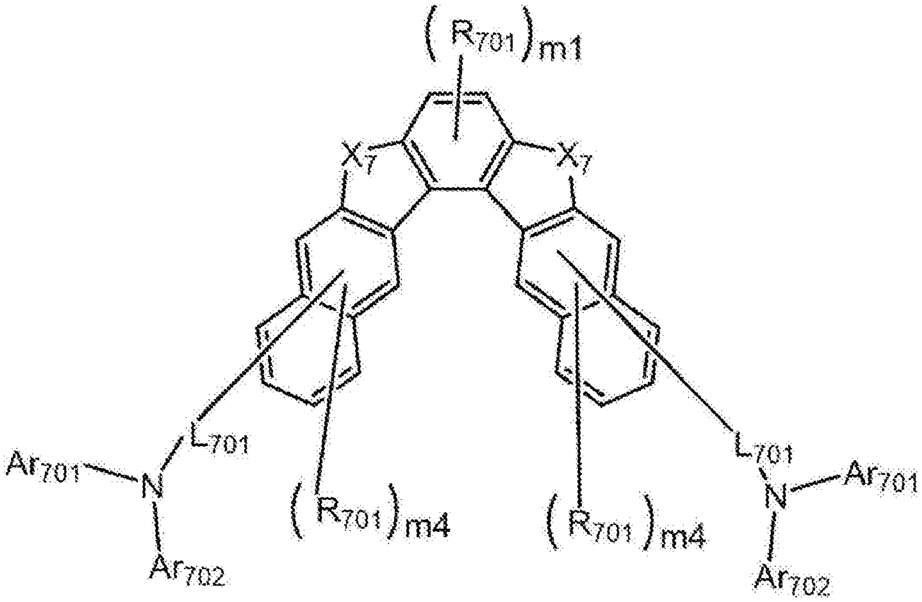

(71-21)

[Formula 453]

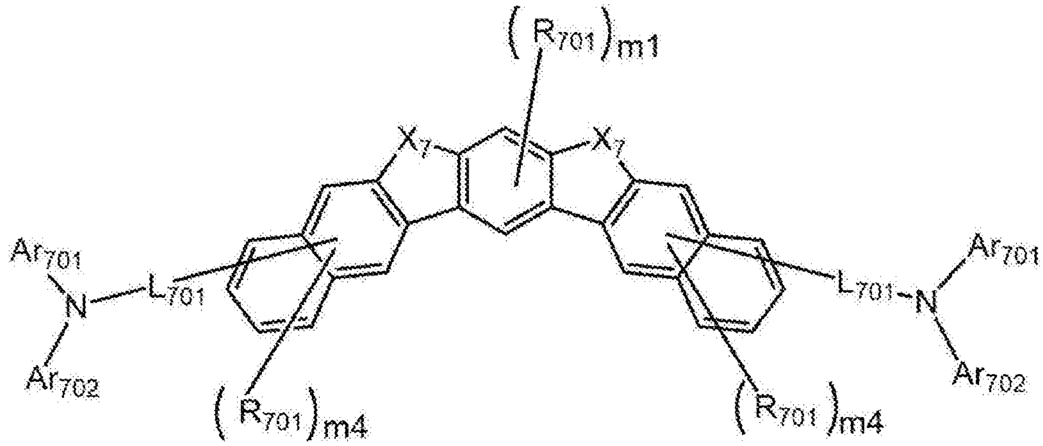

(71-22)

[Formula 454]

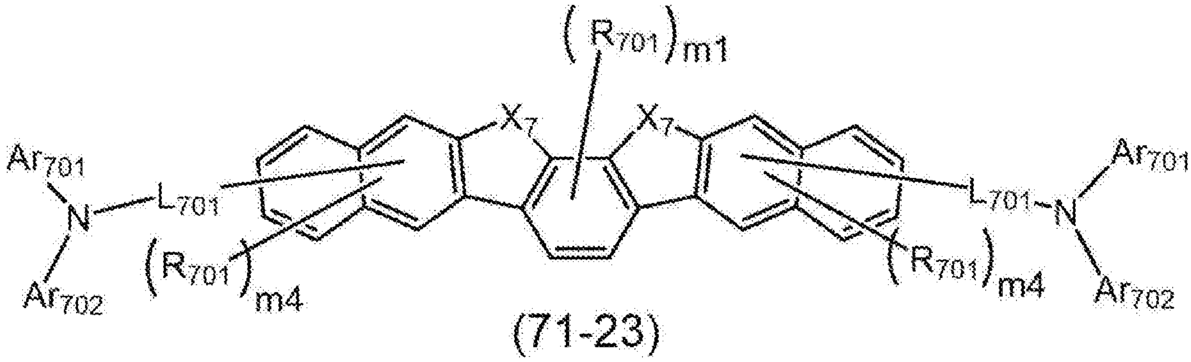

(71-23)

[Formula 455]

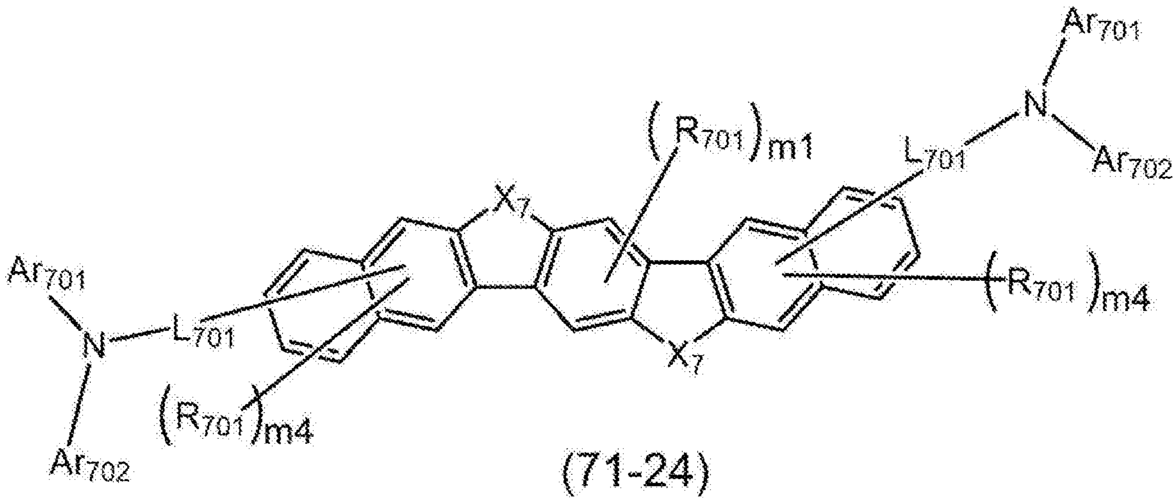

(71-24)

[Formula 456]

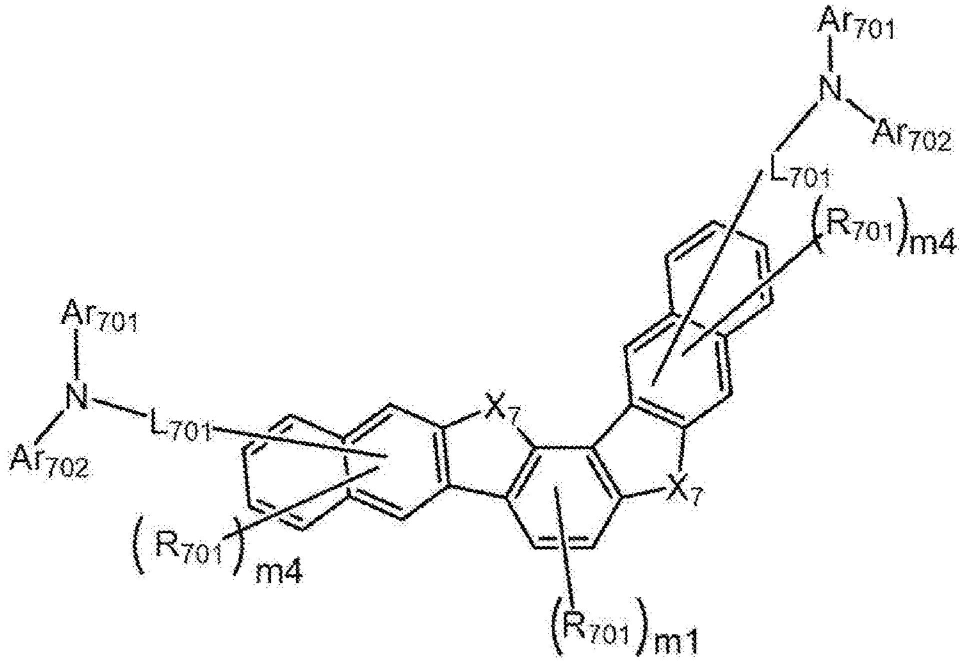

(71-25)

In the formulae (71-21) to (71-25), $R_{701}$, $X_7$, $Ar_{701}$, $Ar_{702}$, $L_{701}$, m1 and m4 respectively represent the same as $R_{701}$, $X_7$, $Ar_{701}$, $Ar_{702}$, $L_{701}$, m1 and m4 in the formula (7).

In an exemplary embodiment, a compound represented by the formula (7) is represented by any one of formulae (71-31) to (71-33) below.

[Formula 457]

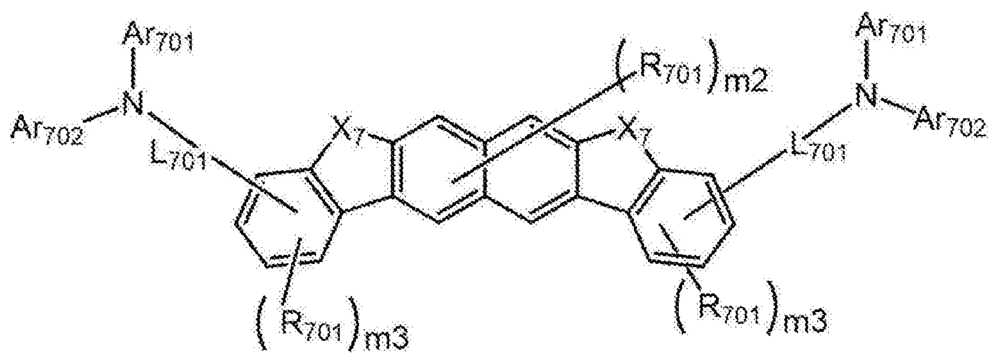

(71-31)

[Formula 458]

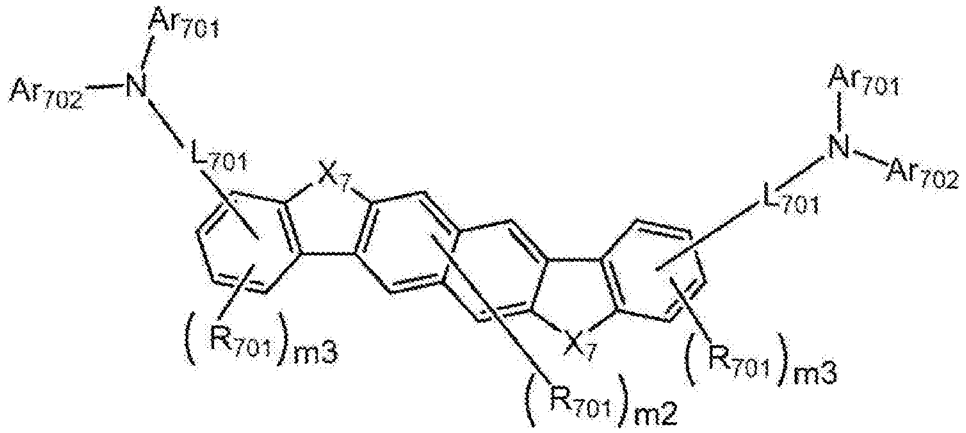

(71-32)

[Formula 459]

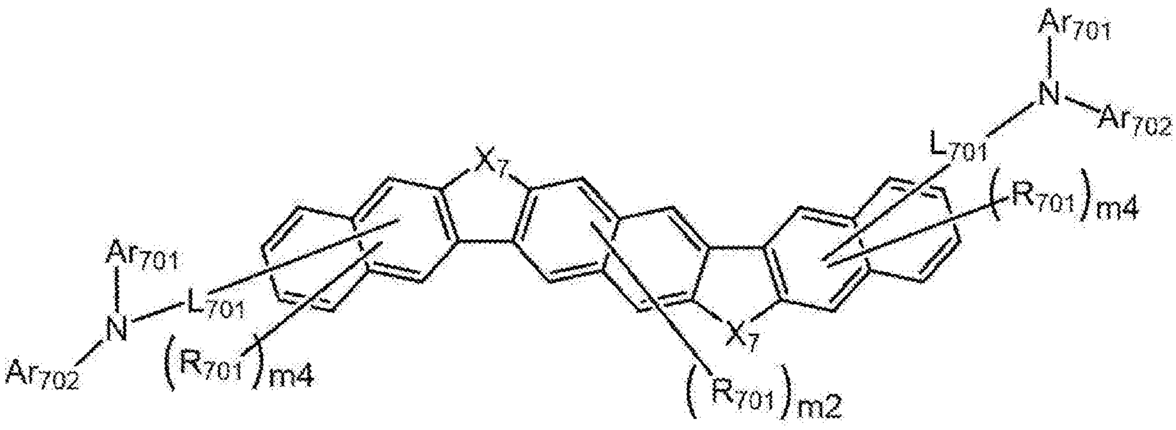

(71-33)

In the formulae (71-31) to (71-33), $R_{701}$, $X_7$, $Ar_{701}$, $Ar_{702}$, $L_{701}$, and m2 to m4 respectively represent the same as $R_{701}$, $X_7$, $Ar_{701}$, $Ar_{702}$, $L_{701}$, and m2 to m4 in the formula (7).

In an exemplary embodiment, $Ar_{701}$ and $Ar_{702}$ are each independently a substituted or unsubstituted aryl group having 6 to 50 ring carbon atoms.

In an exemplary embodiment, one of $Ar_{701}$ and $Ar_{702}$ is a substituted or unsubstituted aryl group having 6 to 50 ring carbon atoms, and the other of $Ar_{701}$ and $Ar_{702}$ is a substituted or unsubstituted heterocyclic group having 5 to 50 ring atoms.

Specific examples of a compound represented by the formula (7) include compounds shown below.

[Formula 460]

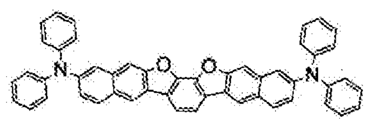
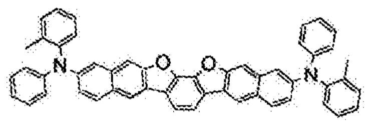
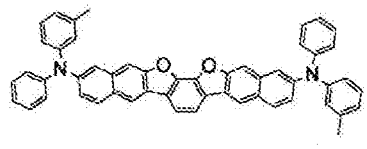
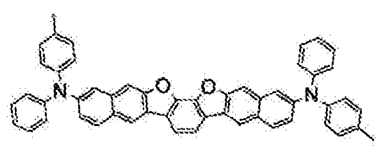
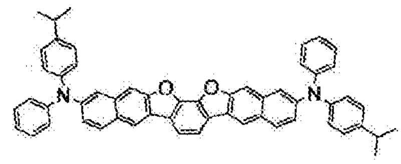
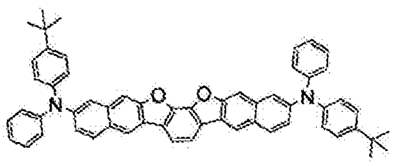
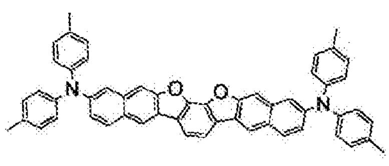
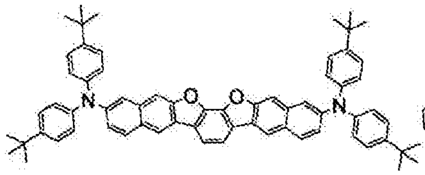
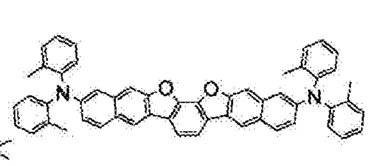
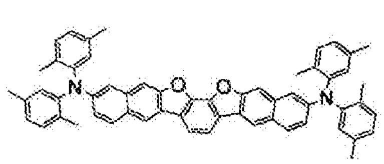
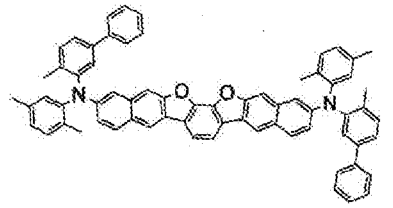
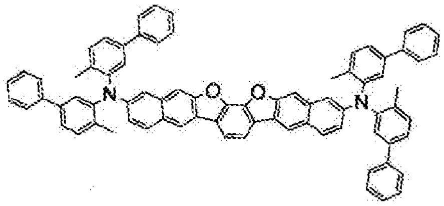
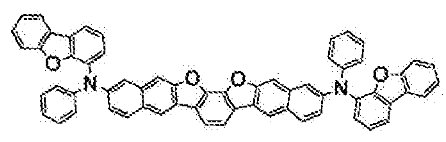
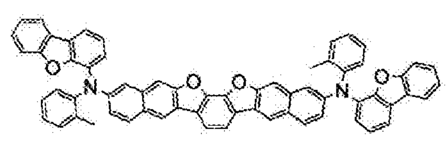
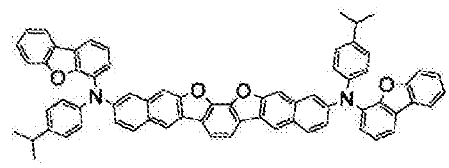
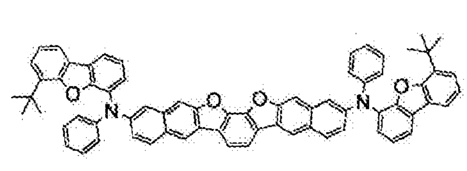
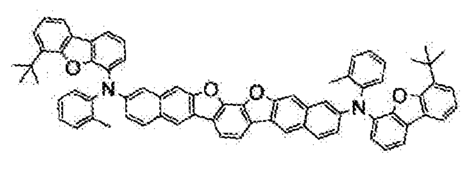
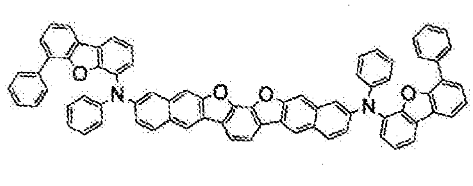
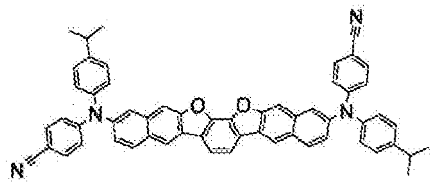

[Formula 461]

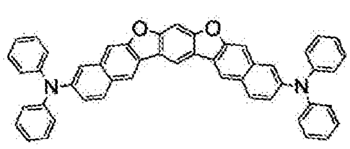
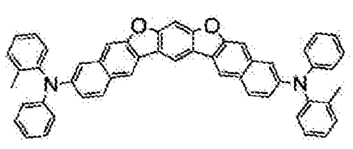
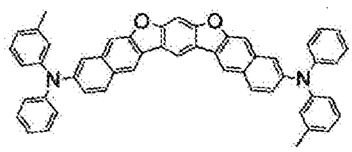

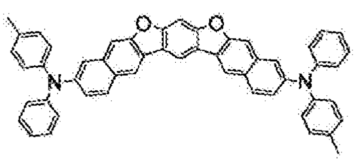
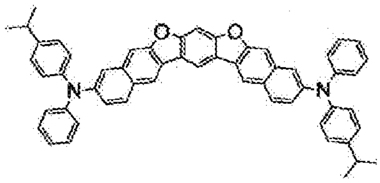
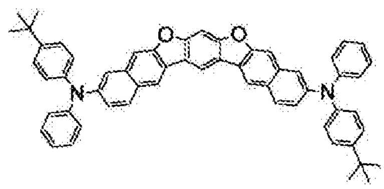

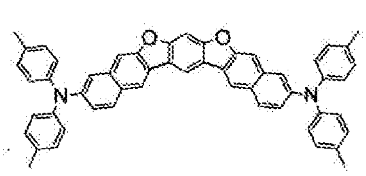
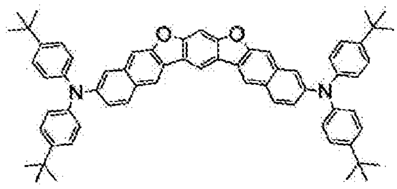
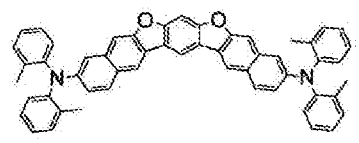

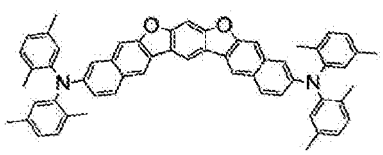
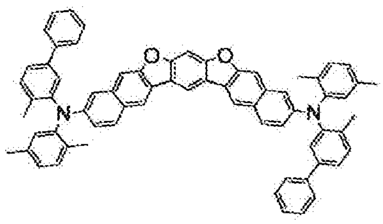

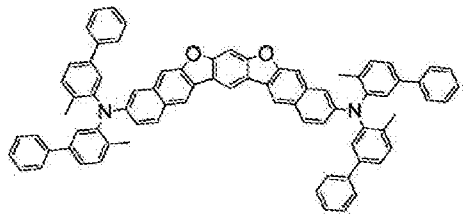
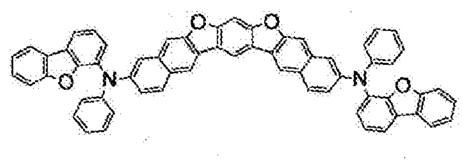

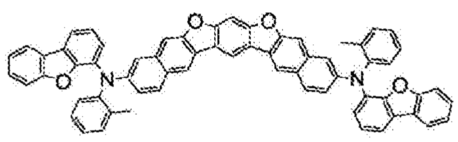
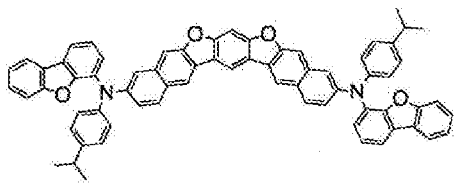

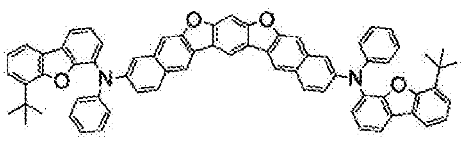
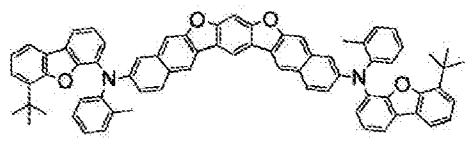

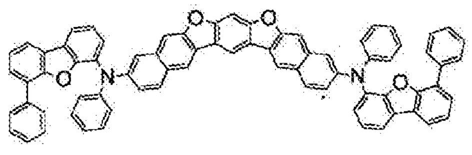
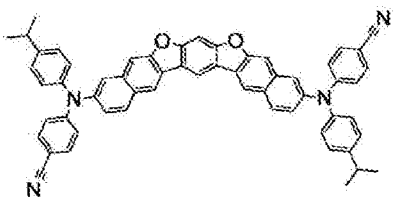

[Formula 462]

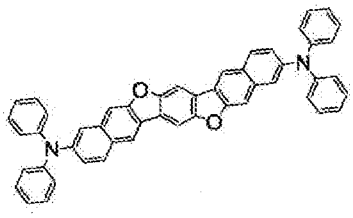
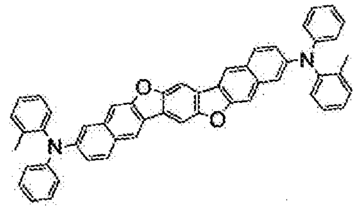
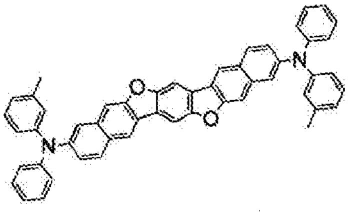
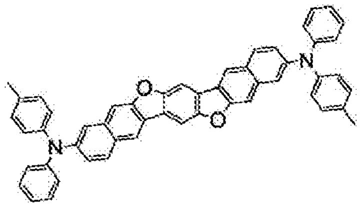
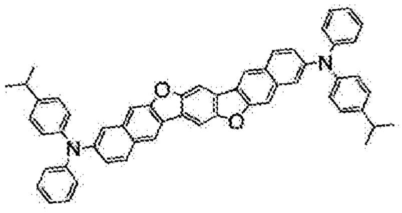
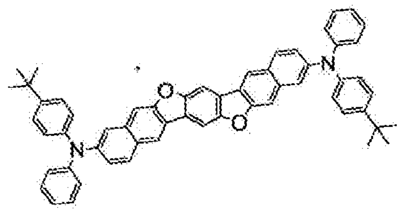
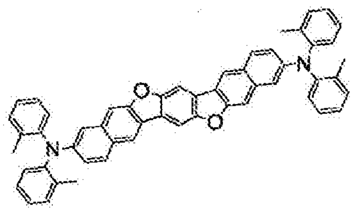
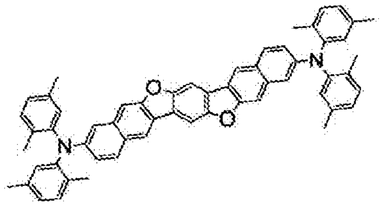
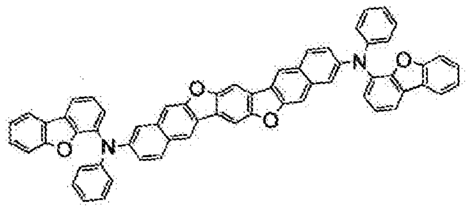
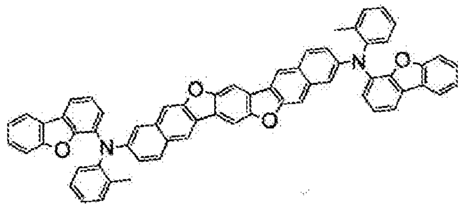
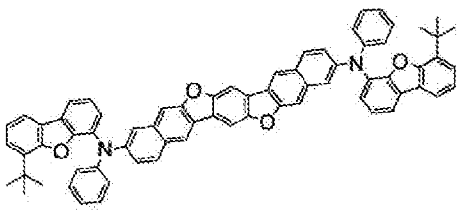
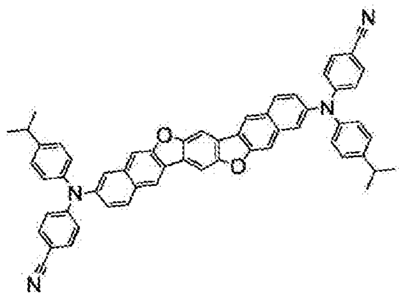
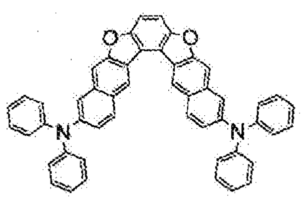
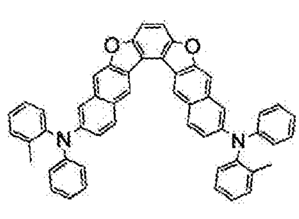
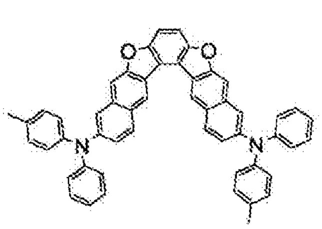

[Formula 463]

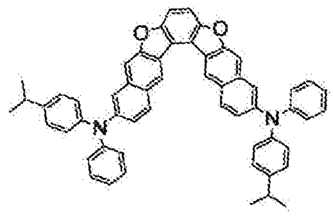
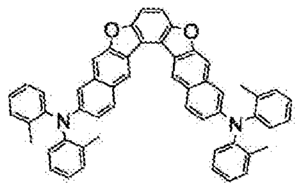
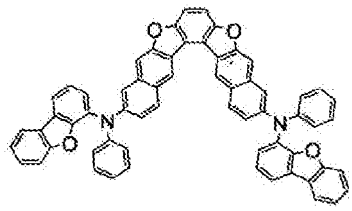
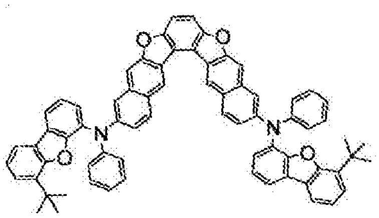
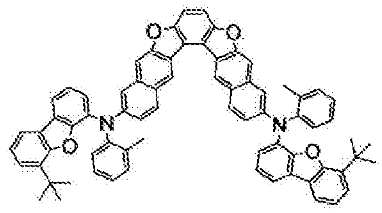
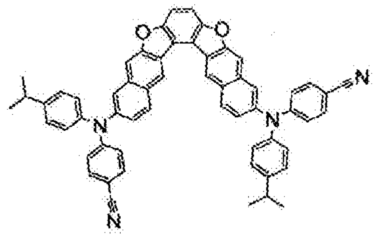
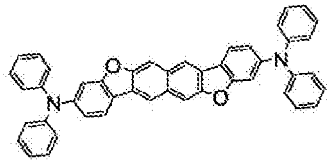
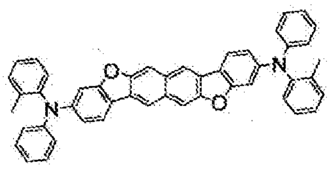
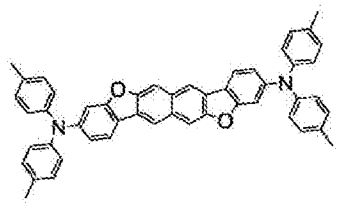
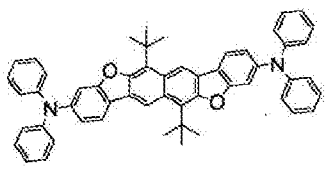
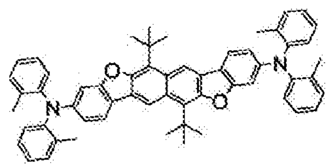
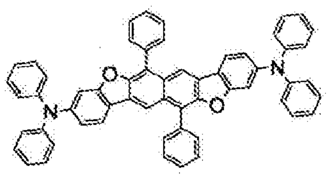
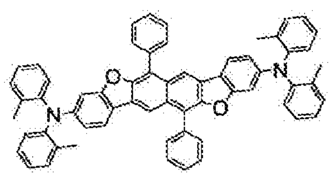
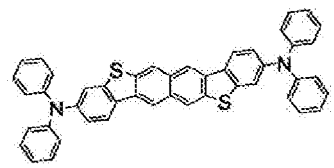
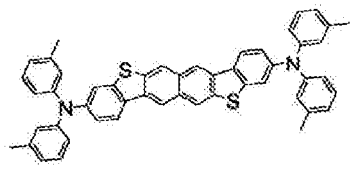
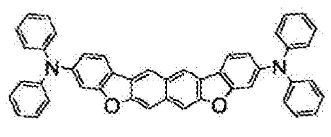
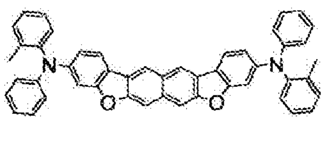
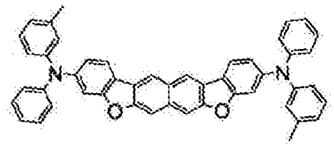
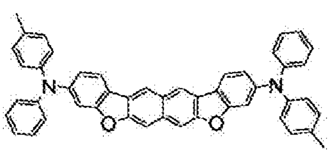
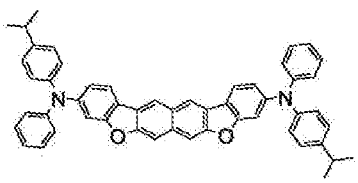
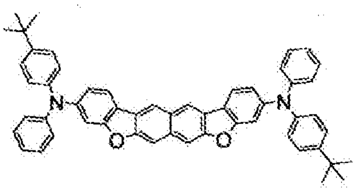

[Formula 464]

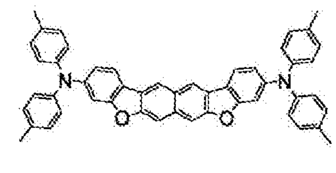
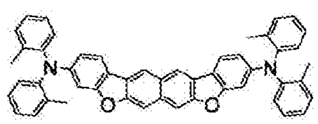
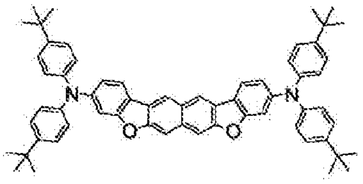

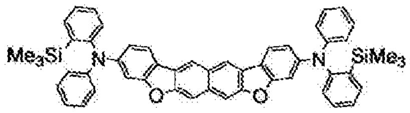
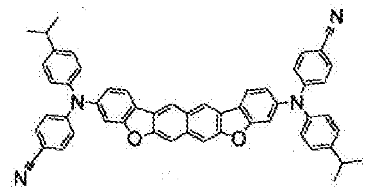

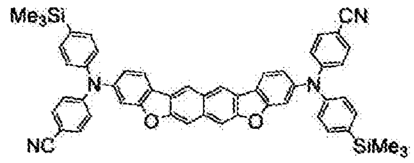
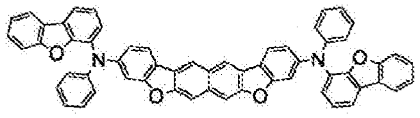

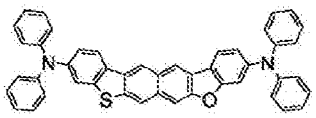
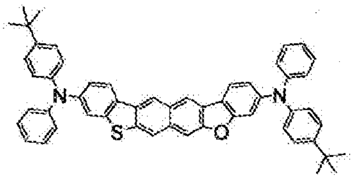
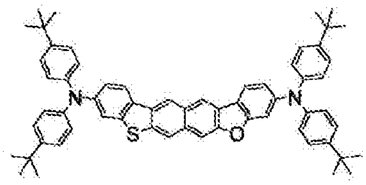

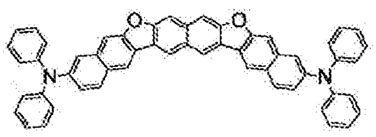
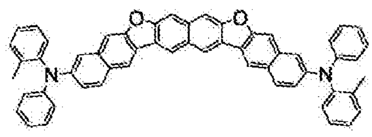

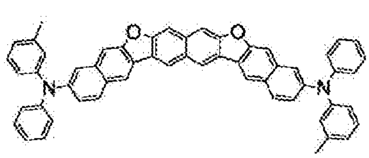
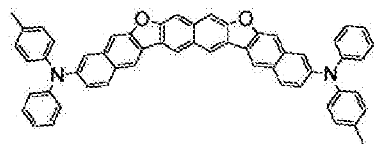

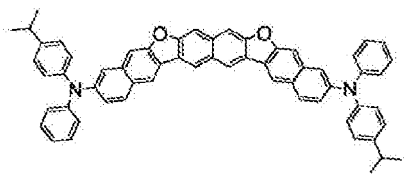
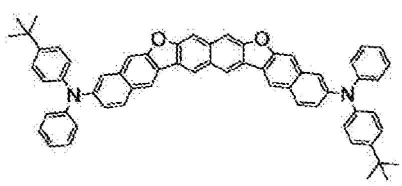

[Formula 465]

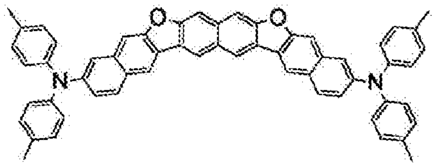

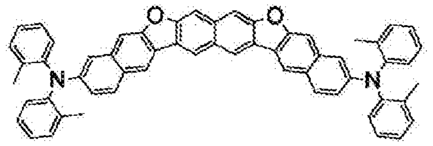

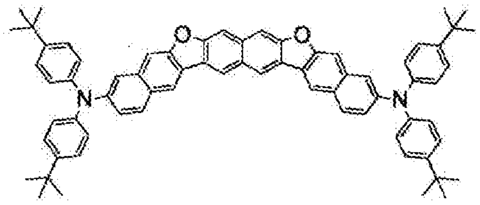

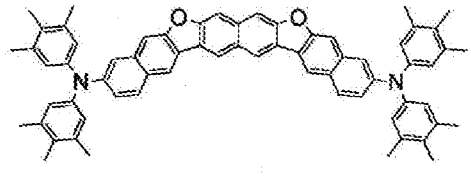

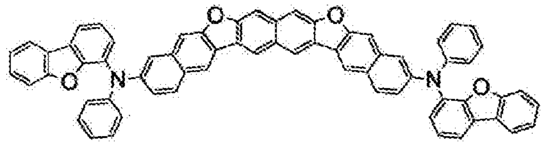

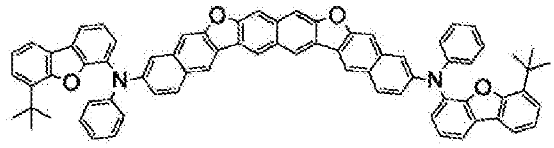

Compound Represented by Formula (8)

A compound represented by the formula (8) will be described below.

[Formula 466]

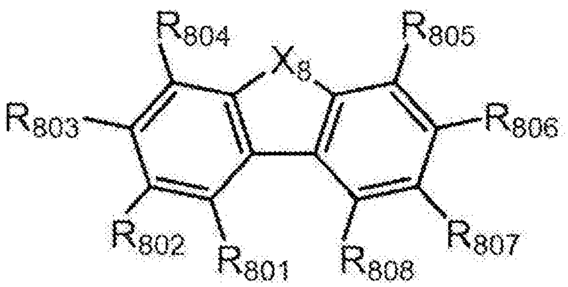

(8)

In the formula (8): at least one combination of $R_{801}$ and $R_{802}$, $R_{802}$ and $R_{803}$, or $R_{803}$ and $R_{804}$ are mutually bonded to form a divalent group represented by a formula (82) below; and
at least one combination of $R_{805}$ and $R_{806}$, $R_{806}$ and $R_{807}$, or $R_{807}$ and $R_{808}$ are mutually bonded to form a divalent group represented by a formula (83) below.

[Formula 467]

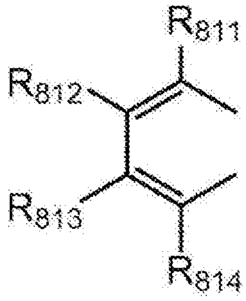

(82)

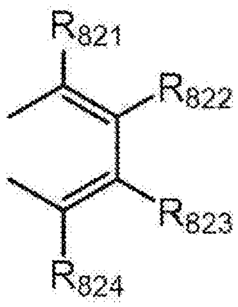

(83)

At least one of $R_{801}$ to $R_{804}$ not forming the divalent group represented by the formula (82) or $R_{811}$ to $R_{814}$ is a monovalent group represented by a formula (84) below;

at least one of $R_{805}$ to $R_{808}$ not forming the divalent group represented by the formula (83) or $R_{821}$ to $R_{824}$ is a monovalent group represented by a formula (84) below;
$X_8$ is an oxygen atom, a sulfur atom, or $NR_{809}$; and
$R_{801}$ to $R_{808}$ not forming the divalent group represented by the formula (82) or (83) and not being the monovalent group represented by the formula (84), $R_{811}$ to $R_{814}$ and $R_{821}$ to $R_{824}$ not being the monovalent group represented by the formula (84), and $R_{809}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group Represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

[Formula 468]

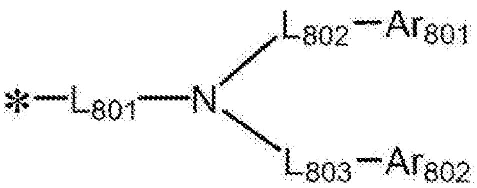

(84)

In the formula (84): $Ar_{801}$ and $Ar_{802}$ are each independently a substituted or unsubstituted aryl group having 6 to 50 ring carbon atoms, or a substituted or unsubstituted heterocyclic group having 5 to 50 ring atoms;

$L_{801}$ to $L_{803}$ are each independently a single bond, a substituted or unsubstituted arylene group having 6 to 30 ring carbon atoms, a substituted or unsubstituted divalent heterocyclic group having 5 to 30 ring atoms, or a divalent linking group formed by bonding two, three or four groups selected from the group consisting of a substituted or unsubstituted arylene group having 6 to 30 ring carbon atoms and a substituted or unsubstituted divalent heterocyclic group having 5 to 30 ring atoms; and

* in the formula (84) represents a bonding position to the cyclic structure represented by the formula (8), a group represented by the formula (82), or a group represented by the formula (83).

In the formula (8), the positions for the divalent group represented by the formula (82) and the divalent group represented by the formula (83) to be formed are not specifically limited but the divalent groups may be formed at any possible positions on $R_{801}$ to $R_{808}$.

In an exemplary embodiment, a compound represented by the formula (8) is represented by any one of formulae (81-1) to (81-6) below.

[Formula 469]

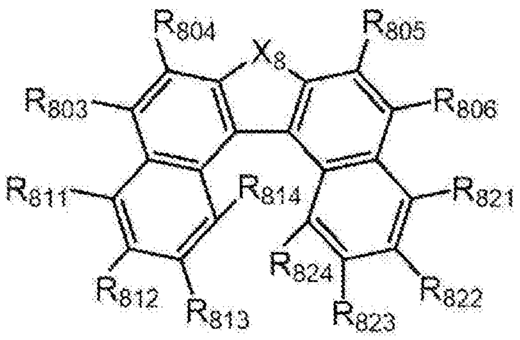

(81-1)

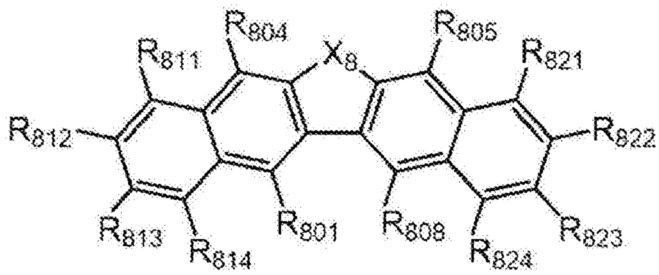

(81-2)

[Formula 470]

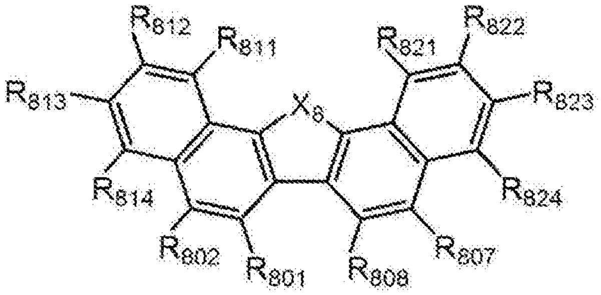

(81-3)

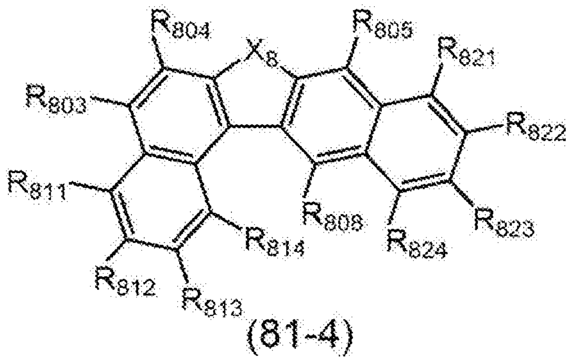

(81-4)

[Formula 471]

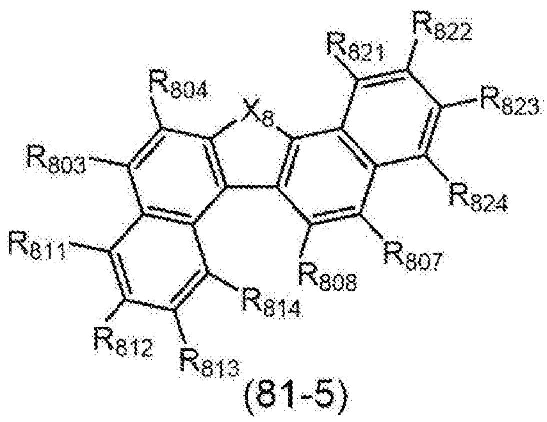

(81-5)

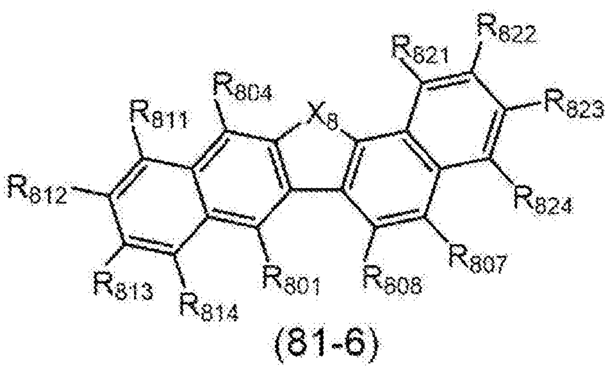

(81-6)

In the formulae (81-1) to (81-6):

$X_8$ represents the same as $X_8$ in the formula (8);

at least two of $R_{801}$ to $R_{824}$ are each a monovalent group represented by the formula (84); and

$R_{801}$ to $R_{824}$ that are not the monovalent group represented by the formula (84) are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group Represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

In an exemplary embodiment, a compound represented by the formula (8) is represented by any one of formulae (81-7) to (81-18) below.

[Formula 472]

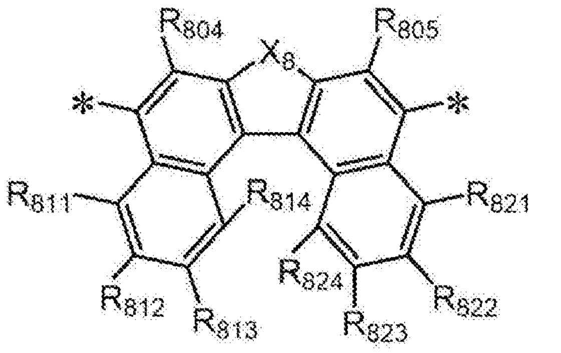

(81-7)

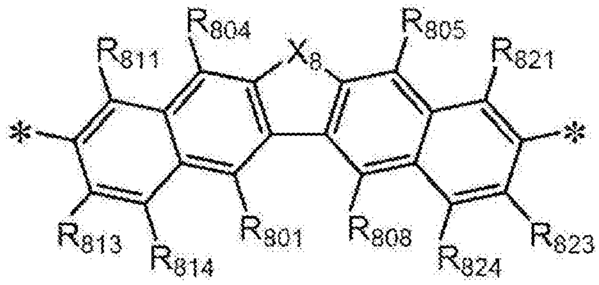

(81-8)

[Formula 473]

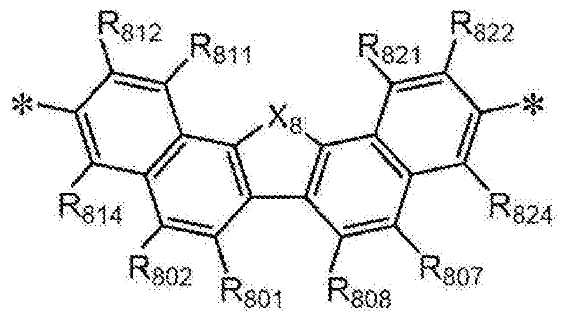

(81-9)

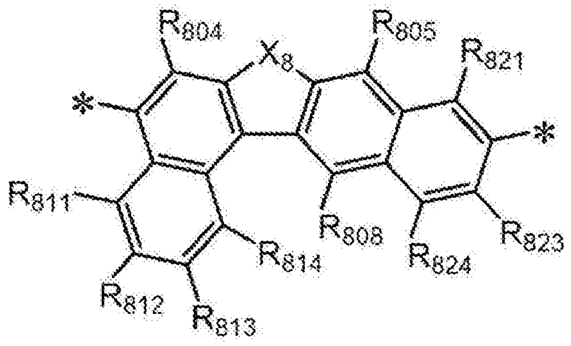

(81-10)

[Formula 474]

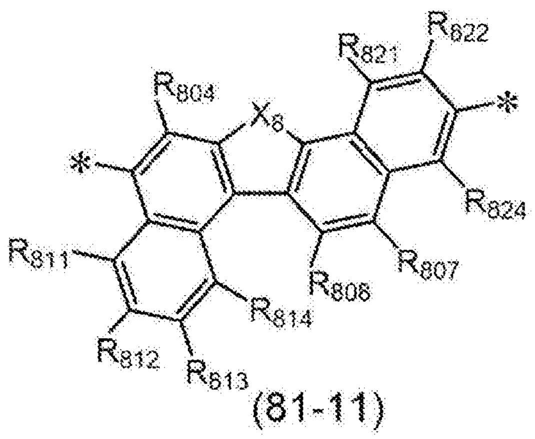

(81-11)

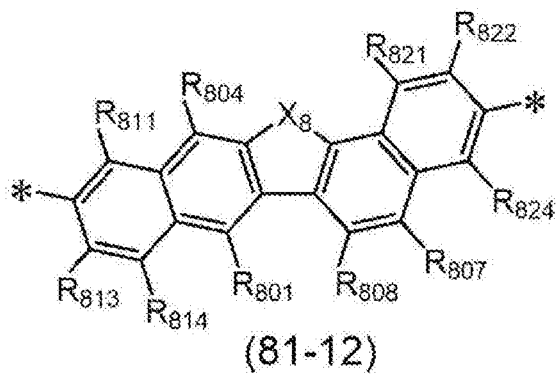

(81-12)

[Formula 475]

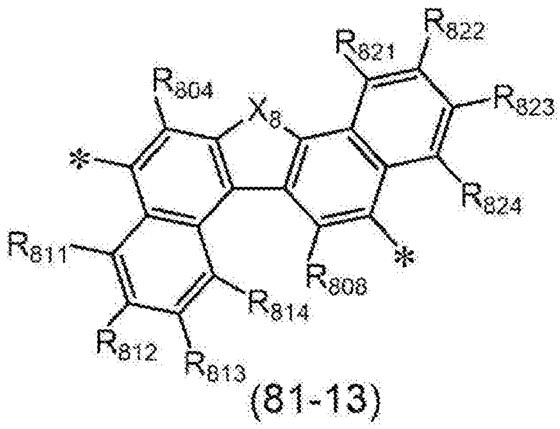

(81-13)

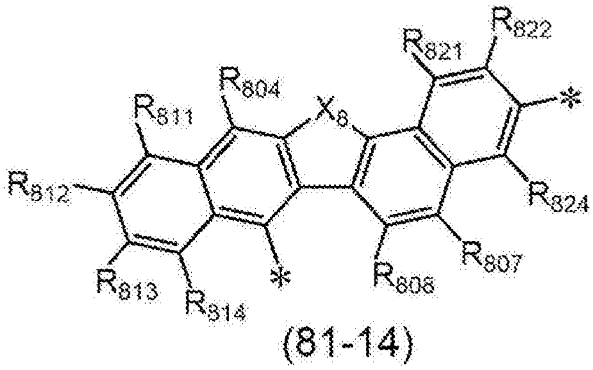

(81-14)

[Formula 476]

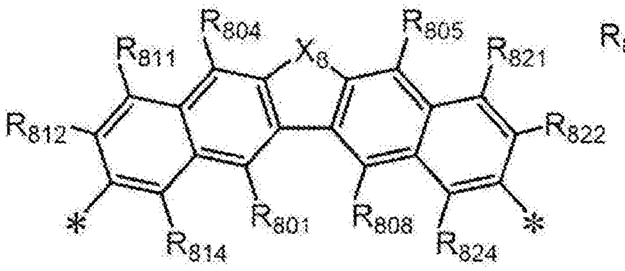

(81-15)

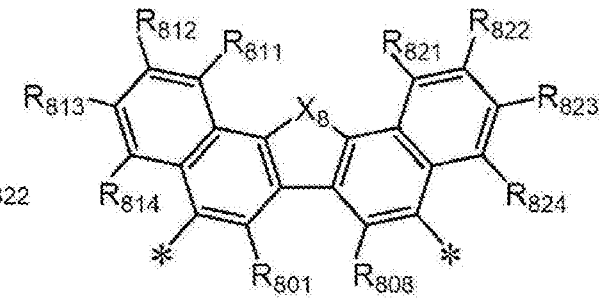

(81-16)

[Formula 477]

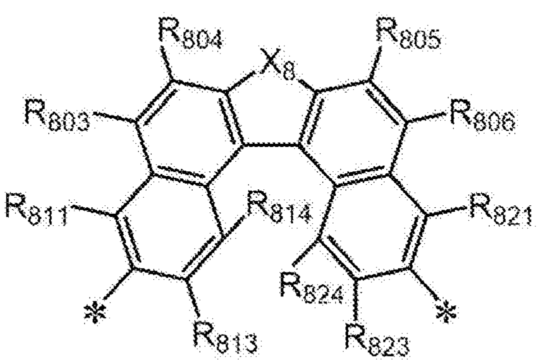

(81-17)

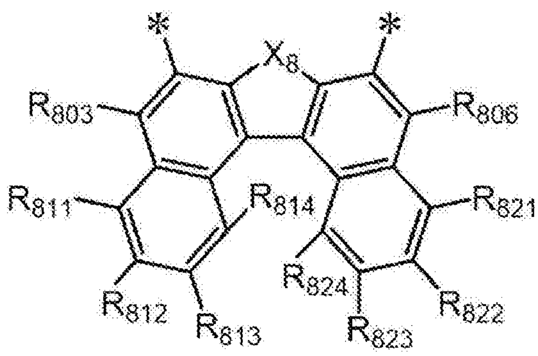

(81-18)

In the formulae (81-7) to (81-18):

$X_8$ represents the same as $X_8$ in the formula (8);
* is a single bond bonded to a monovalent group represented by the formula (84); and
$R_{801}$ to $R_{824}$ each independently represent the same as $R_{801}$ to $R_{824}$ that are each not a monovalent group represented by the formula (84) in the formulae (81-1) to (81-6).

$R_{801}$ to $R_{808}$ not forming the divalent group represented by the formula (82) or (83) and not being the monovalent group represented by the formula (84), and $R_{811}$ to $R_{814}$ and $R_{821}$ to $R_{824}$ not being the monovalent group represented by the formula (84) are preferably each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms.

The monovalent group represented by the formula (84) is preferably represented by a formula (85) or (86) below.

[Formula 478]

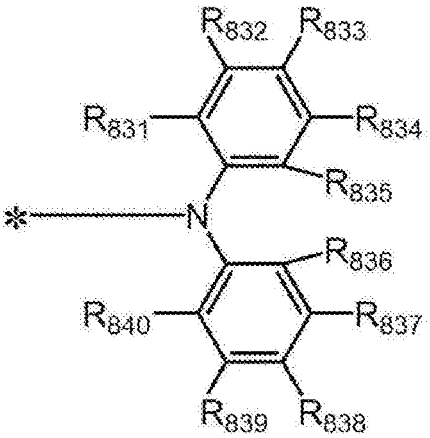

(85)

In the formula (85): $R_{831}$ to $R_{840}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group Represented by $-O-(R_{904})$, a group represented by - $S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms; and

* in the formula (85) represents the same as * in the formula (84).

[Formula 479]

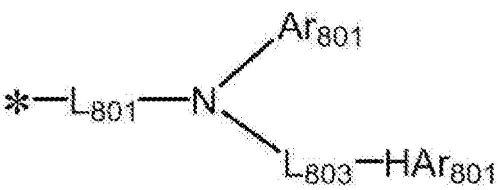

(86)

In the formula (86): $Ar_{801}$, $L_{801}$, and $L_{803}$ represent the same as $Ar_{801}$, $L_{801}$, and $L_{803}$ in the formula (84); and $HAr_{801}$ is a structure represented by a formula (87).

[Formula 480]

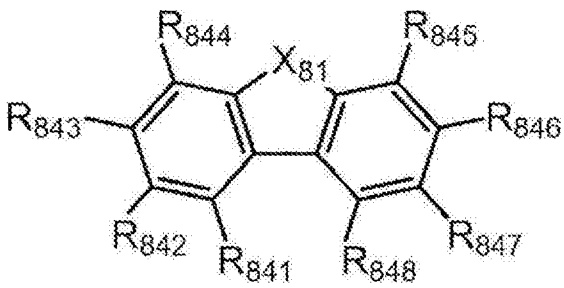

(87)

In the formula (87):

$X_{81}$ is an oxygen atom or a sulfur atom;
one of $R_{841}$ to $R_{848}$ is a single bond with $L_{803}$; and
$R_{841}$ to $R_{848}$ not being the single bond are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group Represented by -O-($R_{904}$), a group represented by - S-($R_{905}$), a group represented by -N($R_{906}$)($R_{907}$), a halogen atom, a cyano group, a nitro group, a substituted or unsubstituted aryl group having 6 to 50 ring carbon atoms, or a substituted or unsubstituted heterocyclic group having 5 to 50 ring atoms.

Specific examples of a compound represented by the formula (8) include compounds shown below as well as the compounds disclosed in WO 2014/104144.

[Formula 481]

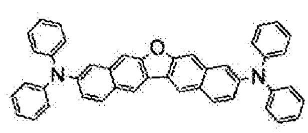
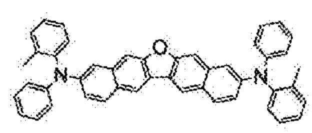
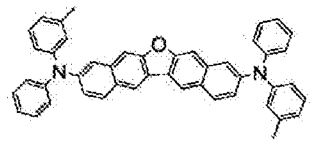

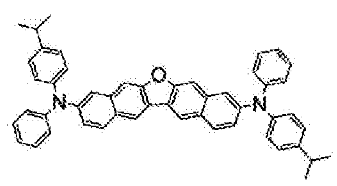
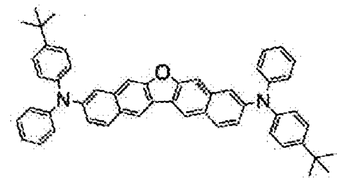
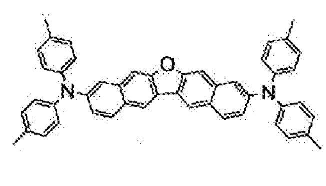

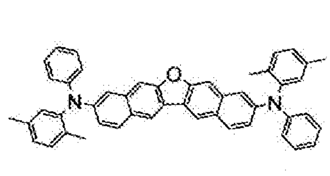
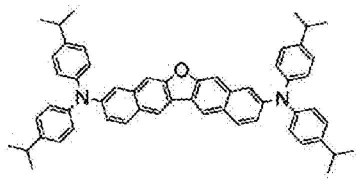
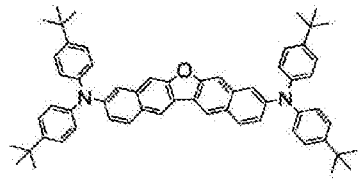

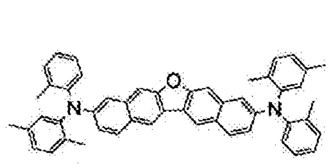
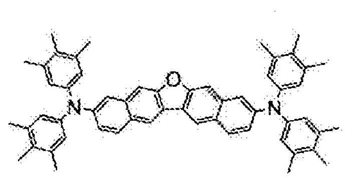
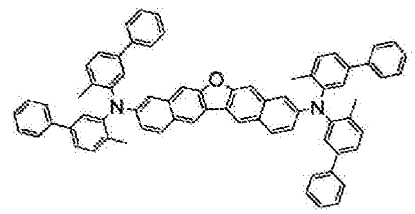

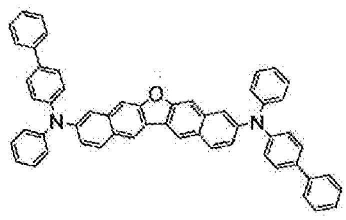
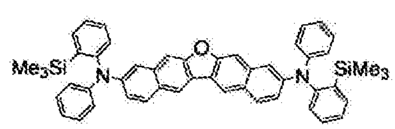
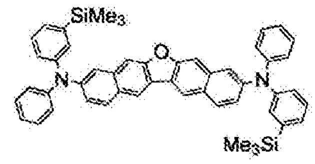

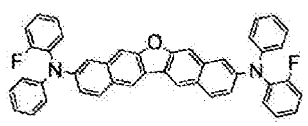
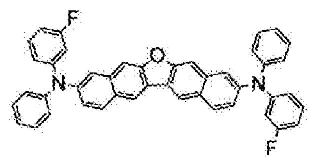
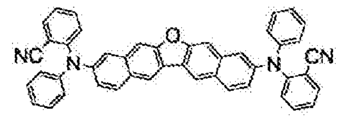

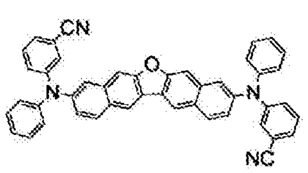
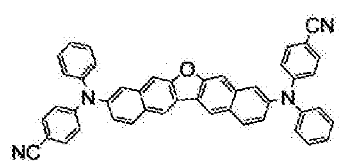
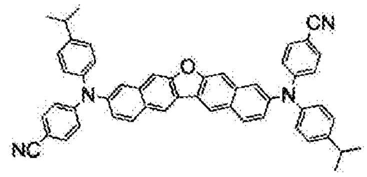

[Formula 482]

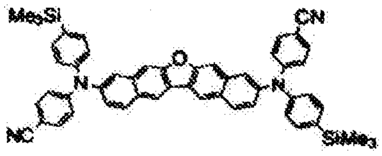
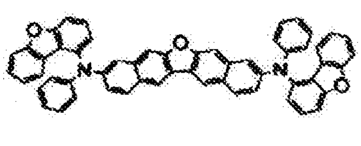
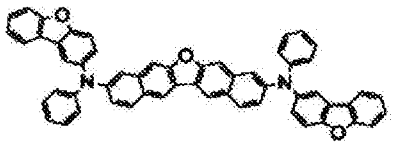

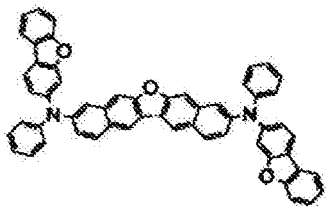
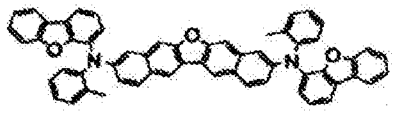
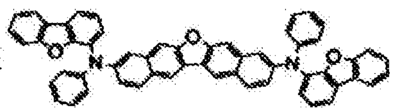

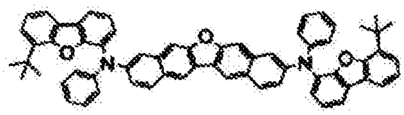
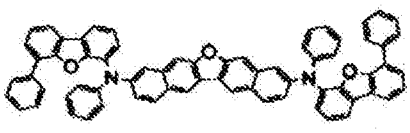
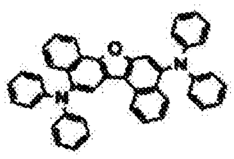

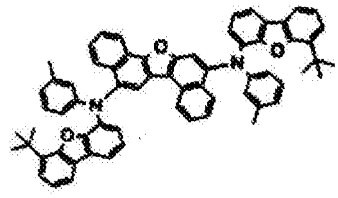
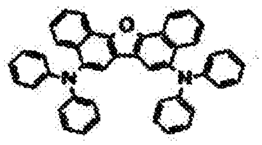
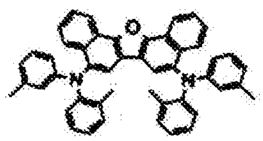

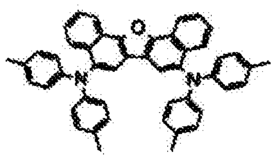

[Formula 483]

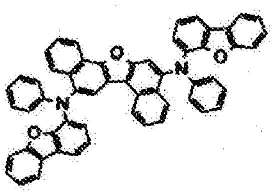
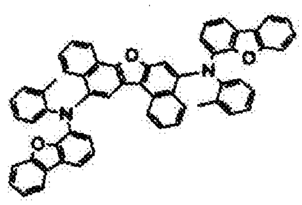
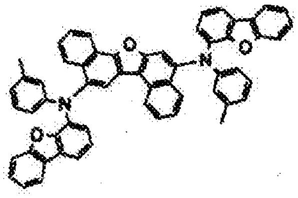
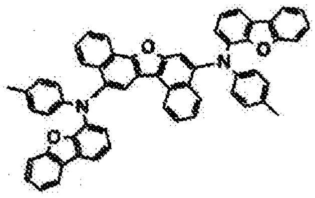
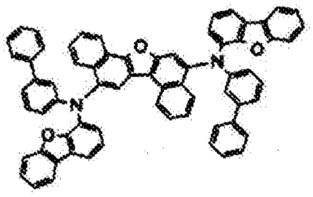
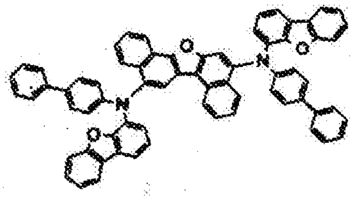
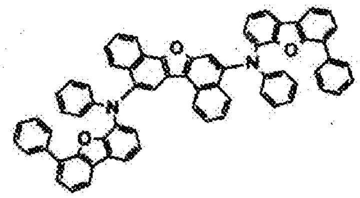
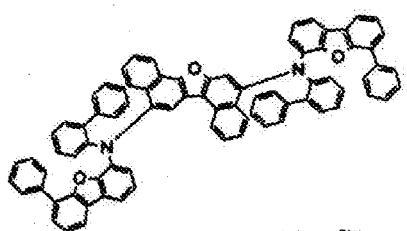
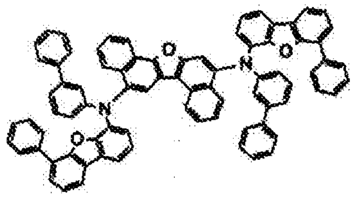
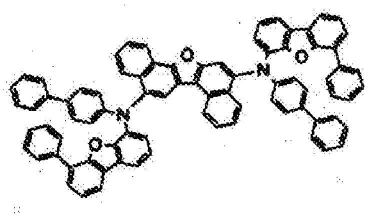
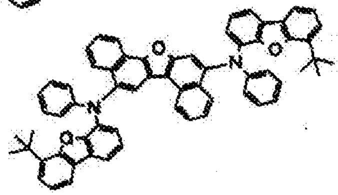
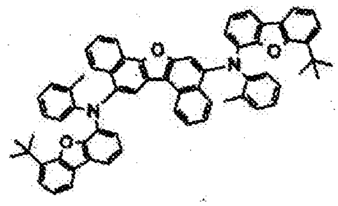

[Formula 484]

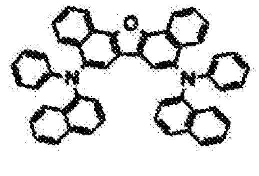
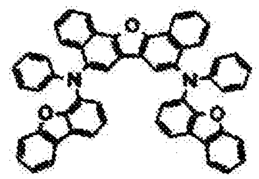
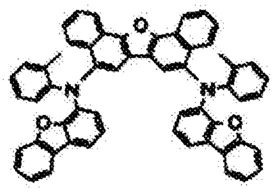
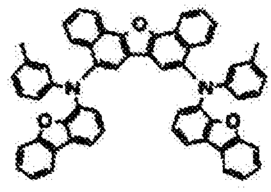

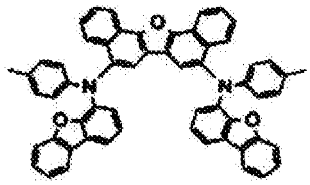
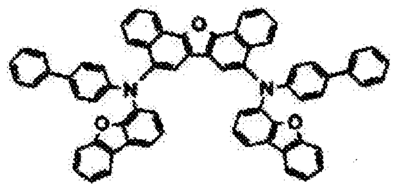

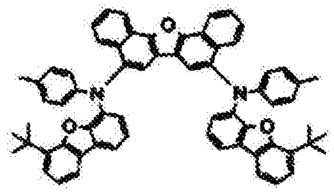
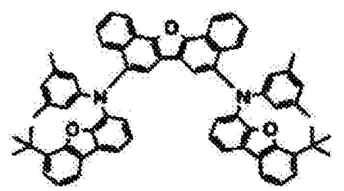

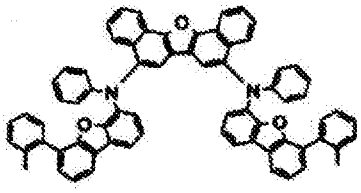
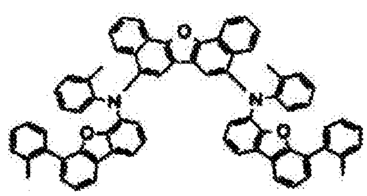

[Formula 485]

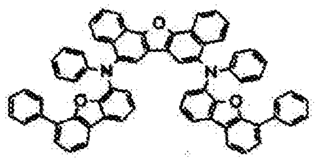

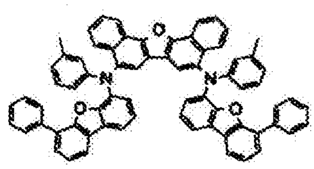

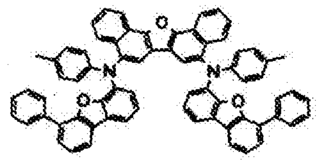

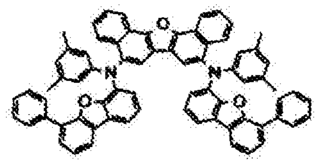

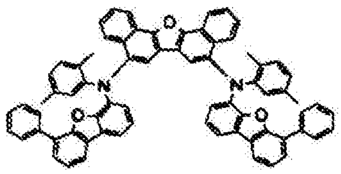

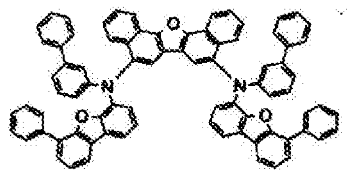

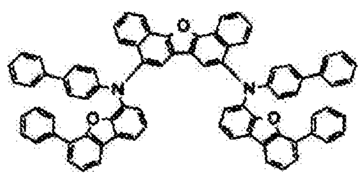

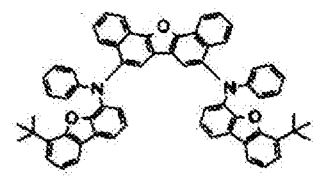

[Formula 486]

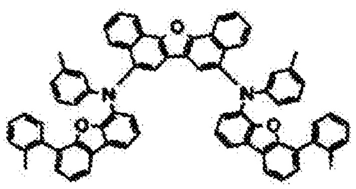
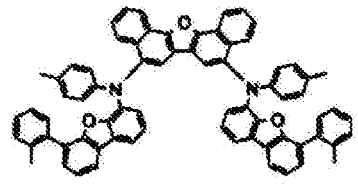
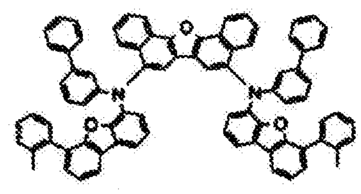

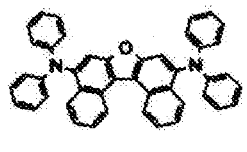
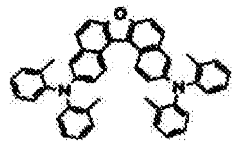
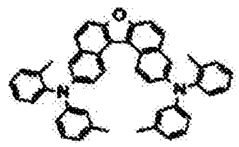

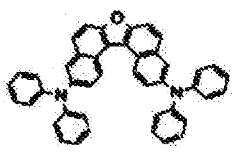
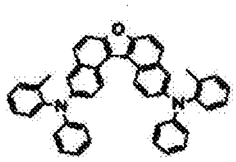
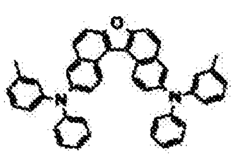
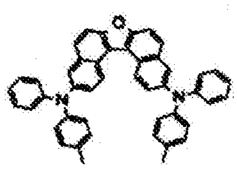

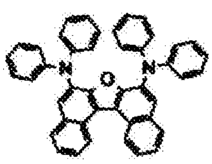
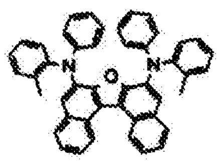
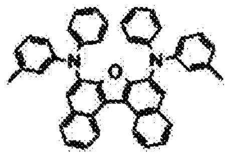
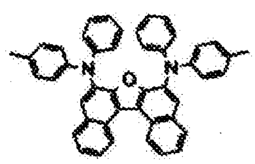

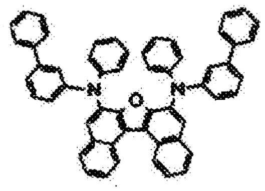
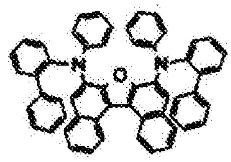

Compound Represented by Formula (9)

A compound represented by the formula (9) will be described below.

[Formula 487]

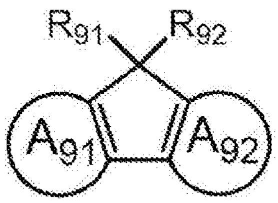

(9)

In the formula (9): $A_{91}$ ring and $A_{92}$ ring are each independently a substituted or unsubstituted aromatic hydrocarbon ring having 6 to 50 ring carbon atoms, or a substituted or unsubstituted heterocycle having 5 to 50 ring atoms; and at least one ring selected from the group consisting of $A_{91}$ ring and $A_{92}$ ring is bonded to * in a structure represented by a formula (92).

[Formula 488]

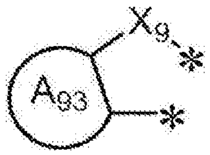

(92)

In the formula (92): $A_{93}$ ring is a substituted or unsubstituted aromatic hydrocarbon ring having 6 to 50 ring carbon atoms, or a substituted or unsubstituted heterocycle having 5 to 50 ring atoms;

$X_9$ is $NR_{93}$, $C(R_{94})(R_{95})$, $Si(R_{96})(R_{97})$, $Ge(R_{98})(R_{99})$, an oxygen atom, a sulfur atom, or a selenium atom;
$R_{91}$ and $R_{92}$ are mutually bonded to form a substituted or unsubstituted monocyclic ring, mutually bonded to form a substituted or unsubstituted fused ring, or not mutually bonded; and
$R_{91}$ and $R_{92}$ forming neither the substituted or unsubstituted monocyclic ring nor the substituted or unsubstituted fused ring, and $R_{93}$ to $R_{99}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group Represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

At least one ring selected from the group consisting of $A_{91}$ ring and $A_{92}$ ring is bonded to a bond * of a structure represented by the formula (92). In other words, the ring-forming carbon atoms of the aromatic hydrocarbon ring or the ring atoms of the heterocycle of the $A_{91}$ ring in an exemplary embodiment are bonded to the bonds * in a structure represented by the formula (92). Further, the ring-forming carbon atoms of the aromatic hydrocarbon ring or the ring atoms of the heterocycle of the $A_{92}$ ring in an exemplary embodiment are bonded to the bonds * in a structure represented by the formula (92).
In an exemplary embodiment, a group represented by a formula (93) below is bonded to one or both of the $A_{91}$ ring and $A_{92}$ ring.

[Formula 489]

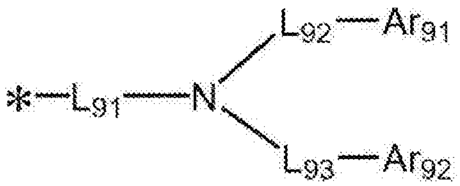

(93)

In the formula (93): $Ar_{91}$ and $Ar_{92}$ are each independently a substituted or unsubstituted aryl group having 6 to 50 ring carbon atoms, or a substituted or unsubstituted heterocyclic group having 5 to 50 ring atoms;

$L_{91}$ to $L_{93}$ are each independently a single bond, a substituted or unsubstituted arylene group having 6 to 30 ring carbon atoms, a substituted or unsubstituted divalent heterocyclic group having 5 to 30 ring atoms, or a divalent linking group formed by bonding two, three or four groups selected from the group consisting of a substituted or unsubstituted arylene group having 6 to 30 ring carbon atoms and a substituted or unsubstituted divalent heterocyclic group having 5 to 30 ring atoms; and
* in the formula (93) represents a bonding position to one of the $A_{91}$ ring and the $A_{92}$ ring.

In an exemplary embodiment, in addition to the $A_{91}$ ring, the ring-forming carbon atoms of the aromatic hydrocarbon ring or the ring atoms of the heterocycle of the $A_{92}$ ring are bonded to the bonds* in a structure represented by the formula (92). In this case, the moieties represented by the formula (92) may be mutually the same or different.
In an exemplary embodiment, $R_{91}$ and $R_{92}$ are each independently a substituted or unsubstituted aryl group having 6 to 50 ring carbon atoms.
In an exemplary embodiment, $R_{91}$ and $R_{92}$ are mutually bonded to form a fluorene structure.

In an exemplary embodiment, the rings $A_{91}$ and $A_{92}$ are each independently a substituted or unsubstituted aromatic hydrocarbon ring having 6 to 50 ring carbon atoms, example of which is a substituted or unsubstituted benzene ring.

In an exemplary embodiment, the ring $A_{93}$ is a substituted or unsubstituted aromatic hydrocarbon ring having 6 to 50 ring carbon atoms, example of which is a substituted or unsubstituted benzene ring.

In an exemplary embodiment, $X_9$ is an oxygen atom or a sulfur atom.

Specific examples of a compound represented by the formula (9) include compounds shown below.

[Formula 490]

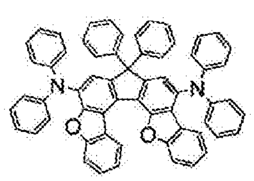
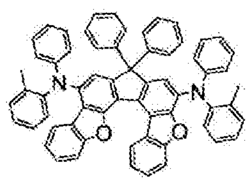
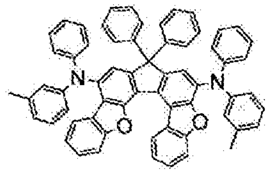

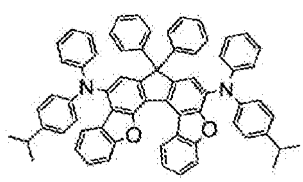
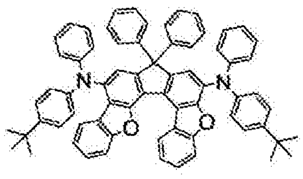
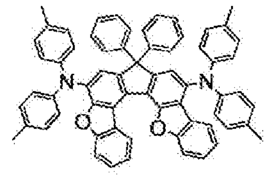

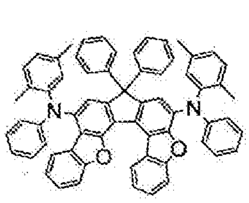
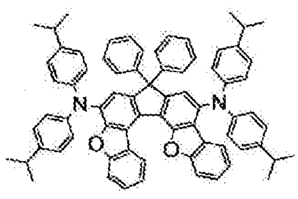
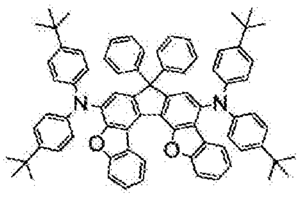

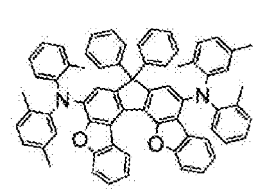
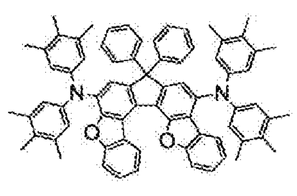
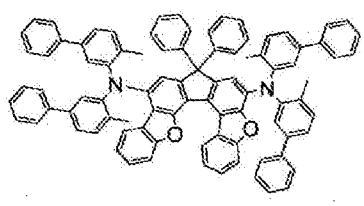

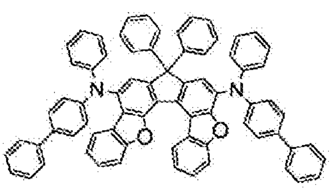
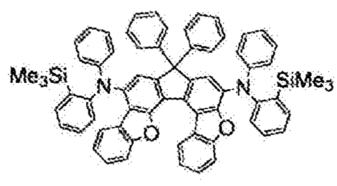
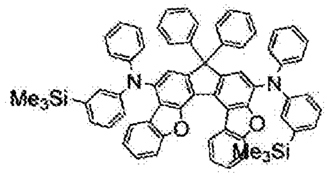

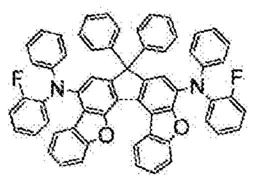
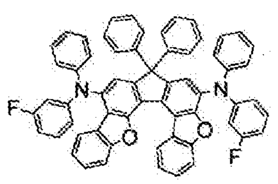
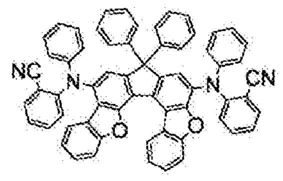

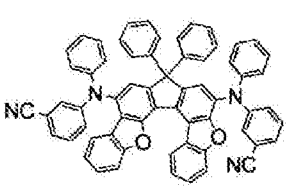
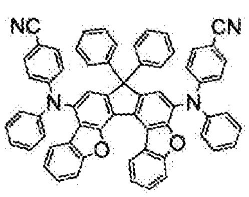
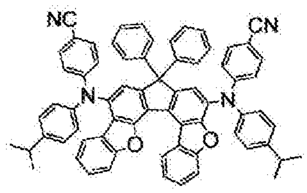

[Formula 491]

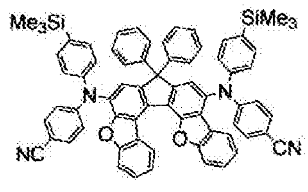
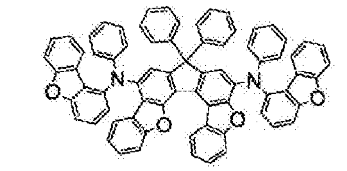
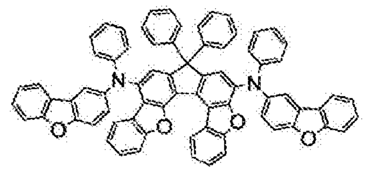

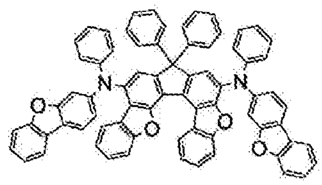
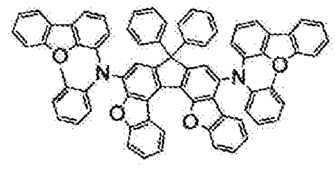
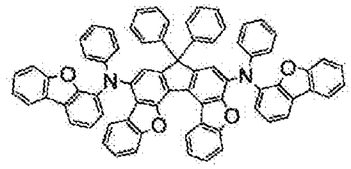

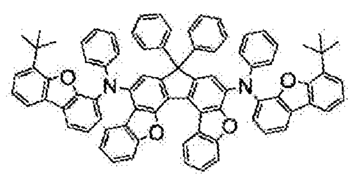
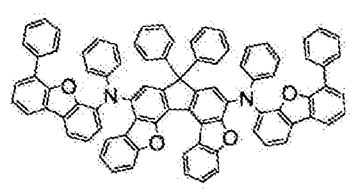

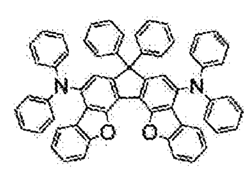
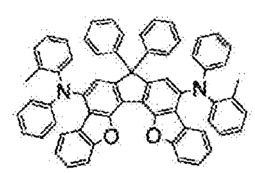
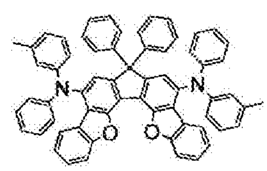

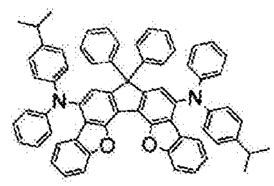
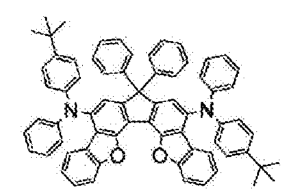
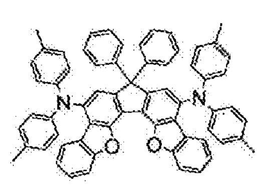

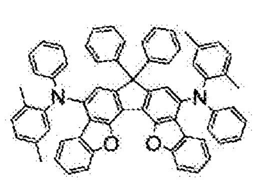
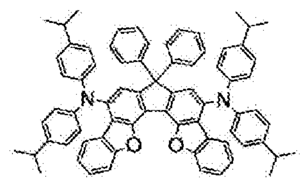
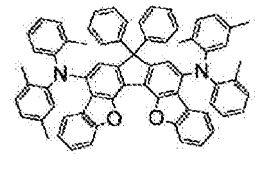

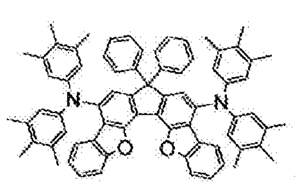
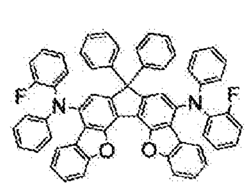
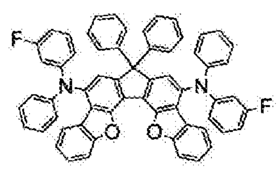

[Formula 492]

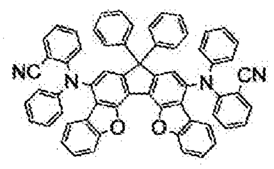
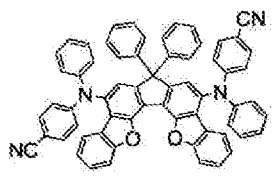
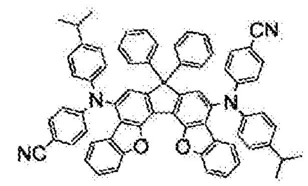

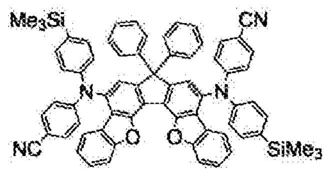
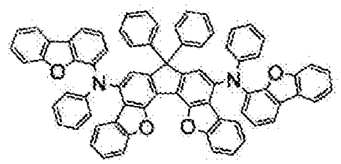

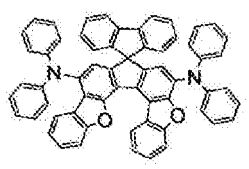
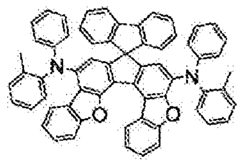
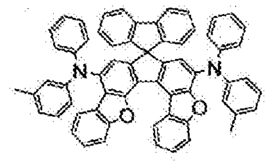

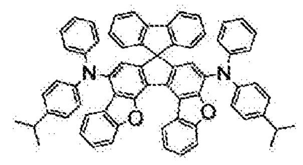
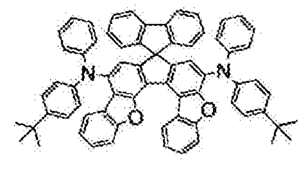
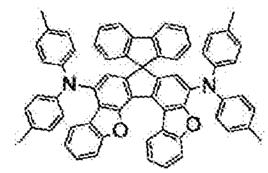

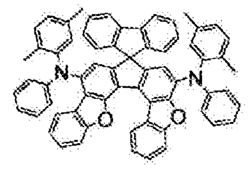
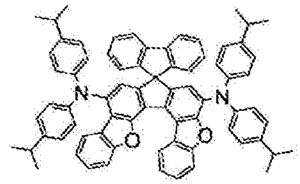
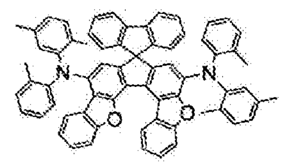

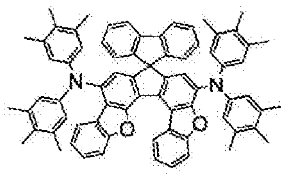
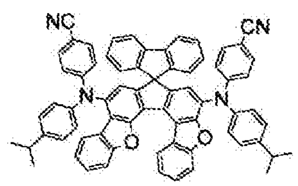
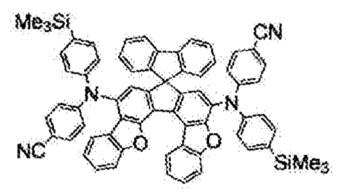

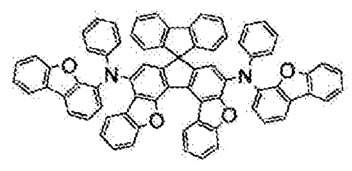

[Formula 493]

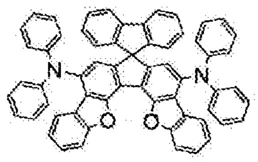
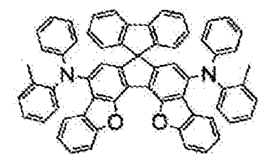
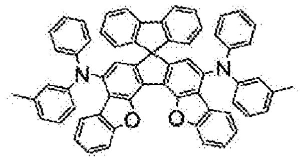

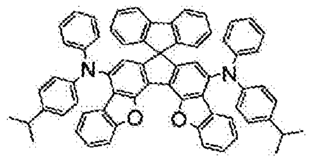
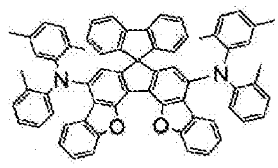
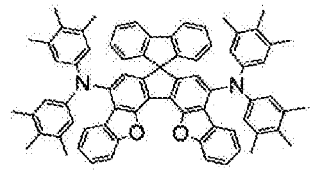

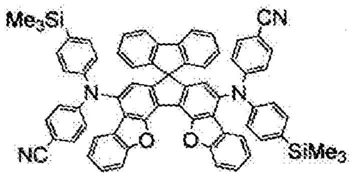
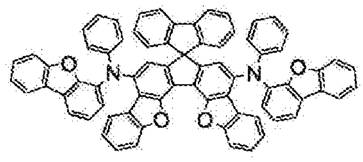

Compound Represented by Formula (10)

A compound represented by the formula (10) will be described below.

[Formula 494]

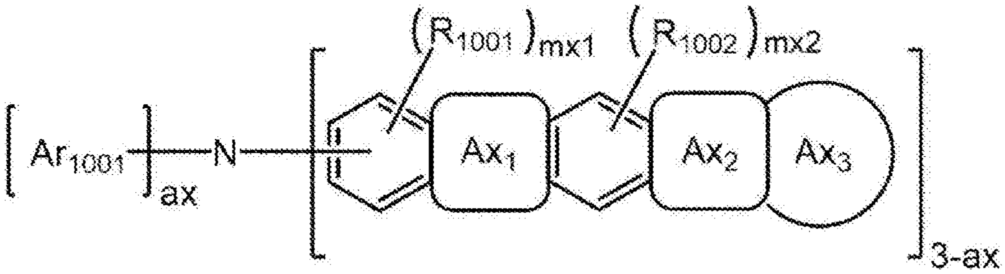

(10)

[Formula 495]

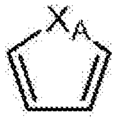
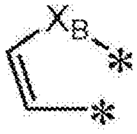

(10a) (10b)

In the formula (10):

$Ax_1$ ring is a ring represented by the formula (10a) and fused with adjacent ring(s) at any position(s);
$Ax_2$ ring is a ring represented by the formula (10b) and fused with adjacent ring(s) at any position(s);
two * in the formula (10b) are bonded to $Ax_3$ ring at any position(s);
$X_A$ and $X_B$ are each independently $C(R_{1003})(R_{1004})$, $Si(R_{1006})(R_{1006})$, an oxygen atom or a sulfur atom;
$Ax_3$ ring is a substituted or unsubstituted aromatic hydrocarbon ring having 6 to 50 ring carbon atoms, or a substituted or unsubstituted heterocycle having 5 to 50 ring atoms;
$Ar_{1001}$ is a substituted or unsubstituted aryl group having 6 to 50 ring carbon atoms, or a substituted or unsubstituted heterocyclic group having 5 to 50 ring atoms;
$R_{1001}$ to $R_{1006}$ are each independently a hydrogen atom, a substituted or unsubstituted alkyl group having 1 to 50

carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group Represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms;

mx1 is 3, mx2 is 2;

a plurality of $R_{1001}$ are are mutually the same or different;

a plurality of $R_{1002}$ are mutually the same or different;

ax is 0, 1, or 2;

when ax is 0 or 1, the structures enclosed by brackets indicated by "3-ax" are mutually the same or different; and when ax is 2, a plurality of $Ar_{1001}$ are mutually the same or different.

In an exemplary embodiment, $Ar_{1001}$ is a substituted or unsubstituted aryl group having 6 to 50 ring carbon atoms.

In an exemplary embodiment, $Ax_3$ ring is a substituted or unsubstituted aromatic hydrocarbon ring having 6 to 50 ring carbon atoms, example of which is a substituted or unsubstituted benzene ring, a substituted or unsubstituted naphthalene ring, or a substituted or unsubstituted anthracene ring.

In an exemplary embodiment, $R_{1003}$ and $R_{1004}$ are each independently a substituted or unsubstituted alkyl group having 1 to 50 carbon atoms.

In an exemplary embodiment, ax is 1.

Specific examples of a compound represented by the formula (10) include compounds shown below.

[Formula 496]

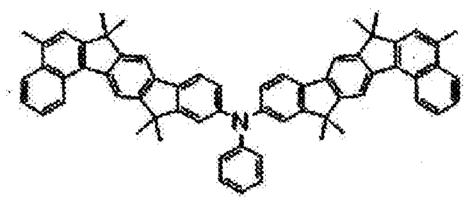

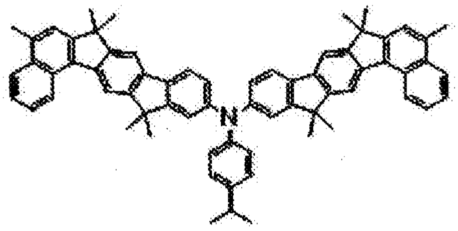

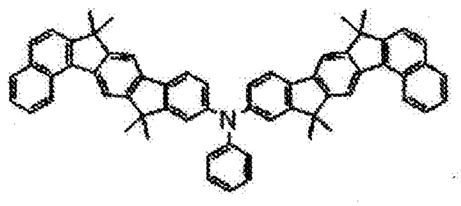

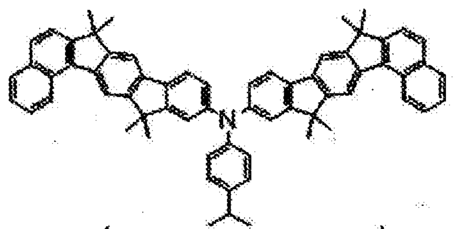

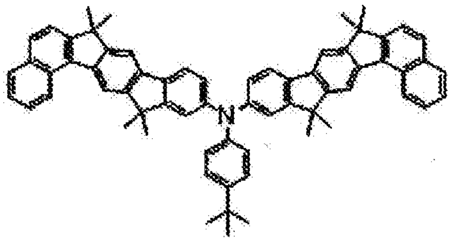

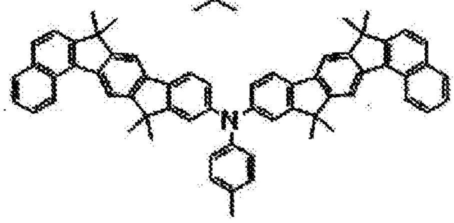

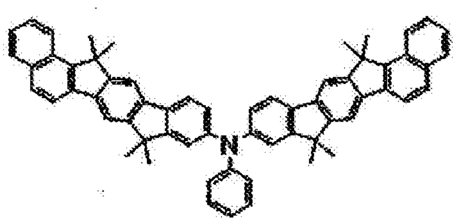

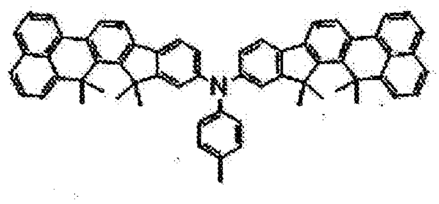

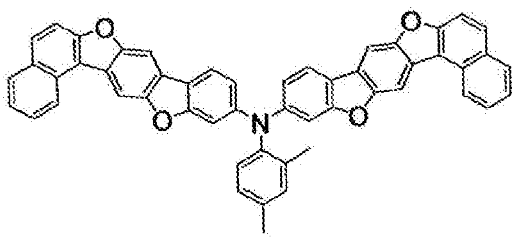

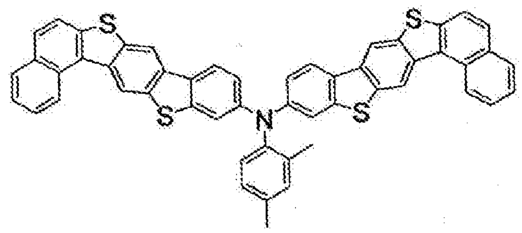

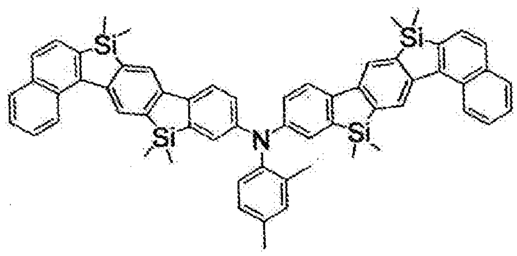

In an exemplary embodiment, the emitting layer contains, as at least one of the third compound or the fourth compound, at least one compound selected from the group consisting of a compound represented by the formula (4), a compound represented by the formula (5), a compound represented by the formula (7), a compound represented by the formula (8), a compound represented by the formula (9), and a compound represented by a formula (63a) below.

[Formula 497]

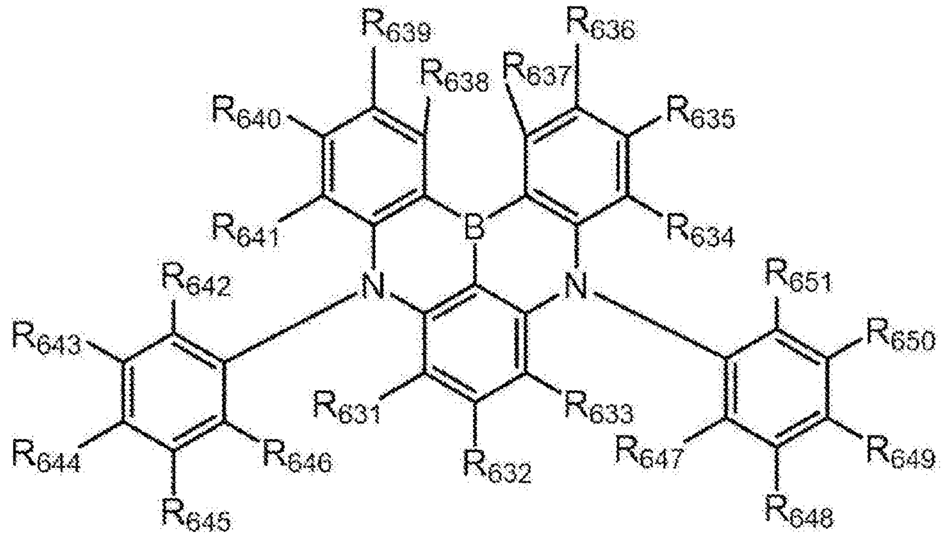

(63a)

In the formula (63a):

$R_{631}$ is bonded with $R_{646}$ to form a substituted or unsubstituted heterocycle, or not bonded to form no substituted or unsubstituted heterocycle;

$R_{633}$ is bonded with $R_{647}$ to form a substituted or unsubstituted heterocycle, or not bonded to form no substituted or unsubstituted heterocycle;

$R_{634}$ is bonded with $R_{651}$ to form a substituted or unsubstituted heterocycle, or not bonded to form no substituted or unsubstituted heterocycle;

$R_{641}$ is bonded with $R_{642}$ to form a substituted or unsubstituted heterocycle, or not bonded to form no substituted or unsubstituted heterocycle;

at least one combination of adjacent two or more of $R_{631}$ to $R_{651}$ are mutually bonded to form a substituted or unsubstituted monocyclic ring, mutually bonded to form a substituted or unsubstituted fused ring, or not mutually bonded;

$R_{631}$ to $R_{651}$ not forming the substituted or unsubstituted heterocycle, the substituted or unsubstituted monocyclic ring, and the substituted or unsubstituted fused ring are each independently a hydrogen atom, a halogen atom, a cyano group, a nitro group, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group Represented by $-O-(R_{904})$, a group represented by $-S-(R_{905})$, a group represented by $-N(R_{906})(R_{907})$, a substituted or unsubstituted aryl group having 6 to 50 ring carbon atoms, or a substituted or unsubstituted heterocyclic group having 5 to 50 ring atoms; and

at least one of $R_{631}$ to $R_{651}$ not forming the substituted or unsubstituted heterocycle, the substituted or unsubstituted monocyclic ring, and the substituted or unsubstituted fused ring is a halogen atom, a cyano group, a nitro group, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group Represented by $-O-(R_{904})$, a group represented by $- S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms.

In an exemplary embodiment, a compound represented by the formula (4) is a compound represented by the formula (41-3), the formula (41-4), or the formula (41-5), the A1 ring in the formula (41-5) being a substituted or unsubstituted fused aromatic hydrocarbon ring having 10 to 50 ring carbon atoms, or a substituted or unsubstituted fused heterocycle having 8 to 50 ring atoms.

In an exemplary embodiment, the substituted or unsubstituted fused aromatic hydrocarbon ring having 10 to 50 ring carbon atoms in the formulae (41-3), (41-4) and (41-5) is a substituted or unsubstituted naphthalene ring, a substituted or unsubstituted anthracene ring, or a substituted or unsubstituted fluorene ring; and

the substituted or unsubstituted fused heterocycle having 8 to 50 ring atoms is a substituted or unsubstituted dibenzofuran ring, a substituted or unsubstituted carbazole ring, or a substituted or unsubstituted dibenzothiophene ring.

In an exemplary embodiment, the substituted or unsubstituted fused aromatic hydrocarbon ring having 10 to 50 ring carbon atoms in the formula (41-3), (41-4) or (41-5) is a substituted or unsubstituted naphthalene ring, or a substituted or unsubstituted fluorene ring; and

the substituted or unsubstituted fused heterocycle having 8 to 50 ring atoms is a substituted or unsubstituted dibenzofuran ring, a substituted or unsubstituted carbazole ring, or a substituted or unsubstituted dibenzothiophene ring.

In an exemplary embodiment, a compound represented by the formula (4) is selected from the group consisting of a compound represented by a formula (461) below, a compound represented by a formula (462) below, a compound represented by a formula (463) below, a compound represented by a formula (464) below, a compound represented by a formula (465) below, a compound represented by a formula (466) below, and a compound represented by a formula (467) below.

[Formula 498]

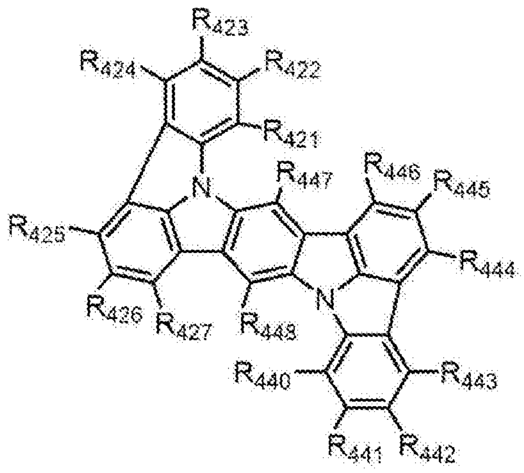

(461)

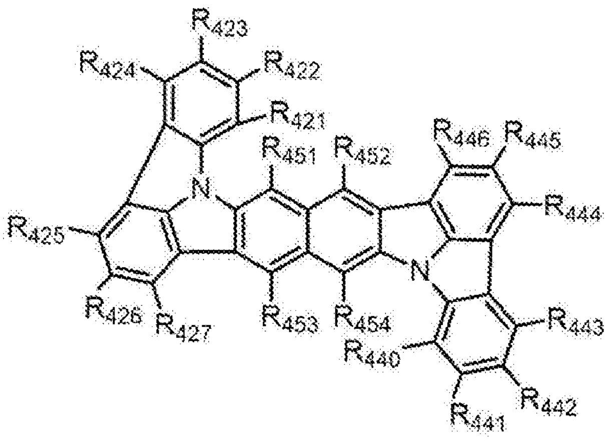

(462)

[Formula 499]

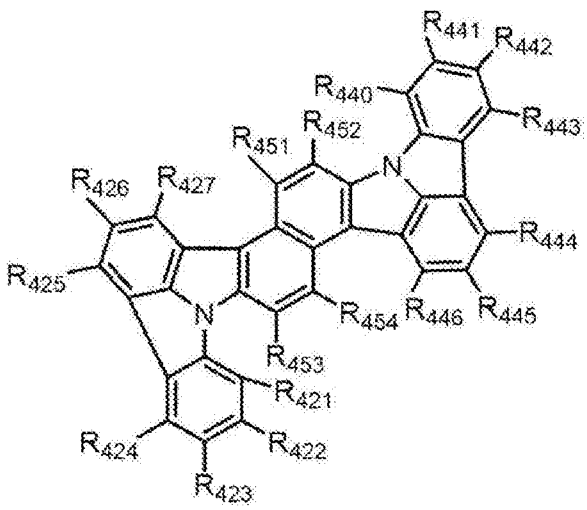

(463)

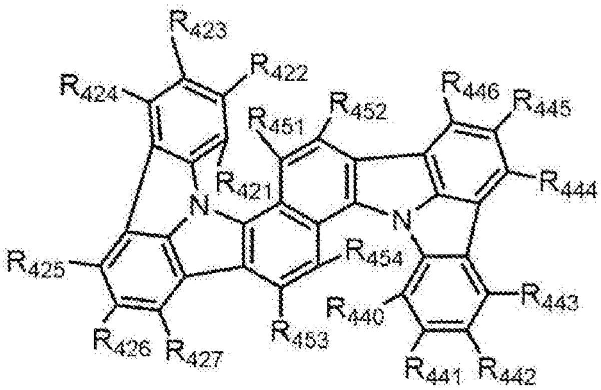

(464)

[Formula 500]

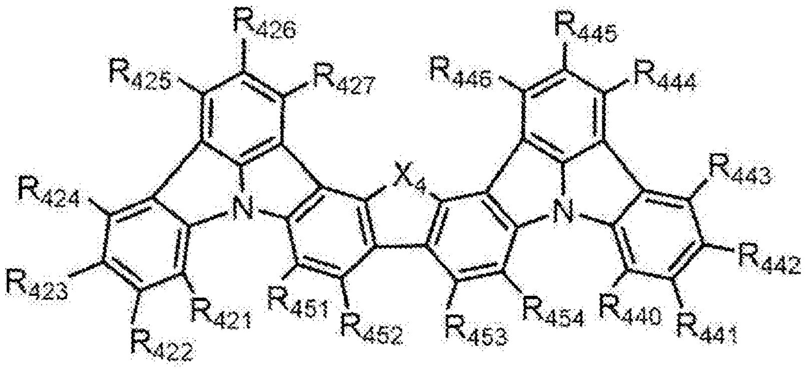

(465)

[Formula 501]

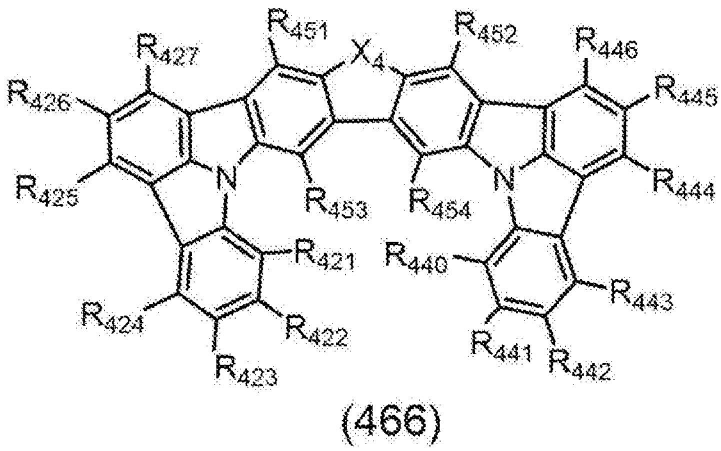

(466)

[Formula 502]

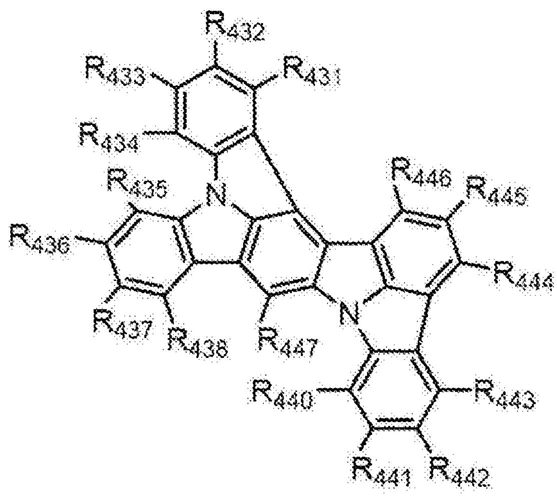

(467)

In the formulae (461) to (467): at least one combination of adjacent two or more of $R_{421}$ to $R_{427}$, $R_{431}$ to $R_{436}$,

$R_{440}$ to $R_{448}$, and $R_{451}$ to $R_{454}$ are mutually bonded to form a substituted or unsubstituted monocyclic ring, mutually bonded to form a substituted or unsubstituted fused ring, or not mutually bonded;

$R_{421}$ to $R_{427}$, $R_{431}$ to $R_{436}$, $R_{440}$ to $R_{448}$ and $R_{451}$ to $R_{454}$ forming neither the substituted or unsubstituted monocyclic ring nor the substituted or unsubstituted fused ring, and $R_{437}$ and $R_{438}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group Represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms;
$X_4$ is an oxygen atom, $NR_{801}$, or $C(R_{802})(R_{803})$;
$R_{801}$, $R_{802}$, and $R_{803}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;
a substituted or unsubstituted alkyl group having 1 to 50 carbon atoms, or a substituted or unsubstituted aryl group having 6 to 50 ring carbon atoms;
when a plurality of $R_{801}$ are present, the plurality of $R_{801}$ are mutually the same or different;
when a plurality of $R_{802}$ are present, the plurality of $R_{802}$ are mutually the same or different; and
when a plurality of $R_{803}$ are present, the plurality of $R_{803}$ are mutually the same or different.

In an exemplary embodiment, $R_{421}$ to $R_{427}$ and $R_{440}$ to $R_{448}$ are each independently a hydrogen atom, a substituted or unsubstituted aryl group having 6 to 50 ring carbon atoms, or a substituted or unsubstituted heterocyclic group having 5 to 50 ring atoms.
In an exemplary embodiment, $R_{421}$ to $R_{427}$ and $R_{440}$ to $R_{447}$ are each independently selected from the group consisting of a hydrogen atom, a substituted or unsubstituted aryl group having 6 to 18 ring carbon atoms, and a substituted or unsubstituted heterocyclic group having 5 to 18 ring atoms.
In an exemplary embodiment, a compound represented by the formula (41-3) is a compound represented by a formula (41-3-1) below.

[Formula 503]

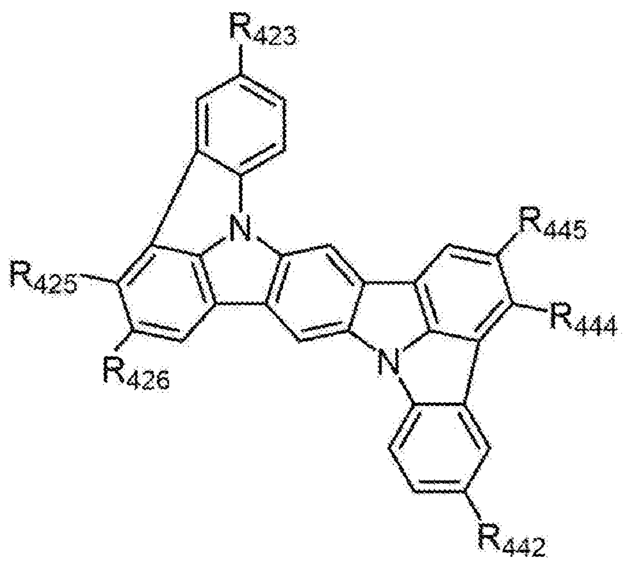

(41-3-1)

In the formula (41-3-1), $R_{423}$, $R_{425}$, $R_{426}$, $R_{442}$, $R_{444}$ and $R_{445}$ each independently represent the same as $R_{423}$, $R_{425}$, $R_{426}$, $R_{442}$, $R_{444}$ and $R_{445}$ in the formula (41-3).
In an exemplary embodiment, a compound represented by the formula (41-3) is a compound represented by a formula (41-3-2) below.

[Formula 504]

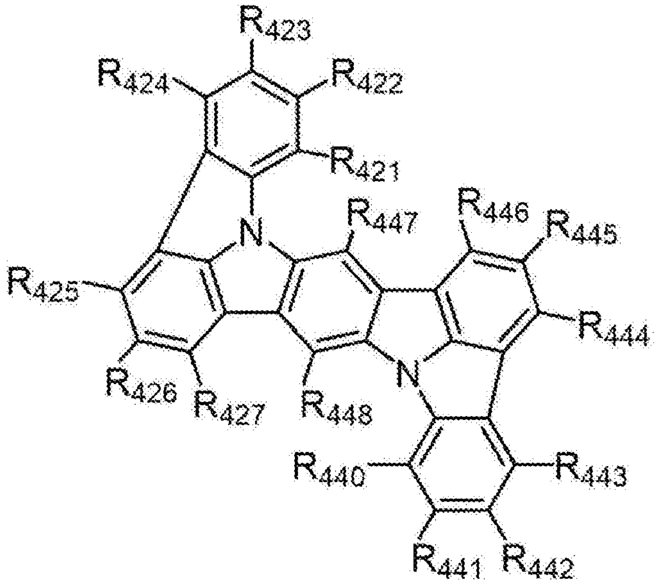

(41-3-2)

In the formula (41-3-2), $R_{421}$ to $R_{427}$ and $R_{440}$ to $R_{448}$ each independently represent the same as $R_{421}$ to $R_{427}$ and $R_{440}$ to $R_{448}$ in the formula (41-3); and

at least one of $R_{421}$ to $R_{427}$ or $R_{440}$ to $R_{446}$ is a group represented by - $N(R_{906})(R_{907})$.

In an exemplary embodiment, two of $R_{421}$ to $R_{427}$ and $R_{440}$ to $R_{446}$ in the formula (41-3-2) are each a group represented by $-N(R_{906})(R_{907})$.

In an exemplary embodiment, a compound represented by the formula (41-3-2) is a compound represented by a formula (41-3-3) below.

[Formula 505]

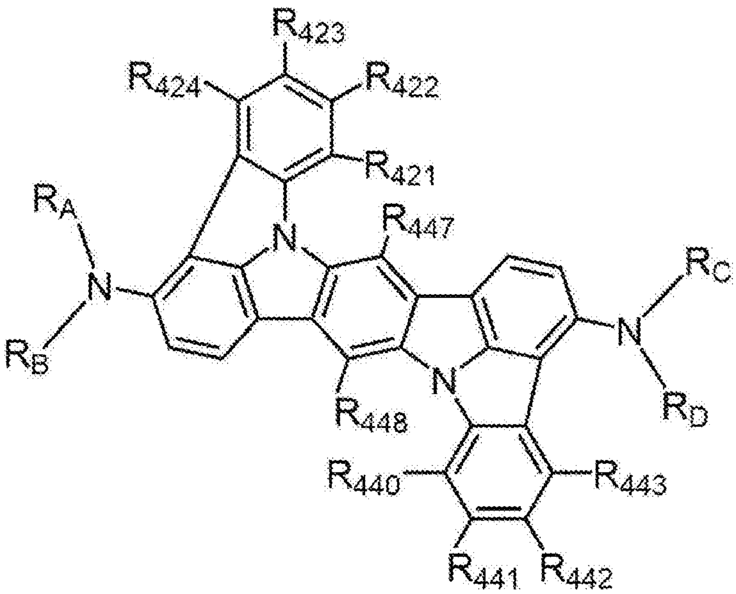

(41-3-3)

In the formula (41-3-3), $R_{421}$ to $R_{424}$, $R_{440}$ to $R_{443}$, $R_{447}$, and $R_{448}$ each independently represent the same as $R_{421}$ to $R_{424}$, $R_{440}$ to $R_{443}$, $R_{447}$, and $R_{448}$ in the formula (41-3); and

$R_A$, $R_B$, Rc, and $R_D$ are each independently a substituted or unsubstituted aryl group having 6 to 18 ring carbon atoms, or a substituted or unsubstituted heterocyclic group having 5 to 18 ring atoms.

In an exemplary embodiment, a compound represented by the formula (41-3-3) is a compound represented by a formula (41-3-4) below.

[Formula 506]

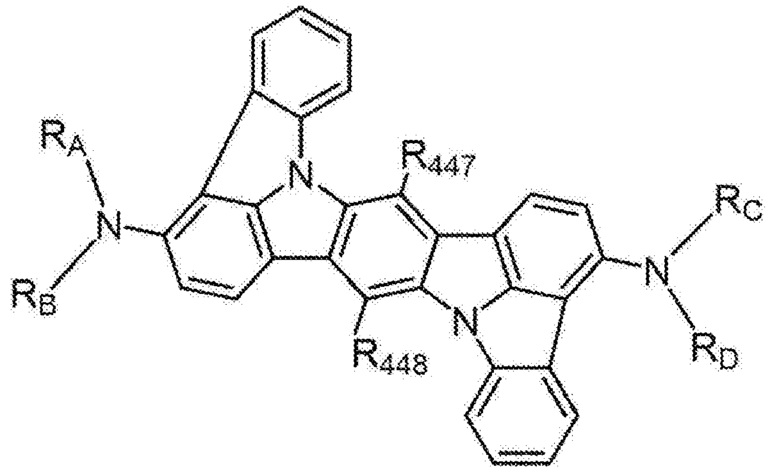

(41-3-4)

In the formula (41-3-4), $R_{447}$, $R_{448}$, $R_A$, $R_B$, Rc and $R_D$ each independently represent the same as $R_{447}$, $R_{448}$, $R_A$, $R_B$, Rc and $R_D$ in the formula (41-3-3).

In an exemplary embodiment, $R_A$, $R_B$, $R_C$, and $R_D$ are each independently a substituted or unsubstituted aryl group having 6 to 18 ring carbon atoms.

In an exemplary embodiment, $R_A$, $R_B$, $R_C$, and $R_D$ are each independently a substituted or unsubstituted phenyl group.

In an exemplary embodiment, $R_{447}$ and $R_{448}$ are each a hydrogen atom.

In an exemplary embodiment, a substituent for "substituted or unsubstituted" group in each of the formulae is an unsubstituted alkyl group having 1 to 50 carbon atoms, an unsubstituted alkenyl group having 2 to 50 carbon atoms, an unsubstituted alkynyl group having 2 to 50 carbon atoms, an unsubstituted cycloalkyl group having 3 to 50 ring carbon atoms, $-Si(R_{901a})(R_{902a})(R_{903a})$, $-O-(R_{904a})$, $-S-(R_{905a})$, $-N(R_{906a})(R_{907a})$, a halogen atom, a cyano group, a nitro group, an unsubstituted aryl group having 6 to 50 ring carbon atoms, or an unsubstituted heterocyclic group having 5 to 50 ring atoms;

$R_{901a}$ to $R_{907a}$ are each independently a hydrogen atom, an unsubstituted alkyl group having 1 to 50 carbon atoms, an unsubstituted aryl group having 6 to 50 ring carbon atoms, or an unsubstituted heterocyclic group having 5 to 50 ring atoms;
when two or more $R_{901a}$ are present, the two or more $R_{901a}$ are mutually the same or different;
when two or more $R_{902a}$ are present, the two or more $R_{902a}$ are mutually the same or different;
when two or more $R_{903a}$ are present, the two or more $R_{903a}$ are mutually the same or different;
when two or more $R_{904a}$ are present, the two or more $R_{904a}$ are mutually the same or different;
when two or more $R_{905a}$ are present, the two or more $R_{905a}$ are mutually the same or different;
when two or more $R_{906a}$ are present, the two or more $R_{906a}$ are mutually the same or different; and
when two or more $R_{907a}$ are present, the two or more $R_{907a}$ are mutually the same or different.

In an exemplary embodiment, a substituent for "substituted or unsubstituted" group in each of the formulae is an unsubstituted alkyl group having 1 to 50 carbon atoms, an unsubstituted aryl group having 6 to 50 ring carbon atoms, or an unsubstituted heterocyclic group having 5 to 50 ring atoms.

In an exemplary embodiment, a substituent for "substituted or unsubstituted" group in each of the formulae is an unsubstituted alkyl group having 1 to 18 carbon atoms, an unsubstituted aryl group having 6 to 18 ring carbon atoms, or an unsubstituted heterocyclic group having 5 to 18 ring atoms.

In the organic EL device according to the exemplary embodiment, it is preferable that the first emitting layer further contains the third compound that fluoresces and the third compound is a compound emitting a light having a main peak wavelength in a range from 430 nm to 480 nm.

In the organic EL device according to the exemplary embodiment, it is preferable that the second emitting layer further contains the fourth compound that fluoresces and the fourth compound is a compound emitting a light having a main peak wavelength in a range from 430 nm to 480 nm.

A measurement method of a main peak wavelength of the compound is as follows. A toluene solution of a measurement target compound at a concentration ranging from $10^{-6}$ mol/L to $10^{-5}$ mol/L is prepared and put in a quartz

cell. An emission spectrum (ordinate axis: emission intensity, abscissa axis: wavelength) of the thus-obtained sample is measured at a normal temperature (300K). The emission spectrum is measurable using a spectrophotometer (machine name: F-7000) manufactured by Hitachi High-Tech Science Corporation. It should be noted that the machine for measuring the emission spectrum is not limited to the machine used herein.

A peak wavelength of the emission spectrum exhibiting the maximum luminous intensity is defined as a main peak wavelength. It should be noted that the main peak wavelength is sometimes referred to as a fluorescence main peak wavelength (FL-peak) herein.

In a case where the first emitting layer contains the first compound and the third compound in the organic EL device according to the exemplary embodiment, it is preferable that the first compound is a host material (sometimes referred to as a matrix material) and the third compound is a dopant material (sometimes referred to as a guest material, emitter, or luminescent material).

When the first emitting layer of the organic EL device according to the exemplary embodiment contains the first compound and the third compound, a singlet energy $S_1(H1)$ of the first compound and a singlet energy $S_1(D3)$ of the third compound preferably satisfy a relationship of a numerical formula (Numerical Formula 1) below.

$$S_1(H1) > S_1(D3) \qquad \text{(Numerical Formula 1)}$$

In a case where the second emitting layer contains the second compound and the fourth compound in the organic EL device according to the exemplary embodiment, it is preferable that the second compound is a host material (sometimes referred to as a matrix material) and the fourth compound is a dopant material (sometimes referred to as a guest material, emitter, or luminescent material).

When the second emitting layer of the organic EL device according to the exemplary embodiment contains the second compound and the fourth compound, a singlet energy $S_1(H2)$ of the second compound and a singlet energy $S_1(D4)$ of the fourth compound preferably satisfy a relationship of a numerical formula (Numerical Formula 2) below.

$$S_1(H2) > S_1(D4) \qquad \text{(Numerical Formula 2)}$$

Singlet Energy $S_1$

A method of measuring a singlet energy $S_1$ with use of a solution (sometimes referred to as a solution method) is exemplified by a method below.

A toluene solution of a measurement target compound at a concentration ranging from $10^{-5}$ mol/L to $10^{-4}$ mol/L is prepared and put in a quartz cell. An absorption spectrum (ordinate axis: absorption intensity, abscissa axis: wavelength) of the thus-obtained sample is measured at a normal temperature (300K). A tangent is drawn to the fall of the absorption spectrum close to the long-wavelength region, and a wavelength value λedge (nm) at an intersection of the tangent and the abscissa axis is assigned to a conversion equation (F2) below to calculate singlet energy.

$$\text{Conversion Equation (F2): } S_1 \text{ [eV]} = 1239.85/\lambda\text{edge}$$

Any device for measuring absorption spectrum is usable. For instance, a spectrophotometer (U3310 manufactured by Hitachi, Ltd.) is usable.

The tangent to the fall of the absorption spectrum close to the long-wavelength region is drawn as follows. While moving on a curve of the absorption spectrum from the local maximum value closest to the long-wavelength region, among the local maximum values of the absorption spectrum, in a long-wavelength direction, a tangent at each point on the curve is checked. An inclination of the tangent is decreased and increased in a repeated manner as the curve falls (i.e., a value of the ordinate axis is decreased). A tangent drawn at a point where the inclination of the curve is the local minimum closest to the long-wavelength region (except when absorbance is 0.1 or less) is defined as the tangent to the fall of the absorption spectrum close to the long-wavelength region.

The local maximum absorbance of 0.2 or less is not counted as the above-mentioned local maximum absorbance closest to the long-wavelength region.

In the organic EL device according to the exemplary embodiment, an electron mobility μH1 of the first compound and an electron mobility μH2 of the second compound also preferably satisfy a relationship of a numerical formula (Numerical Formula 3) below.

$$\mu H2 > \mu H1 \qquad \text{(Numerical Formula 3)}$$

When the first compound and the second compound satisfy the relationship of the numerical formula (Numerical Formula 3), a recombination ability of holes and electrons in the first emitting layer is improved.

The electron mobility can be measured according to impedance spectroscopy.

A measurement target layer having a thickness in a range from 100 nm to 200 nm is held between the anode and the cathode, to which a small alternating voltage of 100 mV or less is applied while a bias DC voltage is applied. A value of an alternating current (absolute value and phase) which flows at this time is measured. This measurement is performed while changing a frequency of the alternating voltage, and complex impedance (Z) is calculated from the current value and the voltage value. A frequency dependency of the imaginary part (ImM) of the modulus M = iωZ (i: imaginary unit, ω: angular frequency) is obtained. The reciprocal number of a frequency ω at which the ImM becomes the maximum is defined as a response time of electrons carried in the measurement target layer. The electron mobility is calculated by the following equation.

$$\text{Electron Mobility} = (\text{Film Thickness of Measurement Target Layer})^2/(\text{Response Time} \bullet \text{Voltage})$$

The first emitting layer and the second emitting layer preferably do not contain a phosphorescent material (dopant material).

The first emitting layer and the second emitting layer preferably do not contain a heavy metal complex and a phosphorescent rare earth metal complex. Examples of the heavy-metal complex herein include iridium complex, osmium complex, and platinum complex.

Further, the first emitting layer and the second emitting layer also preferably do not contain a metal complex.

Film Thickness of Emitting Layer

A film thickness of the emitting layer of the organic EL device according to the exemplary embodiment is preferably in a range from 5 nm to 50 nm, more preferably in a range from 7 nm to 50 nm, further preferably in a range from 10 nm to 50 nm. When the film thickness of the emitting layer is 5 nm or more, the emitting layer is easily formable and chromaticity is easily adjustable. When the film thickness of the emitting layer is 50 nm or less, a rise in the drive voltage is easily reducible.

Content Ratios of Compounds in Emitting Layer

When the first emitting layer contains the first compound and the third compound, a content ratio of each of the first compound and the third compound in the first emitting layer preferably falls, for instance, within a range below.

The content ratio of the first compound is preferably in a range from 80 mass% to 99 mass%, more preferably in a range from 90 mass% to 99 mass%, further preferably in a range from 95 mass% to 99 mass%.

The content ratio of the third compound is preferably in a range from 1 mass% to 10 mass%, more preferably in a range from 1 mass% to 7 mass%, further preferably in a range from 1 mass% to 5 mass%.

The upper limit of the total of the content ratios of the first compound and the third compound in the first emitting layer is 100 mass%.

It is not excluded that the first emitting layer of the exemplary embodiment further contains a material(s) other than the first and third compounds.

The first emitting layer may contain a single type of the first compound or two or more types of the first compound. The first emitting layer may contain a single type of the third compound or two or more types of the third compound.

When the second emitting layer contains the second compound and the fourth compound, a content ratio of each of the second compound and the fourth compound in the second emitting layer preferably falls, for instance, within a range below.

The content ratio of the second compound is preferably in a range from 80 mass% to 99 mass%, more preferably in a range from 90 mass% to 99 mass%, further preferably in a range from 95 mass% to 99 mass%.

The content ratio of the fourth compound is preferably in a range from 1 mass% to 10 mass%, more preferably in a range from 1 mass% to 7 mass%, further preferably in a range from 1 mass% to 5 mass%.

The upper limit of the total of the content ratios of the second compound and the fourth compound in the second emitting layer is 100 mass%.

It is not excluded that the second emitting layer of the exemplary embodiment further contains a material(s) other than the second and fourth compounds.

The second emitting layer may contain a single type of the second compound or two or more types of the second compound. The second emitting layer may contain a single type of the fourth compound or two or more types of the fourth compound.

An arrangement of an organic EL device will be further described below. It should be noted that the reference numerals will be sometimes omitted below.

Substrate

The substrate is used as a support for the organic EL device. For instance, glass, quartz, plastics and the like are usable for the substrate. A flexible substrate is also usable. The flexible substrate is a bendable substrate, which is exemplified by a plastic substrate. Examples of the material for the plastic substrate include polycarbonate, polyarylate, polyethersulfone, polypropylene, polyester, polyvinyl fluoride, polyvinyl chloride, polyimide, and polyethylene naphthalate. Moreover, an inorganic vapor deposition film is also usable.

Anode

Metal, an alloy, an electrically conductive compound, a mixture thereof, or the like having a large work function (specifically, 4.0 eV or more) is preferably used as the anode formed on the substrate. Specific examples of the material include ITO (Indium Tin Oxide), indium oxide-tin oxide containing silicon or silicon oxide, indium oxide-zinc oxide, indium oxide containing tungsten oxide and zinc oxide, and graphene. In addition, gold (Au), platinum (Pt), nickel (Ni), tungsten (W), chrome (Cr), molybdenum (Mo), iron (Fe), cobalt (Co), copper (Cu), palladium (Pd), titanium (Ti), and nitrides of a metal material (e.g., titanium nitride) are usable.

The material is typically formed into a film by a sputtering method. For instance, the indium oxide-zinc oxide can be formed into a film by the sputtering method using a target in which zinc oxide in a range from 1 mass% to 10 mass% is added to indium oxide. Moreover, for instance, the indium oxide containing tungsten oxide and zinc oxide can be formed by the sputtering method using a target in which tungsten oxide in a range from 0.5 mass% to 5 mass% and zinc oxide in a range from 0.1 mass% to 1 mass% are added to indium oxide. In addition, the anode may be formed by a vacuum deposition method, a coating method, an inkjet method, a spin coating method or the like.

Among the organic layers formed on the anode, since the hole injecting layer adjacent to the anode is formed of a composite material into which holes are easily injectable irrespective of the work function of the anode, a material usable as an electrode material (e.g., metal, an alloy, an electroconductive compound, a mixture thereof, and the elements belonging to the group 1 or 2 of the periodic table) is also usable for the anode.

A material having a small work function such as elements belonging to Groups 1 and 2 in the periodic table of the elements, specifically, an alkali metal such as lithium (Li) and cesium (Cs), an alkaline earth metal such as magnesium (Mg), calcium (Ca) and strontium (Sr), alloys (e.g., MgAg and AlLi) including the alkali metal or the alkaline earth metal, a rare earth metal such as europium (Eu) and ytterbium (Yb), alloys including the rare earth metal are also usable for the anode. It should be noted that the vacuum deposition method and the sputtering method are usable for forming the anode using the alkali metal, alkaline earth metal and the alloy thereof. Further, when a silver paste is used for the anode, the coating method and the inkjet method are usable.

Cathode

It is preferable to use metal, an alloy, an electroconductive compound, a mixture thereof, or the like having a small work function (specifically, 3.8 eV or less) for the cathode. Examples of the material for the cathode include elements belonging to Groups 1 and 2 in the periodic table of the elements, specifically, the alkali metal such as lithium (Li) and cesium (Cs), the alkaline earth metal such as magnesium (Mg), calcium (Ca) and strontium (Sr), alloys (e.g., MgAg and AlLi) including the alkali metal or the alkaline earth metal, the rare earth metal such as europium (Eu) and ytterbium (Yb), and alloys including the rare earth metal.

It should be noted that the vacuum deposition method and the sputtering method are usable for forming the cathode using the alkali metal, alkaline earth metal and the alloy thereof. Further, when a silver paste is used for the cathode, the coating method and the inkjet method are usable.

By providing the electron injecting layer, various conductive materials such as Al, Ag, ITO, graphene, and indium oxide-tin oxide containing silicon or silicon oxide may be used for forming the cathode regardless of the work function. The conductive materials can be formed into a film using the sputtering method, inkjet method, spin coating method and the like.

Hole Injecting Layer

The hole injecting layer is a layer containing a substance exhibiting a high hole injectability. Examples of the substance exhibiting a high hole injectability include molybdenum oxide, titanium oxide, vanadium oxide, rhenium oxide, ruthenium oxide, chrome oxide, zirconium oxide, hafnium oxide, tantalum oxide, silver oxide, tungsten oxide, and manganese oxide.

In addition, the examples of the highly hole-injectable substance further include: an aromatic amine compound, which is a low-molecule organic compound, such as 4,4',4"-tris(N,N-diphenylamino)triphenylamine (abbreviation: TDATA), 4,4',4"-tris[N-(3-methylphenyl)-N-phenylamino]triphenylamine (abbreviation: MTDATA), 4,4'-bis[N-(4-diphenylaminophenyl)-N-phenylamino]biphenyl (abbreviation: DPAB), 4,4'-bis(N-{4-[N'-(3-methylphenyl)-N'-phenylamino]phenyl} -N-phenylamino)biphenyl (abbreviation: DNTPD), 1,3,5-tris[N-(4-diphenylaminophenyl)-N-phenylamino]benzene (abbreviation: DPA3B), 3-[N-(9-phenylcarbazole-3-yl)-N-phenylamino]-9-phenylcarbazole (abbreviation: PCzPCA1), 3,6-bis[N-(9-phenylcarbazole-3-yl)-N-phenylamino]-9-phenylcarbazole (abbreviation: PCzPCA2), and 3-[N-(1-naphthyl)-N-(9-phenylcarbazole-3-yl)amino]-9-phenylcarbazole (abbreviation: PCzPCN1); and dipyrazino[2,3-f:20,30-h]quinoxaline-2,3,6,7,10,11-hexacarbonitrile (HAT-CN).

In addition, a high polymer compound (e.g., oligomer, dendrimer and polymer) is usable as the substance exhibiting a high hole injectability. Examples of the high-molecule compound include poly(N-vinylcarbazole) (abbreviation: PVK), poly(4-vinyltriphenylamine) (abbreviation: PVTPA), poly[N-(4-{N'-[4-(4-diphenylamino)phenyl]phenyl-N'-phenylamino}phenyl)methacrylamide] (abbreviation: PTPDMA), and poly[N,N'-bis(4-butylphenyl)-N,N'-bis(phenyl)benzidine] (abbreviation: Poly-TPD). Moreover, an acid-added high polymer compound such as poly(3,4-ethylenedioxythiophene)/poly(styrene sulfonic acid) (PEDOT/PSS) and polyaniline/poly(styrene sulfonic acid) (PAni/PSS) are also usable.

Hole Transporting Layer

The hole transporting layer is a layer containing a highly hole-transporting substance. An aromatic amine compound, carbazole derivative, anthracene derivative and the like are usable for the hole transporting layer. Specific examples of a material for the hole transporting layer include 4,4'-bis[N-(1-naphthyl)-N-phenylamino]biphenyl (abbreviation: NPB), N,N'-bis(3-methylphenyl)-N,N'-diphenyl-[1,1'-biphenyl]-4,4'-diamine (abbreviation: TPD), 4-phenyl-4'-(9-phenylfluorene-9-yl)triphenylamine (abbreviation: BAFLP), 4,4'-bis[N-(9,9-dimethylfluorene-2-yl)-N-phenylamino]biphenyl (abbreviation: DFLDPBi), 4,4',4"-tris(N,N-diphenylamino)triphenylamine (abbreviation: TDATA), 4,4',4''-tris[N-(3-methylphenyl)-N-phenylamino]triphenylamine (abbreviation: MTDATA), and 4,4'-bis[N-(spiro-9,9'-bifluorene-2-yl)-N-phenylamino]biphenyl (abbreviation: BSPB). The above-described substances mostly have a hole mobility of $10^{-6}$ $cm^2/(V\cdot s)$ or more.

For the hole transporting layer, a carbazole derivative such as CBP, 9-[4-(N-carbazolyl)]phenyl-10-phenylanthracene (CzPA), and 9-phenyl-3-[4-(10-phenyl-9-anthryl)phenyl]-9H-carbazole (PCzPA) and an anthracene derivative such as t-BuDNA, DNA, and DPAnth may be used. A high polymer compound such as poly(N-vinylcarbazole) (abbreviation: PVK) and poly(4-vinyltriphenylamine) (abbreviation: PVTPA) is also usable.

However, in addition to the above substances, any substance exhibiting a higher hole transportability than an electron transportability may be used. It should be noted that the layer containing the substance exhibiting a high hole transportability may be not only a single layer but also a laminate of two or more layers formed of the above substance(s).

Electron Transporting Layer

The electron transporting layer is a layer containing a highly electron-transporting substance. For the electron transporting layer, 1) a metal complex such as an aluminum complex, beryllium complex, and zinc complex, 2) a hetero aromatic compound such as imidazole derivative, benzimidazole derivative, azine derivative, carbazole derivative, and phenanthroline derivative, and 3) a high polymer compound are usable. Specifically, as a low-molecule organic compound, a metal complex such as Alq, tris(4-methyl-8-quinolinato)aluminum (abbreviation: $Almq_3$), bis(10-hydroxybenzo[h]quinolinato)beryllium (abbreviation: $BeBq_2$), BAlq, Znq, ZnPBO and ZnBTZ is usable. In addition to the metal complex, a heteroaromatic compound such as 2-(4-biphenylyl)-5-(4-tert-butylphenyl)-1,3,4-oxadiazole (abbreviation: PBD), 1,3-bis[5-(ptert-butylphenyl)-1,3,4-oxadiazole-2-yl]benzene (abbreviation: OXD-7), 3-(4-tert-butylphenyl)-4-phenyl-5-(4-biphenylyl)-1,2,4-triazole (abbreviation: TAZ), 3-(4-tert-butylphenyl)-4-(4-ethylphenyl)-5-(4-biphenylyl)-1,2,4-triazole (abbreviation: p-EtTAZ), bathophenanthroline (abbreviation: BPhen), bathocuproine (abbreviation: BCP), and 4,4'-bis(5-methylbenzoxazole-2-yl)stilbene (abbreviation: BzOs) is usable. In the exemplary embodiment, a benzimidazole compound is preferably usable. The above-described substances mostly have an electron mobility of $10^{-6}$ $cm^2/Vs$ or more. It should be noted that any substance other than the above substance may be used for the electron transporting layer as long as the substance exhibits a higher electron transportability than the hole transportability. The electron

transporting layer may be provided in the form of a single layer or a laminate of two or more layers of the above substance(s).

Specific examples of the compound usable for the electron transporting layer include compounds below. It should however be noted that the invention is not limited to the specific examples of the compound.

[Formula 507]

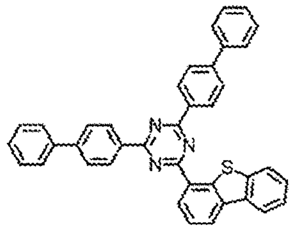
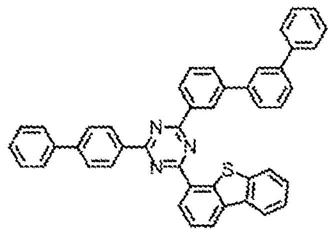
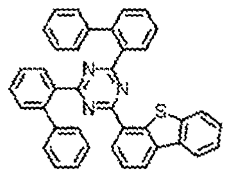
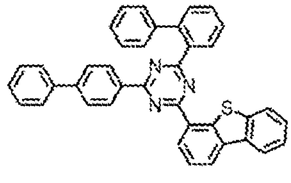
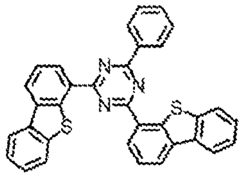
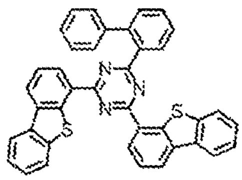
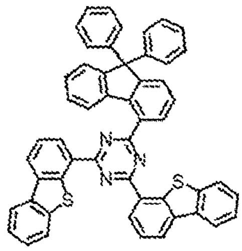
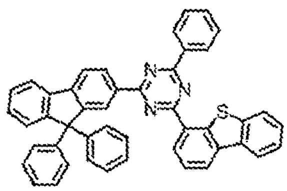
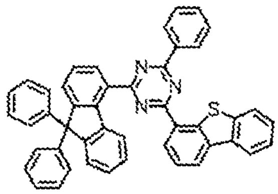
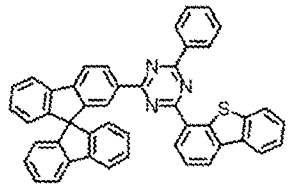
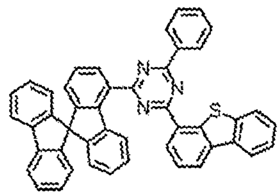
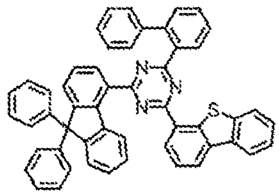
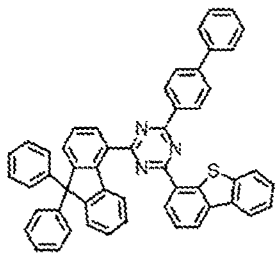
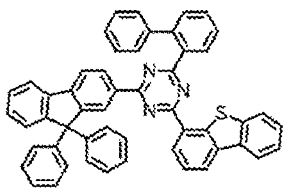
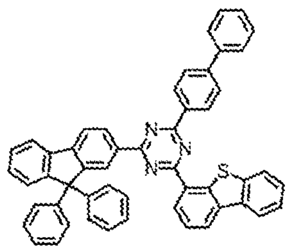

Further, a high polymer compound is usable for the electron transporting layer. For instance, poly[(9,9-dihexylfluorene-2,7-diyl)-co-(pyridine-3,5-diyl)] (abbreviation: PF-Py), poly[(9,9-dioctylfluorene-2,7-diyl)-co-(2,2'-bipyridine-6,6'-diyl)] (abbreviation: PF-BPy) and the like are usable.

Electron Injecting Layer

The electron injecting layer is a layer containing a highly electron-injectable substance. Examples of a material for the electron injecting layer include an alkali metal, alkaline earth metal and a compound thereof, examples of which include lithium (Li), cesium (Cs), calcium (Ca), lithium fluoride (LiF), cesium fluoride (CsF), calcium fluoride ($CaF_2$), and lithium oxide (LiOx). In addition, the alkali metal, alkaline earth metal or the compound thereof may be added to the substance exhibiting the electron transportability in use. Specifically, for instance, magnesium (Mg) added to Alq may be used. In this case, the electrons can be more efficiently injected from the cathode.

Alternatively, the electron injecting layer may be provided by a composite material in a form of a mixture of the organic compound and the electron donor. Such a composite material exhibits excellent electron injectability and electron

transportability since electrons are generated in the organic compound by the electron donor. In this case, the organic compound is preferably a material excellent in transporting the generated electrons. Specifically, the above examples (e.g., the metal complex and the hetero aromatic compound) of the substance forming the electron transporting layer are usable. As the electron donor, any substance exhibiting electron donating property to the organic compound is usable. Specifically, the electron donor is preferably alkali metal, alkaline earth metal and rare earth metal such as lithium, cesium, magnesium, calcium, erbium and ytterbium. The electron donor is also preferably alkali metal oxide and alkaline earth metal oxide such as lithium oxide, calcium oxide, and barium oxide. Moreover, a Lewis base such as magnesium oxide is usable. Further, the organic compound such as tetrathiafulvalene (abbreviation: TTF) is usable.

Layer Formation Method

A method for forming each layer of the organic EL device in the exemplary embodiment is subject to no limitation except for the above particular description. However, known methods of dry film-forming such as vacuum deposition, sputtering, plasma or ion plating and wet film-forming such as spin coating, dipping, flow coating or ink-jet are applicable.

Film Thickness

A film thickness of each of the organic layers of the organic EL device in the exemplary embodiment is not limited unless otherwise specified in the above. In general, the thickness preferably ranges from several nanometers to 1 μm because excessively small film thickness is likely to cause defects (e.g. pin holes) and excessively large thickness leads to the necessity of applying high voltage and consequent reduction in efficiency.

Emission Wavelength of Organic EL Device

The organic electroluminescence device according to the exemplary embodiment preferably emits light having a main peak wavelength in a range from 430 nm to 480 nm when the organic electroluminescence device is driven.

The main peak wavelength of the light emitted from the organic EL device when being driven is measured as follows. Voltage is applied on the organic EL devices such that a current density becomes 10 $mA/cm^2$, where spectral radiance spectrum is measured by a spectroradiometer CS-2000 (manufactured by Konica Minolta, Inc.). A peak wavelength of an emission spectrum, at which the luminous intensity of the resultant spectral radiance spectrum is at the maximum, is measured and defined as the main peak wavelength (unit: nm).

The organic EL device according to the exemplary embodiment can improve device performance. Moreover, in the organic EL device according to the exemplary embodiment in which a plurality of emitting layers are layered, since the emitting layer close to the anode (first emitting layer) contains the compound according to the first exemplary embodiment, a balance between a luminous efficiency and a lifetime can be expected to be favorable.

Fourth Exemplary Embodiment

Electronic Device

An electronic device according to a fourth exemplary embodiment is installed with any one of the organic EL devices according to the above exemplary embodiments. Examples of the electronic device include a display device and a light-emitting unit. Examples of the display device include a display component (e.g., an organic EL panel module), TV, mobile phone, tablet and personal computer. Examples of the light-emitting unit include an illuminator and a vehicle light.

Modification of Exemplary Embodiments

The scope of the invention is not limited by the above-described exemplary embodiments but includes any modification and improvement as long as such modification and improvement are compatible with the invention.

For instance, the emitting layer does not necessarily include a single layer or two emitting layers, but may include a plurality, i.e., exceeding two, of emitting layers layered. In a case where the organic EL device includes a plurality of emitting layers, it is only required that one or two of the emitting layers satisfy the conditions described in the above exemplary embodiments. For instance, the rest of the emitting layers may be a fluorescent emitting layer or a phosphorescent emitting layer with use of emission caused by electron transfer from the triplet excited state directly to the ground state.

When the organic EL device includes a plurality of emitting layers, these emitting layers may be mutually adjacently provided, or may form a so-called tandem organic EL device, in which a plurality of emitting units are layered

via an intermediate layer.

For instance, a blocking layer may be provided adjacent to at least one of a side of the emitting layer close to the anode or a side of the emitting layer close to the cathode. The blocking layer is preferably provided in contact with the emitting layer to block at least any of holes, electrons, excitons or combinations thereof.

For instance, when the blocking layer is provided in contact with the side of the emitting layer close to the cathode, the blocking layer permits transport of electrons, and blocks holes from reaching a layer provided closer to the cathode (e.g., the electron transporting layer) beyond the blocking layer. When the organic EL device includes the electron transporting layer, the blocking layer is preferably interposed between the emitting layer and the electron transporting layer.

When the blocking layer is provided in contact with the side of the emitting layer close to the anode, the blocking layer permits transport of holes and blocks electrons from reaching a layer provided closer to the anode (e.g., the hole transporting layer) beyond the blocking layer. When the organic EL device includes the hole transporting layer, the blocking layer is preferably interposed between the emitting layer and the hole transporting layer.

Alternatively, the blocking layer may be provided adjacent to the emitting layer so that the excitation energy does not leak out from the emitting layer toward neighboring layer(s). The blocking layer blocks excitons generated in the emitting layer from being transferred to a layer(s) (e.g., the electron transporting layer and the hole transporting layer) closer to the electrode(s) beyond the blocking layer.

The emitting layer is preferably bonded with the blocking layer.

Specific structure, shape and the like of the components in the invention may be designed in any manner as long as an object of the invention can be achieved. Examples

The invention will be described in further detail with reference to Examples. It should be noted that the scope of the invention is by no means limited to Examples.

Compounds

Structures of a compound represented by a formula (1000B) used for manufacturing organic EL devices in Examples 1 to 31 are shown below.

[Formula 508]

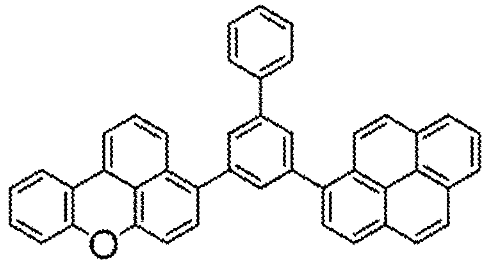

BH1-1

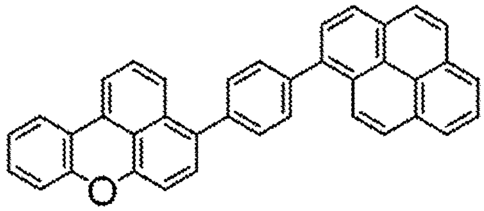

BH1-2

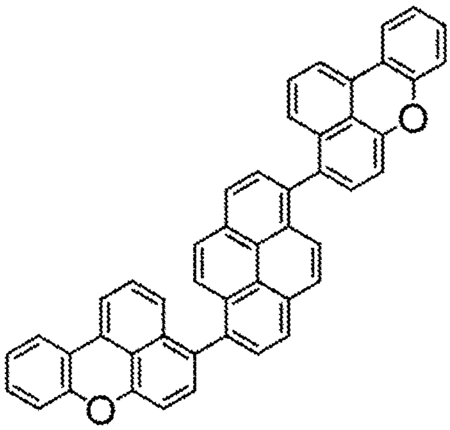

BH1-3

[Formula 509]

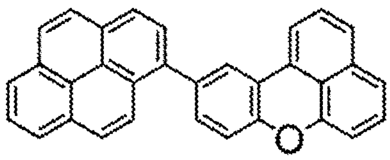

BH1-4

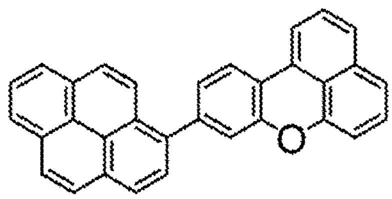

BH1-5

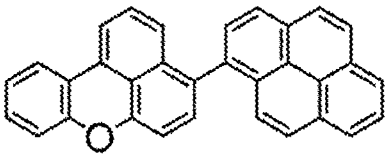

BH1-6

[Formula 510]

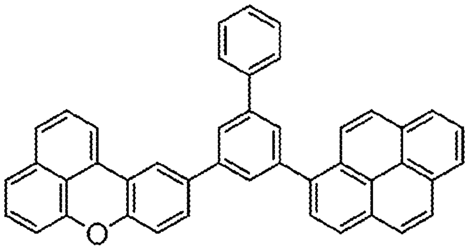

**BH1-7**

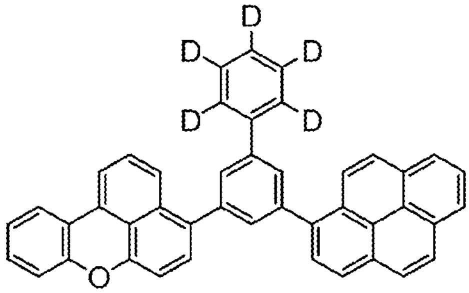

**BH1-8**

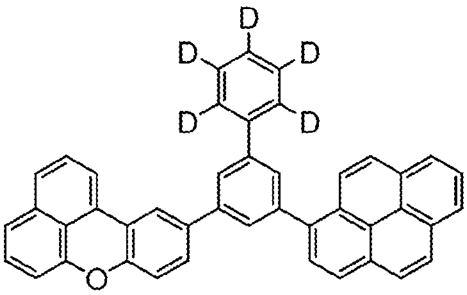

**BH1-9**

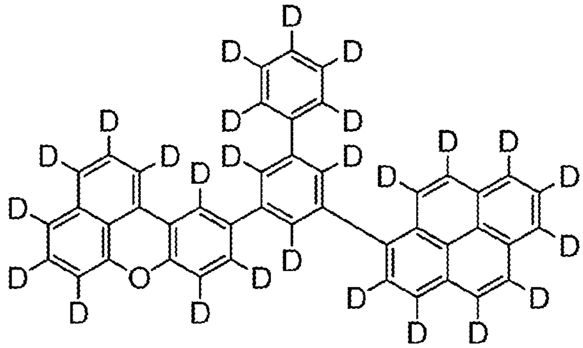

**BH1-10**

[Formula 511]

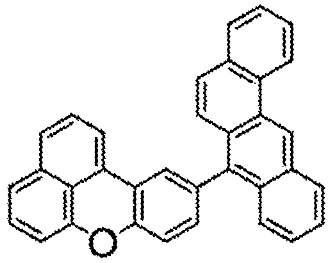

**BH1-11**

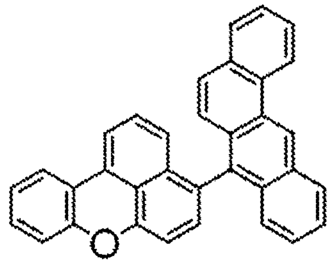

**BH1-12**

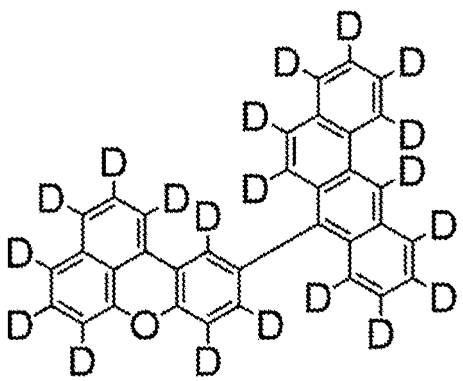

**BH1-13**

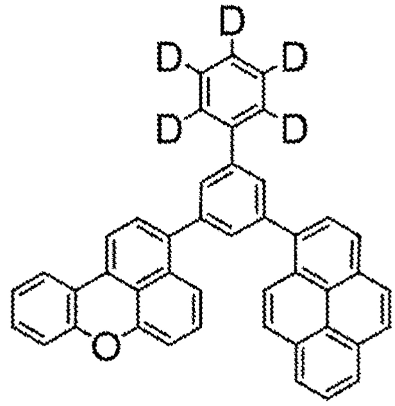

**BH1-14**

[Formula 512]

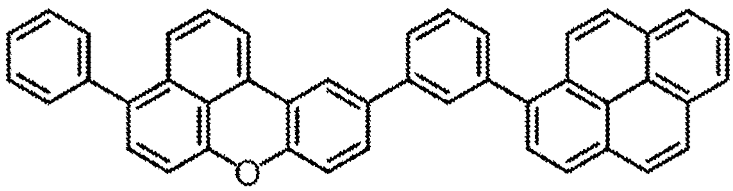

BH1-22

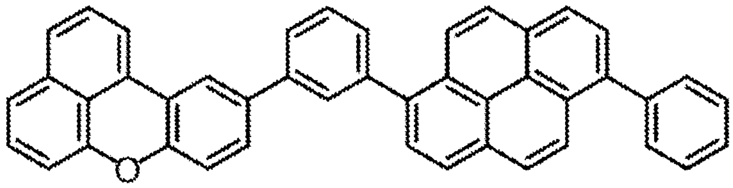

BH1-23

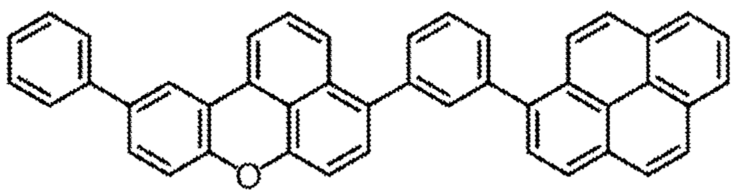

BH1-24

[Formula 513]

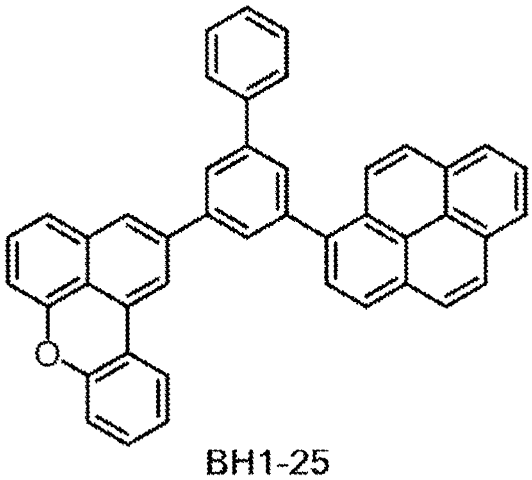

BH1-25

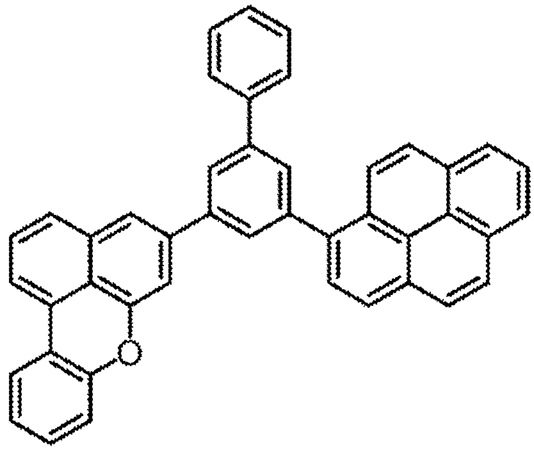

BH1-26

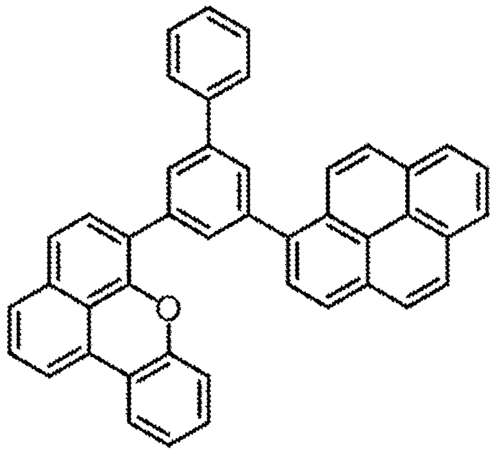

BH1-27

Structures of compounds used for manufacturing organic EL devices in Comparatives 4 to 7 are shown below.

[Formula 514]

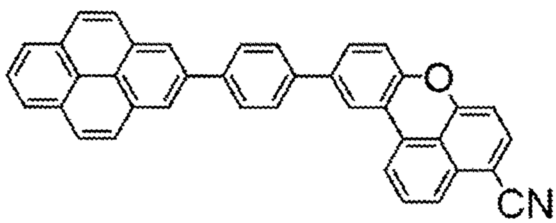

**Ref-BH1-1**

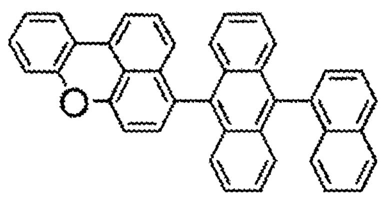

**Ref-BH1-2**

Structures of other compounds used for manufacturing organic EL devices in Examples 1 to 31 and Comparatives 1 to 9 are shown below.

[Formula 515]

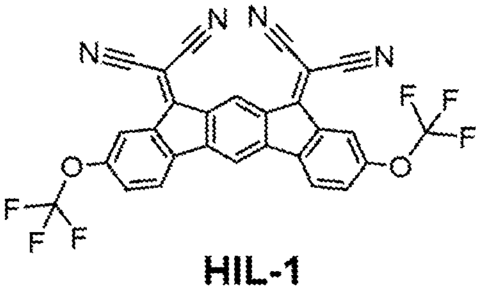

HIL-1

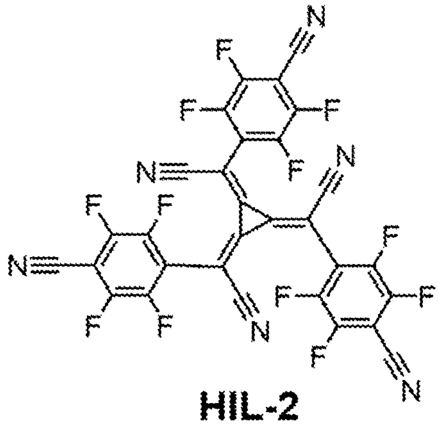

HIL-2

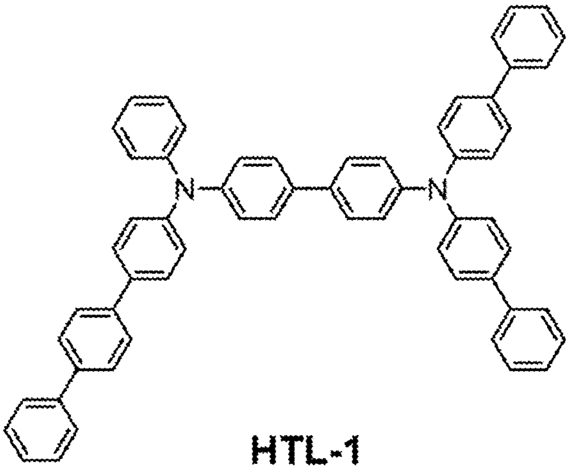

HTL-1

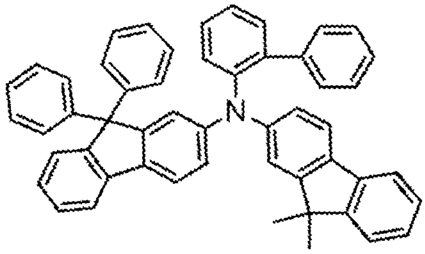

HTL-2

[Formula 516]

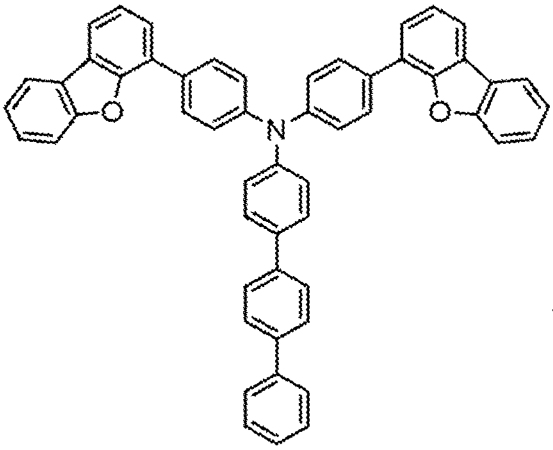

EBL-1

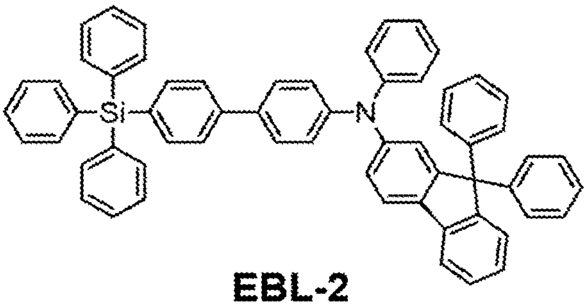

EBL-2

[Formula 517]

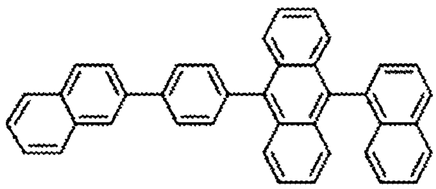

BH2-3

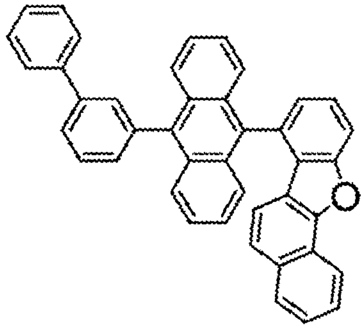

BH2-4

[Formula 518]

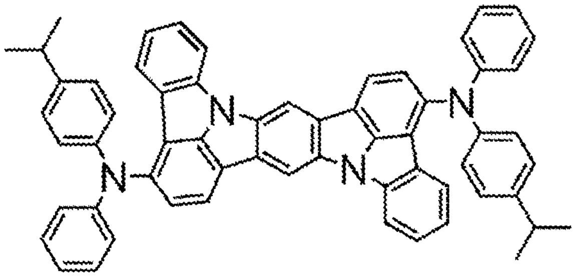

BD-1

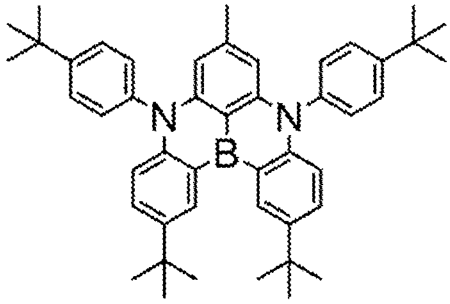

BD-2

[Formula 519]

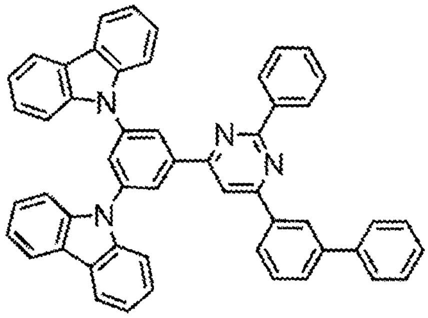
aET-1

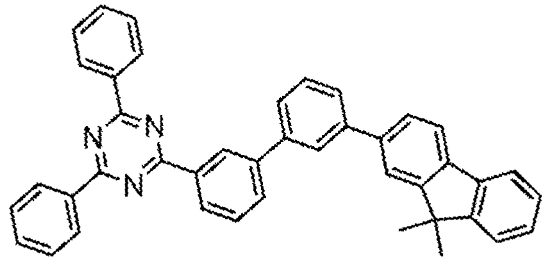
aET-2

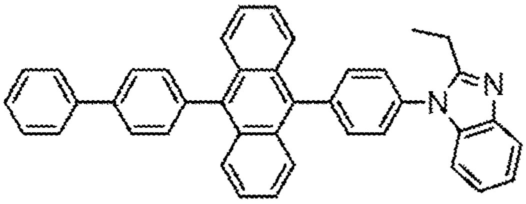
bET-1

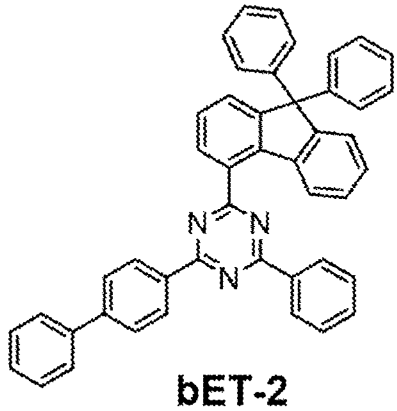
bET-2

Preparation of Organic EL Device

The organic EL devices were manufactured and evaluated as follows.

Example 1

A glass substrate (size: 25 mm × 75 mm × 1.1 mm thick, manufactured by Geomatec Co., Ltd.) having an ITO (Indium Tin Oxide) transparent electrode (anode) was ultrasonic-cleaned in isopropyl alcohol for five minutes, and then UV-ozone-cleaned for 30 minutes. A film thickness of the ITO transparent electrode was 130 nm.

The cleaned glass substrate having the transparent electrode line was attached to a substrate holder of a vacuum deposition apparatus. First, a compound HIL-1 was vapor-deposited on a surface of the glass substrate, where the transparent electrode line was provided, to cover the transparent electrode, thereby forming a 5-nm-thick hole injecting layer.

Subsequent to the formation of the hole injecting layer, a compound HTL-1 was vapor-deposited to form an 80-nm-thick first hole transporting layer.

Subsequent to the formation of the first hole transporting layer, a compound EBL-1 was vapor-deposited to form a 10-nm-thick second hole transporting layer (also referred to as an electron blocking layer).

A compound BH1-1 as the first compound and a compound BD-1 as the third compound were co-deposited on the second hole transporting layer such that a ratio of the compound BD-1 accounted for 2 mass%, thereby forming a 5-nm-thick first emitting layer.

A compound BH2-3 as the second compound and a compound BD-1 as the fourth compound were co-deposited on the first emitting layer such that a ratio of the compound BD-1 accounted for 2 mass%, thereby forming a 20-nm-thick second emitting layer.

A compound aET-1 was vapor-deposited on the second emitting layer to form a 10-nm-thick first electron transporting layer (also referred to as a hole blocking layer).

A compound bET-1 was vapor-deposited on the first electron transporting layer to form a 15-nm-thick second electron transporting layer.

LiF was vapor-deposited on the second electron transporting layer to form a 1-nm-thick electron injecting layer.

Metal (Al) was vapor-deposited on the electron injecting layer to form an 80-nm-thick cathode.

A device arrangement of the organic EL device in Example 1 is roughly shown as follows.

ITO(130) / HIL-1(5) / HTL-1(80) / EBL-1(10) / BH1-1:BD-1(5,98%:2%) / BH2-3:BD-1(20,98%:2%) / aET-1(10) / bET-1(15) / LiF(1) / Al(80)

The numerals in parentheses represent film thickness (unit: nm).

The numerals (98%:2%) represented by percentage in the same parentheses indicate a ratio (mass%) between the compound BH1-1 or BH2-3 and the compound BD-1 in the first emitting layer or the second emitting layer. Similar notations apply to the description below.

Examples 2, 3, 4, 5, 6, 7, 8, 9, 10, 11, 12, 13, and 14

Each of organic EL devices of Examples 2, 3, 4, 5, 6, 7, 8, 9, 10, 11, 12, 13, and 14 was manufactured in the same manner as in Example 1 except that the first compound of the first emitting layer was replaced by compounds shown in Table 1.

Comparative 1

An organic EL device of Comparative 1 was manufactured in the same manner as in Example 1 except that a 25-nm-thick second emitting layer was formed on the second hole transporting layer without forming the first emitting layer as shown in Table 1.

Comparative 2

An organic EL device of Comparative 2 was manufactured in the same manner as in Example 1 except that a 25-nm-thick first emitting layer was formed as the emitting layer and the first electron transporting layer was formed on the first emitting layer without forming the second emitting layer as shown in Table 1.

Comparatives 3, 4, and 5

Each of organic EL devices of Comparatives 3, 4, and 5 was manufactured in the same manner as in Comparative 2 except that the first compound of the first emitting layer was replaced by compounds shown in Table 1.

Comparatives 6 and 7

Each of organic EL devices of Comparatives 6 and 7 was manufactured in the same manner as in Example 1 except that the first compound of the first emitting layer was replaced by compounds shown in Table 1.

Table 1

| | First Emitting Layer | | | Second Emitting Layer | | | EQE [%] | LT95 [h] |
|---|---|---|---|---|---|---|---|---|
| | First Compound | Third Compound | Thickness [nm] | Second Compound | Fourth Compound | Thickness [nm] | | |
| Ex. 1 | BH1-1 | BD-1 | 5 | BH2-3 | BD-1 | 20 | 10.3 | 140 |
| Ex. 2 | BH1-2 | BD-1 | 5 | BH2-3 | BD-1 | 20 | 10.1 | 135 |
| Ex. 3 | BH1-3 | BD-1 | 5 | BH2-3 | BD-1 | 20 | 9.9 | 130 |
| Ex. 4 | BH1-4 | BD-1 | 5 | BH2-3 | BD-1 | 20 | 10.2 | 140 |
| Ex. 5 | BH1-5 | BD-1 | 5 | BH2-3 | BD-1 | 20 | 10.0 | 135 |
| Ex. 6 | BH1-6 | BD-1 | 5 | BH2-3 | BD-1 | 20 | 10.0 | 135 |
| Ex. 7 | BH1-7 | BD-1 | 5 | BH2-3 | BD-1 | 20 | 10.1 | 165 |
| Ex. 8 | BH1-8 | BD-1 | 5 | BH2-3 | BD-1 | 20 | 10.3 | 160 |
| Ex. 9 | BH1-9 | BD-1 | 5 | BH2-3 | BD-1 | 20 | 10.1 | 195 |
| Ex. 10 | BH1-10 | BD-1 | 5 | BH2-3 | BD-1 | 20 | 10.0 | 215 |
| Ex. 11 | BH1-11 | BD-1 | 5 | BH2-3 | BD-1 | 20 | 10.5 | 125 |
| Ex. 12 | BH1-12 | BD-1 | 5 | BH2-3 | BD-1 | 20 | 10.5 | 125 |
| Ex. 13 | BH1-13 | BD-1 | 5 | BH2-3 | BD-1 | 20 | 10.5 | 150 |

(continued)

| | First Emitting Layer | | | Second Emitting Layer | | | EQE [%] | LT95 [h] |
|---|---|---|---|---|---|---|---|---|
| | First Compound | Third Compound | Thickness [nm] | Second Compound | Fourth Compound | Thickness [nm] | | |
| Ex. 14 | BH1-14 | BD-1 | 5 | BH2-3 | BD-1 | 20 | 10.4 | 150 |
| Comp. 1 | - | - | - | BH2-3 | BD-1 | 25 | 9.8 | 130 |
| Comp. 2 | BH1-1 | BD-1 | 25 | - | - | - | 8.2 | 10 |
| Comp. 3 | BH1-3 | BD-1 | 25 | - | - | - | 7.9 | 8 |
| Comp. 4 | Ref-BH1-1 | BD-1 | 25 | - | - | - | 7.1 | 4 |
| Comp. 5 | Ref-BH1-2 | BD-1 | 25 | - | - | - | 8.1 | 55 |
| Comp. 6 | Ref-BH1-1 | BD-1 | 5 | BH2-3 | BD-1 | 20 | 7.4 | 25 |
| Comp. 7 | Ref-BH1-2 | BD-1 | 5 | BH2-3 | BD-1 | 20 | 8.2 | 75 |

Evaluation of Organic EL Device

The organic EL devices in Examples 1 to 31 and Comparatives 1 to 9 were evaluated as follows. Evaluation results are shown in Tables 1, 2, 3, and 4.

External Quantum Efficiency (EQE)

Voltage was applied on the organic EL devices so that a current density was 10 $mA/cm^2$, where spectral radiance spectrum was measured by a spectroradiometer CS-2000 (manufactured by Konica Minolta, Inc.). The external quantum efficiency EQE (unit: %) was calculated based on the obtained spectral-radiance spectra, assuming that the spectra was provided under a Lambertian radiation.

Lifetime (LT95)

Voltage was applied on the resultant organic EL devices such that a current density was 50 $mA/cm^2$, where a time (LT95 (unit: hr)) elapsed before a luminance intensity was reduced to 95% of the initial luminance intensity was measured.

Example 15

An organic EL device of Example 15 was manufactured in the same manner as in Example 1 except that the third compound of the first emitting layer and the fourth compound of the second emitting layer were replaced by compounds shown in Table 2.

Examples 16, 17, 18, 19, 20, 21, 22, 23, and 24

Each of organic EL devices of Examples 16, 17, 18, 19, 20, 21, 22, 23, and 24 was manufactured in the same manner as in Example 15 except that the first compound of the first emitting layer was replaced by compounds shown in Table 2.

Comparative 8

An organic EL device of Comparative 8 was manufactured in the same manner as in Example 15 except that a 25-nm-thick second emitting layer was formed on the second hole transporting layer without forming the first emitting layer as shown in Table 2.

Table 2

| | First Emitting Layer | | | Second Emitting Layer | | | EQE [%] | LT95 [h] |
|---|---|---|---|---|---|---|---|---|
| | First Compound | Third Compound | Thickness [nm] | Second Compound | Fourth Compound | Thickness [nm] | | |
| Ex. 15 | BH1-1 | BD-2 | 5 | BH2-3 | BD-2 | 20 | 9.5 | 140 |
| Ex. 16 | BH1-6 | BD-2 | 5 | BH2-3 | BD-2 | 20 | 9.4 | 135 |
| Ex. 17 | BH1-7 | BD-2 | 5 | BH2-3 | BD-2 | 20 | 9.3 | 165 |
| Ex. 18 | BH1-8 | BD-2 | 5 | BH2-3 | BD-2 | 20 | 9.5 | 160 |
| Ex. 19 | BH1-9 | BD-2 | 5 | BH2-3 | BD-2 | 20 | 9.3 | 190 |
| Ex. 20 | BH1-10 | BD-2 | 5 | BH2-3 | BD-2 | 20 | 9.2 | 210 |
| Ex. 21 | BH1-11 | BD-2 | 5 | BH2-3 | BD-2 | 20 | 9.7 | 120 |
| Ex. 22 | BH1-12 | BD-2 | 5 | BH2-3 | BD-2 | 20 | 9.7 | 115 |
| Ex. 23 | BH1-13 | BD-2 | 5 | BH2-3 | BD-2 | 20 | 9.7 | 145 |
| Ex. 24 | BH1-14 | BD-2 | 5 | BH2-3 | BD-2 | 20 | 9.6 | 150 |
| Comp. 8 | - | - | - | BH2-3 | BD-2 | 25 | 9.2 | 130 |

Example 25

A glass substrate (size: 25 mm × 75 mm × 1.1 mm thick, manufactured by Geomatec Co., Ltd.) having an ITO (Indium Tin Oxide) transparent electrode (anode) was ultrasonic-cleaned in isopropyl alcohol for five minutes, and then UV-ozone-cleaned for 30 minutes. A film thickness of the ITO transparent electrode was 130 nm.

The cleaned glass substrate having the transparent electrode line was attached to a substrate holder of a vacuum deposition apparatus. First, a compound HTL-2 and a compound HIL-2 were co-deposited on a surface of the glass substrate, where the transparent electrode line was provided, to cover the transparent electrode, thereby forming a 10-nm-thick hole injecting layer. The compound HTL-2 and the compound HIL-2 were set at 90 mass% and 10 mass%, respectively, in ratio in the hole injecting layer.

Subsequent to the formation of the hole injecting layer, the compound HTL-2 was vapor-deposited to form an 85-nm-thick first hole transporting layer.

Subsequent to the formation of the first hole transporting layer, a compound EBL-2 was vapor-deposited to form a 5-nm-thick second hole transporting layer (also referred to as an electron blocking layer).

The compound BH1-1 as the first compound and a compound BD-2 as the third compound were co-deposited on the second hole transporting layer such that a ratio of the compound BD-2 accounted for 2 mass%, thereby forming a 5-nm-thick first emitting layer.

A compound BH2-4 as the second compound and the compound BD-2 as the fourth compound were co-deposited on the first emitting layer such that a ratio of the compound BD-2 accounted for 2 mass%, thereby forming a 15-nm-thick second emitting layer.

A compound aET-2 was vapor-deposited on the second emitting layer to form a 5-nm-thick first electron transporting layer (also referred to as a hole blocking layer).

A compound bET-2 and a compound Liq were co-deposited on the first electron transporting layer to form a 25-nm-thick second electron transporting layer. The compound bET-2 and the compound Liq were set at 50 mass% and 50 mass%, respectively, in ratio in the second electron transporting layer. Liq is an abbreviation of (8-quinolinolato)lithium ((8-Quinolinolato)lithium).

The compound Liq was vapor-deposited on the second electron transporting layer to form a 1-nm-thick electron injecting layer.

Metal (Al) was vapor-deposited on the electron injecting layer to form an 80-nm-thick cathode.

A device arrangement of the organic EL device of Example 25 is roughly shown as follows.

ITO(130) / HTL-2:HIL-2(10,90%:10%) / HTL-2(85) / EBL-2(5) / BH1-1:BD-2(5,98%:2%) / BH2-4:BD-2(15,98%:2%) / aET-2(5) / bET-2:Liq(25,50%:50%) / Liq(1) / Al(80)

The numerals in parentheses represent film thickness (unit: nm).

The numerals (90%:10%) represented by percentage in the same parentheses indicate a ratio (mass%) between the compound HTL-2 and the compound HIL-2 in the hole injecting layer. The numerals (98%:2%) represented by

percentage in the same parentheses indicate a ratio (mass%) between the compound BH1-1 or BH2-4 and the compound BD-2 in the first emitting layer or the second emitting layer. The numerals (50%:50%) represented by percentage in the same parentheses indicate a ratio (mass%) between the compound bET-2 and the compound Liq in the second electron transporting layer. Similar notations apply to the description below.

Comparative 9

An organic EL device of Comparative 9 was manufactured in the same manner as in Example 25 except that a 20-nm-thick second emitting layer was formed on the second hole transporting layer without forming the first emitting layer as shown in Table 3.

Table 3

| | First Emitting Layer | | | Second Emitting Layer | | | EQE [%] | LT95 [h] |
|---|---|---|---|---|---|---|---|---|
| | First Compound | Third Compound | Thickness [nm] | Second Compound | Fourth Compound | Thickness [nm] | | |
| Ex. 25 | BH1-1 | BD-2 | 5 | BH2-4 | BD-2 | 15 | 9.9 | 80 |
| Comp. 9 | - | - | - | BH2-4 | BD-2 | 20 | 9.9 | 40 |

Example 26

A glass substrate (size: 25 mm × 75 mm × 1.1 mm thick, manufactured by Geomatec Co., Ltd.) having an ITO (Indium Tin Oxide) transparent electrode (anode) was ultrasonic-cleaned in isopropyl alcohol for five minutes, and then UV-ozone-cleaned for 30 minutes. A film thickness of the ITO transparent electrode was 130 nm.

The cleaned glass substrate having the transparent electrode line was attached to a substrate holder of a vacuum deposition apparatus. First, the compound HIL-1 was vapor-deposited on a surface of the glass substrate, where the transparent electrode line was provided, to cover the transparent electrode, thereby forming a 5-nm-thick hole injecting layer.

Subsequent to the formation of the hole injecting layer, the compound HTL-1 was vapor-deposited to form an 80-nm-thick first hole transporting layer.

Subsequent to the formation of the first hole transporting layer, the compound EBL-1 was vapor-deposited to form a 10-nm-thick second hole transporting layer (also referred to as the electron blocking layer).

A compound BH1-22 as the first compound and the compound BD-1 as the third compound were co-deposited on the second hole transporting layer such that a ratio of the compound BD-1 accounted for 2 mass%, thereby forming a 5-nm-thick first emitting layer.

A compound BH2-3 as the second compound and the compound BD-1 as the fourth compound were co-deposited on the first emitting layer such that a ratio of the compound BD-1 accounted for 2 mass%, thereby forming a 20-nm-thick second emitting layer.

A compound aET-1 was vapor-deposited on the second emitting layer to form a 10-nm-thick first electron transporting layer (also referred to as the hole blocking layer).

The compound bET-1 was vapor-deposited on the first electron transporting layer to form a 15-nm-thick second electron transporting layer.

LiF was vapor-deposited on the second electron transporting layer to form a 1-nm-thick electron injecting layer.

Metal (Al) was vapor-deposited on the electron injecting layer to form an 80-nm-thick cathode.

A device arrangement of the organic EL device of Example 26 is roughly shown as follows.

ITO(130) / HIL-1(5) / HTL-1(80) / EBL-1(10) / BH1-22:BD-1(5,98%:2%) / BH2-3:BD-1(20,98%:2%) / aET-1(10) / bET-1(15) / LiF(1) / Al(80)

The numerals in parentheses represent film thickness (unit: nm).

The numerals (98%:2%) represented by percentage in the same parentheses indicate a ratio (mass%) between the compound BH1-22 or BH2-3 and the compound BD-1 in the first emitting layer or the second emitting layer. Similar notations apply to the description below.

Examples 27, 28, 29, 30, and 31

Each of organic EL devices of Examples 27, 28, 29, 30, and 31 was manufactured in the same manner as in Example 26 except that the first compound of the first emitting layer was replaced by compounds shown in Table 4.

Table 4

| | First Emitting Layer | | | Second Emitting Layer | | | EQE [%] | LT95 [h] |
|---|---|---|---|---|---|---|---|---|
| | First Compound | Third Compound | Thickness [nm] | Second Compound | Fourth Compound | Thickness [nm] | | |
| Ex. 26 | BH1-22 | BD-1 | 5 | BH2-3 | BD-1 | 20 | 10.1 | 193 |
| Ex. 27 | BH1-23 | BD-1 | 5 | BH2-3 | BD-1 | 20 | 10.3 | 143 |
| Ex. 28 | BH1-24 | BD-1 | 5 | BH2-3 | BD-1 | 20 | 10.3 | 171 |
| Ex. 29 | BH1-25 | BD-1 | 5 | BH2-3 | BD-1 | 20 | 10.3 | 130 |
| Ex. 30 | BH1-26 | BD-1 | 5 | BH2-3 | BD-1 | 20 | 10.1 | 95 |
| Ex. 31 | BH1-27 | BD-1 | 5 | BH2-3 | BD-1 | 20 | 9.9 | 132 |

Synthesis of Compounds

Synthesis Example 1: Synthesis of Compound BH1-1

The compound BH1-1 was synthesized through a synthesis pathway below.

[Formula 520]

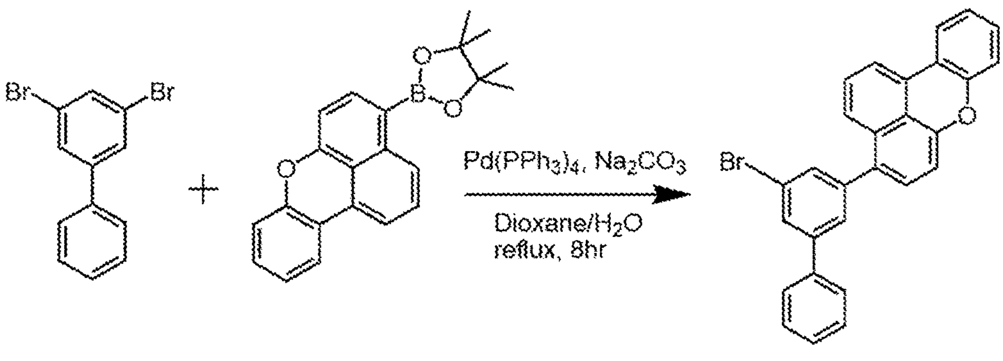


M1

Synthesis of Intermediate M1

Under argon atmosphere, 8.21 g of 3,5-dibromo-1,1'-biphenyl, 5.52 g of 4-(4,4,5,5-tetramethyl-1,3,2-dioxaborane-2-yl)benzo[kl]xanthene synthesized by a known method, 0.82 g of tetrakis(triphenylphosphine)palladium(0), 4.22 g of sodium carbonate, 150 mL of 1,4-dioxane, and 40 mL of water were put into a three-necked flask and refluxed for eight hours with stirring. After the reflux with stirring, the obtained solution was cooled to the room temperature. After the solution was cooled, the deposited solid was filtered and collected. The obtained solid was sequentially washed with water and methanol, and then repeatedly recrystallized with toluene to obtain 4.75 g of a light yellow solid (yield of 66%). The obtained compound was subjected to FD-MS (Field Desorption Mass Spectrometry) analysis and was identified as an intermediate M1.

[Formula 521]

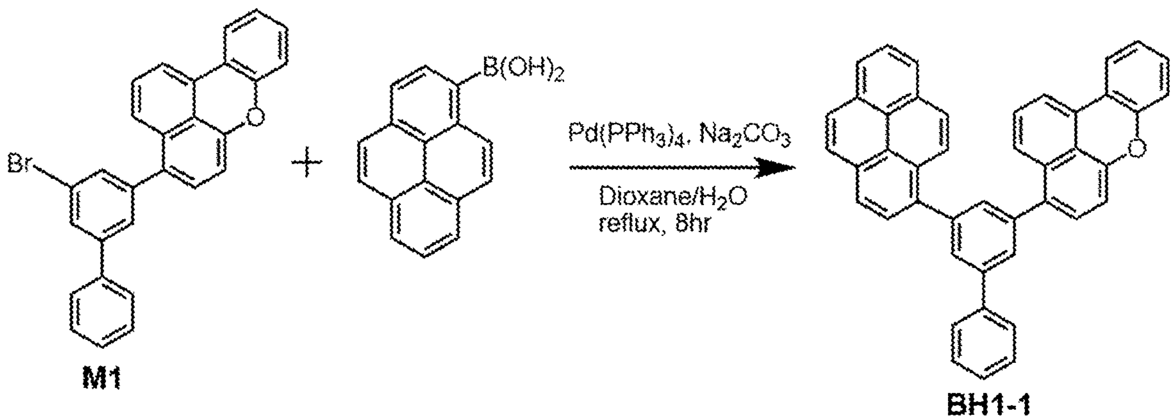


M1

BH1-1

Synthesis of Compound BH1-1

Under argon atmosphere, 13.21 g of the intermediate M1, 2.53 g of pyrene-1-ylboronic acid synthesized by a known method, 0.22 g of tetrakis(triphenylphosphine)palladium(0), 2.22 g of sodium carbonate, 50 mL of 1,4-dioxane, and 10 mL of water were put into a three-necked flask and refluxed for eight hours with stirring. After the reflux with stirring, the obtained solution was cooled to the room temperature. After the solution was cooled, the deposited solid was filtered and collected. The obtained solid was sequentially washed with water and methanol, and then repeatedly recrystallized with toluene to obtain 4.70 g of a light yellow solid (yield of 80%). As a result of FD-MS analysis, the light yellow solid was a compound BH1-1 and m/e was equal to 571 while a molecular weight was 570.69.

Synthesis Example 2: Synthesis of Compound BH1-2

A compound BH1-2 was synthesized through a synthesis pathway below.

[Formula 522]

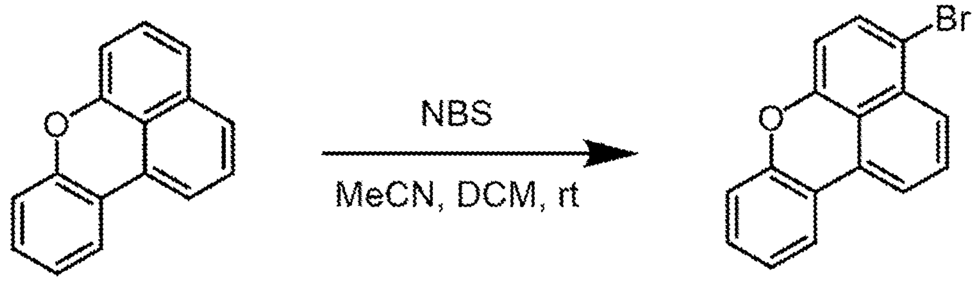


Synthesis of 4-Bromobenzo[kl]xanthene

Under argon atmosphere, 13.5 g of N-bromosuccinimide was added to 400 mL of a mixed solution including 15.0 g of benzo[kl]xanthene in dichloromethane and acetonitrile (3:1). The obtained solution was stirred at the room temperature for ten minutes. After the solution was stirred, the deposited solid was filtered and collected. The obtained solid was washed with a mixed solvent of acetonitrile and methanol to obtain 15.6 g of a white solid (yield of 77%). As a result of FD-MS analysis, the white solid was a 4-bromobenzo[kl]xanthene compound and m/e was equal to 298 while a molecular weight was 297.15. DCM is the abbreviation for dichloromethane, MeCN is the abbreviation for acetonitrile, and NBS is the abbreviation for N-bromosuccinimide.

[Formula 523]

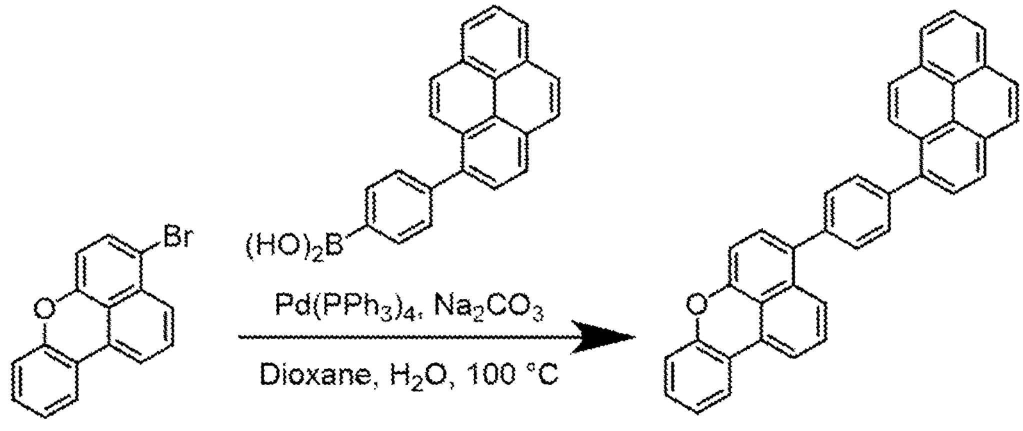


BH1-2

Synthesis of Compound BH1-2

Under argon atmosphere, 3.00 g of 4-bromobenzo[kl]xanthene, 3.25 g of 4-(pyrene-1-yl)phenylboronic acid, 0.46 g of tetrakis(triphenylphosphine)palladium(0), 3.21 g of sodium carbonate, 50 mL of 1,4-dioxane, and 15 mL of ion-exchange water were put into a flask and refluxed for 18 hours with stirring. After the reflux with stirring, the obtained solution was cooled to the room temperature. After the solution was cooled, the deposited solid was filtered and collected. The obtained solid was sequentially washed with water and acetone, and then recrystallized with a mixed solvent of toluene and cyclohexane to obtain 2.34 of a light yellow solid (yield of 47%). As a result of FD-MS analysis, the light yellow solid was a compound BH1-2 and m/e was equal to 495 while a molecular weight was 494.59.

Synthesis Example 3: Synthesis of Compound BH1-3

A compound BH1-3 was synthesized through a synthesis pathway below.

[Formula 524]

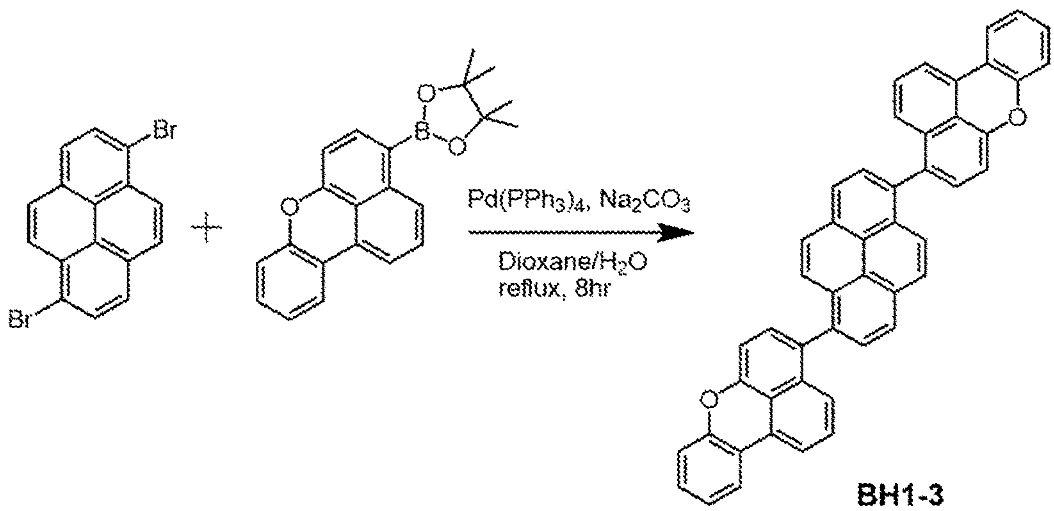


BH1-3

Synthesis of Compound BH1-3

Under argon atmosphere, 5.00 g of 1,6-dibromo pyrene, 9.59 g of 4-(4,4,5,5-tetramethyl-1,3,2-dioxaborane-2-yl)benzo[kl]xanthene synthesized by a known method, 0.32 g of tetrakis(triphenylphosphine)palladium(0), 2.88 g of

sodium carbonate, 60 mL of 1,4-dioxane, and 9.9 mL of water were put into a three-necked flask and refluxed for eight hours with stirring. After the reflux with stirring, the obtained solution was cooled to the room temperature. After the solution was cooled, the deposited solid was filtered and collected. The obtained solid was sequentially washed with water and methanol, and then repeatedly recrystallized with toluene to obtain 6.59 g of a light yellow solid (yield of 75%). The obtained compound was subjected to FD-MS analysis and was identified as the compound BH1-3.

Synthesis Example 4: Synthesis of Compound BH1-4

A compound BH1-4 was synthesized through a synthesis pathway below.

[Formula 525]

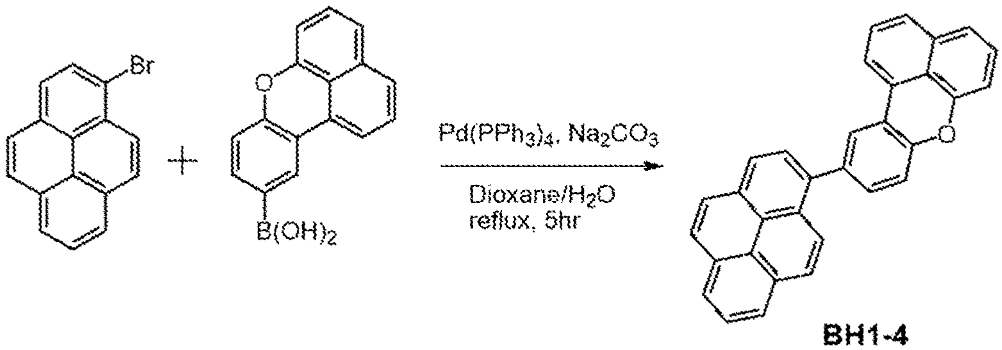


**BH1-4**

Synthesis of Compound BH1-4

Under argon atmosphere, 4.00 g of 1-bromopyrene, 3.70 g of benzo[kl]xanthene-10-yl-boronic acid synthesized by a known method, 0.33 g of tetrakis(triphenylphosphine)palladium(0), 3.00 g of sodium carbonate, 61 mL of 1,4-dioxane, and 10.0 mL of water were put into a three-necked flask and refluxed for five hours with stirring. After the reflux with stirring, the obtained solution was cooled to the room temperature. After the solution was cooled, the deposited solid was filtered and collected. The obtained solid was sequentially washed with water and methanol, and then repeatedly recrystallized with toluene to obtain 3.50 g of a light yellow solid (yield of 59%). The obtained compound was subjected to FD-MS analysis and was identified as the compound BH1-4.

Synthesis Example 5: Synthesis of Compound BH1-5

A compound BH1-5 was synthesized through a synthesis pathway below.

[Formula 526]

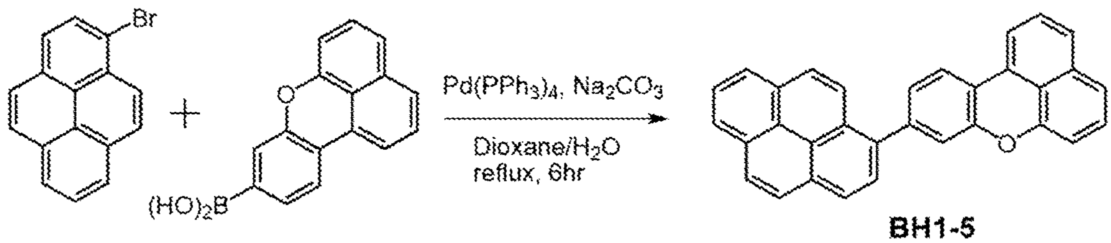


**BH1-5**

Synthesis of Compound BH1-5

Under argon atmosphere, 4.00 g of 1-bromopyrene, 3.83 g of benzo[kl]xanthene-9-yl-boronic acid synthesized by a known method, 0.65 g of tetrakis(triphenylphosphine)palladium(0), 2.42 g of sodium carbonate, 120 mL of 1,4-dioxane, and 10.0 mL of water were put into a three-necked flask and refluxed for six hours with stirring. After the reflux with stirring, the obtained solution was cooled to the room temperature. After the solution was cooled, the deposited solid was filtered and collected. The obtained solid was sequentially washed with water and methanol, and then repeatedly recrystallized with toluene to obtain 3.80 g of a light yellow solid (yield of 64%). The obtained compound was subjected to FD-MS analysis and was identified as the compound BH1-5.

Synthesis Example 6: Synthesis of Compound BH1-6

A compound BH1-6 was synthesized through a synthesis pathway below.

[Formula 527]

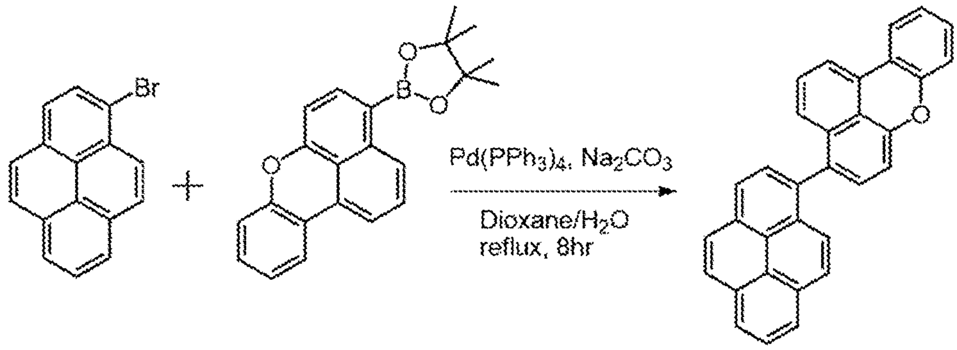


**BH1-6**

Synthesis of Compound BH1-6

Under argon atmosphere, 3.01 g of 1-bromopyrene, 3.70 g of 4-(4,4,5,5-tetramethyl-1,3,2-dioxaborane-2-yl)-benzo[kl]xanthene synthesized by a known method, 0.50 g of tetrakis(triphenylphosphine)palladium(0), 1.79 g of sodium carbonate, 100 mL of 1,4-dioxane, and 10 mL of water were put into a three-necked flask and refluxed for eight hours with stirring. After the reflux with stirring, the obtained solution was cooled to the room temperature. After the solution was cooled, the deposited solid was filtered and collected. The obtained solid was sequentially washed with water and methanol, and then repeatedly recrystallized with toluene to obtain 3.09 g of a light yellow solid (yield of 69%). The obtained compound was subjected to FD-MS analysis and was identified as the compound BH1-6.

Synthesis Example 7: Synthesis of Compound BH1-7

An intermediate M2 was synthesized through a synthesis pathway below.

[Formula 528]

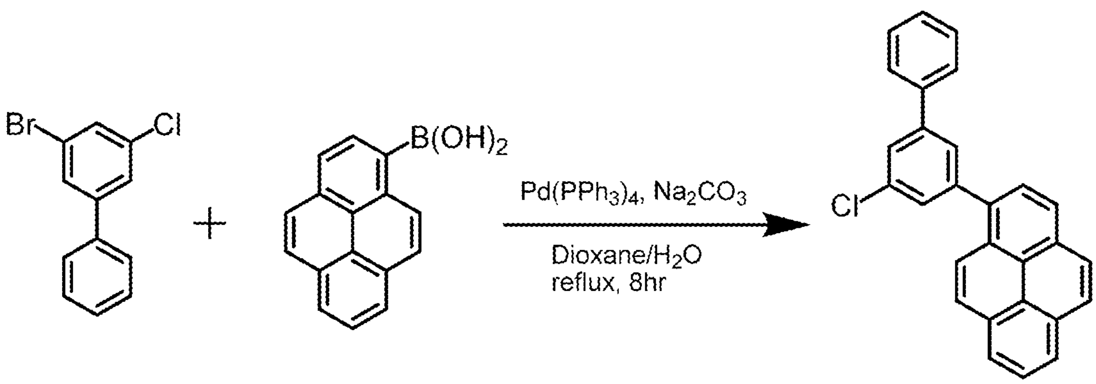


**M2**

Synthesis of Intermediate M2

Under argon atmosphere, 5.00 g of 3-chloro-5-bormo-1,1'-biphenyl, 4.60 g of pyrene-1-ylboronic acid, 0.648 g of tetrakis(triphenylphosphine)palladium(0), 3.96 g of sodium carbonate, 20 mL of 1,4-dioxane, and 20 mL of water were put into a three-necked flask and refluxed for eight hours with stirring. After the reflux with stirring, the obtained

solution was cooled to the room temperature. After the solution was cooled, the deposited solid was filtered and collected. The obtained solid was sequentially washed with water and methanol, and then repeatedly recrystallized with toluene to obtain 7.16 g of a light yellow solid (yield of 99%). The obtained compound was subjected to FD-MS (Field Desorption Mass Spectrometry) analysis and was identified as the intermediate M2.

A compound BH1-7 was synthesized through a synthesis pathway below.

[Formula 529]

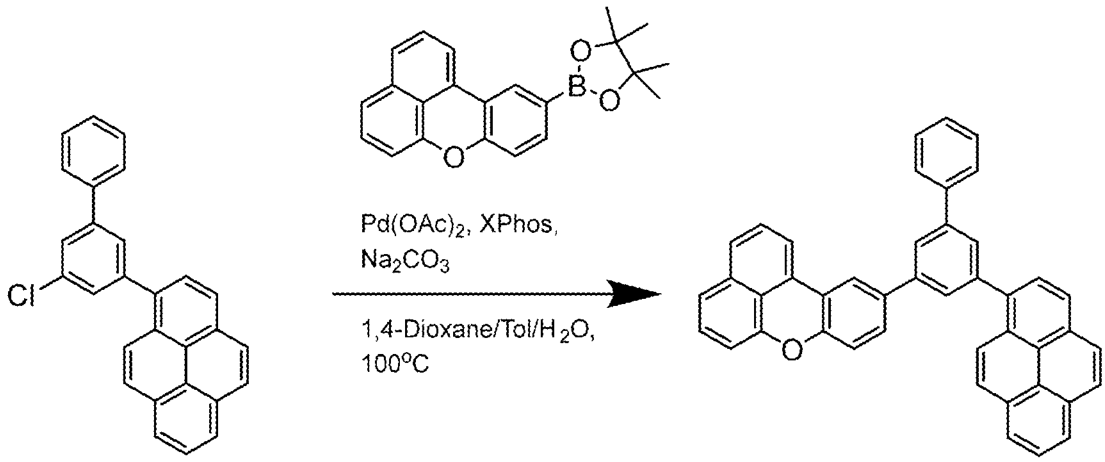


**M2** **BH1-7**

Synthesis of Compound BH1-7

Under argon atmosphere, 60.0 g of the intermediate M2 (chloro intermediate), 55.8 g of a boronic acid pinacol intermediate synthesized by a known method, 1.05 g of palladium acetate(II), 32.7 g of sodium carbonate, 500 mL of toluene, 450 mL of 1 ,4-dioxane, and 200 mL of ion-exchange water were put into a flask and refluxed for six hours with stirring. After the reflux with stirring, the obtained solution was cooled to the room temperature. After the solution was cooled, the deposited solid was filtered and collected. The obtained solid was sequentially washed with water and acetone, and then recrystallized with a mixed solvent of toluene and hexane to obtain 64.3 g of a light yellow solid (yield of 72%). As a result of FD-MS analysis, the light yellow solid was the compound BH1-7 and m/e was equal to 571 while a molecular weight was 570.69.

Synthesis Example 8: Synthesis of Compound BH1-8

An intermediate M3 was synthesized through a synthesis pathway below.

[Formula 530]

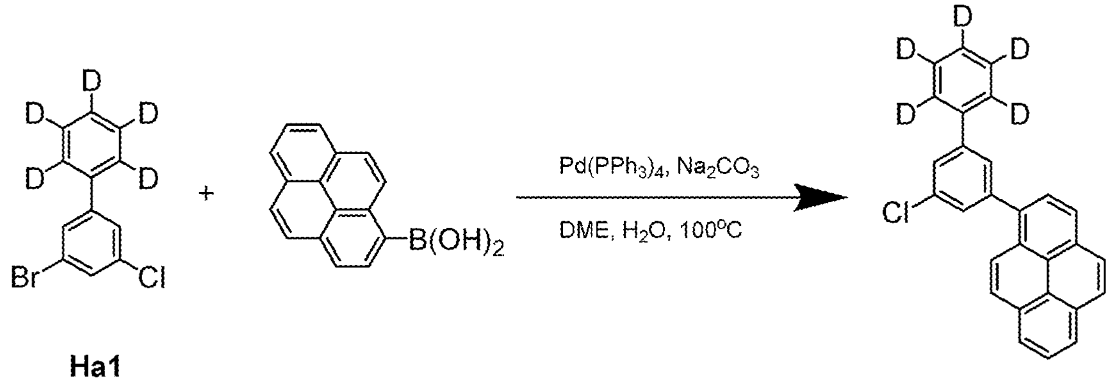


Ha1

M3

Synthesis of Intermediate M3

Under argon atmosphere, 1.05 g of a halogen intermediate Ha1, 0.95 g of pyrene-1-ylboronic acid synthesized by a known method, 0.13 g of tetrakis(triphenylphosphine)palladium(0) ($Pd(PPh_3)_4$), 0.82 g of sodium carbonate, 8 mL of DME, and 4 mL of ion-exchange water were put into a flask and stirred for 18 hours at 100 degrees C. After being stirred, the obtained solution was cooled to the room temperature. After the solution was cooled, an organic layer was washed with saturated saline solution and then added with sodium sulfate to be dried. After the drying, filtration and concentration under reduced pressure were conducted. The crude product was purified by silica-gel chromatography using a mixed solvent of toluene and hexane to obtain 1.40 g of a light yellow solid (yield of 92%). As a result of FD-MS analysis, the light yellow solid was the intermediate M3 and m/e was equal to 394 while a molecular weight was 393.92.

An intermediate M4 was synthesized through a synthesis pathway below.

[Formula 531]

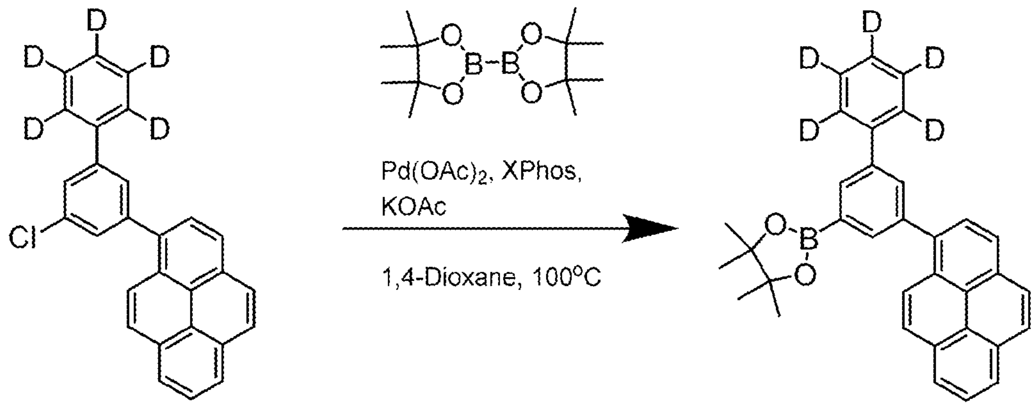


M3

M4

Synthesis of Intermediate M4

Under argon atmosphere, 1.93 g of the intermediate M3 (chloro intermediate), 2.00 g of bis(pinacolato)diboron, 0.02 g of palladium acetate(II), 0.50 g of potassium acetate, and 6 mL of 1,4-dioxane were put into a flask and stirred for seven hours at 100 degrees C. After being stirred, the obtained solution was cooled to the room temperature. After the solution was cooled, an organic layer was washed with saturated saline solution and then added with sodium sulfate to be dried. After the drying, filtration and concentration under reduced pressure were conducted. The crude product was purified by silica-gel chromatography using toluene to obtain 1.00 g of a light yellow solid (yield of 81%). As a result of FD-MS analysis, the light yellow solid was the intermediate M4 and m/e was equal to 486 while a molecular weight was 485.44.

A compound BH1-8 was synthesized through a synthesis pathway below.

[Formula 532]

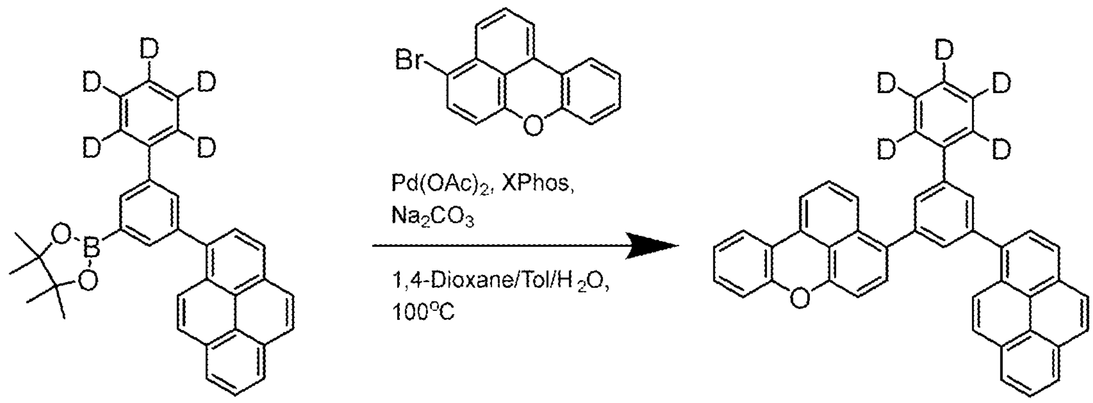


M4

BH1-8

Synthesis of Compound BH1-8

Under argon atmosphere, 25.0 g of the intermediate M4 (boronic acid pinacol intermediate), 15.3 g of a bromo intermediate synthesized by a known method, 2.98 g of tetrakis(triphenylphosphine)palladium(0) ($Pd(PPh_3)_4$), 13.8 g of sodium carbonate, 520 mL of 1,4-dioxane, and 200 mL of ion-exchange water were put into a flask and refluxed for 18 hours with stirring. After the reflux with stirring, the obtained solution was cooled to the room temperature. After the solution was cooled, the deposited solid was filtered and collected. The obtained solid was sequentially washed with water and acetone, and then recrystallized with a mixed solvent of toluene and hexane to obtain 11.0 g of a light yellow solid (yield of 37%). As a result of FD-MS analysis, the light yellow solid was the compound BH1-8 and m/e was equal to 575 while a molecular weight was 575.72.

Synthesis Example 9: Synthesis of Compound BH1-9

A compound BH1-9 was synthesized through a synthesis pathway below.

[Formula 533]

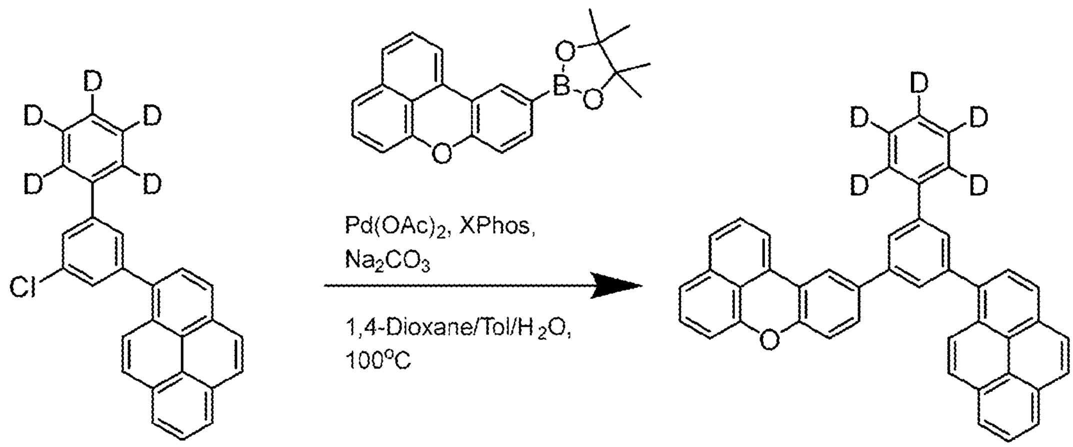


M3

BH1-9

Synthesis of Compound BH1-9

Under argon atmosphere, 60.0 g of the intermediate M3 (chloro intermediate) synthesized in the same manner as in Synthesis Example 8, 55.1 g of a boronic acid pinacol intermediate synthesized by a known method, 1.03 g of palladium(II) acetate, 32.3 g of sodium carbonate, 450 mL of toluene, 450 mL of 1,4-dioxane, and 170 mL of ion-exchange water were put into a flask and refluxed for six hours with stirring. After the reflux with stirring, the obtained solution was cooled to the room temperature. After the solution was cooled, the deposited solid was filtered and collected. The obtained solid was sequentially washed with water and acetone, and then recrystallized with a mixed solvent of toluene and hexane to obtain 66.9 g of a light yellow solid (yield of 76%). As a result of FD-MS analysis, the light yellow solid was the compound BH1-9 and m/e was equal to 576 while a molecular weight was 575.72.

Synthesis Example 10: Synthesis of Compound BH1-10

A compound BH1-10 was synthesized through a synthesis pathway below.

[Formula 534]

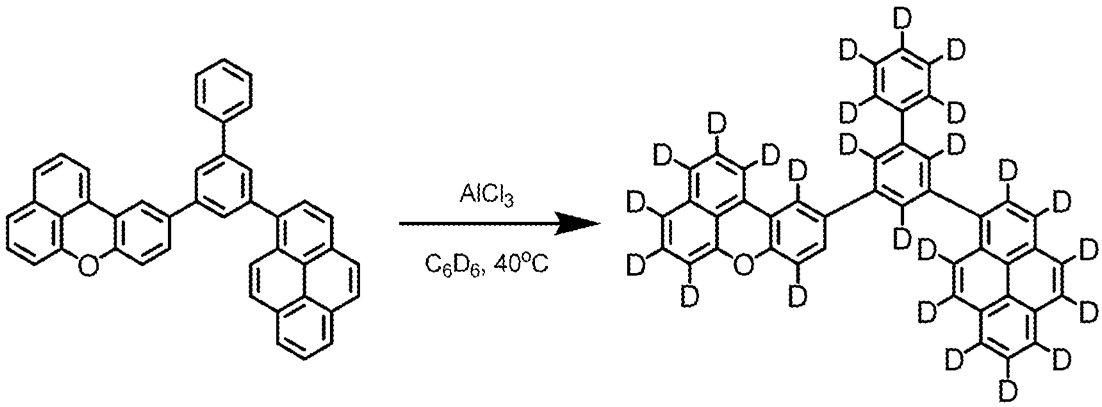


BH1-7 BH1-10

Synthesis of Compound BH1-10 (Deuterium Form)

Under argon atmosphere, 20.0 g of the compound BH1-7 (protium form) as a non-deuterated aromatic compound, 4.67 g of aluminum chloride, 1200 mL of benzene $d_6$ were put into a flask and refluxed at 40 degrees C for 29 hours with stirring. After the reflux with stirring, the obtained solution was cooled to the room temperature. After the solution was cooled, the deposited solid was filtered and collected. The obtained solid was sequentially washed with water and acetone. The washed solid was recrystallized with a mixed solvent of toluene and hexane to obtain 12.7 g of an orange solid (yield of 61%). As a result of FD-MS analysis, the orange solid was the compound BH1-10 in a form of deuterium (deuterated aromatic compound) and m/e was equal to 595 while a molecular weight was 595.84.

Synthesis Example 11: Synthesis of Compound BH1-11

A compound BH1-11 was synthesized through a synthesis pathway below.

[Formula 535]

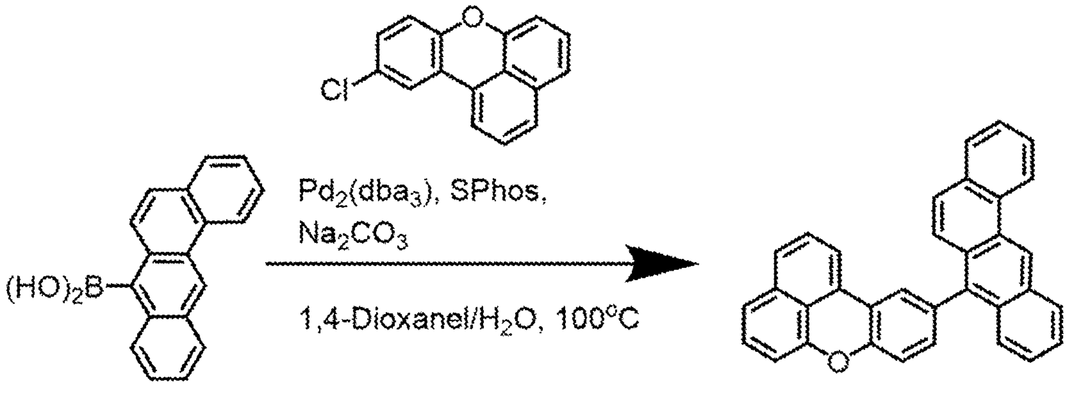


BH1-11

Synthesis of Compound BH1-11

Under argon atmosphere, 3.79 g of 10-chlorobenzo[kl]xanthene, 8.16 g of tetraphene-7-ylboronic acid synthesized by a known method, 0.41 g of tris(dibenzylideneacetone)dipalladium(0), 1.48 g of 2-dicyclohexylphosphino-2',6'-dimethoxy-1,1'-biphenyl, 4.77 g of sodium carbonate, 150 mL of 1,4-dioxane, and 20 mL of ion-exchange water were put into a flask and refluxed at 100 degrees C for six hours with stirring. After the reflux with stirring, the obtained solution was cooled to the room temperature. After the solution was cooled, the deposited solid was filtered and collected. The obtained solid was sequentially washed with water and acetone, and then recrystallized with a mixed solvent of toluene and hexane to obtain 2.10 g of a light yellow solid (yield of 32%). As a result of FD-MS analysis, the light yellow solid was the compound BH1-11 and m/e was equal to 445 while a molecular weight was 444.53.

Synthesis Example 12: Synthesis of Compound BH1-12

A compound BH- was synthesized through a synthesis pathway below.

[Formula 536]

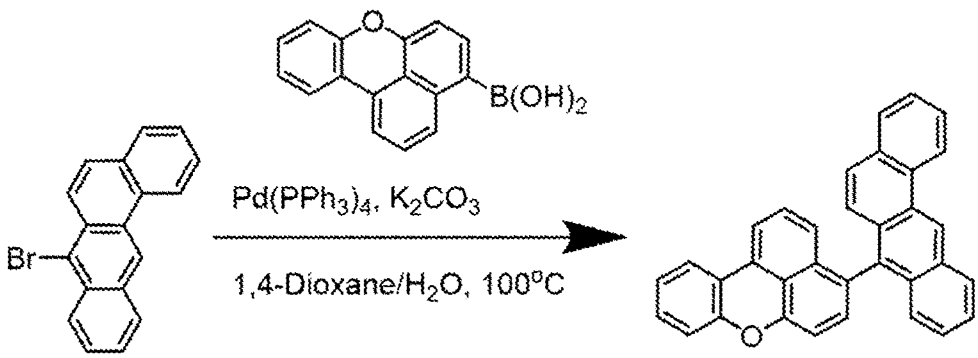


M5

BH1-12

Synthesis of Compound BH1-12

Under argon atmosphere, 2.40 g of an intermediate M5 (bromo intermediate), 2.37 g of a boronic acid pinacol intermediate synthesized by a known method, 0.18 g of tetrakis(triphenylphosphine)palladium(0), 2.16 g of potassium carbonate, 36 mL of 1,4-dioxane, and 6 mL of ion-exchange water were put into a flask and refluxed at 100 degrees C for six hours with stirring. After the reflux with stirring, the obtained solution was cooled to the room temperature. After the solution was cooled, the deposited solid was filtered and collected. The obtained solid was sequentially washed with water and acetone, and then recrystallized with a mixed solvent of toluene and hexane to obtain 2.4 g of a light yellow

solid (yield of 69%). As a result of FD-MS analysis, the light yellow solid was the compound BH1-12 and m/e was equal to 445 while a molecular weight was 444.53.

Synthesis Example 13: Synthesis of Compound BH1-13

A compound BH1-13 was synthesized through a synthesis pathway below.

[Formula 537]

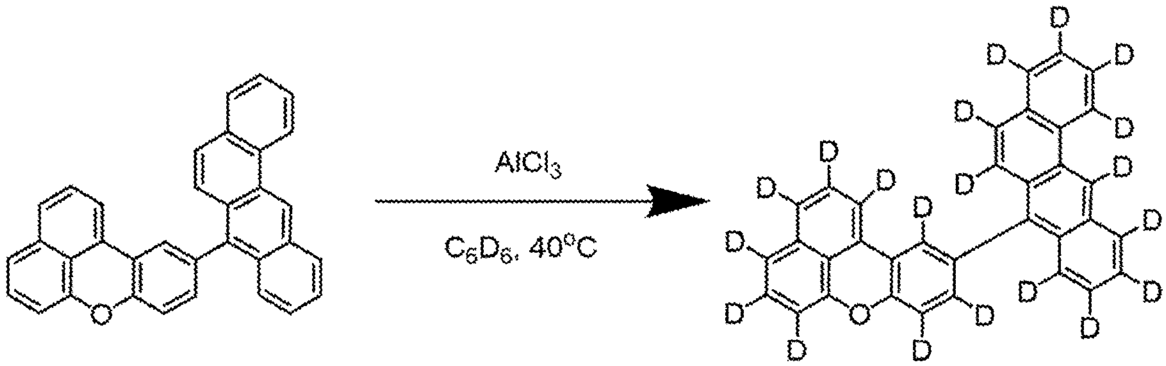


BH1-11 BH1-13

Synthesis of Compound BH1-13

Under argon atmosphere, 19.0 g of the compound BH1-11 (protium form) as a non-deuterated aromatic compound, 4.6 g of aluminum chloride, and 1140 mL of benzene $d_6$ were put into a flask and refluxed at 40 degrees C for 29 hours with stirring. After the reflux with stirring, the obtained solution was cooled to the room temperature. After the solution was cooled, the deposited solid was filtered and collected. The obtained solid was sequentially washed with water and acetone. The washed solid was recrystallized with a mixed solvent of toluene and hexane to obtain 14.0 g of an orange solid (yield of 71%). As a result of FD-MS analysis, the orange solid was the compound BH1-13 as a deuterated aromatic compound and m/e was equal to 463 while a molecular weight was 462.64.

Synthesis Example 14: Synthesis of Compound BH1-14

A compound BH1-14 was synthesized through a synthesis pathway below.

[Formula 538]

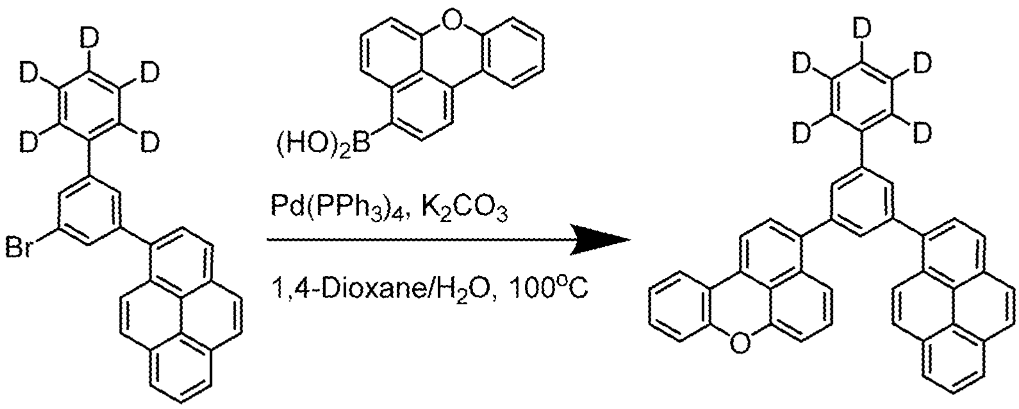


M6

BH1-14

Synthesis of Compound BH1-14

Under argon atmosphere, 2.60 g of an intermediate M6 (bromo intermediate), 1.55 g of a boronic acid intermediate synthesized by a known method, 0.14 g of tetrakis(triphenylphosphine)palladium(0), 1.64 g of potassium carbonate, 40 mL of 1,4-dioxane, and 10 mL of ion-exchange water were put into a flask and refluxed at 100 degrees C for six hours with stirring. After the reflux with stirring, the obtained solution was cooled to the room temperature. After the solution was cooled, the deposited solid was filtered and collected. The obtained solid was sequentially washed with water and acetone, and then recrystallized with a mixed solvent of toluene and hexane to obtain 1.70 g of a light yellow solid (yield of 50%). As a result of FD-MS analysis, the light yellow solid was the compound BH1-14 and m/e was equal to 576 while a molecular weight was 575.72.

Synthesis Example 15: Synthesis of Compound BH1-15

A compound BH1-15 was synthesized through a synthesis pathway below.

[Formula 539]

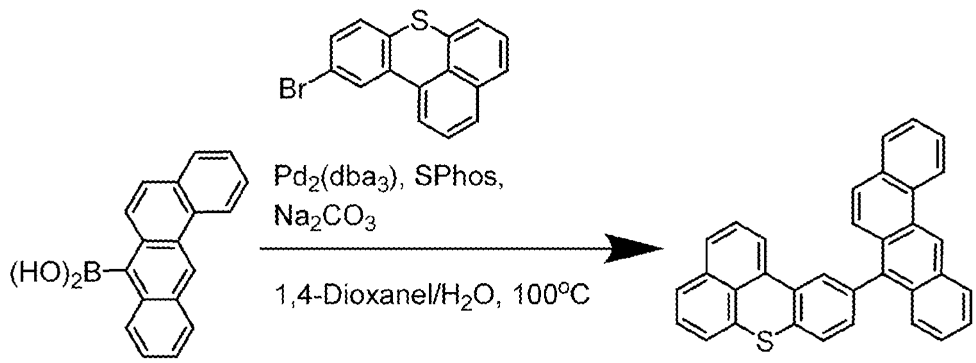


BH1-15

Synthesis of Compound BH1-15

Under argon atmosphere, 4.62 g of 10-bromobenzo[kl]thioxanthene, 8.16 g of tetraphene-7-ylboronic acid synthesized by a known method, 0.52 g of tris(dibenzylideneacetone)dipalladium(0), 1.63 g of 2-dicyclohexylphosphino-2',6'-dimethoxy-1,1'-biphenyl, 4.99 g of sodium carbonate, 150 mL of 1,4-dioxane, and 20 mL of ion-exchange water were put into a flask and refluxed at 100 degrees C for six hours with stirring. After the reflux with stirring, the obtained solution was cooled to the room temperature. After the solution was cooled, the deposited solid was filtered and collected. The obtained solid was sequentially washed with water and acetone, and then recrystallized with a mixed solvent of

toluene and hexane to obtain 3.26 g of a light yellow solid (yield of 48%). As a result of FD-MS analysis, the light yellow solid was the compound BH1-15 and m/e was equal to 461 while a molecular weight was 460.59.

Synthesis Example 16: Synthesis of Compound BH1-16

A compound BH1-16 was synthesized through a synthesis pathway below.

[Formula 540]

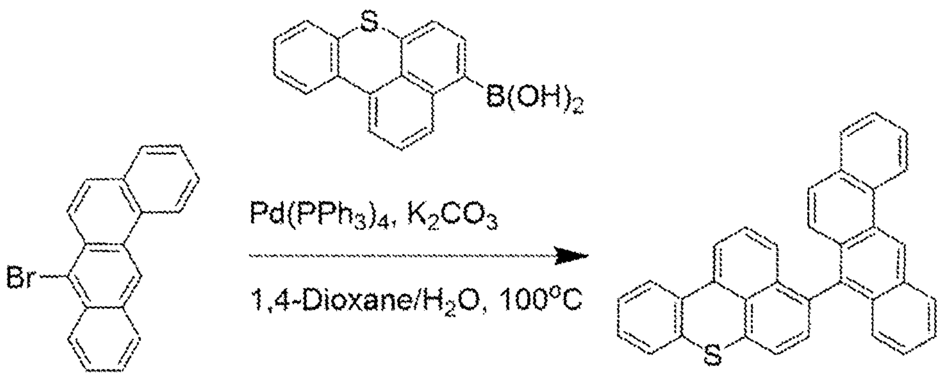


M5

BH1-16

Synthesis of Compound BH1-16

Under argon atmosphere, 2.40 g of the intermediate M5 (bromo intermediate), 2.37 g of a boronic acid intermediate synthesized by a known method, 0.21 g of tetrakis(triphenylphosphine)palladium(0), 2.33 g of potassium carbonate, 40 mL of 1,4-dioxane, and 10 mL of ion-exchange water were put into a flask and refluxed at 100 degrees C for six hours with stirring. After the reflux with stirring, the obtained solution was cooled to the room temperature. After the solution was cooled, the deposited solid was filtered and collected. The obtained solid was sequentially washed with water and acetone, and then recrystallized with a mixed solvent of toluene and hexane to obtain 2.16 g of a light yellow solid (yield of 55%). As a result of FD-MS analysis, the light yellow solid was the compound BH1-16 and m/e was equal to 461 while a molecular weight was 460.59.

Synthesis Example 17: Synthesis of Compound BH1-17

A compound BH1-17 was synthesized through a synthesis pathway below.

[Formula 541]

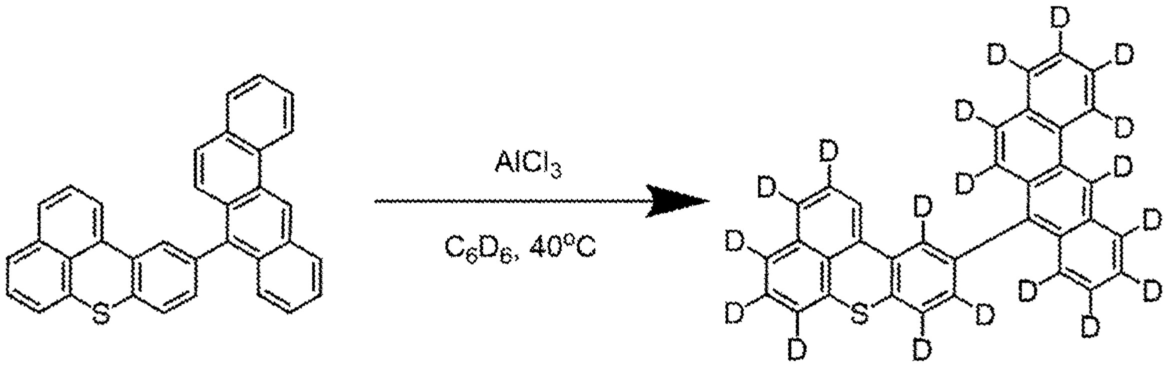


BH1-15 BH1-17

Synthesis of Compound BH1-17

Under argon atmosphere, 19.0 g of the compound BH1-15 (protium form) as a non-deuterated aromatic compound, 4.6 g of aluminum chloride, and 1140 mL of benzene $d_6$ were put into a flask and refluxed at 40 degrees C for 29 hours with stirring. After the reflux with stirring, the obtained solution was cooled to the room temperature. After the solution was cooled, the deposited solid was filtered and collected. The obtained solid was sequentially washed with water and acetone. The washed solid was recrystallized with a mixed solvent of toluene and hexane to obtain 14.6 g of an orange solid (yield of 74%). As a result of FD-MS analysis, the orange solid was the compound BH1-17 as a deuterated aromatic compound and m/e was equal to 480 while a molecular weight was 479.71.

Synthesis Example 18: Synthesis of Compound BH1-18

A compound BH1-18 was synthesized through a synthesis pathway below.

[Formula 542]

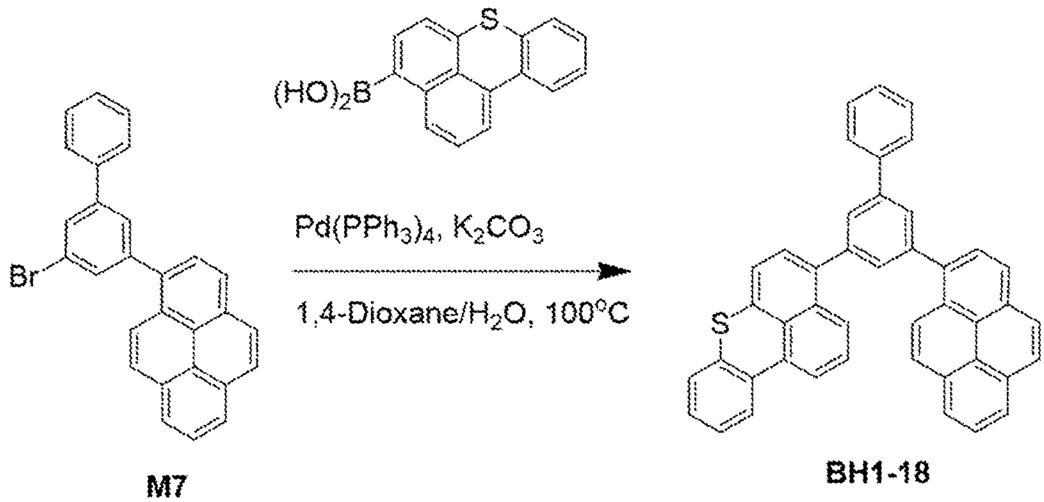


M7 BH1-18

Synthesis of Compound BH1-18

Under argon atmosphere, 2.60 g of an intermediate M7 (bromo intermediate), 2.05 g of a boronic acid intermediate synthesized by a known method, 0.22 g of tetrakis(triphenylphosphine)palladium(0), 1.68 g of potassium carbonate, 50 mL of 1,4-dioxane, and 10 mL of ion-exchange water were put into a flask and refluxed at 100 degrees C for six hours with stirring. After the reflux with stirring, the obtained solution was cooled to the room temperature. After the solution was cooled, the deposited solid was filtered and collected. The obtained solid was sequentially washed with water and acetone, and then recrystallized with a mixed solvent of toluene and hexane to obtain 2.32 g of a light yellow solid (yield of 66%). As a result of FD-MS analysis, the light yellow solid was the compound BH1-18 and m/e was equal

to 587 while a molecular weight was 586.75.

Synthesis Example 19: Synthesis of Compound BH1-19

A compound BH1-19 was synthesized through a synthesis pathway below.

[Formula 543]

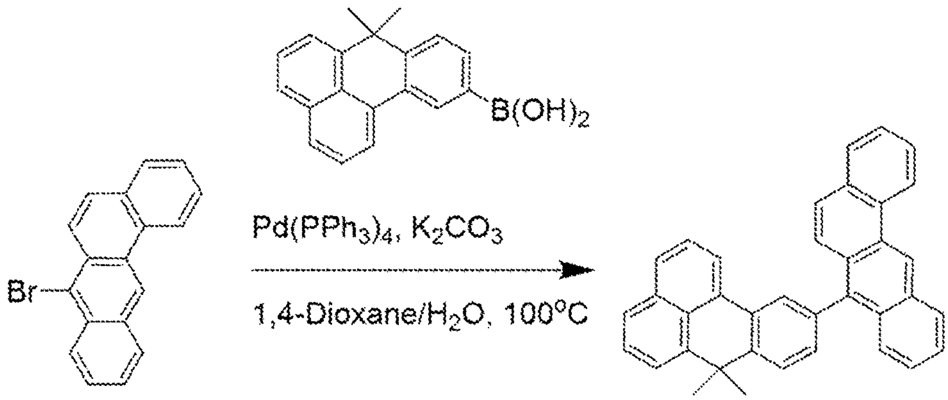


M5

BH1-19

Synthesis of Compound BH1-19

Under argon atmosphere, 2.30 g of the intermediate M5 (bromo intermediate), 2.37 g of a boronic acid intermediate synthesized by a known method, 0.23 g of tetrakis(triphenylphosphine)palladium(0), 2.23 g of potassium carbonate, 40 mL of 1,4-dioxane, and 10 mL of ion-exchange water were put into a flask and refluxed at 100 degrees C for six hours with stirring. After the reflux with stirring, the obtained solution was cooled to the room temperature. After the solution was cooled, the deposited solid was filtered and collected. The obtained solid was sequentially washed with water and acetone, and then recrystallized with a mixed solvent of toluene and hexane to obtain 2.40 g of a light yellow solid (yield of 64%). As a result of FD-MS analysis, the light yellow solid was the compound BH1-19 and m/e was equal to 471 while a molecular weight was 470.62.

Synthesis Example 20: Synthesis of Compound BH1-20

A compound BH1-20 was synthesized through a synthesis pathway below.

[Formula 544]

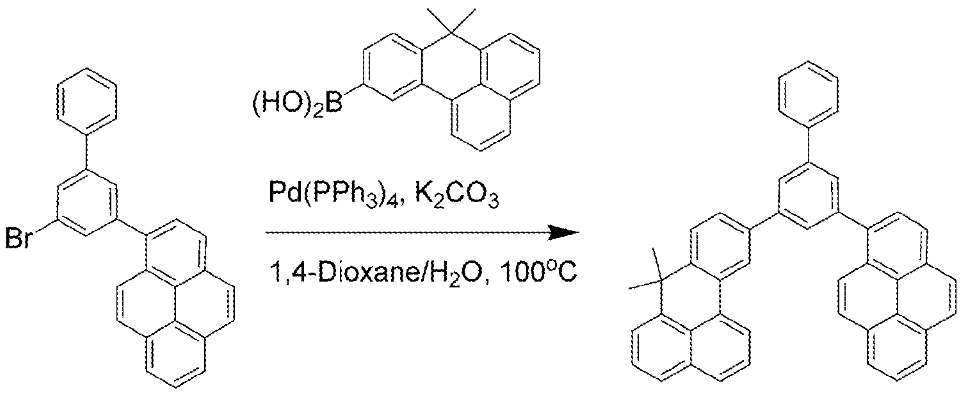


M8 BH1-20

Synthesis of Compound BH1-20

Under argon atmosphere, 2.60 g of an intermediate M8 (bromo intermediate), 2.02 g of a boronic acid intermediate synthesized by a known method, 0.25 g of tetrakis(triphenylphosphine)palladium(0), 1.67 g of potassium carbonate, 50 mL of 1,4-dioxane, and 10 mL of ion-exchange water were put into a flask and refluxed at 100 degrees C for six hours with stirring. After the reflux with stirring, the obtained solution was cooled to the room temperature. After the solution was cooled, the deposited solid was filtered and collected. The obtained solid was sequentially washed with water and acetone, and then recrystallized with a mixed solvent of toluene and hexane to obtain 2.89 g of a light yellow solid (yield of 69%). As a result of FD-MS analysis, the light yellow solid was the compound BH1-20 and m/e was equal to 597 while a molecular weight was 596.77.

Synthesis Example 21: Synthesis of Compound BH1-21

An intermediate M9 was synthesized through a synthesis pathway below.

[Formula 545]

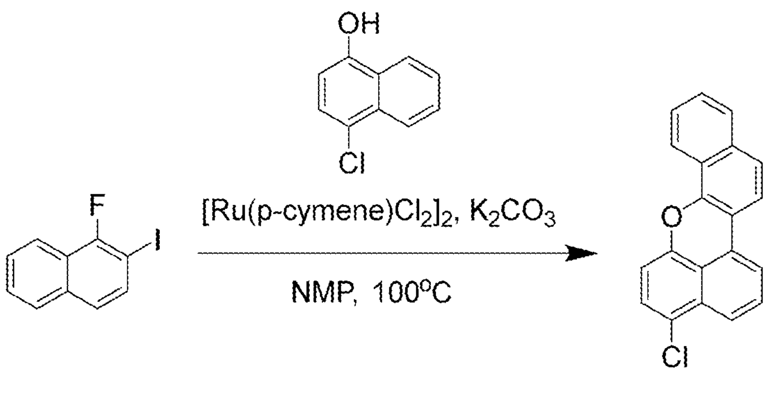


M9

Synthesis of Intermediate M9

Under argon atmosphere, 10.0 g of 1-fluoro-2-iodonaphthalene, 6.57 g of 4-chloro-1-naphthol, 0.90 g of dichloro(p-cymene)ruthenium(II) (dimer), 7.21 g of potassium carbonate, and 200 mL of N-methyl-2-pyrrolidone were put into a flask and refluxed at 100 degrees C for six hours with stirring. After the reflux with stirring, the obtained solution was cooled to the room temperature. After the solution was cooled, the deposited solid was filtered and collected. The obtained solid was sequentially washed with water and acetone, and then recrystallized with a mixed solvent of toluene and hexane to obtain 1.20 g of a light yellow solid (yield of 11%). As a result of FD-MS analysis, the light yellow solid was the intermediate M9 and m/e was equal to 303.

A compound BH1-21 was synthesized through a synthesis pathway below.

[Formula 546]

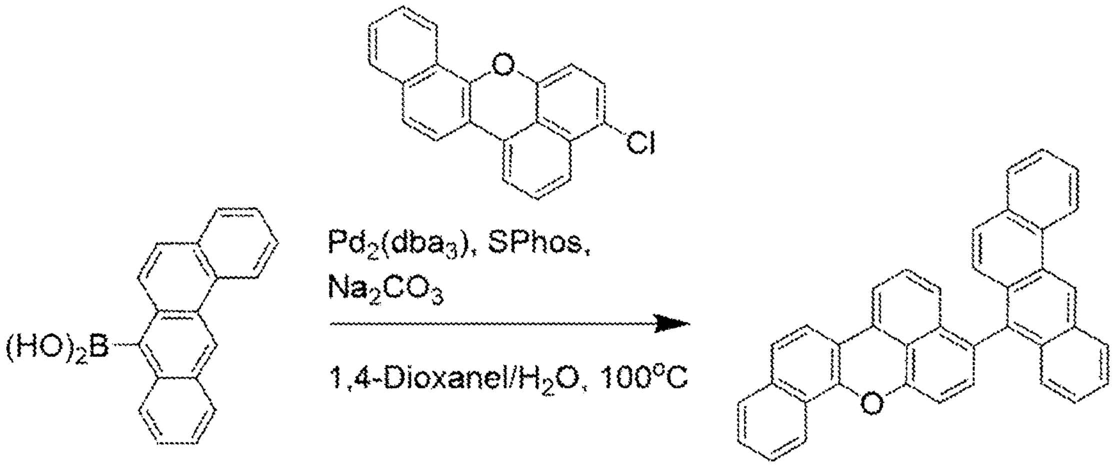


BH1-21

Synthesis of Compound BH1-21

Under argon atmosphere, 4.88 g of the intermediate M9, 8.16 g of tetraphene-7-yl boronic acid synthesized by a known method, 0.62 g of tris(dibenzylideneacetone)dipalladium(0), 1.77 g of 2-dicyclohexylphosphino-2',6'-dimethoxy-1,1'-biphenyl, 5.11 g of sodium carbonate, 150 mL of 1,4-dioxane, and 20 mL of ion-exchange water were put into a flask and refluxed at 100 degrees C for six hours with stirring. After the reflux with stirring, the obtained solution was cooled to the room temperature. After the solution was cooled, the deposited solid was filtered and collected. The obtained solid was sequentially washed with water and acetone, and then recrystallized with a mixed solvent of toluene and hexane to obtain 3.82 g of a light yellow solid (yield of 48%). As a result of FD-MS analysis, the light yellow solid was a compound BH1-21 and m/e was equal to 495 while a molecular weight was 494.59.

Synthesis Example 22: Synthesis of Compound BH1-22

A compound X-1 was synthesized through a synthesis pathway below.

[Formula 547]

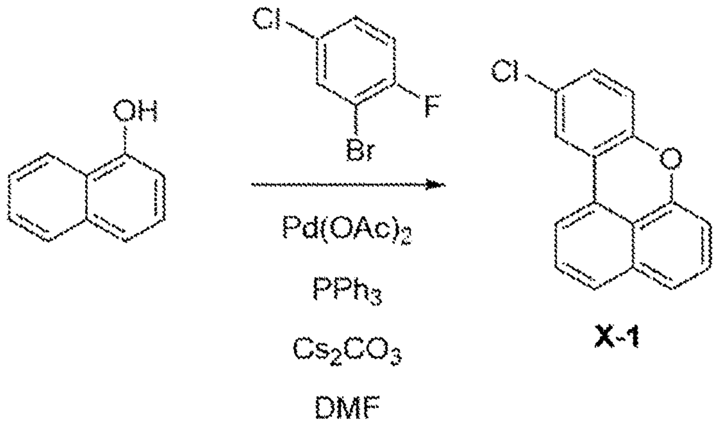


X-1

Synthesis of Compound X-1

1-hydroxynaphthalene (1.4 g), 2-bromo-4-chloro-1-fluorobenzene (2.5 g), diacetoxy palladium (0.11 g), triphenylphosphine (0.53 g), and cesium carbonate (13 g) were added to dimethyl formamide (50 mL). Under argon atmosphere, the obtained solution was heated to 140 degrees C and stirred for six hours. After the reaction solution was stirred, a solvent was distilled away from the reaction solution under reduced pressure. The obtained residue was subjected to silica-gel column chromatography to obtain 1.2 g of a white solid (yield of 49%). As a result of FD-MS analysis, the white solid was the compound X-1 and m/e was equal to 253 while a molecular weight was 252.70.

A compound X-2 was synthesized through a synthesis pathway below.

[Formula 548]

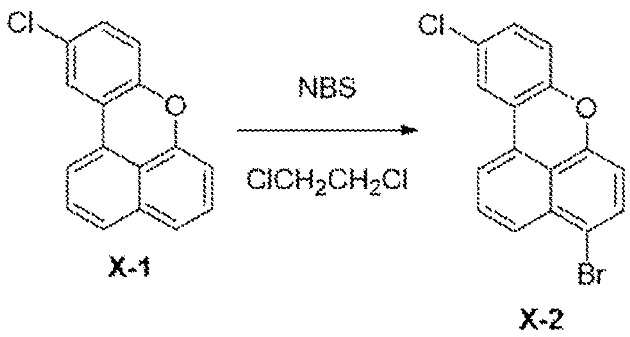


Synthesis of Compound X-2

The compound X-1 (10 g) was dissolved in 1,2-dichloroethane (200 mL), to which N-bromosuccinimide (NBS) (6.7g) was added. Under nitrogen atmosphere, the reaction solution was heated to 80 degrees C and stirred for six hours. After the reaction solution was stirred, a sodium carbonate aqueous solution was added to the reaction solution. The deposited solid was collected by filtration and recrystallized with toluene to obtain 7.2 g of a light yellow solid (yield of 55%). As a result of FD-MS analysis, the light yellow solid was the compound X-2 and m/e was equal to 332 while a molecular weight was 331.59.

A compound X-3 was synthesized through a synthesis pathway below.

[Formula 549]

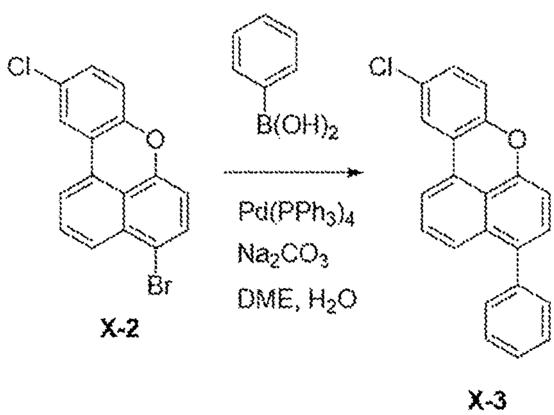


Synthesis of Compound X-3

The compound X-2 (5.0 g), phenylboronic acid (2.8 g), and tetrakis(triphenylphosphine)palladium (0.5 g) were added to dimethoxyethane (150 mL), to which a sodium carbonate aqueous solution (2.0M, 19 mL) was added. The obtained solution was stirred overnight at 80 degrees C under nitrogen atmosphere. After the solution was stirred, a solvent was distilled away from the solution under reduced pressure. The obtained residue was subjected to silica-gel column chromatography to obtain 3.9 g of a yellow solid (yield of 78%). As a result of FD-MS analysis, the yellow solid

was the compound X-3 and m/e was equal to 329 while a molecular weight was 328.80.

A compound BH1-22 was synthesized through a synthesis pathway below.

[Formula 550]

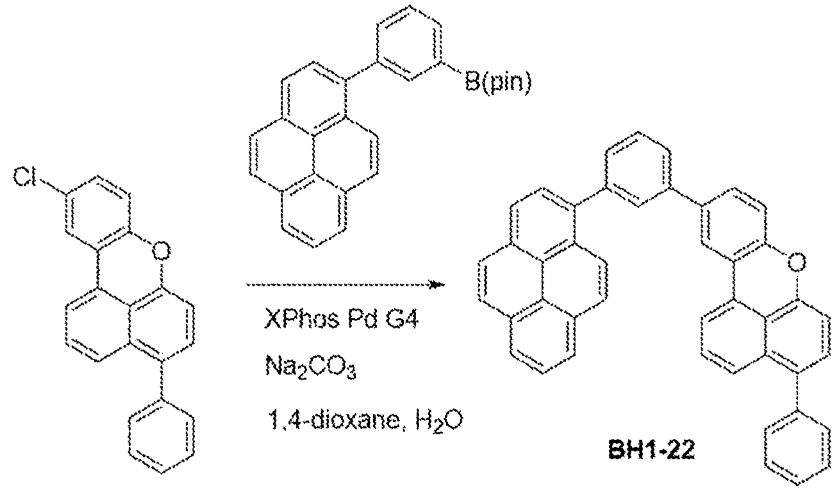


X-3

BH1-22

Synthesis of Compound BH1-22

The compound X-3 (0.8 g), 4,4,5,5-tetramethyl-2-[3-(1 -pyrenyl)phenyl]-1,3,2-dioxaborolane (1.0 g), and XPhos Pd G4 (0.1 g) were dissolved in 1,4-dioxane (13 mL), to which a sodium carbonate aqueous solution (2.0M, 3.8 mL) was added. Under argon atmosphere, the obtained solution was heated to 100 degrees C and stirred for one hour.

After being stirred, the reaction solution was filtered to obtain a solid. The solid was recrystallized with 1,4-dioxane to obtain 1.0 g of a light yellow solid (yield of 71%). As a result of FD-MS analysis, the light yellow solid was the compound BH1-22 and m/e was equal to 571 while a molecular weight was 570.69.

Synthesis Example 23: Synthesis of Compound BH1-23

A compound P-1 was synthesized through a synthesis pathway below.

[Formula 551]

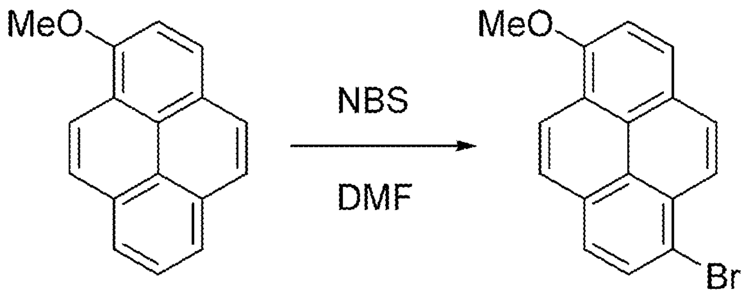


P-1

Synthesis of Compound P-1

1-methoxypyrene (5.0 g) was dissolved in N,N-dimethylformamide (DMF) (100 mL) and cooled to 0 degree C in an ice bath. N-bromosuccinimide (NBS) (3.6 g) was added to this solution. The obtained solution was stirred for two hours, and subsequently stirred for another two hours at the room temperature. After being stirred, the reaction solution was added with water to deposit a solid. The solid was collected by filtration to obtain 6.3 g of a colorless solid (yield of

94%). As a result of FD-MS analysis, the colorless solid was the compound P-1 and m/e was equal to 311 while a molecular weight was 311.18.

A compound P-2 was synthesized through a synthesis pathway below.

[Formula 552]

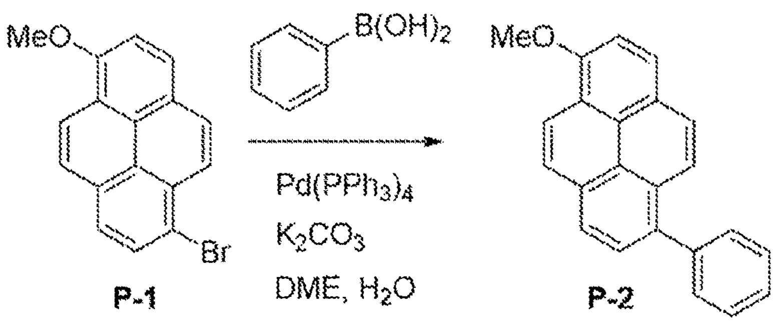


Synthesis of Compound P-2

The compound P-1 (5.8 g), phenylboronic acid (2.95 g), and tetrakis(triphenylphosphine)palladium (0.64 g) were dissolved in dimethoxyethane (80 mL), to which an aqueous solution (16 mL) of potassium carbonate (5.2 g) was added. Under argon atmosphere, the reaction solution was stirred for eight hours while being heated to 80 degrees C. After the reaction solution was stirred, a solvent was distilled away from the reaction solution under reduced pressure. The obtained residue was subjected to gel filtration chromatography to obtain 4.6 g of a colorless solid (yield of 80%). As a result of FD-MS analysis, the colorless solid was the compound P-2 and m/e was equal to 308 while a molecular weight was 308.38.

A compound P-3 was synthesized through a synthesis pathway below.

[Formula 553]

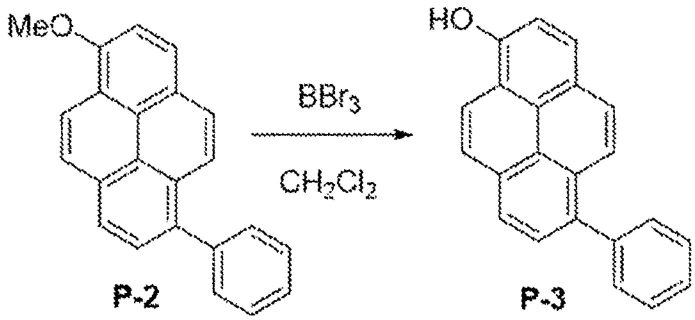


Synthesis of Compound P-3

The compound P-2 (4.6 g) was dissolved in dichloromethane (100 mL), to which 60 mL of boron tribromide in dichloromethane (1.0 M) was added dropwise under ice-cooling. Subsequently, the obtained solution was stirred at the room temperature for two days. The stirred reaction solution was added to ice water and extracted with ethyl acetate. Subsequently, an organic layer from which a solvent was distilled away was subjected to silica-gel column chromatography to obtain 4.2 g of a colorless solid (yield of 95%). As a result of FD-MS analysis, the colorless solid was the compound P-3 and m/e was equal to 294 while a molecular weight was 294.35.

A compound P-4 was synthesized through a synthesis pathway below.

[Formula 554]

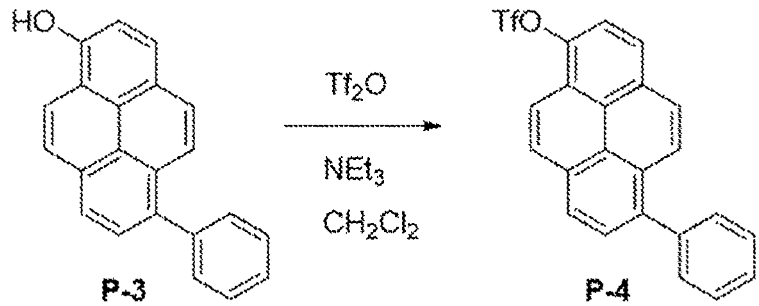


P-3 P-4

Synthesis of Compound P-4

The compound P-3 (4.3 g) and trimethylamine (3.0 g) were dissolved in dichloromethane (50 mL), to which trifluoromethanesulfonic anhydride (5.0 g) was added while being cooled in an ice bath. After the reaction solution was stirred for two hours, a solvent was distilled away from the reaction solution under reduced pressure. The obtained residue was subjected to silica-gel column chromatography to obtain 4.3 g of a white solid (yield of 85%). As a result of FD-MS analysis, the white solid was the compound P-4 and m/e was equal to 426 while a molecular weight was 426.41.

A compound P-5 was synthesized through a synthesis pathway below.

[Formula 555]

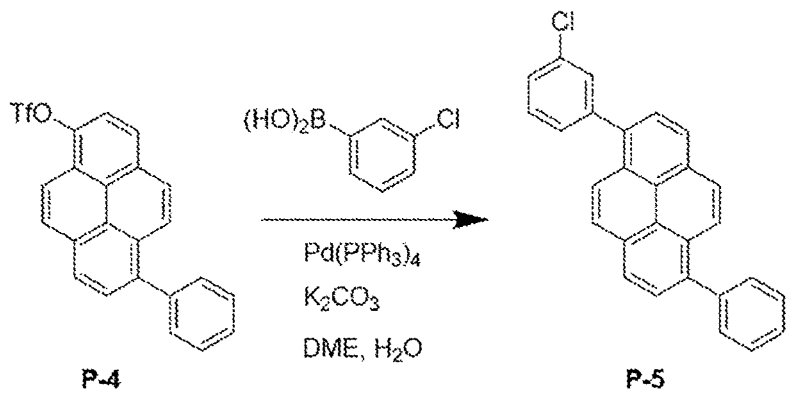


P-4 P-5

Synthesis of Compound P-5

The compound P-4 (1.7 g), 3-chlorophenylboronic acid (0.69 g), and tetrakis(triphenylphosphine)palladium (0.23 g) were dissolved in dimethoxyethane (20 mL), to which an aqueous solution (4.0 mL) of potassium carbonate (1.1 g) was added. The obtained solution was stirred at 80 degrees C for five hours under argon atmosphere. After the reaction solution was stirred, a solvent was distilled away from the reaction solution under reduced pressure. The obtained residue was subjected to gel filtration chromatography to obtain 1.2 g of a white solid (yield of 78%). As a result of FD-MS analysis, the white solid was the compound P-5 and m/e was equal to 389 while a molecular weight was 388.89.

A compound BH1-23 was synthesized through a synthesis pathway below.

[Formula 556]

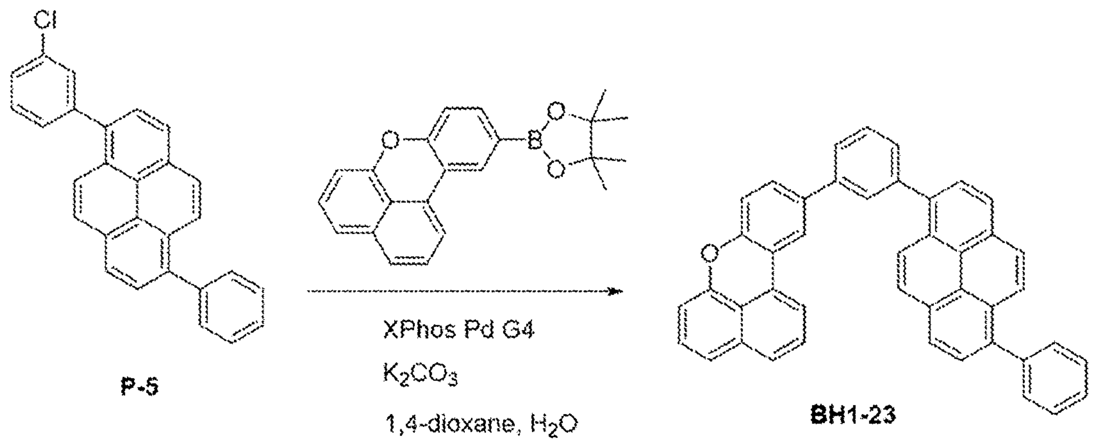


P-5 BH1-23

Synthesis of Compound BH1-23

The compound P-5 (1.2 g), benzo[kl]xanthene-10-yl-boronic acid (3.70 g), and XPhos Pd G4 (0.13 g) were added to and dissolved in 1,4-dioxane (15 mL), to which an aqueous solution (3.0 mL) of potassium carbonate (1.3 g) was added. Under argon atmosphere, the obtained solution was heated for reflux with stirring for two hours. After being stirred, the reaction solution was filtered to obtain a solid. The solid was purified by silica-gel column chromatography to obtain 0.58 g of a white solid (yield of 33%). As a result of FD-MS analysis, the white solid was the compound BH1-23 and m/e was equal to 571 while a molecular weight was 570.69.

Synthesis Example 24: Synthesis of Compound BH1-24

A compound X-4 was synthesized through a synthesis pathway below.

[Formula 557]

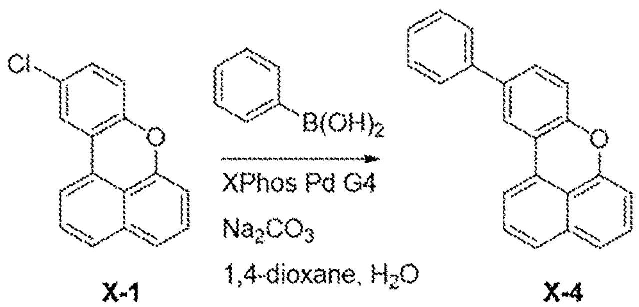


X-1 X-4

Synthesis of Compound X-4

The compound X-1 (1.0 g), phenylboronic acid (0.9 g), and XPhos Pd G4 (0.17 g) were added to 1,4-dioxane (50 mL), to which a sodium carbonate aqueous solution (2.0M, 6.0 mL) was added. Under argon atmosphere, the obtained solution was heated at 100 degrees C for six hours with stirring.

After the reaction solution was stirred, a solvent was distilled away from the reaction solution under reduced pressure. The obtained residue was subjected to silica-gel column chromatography to obtain 0.7 g of a light yellow solid (yield of 60%). As a result of FD-MS analysis, the light yellow solid was the compound X-4 and m/e was equal to 294 while a molecular weight was 294.35.

A compound X-5 was synthesized through a synthesis pathway below.

[Formula 558]

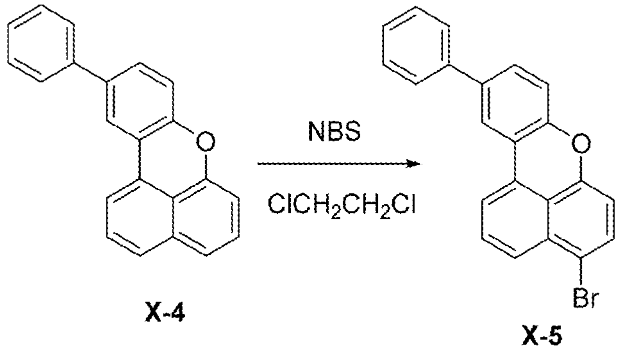


X-4

X-5

Synthesis of Compound X-5

The compound X-4 (1.0 g) was dissolved in 1,2-dichloroethane (20 mL), to which N-bromosuccinimide (NBS) (0.61g) was added. Under nitrogen atmosphere, the reaction solution was heated to 80 degrees C and stirred for six hours. After the reaction solution was stirred, a sodium carbonate aqueous solution was added to the reaction solution. The deposited solid was collected by filtration and recrystallized with toluene to obtain 0.63 g of a yellow solid (yield of 50%). As a result of FD-MS analysis, the yellow solid was the compound X-5 and m/e was equal to 373 while a molecular weight was 373.25.

A compound BH1-24 was synthesized through a synthesis pathway below.

[Formula 559]

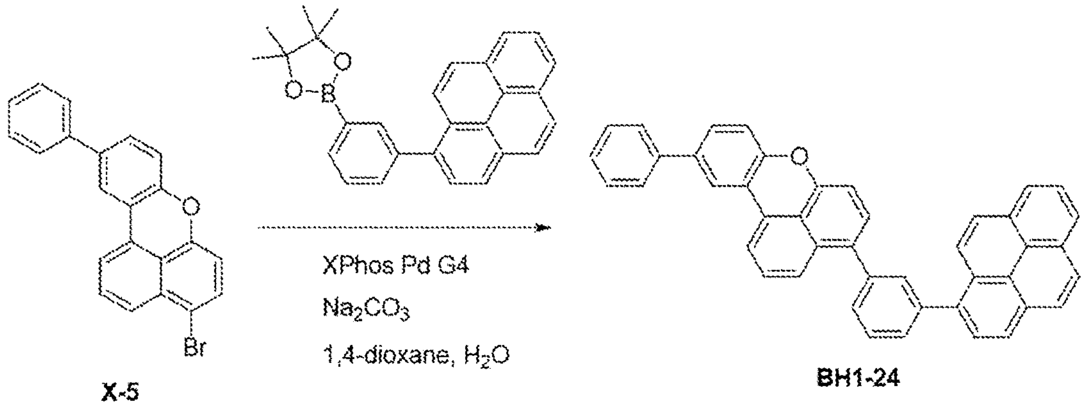


X-5

BH1-24

Synthesis of Compound BH1-24

The compound X-5 (1.0 g), 4,4,5,5-tetramethyl-2-[3-(1 -pyrenyl)phenyl]-1,3,2-dioxaborolane (1.3 g), and XPhos Pd G4 (0.1 g) were dissolved in 1,4-dioxane (20 mL), to which a sodium carbonate aqueous solution (2.0M, 4.0 mL) was added. Under argon atmosphere, the obtained solution was heated to 100 degrees C and stirred for one hour.

After being stirred, the reaction solution was filtered to obtain a solid. The solid was recrystallized with 1,4-dioxane to obtain 1.0 g of a light yellow solid (yield of 65%). As a result of FD-MS analysis, the light yellow solid was the compound BH1-24 and m/e was equal to 571 while a molecular weight was 570.69.

Synthesis Example 25: Synthesis of Compound BH1-25

A compound X-6 was synthesized through a synthesis pathway below.

[Formula 560]

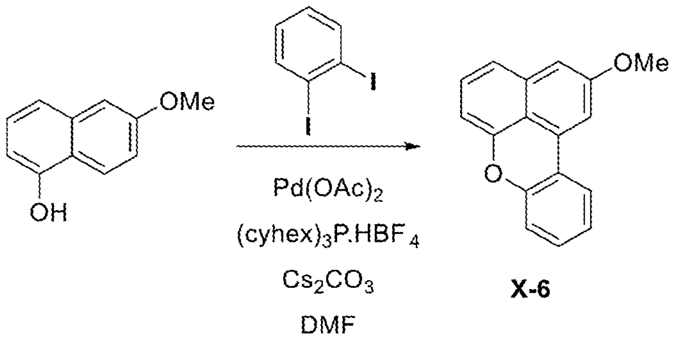


X-6

Synthesis of Compound X-6

6-methoxy-1-naphthol (0.17 g), 1,2-diiodebenzene (0.40 g), palladium acetate (0.02 g), tricyclohexylphosphine tetrafluoroborate (0.07 g), and cesium carbonate (1.17 g) were added to N,N-dimethylformamide (DMF) (10 mL) and stirred at 140 degrees C for four hours under argon atmosphere. After being stirred, the reaction solution was purified by silica-gel column chromatography to obtain 0.25 g of a colorless solid (yield of 72%). As a result of FD-MS analysis, the colorless solid was the compound X-6 and m/e was equal to 248 while a molecular weight was 248.28.

A compound X-7 was synthesized through a synthesis pathway below.

[Formula 561]

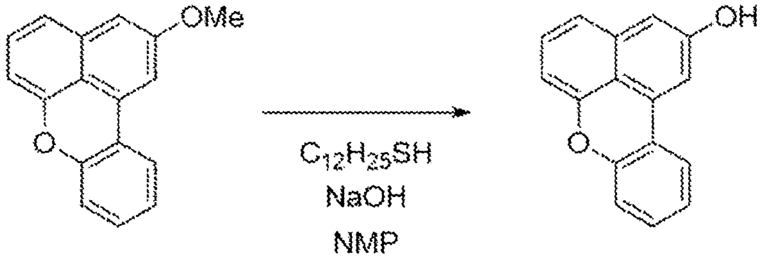


X-6 X-7

[1129]

Synthesis of Compound X-7

The compound X-6 (1.0 g), dodecanethiol (1.2 g), and sodium hydroxide (0.48 g) were added to N-methylpyrrolidone (NMP) (40 mL). The obtained solution was stirred overnight at 150 degrees C under argon atmosphere. After being stirred, the reaction solution was purified by column chromatography. The obtained crude product was recrystallized with toluene to obtain 0.74 g of a colorless solid (yield of 78%). As a result of FD-MS analysis, the colorless solid was the compound X-7 and m/e was equal to 234 while a molecular weight was 234.25.

A compound X-8 was synthesized through a synthesis pathway below.

[Formula 562]

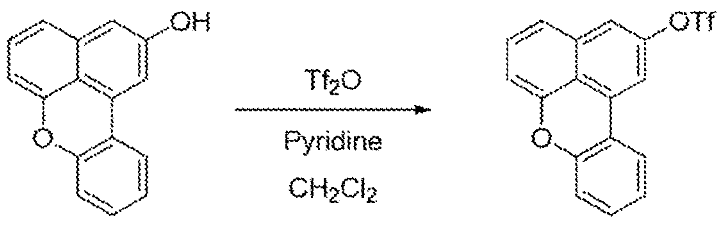


X-7 X-8

Synthesis of Compound X-8

The compound X-7 (2.47 g), trifluoromethanesulfonic anhydride (3.57 g), and pyridine (1.00 g) were added to dichloromethane (75 mL) under ice-cooling. The obtained solution was stirred at the room temperature for seven hours under argon atmosphere. After being stirred, the reaction solution was purified by silica-gel column chromatography to obtain 3.47 g of a colorless solid (yield of 90%). As a result of FD-MS analysis, the colorless solid was the compound X-8 and m/e was equal to 366 while a molecular weight was 366.31.

A compound BH1-25 was synthesized through a synthesis pathway below.

[Formula 563]

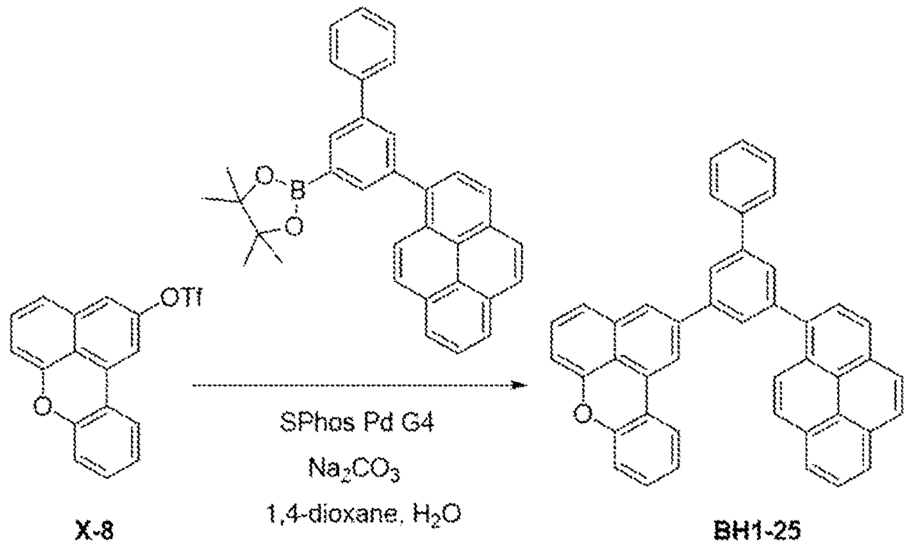


X-8 BH1-25

Synthesis of Compound BH1-25

The compound X-8 (2.0 g), 4,4,5,5-tetramethyl-2-(5-(pyrene-1-yl)-[1,1'-biphenyl]-3-yl)-1,3,2-dioxaborolane (2.6 g), SPhos Pd G4 (0.22 g), and a sodium carbonate aqueous solution (2M, 8.2 mL) were added to 1,4-dioxane (55 mL). Under argon atmosphere, the obtained solution was stirred overnight at 90 degrees C. After the reaction solution was stirred, a solvent was distilled away from the reaction solution under reduced pressure. The obtained residue was recrystallized with toluene to obtain 2.2 g of a light yellow solid (yield of 70%). As a result of FD-MS analysis, the light yellow solid was the compound BH1-25 and m/e was equal to 571 while a molecular weight was 570.69.

Synthesis Example 26: Synthesis of Compound BH1-26

A compound X-10 was synthesized through a synthesis pathway below.

[Formula 564]

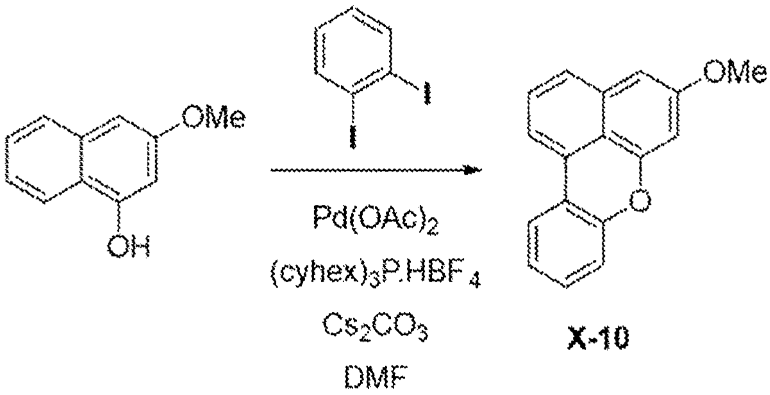


X-10

Synthesis of Compound X-10

3-methoxy-1-naphthol (5.2 g), 1,2-diiodebenzene (12 g), palladium acetate (0.67 g), tricyclohexylphosphine tetrafluoroborate (2.2 g), and cesium carbonate (35 g) were added to N,N-dimethylformamide (DMF) (100 mL) and stirred at 140 degrees C for six hours under argon atmosphere. After being stirred, the reaction solution was purified by silica-gel column chromatography to obtain 3.7 g of a colorless solid (yield of 50%). As a result of FD-MS analysis, the colorless solid was the compound X-10 and m/e was equal to 248 while a molecular weight was 248.28.

A compound X-11 was synthesized through a synthesis pathway below.

[Formula 565]

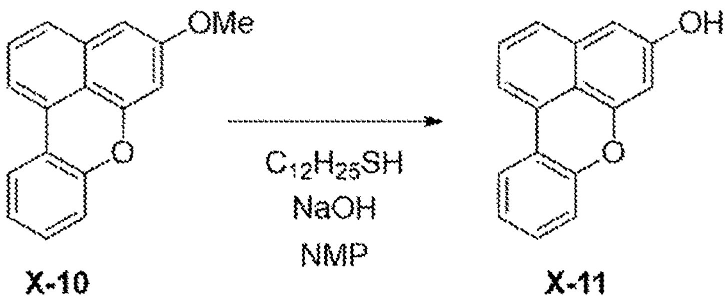


X-10 X-11

Synthesis of Compound X-11

The compound X-10 (4.0 g), dodecanethiol (10 g), and sodium hydroxide (3.8 g) were added to N-methylpyrrolidone (NMP) (160 mL). The obtained solution was stirred at 140 degrees C for five hours under argon atmosphere. After being stirred, the reaction solution was purified by silica-gel column chromatography to obtain 3.56 g of a colorless solid (yield of 95%). As a result of FD-MS analysis, the colorless solid was the compound X-11 and m/e was equal to 234 while a molecular weight was 234.25.

A compound X-12 was synthesized through a synthesis pathway below.

[Formula 566]

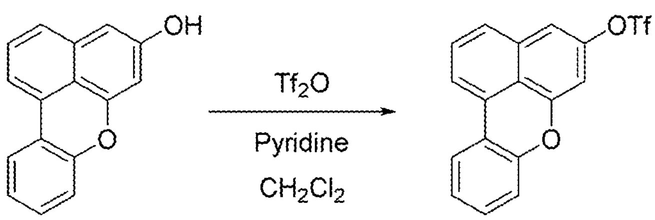


X-11 X-12

Synthesis of Compound X-12

The compound X-11 (3.5 g), trifluoromethanesulfonic anhydride (5.2 g), and pyridine (1.4 g) were added to dichloromethane (150 mL) under ice-cooling. The obtained solution was stirred at the room temperature for three hours under argon atmosphere. After being stirred, the reaction solution was purified by silica-gel column chromatography to obtain 1.3 g of a colorless solid (yield of 24%). As a result of FD-MS analysis, the colorless solid was the compound X-12 and m/e was equal to 366 while a molecular weight was 366.31.

A compound BH1-26 was synthesized through a synthesis pathway below.

[Formula 567]

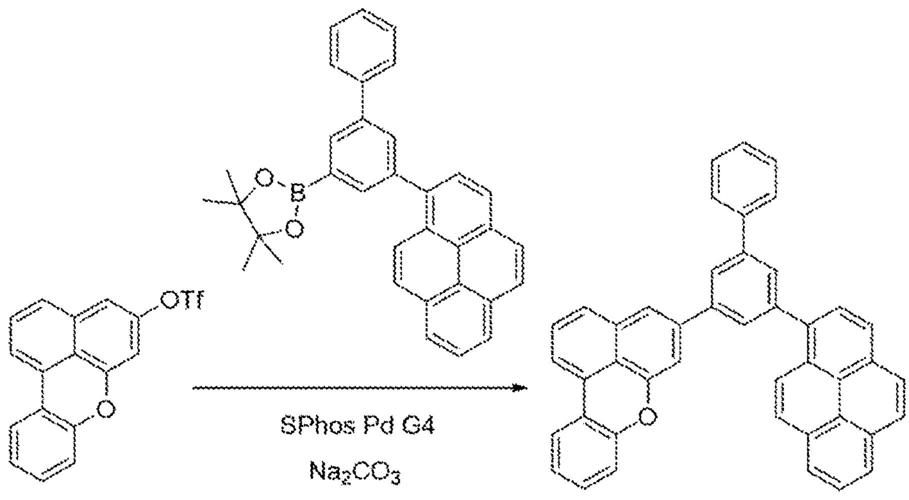


X-12 BH1-26

Synthesis of Compound BH1-26

The compound X-12 (1.2 g), 4,4,5,5-tetramethyl-2-(5-(pyrene-1-yl)-[1,1'-biphenyl]-3-yl)-1,3,2-dioxaborolane (1.5 g), SPhos Pd G4 (0.12 g), and a sodium carbonate aqueous solution (2M, 3.8 mL) were added to 1,4-dioxane (30 mL). Under argon atmosphere, the obtained solution was stirred at 90 degrees C for seven hours. After the reaction solution was stirred, a solvent was distilled away from the reaction solution under reduced pressure. The obtained residue was recrystallized with toluene to obtain 0.77 g of a light yellow solid (yield of 45%). As a result of FD-MS analysis, the light yellow solid was the compound BH1-26 and m/e was equal to 571 while a molecular weight was 570.69.

Synthesis Example 27: Synthesis of Compound BH1-27

A compound X-14 was synthesized through a synthesis pathway below.

[Formula 568]

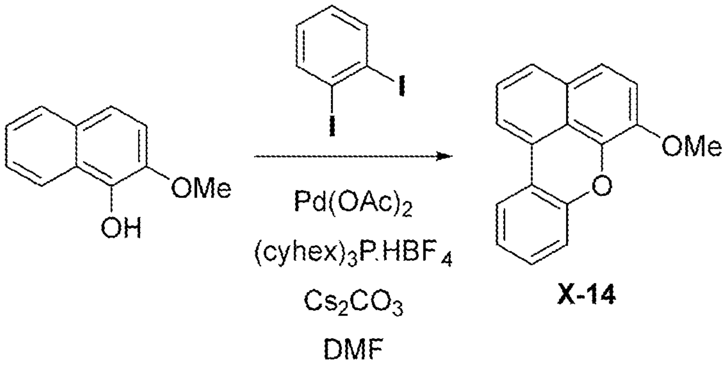


X-14

Synthesis of Compound X-14

2-methoxy-1-naphthol (1.7 g), 1,2-diiodebenzene (4.0 g), palladium acetate (0.23 g), tricyclohexylphosphine tetrafluoroborate (0.74 g), and cesium carbonate (12 g) were added to N,N-dimethylformamide (DMF) (33 mL) and stirred at 140 degrees C for seven hours under argon atmosphere. After being stirred, the reaction solution was purified by silica-gel column chromatography to obtain 2.2 g of a colorless solid (yield of 45%). As a result of FD-MS analysis, the colorless solid was the compound X-14 and m/e was equal to 248 while a molecular weight was 248.28.

A compound X-15 was synthesized through a synthesis pathway below.

[Formula 569]

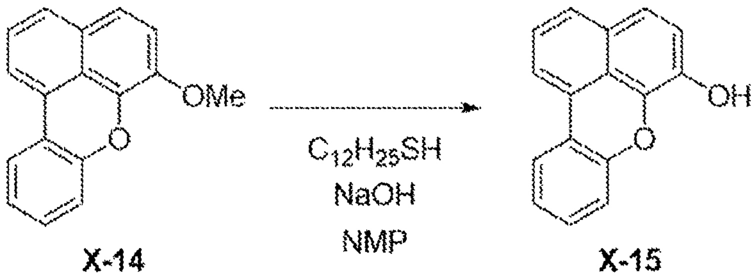


X-14 X-15

Synthesis of Compound X-15

The compound X-14 (2.2 g), dodecanethiol (5.7 g), and sodium hydroxide (2.2 g) were added to N-methylpyrrolidone (NMP) (90 mL). The obtained solution was stirred at 140 degrees C for five hours under argon atmosphere. After being stirred, the reaction solution was purified by silica-gel column chromatography to obtain 1.4 g of a colorless solid (yield of 70%). As a result of FD-MS analysis, the colorless solid was the compound X-15 and m/e was equal to 234 while a molecular weight was 234.25.

A compound X-16 was synthesized through a synthesis pathway below.

[Formula 570]

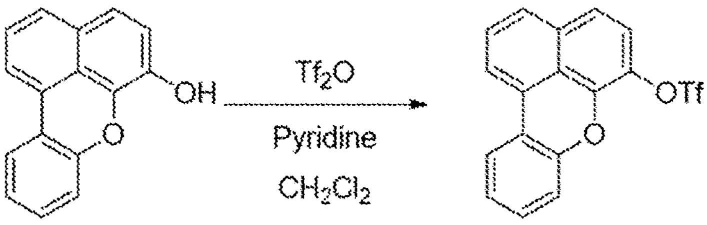


X-15 X-16

Synthesis of Compound X-16

The compound X-15 (1.4 g), trifluoromethanesulfonic anhydride (2.1 g), and pyridine (0.57 g) were added to

dichloromethane (60 mL) under ice-cooling. The obtained solution was stirred at the room temperature for four hours under argon atmosphere. After being stirred, the reaction solution was purified by silica-gel column chromatography to obtain 1.6 g of a colorless solid (yield of 71 %). As a result of FD-MS analysis, the colorless solid was the compound X-16 and m/e was equal to 366 while a molecular weight was 366.31.

A compound BH1-27 was synthesized through a synthesis pathway below.

[Formula 571]

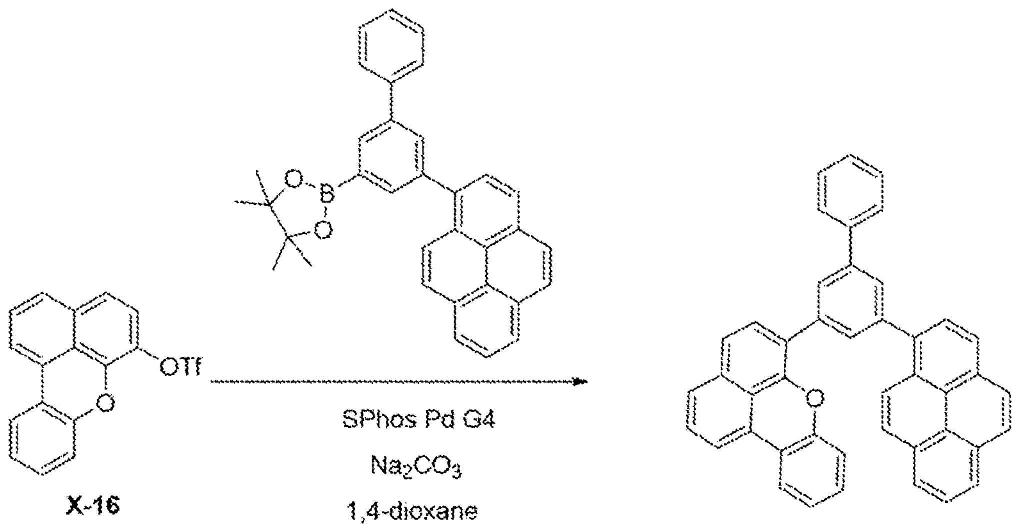


X-16

BH1-27

Synthesis of Compound BH1-27

The compound X-16 (1.4 g), 4,4,5,5-tetramethyl-2-(5-(pyrene-1-yl)-[1,1'-biphenyl]-3-yl)-1,3,2-dioxaborolane (1.7 g), SPhos Pd G4 (0.28 g), and a sodium carbonate aqueous solution (2M, 4.4 mL) were added to 1,4-dioxane (55 mL). Under argon atmosphere, the obtained solution was stirred at 100 degrees C for seven hours. After the reaction solution was stirred, a solvent was distilled away from the reaction solution under reduced pressure. The obtained residue was recrystallized with toluene to obtain 0.92 g of a light yellow solid (yield of 46%). As a result of FD-MS analysis, the light yellow solid was the compound BH1-27 and m/e was equal to 571 while a molecular weight was 570.69.

Synthesis Example 28: Synthesis of Compound BH1-28

A compound X-9 was synthesized through a synthesis pathway below.

[Formula 572]

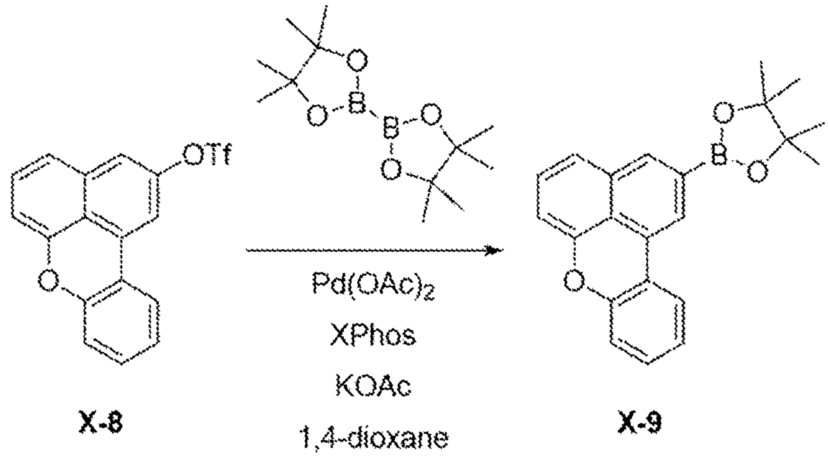


X-8

X-9

Synthesis of Compound X-9

The compound X-8 (2.4 g), bis(pinacolato)diboron (3.5 g), palladium acetate (0.1 g), XPhos (0.4 g), and potassium acetate (2.7 g) were added to 1,4-dioxane (50 mL). Under argon atmosphere, the obtained solution was stirred at 100 degrees C for six hours. After being stirred, the reaction solution was purified by silica-gel column chromatography to obtain 2.0 g of a colorless solid (yield of 89%). As a result of FD-MS analysis, the colorless solid was the compound X-9 and m/e was equal to 344 while a molecular weight was 344.22.

A compound BH1-28 was synthesized through a synthesis pathway below.

[Formula 573]

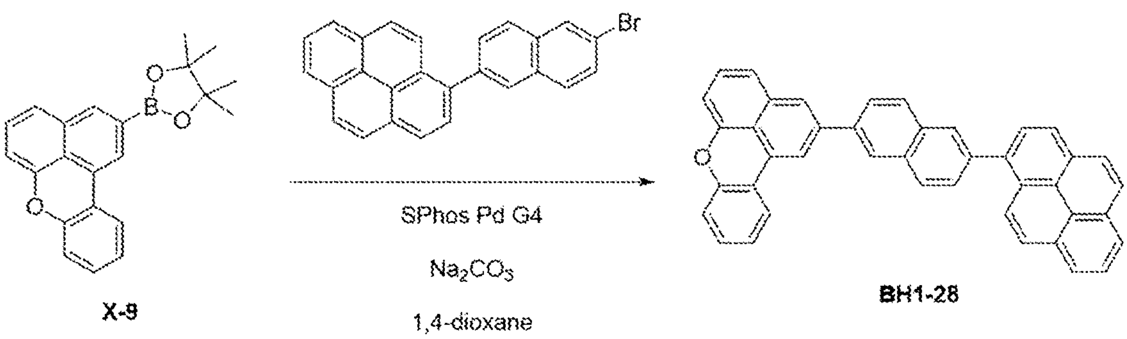


X-9

BH1-28

Synthesis of Compound BH1-28

The compound X-9 (1.2 g), 1-(6-bromonaphthalene-2-yl)-pyrene (1.3 g), SPhos Pd G4 (0.15 g), and a sodium carbonate aqueous solution (2M, 5.2 mL) were added to 1,4-dioxane (40 mL). Under argon atmosphere, the obtained solution was stirred overnight at 90 degrees C. After the reaction solution was stirred, a solvent was distilled away from the reaction solution under reduced pressure. The obtained residue was recrystallized with toluene to obtain 0.85 g of a yellow solid (yield of 45%). As a result of FD-MS analysis, the yellow solid was the compound BH1-28 and m/e was equal to 545 while a molecular weight was 544.65.

Synthesis Example 29: Synthesis of Compound BH1-29

A compound X-13 was synthesized through a synthesis pathway below.

[Formula 574]

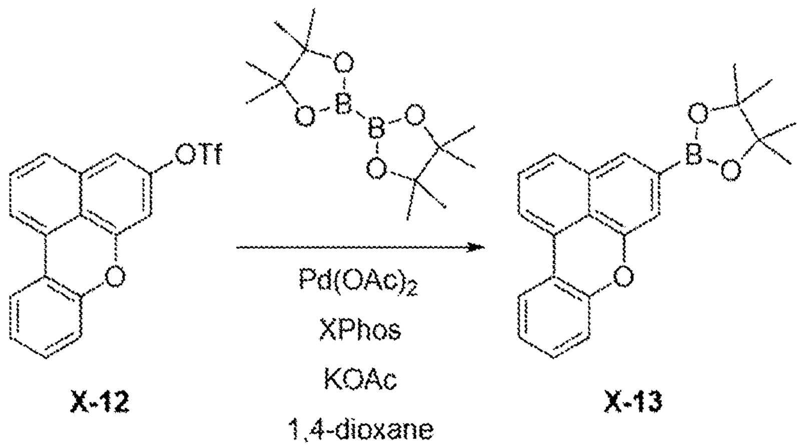


X-12 X-13

Synthesis of Compound X-13

The compound X-12 (2.2 g), bis(pinacolato)diboron (3.1 g), palladium acetate (0.1 g), XPhos (0.3 g), and potassium acetate (1.8 g) were added to 1,4-dioxane (50 mL). Under argon atmosphere, the obtained solution was stirred at 100 degrees C for six hours. After being stirred, the reaction solution was purified by silica-gel column chromatography to obtain 1.8 g of a colorless solid (yield of 87%). As a result of FD-MS analysis, the colorless solid was the compound X-13 and m/e was equal to 344 while a molecular weight was 344.22.

A compound BH1-29 was synthesized through a synthesis pathway below.

[Formula 575]

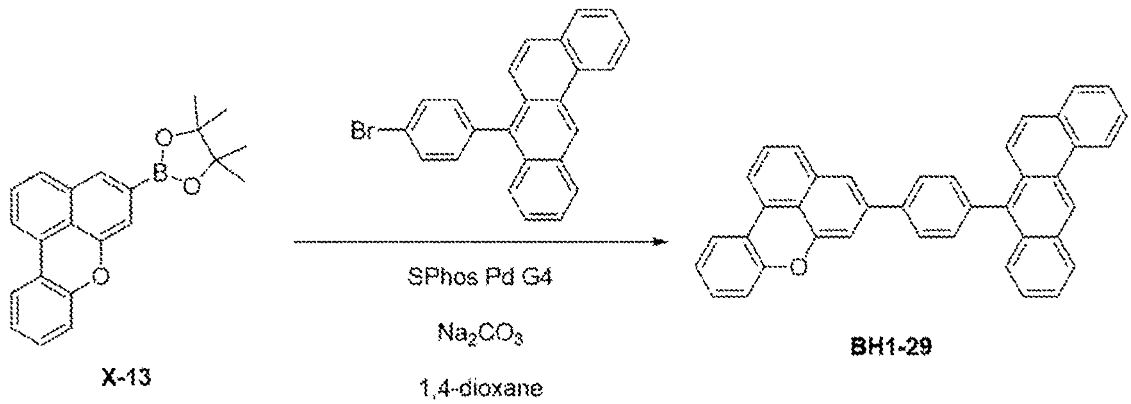


X-13 BH1-29

Synthesis of Compound BH1-29

The compound X-13 (3.1 g), 7-(4-bromophenyl)-tetraphene (3.5 g), SPhos Pd G4 (0.32 g), and a sodium carbonate aqueous solution (2M, 15 mL) were added to 1,4-dioxane (100 mL). Under argon atmosphere, the obtained solution was stirred at 90 degrees C for five hours. After the reaction solution was stirred, a solvent was distilled away from the reaction solution under reduced pressure. The obtained residue was recrystallized with toluene to obtain 3.1 g of a light yellow solid (yield of 66%). As a result of FD-MS analysis, the light yellow solid was the compound BH1-29 and m/e was equal to 521 while a molecular weight was 520.63.

Synthesis Example 30: Synthesis of Compound BH1-30

A compound X-17 was synthesized through a synthesis pathway below.

[Formula 576]

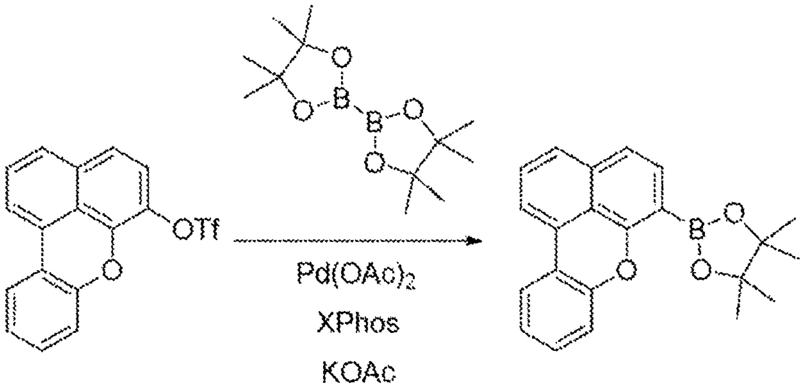


X-16 X-17

Synthesis of Compound X-17

The compound X-16 (1.5 g), bis(pinacolato)diboron (2.1 g), palladium acetate (0.05 g), XPhos (0.2 g), and potassium acetate (1.2 g) were added to 1,4-dioxane (50 mL). Under argon atmosphere, the obtained solution was stirred at 100 degrees C for six hours. After being stirred, the reaction solution was purified by silica-gel column chromatography to obtain 1.1 g of a colorless solid (yield of 78%). As a result of FD-MS analysis, the colorless solid was the compound X-17 and m/e was equal to 344 while a molecular weight was 344.22.

A compound BH1-30 was synthesized through a synthesis pathway below.

[Formula 577]

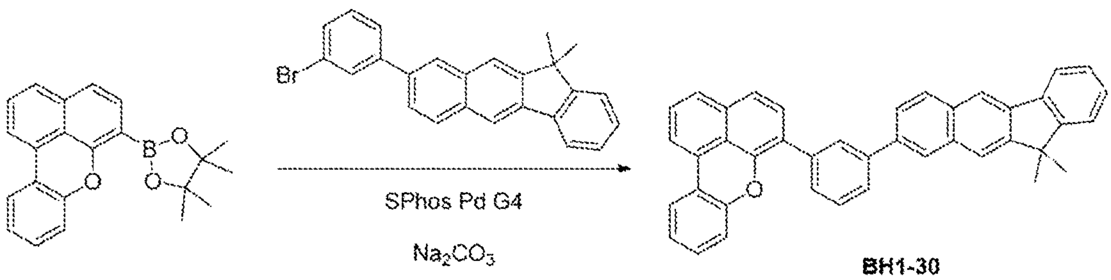


X-17 BH1-30

Synthesis of Compound BH1-30

The compound X-17 (1.4 g), 8-(3-bromophenyl)-11,11-dimethyl-11H-benzo[b]fluorene (1.6 g), SPhos Pd G4 (0.06 g), and a sodium carbonate aqueous solution (2M, 6.1 mL) were added to 1,4-dioxane (40 mL). Under argon atmosphere, the obtained solution was stirred overnight at 90 degrees C. After the reaction solution was stirred, a solvent was distilled away from the reaction solution under reduced pressure. The obtained residue was recrystallized with toluene to obtain 1.9 g of a colorless solid (yield of 89%). As a result of FD-MS analysis, the colorless solid was the compound BH1-30 and m/e was equal to 537 while a molecular weight was 536.67.

EXPLANATION OF CODE(S)

1... organic EL device, 2... substrate, 3... anode, 4... cathode, 51...first emitting layer, 52...second emitting layer, 6... hole injecting layer, 7... hole transporting layer, 8...electron transporting layer, 9...electron injecting layer

## Claims

**1.** A compound comprising: at least one group represented by a formula (11) below; and a single benz[de]anthracene

derivative skeleton represented by a formula (1000) below in a molecule,

[Formula 1]

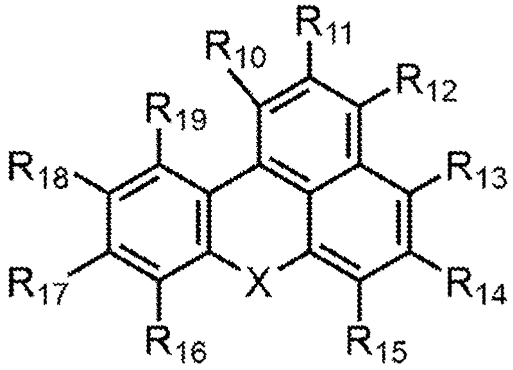

(1000)

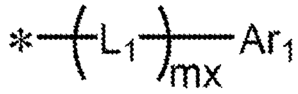

(11)

where: in the formula (1000):

X is an oxygen atom, a sulfur atom, $C(R_{2001})(R_{2002})$, or $Si(R_{2003})(R_{2004})$;
$R_{2001}$ to $R_{2004}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms;
at least one combination of adjacent two or more of $R_{10}$ to $R_{19}$ are mutually bonded to form a substituted or unsubstituted monocyclic ring, mutually bonded to form a substituted or unsubstituted fused ring, or not mutually bonded;
$R_{10}$ to $R_{19}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $- S-(R_{905})$, a group represented by $-N(R_{906})(R_{907})$, a substituted or unsubstituted aralkyl group having 7 to 50 carbon atoms, a group represented by $-C(=O)R_{801}$, a group represented by $-COOR_{802}$, a halogen atom, a nitro group, a substituted or unsubstituted aryl group having 6 to 50 ring carbon atoms, a substituted or unsubstituted heterocyclic group having 5 to 50 ring atoms, or a group represented by the formula (11);
at least one of $R_{10}$ to $R_{19}$ is a group represented by the formula (11);
when a plurality of groups represented by the formula (11) are present, the plurality of groups represented by the formula (11) are mutually the same or different;
$L_1$ is a substituted or unsubstituted arylene group having 6 to 15 ring carbon atoms, or a substituted or unsubstituted divalent heterocyclic group having 5 to 15 ring atoms;
mx is 1, 2, or 3;
when two or more $L_1$ are present, the two or more $L_1$ are mutually the same or different;
$Ar_1$ is a substituted or unsubstituted aryl group including four or more rings;
when two or more $Ar_1$ are present, the two or more $Ar_1$ are mutually the same or different;
* in the formula (11) represents a bonding position;
in a compound represented by the formula (1000), $R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{801}$ are present, the plurality of $R_{801}$ are mutually the same or different; and
when a plurality of $R_{302}$ are present, the plurality of $R_{302}$ are mutually the same or different.

**2.** The compound according to claim 1, wherein at least one combination of a combination of $R_{10}$ and $R_{11}$, a combination of $R_{11}$ and $R_{12}$, a combination of $R_{12}$ and $R_{13}$, a combination of $R_{13}$ and $R_{14}$, a combination of $R_{14}$ and $R_{15}$, a combination of $R_{16}$ and $R_{17}$, a combination of $R_{17}$ and $R_{18}$, a combination of $R_{18}$ and $R_{19}$, or a combination of $R_{19}$ and $R_{10}$ are mutually bonded to form a substituted or unsubstituted monocyclic ring, or mutually bonded to form a substituted or unsubstituted fused ring.

**3.** The compound according to claim 2, wherein one combination of the combination of $R_{13}$ and $R_{14}$, the combination of $R_{16}$ and $R_{17}$, and the combination of $R_{19}$ and $R_{10}$ are mutually bonded to form a substituted or unsubstituted benzene ring.

**4.** The compound according to claim 1, wherein the compound comprises: at least one group represented by the formula (11); and a single benzoxanthene ring represented by a formula (1) below in a molecule,

[Formula 2]

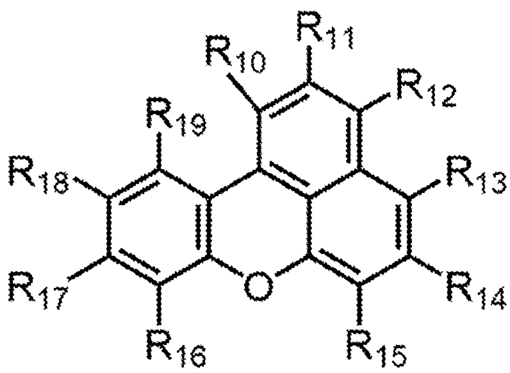

(1)

where: $R_{10}$ to $R_{19}$ each independently represent the same as $R_{10}$ to $R_{19}$ in the formula (1000);
none of combinations of adjacent two or more of $R_{10}$ to $R_{19}$ are mutually bonded; and
$Ar_1$, $L_1$, and mx respectively represent the same as $Ar_1$, $L_1$, and mx in the formula (11).

**5.** The compound according to claim 4, wherein the compound comprising at least one group represented by the formula (11) and a single benzoxanthene ring represented by the formula (1) in a molecule is represented by a formula (121) or (122),

[Formula 3]

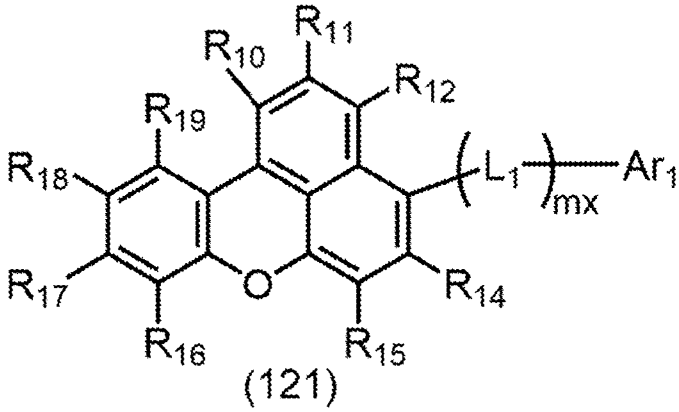

(121)

[Formula 4]

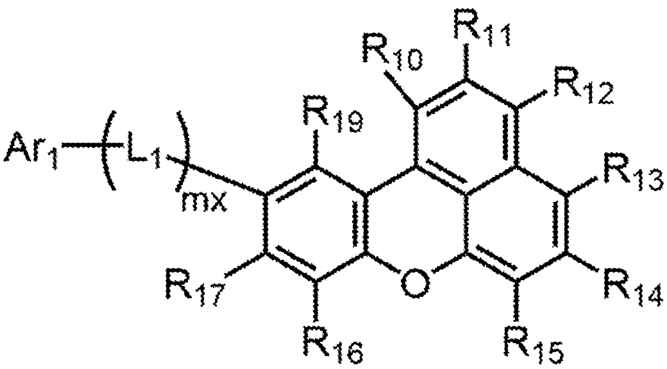

(122)

where: $R_{10}$ to $R_{19}$ each independently represent the same as $R_{10}$ to $R_{19}$ in the formula (1); and Ar1, $L_1$, and mx respectively represent the same as $Ar_1$, $L_1$, and mx in the formula (11).

**6.** The compound according to any one of claims 1 to 5, wherein $R_{10}$ to $R_{19}$ not being a group represented by the formula (11) are each independently a hydrogen atom, a substituted or unsubstituted alkyl group having 1 to 50 carbon atoms, a substituted or unsubstituted cycloalkyl group having 3 to 50 ring carbon atoms, or a substituted or unsubstituted aryl group having 6 to 50 ring carbon atoms.

**7.** The compound according to any one of claims 1 to 6, wherein $Ar_1$ is an aryl group in which at least four substituted or unsubstituted benzene rings are fused.

**8.** The compound according to claim 1, wherein the compound comprising at least one group represented by the formula (11) and a single benz[de]anthracene derivative skeleton represented by the formula (1000) in a molecule is represented by a formula (123), (124), (125), or (126) below,

[Formula 5]

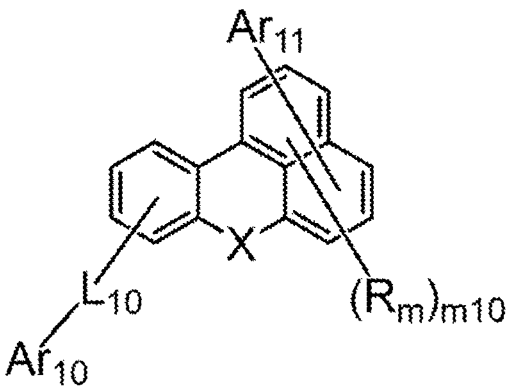

(123)

[Formula 6]

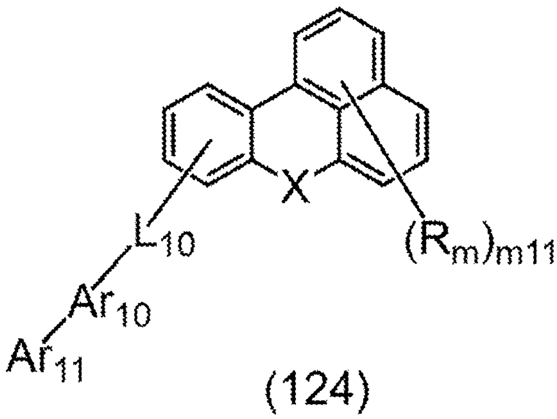

(124)

[Formula 7]

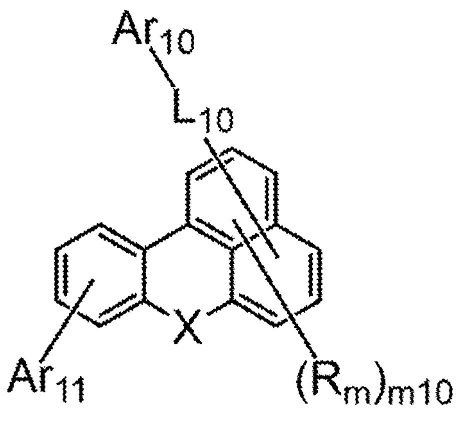

(125)

[Formula 8]

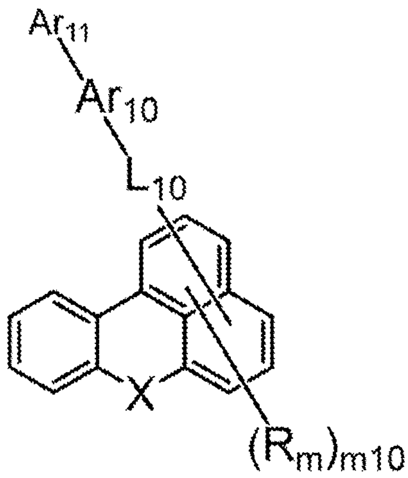

(126)

where: X is an oxygen atom, a sulfur atom, $C(R_{2001})(R_{2002})$, or $Si(R_{2003})(R_{2004})$;

$R_{2001}$ to $R_{2004}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms;

$L_{10}$ is a substituted or unsubstituted arylene group having 6 to 15 ring carbon atoms, or a substituted or unsubstituted divalent heterocyclic group having 5 to 15 ring atoms;

$Ar_{10}$ is an aryl group in which at least four substituted or unsubstituted benzene rings are fused;

$Ar_{11}$ is a substituted or unsubstituted aryl group having 6 to 14 ring carbon atoms;

$Ar_{11}$ is not a substituted or unsubstituted anthryl group;

m10 is 5;

m11 is 6;

Rm is each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by $-C(=O)R_{801}$, a group represented by $-COOR_{802}$, a halogen atom, a nitro group, a substituted or unsubstituted aryl group having 6 to 50 ring carbon atoms, or a substituted or unsubstituted heterocyclic group having 5 to 50 ring atoms;

a plurality of Rm are not mutually bonded;

$R_{901}$, $R_{902}$, $R_{903}$, $R_{904}$, $R_{905}$, $R_{906}$, $R_{907}$, $R_{801}$, and $R_{302}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{901}$ are present, the plurality of $R_{901}$ are mutually the same or different;

when a plurality of $R_{902}$ are present, the plurality of $R_{902}$ are mutually the same or different;

when a plurality of $R_{903}$ are present, the plurality of $R_{903}$ are mutually the same or different;

when a plurality of $R_{904}$ are present, the plurality of $R_{904}$ are mutually the same or different;

when a plurality of $R_{905}$ are present, the plurality of $R_{905}$ are mutually the same or different;

when a plurality of $R_{906}$ are present, the plurality of $R_{906}$ are mutually the same or different;

when a plurality of $R_{907}$ are present, the plurality of $R_{907}$ are mutually the same or different;

when a plurality of $R_{801}$ are present, the plurality of $R_{801}$ are mutually the same or different; and

when a plurality of $R_{302}$ are present, the plurality of $R_{302}$ are mutually the same or different.

**9.** The compound according to claim 8, wherein a compound represented by the formula (123) is represented by a formula (1230) below, a compound represented by the formula (124) is represented by a formula (1240) below, a compound represented by the formula (125) is represented by a formula (1250) below, and a compound represented by the formula (126) is represented by a formula (1260) below,

[Formula 9]

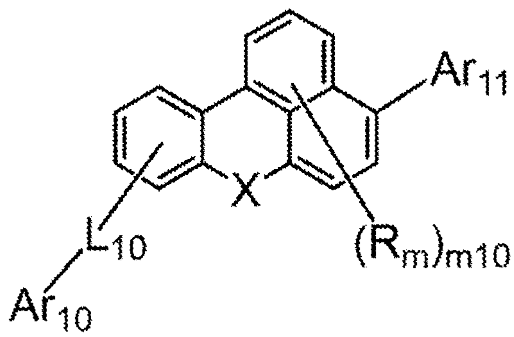

(1230)

[Formula 10]

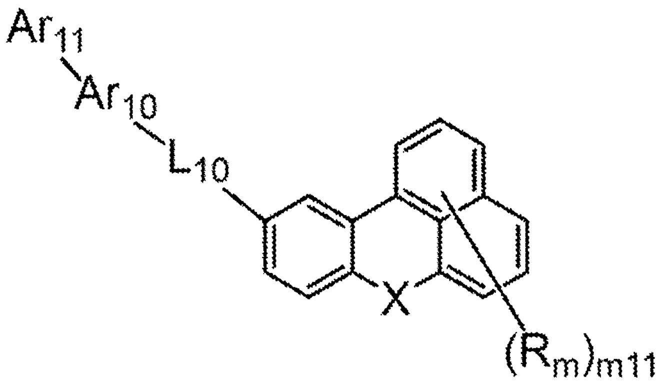

(1240)

[Formula 11]

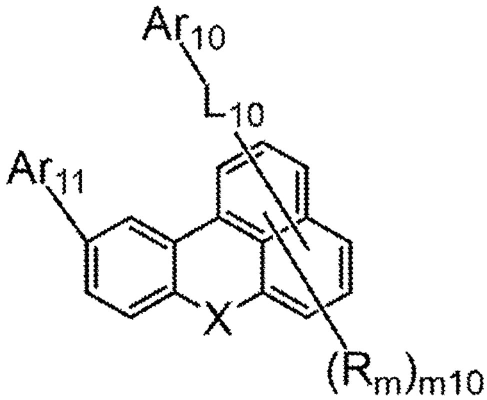

(1250)

[Formula 12]

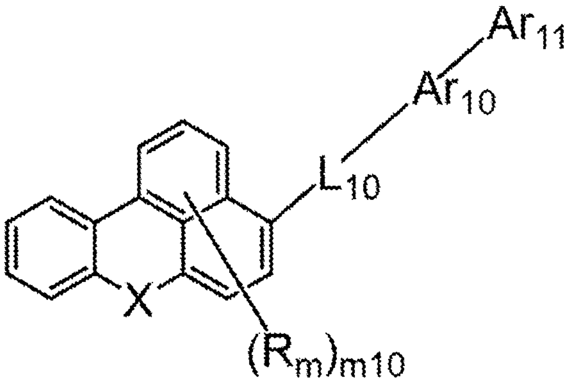

(1260)

where: X, $L_{10}$, $Ar_{10}$, $Ar_{11}$, m10, m11, and Rm each independently represent the same as $L_{10}$, $Ar_{10}$, $Ar_{11}$, m10, m11, and Rm in the formulae (123), (124), (125), and (126).

**10.** The compound according to claim 8 or 9, wherein X is an oxygen atom.

**11.** A compound comprising: at least one group represented by a formula (110A) below; and a single benz[de]anthracene derivative skeleton represented by a formula (1000A) below in a molecule,

[Formula 13]

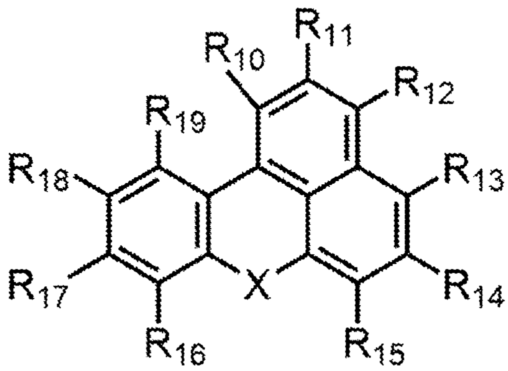

(1000A)

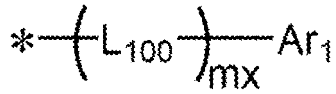

(110A)

where in the formula (1000A):

X is an oxygen atom, a sulfur atom, $C(R_{2001})(R_{2002})$, or $Si(R_{2003})(R_{2004})$;
$R_{2001}$ to $R_{2004}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms;
at least one combination of adjacent two or more of $R_{10}$ to $R_{19}$ are mutually bonded to form a substituted or unsubstituted monocyclic ring, mutually bonded to form a substituted or unsubstituted fused ring, or not mutually bonded;
$R_{10}$ to $R_{19}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by - $S-(R_{905})$, a group represented by $-N(R_{906})(R_{907})$, a substituted or unsubstituted aralkyl group having 7 to 50 carbon atoms, a group represented by $-C(=O)R_{801}$, a group represented by $-COOR_{802}$, a halogen atom, a nitro group, a substituted or unsubstituted aryl group having 6 to 50 ring carbon atoms, a substituted or unsubstituted heterocyclic group having 5 to 50 ring atoms, or a group represented by the formula (110A);
at least one of $R_{13}$ or $R_{18}$ is a group represented by the formula (110A);
when a plurality of groups represented by the formula (110A) are present, the plurality of groups represented by the formula (110A) are mutually the same or different;
$L_{100}$ is a single bond, a substituted or unsubstituted arylene group having 6 to 50 ring carbon atoms, or a substituted or unsubstituted divalent heterocyclic group having 5 to 50 ring atoms;
mx is 1, 2, or 3;
when two or more $L_{100}$ are present, the two or more $L_{100}$ are mutually the same or different;
$Ar_1$ is a substituted or unsubstituted aryl group including four or more rings;
when two or more $Ar_1$ are present, the two or more $Ar_1$ are mutually the same or different;
* in the formula (110A) represents a bonding position;
in a compound represented by the formula (1000A), $R_{901}$, $R_{902}$, $R_{903}$, $R_{904}$, $R_{905}$, $R_{906}$, $R_{907}$, $R_{801}$, and $R_{302}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;
when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;

when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{801}$ are present, the plurality of $R_{801}$ are mutually the same or different; and
when a plurality of $R_{302}$ are present, the plurality of $R_{302}$ are mutually the same or different.

**12.** The compound according to claim 11, wherein at least one combination of a combination of $R_{10}$ and $R_{11}$, a combination of $R_{11}$ and $R_{12}$, a combination of $R_{12}$ and $R_{13}$, a combination of $R_{13}$ and $R_{14}$, a combination of $R_{14}$ and $R_{15}$, a combination of $R_{16}$ and $R_{17}$, a combination of $R_{17}$ and $R_{18}$, a combination of $R_{18}$ and $R_{19}$, or a combination of $R_{19}$ and $R_{10}$ are mutually bonded to form a substituted or unsubstituted monocyclic ring, or mutually bonded to form a substituted or unsubstituted fused ring.

**13.** The compound according to claim 12, wherein one combination of the combination of $R_{13}$ and $R_{14}$, the combination of $R_{16}$ and $R_{17}$, and the combination of $R_{19}$ and $R_{10}$ are mutually bonded to form a substituted or unsubstituted benzene ring.

**14.** The compound according to claim 11, wherein the compound comprises: at least one group represented by the formula (110A); and a single benzoxanthene ring represented by a formula (100A) below in a molecule,

[Formula 14]

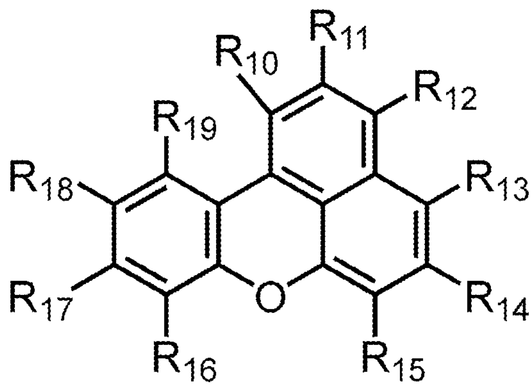

(100A)

where: $R_{10}$ to $R_{19}$ each independently represent the same as $R_{10}$ to $R_{19}$ in the formula (1000A);
none of combinations of adjacent two or more of $R_{10}$ to $R_{19}$ are mutually bonded; and
$Ar_1$, $L_1$, and mx respectively represent the same as $Ar_1$, $L_1$, and mx in the formula (110A).

**15.** The compound according to claim 14, wherein the compound comprising at least one group represented by the formula (110A) and a single benzoxanthene ring represented by the formula (100A) below in a molecule is represented by a formula (121A) or (122A),

[Formula 15]

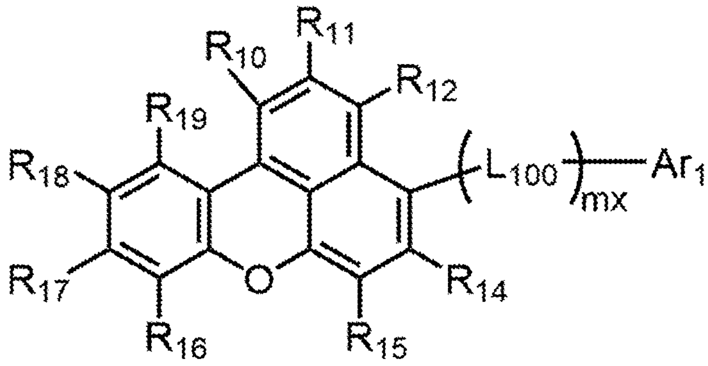

(121A)

[Formula 16]

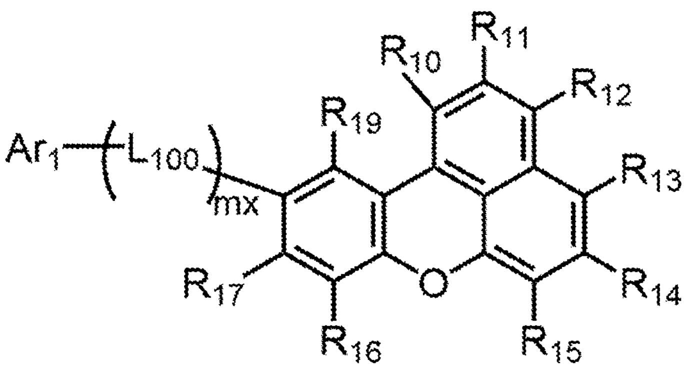

(122A)

where: $R_{10}$ to $R_{19}$ each independently represent the same as $R_{10}$ to $R_{19}$ in the formula (100A), and $Ar_1$, $L_{100}$, and mx respectively represent the same as $Ar_1$, $L_{100}$, and mx in the formula (110A).

**16.** The compound according to any one of claims 11 to 15, wherein $R_{10}$ to $R_{19}$ not being a group represented by the formula (110A) are each independently a hydrogen atom, a substituted or unsubstituted alkyl group having 1 to 50 carbon atoms, a substituted or unsubstituted cycloalkyl group having 3 to 50 ring carbon atoms, or a substituted or unsubstituted aryl group having 6 to 50 ring carbon atoms.

**17.** The compound according to any one of claims 1 to 16, wherein a group represented by $-(L_1)_{mx}-$, a group represented by $-L_{10}-$, or a group represented by $-(L_{100})_{mx}-$ is a group represented by one of formulae (111) to (120) below,

[Formula 17]

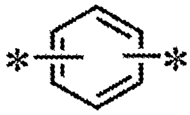

(111)

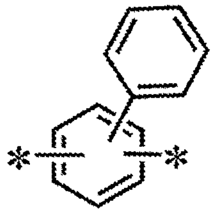

(112)

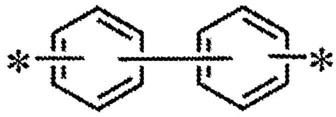

(113)

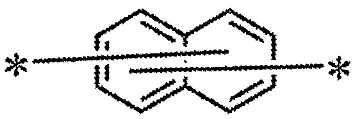

(114)

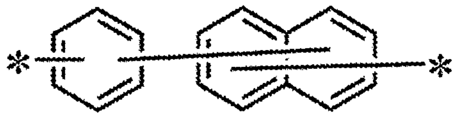

(115)

[Formula 18]

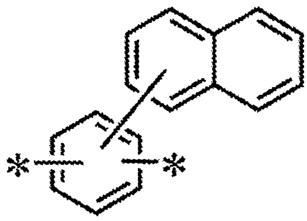

(116)

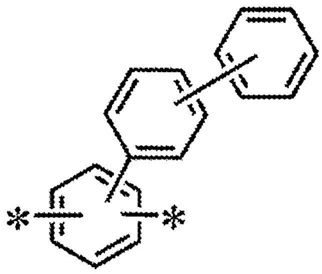

(117)

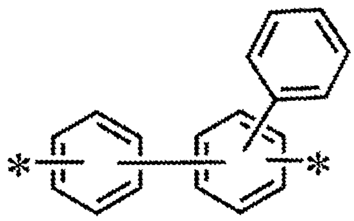

(118)

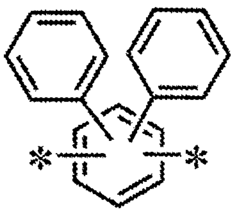

(119)

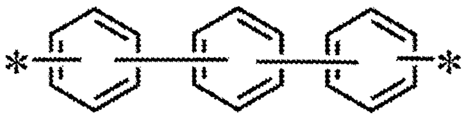

(120)

* in the formulae (111) to (120) represents a bonding position.

**18.** The compound according to any one of claims 11 to 17, wherein $Ar_1$ is an aryl group in which at least four substituted or unsubstituted benzene rings are fused.

**19.** The compound according to any one of claims 1 to 18, wherein $Ar_1$ or $Ar_{10}$ is a group represented by a formula (1100), (1200), (1300), (1400), (1500), (1600), (1700), or (1800) below,

[Formula 19]

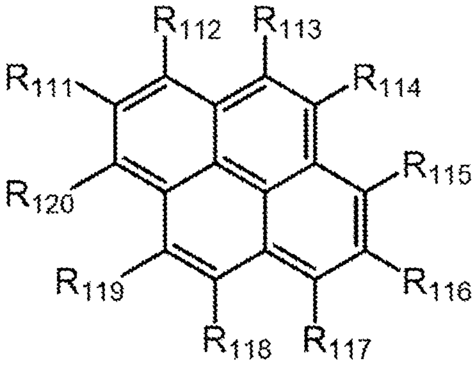

(1100)

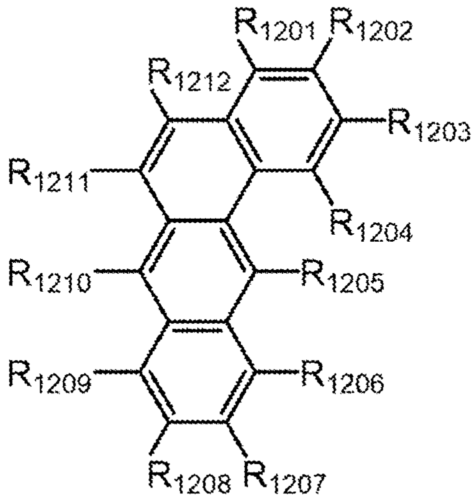

(1200)

[Formula 20]

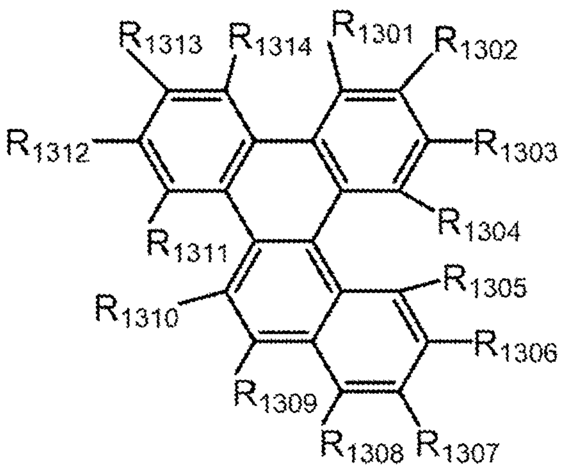

(1300)

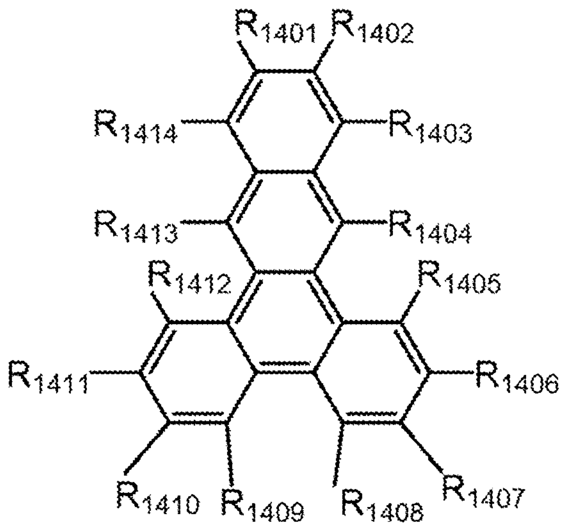

(1400)

[Formula 21]

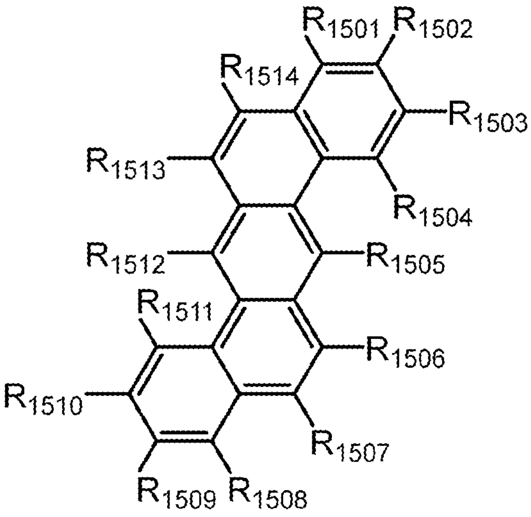

(1500)

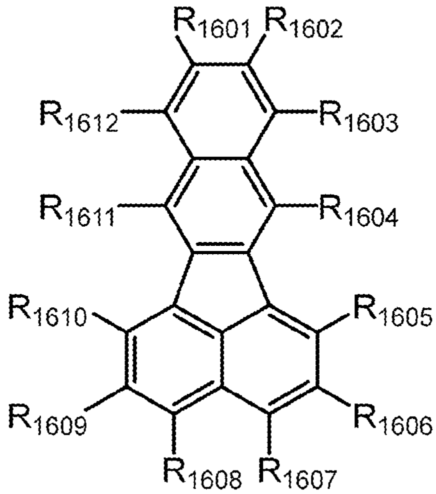

(1600)

[Formula 22]

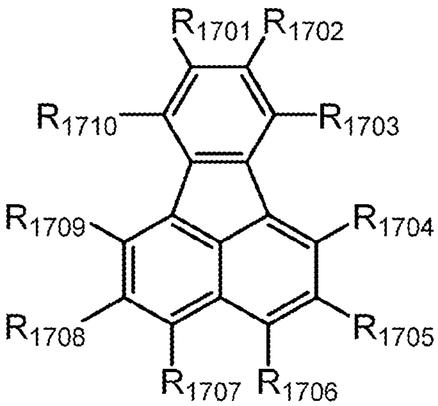

(1700)

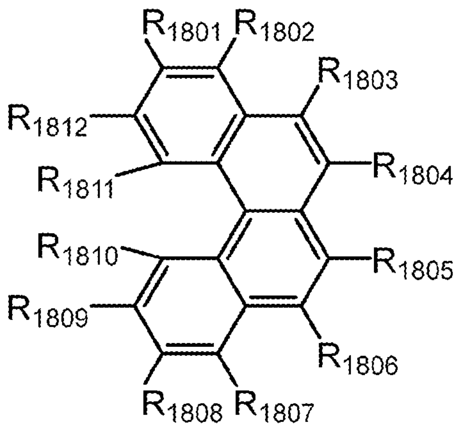

(1800)

where: in the formula (1100), one of $R_{111}$ to $R_{120}$ is a bond;
in the formula (1200), one of $R_{1201}$ to $R_{1212}$ is a bond;
in the formula (1300), one of $R_{1301}$ to $R_{1314}$ is a bond;
in the formula (1400), one of $R_{1401}$ to $R_{1414}$ is a bond;
in the formula (1500), one of $R_{1501}$ to $R_{1514}$ is a bond;
in the formula (1600), one of $R_{1601}$ to $R_{1612}$ is a bond;
in the formula (1700), one of $R_{1701}$ to $R_{1710}$ is a bond;
in the formula (1800), one of $R_{1801}$ to $R_{1812}$ is a bond;
$R_{111}$ to $R_{120}$ not being a bond, $R_{1201}$ to $R_{1212}$ not being a bond, $R_{1301}$ to $R_{1314}$ not being a bond, $R_{1401}$ to $R_{1414}$ not being a bond, $R_{1501}$ to $R_{1514}$ not being a bond, $R_{1601}$ to $R_{1612}$ not being a bond, $R_{1701}$ to $R_{1710}$ not being a bond, and $R_{1801}$ to $R_{1812}$ not being a bond are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having

3 to 50 ring carbon atoms, a group represented by -Si($R_{901}$)($R_{902}$)($R_{903}$), a group represented by -O-($R_{904}$), a group represented by -S-($R_{905}$), a group represented by -N($R_{906}$)($R_{907}$), a substituted or unsubstituted aralkyl group having 7 to 50 carbon atoms, a group represented by -C(=O)$R_{801}$, a group represented by -COO$R_{802}$, a halogen atom, a nitro group, a substituted or unsubstituted aryl group having 6 to 50 ring carbon atoms, or a substituted or unsubstituted heterocyclic group having 5 to 50 ring atoms.

**20.** The compound according to claim 19, wherein $R_{111}$ to $R_{120}$ not being a bond, $R_{1201}$ to $R_{1212}$ not being a bond, $R_{1301}$ to $R_{1314}$ not being a bond, $R_{1401}$ to $R_{1414}$ not being a bond, $R_{1501}$ to $R_{1514}$ not being a bond, $R_{1601}$ to $R_{1612}$ not being a bond, $R_{1701}$ to $R_{1710}$ not being a bond, and $R_{1801}$ to $R_{1812}$ not being a bond are each independently a hydrogen atom, a substituted or unsubstituted alkyl group having 1 to 50 carbon atoms, a substituted or unsubstituted cycloalkyl group having 3 to 50 ring carbon atoms, or a substituted or unsubstituted aryl group having 6 to 50 ring carbon atoms.

**21.** The compound according to any one of claims 1 to 20, wherein all of the "substituted or unsubstituted" groups are "unsubstituted" groups.

**22.** An organic electroluminescence device comprising:

an anode;
a cathode; and
at least one organic layer disposed between the anode and the cathode, wherein
the at least one organic layer comprises an emitting layer, and
at least one of the at least one organic layer comprises a compound according to any one of claims 1 to 21.

**23.** The organic electroluminescence device according to claim 22, wherein the emitting layer comprises the compound.

**24.** An organic electroluminescence device comprising:

an anode;
a cathode; and
at least one emitting layer disposed between the anode and the cathode, wherein
the at least one emitting layer comprises a first emitting layer and a second emitting layer,
the first emitting layer comprises a first compound, and
the first compound is a compound represented by a formula (1000B) below and comprising at least one group represented by a formula (110) below,

[Formula 23]

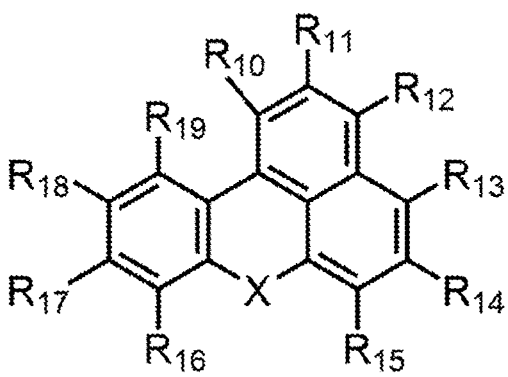

(1000B)

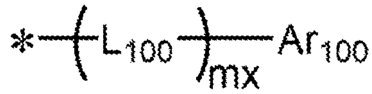

(110)

where: in the formula (1000B):

X is an oxygen atom, a sulfur atom, C($R_{2001}$)($R_{2002}$), or Si($R_{2003}$)($R_{2004}$);

$R_{2001}$ to $R_{2004}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms;

at least one combination of adjacent two or more of $R_{10}$ to $R_{19}$ are mutually bonded to form a substituted or unsubstituted monocyclic ring, mutually bonded to form a substituted or unsubstituted fused ring, or not mutually bonded;

$R_{10}$ to $R_{19}$ forming neither the substituted or unsubstituted monocyclic ring nor the substituted or unsubstituted fused ring are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by - $S-(R_{905})$, a group represented by $-N(R_{906})(R_{907})$, a substituted or unsubstituted aralkyl group having 7 to 50 carbon atoms, a group represented by $-C(=O)R_{801}$, a group represented by $-COOR_{802}$, a halogen atom, a nitro group, a substituted or unsubstituted aryl group having 6 to 50 ring carbon atoms, a substituted or unsubstituted heterocyclic group having 5 to 50 ring atoms, or a group represented by the formula (110);

at least one of $R_{10}$ to $R_{19}$ is a group represented by the formula (110);

when a plurality of groups represented by the formula (110) are present, the plurality of groups represented by the formula (110) are mutually the same or different;

$L_{100}$ is a single bond, a substituted or unsubstituted arylene group having 6 to 50 ring carbon atoms, or a substituted or unsubstituted divalent heterocyclic group having 5 to 50 ring atoms;

mx is 1, 2, or 3;

when two or more $L_{100}$ are present, the two or more $L_{100}$ are mutually the same or different;

$Ar_{100}$ is a substituted or unsubstituted aryl group including three or more rings or a substituted or unsubstituted heterocyclic group including two or more aromatic rings and one or more heterocyclic rings;

$Ar_{100}$ does not include an anthracene ring;

when two or more $Ar_{100}$ are present, the two or more $Ar_{100}$ are mutually the same or different; and

* in the formula (110) represents a bonding position;

in the first compound represented by the formula (1000B), $R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{901}$ are present, the plurality of $R_{901}$ are mutually the same or different;

when a plurality of $R_{902}$ are present, the plurality of $R_{902}$ are mutually the same or different;

when a plurality of $R_{903}$ are present, the plurality of $R_{903}$ are mutually the same or different;

when a plurality of $R_{904}$ are present, the plurality of $R_{904}$ are mutually the same or different;

when a plurality of $R_{905}$ are present, the plurality of $R_{905}$ are mutually the same or different;

when a plurality of $R_{906}$ are present, the plurality of $R_{906}$ are mutually the same or different;

when a plurality of $R_{907}$ are present, the plurality of $R_{907}$ are mutually the same or different;

when a plurality of $R_{801}$ are present, the plurality of $R_{801}$ are mutually the same or different; and

when a plurality of $R_{802}$ are present, the plurality of $R_{802}$ are mutually the same or different.

**25.** The organic electroluminescence device according to claim 24, wherein at least one combination of a combination of $R_{10}$ and $R_{11}$, a combination of $R_{11}$ and $R_{12}$, a combination of $R_{12}$ and $R_{13}$, a combination of $R_{13}$ and $R_{14}$, a combination of $R_{14}$ and $R_{15}$, a combination of $R_{16}$ and $R_{17}$, a combination of $R_{17}$ and $R_{18}$, a combination of $R_{18}$ and $R_{19}$, or a combination of $R_{19}$ and $R_{10}$ are mutually bonded to form a substituted or unsubstituted monocyclic ring, or mutually bonded to form a substituted or unsubstituted fused ring.

**26.** The organic electroluminescence device according to claim 25, wherein one combination of the combination of $R_{13}$ and $R_{14}$, the combination of $R_{16}$ and $R_{17}$, and the combination of $R_{19}$ and $R_{10}$ are mutually bonded to form a substituted or unsubstituted benzene ring.

**27.** The organic electroluminescence device according to claim 24, wherein the first compound is a compound comprising at least one group represented by the formula (110) and represented by a formula (100) below,

[Formula 24]

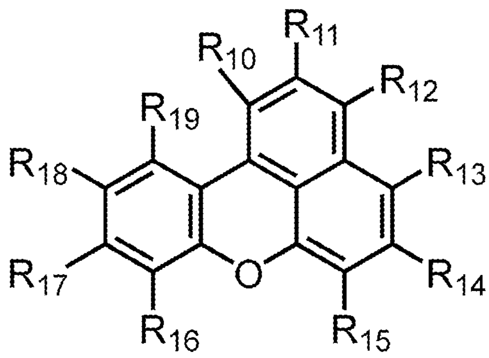

(100)

where: $R_{10}$ to $R_{19}$ each independently represent the same as $R_{10}$ to $R_{19}$ in the formula (1000B); none of combinations of adjacent two or more of $R_{10}$ to $R_{19}$ are mutually bonded; and $Ar_{100}$, $L_{100}$, and mx respectively represent the same as $Ar_{100}$, $L_{100}$, and mx in the formula (110).

**28.** The organic electroluminescence device according to any one of claims 24 to 27, wherein

the first emitting layer is disposed between the anode and the cathode, and
the second emitting layer is disposed between the first emitting layer and the cathode.

**29.** The organic electroluminescence device according to any one of claims 24 to 28, wherein the first emitting layer is in direct contact with the second emitting layer.

**30.** The organic electroluminescence device according to any one of claims 24 to 29, wherein the first compound is represented by a formula (101) or (102) below,

[Formula 25]

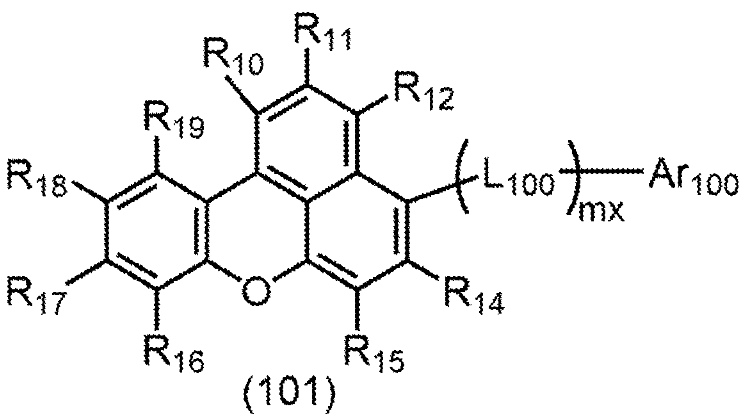

(101)

[Formula 26]

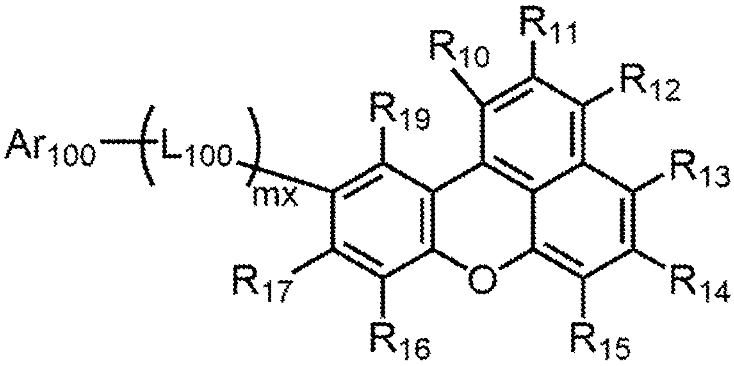

(102)

where: $R_{10}$ to $R_{19}$ each independently represent the same as $R_{10}$ to $R_{19}$ in the formula (1000B); none of combinations of adjacent two or more of $R_{10}$ to $R_{19}$ are mutually bonded; and $Ar_{100}$, $L_{100}$, and mx respectively represent the same as $Ar_{100}$, $L_{100}$, and mx in the formula (110).

**31.** The organic electroluminescence device according to any one of claims 24 to 30, wherein $R_{10}$ to $R_{19}$ not being a group represented by the formula (110) are each independently a hydrogen atom, a substituted or unsubstituted alkyl group having 1 to 50 carbon atoms, a substituted or unsubstituted cycloalkyl group having 3 to 50 ring carbon atoms, or a substituted or unsubstituted aryl group having 6 to 50 ring carbon atoms.

**32.** The organic electroluminescence device according to claim 24, wherein the compound represented by the formula (1000B) and comprising at least one group represented by the formula (110) is represented by a formula (123), (124), (125), or (126) below,

[Formula 27]

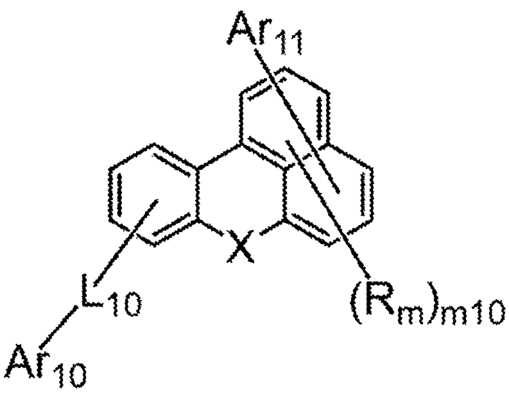

(123)

[Formula 28]

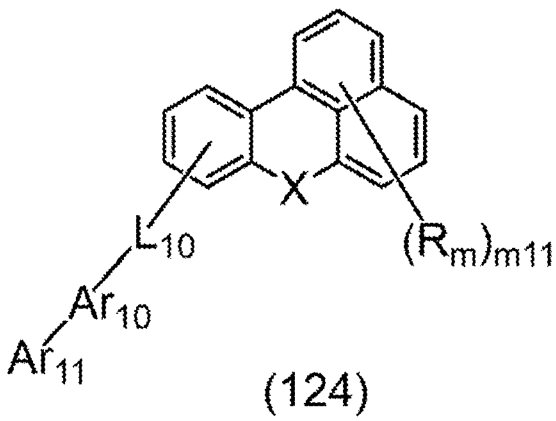

(124)

[Formula 29]

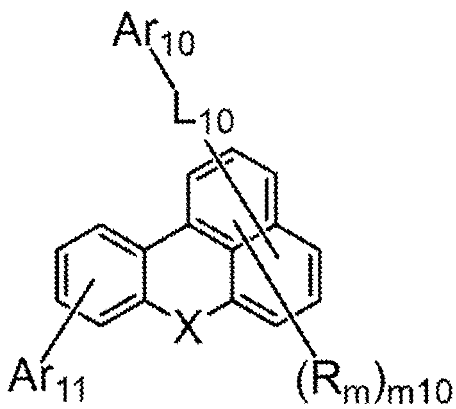

(125)

[Formula 30]

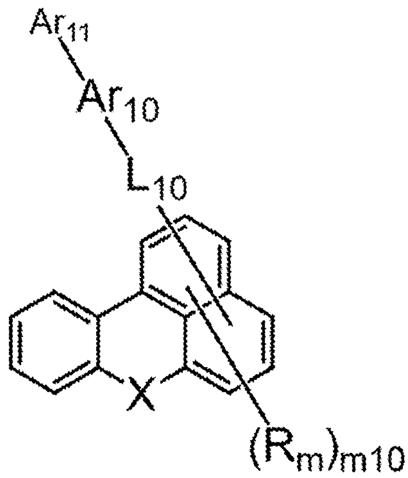

(126)

where: X is an oxygen atom, a sulfur atom, $C(R_{2001})(R_{2002})$, or $Si(R_{2003})(R_{2004})$;

$R_{2001}$ to $R_{2004}$ are each independently a hydrogen atom, a substituted or unsubstituted alkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a halogen atom, a cyano group, a nitro group, a substituted or unsubstituted aryl group having 6 to 50 ring carbon atoms, or a substituted or unsubstituted heterocyclic group having 5 to 50 ring atoms;

$L_{10}$ is a substituted or unsubstituted arylene group having 6 to 15 ring carbon atoms, or a substituted or unsubstituted divalent heterocyclic group having 5 to 15 ring atoms;

$Ar_{10}$ is an aryl group in which at least four substituted or unsubstituted benzene rings are fused;

$Ar_{11}$ is a substituted or unsubstituted aryl group having 6 to 14 ring carbon atoms;

$Ar_{11}$ is not a substituted or unsubstituted anthryl group;

m10 is 5;

m11 is 6;

Rm is each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl group having 7 to 50 carbon atoms, a group represented by $-C(=O)R_{801}$, a group represented by $-COOR_{802}$, a halogen atom, a nitro group, a substituted or unsubstituted aryl group having 6 to 50 ring carbon atoms, or a substituted or unsubstituted heterocyclic group having 5 to 50 ring atoms;

a plurality of Rm are not mutually bonded;

$R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{901}$ are present, the plurality of $R_{901}$ are mutually the same or different;

when a plurality of $R_{902}$ are present, the plurality of $R_{902}$ are mutually the same or different;

when a plurality of $R_{903}$ are present, the plurality of $R_{903}$ are mutually the same or different;

when a plurality of $R_{904}$ are present, the plurality of $R_{904}$ are mutually the same or different;

when a plurality of $R_{905}$ are present, the plurality of $R_{905}$ are mutually the same or different;

when a plurality of $R_{906}$ are present, the plurality of $R_{906}$ are mutually the same or different;

when a plurality of $R_{907}$ are present, the plurality of $R_{907}$ are mutually the same or different;

when a plurality of $R_{801}$ are present, the plurality of $R_{801}$ are mutually the same or different; and

when a plurality of $R_{802}$ are present, the plurality of $R_{802}$ are mutually the same or different.

**33.** The organic electroluminescence device according to claim 32, wherein a compound represented by the formula (123) is represented by a formula (1230) below, a compound represented by the formula (124) is represented by a formula (1240) below, a compound represented by the formula (125) is represented by a formula (1250) below, and a compound represented by the formula (126) is represented by a formula (1260) below,

[Formula 31]

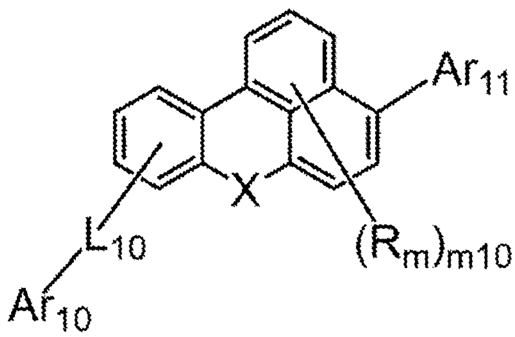

(1230)

[Formula 32]

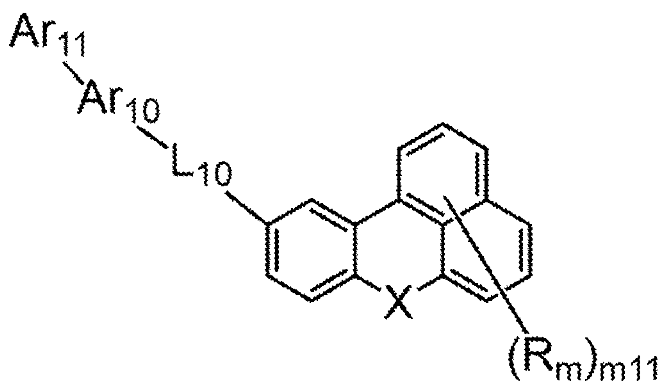

(1240)

[Formula 33]

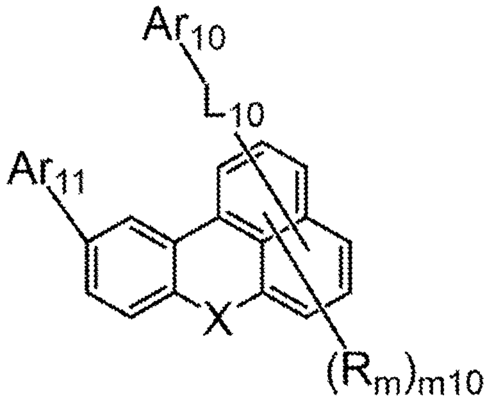

(1250)

[Formula 34]

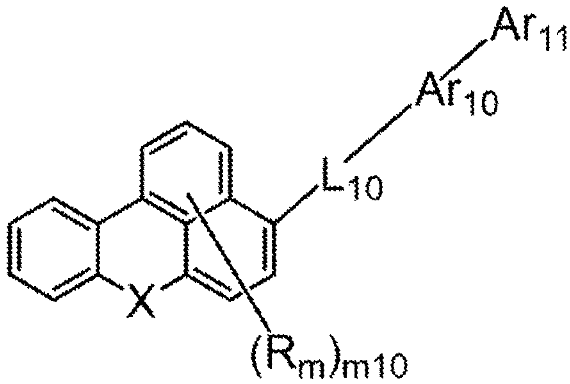

(1260)

where: X, $L_{10}$, $Ar_{10}$, $Ar_{11}$, m10, m11, and Rm each independently represent the same as $L_{10}$, $Ar_{10}$, $Ar_{11}$, m10, m11, and Rm in the formulae (123), (124), (125), and (126).

**34.** The organic electroluminescence device according to claim 32 or 33, wherein X is an oxygen atom.

**35.** The organic electroluminescence device according to any one of claims 24 to 31, wherein $Ar_{100}$ is a group represented by a formula (1100), (1200), (1300), (1400), (1500), (1600), (1700), or (1800),

[Formula 35]

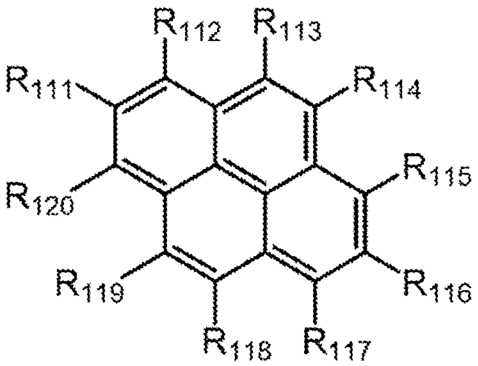

(1100)

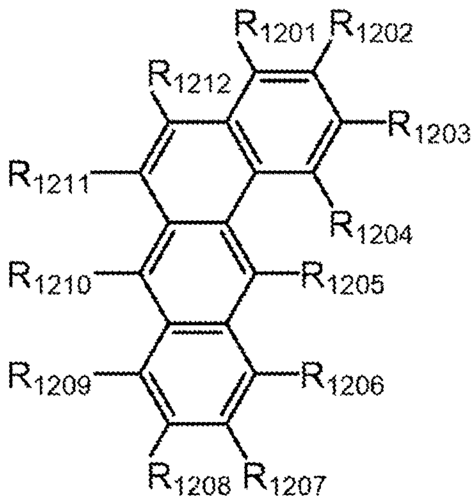

(1200)

[Formula 36]

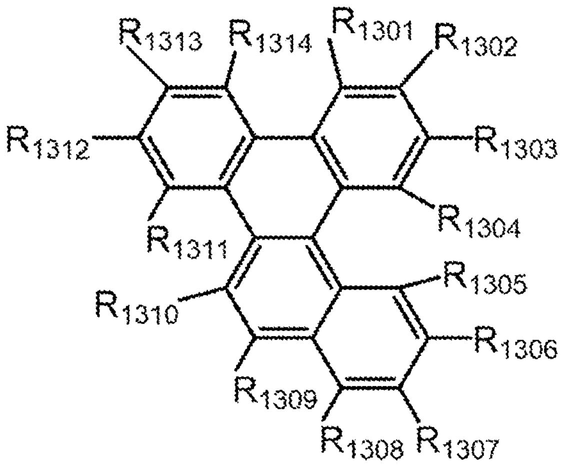

(1300)

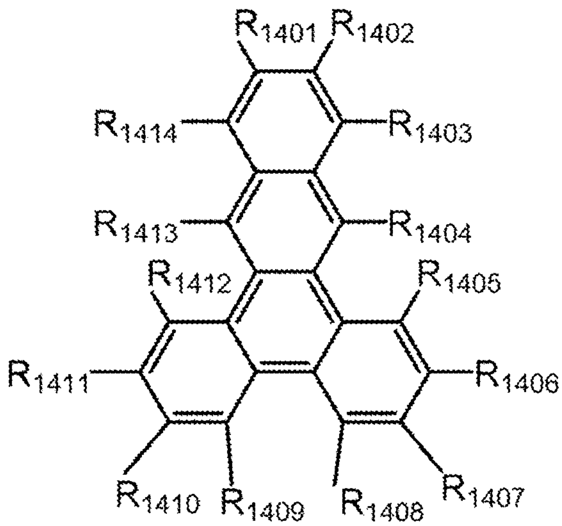

(1400)

[Formula 37]

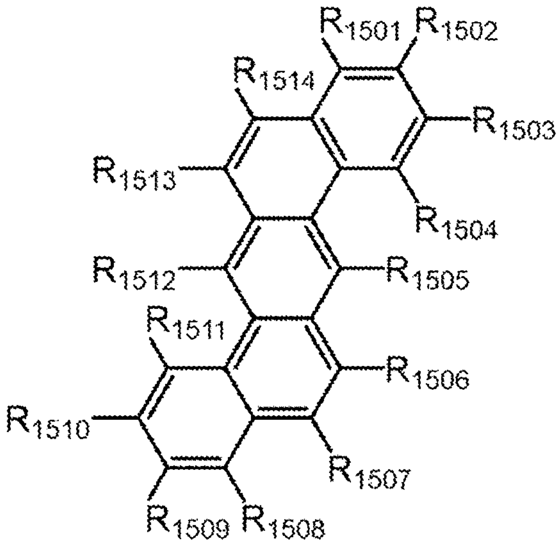

(1500)

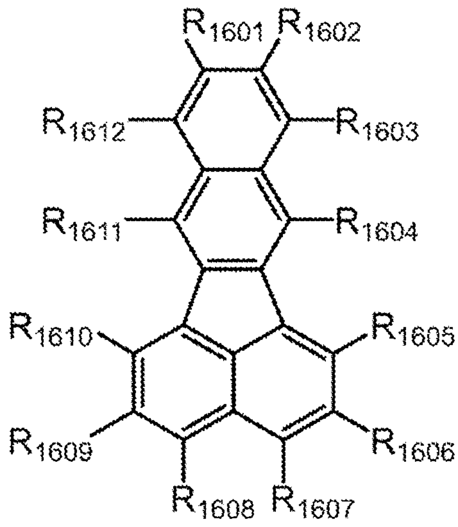

(1600)

[Formula 38]

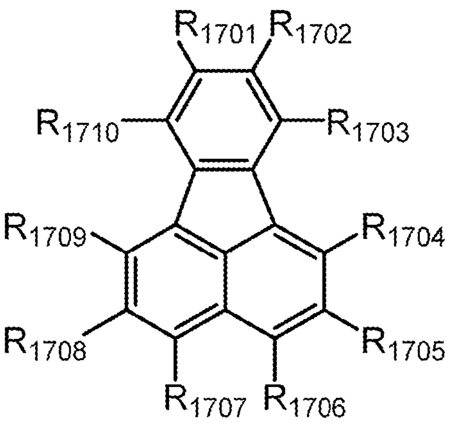

(1700)

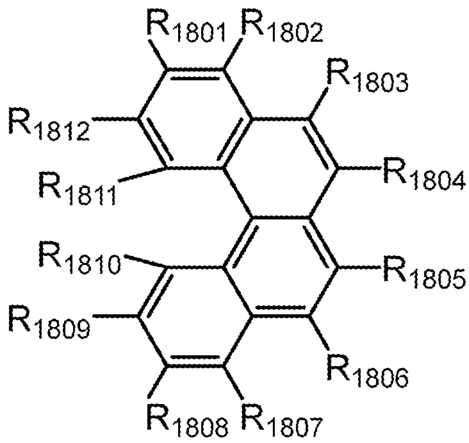

(1800)

where: in the formula (1100), one of $R_{111}$ to $R_{120}$ is a bond;
in the formula (1200), one of $R_{1201}$ to $R_{1212}$ is a bond;
in the formula (1300), one of $R_{1301}$ to $R_{1314}$ is a bond;
in the formula (1400), one of $R_{1401}$ to $R_{1414}$ is a bond;
in the formula (1500), one of $R_{1501}$ to $R_{1514}$ is a bond;
in the formula (1600), one of $R_{1601}$ to $R_{1612}$ is a bond;
in the formula (1700), one of $R_{1701}$ to $R_{1710}$ is a bond;
in the formula (1800), one of $R_{1801}$ to $R_{1812}$ is a bond;
$R_{111}$ to $R_{120}$ not being a bond, $R_{1201}$ to $R_{1212}$ not being a bond, $R_{1301}$ to $R_{1314}$ not being a bond, $R_{1401}$ to $R_{1414}$ not being a bond, $R_{1501}$ to $R_{1514}$ not being a bond, $R_{1601}$ to $R_{1612}$ not being a bond, $R_{1701}$ to $R_{1710}$ not being a bond, and $R_{1801}$ to $R_{1812}$ not being a bond are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by $-Si(R_{901})(R_{902})(R_{903})$, a group represented by $-O-(R_{904})$, a group represented by $-S-(R_{905})$, a group represented by $-N(R_{906})(R_{907})$, a substituted or unsubstituted aralkyl

group having 7 to 50 carbon atoms, a group represented by -C(=O)$R_{801}$, a group represented by -COO$R_{802}$, a halogen atom, a nitro group, a substituted or unsubstituted aryl group having 6 to 50 ring carbon atoms, or a substituted or unsubstituted heterocyclic group having 5 to 50 ring atoms.

**36.** The organic electroluminescence device according to any one of claims 24 to 35, wherein the second emitting layer comprises a second compound represented by a formula (2) below,

[Formula 39]

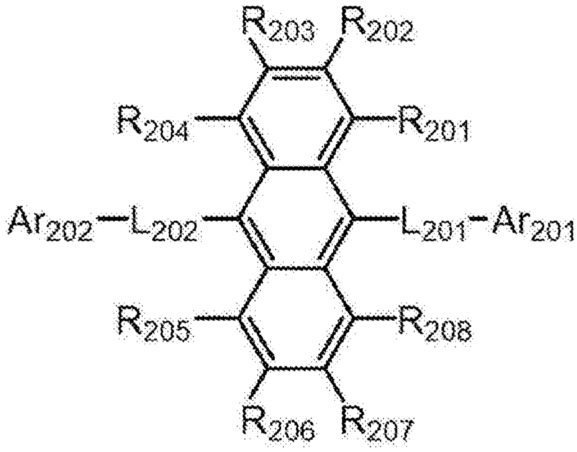

(2)

where: $R_{201}$ to $R_{208}$ are each independently a hydrogen atom, a substituted or unsubstituted alkyl group having 1 to 50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by -S-($R_{905}$), a group represented by -N($R_{906}$)($R_{907}$), a substituted or unsubstituted aralkyl group having 7 to 50 carbon atoms, a group represented by -C(=O)$R_{801}$, a group represented by -COO$R_{802}$, a halogen atom, a nitro group, a substituted or unsubstituted aryl group having 6 to 50 ring carbon atoms, or a substituted or unsubstituted heterocyclic group having 5 to 50 ring atoms;

$L_{201}$ and $L_{202}$ are each independently a single bond, a substituted or unsubstituted arylene group having 6 to 50 ring carbon atoms, or a substituted or unsubstituted divalent heterocyclic group having 5 to 50 ring atoms;

$Ar_{201}$ and $Ar_{202}$ are each independently a substituted or unsubstituted aryl group having 6 to 50 ring carbon atoms, or a substituted or unsubstituted heterocyclic group having 5 to 50 ring atoms;

in the second compound represented by the formula (2), $R_{901}$, $R_{902}$, $R_{903}$, $R_{904}$, $R_{905}$, $R_{906}$, $R_{907}$, $R_{801}$, and $R_{802}$ are each independently a hydrogen atom, a substituted or unsubstituted alkyl group having 1 to 50 carbon atoms, a substituted or unsubstituted cycloalkyl group having 3 to 50 ring carbon atoms, a substituted or unsubstituted aryl group having 6 to 50 ring carbon atoms, or a substituted or unsubstituted heterocyclic group having 5 to 50 ring atoms;

when a plurality of $R_{901}$ are present, the plurality of $R_{901}$ are mutually the same or different;
when a plurality of $R_{902}$ are present, the plurality of $R_{902}$ are mutually the same or different;
when a plurality of $R_{903}$ are present, the plurality of $R_{903}$ are mutually the same or different;
when a plurality of $R_{904}$ are present, the plurality of $R_{904}$ are mutually the same or different;
when a plurality of $R_{905}$ are present, the plurality of $R_{905}$ are mutually the same or different;
when a plurality of $R_{906}$ are present, the plurality of $R_{906}$ are mutually the same or different;
when a plurality of $R_{907}$ are present, the plurality of $R_{907}$ are mutually the same or different;
when a plurality of $R_{801}$ are present, the plurality of $R_{801}$ are mutually the same or different; and
when a plurality of $R_{802}$ are present, the plurality of $R_{802}$ are mutually the same or different.

**37.** The organic electroluminescence device according to claim 36, wherein

$R_{201}$ to $R_{208}$ are each independently a hydrogen atom, a substituted or unsubstituted alkyl group having 1 to

50 carbon atoms, a substituted or unsubstituted haloalkyl group having 1 to 50 carbon atoms, a substituted or unsubstituted alkenyl group having 2 to 50 carbon atoms, a substituted or unsubstituted alkynyl group having 2 to 50 carbon atoms, a substituted or unsubstituted cycloalkyl group having 3 to 50 ring carbon atoms, a group represented by -Si($R_{901}$)($R_{902}$)($R_{903}$), a group represented by -O-($R_{904}$), a group represented by -S-($R_{905}$), a group represented by -N($R_{906}$)($R_{907}$), a substituted or unsubstituted aralkyl group having 7 to 50 carbon atoms, a group represented by -C(=O)$R_{801}$, a group represented by -COO$R_{802}$, a halogen atom, or a nitro group;

$L_{201}$ and $L_{202}$ are each independently a single bond, a substituted or unsubstituted arylene group having 6 to 50 ring carbon atoms, or a substituted or unsubstituted divalent heterocyclic group having 5 to 50 ring atoms; and

$Ar_{201}$ and $Ar_{202}$ are each independently a substituted or unsubstituted aryl group having 6 to 50 ring carbon atoms, or a substituted or unsubstituted heterocyclic group having 5 to 50 ring atoms.

**38.** The organic electroluminescence device according to claim 36 or 37, wherein

$L_{201}$ and $L_{202}$ are each independently a single bond, or a substituted or unsubstituted arylene group having 6 to 50 ring carbon atoms; and

$Ar_{201}$ and $Ar_{202}$ are each independently a substituted or unsubstituted aryl group having 6 to 50 ring carbon atoms.

**39.** The organic electroluminescence device according to any one of claims 36 to 38, wherein $Ar_{201}$ and $Ar_{202}$ are each independently a phenyl group, a naphthyl group, a phenanthryl group, a biphenyl group, a terphenyl group, a diphenylfluorenyl group, a dimethylfluorenyl group, a benzodiphenylfluorenyl group, a benzodimethylfluorenyl group, a dibenzofuranyl group, a dibenzothienyl group, a benzoxanthenyl group, a naphthobenzofuranyl group, or a naphthobenzothienyl group.

**40.** The organic electroluminescence device according to any one of claims 36 to 39, wherein the second compound represented by the formula (2) is a compound represented by a formula (201), (202), (203), (204), (205), (206), (207), (208), or (209),

[Formula 40]

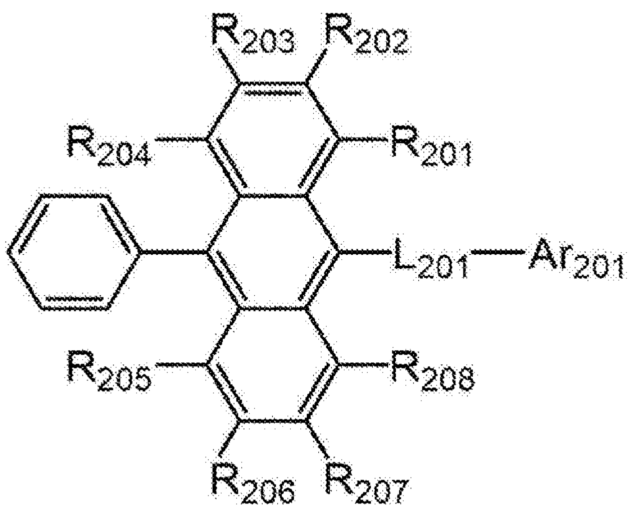

(201)

[Formula 41]

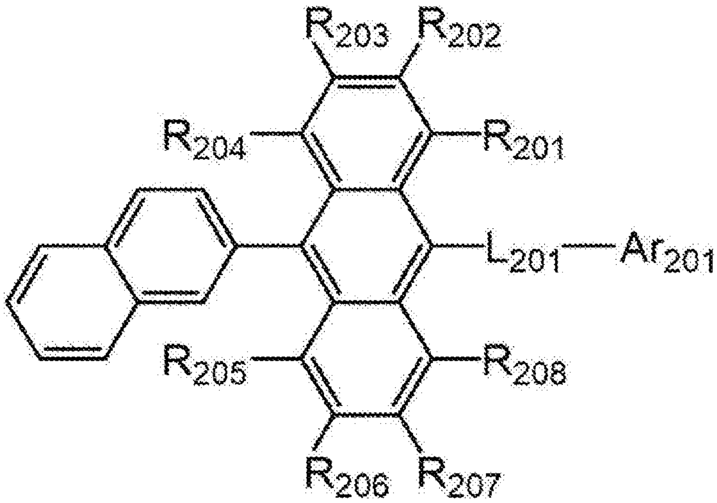

(202)

[Formula 42]

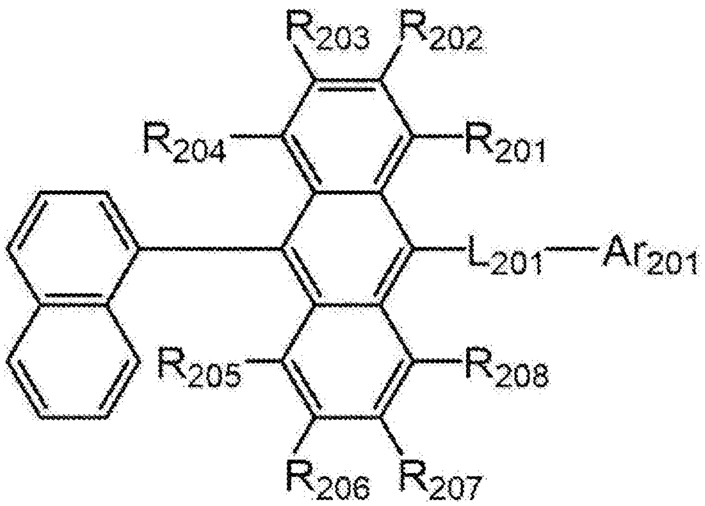

(203)

[Formula 43]

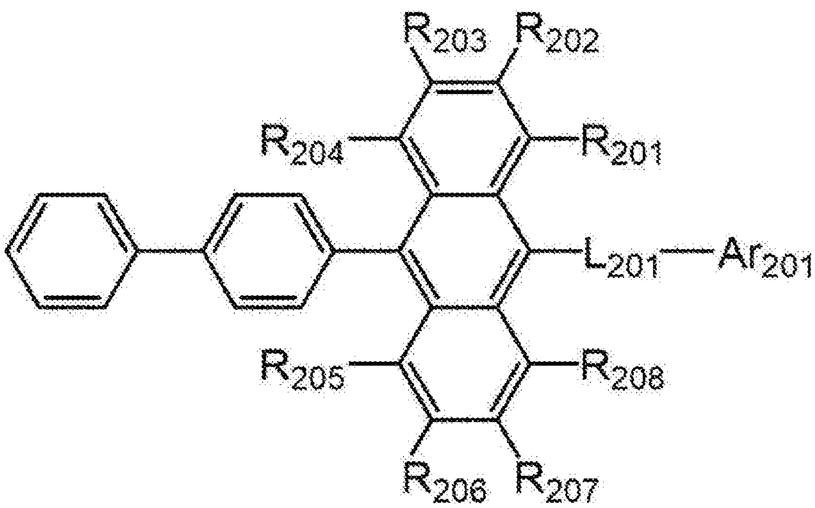

(204)

[Formula 44]

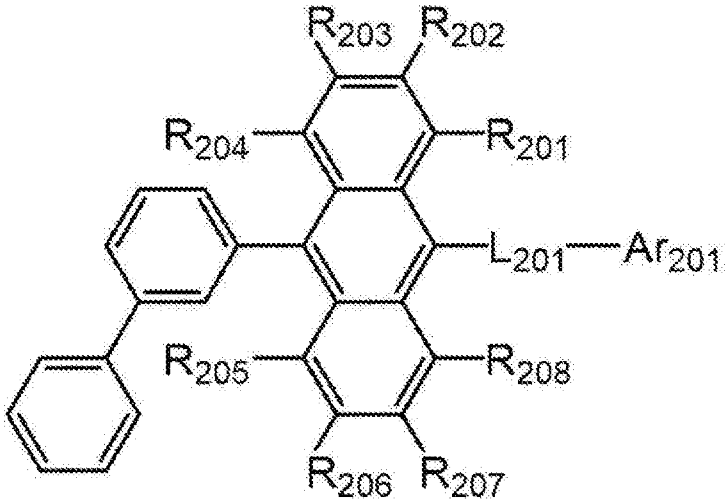

(205)

[Formula 45]

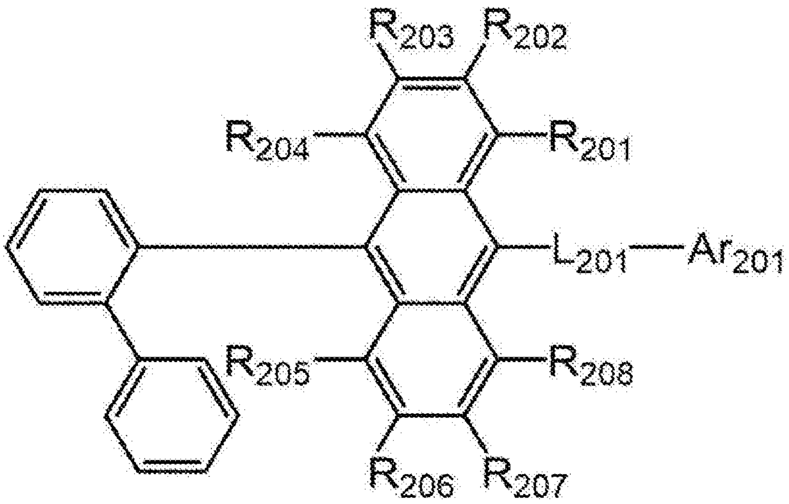

(206)

[Formula 46]

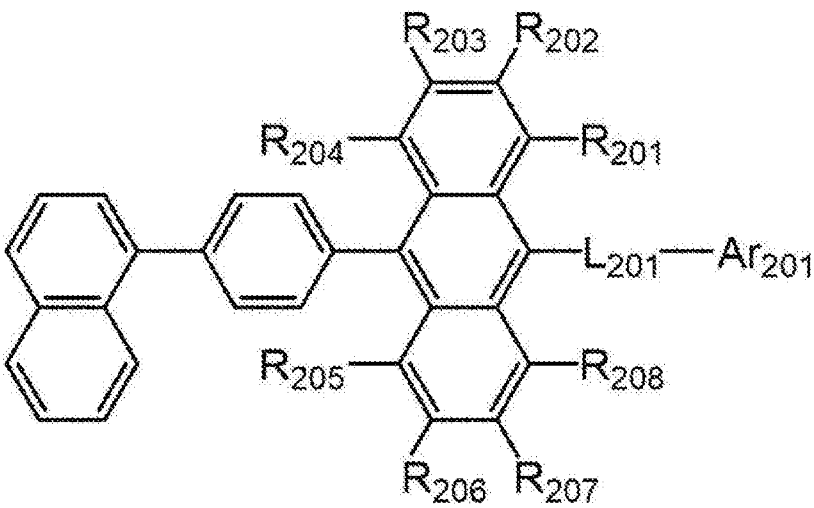

(207)

[Formula 47]

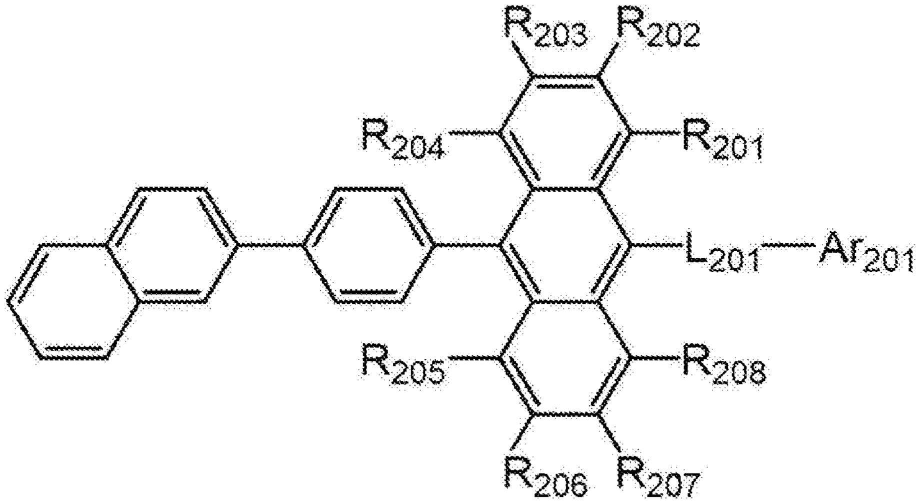

(208)

[Formula 48]

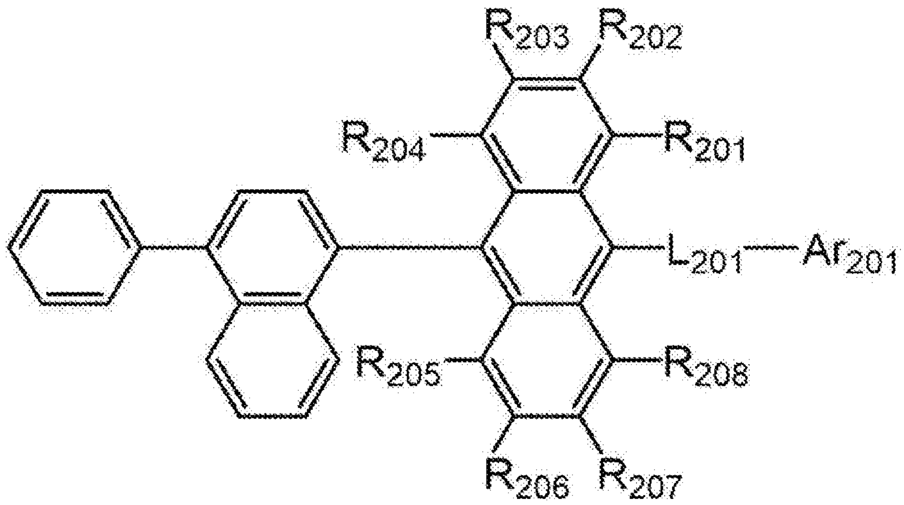

(209)

where: $L_{201}$ and $Ar_{201}$ represent the same as $L_{201}$ and $Ar_{201}$ in the formula (2); and $R_{201}$ to $R_{208}$ each independently represent the same as $R_{201}$ to $R_{208}$ in the formula (2).

**41.** The organic electroluminescence device according to any one of claims 24 to 40, further comprising: a hole transporting layer between the anode and the emitting layer.

**42.** The organic electroluminescence device according to any one of claims 24 to 41, further comprising: an electron transporting layer between the cathode and the emitting layer.

**43.** The organic electroluminescence device according to any one of claims 24 to 42, wherein

the first emitting layer further comprises a third compound that fluoresces, and
the third compound is a compound that emits light having a main peak wavelength in a range from 430 nm to 480 nm.

**44.** The organic electroluminescence device according to any one of claims 24 to 43, wherein

the second emitting layer further comprises a fourth compound that fluoresces, and

the fourth compound is a compound that emits light having a main peak wavelength in a range from 430 nm to 480 nm.

**45.** An electronic device comprising the organic electroluminescence device according to any one of claims 22 to 44.

FIG.1

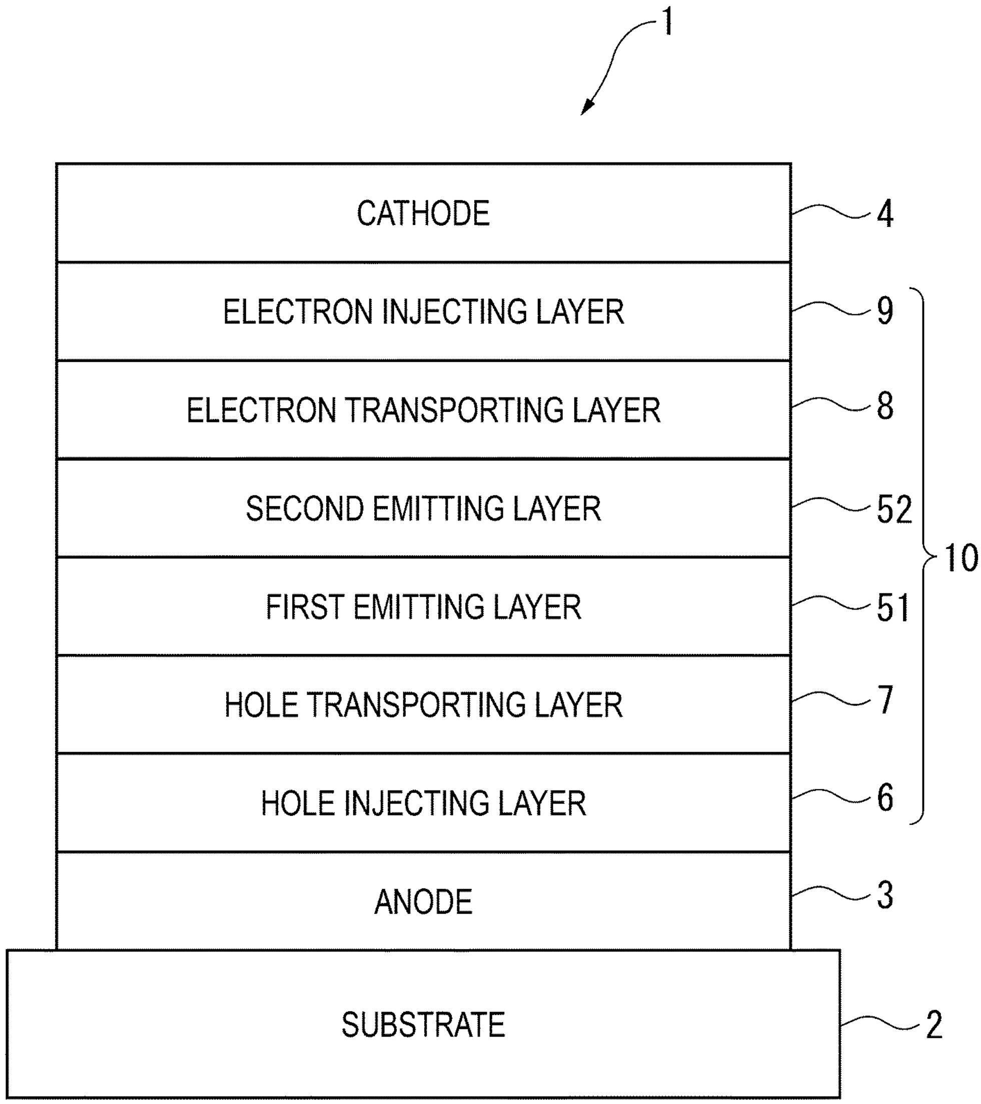
1
CATHODE
4
ELECTRON INJECTING LAYER
9
ELECTRON TRANSPORTING LAYER
8
SECOND EMITTING LAYER
52
10
FIRST EMITTING LAYER
51
HOLE TRANSPORTING LAYER
7
HOLE INJECTING LAYER
6
ANODE
3
SUBSTRATE
2

## INTERNATIONAL SEARCH REPORT

International application No.
**PCT/JP2021/036397**

**A. CLASSIFICATION OF SUBJECT MATTER**

***C07C 13/66***(2006.01)i; ***C07D 335/04***(2006.01)i; ***C09K 11/06***(2006.01)i; ***C07D 311/78***(2006.01)i; ***H05B 33/12***(2006.01)i; ***H01L 51/50***(2006.01)i

FI: C07D311/78 CSP; C07C13/66; C07D335/04; C09K11/06 610; C09K11/06 630; C09K11/06 650; C09K11/06 660; C09K11/06 690; H05B33/12 C; H05B33/14 B; H05B33/22 B; H05B33/22 D

According to International Patent Classification (IPC) or to both national classification and IPC

**B. FIELDS SEARCHED**

Minimum documentation searched (classification system followed by classification symbols)

C07C13/66; C07D335/04; C09K11/06; C07D311/78; H05B33/12; H01L51/50

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2014-009220 A (CANON INC) 20 January 2014 (2014-01-20)<br>claims, compounds A2, A3, A8, A13, A21, B1, B2, B4, C1, C5, D1, D5, D6, D9, D10, examples | 1-3, 7, 17-18, 21-23, 45 |
| X | KR 10-2019-0055686 A (NANJING TOPTO MATERIALS CO., LTD.) 23 May 2019 (2019-05-23)<br>claims, compounds 54, 99, examples | 1-2, 17, 21-23, 45 |
| X | CN 107827853 A (NANJING TOPTO SEMICONDUCTOR MATERIAL CO., LTD.) 23 March 2018 (2018-03-23)<br>claims, compound 106, examples | 1-2, 21-23, 45 |
| X | US 2015/0001479 A1 (SAMSUNG DISPLAY CO., LTD.) 01 January 2015 (2015-01-01)<br>claims, compounds 57, 58, 61-63, 66, examples | 1, 4-6, 17, 22-23, 45 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **23 December 2021** | **11 January 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No. **PCT/JP2021/036397**

**C. DOCUMENTS CONSIDERED TO BE RELEVANT**

| Category* | Citation of document, with indication, where appropriate, of the relevant passages | Relevant to claim No. |
|---|---|---|
| X | WO 2015/122711 A1 (DOOSAN CORPORATION) 20 August 2015 (2015-08-20) claims, examples 19, 35, table 1 | 1, 4-7, 21-23, 45 |
| Y | claims, examples 19, 35, table 1 | 8-10, 21-23, 45 |
| X | CN 103102299 A (JILIN OPTICAL AND ELECTRONIC MATERIALS CO., LTD.) 15 May 2013 (2013-05-15) claims, examples 5, 8, tables 1, 2 | 1, 6-7, 17-23, 45 |
| Y | claims, examples 5, 8, tables 1, 2 | 8-10, 17-23, 45 |
| X | JP 2013-528927 A (ROHM AND HAAS ELECTRONIC MATERIALS KOREA LTD) 11 July 2013 (2013-07-11) claims, compounds 21, 23, 25, 31, 33, 34, 53, etc., examples, definition of chemical formula 1, R3-R9 | 1, 6-10, 19-23, 45 |
| Y | claims, compounds 21, 23, 25, 31, 33, 34, 53, etc., examples, definition of chemical formula 1, R3-R9 | 8-10, 19-23, 45 |
| Y | US 2015/0270498 A1 (SAMSUNG DISPLAY CO., LTD.) 24 September 2015 (2015-09-24) claims, examples, table 2 | 8-10, 17-23, 45 |
| P, X | WO 2021/025162 A1 (IDEMITSU KOSAN CO) 11 February 2021 (2021-02-11) claims, paragraphs [0169], [0177], compound BH-4, examples | 1, 4-7, 19-23, 45 |
| P, X | WO 2021/090934 A1 (IDEMITSU KOSAN CO) 14 May 2021 (2021-05-14) claims, compound PY3, examples | 1, 4-7, 17-23, 45 |
| P, X | WO 2021/132535 A1 (IDEMITSU KOSAN CO) 01 July 2021 (2021-07-01) claims, compound 1BH-8, examples | 1, 4-7, 17-23, 45 |
| E, X | WO 2021/201176 A1 (IDEMITSU KOSAN CO) 07 October 2021 (2021-10-07) claims, examples, synthesis examples 3-5 | 1, 4-10, 17-23, 45 |
| E, X | WO 2021/210305 A1 (IDEMITSU KOSAN CO) 21 October 2021 (2021-10-21) claims, examples, compounds BH1-90 to BH1-93 | 1, 4-7, 17-23, 45 |

Form PCT/ISA/210 (second sheet) (January 2015)

**INTERNATIONAL SEARCH REPORT**

International application No.
**PCT/JP2021/036397**

**Box No. III Observations where unity of invention is lacking (Continuation of item 3 of first sheet)**

This International Searching Authority found multiple inventions in this international application, as follows:

Examining the relationship among independent claims 1, 11, and 24, a common technical feature shared by the invention in claim 1 and the invention in the other independent claims is that the inventions pertain to a compound in which a group having at least one fused ring group is bonded to a benzo[de]anthracene derivative skeleton.

However, the compound having the aforementioned structure is disclosed in documents 1-4 (document 1: claims, compounds A2, A3, A8, A13, A21, B1, B2, B4, C1, C5, D1, D5, D6, D9, and D10, and examples; document 2: claims, compounds 54 and 99, and examples; document 3: claims, compound 106, and examples; and document 4: claims and compounds 57, 58, 61-63, and 66), and thus said technical feature is not a special technical feature.

Moreover, there are no other same or corresponding special technical features among these independent claims.

Therefore, the inventions in the aforementioned independent claims cannot be said to be linked so as to form a single general inventive concept, and the present invention is considered to disclose the following three different inventions that do not comply with the requirement of unity of invention.

(Invention 1) Claims 1-10 and parts referring to any of claims 1-10 in claims 17-23 and 45

The invention pertaining to: a compound having at least one group represented by general formula (11) and containing only one benzo[de]anthracene derivative skeleton represented by general formula (1000) in a molecule; or an organic electroluminescent element including said compound

(Invention 2) Claims 11-16 and parts referring to any of claims 11-16 in claims 17-23 and 45

The invention pertaining to: a compound having at least one group represented by general formula (110A) and containing only one benzo[de]anthracene derivative skeleton represented by general formula (1000A) in a molecule; or an organic electroluminescent element including said compound

(Invention 3) Claims 24-44 and parts referring to any of claims 24-44 in claim 45

The invention pertaining to an organic electroluminescent element that includes a positive electrode, a negative electrode, and a light emitting layer disposed between the positive electrode and the negative electrode, wherein the light emitting layer includes a first light emitting layer and a second light emitting layer, the first light emitting layer contains a first compound, and the first compound has at least one group represented by general formula (110) and is a compound represented by general formula (1000B)

Document 1: JP 2014-009220 A (CANON INC)
Document 2: KR 10-2019-0055686 A (NANJING TOPTOMATERIALS CO., LTD.)
Document 3: CN 107827853 A (NANJING TOPTOSEMICONDUCTOR MATERIAL CO., LTD.)
Document 4: US 2015/0001479 A1 (SAMSUNG DISPLAYCO., LTD.)

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **(Invention 1) Claims 1-10 and parts referring to any of claims 1-10 in claims 17-23 and 45**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
**Information on patent family members**

International application No.
**PCT/JP2021/036397**

| Patent document cited in search report | Publication date (day/month/year) | Patent family member(s) | Publication date (day/month/year) |
|---|---|---|---|
| JP 2014-009220 A | 20 January 2014 | US 2015/0194609 A1<br>claims, compounds 2, A3, A8, A13, A21, B1, B2, B4, C1, C5, D1, D5, D6, D9, D10, examples<br>WO 2014/007186 A1<br>EP 2870149 A1<br>CN 104411694 A<br>KR 10-2015-0024426 A | |
| KR 10-2019-0055686 A | 23 May 2019 | CN 108148037 A | |
| CN 107827853 A | 23 March 2018 | KR 10-2019-0055685 A | |
| US 2015/0001479 A1 | 01 January 2015 | KR 10-2015-0003564 A | |
| WO 2015/122711 A1 | 20 August 2015 | KR 10-2015-0096152 A | |
| CN 103102299 A | 15 May 2013 | (Family: none) | |
| JP 2013-528927 A | 11 July 2013 | WO 2011/115378 A1<br>claims, compounds 21, 23, 25, 31, 33, 34, 53, etc., examples, definition of chemical formula 1, R3-R9<br>KR 10-2011-0104765 A<br>TW 201211058 A<br>CN 102933530 A | |
| US 2015/0270498 A1 | 24 September 2015 | KR 10-2015-0109522 A | |
| WO 2021/025162 A1 | 11 February 2021 | WO 2021/025163 A1 | |
| WO 2021/090934 A1 | 14 May 2021 | (Family: none) | |
| WO 2021/132535 A1 | 01 July 2021 | (Family: none) | |
| WO 2021/201176 A1 | 07 October 2021 | (Family: none) | |
| WO 2021/210305 A1 | 21 October 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2015270498
- US 2015001479
- US 2015069344
- US 2017331051
- WO 2014104144 A